# EUROPEAN PATENT SPECIFICATION

(11) **EP 3 630 130 B1**
(45) Date of publication and mention of the grant of the patent: **31.08.2022**
(21) Application number: 18809986.5
(22) Date of filing: 01.06.2018
(51) Int. Cl.: A61K 39/00, C07K 16/30, A61P 35/00, C07K 16/18, C07K 16/28, G01N 33/50

(54) **A METHOD TO CREATE PERSONALIZED CANCER VACCINES**
VERFAHREN ZUR ERZEUGUNG VON PERSONALISIERTEN KREBSIMPFSTOFFEN
PROCÉDÉ DE CRÉATION DE VACCINS ANTICANCÉREUX PERSONNALISÉS

(30) Priority: 02.06.2017 US 201762514689 P; 02.06.2017 US 201762514679 P
(43) Date of publication of application: 08.04.2020
(73) Proprietor: Arizona Board of Regents on behalf of Arizona State University, Scottsdale, AZ 85257-9908 (US)
(72) Inventor: JOHNSTON, Stephen Albert, Tempe, Arizona 85284 (US); SHEN, Luhui, Tempe, Arizona 85284 (US)
(74) Representative: Avidity IP
(86) International application number: PCT/US2018/035741
(87) International publication number: WO 2018/223092

(56) References cited:
- AU-A1- 2015 264 880
- US-A1- 2015 079 119
- US-A1- 2015 241 420
- Jian Zhang ET AL: "Frameshift Antigens for Cancer Vaccine Development", , 31 May 2018 (2018-05-31), XP055767123, Retrieved from the Internet: URL:https://core.ac.uk/download/pdf/157755 541.pdf [retrieved on 2021-01-20]
- YANNELLI J R ET AL: "Development of an autologous canine cancer vaccine system for resectable malignant tumors in dogs", VETERINARY IMMUNOLOGY AND IMMUNOPATHOLOGY, vol. 182, 31 December 2016 (2016-12-31), pages 95-100, XP029810135, ISSN: 0165-2427, DOI: 10.1016/J.VETIMM.2016.10.011
- Duan Hu ET AL: "Early Detection and Treatment of Breast Cancer by Random Peptide Array in neuN Transgenic Mouse Model", , 30 June 2015 (2015-06-30), XP055779522, Retrieved from the Internet: URL:https://core.ac.uk/download/pdf/795812 92.pdf [retrieved on 2021-02-24]
- WHITTEMORE KURT ET AL: "A General Method to Discover Epitopes from Sera", PLOS ONE, vol. 11, no. 6, 14 June 2016 (2016-06-14), page e0157462, XP055779544, DOI: 10.1371/journal.pone.0157462 Retrieved from the Internet: URL:https://storage.googleapis.com/plos-co rpus-prod/10.1371/journal.pone.0157462/1/p one.0157462.pdf?X-Goog-Algorithm=GOOG4-RSA -SHA256&X-Goog-Credential=wombat-sa@plos-p rod.iam.gserviceaccount.com/20210224/auto/ storage/goog4_request&X-Goog-Date=20210224 T160956Z&X-Goog-Expires=3600&X-Goog-Signed Headers=ho>
- MASATOSHI HIRAYAMA ET AL: "The present status and future prospects of peptide-based cancer vaccines", INTERNATIONAL IMMUNOLOGY, vol. 28, no. 7, 28 May 2016 (2016-05-28), pages 319-328, XP055515960, GB ISSN: 0953-8178, DOI: 10.1093/intimm/dxw027
- GUO, C et al.: "Therapeutic Cancer Vaccines: Past, Present and Future", Advances in Cancer Research, vol. 119, 2013, pages 421-475, XP055540460,

## Description

The present invention relates to personalized vaccine compositions for use in treating cancer or for determining wether an individual has a cancer.

### BACKGROUND

There is a resurgence of enthusiasm to use the immune system to treat cancer. This is most notable with respect to using checkpoint inhibitors (as antibodies) to release the existing immune response in the patient to their own tumor. Yet, only a small proportion of the patients treated have a positive response. The evidence to date is that whether a patient has an effective response to the treatment depends on the nature of the immune response they have established against the tumor. More specifically, the level and quality of the immune response to neopeptides in the cancer seems to be most important. This has led to the concept of personal cancer vaccines to bolster the response of the inhibitors and therefore, to increase the response rate. Yannelli et al, Vet Immunology and Immunopathology, v 182, 95 - 100 (2016), AU2015/264880, US2015/079119 and US2015/241420 disclose vaccines, methods of detection of cancer or of peptides.

### SUMMARY

The present invention provides a personalized vaccine composition for use in reducing tumor size, inhibiting tumor growth, reducing tumor burden, increasing survival, or increasing cancer-free survival in an individual wherein the vaccine composition comprises: one or more peptides determined to have immunoreactivity with a biological sample of the cancer from the individual, wherein the one or more peptides comprise peptides encoded by a frameshifted mRNA expressed by a cancer cell, and wherein the one or more peptides comprise peptides having a sequence selected from SEQ ID NO: 1-7264, and wherein administering the vaccine composition elicits an immune response in the individual against the cancer. The present invention is as set out in the claims. Other disclosures herein not falling within the claims are for the assistance of the skilled person in understanding and practicing the disclosed invention. For the avoidance of doubt, it is noted that the present invention does not relate to methods of treatment of the human or animal body. Also disclosed are methods of treating an individual in need of treatment for cancer using such compositions. Some such methods comprising, a) identifying peptides that are immunoreactive with a biological sample from the individual in a first population of peptides; b) preparing a vaccine composition comprising a second population of peptides comprising one or more peptides identified in step a) or a nucleic acid sequence encoding the second population of peptides; and c) administering an effective amount of the vaccine composition to the individual, thereby treating the cancer. In some cases, the method comprises obtaining the biological sample from the individual. Often, treating the cancer comprises reducing tumor size, inhibiting tumor growth, reducing tumor burden, increasing survival, or increasing cancer-free survival. In some cases, administering the vaccine composition elicits an immune response in the individual against the cancer. Sometimes, the second population of peptides is a subpopulation of the first population. Often, the identified peptide elicits a positive response in an antibody assay or a T cell assay performed on the biological sample from the individual. In some cases, the biological sample is selected from the group consisting of blood, plasma, serum, thymus, bone marrow, spleen, lymph node, bronchoalveolar lavage, breast, central nervous system, cerebrospinal fluid, eye, tears, gastrointestinal tract, saliva, feces, urine, heart, kidney, liver, lung, muscle, pancreas, peripheral nervous system, saliva, skin, thyroid, trachea, and tumor. Often, the biological sample comprises cells selected from B cells, T cells, CD4+ T cells, CD8+ T cells, Th17 cells, and combinations thereof. Alternatively and in combination, the biological sample comprises an antibody. Often, identifying comprises determining immunoreactivity of the first population of peptides to the biological sample using antibody reactivity. In some cases, antibody reactivity is detected using an antibody assay selected from the group consisting of ELISA, radio-immuno assay, western blot, surface plasmon resonance, immunostaining, immunoprecipitation, mass spectrometry, phage display, ELISPOT, flow cytometry, cytometric bead array, immunohistochemistry, high density array, microarray, delayed-type hypersensitivity (DTH), and combinations thereof. Alternately, or in combination, identifying comprises determining immunoreactivity of the first population of peptides to the biological sample using a T cell response. Often, the T cell response is detected using an assay selected from the group consisting of proliferation assay, 3H-thymidine assay, BrdU assay, CFSE assay, cytokine secretion assay, ELISA assay, ELISPOT assay, intracellular staining assay, quantitative rtPCR assay, cytometric bead array assay, cytotoxicity assay, 51-chromium assay, degranulation assay, granulysin assay, granzyme A assay, granzyme B assay, and perforin assay. In some cases, the first population of peptides comprises peptides encoded by a frameshifted mRNA expressed by a cancer cell. Sometimes, a frameshifted mRNA is created in a splicing error or a transcription insertion or deletion error. Often, the first population of peptides comprises peptides having a sequence selected from SEQ ID NO: 1-7264. In some cases, each of the first population of peptides binds to at least one MHC subtype. Sometimes, each of the first population of peptides comprises at least one T cell epitope. In some cases, each of the first population of peptides comprises at least on B cell epitope. Often, the first population of peptides is bound to a substrate. In some cases, the first population of peptides is part of an array or a phage display library. Often, the vaccine composition comprises a pharmaceutically acceptable adjuvant or excipient. Often, the adjuvant is selected from the group consisting of ABM2, AS01B, AS02, AS02A, Adjumer, Adjuvax, Algammulin, Alum, Aluminum phosphate, Aluminum potassium sulfate, Bordetella pertussis, Calcitriol, Chitosan, Cholera toxin, CpG, Dibutyl phthalate, Dimethyldioctadecylammonium bromide (DDA), Freund's adjuvant, Freund's complete, Freund's incomplete (IFA), GM-CSF, GMDP, Gamma Inulin, Glycerol, HBSS (Hank's Balanced Salt Solution), IL-12, IL-2, Imiquimod, Interferon-Gamma, ISCOM, Lipid Core Peptide (LCP), Lipofectin, Lipopolysaccharide (LPS), Liposomes, MF59, MLP+TDM, Monophosphoryl lipid A, Montanide IMS-1313, Montanide ISA 206, Montanide ISA 720, Montanide ISA-51, Montanide ISA-50, nor-MDP, Oil-in-water emulsion, P1005 (non-ionic copolymer), Pam3Cys (lipoprotein), Pertussis toxin, Poloxamer, QS21, RaLPS, Ribi, Saponin, Seppic ISA 720, Soybean Oil, Squalene, Syntex Adjuvant Formulation (SAF), Synthetic polynucleotides (poly IC/poly AU), TiterMax Tomatine, Vaxfectin, XtendIII, and Zymosan. In some cases, the vaccine composition comprises an immune checkpoint inhibitor. Often, the immune checkpoint inhibitor is selected from one or more of the group consisting of a PD-1 inhibitor, a PD-L1 inhibitor, and a CTLA-4 inhibitor. Often, the immune checkpoint inhibitor is selected from one or more of the group consisting of Pembrolizumab, Nivolumab, and Atezolizumab. Sometimes, the vaccine is administered via a route selected from the group consisting of subcutaneous, intradermal, intramuscular, intranasal, intravenous, and sublingual. In some cases, the individual is a mammal. In some cases, the individual is a human, a cat, a mouse, a rat, a rabbit, a horse, a cow, or a pig. Often, the cancer is selected from the group consisting of Acute lymphoblastic leukemia, Acute monocytic leukemia, Acute myeloid leukemia, Acute promyelocytic leukemia, Adenocarcinoma, Adult T-cell leukemia, Astrocytoma, Bladder cancer, Bone Cancer, Brain Tumor, Breast Cancer, Burkitt's lymphoma, Carcinoma, Cervical Cancer, Chronic Lymphocytic Leukemia, Chronic myelogenous leukemia, Colon Cancer, Colorectal cancer, Endometrial cancer, Glioblastoma multiforme, Glioma, Hepatocellular carcinoma, Hodgkin's lymphoma, Inflammatory breast cancer, Kidney Cancer, Leukemia, Lung cancer, Lymphoma, Malignant Mesothelioma, Medulloblastoma, Melanoma, Multiple myeloma, Neuroblastoma, Non-Hodgkin Lymphoma, Non-Small Cell Lung Cancer, Ovarian Cancer, Pancreatic Cancer, Pituitary tumor, Prostate cancer, Retinoblastoma, Skin Cancer, Small Cell Lung Cancer, Squamous cell carcinoma, Stomach cancer, T-cell leukemia, T-cell lymphoma, Thyroid cancer, and Wilms' tumor.

Also described herein are methods of eliciting an immune response in an individual having cancer. Some such methods comprising, a) identifying peptides that are immunoreactive with a biological sample in a first population of peptides; b) preparing a vaccine composition comprising a second population of peptides comprising one or more peptides identified in step a) or a nucleic acid sequence encoding the second population of peptides; and c) administering the vaccine composition to the individual, wherein administering the vaccine composition elicits an immune response in the individual against the cancer. In some cases, the method comprises obtaining the biological sample from the individual. Often, the method comprises treating the cancer. In some cases, treating the cancer comprises reducing tumor size, inhibiting tumor growth, reducing tumor burden, increasing survival, or increasing cancer-free survival. Often, the second population of peptides is a subpopulation of the first population. In some cases, the identified peptide elicits a positive response in an antibody assay or a T cell assay performed on the biological sample from the individual. In some cases, the immune response is a T cell response. Alternately, or in combination, the immune response is an antibody response. Often, the immune response is directed to a cancer. In some cases, the cancer is selected from the group consisting of Acute lymphoblastic leukemia, Acute monocytic leukemia, Acute myeloid leukemia, Acute promyelocytic leukemia, Adenocarcinoma, Adult T-cell leukemia, Astrocytoma, Bladder cancer, Bone Cancer, Brain Tumor, Breast Cancer, Burkitt's lymphoma, Carcinoma, Cervical Cancer, Chronic Lymphocytic Leukemia, Chronic myelogenous leukemia, Colon Cancer, Colorectal cancer, Endometrial cancer, Glioblastoma multiforme, Glioma, Hepatocellular carcinoma, Hodgkin's lymphoma, Inflammatory breast cancer, Kidney Cancer, Leukemia, Lung cancer, Lymphoma, Malignant Mesothelioma, Medulloblastoma, Melanoma, Multiple myeloma, Neuroblastoma, Non-Hodgkin Lymphoma, Non-Small Cell Lung Cancer, Ovarian Cancer, Pancreatic Cancer, Pituitary tumor, Prostate cancer, Retinoblastoma, Skin Cancer, Small Cell Lung Cancer, Squamous cell carcinoma, Stomach cancer, T-cell leukemia, T-cell lymphoma, Thyroid cancer, and Wilms' tumor. Often, the biological sample is selected from the group consisting of blood, plasma, serum, thymus, bone marrow, spleen, lymph node, bronchoalveolar lavage, breast, central nervous system, cerebrospinal fluid, eye, tears, gastrointestinal tract, saliva, feces, urine, heart, kidney, liver, lung, muscle, pancreas, peripheral nervous system, saliva, skin, thyroid, trachea, and tumor. In some cases, the biological sample comprises cells selected from B cells, T cells, CD4+ T cells, CD8+ T cells, Th17 cells, and combinations thereof. Alternately, or in combination, the biological sample comprises an antibody. Often, identifying comprises determining immunoreactivity of the first population of peptides to the biological sample using antibody reactivity. In some cases, antibody reactivity is detected using an antibody assay selected from the group consisting of ELISA, radio-immuno assay, western blot, surface plasmon resonance, immunostaining, immunoprecipitation, mass spectrometry, phage display, flow cytometry, cytometric bead array, immunohistochemistry, high density array, microarray and combinations thereof. In some cases, identifying comprises determining immunoreactivity of the first population of peptides to the biological sample using a T cell response. In some cases, the T cell response is detected using an assay selected from the group consisting of proliferation assay, 3H-thymidine assay, BrdU assay, CFSE assay, cytokine secretion assay, ELISPOT assay, intracellular staining assay, quantitative rtPCR assay, cytometric bead array assay, cytotoxicity assay, 51-chromium assay, degranulation assay, granulysin assay, granzyme A assay, granzyme B assay, and perforin assay. Often, the first population of peptides comprises peptides encoded by a frameshifted mRNA expressed by a cancer cell. In some cases, a frameshifted mRNA is created in a splicing error or a transcription insertion or deletion error in a microsatellite. Often, the first population of peptides comprises peptides having a sequence selected from SEQ ID NO: in 1-7264. In some cases, each of the first population of peptides binds to at least one MHC subtype. Often, each of the first population of peptides comprises at least one T cell epitope. In some cases, each of the first population of peptides comprises at least on B cell epitope. Often, the first population of peptides is bound to a substrate. In some cases, the first population of peptides is part of an array or a phage display library. Often, the vaccine composition comprises a pharmaceutically acceptable adjuvant or excipient. Sometimes, the adjuvant is selected from the group consisting of ABM2, AS01B, AS02, AS02A, Adjumer, Adjuvax, Algammulin, Alum, Aluminum phosphate, Aluminum potassium sulfate, Bordetella pertussis, Calcitriol, Chitosan, Cholera toxin, CpG, Dibutyl phthalate, Dimethyldioctadecylammonium bromide (DDA), Freund's adjuvant, Freund's complete, Freund's incomplete (IFA), GM-CSF, GMDP, Gamma Inulin, Glycerol, HBSS (Hank's Balanced Salt Solution), IL-12, IL-2, Imiquimod, Interferon-Gamma, ISCOM, Lipid Core Peptide (LCP), Lipofectin, Lipopolysaccharide (LPS), Liposomes, MF59, MLP+TDM, Monophosphoryl lipid A, Montanide IMS-1313, Montanide ISA 206, Montanide ISA 720, Montanide ISA-51, Montanide ISA-50, nor-MDP, Oil-in-water emulsion, P1005 (non-ionic copolymer), Pam3Cys (lipoprotein), Pertussis toxin, Poloxamer, QS21, RaLPS, Ribi, Saponin, Seppic ISA 720, Soybean Oil, Squalene, Syntex Adjuvant Formulation (SAF), Synthetic polynucleotides (poly IC/poly AU), TiterMax Tomatine, Vaxfectin, XtendIII, and Zymosan. Often, the vaccine composition comprises an immune checkpoint inhibitor. In some cases, the immune checkpoint inhibitor is selected from one or more of the group consisting of a PD-1 inhibitor, a PD-L1 inhibitor, and a CTLA-4 inhibitor. In some cases, the immune checkpoint inhibitor is selected from one or more of the group consisting of Pembrolizumab, Nivolumab, and Atezolizumab. Often, the vaccine is administered via a route selected from the group consisting of subcutaneous, intradermal, intramuscular, intranasal, intravenous, orally, intradermal and sublingual. In some cases, the individual is a mammal. Often, the individual is a human, a cat, a mouse, a rat, a rabbit, a horse, a cow, or a pig.

Also described herein are methods of reducing risk of developing cancer in an individual. Some such methods comprising, a) identifying peptides that are immunoreactive with a biological sample in a first population of peptides; b) preparing a vaccine composition comprising a second population of peptides comprising one or more peptides identified in step a) or a nucleic acid sequence encoding the second population of peptides; and c) administering the vaccine composition to the individual, wherein administering the vaccine composition elicits an immune response in the individual against the cancer and wherein the risk of developing cancer in the individual is reduced compared to an individual who did not receive the vaccine. In some cases, the method comprises obtaining the biological sample from the individual. Often, administering the vaccine composition elicits an immune response in the individual against the cancer. In some cases, the second population of peptides is a subpopulation of the first population. Often, the identified peptide elicits a positive response in an antibody assay or a T cell assay performed on the biological sample from the individual. Sometimes, the biological sample is selected from the group consisting of blood, plasma, serum, thymus, bone marrow, spleen, lymph node, bronchoalveolar lavage, breast, central nervous system, cerebrospinal fluid, eye, tears, gastrointestinal tract, saliva, feces, urine, heart, kidney, liver, lung, muscle, pancreas, peripheral nervous system, saliva, skin, thyroid, trachea, and tumor. Often, the biological sample comprises cells selected from B cells, T cells, CD4+ T cells, CD8+ T cells, Th17 cells, and combinations thereof. In some cases, the biological sample comprises an antibody. Often, identifying comprises determining immunoreactivity of the first population of peptides to the biological sample using antibody reactivity. In some cases, antibody reactivity is detected using an antibody assay selected from the group consisting of ELISA, radio-immuno assay, western blot, surface plasmon resonance, immunostaining, immunoprecipitation, mass spectrometry, phage display, ELISPOT, flow cytometry, cytometric bead array, immunohistochemistry, high density array, microarray, delayed-type hypersensitivity (DTH), and combinations thereof. Often, identifying comprises determining immunoreactivity of the first population of peptides to the biological sample using a T cell response. In some cases, the T cell response detected using an assay selected from the group consisting of proliferation assay, 3H-thymidine assay, BrdU assay, CFSE assay, cytokine secretion assay, ELISA assay, ELISPOT assay, intracellular staining assay, quantitative rtPCR assay, cytometric bead array assay, cytotoxicity assay, 51-chromium assay, degranulation assay, granulysin assay, granzyme A assay, granzyme B assay, and perforin assay. In some cases, the first population of peptides comprises peptides encoded by a frameshifted mRNA expressed by a cancer cell. Often, a frameshifted mRNA is created in a splicing error or aby a transcription insertion or deletion error in a microsatellite. In some cases, the first population of peptides comprises peptides having a sequence selected from SEQ ID NO: 1-7264. Often, each of the first population of peptides binds to at least one MHC subtype. In some cases, each of the first population of peptides comprises at least one T cell epitope. Alternately, or in combination, each of the first population of peptides comprises at least on B cell epitope. In some cases, the first population of peptides is bound to a substrate. Often, the first population of peptides is part of an array or a phage display library. Often, the vaccine composition comprises a pharmaceutically acceptable adjuvant or excipient. In some cases, the adjuvant is selected from the group consisting of ABM2, AS01B, AS02, AS02A, Adjumer, Adjuvax, Algammulin, Alum, Aluminum phosphate, Aluminum potassium sulfate, Bordetella pertussis, Calcitriol, Chitosan, Cholera toxin, CpG, Dibutyl phthalate, Dimethyldioctadecylammonium bromide (DDA), Freund's adjuvant, Freund's complete, Freund's incomplete (IFA), GM-CSF, GMDP, Gamma Inulin, Glycerol, HBSS (Hank's Balanced Salt Solution), IL-12, IL-2, Imiquimod, Interferon-Gamma, ISCOM, Lipid Core Peptide (LCP), Lipofectin, Lipopolysaccharide (LPS), Liposomes, MF59, MLP+TDM, Monophosphoryl lipid A, Montanide IMS-1313, Montanide ISA 206, Montanide ISA 720, Montanide ISA-51, Montanide ISA-50, nor-MDP, Oil-in-water emulsion, P1005 (non-ionic copolymer), Pam3Cys (lipoprotein), Pertussis toxin, Poloxamer, QS21, RaLPS, Ribi, Saponin, Seppic ISA 720, Soybean Oil, Squalene, Syntex Adjuvant Formulation (SAF), Synthetic polynucleotides (poly IC/poly AU), TiterMax Tomatine, Vaxfectin, XtendIII, and Zymosan. Often, the vaccine composition comprises an immune checkpoint inhibitor. In some cases, the immune checkpoint inhibitor is selected from one or more of the group consisting of a PD-1 inhibitor, a PD-L1 inhibitor, and a CTLA-4 inhibitor. Often, the immune checkpoint inhibitor is selected from one or more of the group consisting of Pembrolizumab, Nivolumab, and Atezolizumab. In some cases, the vaccine is administered via a route selected from the group consisting of subcutaneous, intradermal, intramuscular, intranasal, intravenous, and sublingual. Often, the individual is a mammal. In some cases, the individual is a human, a cat, a mouse, a rat, a rabbit, a horse, a cow, or a pig. In some cases, the cancer is selected from the group consisting of Acute lymphoblastic leukemia, Acute monocytic leukemia, Acute myeloid leukemia, Acute promyelocytic leukemia, Adenocarcinoma, Adult T-cell leukemia, Astrocytoma, Bladder cancer, Bone Cancer, Brain Tumor, Breast Cancer, Burkitt's lymphoma, Carcinoma, Cervical Cancer, Chronic Lymphocytic Leukemia, Chronic myelogenous leukemia, Colon Cancer, Colorectal cancer, Endometrial cancer, Glioblastoma multiforme, Glioma, Hepatocellular carcinoma, Hodgkin's lymphoma, Inflammatory breast cancer, Kidney Cancer, Leukemia, Lung cancer, Lymphoma, Malignant Mesothelioma, Medulloblastoma, Melanoma, Multiple myeloma, Neuroblastoma, Non-Hodgkin Lymphoma, Non-Small Cell Lung Cancer, Ovarian Cancer, Pancreatic Cancer, Pituitary tumor, Prostate cancer, Retinoblastoma, Skin Cancer, Small Cell Lung Cancer, Squamous cell carcinoma, Stomach cancer, T-cell leukemia, T-cell lymphoma, Thyroid cancer, and Wilms' tumor.

Also described herein are methods of designing a personalized vaccine for an individual. Some such methods comprise a) identifying peptides that are immunoreactive with a biological sample in a first population of peptides; b) preparing a vaccine composition comprising a second population of peptides comprising one or more peptides identified in step a) or a nucleic acid sequence encoding the second population of peptides. In some cases, the method comprises obtaining the biological sample from the individual. Often, administration of an effective amount of the vaccine treats the cancer in the individual. In some cases, the cancer comprises reducing tumor size, inhibiting tumor growth, reducing tumor burden, increasing survival, or increasing cancer-free survival. Often, administering the vaccine composition elicits an immune response in the individual against the cancer. Sometimes, the second population of peptides is a subpopulation of the first population. Often, the identified peptide elicits a positive response in an antibody assay or a T cell assay performed on the biological sample from the individual. In some cases, the biological sample is selected from the group consisting of blood, plasma, serum, thymus, bone marrow, spleen, lymph node, bronchoalveolar lavage, breast, central nervous system, cerebrospinal fluid, eye, tears, gastrointestinal tract, saliva, feces, urine, heart, kidney, liver, lung, muscle, pancreas, peripheral nervous system, saliva, skin, thyroid, trachea, and tumor. Often, the biological sample comprises cells selected from B cells, T cells, CD4+ T cells, CD8+ T cells, Th17 cells, and combinations thereof. Alternately or in combination, the biological sample comprises an antibody. Often, identifying comprises determining immunoreactivity of the first population of peptides to the biological sample using antibody reactivity. In some cases, antibody reactivity is detected using an antibody assay selected from the group consisting of ELISA, radio-immuno assay, western blot, surface plasmon resonance, immunostaining, immunoprecipitation, mass spectrometry, phage display, flow cytometry, cytometric bead array, immunohistochemistry, high density array, microarray, and combinations thereof. Often, identifying comprises determining immunoreactivity of the first population of peptides to the biological sample using a T cell response. In some cases, the T cell response is detected using an assay selected from the group consisting of proliferation assay, 3H-thymidine assay, BrdU assay, CFSE assay, cytokine secretion assay, ELISPOT assay, intracellular staining assay, quantitative rtPCR assay, cytometric bead array assay, cytotoxicity assay, 51-chromium assay, degranulation assay, granulysin assay, granzyme A assay, granzyme B assay, and perforin assay. In some cases, the first population of peptides comprises peptides encoded by a frameshifted mRNA expressed by a cancer cell. Often, a frameshifted mRNA is created by a transcription insertion or deletion error in a coding region microsatellite. In some cases, the first population of peptides comprises peptides having a sequence selected from SEQ ID NO: 1-7264. In some cases, each of the first population of peptides binds to at least one MHC subtype. Often, each of the first population of peptides comprises at least one T cell epitope. In some cases, each of the first population of peptides comprises at least on B cell epitope. In some cases, the first population of peptides is bound to a substrate. Often, the first population of peptides is part of an array or a phage display library. In some cases, the vaccine composition comprises a pharmaceutically acceptable adjuvant or excipient. Often, the adjuvant is selected from the group consisting of ABM2, AS01B, AS02, AS02A, Adjumer, Adjuvax, Algammulin, Alum, Aluminum phosphate, Aluminum potassium sulfate, Bordetella pertussis, Calcitriol, Chitosan, Cholera toxin, CpG, Dibutyl phthalate, Dimethyldioctadecylammonium bromide (DDA), Freund's adjuvant, Freund's complete, Freund's incomplete (IFA), GM-CSF, GMDP, Gamma Inulin, Glycerol, HBSS (Hank's Balanced Salt Solution), IL-12, IL-2, Imiquimod, Interferon-Gamma, ISCOM, Lipid Core Peptide (LCP), Lipofectin, Lipopolysaccharide (LPS), Liposomes, MF59, MLP+TDM, Monophosphoryl lipid A, Montanide IMS-1313, Montanide ISA 206, Montanide ISA 720, Montanide ISA-51, Montanide ISA-50, nor-MDP, Oil-in-water emulsion, P1005 (non-ionic copolymer), Pam3Cys (lipoprotein), Pertussis toxin, Poloxamer, QS21, RaLPS, Ribi, Saponin, Seppic ISA 720, Soybean Oil, Squalene, Syntex Adjuvant Formulation (SAF), Synthetic polynucleotides (poly IC/poly AU), TiterMax Tomatine, Vaxfectin, XtendIII, and Zymosan. Often, the vaccine composition includes an immune checkpoint inhibitor. In some cases, the immune checkpoint inhibitor is selected from one or more of the group consisting of a PD-1 inhibitor, a PD-L1 inhibitor, and a CTLA-4 inhibitor. Often, the immune checkpoint inhibitor is selected from one or more of the group consisting of Pembrolizumab, Nivolumab, and Atezolizumab. In some cases, the method further comprises administration of the personalized vaccine to the individual. Often, the vaccine is administered via a route selected from the group consisting of subcutaneous, intradermal, intramuscular, intranasal, intravenous, and sublingual. In some cases, the individual is a mammal. Often, the individual is a human, a cat, a mouse, a rat, a rabbit, a horse, a cow, or a pig. In some cases, the cancer is selected from the group consisting of Acute lymphoblastic leukemia, Acute monocytic leukemia, Acute myeloid leukemia, Acute promyelocytic leukemia, Adenocarcinoma, Adult T-cell leukemia, Astrocytoma, Bladder cancer, Bone Cancer, Brain Tumor, Breast Cancer, Burkitt's lymphoma, Carcinoma, Cervical Cancer, Chronic Lymphocytic Leukemia, Chronic myelogenous leukemia, Colon Cancer, Colorectal cancer, Endometrial cancer, Glioblastoma multiforme, Glioma, Hepatocellular carcinoma, Hodgkin's lymphoma, Inflammatory breast cancer, Kidney Cancer, Leukemia, Lung cancer, Lymphoma, Malignant Mesothelioma, Medulloblastoma, Melanoma, Multiple myeloma, Neuroblastoma, Non-Hodgkin Lymphoma, Non-Small Cell Lung Cancer, Ovarian Cancer, Pancreatic Cancer, Pituitary tumor, Prostate cancer, Retinoblastoma, Skin Cancer, Small Cell Lung Cancer, Squamous cell carcinoma, Stomach cancer, T-cell leukemia, T-cell lymphoma, Thyroid cancer, and Wilms' tumor.

### BRIEF DESCRIPTION OF THE DRAWINGS

The invention is set forth in the appended claims.

A better understanding of the features and advantages of the present disclosure will be obtained by reference to the following detailed description that sets forth non-limiting and nonexhaustive illustrative cases, in which the principles of the disclosure are utilized, and the accompanying drawings of which:

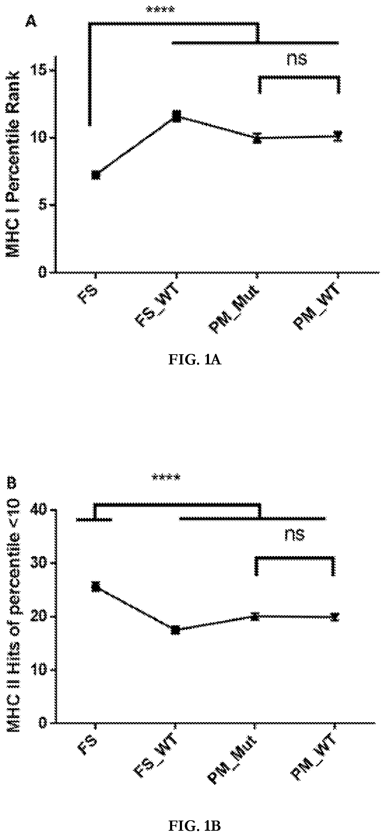

**FIG. 1A** and **FIG. 1B** show a bioinformatics comparison demonstrating that FS are superior neo-antigens to point mutations. MHC Class I and II prediction for MS FS peptides and point mutation peptides. FS: MS FS peptide, FS _WT: FS peptide paired WT peptide, PM Mut: point mutated peptide, PM _WT: point mutation paired WT peptide. Reference human HLA types are used. **FIG. 1A** describes 1,259 10-aa peptides were generated using the same method as i for MHC I prediction, y axis was the best prediction percentile rank for each peptide, reference human HLA types are used. **FIG. 1B** describes 1,242-point mutation peptides were somatic cancer genome missense point mutations, 20 aa peptides were generated from the point mutation site, same number of 20-aa MS FS peptides and paired WT peptides were used for comparison, y axis was number of hits with percentile rank < 10 in MHC II prediction.

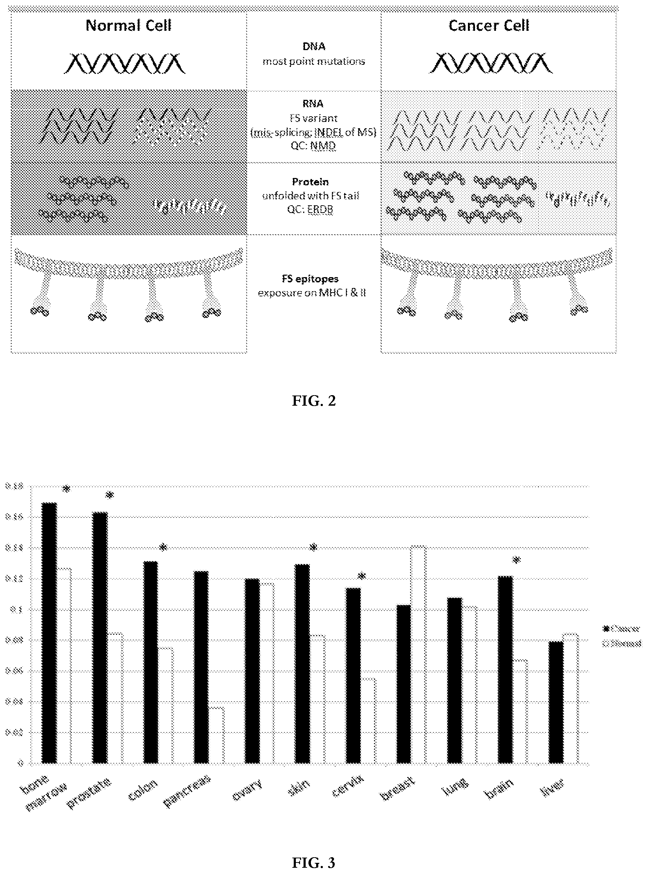

**FIG. 2** shows a model of the FS antigens from the FS RNA variants and illustrates the basic theory behind the importance of RNA generated FS peptides from mis translation through microsatellites. Normal Cell: Errors in DNA replication are very low and repaired. Transcription error rates are higher but also rare as are mis-splicing in intron excision. Any frameshift (FS) transcript with premature stop is the degraded target of nonsense-mediated decay (NMD). Aberrant proteins, including those with frame-shifts are largely eliminated by the protein quality control system. The net result is that very few FS peptides are presented on MHC I/II or escape the cell to be presented to the immune system. Cancer Cell: In general more RNA is produced and increased error rates are observed at the DNA, RNA, and protein levels. More errors are made in DNA replication, but only in dividing cells. Most DNA mutations are single nucleotide changes and encode low or nonimmunogenic peptides. Transcription is generally less accurate, especially through microsatellites (MS), producing insertions and deletions (Indels). Most transcribed genes with MSs in the coding region will have more FS transcripts. RNA splicing is also far less accurate in cancer cells, creating more FS transcripts from each out of frame splicing between exons form the same gene and different genes. The large increase of the FS transcripts from Indels of MS and mis-splicing overwhelms RNA quality control systems, such as NMD. Consequently, more truncated proteins with the FS peptide will be translated. These un-folded proteins, combining with aberrant proteins from other variants, overwhelms the protein quality control system leading to more FS peptides being presented on MHC I/II and mis-secreted or released from the cancer cell and elicit an immune response.
**FIG. 3** shows cancer EST has more MS INDELs than normal EST. An analysis of homopolymer (repeat bp>6) MS INDEL rate in EST database between different cancer and normal libraries. ^{∗} indicate P<0.0001.

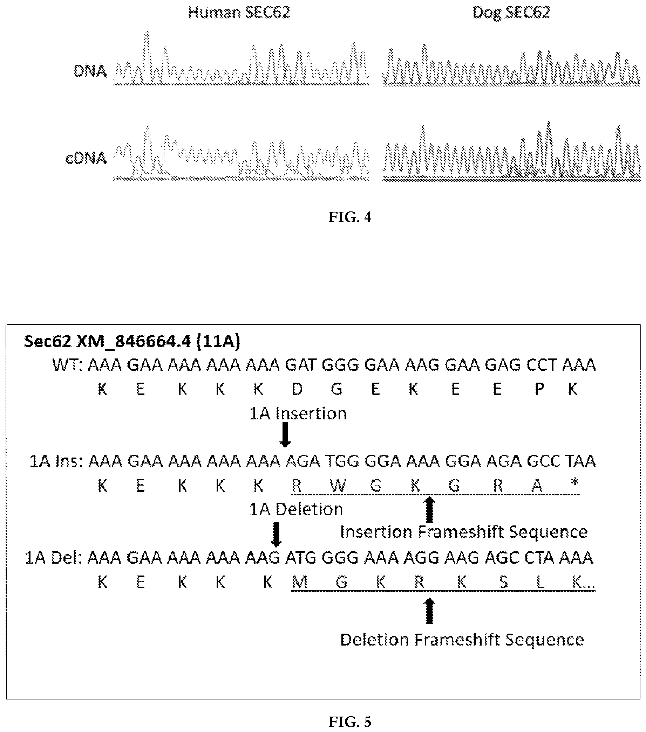

**FIG. 4** shows MS INDELs only exist in RNA not DNA. Examples of the sequence traces of MS region in SEC62 homolog genes in paired DNA/cDNA samples. Four human breast cancer cell lines were sequenced: HTB26, CRL2315, CRL1504, CRL2326; 5 dog mast cell tumors and 3 dog carcinomas. They all showed similar pattern, where the A MSs have an A insertion in RNA not in DNA.
**FIG. 5** shows an example of frameshift peptides produced by either an insertion or deletion in the A MS in the Sec62 gene

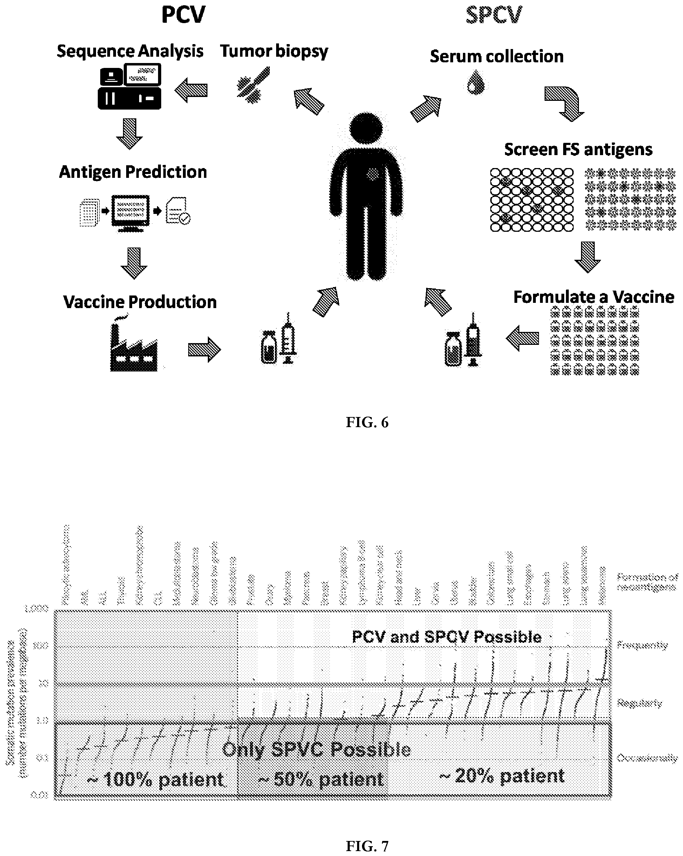

**FIG. 6** shows a comparison of the current approach to making personal cancer vaccines (PCV) versus the much simpler and faster system (SPCV) provided herein.
**FIG. 7** shows SPVC system potentially works with low DNA mutation burden patients, which the regular PCV cannot identify enough neo-antigen for the PCV. The SPCV can cover all cancer patients, including~30% low mutation burden cancer patients.

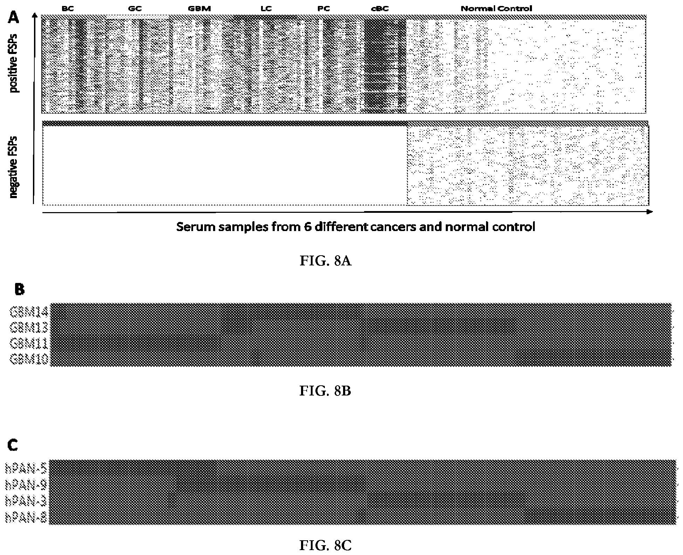

**FIG. 8A, FIG. 8B,** and **FIG. 8C** show 400K FSP array screen antibody reactive in cancer patient and examples of using the SPCV system identify FS antigens for personalized cancer vaccine. **FIG. 8A** shows an example of 500 most common positive FSPs and 500 negative FSPs form 17 serum samples per cancer, BC: breast cancer; GC: gastric cancer; GBM: glioblastoma; LC: lung cancer; PC: pancreatic cancer; cBC: children brain cancer, n=12) and 64 normal control samples. A red spot indicates the serum sample (Y axis) has positive antibody reactive to the FSP (X axis). The positive cut off value of each peptide was calculated by the average plus two fold of standard deviation of the normal controls' signal of that peptide. **FIG. 8B** shows an example of personalize FS antigens selection for 4 GBM patients. 20 FS positive antigens were selected from the highest IgG activity fold change to the cut-off value in each patient. **FIG. 8C** shows an example of personalized FS antigen selection for 4 pancreatic cancer patients.

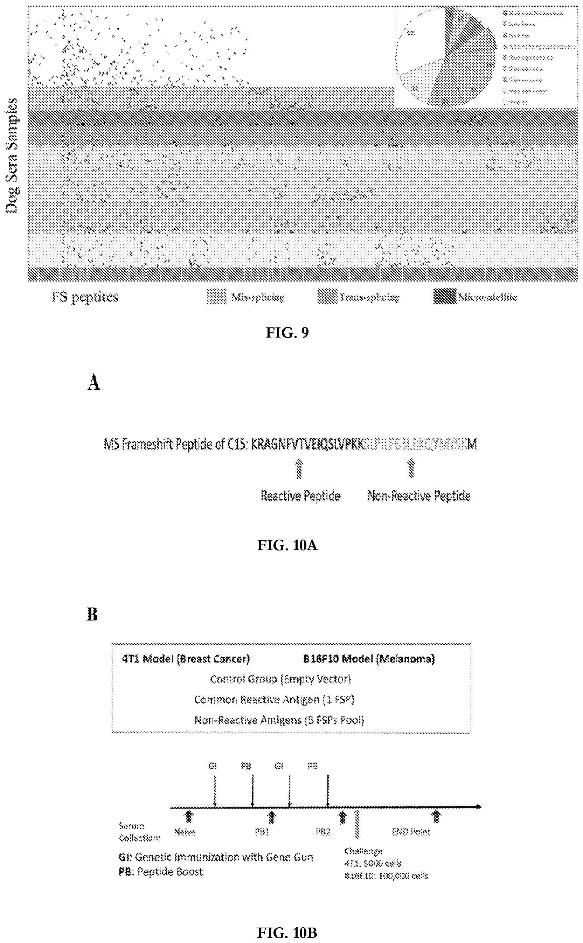

**FIG. 9** shows screen dog cancer serum with a smaller FSP array. The reactive FSPs have protection and correlated with vaccine elicited antibody reactive. The FSP array analysis of the dog serum. 8 types of 116 dog cancer serum and 52 non-cancer serum were analyzed by the small FSP array. Each red spot indicates the serum has positive antibody reactive with this peptide.

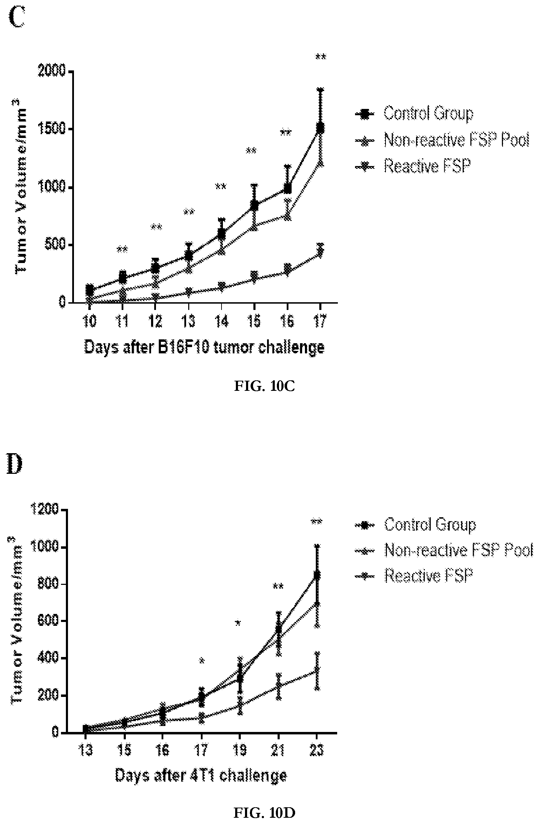

**FIG. 10A, FIG. 10B****,** **FIG. 10C,** and **FIG. 10D** show screen dog cancer serum with a smaller FSP array. Reactive FSPs showed protection in the mouse melanoma and breast cancer models. **FIG. 10A** shows reactive and non-reactive FSPs from the same microsatellite frameshift antigen of CIS. **FIG. 10B** shows 3 groups of mice were used: the control group was immunized with control plasmid, non-reactive FSPs pool and reactive FSP. All 3 groups received 2 rounds of genetic immunization with gene gun and peptide boost via subcutaneous injection. The B16F10 cell line was used for the melanoma model and the 4T1 cell line was used for breast cancer model. Each group had 10 mice. **FIG. 10C** shows reactive FSPs slowed tumor growth significantly compared to the non-reactive FSPs pool and the control group in the melanoma model (student's t-test, p-value <0.01), error bar represented mean ± **SEM.** **FIG. 10D** shows the reactive FSPs offered tumor protection in mouse breast cancer model as well. The tumor volume was significantly lower than control group and non-reactive FSPs pool group (student's t-test, p-value<0.05).

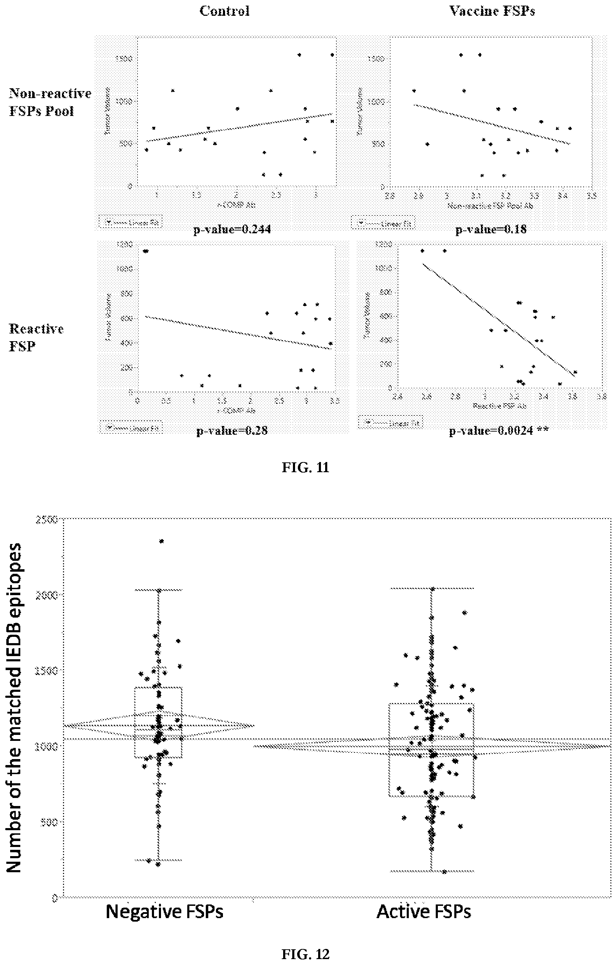

**FIG. 11** shows data screening dog cancer serum with a smaller FSP array. The reactive FSPs were shown to be protective and correlated with vaccine elicited antibody reactivity. Tumor volume was linearly correlated to antibody response against vaccinated reactive FSPs in the 4T1 breast cancer model. Tumor volume at the end point was used for analysis. The antibody response was measured by ELISA after PB2 and at the end point. R-COMP peptide was encoded in a plamsid and used as an internal control for genetic immunization. The p-value was calculated for linear fit of antibody response against tumor volume.
**FIG. 12** shows that the negative FSPs have more pathogen epitopes than the highly active FSPs. This figure compares the negative FSPs with active FSPs in cancers. All negative FSPs and the FSPs with >10% positive rate in cancers from **FIG. 9** were selected for analysis. A BLAST search against the Immune Epitope Database (IEDB) shows differential similarity with pathogen of peptides from these two sets of FSPs. Each peptide was searched using BLAST against epitopes in the IEDB and all the epitopes having an e value less than 10,000 were recorded as the matches. The number of the matched epitopes of each peptide was put into boxplot and represented the pathogen similarity of that peptide. The difference in similarity was statistically calculated with T-test P<0.05.

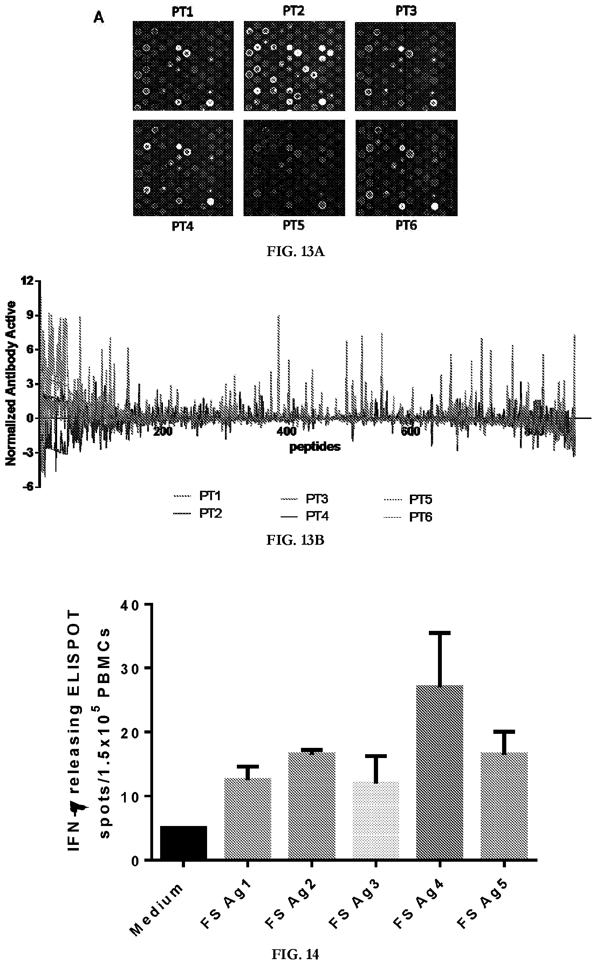

**FIG. 13A** and **FIG. 13B** show identification of personal reactive FS antigens and detection of the T cell responses to some of these FS antigens in the same dog. Screening of 6 dogs with cancer for specific cancer vaccine candidates. **FIG. 13A** shows a corner of the scanned FS array of each cancer dog analysis. **FIG. 13B** shows normalized antibody active to each peptide of each cancer dog. Both **FIG. 13A** and **FIG. 13B** showed each dog has personal highly reactive FS peptides.
**FIG. 14** shows identification of personal reactive FS antigens and detection of the T cell responses to some of these FS antigens in the same dog. Testing T-cell reactivity of candidate peptides for vaccine. For a specific dog screened using the arrays in FIG. 9, 5 FS antigens that were highly antibody reactive were tested in ELI-Spot assays. PBMCs from the dog blood were purified, stimulated with pooled peptides from each FS antigen candidates and the number of positive cells counted. The number for positive for each FS antigen stimulant is shown. As can be seen all 5 FS antigens showed significant T-cell reactivity compare to the medium control

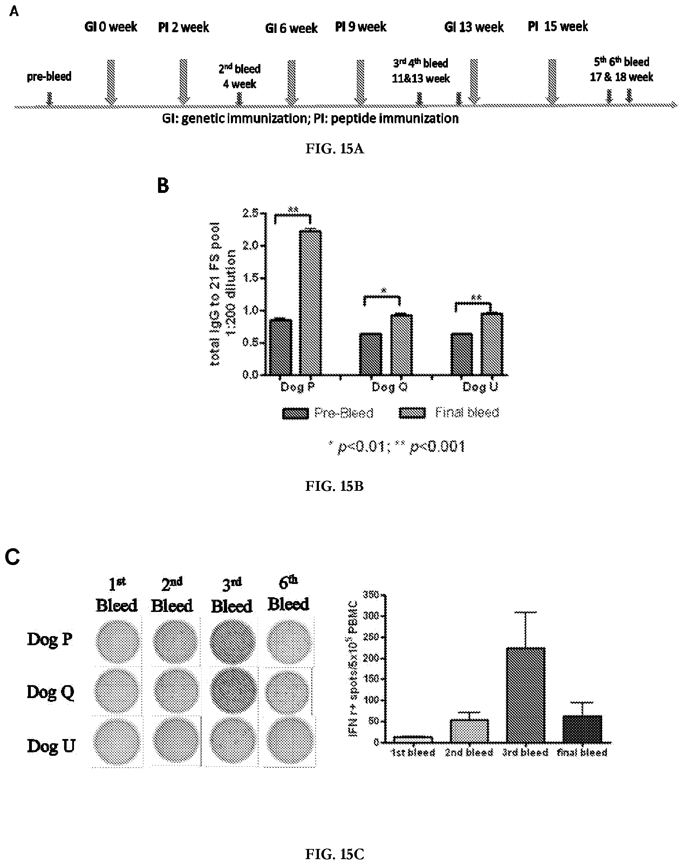

**FIG. 15A, FIG. 15B,** and **FIG. 15C** show the result of the dog cancer vaccine safety trial. **FIG. 15A** shows an exemplary trial schedule. **FIG. 15B** shows an ELISA analysis of the IgG reactive to the vaccine. **FIG. 15C** shows IFN-r ELISPOT analysis of the T cell response to the vaccine.

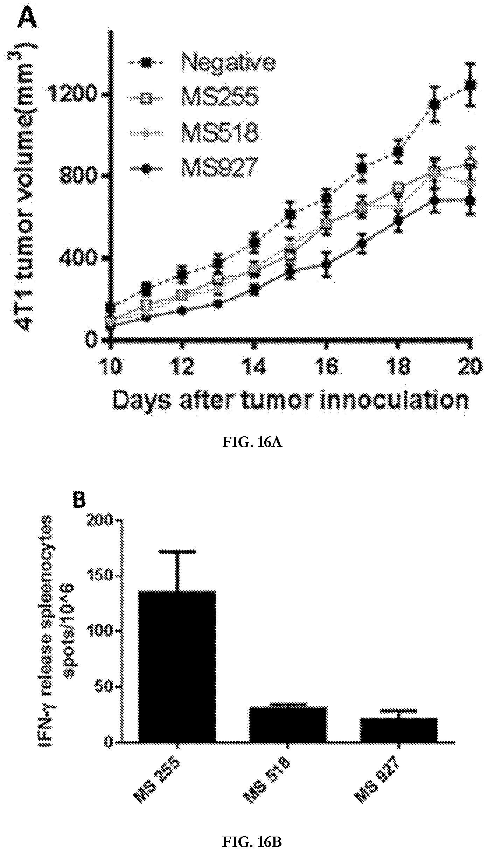

**FIG. 16** **A** and **FIG. 16B** show that FSPs have significant protection as vaccine candidates in different mouse tumor model for the three selected MS FSPs. **FIG. 16A** shows tumor growth curve by three MS FS antigens immunization with 4T1-BALB/c tumor model. Mice (n = 10 per group) were genetically immunized at 8 weeks of age, boosted twice genetically 3 weeks later with two days apart and another peptide boost 2 weeks later. Mice were challenged 2 week later by subcutaneous injection of 5×10³ 4T1 cells. All vaccine groups significantly delayed tumor growth. **FIG. 16B** shows ELISPOT analysis of the three MS FS antigen immunization. 3 mice were genetically immunized with pool of the three MS FA antigens and genetically boosted 6 weeks later. Immunized mice were challenged by 5×10³ 4T1 cells 3 weeks later. Splenocytes were collected at 19 days after tumor challenge and pool three splenocytes for the assay. Error bars represent SD.

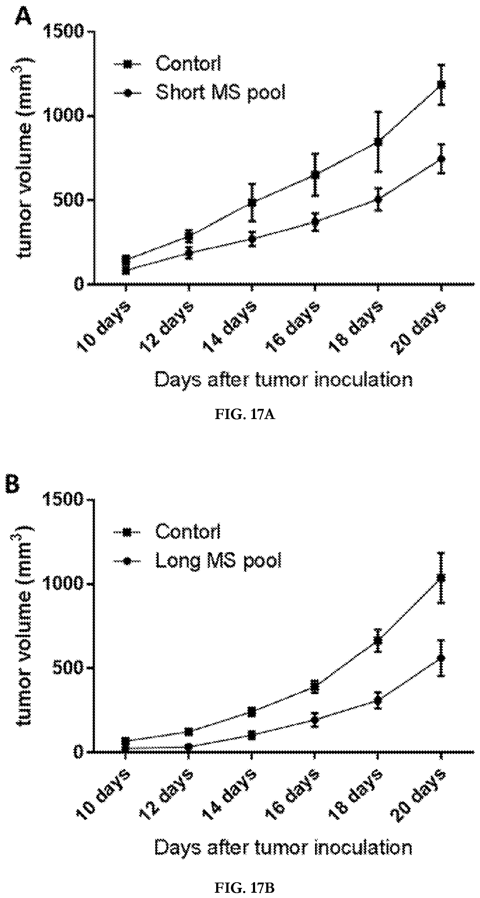

**FIG. 17A** and **FIG. 17B** show that FSPs have significant protection as vaccine candidates in different mouse tumor model. Tumor growth of curve the vaccine test of the two FSPs pool from short and long MS in 4T1-BALB/c tumor model. **FIG. 17A** shows tumor growth curve with short MS FSPs vaccine group. **FIG. 17B** shows tumor growth curve with long MS FSPs vaccine group.

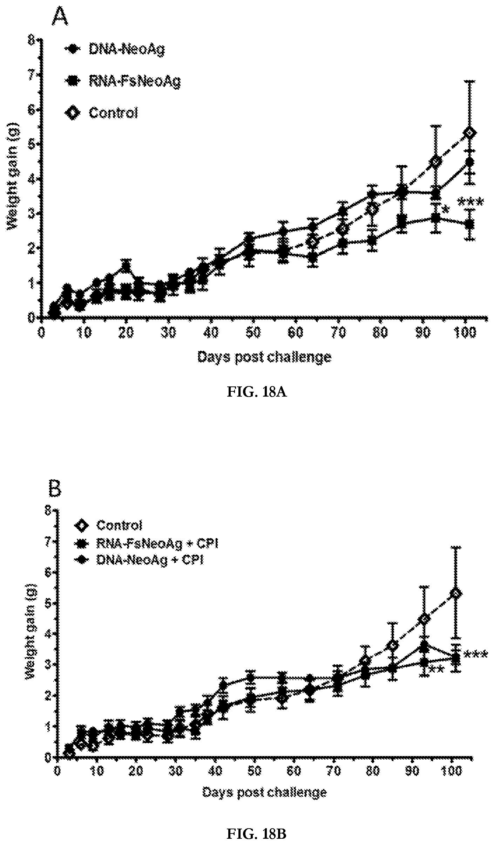

**FIG. 18** shows that FSPs have significant protection as vaccine candidates in different mouse tumor model. Therapeutic immunization with FS antigens against ovarian cancer in mouse model. **FIG. 18A** shows female C57BL/6 mice (n=10) were challenged intraperitoneally with ID8 (5 x 10⁵ cells/mice) at day 0 and then, immunized subcutaneously 3 times with pool of FSPs (RNA-FsNeoAg)(10 FS from MS INDEL and 3 FS from mis-splicing) or neo antigens from DNA mutations (DNA-NeoAg) at days 3, 6 and 78. Control group mice were injected PBS buffer. **FIG. 18B** shows the vaccine group received additionally a checkpoint inhibitor treatment (CPI) composed by antibody anti-PD-L1 and CTLA-4, six times divided in two sets. Mice were weighing once a week until reach endpoint.

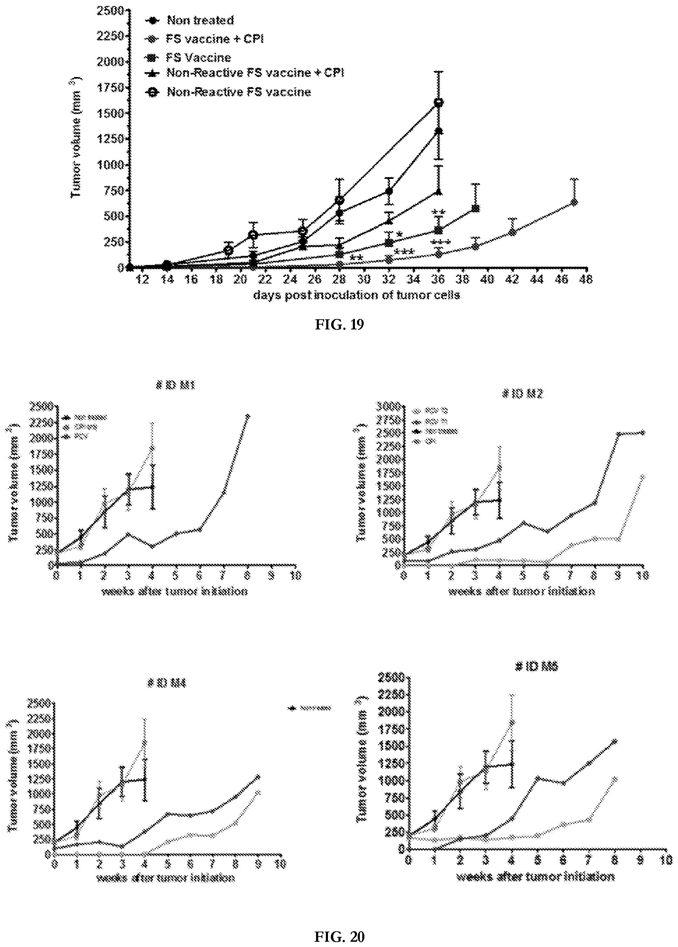

**FIG. 19** shows the SPCV system successfully identified protective personalized cancer vaccines in two mouse tumor models. Protection by personalized pool FS neoantigens in 4T1 BALB/c tumor model. Serum from BALB/c mice (n=10/group) were collected two days previous and 7-days post subcutaneous injection of 4T1 tumor cells (500 cells/mouse). Both sera were run on microarray slides containing peptides for human FS neoantigens and the 10 mouse homologs neoantigens with higher median normalized signal on the array were selected as targets for the vaccines formulations for each mice. Mice were vaccinated subcutaneously on days 12 and 19. For the immunization group with the combined immunotherapy, the antibody treatment, anti-PD-L1 and CTLA-4, were administrated intraperitoneally on days 12, 15, 19, 22. Control group (non-treated) was immunized with PBS at the same schedule as the vaccine groups. Non-reactive groups were immunized with Fs antigens without signal in the microarray before and after tumor challenge, and followed the same vaccine regimen as the other groups. ^{∗} p<0.05; ^{∗∗}p<0.01; ^{∗∗∗}p<0.001, analyzed by two-way ANOVA with *Bonferroni* multiple comparisons post-test.

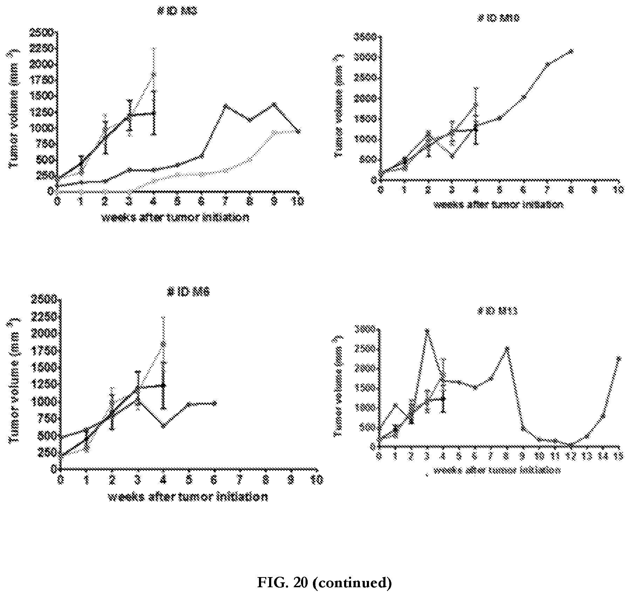

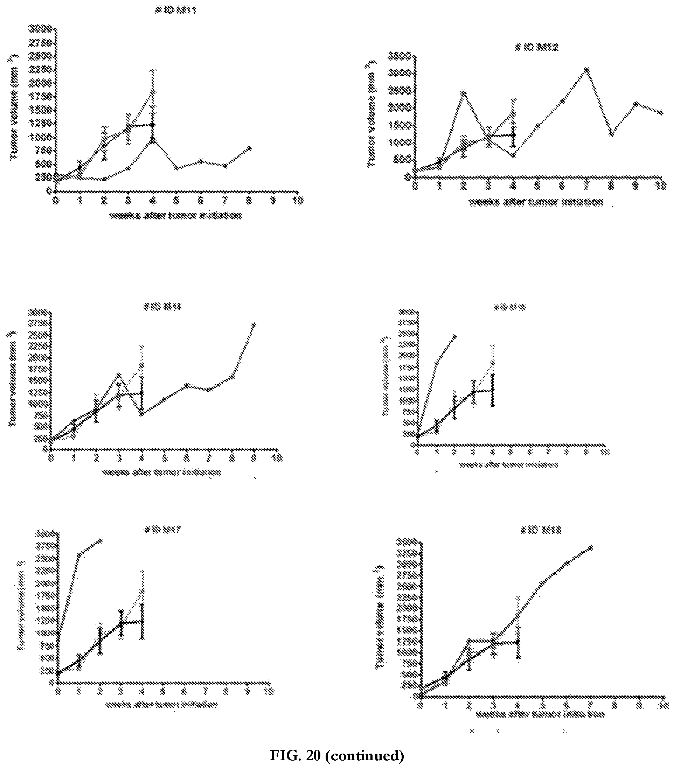

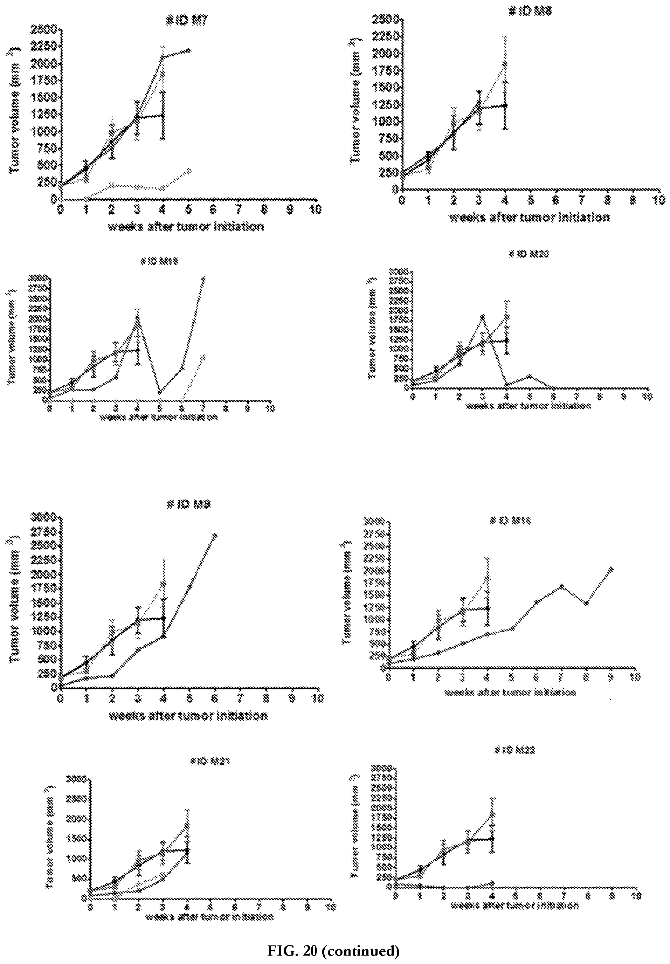

**FIG. 20** shows the SPCV system successfully identified protective personalized cancer vaccines in two mouse tumor models. Female FVB/N mice (n= 22) were evaluated weekly for the presence of palpable tumor, when tumor was detected sample blood was collected and run on microarray slides containing peptides for human FS neoantigens. The 10 mouse homologs neoantigens with higher median normalized signal compared to age matched FVB/NJ samples on the array were selected as targets for the vaccines formulations for each mice. Mice were vaccinated subcutaneously with pool of Fs peptide with minimum two doses and maximum seven doses, as needed to maintain tumor control, with intervals 1-2 weeks between doses. Each vaccine dose was followed by checkpoint inhibitor treatment (antibody anti-PD-L1 and anti-CTLA-4) (CPI), two doses, three days a part. As a control, animals were immunized with PBS (non-treated) at the same schedule as the vaccine groups or only with CPI treatment (CPI only), twice per animal. Personalized Fs vaccine conferred 68.2 % of protection.

### DETAILED DESCRIPTION

Described herein are personalized cancer vaccines that overcome the limitations of conventional methods. The advent of the use of immunotherapeutics has resulted in emphasis on the importance of neo-epitopes in the immune response to cancer. Neo-epitopes are peptides that are normally not produced to an immunologically recognized level in healthy cells, but are so in tumors. They are essentially foreign epitopes. Of the neo-epitopes, frameshifts peptides are the best antigens as shown in FIG. 1. FS neo-antigens were the basis for the first tumor agnostic approval of a checkpoint inhibitor. It has been established based on sequencing the DNA of ~100,000 tumors that millions of neo-antigens are produced by tumors, with very few of them repeated in different tumors, even among tumors the same type and FS mutation at the DNA level are very rare. Therefore, it has been conventionally thought that to develop a personalized cancer vaccine, each tumor would need to be sequenced as well as the non-tumor cells (germline) for each person. Further, it is not feasible to create a library of all the possible neo-antigens to create a personal vaccine. Therefore, each set of neoantigens would have to be customized and manufactured for each patient after the determination of the appropriated vaccine components. Since most neo-antigens are the product of point mutations, and only a small portion of these are immunogenic (~3%) in patients, current methods involve application of a complicated informatics approach to predict the best vaccine components. The current basic concept as described in the art is that personal neoantigens in one's tumor are developed by sequencing the patient's tumor. The germline sequence of the patient would also be analyzed for comparison purposes. The developed neoantigens would then be informatically screened to predict which are likely to be immunogenic, and a vaccine manufactured for these particular antigens and delivered in a timely fashion to the patient. In some instances, it is envisioned that this personal vaccine would be delivered with a checkpoint inhibitor. However, personal cancer vaccine development and the checkpoint inhibitor are both expensive. We have developed a system to create a personal vaccine that is much faster and less expensive than existing methods, yielding more immunogenic antigens for a personal cancer vaccine.

We have discovered that a smaller subset of neo-antigens ("frameshift neo-antigens") can be utilized to develop a personalized cancer vaccine. This is an important advance because the number of frameshift (FS) neo-antigens is much smaller than the total of all possible neo-antigens. We have discovered that FS peptides are frequently and recurrently produced in tumor cells. These FS peptides are produced by insertion and deletion variants (INDELS) occurring in microsatellite regions or by mis-splicing of RNA **(****FIG. 2****).** The microsatellite generated FS are predictable and will be made in each tumor that expresses the gene containing them. The mis-splicing events are also predictable and the ones that actually occur are detectable by the FS peptide arrays described. Our discovery that FS antigens occur more frequently and more recurrently in different tumors in the RNA by transcription or mis-splicing is surprising **(****FIG. 3****).** These INDEL variants only exist in the RNA, not in the DNA **(****FIG. 4****).** There are approximately 14,000 potential FS peptides transcribed from microsatellites in exons and approximately 200,000 potential FS from mis-splicing. We have also discovered that tumors make INDEL variants **(****FIG. 5****)** in most microsatellites and that mis-splicing is also recurrent at the same genes in tumors. We can also further limit the number of FS vaccine candidates by restricting them to ones that are at least 8 amino acids long and/or in oncogenes, essential genes and or highly expressed genes. These genes would be more difficult for a tumor to evolve away from. Therefore, it is only necessary to screen a limited number of approximately 1000 to 200,000 FS peptides to determine the components of a personal vaccine. This screen can be done in a day or less, rather than a month or more by current practice, and at much lower cost.

Disclosed herein are several methods and systems for such non-sequencing based screens. All approaches to date described in the art begin with sequencing the DNA and RNA of the patient. An important aspect of our approach is that our immunological screens indicate that the FS peptides are produced by the tumor and are reactive with the immune system, which is not the case with current protocols which only sequence the RNA to determine if the gene is transcribed. Further, the number of candidate FS antigens could be restricted to a low enough number to allow the vaccine components, *i.e.* frameshift neo-antigens, to be pre-synthesized and the personal vaccine readily formulated for each patient. This potentially simplifies regulatory approval of the personal vaccine approach by making uniform certain constituents of the personal vaccine across patient populations.

A comparison of the current approach to making personal cancer vaccines (PCV) (as described in patents, for example US 2016101170) to our method of making simple personal cancer vaccines (SPCV) disclosed herein is illustrated in **FIG. 6****.** The two systems vary fundamentally at almost every step:
Step 1. The PCV requires taking a biopsy of the patient's tumor. In contrast, the SPCV only requires a small amount of blood. Because blood can readily be obtained, unlike tumor biopsies, the patient can be continuously monitored relative to the vaccine response and tumor status.
Step 2. In the PCV the tumor DNA and RNA are sequenced, as well as the patient's germline, to find to determine candidate tumor mutations. In the SPCV, blood samples are assayed to directly determine which tumor variants are both expressed and have an immune response in the patient.
Step 3. The PCV system requires applying an algorithm to estimate which of the mutations might be expressed as peptides and the immune system would respond to. This is not required by SPCV, since any expressed FS of 8aa or longer would be highly likely to elicit an immune response. Moreover, since the screen is for immune reactivity, the results directly indicate that the neo-antigen is expressed and immune reactive.
Step 4. In several cases of the peptide arrays in SPCV, the numbers are small enough that the peptides could be pre-made. This would allow, for example, screening candidate peptides directly in T-cell assays to determine specific immunogenicity in patients. This is not practical for the PCV approach as it would take too long to get the information.
Step 5. In the PCV system it is expected it will take at least 2 weeks or more to manufacture the vaccine for the patient. Currently it takes months. Because the domain of possible mutations in PCV assays is so large, the manufacturing will need to be specific for each person. In contrast, in several cases of the SPCV arrays, the number of variants are small enough such that the collection of possible vaccines can be pre-made, greatly reducing the time to get back to the patient. This would also allow the vaccine components to be pre-validated by the FDA, which currently cannot be done for the PCV system and thus poses a potential regulatory problem. These comparisons are summarized in Tables 1 and 2. In the PCV system even if the manufacture of the vaccine as peptide or nucleic acid can be improved to take less time, the SPCV system is inherently still better as the FS antigens will be much more broadly immunogenic.
Step 6. The current PCV cannot find enough neo-antigens from low mutation burden cancer patients, which is about 30% of total cancers and almost 100% of certain types of cancer. The SPCV potentially works all types of cancers **(****FIG. 7****).**

**Table 1: Comparison of PCV versus SPCV**

| | **Traditional PCV** | **SPCV** |
|---|---|---|
| **Ag Identification** | ≥ 2 weeks | 1 ~5 days |
| **Ag Immunogenicity** | Algorithm Predicted (3%) | confirmed |
| **Vaccine Production** | ≥ 2 weeks | 1 days |
| **Cost** | $$$ | $ |

**Table 2: Peptide Numbers**

| **FS mutation category** | **peptide number** | **methods for screening** |
|---|---|---|
| All Possible FS mutations | ~2^{∗}10⁶ | Phage or RNA display |
| All possible splice variants FS | ~6^{∗} 10⁵ | In situ synthesized arrays |
| All MS FS | ~2^{∗}10⁴ | Spotted arrays |
| All MS FS in Driver genes | ~2^{∗}10³ | Spotted arrays or micro titer plates |
| All MS FS in Essential genes | ~1.6^{∗}10³ | Spotted arrays or micro titer plates |

Disclosed herein are streamlined, cost effective, and efficacious methods to design and produce personal vaccines, such as personal cancer vaccines. Methods herein include methods of determining the optimal components of a vaccine to be given to an individual to treat the cancer in that individual. Such methods include determining whether a candidate vaccine peptide is both expressed in the tumor of the individual and elicits an immune response in the individual. In some cases, the methods herein disclosed comprise obtaining a blood sample from an individual diagnosed with a cancer or a pre-cancer, diluting the blood sample in a buffer, contacting the diluted blood sample to a collection of peptides, wherein the collection of peptides comprises predicted frame-shift peptides that may be produced by a cancer cell from the individual. In some cases, the collection of peptides is an array, a high density array, a phage library, a plate-based assay, or other means of assembling a pre-determined collection of frame-shift peptides for testing against patient samples, including blood, serum, plasma, saliva, cerebrospinal fluid, and others. The results of the assay determine which peptides have strong (e.g. K_{D}>10⁻⁸) binding to antibodies in the patient's blood compared to an average binding in blood from people without cancer or compared to a sample taken from the individual before they had cancer. In additional cases, variant peptides could be used in assays for T-cell reactivity using cells from the same patient. Peptides eliciting a strong response in T-cell activity assays are more likely to be good vaccine components, an assay which can be personalized with an individual's T-cell samples. Those peptides with high reactivity can form the basis of a personal vaccine to treat the individual's cancer. Variants or FS antigens herein are the products of alterations at the RNA level, such as errors in transcription of RNA processing. This is in contrast to germline "mutations" which occur in the DNA and are heritable.

Vaccines designed using methods herein could comprise the personalized set of peptides in a number of forms, including as a DNA vaccine, a peptide vaccine, an RNA vaccine, a viral vaccine, a bacterial vaccine or combinations thereof. Vaccines herein can also be loaded or incorporated into antigen presenting cells, such as dendritic cells or macrophages and the loaded cells administered to the individual. Alternatively, genes encoding the vaccine antigens can be used to transform antigen presenting cells through techniques known by those of skill in the art such as CRISPR, transfection, viral or vector transduction, or other gene transfer or incorporation technology.

Vaccines designed using methods herein could also be given to an individual to prevent reoccurrence of a cancer. Collections of peptides herein developed in individual patients could also be used, for example, to diagnose cancer in other patients.

The variant peptides comprising the collection for screening could be from several sources. They could be peptides known to result from point mutations, frame shifts, deletions/insertions or translocation in tumor DNA. Because these types of mutations are personal and occur infrequently, it would take a large number of peptides to represent all of them. Conventional practice is to determine neo-antigens encoded at the DNA level and then confirm expression at the RNA level. We have unexpectedly discovered that errors occur much more frequently at the RNA processing level. Since microsatellites in coding regions are predicted and limited in number, one can predict a small set of FS peptides resulting from insertion or deletions during transcription that will produce FS neoantigens. Therefore, methods herein, in some cases, comprise screening frameshift variants formed from 1) insertions or deletions in microsatellites in coding regions or 2) from mis-splicing events either in or between genes that create an out-of-frame fusion. These variants have several attractive features as sources for a personal vaccine component. First, frameshift variants generally have variant peptide sequences of over more than 8 amino acids. In contrast with point mutations that often only alters one amino acid, a FS variant is completely foreign sequence and therefore is much more likely to be immunogenic. Our work indicates that there are only a few thousand frameshifts from microsatellite insertion/deletions that are more than 8 amino acids long. Frameshifts of 8-60 amino acids long are very likely to include MHCI and MHCII epitopes. Further, because of their increased immunogenicity, FS variants are much more likely to create both T- and B-cell responses. Therefore, fewer peptides are required to be screened to determine vaccine components. Point mutation neo-epitopes are unlikely to produce both B and T-cell responses. Even though the arrays we developed **(****FIG. 8** **A** and **FIG. 9****)** record B-cell responses to the FS peptides, the antibody response level on the arrays correlates with the tumor protection **(****FIG. 10** and **FIG. 11****).** This may be because the antibody response requires CD4 help and it has been discovered that CD4 responses are important for protection. Further, the realm of FS space is much more restricted than that of all possible point mutations. This is particularly true for INDELS in microsatellites in coding regions. There are 2 possible FS that can be predicted from each of the ~7000 microsatellites in coding regions. These numbers become smaller as putative peptides are filtered for restrictions for minimal length (e.g. >7aa) and the probability of eliciting immune responses. This makes it feasible to have a pre-existing set of FS peptides made that can be used to screen the T-cells of a patient for reactivity.

Peptides to be screened by the methods herein are produced and displayed in a number of ways. For example, in some cases the peptide candidates are synthesized and spotted on arrays. In some cases, arrays have about 100 selected FS peptides. In some cases, arrays have about 200 selected FS peptides. In some cases, arrays have about 300 selected FS peptides. In some cases, arrays have about 400 selected FS peptides. In some cases, arrays have about 500 selected FS peptides. In some cases, arrays have about 600 selected FS peptides. In some cases, arrays have about 700 selected FS peptides. In some cases, arrays have about 800 selected FS peptides. In some cases, arrays have about 900 selected FS peptides. In some cases, arrays have about 1000 selected FS peptides. In some cases, arrays have about 10,000 selected FS peptides. In some cases, arrays have about 20,000 selected FS peptides. In some cases, in-situ synthesis could produce an array having 1,000,000 or more peptides per array, or at least 1000, 10,000, 100,000 or 400,000 **(****FIG. 8****,** **FIG. 9****)** peptides per array.

In an exemplary method of developing a personalized cancer vaccine, the blood from a cancer patient is diluted and applied to the array. After incubation the array is washed and the bound patient's antibodies are detected with a labeled secondary antibody, for example a fluorescently labeled secondary antibody. The secondary antibody can be to any combination of one or more isotypes, including but not limited to IgG1, IgG2, IgG3, IgG4, IgA1, IgA2, IgD, IgM, and IgE. The level of fluorescence indicates the amount and/or affinity of the antibody to the FS peptide. We have shown that the relative binding correlates to protection **(****FIG. 11****).** The assay may be optimized to selectively detect high affinity binding of antibodies to their cognate FS antigen. The reactivity can be compared to the reactivity seen in healthy people without cancer or to a previous baseline blood sample of that same patient. The readout is the antibody response to particular FS peptides. A positive response indicates that the peptide must be made in that tumor and that the immune system has reacted to it. Sequencing the DNA or RNA from the tumor does not convey either of these important pieces of information. Sequence analysis of the negative IgG reactive FSPs showed that these FSPs contain more pathogen epitopes **(****FIG. 12****).** It is contemplated that the frequently contacting different pathogen in regular life induces the tolerance to these epitopes and consequently, decreases the effective immune responses to the FPSs containing these epitopes. The approach disclosed herein gives a direct readout of the antibody response to the FS peptides, which is important information in designing a vaccine. Antibody binding to a FS peptide could also be detected by any number of label-free methods.

Accordingly, in some cases, selecting candidate FSPs for a personalized cancer vaccine herein, comprises determining the sequence similarity to known pathogen epitopes and selecting FSPs that do not show sequence similarity to known pathogen epitopes. Known pathogen epitopes comprise peptides catalogued in the Immune Epitope Database (IEDB, available on the internet at iedb.org). In some cases, candidate FSPs are searched via a BLAST algorithm against pathogen epitopes in the IEDB. In some cases, a candidate FSP is selected for having a BLAST e value less than 10,000.

A T-cell response, in some cases, is important for killing cancer cells. Since the FS peptides are generally 8 aa or longer, it is very likely that a FS peptide will have a region that would bind to the patient's MHC to initiate an immune response. MHC binding can be predicted from commonly available algorithms. Alternatively, the blood sample from the patient could be screened for T-cell activity to the peptide candidates using a T cell assay, such as a proliferation assay, a cytokine assay, a cytotoxicity assay, a degranulation assay, flow cytometry, or combination thereof.

Methods of designing personalized cancer vaccines disclosed herein would not only provide much more relevant information for making a vaccine, but it would have benefits over the existing methods such as simplicity and cost efficacy. It would not require a biopsy of the tumor with the inherent cost, discomfort and danger. The assay itself would be simpler, less expensive and faster than deep sequencing the tumor DNA. The mutations identified in the DNA are most likely to be the result of point mutations which have a low probability of being produced by a patient, or at least immunogenic. Current sequencing protocols are very poor at identifying insertions/deletions in microsatellites or mis-splicing events. The best source of peptides (FS) would be missed by current sequencing protocols. Sequencing a biopsy will only identify mutations in that part of the tumor and mutations in other parts will be missed. By methods disclosed herein, all immune reactive parts of the tumor will be identified. The identification of vaccine components by sequencing will require the application of proprietary algorithms and other assays to identify potentially produced and immunogenic peptides. In contrast, this information is directly readout from our assay. While the broadest application may be for cancer, the same procedure would apply for a vaccine against other chronic diseases. Vaccines based on the MS FS herein could also could eliminate cells that are not tumors but are aberrant for other reasons. Such aberrant, dysfunctional cells play a role in diseases such as diabetes, Alzheimer's disease, aging, autoimmune disease, chronic infections, and other diseases.

### Microsatellite and Splicing FS Variants

Methods herein, in some cases, comprise methods of frameshift variant development for inclusion in personal cancer vaccine development. Frameshift variants, as referred to herein, are alterations in an mRNA caused by errors in transcription, causing an insertion or deletion (INDEL) of one or two nucleotides in the mRNA or by mis-splicing of RNA resulting in a change in the amino acids of the resulting protein that are encoded after the frameshift variant. Methods of frameshift variant development herein include but are not limited to mRNA sequencing and array based hybridization. In some cases, frameshift peptides are developed by bioinformatics analysis of already available sequence data. FS variants peptides due to INDELs in MS can be directly inferred from the genome sequence data. Any INDEL in a coding sequence will produce FS if expressed. On the other hand a specific exon mis-splicing may or may not be produced. Therefore, this application focuses on the MS FS, all of which will be produced.

In some cases, mRNA sequencing for development of frameshift variants herein includes a method where mRNA from a tumor or cancer tissue is sequenced. In some cases, mRNA is purified from a tumor or cancer tissue from a patient. In some cases, mRNA is isolated from total mRNA from the tumor or cancer tissue. In some cases, mRNA is isolated using oligo-dT purification of total RNA. In some cases, mRNA is targeted for sequencing using an oligo-dT to prime the RNA sample. In some cases, the mRNA is amplified before sequencing. In some cases, the mRNA is amplified by PCR before sequencing. In an case the mRNA is sequenced by random priming of the cDNA to detect FS sequences. In some cases the mRNA is amplified by RT-PCR before sequencing. In some cases, mRNA sequencing comprises targeted sequencing of an mRNA having a microsatellite in the transcript. In some cases, mRNA is sequenced using at least one technique selected from Sanger sequencing, pyro-sequencing, ion semiconductor sequencing, polony sequencing, sequencing by ligation, nanoball sequencing, and single molecule sequencing.

Variants identified from mRNA sequencing are classified by type of variant. Variants may arise from mutations in DNA or alterations in the RNA during transcription or splicing herein, which include but are not limited to point mutations, silent mutations, insertions, deletions, cis-splicing errors, and trans-splicing errors. Of these, only insertions, deletions, cis-splicing errors, and trans-splicing errors are expected to lead to a frameshift in a protein produced from the mutant mRNA. Confirmed frameshift variants are those that when translated produce a protein with a different amino acid sequence at more than one residue at residues C-terminal to the alteration. Frameshifted polypeptide sequences resulting from frameshift variants are assembled for further analysis.

In some cases frameshift mutations are predicted based on microsatellite location in the genome. As transcripts having a microsatellite are more prone to transcription errors, frameshift polypeptides can be predicted to be resulting from an insertion or a deletion of one or two basepairs. Alternatively, frameshift polypeptides can be predicted by bioinformatics prediction of cis and/or trans splicing errors. A selection of all possible frameshift peptides can be assembled for further analysis.

### Peptides from Frameshifts from MS Indels and Mis-Splicing

Frameshifted polypeptide sequences, determined by mRNA sequencing or prediction, are further analyzed to determine immunoreactivity. In some cases, immunoreactivity is measured by MHC or HLA binding. In some cases, immunoreactivity is measured by antibody binding. In some cases, immunoreactivity is measured by T cell activity. In some cases, immunoreactivity is measured by antibody binding and T cell activity.

Binding to MHC is required for T cell activity and can be determined by binding assays. Alternatively, in silico methods of MHC binding are used to predict binding of a peptide to a MHC subtype. Data of peptides binding to MHC subtype molecules are used to develop binding prediction algorithms. These algorithms calculate scoring matrices that quantify the contribution of each residue in a fixed-length peptide to binding to an MHC molecule. Algorithms predict binding of a peptide to class I MHC or class II MHC. Algorithms to predict class I MHC binding include but are not limited to Artificial neural network (ANN), Stabilized matrix method (SMM), SMM with a Peptide:MHC Binding Energy Covariance matrix (SMMPMBEC), Scoring Matrices derived from Combinatorial Peptide Libraries (Comblib_Sidney2008), Consensus, NetMHCpan, NetMHCcons and PickPocket. Algorithms to predict class II MHC binding, include but are not limited to Consensus method, Combinatorial library, NN-align (netMHCII-2.2), SMM-align (netMHCII-1.1), Sturniolo, and NetMHCIIpan. The entire population of frameshift polypeptides is then scanned using one or more of the above algorithms for peptides binding to an MHC subtype molecule with a predicted affinity of IC50 <500 nM.

### Identifying Frameshift Peptide Antibody Response

Candidate frameshift peptides for personalized cancer vaccines, in some cases, are screened for antibody reactivity in an individual needing treatment using a personalized cancer vaccine. Antibody reactivity is determined using an assay for antibody binding to a peptide. In some cases, peptides for antibody screening are bound to a substrate, such as a plate, a glass slide, a bead, or other substrate. Assays for antibody binding include but are not limited to ELISA, radio-immuno assay, western blot, surface plasmon resonance, immunostaining, immunoprecipitation, mass spectrometry, phage display, flow cytometry, cytometric bead array, immunohistochemistry, high density array, microarray, and combinations thereof.

In an exemplary case, a blood sample is obtained from an individual needing treatment for breast cancer. The blood sample is diluted in an appropriate buffer and applied to a peptide microarray spotted with vaccine candidate frameshift peptides. The diluted blood sample is incubated with the peptide microarray overnight and the peptide microarray is then washed and exposed to a secondary antibody that binds to human IgG bound to the microarray. The secondary antibody is conjugated with Alexa Fluor 488 and the microarray is analyzed for fluorescence at each peptide spot. The peptides bound to the individual's antibodies are deemed immunoreactive and selected for the individual's personalized cancer vaccine.

In another exemplary case, an individual is given an intradermal injection of each candidate peptide in a pre-determined pattern on the patient's back to measure delayed-type hypersensitivity (DTH) response. Erythema and induration are measured at 24, 48, and 72 hours. The peptides which elicit a DTH response are deemed immunoreactive and selected for the individual's personalized cancer vaccine.

### Identifying Frameshift Peptide T Cell Response

In some cases, candidate frameshift peptides are screened for T cell activity in cells obtained from an individual needing treatment using a personalized cancer vaccine. T cell activity is determined using a T cell assay measuring proliferation, cytokine secretion, cytotoxicity, or degranulation in response to a frameshift peptide bound to an antigen presenting cell. T cell assays include but are not limited to proliferation assay, 3H-thymidine assay, BrdU assay, CFSE assay, cytokine secretion assay, ELISA assay, ELISPOT assay, intracellular staining assay, quantitative rtPCR assay, cytometric bead array assay, MHC-tetramer binding assay, cytotoxicity assay, 51-chromium assay, degranulation assay, granulysin assay, granzyme A assay, granzyme B assay, and perforin assay.

In an exemplary case, a blood sample is obtained from an individual needing treatment for breast cancer. PBMCs are isolated from the blood sample and the PBMCs are cultured to expand T cells in the sample and the T cells are incubated in culture media containing one or more candidate peptides for a cytokine release assay. The production of IFN-γ is analyzed in ELISPOT assays. Flatbottom 96-well nitrocellulose plates are prepared and coated with either anti-human IFN-γ. Cells were then incubated at a density of 1 × 10⁵/well either with peptide pools or individual peptides (10 µg/ml), PHA (10 µg/ml), or medium (containing 1% DMSO corresponding to the percentage of DMSO in the pools/peptides) as a control. After 24 hours, cells are removed, and plates are incubated with HRP-conjugated anti-human IFN-γ Ab (Clone 7-B6-1, Mabtech) at 37° C. After 2 hours, spots corresponding to the HRP-conjugated Ab (IFN-γ) are developed with 3-amino-9-ethylcarvazole solution (Sigma-Aldrich, St. Louis, Mo.). Spots are counted by computer-assisted image analysis (Zeiss, KS-ELISPOT reader, Munich, Germany). Each assay is performed in triplicate. The level of statistical significance is determined with a Student's t-test using the mean of triplicate values of the response against relevant pools or individual peptides versus the response against the DMSO control. Criteria for peptide pool positivity are 100 spot-forming cells (SFCs)/10⁶ PBMC, p≦0.05 and a stimulation index (SI) ≧2, while criteria for individual peptide positivity are ≧20 SFC/10⁶ PBMC, p≦0.05, and a SI ≧2.

### Platforms for Screening Peptides for Personalized Cancer Vaccines

In some cases, disclosed herein are array platforms that allow for development of peptides suitable for personalized cancer vaccines. The array platforms comprise a plurality of individual features on the surface of the array. Each feature typically comprises a plurality of individual peptides synthesized *in situ* on the surface of the array or spotted on the surface, wherein the molecules are identical within a feature, but the sequence or identity of the molecules differ between features. Such array molecules include the synthesis of large synthetic peptide arrays.

The peptide arrays can include control sequences that match epitopes of well characterized monoclonal antibodies (mAbs). Binding patterns to control sequences and to library peptides can be measured to qualify the arrays and the assay process. Additionally, inter wafer signal precision can be determined by testing sample replicates e.g. plasma samples, on arrays from different wafers and calculating the coefficients of variation (CV) for all library peptides. Precision of the measurements of binding signals can be determined as an aggregate of the inter-array, inter-slide, inter-wafer and inter-day variations made on arrays synthesized on wafers of the same batch (within wafer batches). Additionally, precision of measurements can be determined for arrays on wafers of different batches (between wafer batches). In some cases, measurements of binding signals can be made within and/or between wafer batches with a precision varying less than 5%, less than 10%, less than 15%, less than 20%, less than 25%, or less than 30%.

The technologies disclosed herein include a photolithographic array synthesis platform that merges semiconductor manufacturing processes and combinatorial chemical synthesis to produce array-based libraries on silicon wafers. By utilizing the tremendous advancements in photolithographic feature patterning, the array synthesis platform is highly-scalable and capable of producing combinatorial peptide libraries with 40 million features on an 8-inch wafer. Photolithographic array synthesis is performed using semiconductor wafer production equipment in a class 10,000 cleanroom to achieve high reproducibility. When the wafer is diced into standard microscope slide dimensions, each slide contains more than 3 million distinct chemical entities. Maskless photolithography can also be used to create peptides arrays.

In some cases, arrays with peptide libraries produced by photolithographic technologies disclosed herein are used for immune-based assays. Using a patient's antibody repertoire from a biological sample bound to the arrays, a fluorescence binding profile image of the bound array provides sufficient information to classify which peptides are reactive with an antibody from the patient.

Platforms disclosed herein comprise a selection of frameshift peptides disclosed herein, such as peptides resulting from an insertion or deletion error in transcription of an mRNA or peptides resulting from a splicing error such as a trans-splicing error or a cis-splicing error. In some cases, platforms herein comprise frameshift peptides comprise peptides having a sequence selected from all MS FS or MS FS from oncogenes, essential genes, highly expressed genes, such as the peptides provided in SEQ ID NO: 1-7264.

In some cases, the array is a wafer-based, photolithographic, *in situ* peptide array produced using reusable masks and automation to obtain arrays of scalable numbers of combinatorial sequence peptides. In some cases, the peptide array comprises about 100, about 500, about 1000, about 2000, about 3000, about 4000, about 5,000, about 6000, about 7000, about 8000, about 9000, about 10,000, about 15,000, about 20,000, about 30,000, about 40,000, about 50,000, about 100,000, about 200,000, about 300,000, about 400,000, about 500,000, or more peptides having different sequences. Multiple copies of each of the different sequence peptides can be situated on the wafer at addressable locations known as features.

In some cases, the array is a glass slide or nitrocellulose membrane having in vitro synthesized peptides spotted in a predetermined pattern and screened for binding of antibodies in a biological sample from a patient.

In some cases, detection of antibody binding on a peptide array poses some challenges that can be addressed by the technologies disclosed herein. Accordingly, in some cases, the arrays and methods disclosed herein utilize specific coatings and functional group densities on the surface of the array that can tune the desired properties necessary for performing assays. For example, non-specific antibody binding on a peptide array may be minimized by coating the silicon surface with a moderately hydrophilic monolayer polyethylene glycol (PEG), polyvinyl alcohol, carboxymethyl dextran, and combinations thereof. In some cases, the hydrophilic monolayer is homogeneous. Second, synthesized peptides are linked to the silicon surface using a spacer that moves the peptide away from the surface so that the peptide is presented to the antibody in an unhindered orientation.

Platforms herein are also contemplated to include peptides in microtiter plates for determining T cell activity in response to frameshift peptides herein. In some cases, microtiter plates include but are not limited to 96 well, 384 well, 1536 well, 3456 well, and 9600 well plates. In some cases, more than one peptide is present in each well of a microtiter plate, i.e., the peptides are pooled and individual peptides eliciting T cell activity are determined by deconvolution of the positive and negative wells in the T cell assay.

### Screening for Immunogenic Peptides

Optionally, it is useful to determine immunogenicity of a candidate frameshift peptide for use in a personalized cancer vaccine. Immunogenicity, as used herein, refers to the ability of a substance, such as a peptide, to elicit an immune response, such as an antibody response or a T cell response, when administered to an individual, for example, in a vaccine formulation. For individuals, such as humans, with cancer it is the immune response to the tumor. In some cases, a peptide that reacts with an antibody or elicits T cell activity in a biological sample from an individual is not immunogenic when administered in a vaccine formulation. In some cases, a peptide that reacts with an antibody or elicits T cell activity in a biological sample from an individual is immunogenic when administered in a vaccine formulation. Immunogenicity is determined by methods of those of skill in the art including in animal model testing and using in silico prediction of immunogenicity. In silico immunogenicity prediction tools are available for free to the public, for example at the Immune Epitope Database and Analysis Resource (www.iedb.org).

Alternatively, mice, such as humanized mice, and mice transgenic for human HLA genes are used to determine the immunogenicity of a candidate frameshift peptide. The candidate frameshift peptide is administered to the transgenic mouse in a vaccine formulation. Response to the vaccine is determined using antibody assays and/or T cell assays described elsewhere herein. In the case of mice that are injected with a tumor or are transgenic to develop tumors, the protection of the frameshift peptide as a vaccine can be determined.

### Methods of Designing a Personalized Vaccine (methods falling outside the scope of the claims)

Described herein are methods of designing a personalized vaccine for an individual, the method comprising, a) obtaining a biological sample from the individual; b) determining immunoreactivity of the biological sample to a first population of peptides; c) preparing a vaccine composition comprising a second population of peptides that have immunoreactivity with the biological sample from the individual, wherein the second population of peptides is a sub-population of the first population. In some cases, the biological sample is selected from the group consisting of blood, plasma, serum, thymus, bone marrow, spleen, lymph node, bronchoalveolar lavage, breast, central nervous system, cerebrospinal fluid, eye, tears, gastrointestinal tract, saliva, feces, urine, heart, kidney, liver, lung, muscle, pancreas, peripheral nervous system, saliva, skin, thyroid, trachea, and tumor. In some cases, the biological sample is blood, serum, plasma, or saliva. In some cases, the biological sample comprises cells selected from B cells, T cells, CD4+ T cells, CD8+ T cells, Th17 cells, and combinations thereof. In some cases, the biological sample comprises an antibody.

Methods described herein comprise administration of one or more immunoreactive polypeptides reactive to immune cells and antibodies, for example from an individual with cancer. In some cases, immunoreactivity comprises antibody reactivity. In some cases, immunoreactivity comprises a T cell response. A T cell response herein includes but is not limited to one or more of proliferation assay, 3H-thymidine assay, BrdU assay, CFSE assay, cytokine secretion assay, ELISA assay, ELISPOT assay, intracellular staining assay, quantitative rtPCR assay, cytometric bead array assay, cytotoxicity assay, 51-chromium assay, degranulation assay, granulysin assay, granzyme A assay, granzyme B assay, and perforin assay. Methods herein, in some cases, screen a first population for peptides binding to antibodies or eliciting a T cell reaction in a biological sample from an individual, wherein the first population of peptides comprises peptides encoded by a frameshifted mRNA expressed by a cancer cell. In some cases, the first population of peptides comprises peptides having a sequence selected from the peptides provided in SEQ ID NO: 1-7264. In some cases, each of the first population of peptides binds to at least one MHC subtype. In some cases, a portion of the first population of peptides binds to more than one MHC subtype. In some cases, each of the first population of peptides binds to at least on HLA subtype. In some cases, each of the first population of peptides comprises at least one T cell epitope. In some cases, at least one of the first population of peptides comprises at least one T cell epitope. In some cases, each of the first population of peptides comprises at least on B cell epitope. In some cases, at least one of the first population of peptides comprises at least on B cell epitope. In some cases, first population of peptides is bound to a substrate.

### Vaccine Compositions and Formulations

Personalized cancer vaccines described herein comprise one or more peptides determined to have immunoreactivity with a biological sample from an individual in need of treatment for cancer. For example, personalized cancer vaccines, in some cases, comprise 1, 2, 3, 4, 5, 6, 7, 8, 9, 10, 11, 12, 13, 14, 15, 16, 17, 18, 19, 20, or more frameshift peptides determined to have immunoreactivity with a sample from an individual in need of treatment for cancer. In some cases, peptides for personalized cancer vaccines are selected from the peptides provided in SEQ ID NO: 1-7264.

In one case the vaccine can consist of plasmids encoding the MS FS variants. DNA or Gene Vaccines consist of a plasmid with a promoter and appropriate transcription and translation control elements. The plasmids may also sequences that encode peptide or protein fusions to the FS peptide to enhance, for example, expression levels, intracellular targeting or proteasomal processing. For example the LAMP sequence when fused to a FS peptide sequence will enhance MHCII responses. In additional aspects, personalized cancer vaccines herein comprise one or more nucleic acids encoding peptides determined to have immunoreactivity with a biological sample. For example, in some cases, personalized cancer vaccines comprise one or more nucleotides encoding 1, 2, 3, 4, 5, 6, 7, 8, 9, 10, 11, 12, 13, 14, 15, 16, 17, 18, 19, 20, or more FS peptides determined to have immunoreactivity with a sample. In some cases, nucleotides encode peptides for personalized cancer vaccines selected from the peptides provided in SEQ IDNO: 1-7264. These plasmids can be introduced by a needle, a gene gun, an aerosol injector, with patches, via microneedles, by abrasion among many forms. In some forms the DNA vaccine is incorporated into liposomes or other forms of nanobodies. They may also be administered by inhalation or ingestion. The plasmid can be introduced into the blood, the thymus, the pancreas, the skin, the muscle, a tumor or other sites.

Alternatively or in combination, the peptides can be encoded in RNA that is directly introduced into the individual, such as a person. The RNA can be chemically synthesized or more commonly in vitro transcribed. The RNA will encode one or more FS peptides and will include signals to enhance stability and translation. The RNA may also include unnatural nucleotides to increase the half-life. These RNAs can be introduced by a needle, a gene gun, an aerosol injector, with patches, via microneedles, by abrasion among many forms. In some forms the RNA is incorporated into liposomes or other forms of nanobodies. They may also be administered by inhalation or ingestion. The RNA can be introduced into the blood, the thymus, the pancreas, the skin, the muscle, a tumor or other sites

Alternatively or in combination, the peptides coding sequences can be introduced into a virus as a vector. The peptide encoding sequences can be fused to other sequences that enhance transcription, translation or presentation to the immune system. These viral vectors include pox viruses, adenovirus, lentiviruses, retroviruses, alpha viruses and others using a needle, a gene gun, an aerosol injector, with patches, via microneedles, by abrasion among many forms. They may also be administered by inhalation or ingestion.

Alternatively or in combination, the peptides can be administered via a bacterial vector. The FS coding sequences are introduced into the bacteria, usually in the form of plasmid or lysogenic phage, and the bacteria administered to the patient. Listeria is commonly used in this way, but other bacteria could be used or developed. The bacteria can be administered by needle to the blood or intraperitoneal injection. Bacteria can also be administered orally.

The peptides can also be delivered as peptides. Usually the peptides are 10aa long or longer, preferably 25aa or longer. 25-40aa long may be ideal. Usually the peptides are fused to a carrier such as albumin, keyhole limpet protein etc. In some forms the peptides are incorporated into liposomes or other forms of nanobodies.

Personalized vaccine formulations herein comprise a personalized vaccine composition in a biologically acceptable formulation, gaseous, liquid or solid, or mixture thereof, which are suitable for one or more routes of administration, in vivo delivery or contact. Such formulations include solvents (aqueous or non-aqueous), solutions (aqueous or non-aqueous), emulsions (e.g., oil-in-water or waterin-oil), suspensions, syrups, elixirs, dispersion and suspension media, coatings, isotonic and absorption promoting or delaying agents, compatible with pharmaceutical administration or in vivo contact or delivery. Aqueous and non-aqueous solvents, solutions and suspensions may include suspending agents and thickening agents. Such pharmaceutically acceptable carriers include tablets (coated or uncoated), capsules (hard or soft), microbeads, powder, granules and crystals. Supplementary active compounds (e.g., preservatives, antibacterial, antiviral and antifungal agents) can also be incorporated into the compositions.

To increase an immune response and efficacy in treating cancer, peptides herein are optionally coupled to another protein such as ovalbumin or keyhole limpet hemocyanin (KLH), thyroglobulin or a toxin such as tetanus or cholera toxin. Peptides herein are also mixed with adjuvants or checkpoint inhibitors.

Adjuvants include, for example: ABM2, AS01B, AS02, AS02A, Adjumer, Adjuvax, Algammulin, Alum, Aluminum phosphate, Aluminum potassium sulfate, Bordetella pertussis, Calcitriol, Chitosan, Cholera toxin, CpG, Dibutyl phthalate, Dimethyldioctadecylammonium bromide (DDA), Freund's adjuvant, Freund's complete, Freund's incomplete (IFA), GM-CSF, GMDP, Gamma Inulin, Glycerol, HBSS (Hank's Balanced Salt Solution), IL-12, IL-2, Imiquimod, Interferon-Gamma, ISCOM, Lipid Core Peptide (LCP), Lipofectin, Lipopolysaccharide (LPS), Liposomes, MF59, MLP+TDM, Monophosphoryl lipid A, Montanide IMS-1313, Montanide ISA 206, Montanide ISA 720, Montanide ISA-51, Montanide ISA-50, nor-MDP, Oil-in-water emulsion, P1005 (non-ionic copolymer), Pam3Cys (lipoprotein), Pertussis toxin, Poloxamer, QS21, RaLPS, Ribi, Saponin, Seppic ISA 720, Soybean Oil, Squalene, Syntex Adjuvant Formulation (SAF), Synthetic polynucleotides (poly IC/poly AU), TiterMax Tomatine, Vaxfectin, XtendIII, and Zymosan.

Checkpoint inhibitors are inhibitors of proteins known to potentiate or inhibit an immune response and accordingly a checkpoint inhibitor increases the immune response. In some instances, tumors are known to upregulate checkpoint inhibitors to evade the immune response in a patient and checkpoint inhibitors allow the immune system of the patient to recognize and eliminate the tumor. Checkpoint inhibitors herein include but are not limited to a PD-1 inhibitor, a PD-L1 inhibitor, a CTLA-4 inhibitor, Pembrolizumab, Nivolumab, or Atezolizumab. They may be administered systemically or locally as an adjuvant.

Cosolvents may be added to a frameshift peptide composition or formulation. Non-limiting examples of cosolvents contain hydroxyl groups or other polar groups, for example, alcohols, such as isopropyl alcohol; glycols, such as propylene glycol, polyethyleneglycol, polypropylene glycol, glycol ether; glycerol; polyoxyethylene alcohols and polyoxyethylene fatty acid esters. Non-limiting examples of cosolvents contain hydroxyl groups or other polar groups, for example, alcohols, such as isopropyl alcohol; glycols, such as propylene glycol, polyethyleneglycol, polypropylene glycol, glycol ether; glycerol; polyoxyethylene alcohols and polyoxyethylene fatty acid esters.

Supplementary compounds (e.g., preservatives, antioxidants, antimicrobial agents including biocides and biostats such as antibacterial, antiviral and antifungal agents) can also be incorporated into the compositions. Pharmaceutical compositions may therefore include preservatives, antioxidants and antimicrobial agents.

Preservatives can be used to inhibit microbial growth or increase stability of ingredients thereby prolonging the shelf life of the pharmaceutical formulation. Suitable preservatives are known in the art and include, for example, EDTA, EGTA, benzalkonium chloride or benzoic acid or benzoates, such as sodium benzoate. Antioxidants include, for example, ascorbic acid, vitamin A, vitamin E, tocopherols, and similar vitamins or provitamins.

Pharmaceutical compositions can be formulated to be compatible with a particular route of administration. Thus, pharmaceutical compositions include carriers, diluents, or excipients suitable for administration by various routes. Exemplary routes of administration for contact or in vivo delivery which a composition can optionally be formulated include inhalation, respiration, intranasal, intubation, intrapulmonary instillation, oral, buccal, intrapulmonary, intradermal, topical, dermal, parenteral, sublingual, subcutaneous, intravascular, intrathecal, intraarticular, intracavity, transdermal, iontophoretic, intraocular, opthalmic, optical, intravenous (i.v.), intramuscular, intraglandular, intraorgan, or intralymphatic.

Formulations suitable for parenteral administration include aqueous and non-aqueous solutions, suspensions or emulsions of the active compound, which preparations are typically sterile and can be isotonic with the blood of the intended recipient. Non-limiting illustrative examples include water, saline, dextrose, fructose, ethanol, animal, vegetable or synthetic oils.

### Methods of Eliciting an Immune response (methods falling outside the scope of the claims)

Also described herein, are methods of eliciting an immune response in an individual having cancer, the methods comprising, a) obtaining a biological sample from the individual; b) determining immunoreactivity of the biological sample to a first population of peptides; c) preparing a vaccine composition comprising a second population of peptides that have immunoreactivity with the biological sample from the individual, wherein the second population of peptides is a sub-population of the first population; and d) administering the vaccine composition to the individual, wherein administering the vaccine composition elicits an immune response in the individual against the cancer.

Methods described herein comprise administration of one or more immunoreactive polypeptides reactive to immune cells and antibodies, for example from an individual with cancer. In some cases, immunoreactivity comprises antibody reactivity. In some cases, immunoreactivity comprises a T cell response. A T cell response herein includes but is not limited to one or more of proliferation assay, 3H-thymidine assay, BrdU assay, CFSE assay, cytokine secretion assay, ELISPOT assay, intracellular staining assay, quantitative rtPCR assay, cytometric bead array assay, cytotoxicity assay, 51-chromium assay, degranulation assay, granulysin assay, granzyme A assay, granzyme B assay, and perforin assay. Methods herein, in some cases, screen a first population for peptides binding to antibodies or eliciting a T cell reaction in a biological sample from an individual, wherein the first population of peptides comprises peptides encoded by a frameshifted mRNA expressed by a cancer cell. In some cases, the first population of peptides comprises peptides having a sequence selected from the peptides provided in SEQ ID NO: 1-7264. In some cases, each of the first population of peptides binds to at least one MHC subtype. In some cases, a portion of the first population of peptides binds to more than one MHC subtype. In some cases, each of the first population of peptides binds to at least on HLA subtype. In some cases, each of the first population of peptides comprises at least one T cell epitope. In some cases, at least one of the first population of peptides comprises at least one T cell epitope. In some cases, each of the first population of peptides comprises at least on B cell epitope. In some cases, at least one of the first population of peptides comprises at least on B cell epitope. In some cases, first population of peptides is bound to a substrate.

### Methods of Treatment (methods falling outside the scope of the claims)

described herein, in some aspects, are methods of treating an individual in need of treatment for a cancer using a personalized cancer vaccine. An "individual" or "patient", used interchangeably herein, refers to an individual or a patient selected from a human, a primate, a mouse, a rat, a rabbit, a cat, a horse, a cow, or a pig. Some such methods comprise, a) obtaining a biological sample from the individual; b) determining immunoreactivity of the biological sample to a first population of peptides; c) preparing a vaccine composition comprising a second population of peptides that have immunoreactivity with the biological sample from the individual, wherein the second population of peptides is a sub-population of the first population; and d) administering the vaccine composition to the individual, wherein administering the vaccine composition elicits an immune response in the individual against the cancer and wherein the cancer in the individual is reduced. In some cases, the biological sample is selected from the group consisting of blood, plasma, serum, thymus, bone marrow, spleen, lymph node, bronchoalveolar lavage, breast, central nervous system, cerebrospinal fluid, eye, tears, gastrointestinal tract, saliva, feces, urine, heart, kidney, liver, lung, muscle, pancreas, peripheral nervous system, saliva, skin, thyroid, trachea, and tumor. In some cases, the biological sample comprises cells selected from B cells, T cells, CD4+ T cells, CD8+ T cells, Th17 cells, and combinations thereof. In some cases, the biological sample comprises an antibody.

Also described herein, in some aspects, are methods of reducing risk of reoccurrence of cancer in an individual, the methods comprising, a) obtaining a biological sample from the individual; b) determining immunoreactivity of the biological sample to a first population of peptides; c) preparing a vaccine composition comprising a second population of peptides that have immunoreactivity with the biological sample from the individual, wherein the second population of peptides is a sub-population of the first population; and d) administering the vaccine composition to the individual, wherein administering the vaccine composition elicits an immune response in the individual against the cancer and wherein the risk of developing cancer again in the individual is reduced compared to an individual who did not receive the vaccine.

Methods described herein comprise administration of one or more polypeptides reactive to immune cells and antibodies, for example in a biological sample from an individual with cancer. In some cases, immunoreactivity comprises antibody reactivity. In some cases, immunoreactivity comprises a T cell response. A T cell response herein includes but is not limited to one or more of proliferation assay, 3H-thymidine assay, BrdU assay, CFSE assay, cytokine secretion assay, ELISPOT assay, intracellular staining assay, quantitative rtPCR assay, cytometric bead array assay, cytotoxicity assay, 51-chromium assay, degranulation assay, granulysin assay, granzyme A assay, granzyme B assay, and perforin assay. Methods herein, in some cases, screen a first population for peptides binding to antibodies or eliciting a T cell reaction in a biological sample from an individual, wherein the first population of peptides comprises peptides encoded by a frameshifted mRNA expressed by a cancer cell. In some cases, the first population of peptides comprises peptides having a sequence selected from SEQ ID NO: 1-7264. In some cases, each of the first population of peptides binds to at least one MHC subtype. In some cases, a portion of the first population of peptides binds to more than one MHC subtype. In some cases, each of the first population of peptides binds to at least on HLA subtype. In some cases, each of the first population of peptides comprises at least one T cell epitope. In some cases, at least one of the first population of peptides comprises at least one T cell epitope. In some cases, each of the first population of peptides comprises at least on B cell epitope. In some cases, at least one of the first population of peptides comprises at least on B cell epitope. In some cases, first population of peptides is bound to a substrate.

Methods described herein comprise administration of a vaccine composition to an individual. Vaccine compositions herein, in some cases, comprise a pharmaceutically acceptable adjuvant or excipient. In some cases, the adjuvant is selected from the group consisting of ABM2, AS01B, AS02, AS02A, Adjumer, Adjuvax, Algammulin, Alum, Aluminum phosphate, Aluminum potassium sulfate, Bordetella pertussis, Calcitriol, Chitosan, Cholera toxin, CpG, Dibutyl phthalate, Dimethyldioctadecylammonium bromide (DDA), Freund's adjuvant, Freund's complete, Freund's incomplete (IFA), GM-CSF, GMDP, Gamma Inulin, Glycerol, HBSS (Hank's Balanced Salt Solution), IL-12, IL-2, Imiquimod, Interferon-Gamma, ISCOM, Lipid Core Peptide (LCP), Lipofectin, Lipopolysaccharide (LPS), Liposomes, MF59, MLP+TDM, Monophosphoryl lipid A, Montanide IMS-1313, Montanide ISA 206, Montanide ISA 720, Montanide ISA-51, Montanide ISA-50, nor-MDP, Oil-in-water emulsion, P1005 (non-ionic copolymer), Pam3Cys (lipoprotein), Pertussis toxin, Poloxamer, QS21, RaLPS, Ribi, Saponin, Seppic ISA 720, Soybean Oil, Squalene, Syntex Adjuvant Formulation (SAF), Synthetic polynucleotides (poly IC/poly AU), TiterMax Tomatine, Vaxfectin, XtendIII, and Zymosan. Vaccine compositions herein, in some cases, are administered via a route selected from the group consisting of subcutaneous, intradermal, intramuscular, intranasal, intravenous, and sublingual. Individuals in need of administration of a personalized vaccine, in some cases are mammals. In some cases, the individual is a human, a cat, a mouse, a rat, a rabbit, a horse, a cow, or a pig.

Methods described herein comprise administration of a personalized vaccine in an individual with cancer. In some cases, the cancer comprises Acanthoma, Acinic cell carcinoma, Acoustic neuroma, Acral lentiginous melanoma, Acrospiroma, Acute eosinophilic leukemia, Acute lymphoblastic leukemia, Acute megakaryoblastic leukemia, Acute monocytic leukemia, Acute myeloblastic leukemia with maturation, Acute myeloid dendritic cell leukemia, Acute myeloid leukemia, Acute promyelocytic leukemia, Adamantinoma, Adenocarcinoma, Adenoid cystic carcinoma, Adenoma, Adenomatoid odontogenic tumor, Adrenocortical carcinoma, Adult T-cell leukemia, Aggressive NK-cell leukemia, AIDS-Related Cancers, AIDS-related lymphoma, Alveolar soft part sarcoma, Ameloblastic fibroma, Anal cancer, Anaplastic large cell lymphoma, Anaplastic thyroid cancer, Angioimmunoblastic T-cell lymphoma, Angiomyolipoma, Angiosarcoma, Appendix cancer, Astrocytoma, Atypical teratoid rhabdoid tumor, Basal cell carcinoma, Basal-like carcinoma, B-cell leukemia, B-cell lymphoma, Bellini duct carcinoma, Biliary tract cancer, Bladder cancer, Blastoma, Bone Cancer, Bone tumor, Brain Stem Glioma, Brain Tumor, Breast Cancer, Brenner tumor, Bronchial Tumor, Bronchioloalveolar carcinoma, Brown tumor, Burkitt's lymphoma, Cancer of Unknown Primary Site, Carcinoid Tumor, Carcinoma, Carcinoma in situ, Carcinoma of the penis, Carcinoma of Unknown Primary Site, Carcinosarcoma, Castleman's Disease, Central Nervous System Embryonal Tumor, Cerebellar Astrocytoma, Cerebral Astrocytoma, Cervical Cancer, Cholangiocarcinoma, Chondroma, Chondrosarcoma, Chordoma, Choriocarcinoma, Choroid plexus papilloma, Chronic Lymphocytic Leukemia, Chronic monocytic leukemia, Chronic myelogenous leukemia, Chronic Myeloproliferative Disorder, Chronic neutrophilic leukemia, Clear-cell tumor, Colon Cancer, Colorectal cancer, Craniopharyngioma, Cutaneous T-cell lymphoma, Degos disease, Dermatofibrosarcoma protuberans, Dermoid cyst, Desmoplastic small round cell tumor, Diffuse large B cell lymphoma, Dysembryoplastic neuroepithelial tumor, Embryonal carcinoma, Endodermal sinus tumor, Endometrial cancer, Endometrial Uterine Cancer, Endometrioid tumor, Enteropathy-associated T-cell lymphoma, Ependymoblastoma, Ependymoma, Epithelioid sarcoma, Erythroleukemia, Esophageal cancer, Esthesioneuroblastoma, Ewing Family of Tumor, Ewing Family Sarcoma, Ewing's sarcoma, Extracranial Germ Cell Tumor, Extragonadal Germ Cell Tumor, Extrahepatic Bile Duct Cancer, Extramammary Paget's disease, Fallopian tube cancer, Fetus in fetu, Fibroma, Fibrosarcoma, Follicular lymphoma, Follicular thyroid cancer, Gallbladder Cancer, Gallbladder cancer, Ganglioglioma, Ganglioneuroma, Gastric Cancer, Gastric lymphoma, Gastrointestinal cancer, Gastrointestinal Carcinoid Tumor, Gastrointestinal Stromal Tumor, Gastrointestinal stromal tumor, Germ cell tumor, Germinoma, Gestational choriocarcinoma, Gestational Trophoblastic Tumor, Giant cell tumor of bone, Glioblastoma multiforme, Glioma, Gliomatosis cerebri, Glomus tumor, Glucagonoma, Gonadoblastoma, Granulosa cell tumor, Hairy Cell Leukemia, Hairy cell leukemia, Head and Neck Cancer, Head and neck cancer, Heart cancer, Hemangioblastoma, Hemangiopericytoma, Hemangiosarcoma, Hematological malignancy, Hepatocellular carcinoma, Hepatosplenic T-cell lymphoma, Hereditary breast-ovarian cancer syndrome, Hodgkin Lymphoma, Hodgkin's lymphoma, Hypopharyngeal Cancer, Hypothalamic Glioma, Inflammatory breast cancer, Intraocular Melanoma, Islet cell carcinoma, Islet Cell Tumor, Juvenile myelomonocytic leukemia, Kaposi Sarcoma, Kaposi's sarcoma, Kidney Cancer, Klatskin tumor, Krukenberg tumor, Laryngeal Cancer, Laryngeal cancer, Lentigo maligna melanoma, Leukemia, Lip and Oral Cavity Cancer, Liposarcoma, Lung cancer, Luteoma, Lymphangioma, Lymphangiosarcoma, Lymphoepithelioma, Lymphoid leukemia, Lymphoma, Macroglobulinemia, Malignant Fibrous Histiocytoma, Malignant fibrous histiocytoma, Malignant Fibrous Histiocytoma of Bone, Malignant Glioma, Malignant Mesothelioma, Malignant peripheral nerve sheath tumor, Malignant rhabdoid tumor, Malignant triton tumor, MALT lymphoma, Mantle cell lymphoma, Mast cell leukemia, Mediastinal germ cell tumor, Mediastinal tumor, Medullary thyroid cancer, Medulloblastoma, Medulloepithelioma, Melanoma, Meningioma, Merkel Cell Carcinoma, Mesothelioma, Metastatic Squamous Neck Cancer with Occult Primary, Metastatic urothelial carcinoma, Mixed Mullerian tumor, Monocytic leukemia, Mouth Cancer, Mucinous tumor, Multiple Endocrine Neoplasia Syndrome, Multiple myeloma, Mycosis Fungoides, Myelodysplastic Disease, Myelodysplastic Syndromes, Myeloid leukemia, Myeloid sarcoma, Myeloproliferative Disease, Myxoma, Nasal Cavity Cancer, Nasopharyngeal Cancer, Nasopharyngeal carcinoma, Neoplasm, Neurinoma, Neuroblastoma, Neurofibroma, Neuroma, Nodular melanoma, Non-Hodgkin lymphoma, Nonmelanoma Skin Cancer, Non-Small Cell Lung Cancer, Ocular oncology, Oligoastrocytoma, Oligodendroglioma, Oncocytoma, Optic nerve sheath meningioma, Oral cancer, Oropharyngeal Cancer, Osteosarcoma, Ovarian cancer, Ovarian Epithelial Cancer, Ovarian Germ Cell Tumor, Ovarian Low Malignant Potential Tumor, Paget's disease of the breast, Pancoast tumor, Pancreatic cancer, Papillary thyroid cancer, Papillomatosis, Paraganglioma, Paranasal Sinus Cancer, Parathyroid Cancer, Penile Cancer, Perivascular epithelioid cell tumor, Pharyngeal Cancer, Pheochromocytoma, Pineal Parenchymal Tumor of Intermediate Differentiation, Pineoblastoma, Pituicytoma, Pituitary adenoma, Pituitary tumor, Plasma Cell Neoplasm, Pleuropulmonary blastoma, Polyembryoma, Precursor T-lymphoblastic lymphoma, Primary central nervous system lymphoma, Primary effusion lymphoma, Primary Hepatocellular Cancer, Primary Liver Cancer, Primary peritoneal cancer, Primitive neuroectodermal tumor, Prostate cancer, Pseudomyxoma peritonei, Rectal Cancer, Renal cell carcinoma, Respiratory Tract Carcinoma Involving the NUT Gene on Chromosome 15, Retinoblastoma, Rhabdomyoma, Rhabdomyosarcoma, Richter's transformation, Sacrococcygeal teratoma, Salivary Gland Cancer, Sarcoma, Schwannomatosis, Sebaceous gland carcinoma, Secondary neoplasm, Seminoma, Serous tumor, Sertoli-Leydig cell tumor, Sex cord-stromal tumor, Sezary Syndrome, Signet ring cell carcinoma, Skin Cancer, Small blue round cell tumor, Small cell carcinoma, Small Cell Lung Cancer, Small cell lymphoma, Small intestine cancer, Soft tissue sarcoma, Somatostatinoma, Soot wart, Spinal Cord Tumor, Spinal tumor, Splenic marginal zone lymphoma, Squamous cell carcinoma, Stomach cancer, Superficial spreading melanoma, Supratentorial Primitive Neuroectodermal Tumor, Surface epithelial-stromal tumor, Synovial sarcoma, T-cell acute lymphoblastic leukemia, T-cell large granular lymphocyte leukemia, T-cell leukemia, T-cell lymphoma, T-cell prolymphocytic leukemia, Teratoma, Terminal lymphatic cancer, Testicular cancer, Thecoma, Throat Cancer, Thymic Carcinoma, Thymoma, Thyroid cancer, Transitional Cell Cancer of Renal Pelvis and Ureter, Transitional cell carcinoma, Urachal cancer, Urethral cancer, Urogenital neoplasm, Uterine sarcoma, Uveal melanoma, Vaginal Cancer, Verner Morrison syndrome, Verrucous carcinoma, Visual Pathway Glioma, Vulvar Cancer, Waldenstrom's macroglobulinemia, Warthin's tumor, or Wilms' tumor.

Described herein, in some aspects, are methods of monitoring treatment of an individual receiving a personalized cancer vaccine. Some such methods comprise, a) obtaining a biological sample from the individual; b) determining immunoreactivity of the biological sample to a first population of peptides; c) comparing immunoreactivity of the biological sample to a previous immunoreactivity in the individual, wherein successful treatment is indicated by reduced immunoreactivity to the first population of peptides. In some cases, the biological sample is selected from the group consisting of blood, plasma, serum, thymus, bone marrow, spleen, lymph node, bronchoalveolar lavage, breast, central nervous system, cerebrospinal fluid, eye, tears, gastrointestinal tract, saliva, feces, urine, heart, kidney, liver, lung, muscle, pancreas, peripheral nervous system, saliva, skin, thyroid, trachea, and tumor. In some cases, the biological sample comprises cells selected from B cells, T cells, CD4+ T cells, CD8+ T cells, Th17 cells, and combinations thereof. In some cases, the biological sample comprises an antibody.

### Methods of Diagnosing Cancer (methods falling outside the scope of the claims)

described herein, in some aspects, are methods of determining whether an individual has cancer. Some such methods comprise, a) obtaining a biological sample from the individual and b) determining immunoreactivity of the biological sample to a first population of peptides, wherein the individual is determined to have cancer if the sample from the individual is immunoreactive with one or more of the peptides. In some cases, the method further comprises treating the individual using a method comprising, a) preparing a vaccine composition comprising a second population of peptides that have immunoreactivity with the biological sample from the individual, wherein the second population of peptides is a sub-population of the first population and b) administering the vaccine composition to the individual, wherein administering the vaccine composition elicits an immune response in the individual against the cancer and wherein the cancer in the individual is reduced. In some cases, the biological sample is selected from the group consisting of blood, plasma, serum, thymus, bone marrow, spleen, lymph node, bronchoalveolar lavage, breast, central nervous system, cerebrospinal fluid, eye, tears, gastrointestinal tract, saliva, feces, urine, heart, kidney, liver, lung, muscle, pancreas, peripheral nervous system, saliva, skin, thyroid, trachea, and tumor. In some cases, the biological sample comprises cells selected from B cells, T cells, CD4+ T cells, CD8+ T cells, Th17 cells, and combinations thereof. In some cases, the biological sample comprises an antibody.

Methods described herein comprise determining whether an individual has cancer by determining whether one or more polypeptides reactive to immune cells and antibodies, for example in a biological sample from the individual. In some cases, immunoreactivity comprises antibody reactivity. In some cases, immunoreactivity comprises a T cell response. A T cell response herein includes but is not limited to one or more of proliferation assay, 3H-thymidine assay, BrdU assay, CFSE assay, cytokine secretion assay, ELISPOT assay, intracellular staining assay, quantitative rtPCR assay, cytometric bead array assay, cytotoxicity assay, 51-chromium assay, degranulation assay, granulysin assay, granzyme A assay, granzyme B assay, and perforin assay. Methods herein, in some cases, screen a first population for peptides binding to antibodies or eliciting a T cell reaction in a biological sample from an individual, wherein the first population of peptides comprises peptides encoded by a frameshifted mRNA expressed by a cancer cell. In some cases, the first population of peptides comprises peptides having a sequence selected from SEQ ID NO: 1-7264. In some cases, each of the first population of peptides binds to at least one MHC subtype. In some cases, a portion of the first population of peptides binds to more than one MHC subtype. In some cases, each of the first population of peptides binds to at least on HLA subtype. In some cases, each of the first population of peptides comprises at least one T cell epitope. In some cases, at least one of the first population of peptides comprises at least one T cell epitope. In some cases, each of the first population of peptides comprises at least on B cell epitope. In some cases, at least one of the first population of peptides comprises at least on B cell epitope. In some cases, first population of peptides is bound to a substrate.

Methods herein comprise determining whether an individual has cancer. In some cases, the cancer comprises Acanthoma, Acinic cell carcinoma, Acoustic neuroma, Acral lentiginous melanoma, Acrospiroma, Acute eosinophilic leukemia, Acute lymphoblastic leukemia, Acute megakaryoblastic leukemia, Acute monocytic leukemia, Acute myeloblastic leukemia with maturation, Acute myeloid dendritic cell leukemia, Acute myeloid leukemia, Acute promyelocytic leukemia, Adamantinoma, Adenocarcinoma, Adenoid cystic carcinoma, Adenoma, Adenomatoid odontogenic tumor, Adrenocortical carcinoma, Adult T-cell leukemia, Aggressive NK-cell leukemia, AIDS-Related Cancers, AIDS-related lymphoma, Alveolar soft part sarcoma, Ameloblastic fibroma, Anal cancer, Anaplastic large cell lymphoma, Anaplastic thyroid cancer, Angioimmunoblastic T-cell lymphoma, Angiomyolipoma, Angiosarcoma, Appendix cancer, Astrocytoma, Atypical teratoid rhabdoid tumor, Basal cell carcinoma, Basal-like carcinoma, B-cell leukemia, B-cell lymphoma, Bellini duct carcinoma, Biliary tract cancer, Bladder cancer, Blastoma, Bone Cancer, Bone tumor, Brain Stem Glioma, Brain Tumor, Breast Cancer, Brenner tumor, Bronchial Tumor, Bronchioloalveolar carcinoma, Brown tumor, Burkitt's lymphoma, Cancer of Unknown Primary Site, Carcinoid Tumor, Carcinoma, Carcinoma in situ, Carcinoma of the penis, Carcinoma of Unknown Primary Site, Carcinosarcoma, Castleman's Disease, Central Nervous System Embryonal Tumor, Cerebellar Astrocytoma, Cerebral Astrocytoma, Cervical Cancer, Cholangiocarcinoma, Chondroma, Chondrosarcoma, Chordoma, Choriocarcinoma, Choroid plexus papilloma, Chronic Lymphocytic Leukemia, Chronic monocytic leukemia, Chronic myelogenous leukemia, Chronic Myeloproliferative Disorder, Chronic neutrophilic leukemia, Clear-cell tumor, Colon Cancer, Colorectal cancer, Craniopharyngioma, Cutaneous T-cell lymphoma, Degos disease, Dermatofibrosarcoma protuberans, Dermoid cyst, Desmoplastic small round cell tumor, Diffuse large B cell lymphoma, Dysembryoplastic neuroepithelial tumor, Embryonal carcinoma, Endodermal sinus tumor, Endometrial cancer, Endometrial Uterine Cancer, Endometrioid tumor, Enteropathy-associated T-cell lymphoma, Ependymoblastoma, Ependymoma, Epithelioid sarcoma, Erythroleukemia, Esophageal cancer, Esthesioneuroblastoma, Ewing Family of Tumor, Ewing Family Sarcoma, Ewing's sarcoma, Extracranial Germ Cell Tumor, Extragonadal Germ Cell Tumor, Extrahepatic Bile Duct Cancer, Extramammary Paget's disease, Fallopian tube cancer, Fetus in fetu, Fibroma, Fibrosarcoma, Follicular lymphoma, Follicular thyroid cancer, Gallbladder Cancer, Gallbladder cancer, Ganglioglioma, Ganglioneuroma, Gastric Cancer, Gastric lymphoma, Gastrointestinal cancer, Gastrointestinal Carcinoid Tumor, Gastrointestinal Stromal Tumor, Gastrointestinal stromal tumor, Germ cell tumor, Germinoma, Gestational choriocarcinoma, Gestational Trophoblastic Tumor, Giant cell tumor of bone, Glioblastoma multiforme, Glioma, Gliomatosis cerebri, Glomus tumor, Glucagonoma, Gonadoblastoma, Granulosa cell tumor, Hairy Cell Leukemia, Hairy cell leukemia, Head and Neck Cancer, Head and neck cancer, Heart cancer, Hemangioblastoma, Hemangiopericytoma, Hemangiosarcoma, Hematological malignancy, Hepatocellular carcinoma, Hepatosplenic T-cell lymphoma, Hereditary breast-ovarian cancer syndrome, Hodgkin Lymphoma, Hodgkin's lymphoma, Hypopharyngeal Cancer, Hypothalamic Glioma, Inflammatory breast cancer, Intraocular Melanoma, Islet cell carcinoma, Islet Cell Tumor, Juvenile myelomonocytic leukemia, Kaposi Sarcoma, Kaposi's sarcoma, Kidney Cancer, Klatskin tumor, Krukenberg tumor, Laryngeal Cancer, Laryngeal cancer, Lentigo maligna melanoma, Leukemia, Lip and Oral Cavity Cancer, Liposarcoma, Lung cancer, Luteoma, Lymphangioma, Lymphangiosarcoma, Lymphoepithelioma, Lymphoid leukemia, Lymphoma, Macroglobulinemia, Malignant Fibrous Histiocytoma, Malignant fibrous histiocytoma, Malignant Fibrous Histiocytoma of Bone, Malignant Glioma, Malignant Mesothelioma, Malignant peripheral nerve sheath tumor, Malignant rhabdoid tumor, Malignant triton tumor, MALT lymphoma, Mantle cell lymphoma, Mast cell leukemia, Mediastinal germ cell tumor, Mediastinal tumor, Medullary thyroid cancer, Medulloblastoma, Medulloepithelioma, Melanoma, Meningioma, Merkel Cell Carcinoma, Mesothelioma, Metastatic Squamous Neck Cancer with Occult Primary, Metastatic urothelial carcinoma, Mixed Mullerian tumor, Monocytic leukemia, Mouth Cancer, Mucinous tumor, Multiple Endocrine Neoplasia Syndrome, Multiple myeloma, Mycosis Fungoides, Myelodysplastic Disease, Myelodysplastic Syndromes, Myeloid leukemia, Myeloid sarcoma, Myeloproliferative Disease, Myxoma, Nasal Cavity Cancer, Nasopharyngeal Cancer, Nasopharyngeal carcinoma, Neoplasm, Neurinoma, Neuroblastoma, Neurofibroma, Neuroma, Nodular melanoma, Non-Hodgkin lymphoma, Nonmelanoma Skin Cancer, Non-Small Cell Lung Cancer, Ocular oncology, Oligoastrocytoma, Oligodendroglioma, Oncocytoma, Optic nerve sheath meningioma, Oral cancer, Oropharyngeal Cancer, Osteosarcoma, Ovarian cancer, Ovarian Epithelial Cancer, Ovarian Germ Cell Tumor, Ovarian Low Malignant Potential Tumor, Paget's disease of the breast, Pancoast tumor, Pancreatic cancer, Papillary thyroid cancer, Papillomatosis, Paraganglioma, Paranasal Sinus Cancer, Parathyroid Cancer, Penile Cancer, Perivascular epithelioid cell tumor, Pharyngeal Cancer, Pheochromocytoma, Pineal Parenchymal Tumor of Intermediate Differentiation, Pineoblastoma, Pituicytoma, Pituitary adenoma, Pituitary tumor, Plasma Cell Neoplasm, Pleuropulmonary blastoma, Polyembryoma, Precursor T-lymphoblastic lymphoma, Primary central nervous system lymphoma, Primary effusion lymphoma, Primary Hepatocellular Cancer, Primary Liver Cancer, Primary peritoneal cancer, Primitive neuroectodermal tumor, Prostate cancer, Pseudomyxoma peritonei, Rectal Cancer, Renal cell carcinoma, Respiratory Tract Carcinoma Involving the NUT Gene on Chromosome 15, Retinoblastoma, Rhabdomyoma, Rhabdomyosarcoma, Richter's transformation, Sacrococcygeal teratoma, Salivary Gland Cancer, Sarcoma, Schwannomatosis, Sebaceous gland carcinoma, Secondary neoplasm, Seminoma, Serous tumor, Sertoli-Leydig cell tumor, Sex cord-stromal tumor, Sezary Syndrome, Signet ring cell carcinoma, Skin Cancer, Small blue round cell tumor, Small cell carcinoma, Small Cell Lung Cancer, Small cell lymphoma, Small intestine cancer, Soft tissue sarcoma, Somatostatinoma, Soot wart, Spinal Cord Tumor, Spinal tumor, Splenic marginal zone lymphoma, Squamous cell carcinoma, Stomach cancer, Superficial spreading melanoma, Supratentorial Primitive Neuroectodermal Tumor, Surface epithelial-stromal tumor, Synovial sarcoma, T-cell acute lymphoblastic leukemia, T-cell large granular lymphocyte leukemia, T-cell leukemia, T-cell lymphoma, T-cell prolymphocytic leukemia, Teratoma, Terminal lymphatic cancer, Testicular cancer, Thecoma, Throat Cancer, Thymic Carcinoma, Thymoma, Thyroid cancer, Transitional Cell Cancer of Renal Pelvis and Ureter, Transitional cell carcinoma, Urachal cancer, Urethral cancer, Urogenital neoplasm, Uterine sarcoma, Uveal melanoma, Vaginal Cancer, Verner Morrison syndrome, Verrucous carcinoma, Visual Pathway Glioma, Vulvar Cancer, Waldenstrom's macroglobulinemia, Warthin's tumor, or Wilms' tumor.

The following peptides are for use in personalized cancer vaccines for humans. CCDS43382.1, Del, A_10, RKRMKTGAIQEKGAIQEKGAMTKRICVVNFEACREMESLSAPEKITLFEAHMARCTSINVLC VRASLIEKLMKEKRKMKRNQVASPQIMQRMSAVNFETI, (SEQIDNO:1); CCDS34380.1, Del, A_10, SEIPEKAGGRRMWMMMEKRKSSWSVLRSSQCQPVMRRMKYPPQNPAEGRKPRVVMFLQP, (SEQIDNO:4); CCDS43478.1, Del, A_10, RKRKREKRSQKSRQNHLQLKSCRRKISNWSLKKVPALKKLRSPLWIF, (SEQIDNO:6); CCDS42127.1, Del, A_10, KSTRREMPSQATPSPPGPWRAALGKEVKRSQSKLRLRNTSP, (SEQIDNO:7); CCDS43314.1, Ins, A_10, KTSDFPMCWLRQSDSRSVYSEGFSGFGMACGMFEMCGV, (SEQIDNO:8); CCDS53862.1, Ins, A_10, KKFIYIHCVDRGLLFPETWGFEQNNTWRQYNETSFL, (SEQIDNO:9); CCDS2648.1, Del, A_10, SLVRLSSCVPVALMSAMTTSSSQKNITPAILTCC, (SEQIDNO:10); CCDS33304.1, Ins, A_10, KNKKILGQEKTEVARNGNERSSASRRTEEINR, (SEQIDNO:11); CCDS4156.1, Ins, A_10, KVWRRKTESKPIPRLPARVSWCNEHRVDNRKL, (SEQIDNO: 12); CCDS33641.1, Del, A_10, RKKKRRTKREREEKSQKRRTCRPTRCSVKSIA, (SEQIDNO: 13); CCDS791.2, Ins, A_10, KTSGFHDLQLRPCNTLTPFLLLQDFWKQILM, (SEQIDNO: 14); CCDS33641.1, Ins, A_10, KEKRREGQRERERRKAKKEEHVGLPGVL, (SEQIDNO: 15); CCDS53731.1, Ins, A_10, KAGREQSQTAGRTIQCFPDRNQAAPFC, (SEQIDNO: 16); CCDS5061.1, Del, A_10, NLHLCYYHSQSNRNKSRQMESLGMKLQ, (SEQIDNO: 17); CCDS13627.1, Ins, A_10, KGQSGCLNIWNRIEPASTYRQYSGTK, (SEQIDNO: 18); CCDS44611.1, Del, A_10, KHYNRKISIKKLNRMKVPLMKLITRV, (SEQIDNO: 19); CCDS8328.2, Del, A_10, KQLRCWNTWAKMFFMVFLIIWQNTMF, (SEQIDNO:20); CCDS46894.1, Del, A_10, ITHPFASQPLGQEMPNNTQIRLLKP, (SEQIDNO:21); CCDS45025.1, Del, A_10, KLKLAPLQVTTTLKLILVMLEIVTE, (SEQIDNO:22); CCDS5061.1, Ins, A_10, KTYTCAITTVKATETKAGKWSRWE, (SEQIDNO:23); CCDS42127.1, Ins, A_10, KNPPGGRCPPRPLQALQVHGEQP, (SEQIDNO:24); CCDS44720.1, Del, A_10, KGVRILKLCSKVSFRVDWITS, (SEQIDNO:25); CCDS46894.1, Ins, A_10, KLLIHLPPSHWDRKCQTTPR, (SEQIDNO:26); CCDS55858.1, Ins, A_10, KNSSNGSRKRKKKCNFRINH, (SEQIDNO:27); CCDS41816.1, Del, A_10, IRHTDTQQDLQVSCGFLQN, (SEQIDNO:28); CCDS43478.1, Ins, A_10, KGREKERKGARKAGKTTYS, (SEQIDNO:29); CCDS55405.1, Del, A_10, MYYPPKTLKGCANPPDYL, (SEQIDNO:30); CCDS55858.1, Del, A_10, KFVKWLKKEEKEVQLQD, (SEQIDNO:31); CCDS9638.1, Del, A_10, KLKDCISTKPISGIVP, (SEQIDNO:32); CCDS7253.1, Ins, A_10, KRVNTAWKTKKTSFSL, (SEQIDNO:33); CCDS43071.1, Del, A_10, RTLESAGLFPWLHTQC, (SEQIDNO:34); CCDS43122.1, Ins, A_10, KIFKIFIYEGKSEKS, (SEQIDNO:35); CCDS59217.1, Ins, A_10, KRKAEDCKGKLEKSV, (SEQIDNO:36); CCDS34380.1, Ins, A_10, KARYPKRQAEEGCG, (SEQIDNO:37); CCDS2346.1, Ins, A_10, KDDQKIRNGVRRRR, (SEQIDNO:38); CCDS3058.1, Ins, A_10, KRKIIEFRIKFLF, (SEQIDNO:39); CCDS41816.1, Ins, A_10, KSGTQTLSRIFK, (SEQIDNO:40); CCDS41293.1, Del, A_10, KVVINLKRLQKF, (SEQIDNO:41); CCDS34171.1, Ins, A_10, KKEKVLLIQFH, (SEQIDNO:42); CCDS11906.1, Ins, A_10, KNGGNGPTQKS, (SEQIDNO:43); CCDS53731.1, Del, A_10, SRKRAVSNSR, (SEQIDNO:44); CCDS717.2, Del, A_10, KPRDICRIS, (SEQIDNO:45); CCDS44611.1, Ins, A_10, KSITTERYP, (SEQIDNO:46); CCDS53862.1, Del, A_10, KVYLHPLCG, (SEQIDNO:47); CCDS55405.1, Ins, A_10, KCITHQRH, (SEQIDNO:48); CCDS3190.1, Ins, A_10, KKFPNGSL, (SEQIDNO:49); CCDS10158.1, Del, A_10, SRKLMSSC, (SEQIDNO:51); CCDS4156.1, Del, A_10, SVEKKDGE, (SEQIDNO:52); CCDS31683.1, Ins, A_11, KKGKERQEGQRPPQKFAGLGFLISPSSCSSWGPGSFTRSCGYPTLCSSWISKREPGELSGVWTS AWRTHAAFTGSQDLCLYKGSLGLHI, (SEQIDNO:53); CCDS8043.1, Del, A_11, NLLCVKCSTCPTYVKGSPSCPLRDLQTLWPILALISMSSIWGTMFSCCRLSLVQSSSWPTVLHL GH, (SEQIDNO:54); CCDS56392.1, Ins, A_11, KKEKKKQHGCKGETGGSVKCGPEGAKHHAVGCPVQMGCQLLFPADPKK, (SEQIDNO:55); CCDS56391.1, Ins, A_11, KKQHGCKGETGGSVKCGPEGAKHHAVGCPVQMGCQLLFPADPKK, (SEQIDNO:56); CCDS33527.2, Del, A_11, KMVRLDLLMRYLKAIKRMKNVYLQKERRLKAGN, (SEQIDNO:58); CCDS5101.1, Ins, A_11, KKEALFLQEVFQAERLLLQEEQEGGWRRR, (SEQIDNO:59); CCDS33143.1, Del, A_11, KTILKKAGIGMCVKVSSIFFINKQKP, (SEQIDNO:60); CCDS43133.1, Del, A_11, KTLRNHLFLIRWIILTFLQKILK, (SEQIDNO:61); CCDS9684.1, Del, A_11, NCSIPTYVKKRKTTNHSSQMTVH, (SEQIDNO:62); CCDS31683.1, Del, A_11, KRKRKTRRTETPPKVRWPWVPH, (SEQIDNO:63); CCDS56392.1, Del, A_11, KRKKKTTWLQRRNWRQC, (SEQIDNO:64); CCDS46478.1, Del, A_11, KKSWRLLLHSSNLI, (SEQIDNO:65); CCDS10374.2, Ins, A_11, KILLNLSCQSCTF, (SEQIDNO:66); CCDS46478.1, Ins, A_11, KRRVGDCYFTAQI, (SEQIDNO:67); CCDS56391.1, Del, A_11, KTTWLQRRNWRQC, (SEQIDNO:68); CCDS3068.1, Ins, A_11, KKAEETEYQLF, (SEQIDNO:70); CCDS43133.1, Ins, A_11, KKPSEIISF, (SEQIDNO:71); CCDS9684.1, Ins, A_11, KIAAYLPM, (SEQIDNO:72); CCDS781.1, Del, A_12, KGLMTLSKMIKKKKNLVI, (SEQIDNO:73); CCDS35085.1, Del, A_7, MQTKVMNRRMPRKMQRRLKKVRKIRRSPVVQTVTQRMMSRKSLRRTPRKMPRKLPRKILR KNLLIQRRMQRKMLRRMQRRMQRRMQRRMKRRMQRRRAS, (SEQIDNO:74); CCDS 13900.2, Ins, A_7, TELLQPHAYHAEALHDPVYTGPERVTVSSLHSRQGLLFSALLGGQSPIHLEPAWSFCLLLRNL FSSFPSLQAATKSGHHKQPHHVTICASSRHGSYI, (SEQIDNO:76); CCDS9460.1, Del, A_7, GHVVIKNMTNTVWRMKRNLIICHHLSLVFQDVPLSKEEFPIHRLSPAFGSVGGAPVPSIFLQIIT SSNSIIHLKPKLQISNQIGPMEISSEEVCLT, (SEQIDNO:77); CCDS41355.1, Del, A_7, KMNMDHQANCLSMSGLTSGGECLMSTMKISLSTVLSQKKSKQQGVPQVSLVEIEFISVKEAA VLVEHAELILQQNCMEKIVNSFLIKYKQKKHP, (SEQIDNO:79); CCDS31477.1, Ins, A_7, MAREKRGPGVCRSTNKKPQTGSWRLCRFSKSIKEGCWKGHQCHVGGFGSKMSYWPGCWA KEEIWTICRTCCANHLGEIQREETSSGTSPAGGN, (SEQIDNO:80); CCDS590.1, Del, A_7, SRWENRLGFCTRGGVYFGTFTVQLLKGETLVNIQVKQKPFMIIVQQLPRAPCLATFPPTMGCR EAPSRQVSFRVLRGHSPRRTHSLHLTILAA, (SEQIDNO:81); CCDS46430.1, Del, A_7, DSDQSLVLLQNILLVQSLREVIKSQKSIRRKVRRGDINLTLQNPMLSERRIKKKKIGKVKKTEL DKDQNQNTNRLRKRLERILVIGILLAAN, (SEQIDNO:82); CCDS4643.1, Del, A_7, TNMENLSRAQNLLNMQQFSVEIKLISVMNVGKLSGTAQNLLGIRESTLERDAMNVMNVGKA LQRAQILLDIGEFTLGKDPLVAKNVGEHST, (SEQIDNO:84); CCDS43027.1, Del, A_7, GDTIFSPSVLSMSQSQLIKSTRSSAETTAPKHFLAQRTLKNLTWRELAITVWPQANGLCQWTI CQGEKSVWQPWLSCLLCTVFVLPHSLF, (SEQIDNO:85); CCDS43226.1, Del, A_7, KRKKKRRASQMIKTKIKTTQRRKRRKRTKRRKRKRRKAKIRKKRRRKKLWLLIPRETHITTG CFASHYLLCTTGQWLLPEHVLMNFNLIT, (SEQIDNO:86); CCDS10425.1, Ins, A_7, NRGALRLRHRLLKALQPPGEPARVCAAIPRTGRLQTGAAPAGLCLPRLDLLCREDSRQLHPPP HQHRPVPAAGHGLPGQGLLRPAAALDP, (SEQIDNO:87); CCDS34353.1, Del, A_7, ERISRLKESIGNILIVTVTQSLTQILTLMMIKRELKPRRKRRKRNTKQRKRRIRKPKKNPVTQA VKTQKRTCQKLPGWSSQMWQILWI, (SEQIDNO:90); CCDS1480.1, Del, A_7, FTTKQQLCLNAIRVFTSMAATQLSVTVTVLGIPQFQSVLKCRLLPLQNLQRPVPQVLGLLTSL QSQIIQDILNLRKEYLTVWMFGSLL, (SEQIDNO:91); CCDS33011.1, Del, A_7, RGRYHKVPRPMILEKATTLSPPTLVLLPCPGSCRPWQNNWQKITTTRGDGRRSRSWKPKAVG PTPCWSPTTRSRPRRRHEIERRPRSY, (SEQIDNO:92); CCDS42415.1, Ins, A_7, SGAGVRPNHHHRRGGHGDGGGHRLPAEPLQSLHAVLHQPPEPEPEAGGRAAADHACPAVQ GQVHSAVLHPEGSLQPLPAHLPLCAARD, (SEQIDNO:93); CCDS47583.1, Del, A_7, VHLPIFPNSLAMVIRMSGCSSSSELTQTRGPRRPPGHQCYLSPHETWKVPRMQPGSRMQRLLK VLRTPQSLLKAQKNLYLHQHQQSSN, (SEQIDNO:94); CCDS54765.1, Del, A_7, WRSYWLEKMGQVNEGKASKPTEKLFKKKSGERESCMKHIRTLGPRRRQRGSFNSTLRGSPS VRLFSNAWRCQKFWKEAIQQSQIYPPS, (SEQIDNO:95); CCDS8518.1, Del, A_7, AASLPRKPRCCSTNNHWRAPNTAVHLHSFPSLTLDRCPASPLTTAPSLPGYHLILIQQQEACSQ PTRNTKTGRDTQVENLPPPSFHI, (SEQIDNO:96); CCDS30960.1, Del, A_7, YIQVKKLISVKNVENVLVVGIISLFITKAYTLERKCGKNIKQHFINVMFVRKFLKANQVWKCI FERIQVKNHTSVKFAISLLELRKH, (SEQIDNO:100); CCDS31778.2, Del, A_7, KSMCKEQELLLNTVDQITQLKELIVLIDKRKNLFCRCCVWVIYTTLMYIIPSISLWKRGVTCSH GTPMDHGKAVPKCVKVFSEET, (SEQIDNO:102); CCDS45068.1, Ins, A_7, NHLGPGGLSAGVAEGWGGPLQAAGAPAPRDHRESLPRAPERERVPEQHPRVPARLRGFPAA GAPVSSFPASSASGNHHSLVCFYV, (SEQIDNO:103); CCDS30621.1, Del, A_7, PCRNAKRMGGWNRSLGKGSVAPSSSVFPIIPAQEFCFRRKSQMILEMRMKMKMSHQKKTLR MKSRHLREGCRRKPQPSPQGRPHL, (SEQIDNO:104); CCDS12712.2, Del, A_7, SVQKRVAAVRKSCRDGYTGSYPVFLRPFVTSHIFSKKMMRQRQSHYCSVLFLSVNYLGGTFP VYQKSRNLQRDKIQETFAQKRIK, (SEQIDNO:105); CCDS2369.1, Del, A_7, WESWQMKTIHCNRIAAVISVTAMQCRKKSHCLQDSYITSTKTRKALITFLTQRQDTSKNIIRLS IWPRQASRLKPSAPNSFLTSY, (SEQIDNO:106); CCDS7282.1, Del, A_7, AKKMREKIKKKKEMMKLMNQNPNGENQAMIKIKKKIEMKGRKKIKEKMILKMMMKLKKIT IKMNMTLWKQKKLRMKKMIGMRKK, (SEQIDNO:107); CCDS5043.1, Del, A_7, SIKKKKNKEGVGREVLVVVVPVAIAELVVLVVLSLALHTVLAQVVVVLLLGLLLLKGKRDT VGVDLQQSKLDVAGVEAILAELK, (SEQIDNO:108); CCDS35009.1, Del, A_7, ARITLGWKEMRSLLCWRTQCPPKSQQSCSSNTTGWGKWSMALWPCLPSCALEPL VLRISRW AACPLPSSRLPAARCTEDTCLL, (SEQIDNO:110); CCDS45641.1, Ins, A_7, NHQQPVSGPRRQCHGALWLHRGPGGRLAAGTAPGHQHAENLYHRHGGCQKEVTVHPDEAT GGRPHRHHQEVKAAFCALLPACS, (SEQIDNO: 111); CCDS42395.1, Ins, A_7, NPTGKRDAFLSGFRDRPDGDPGHEGRDPQDEGQARAAAEAAGEDDPCHGVGGCPQRDRHH PGRGAAQDGQEGAHPHRLPPQAA, (SEQIDNO: 112); CCDS47512.1, Del, A_7, PPIPLPLPCSPPLLRRPCLPLQPRGPPLPPPAARPPPAARPPGVQQPQSEPLRGQPPPPPPHPPLPS PPVPLGPWNGTTMMAT, (SEQIDNO:113); CCDS13265.1, Del, A_7, ARAEVVVMNEREAKVRNGSEVETEKGKRAKAVKESEVEAKRGDGAAQEVEIEDLEAATEV LTPDQNLTVPSEERLGCLIASN, (SEQIDNO: 114); CCDS34357.1, Del, A_7, PTVGRSLTSAGTVGRPSRPGTVSVCISLSTPGRSLTNVTAVGRPSSLSIPLPSMAESTLGRSHM NVRSAGRPSVGVQTSPNT, (SEQIDNO: 115); CCDS53784.1, Del, A_7, ARLLASITGSASTWRRRRVWLTITVTSTMGLGILTPMCWQCSSTGTATIRKWALLSSAAALSL SLHSTPCASSPGQAKCAS, (SEQIDNO: 116); CCDS3806.1, Del, A_7, ARYQPCITLTKMPNYLTCFSRQAAQQQGQLPGASQGCLSVHPLRTSAESVTAKGMKRAPSSH PVAALGHCALSTSPASTSG, (SEQIDNO: 117); CCDS454.1, Del, A_7, IQGKNLLNAMTVEKPIAGRHTLQLIRKFIMERDPLCAMIVGRRLCIKPNSWSTRDFTLERSLM SAVNVGKHSLGTPHLINT, (SEQIDNO: 118); CCDS30744.1, Del, A_7, FRRKAMGHQMDFMRKLIGKDITHLSWMKKATASDPRNPALPKESTFILQVNRKKKKNHIRN LISRLNHCNLKTFLRMLQL, (SEQIDNO: 119); CCDS47512.1, Ins, A_7, NHPSPYHYHAAHHYYDAPAYHYNPEAHHCHHPPHDHHPPHDHQASNNHSPNHY ADNHHH HPHTHHSHPHLSPWDLGTARR, (SEQIDNO: 120); CCDS3158.2, Del, A_7, YMILIESPKVRTEKTTKSWKAKYLLSWLSSLCVLLHFILPEFHILTVKPTIRLTVDCKINCLLLK KQLSFWQQLTFVWIP, (SEQIDNO: 123); CCDS41850.1, Del, A_7, KQLYIQNALNLSRPHSARLTASDTTRTRTCRTWRRSSASAASAWRRSFAASCTRGTSCTPWR AKISTMSTYREKLSGFP, (SEQIDNO: 124); CCDS43337.1, Del, A_7, WRNWKKEVMVKNQMRKMKRKKEAKRKVKKVMMTMTMMPQNRRNMMKKSKKRKMTT LIHTLKMEMILAQTVMTTWMRQPI, (SEQIDNO: 125); CCDS56186.1, Del, A_7, ARAEVVVMNEREAKVRNGSEVETEKGKRAKAVKESEVEAKRGDGAAQEVEIEDLEAATEV LTDDVPEAKVHSEKTRAL, (SEQIDNO: 126); CCDS3505.1, Del, A_7, HQKQKDGWCLQNLVASYLLVQDITVKSCLEKLWKYPDTMSKLLILYQGLKETQWLFVSLN MFILQDQTVCSKTKWFIQ, (SEQIDNO: 127); CCDS5659.1, Ins, A_7, NTRGHVLSSLGRRAAREHGQPATGVPGSESRHRHHDPPAQPRPVRRRGKQGEHPGGRGKQP GQSVPHGPRLAPLAV, (SEQIDNO: 130); CCDS47630.1, Del, A_7, QPLKTKSYSQRQKHQPQKNRNMTYLNLKYKLLKKSLKAEWLQRQCKKGNNHRPRWKVYH LAHLRVYLKKMIRQPKQ, (SEQIDNO:131); CCDS2150.1, Del, A_7, HKQKLSKKWLHYNILTHNFWSNLKLKDLHRNKLSKFSLLPHLAPLSSDPSLFQDKVQCLRFL KVFNSPIHLSRCQ, (SEQIDNO:132); CCDS46152.1, Del, A_7, PWSGSTSMNVTGTLAVLCPTLSWAMNTISEFTPRTSVGSVTHLVSPRTRPASSRQESPSNRSSI RSMTSGWLPSS, (SEQIDNO:134); CCDS56590.1, Ins, A_7, TPETISDQQPEISQARNERGHCECAAECPGENQGTLDAPWKIICPACGMQVGESTSLPGDHATI FRKAGLSDAVK, (SEQIDNO:136); CCDS45285.1, Del, A_7, CSQRRWIHLMRYATAFLCNSVDNMISQCLQFECFLKCRKLVLTPMPLLMVIIIRLFWKVPGLQ EVVVAIFFGQK, (SEQIDNO:137); CCDS3632.1, Del, A_7, IMTSISPYSPRSKSSPARWRGVHGPWRGRRRRKRSTRMTRRRGKTRRRRRLRQRLPRGPNMT MPTPRCLMTPRS, (SEQIDNO:139); CCDS58199.1, Del, A_7, AASLPRKPRCCSTNNHWRAPNTAVHLHSFPSLTLDRYHLILIQQQEACSQPTRNTKTGRDTQV ENLPPPSFHI, (SEQIDNO:141); CCDS7417.1, Del, A_7, CVLFRVSTAETLKGVSLHHFILQLGITECPWWNICYSMELMCMLKIKEALYLCTMHVLMDIM KLQNFLLNMEQ, (SEQIDNO:142); CCDS1479.1, Del, A_7, FTTKQQLCLNAIRVFTSMAATQLSVTVTVLGIPQFQSVLKVLGLLTSLQSQIIQDILNLRKEYL TVWMFGSLL, (SEQIDNO:143); CCDS34723.1, Del, A_7, AGRKNNTFQNVKRHFVKDLGSHQELRVQLQRLILHLMVQILDGRQRSKHGWIDMKKQITTS TVRVFRGRHKE, (SEQIDNO:145); CCDS2314.1, Ins, A_7, GLQCSIRCSRSCTISVCLNRSCDWNNSDCCHLCGGPLHHKEMPQKQQNSQTEAKYRALQFRQ YNKSVHEVNL, (SEQIDNO:146); CCDS4950.1, Ins, A_7, NRLVPGVARFLCQTHLQLGGQECAAAPEQLGHAQYQQPQLPHPQEVLPGCGGVRPRLSRRG GAQDLPETCHL, (SEQIDNO:147); CCDS47339.1, Del, A_7, QQPIHMSSWCCTKIKFTTSRSVIRRKVKFTCWELDSEGKRTFCLCQILLTTSGKCHFCSLMGK TEVPDTSAH, (SEQIDNO:148); CCDS3356.1, Del, A_7, ETTQRGYRTLQKMLKLRTHPGKDSGRTSTVFGRETQSLTKRPEQALTRPWRQPPRSRARQQR KICLMHVNH, (SEQIDNO:149); CCDS3444.1, Ins, A_7, ILTPFSRRAALRRAAVHATSDGSCPLAAWNTEPGDPARHPAGGGAARPLYVHKSPPAASHGL LIGGDGKFQ, (SEQIDNO: 150); CCDS32866.1, Ins, A_7, NRGPTCPQLQRRGPGEGQDQGQGPRAACGEAPCGQEGPAGQQPPAQGRAAGRDQEEAIFGH SCGHLREREA, (SEQIDNO:151); CCDS30975.1, Del, A_7, PPRALPAVQRRPHLNLPRCPQQSLPEKWQYQSPPEKSRCPSRRPLPHRPLQPPQPLSLHPQVK MSPVPHCC, (SEQIDNO:152); CCDS763.1, Ins, A_7, SRPRFHSAADLHYSVSLLPTGGRPIFQLFFIPQTDNEIFTRKCCSVYSSPWHSFHYCTDHSLEFT YEVNWK, (SEQIDNO:153); CCDS43145.1, Ins, A_7, AERSGEQCRSLTVYSEKNKRRQNESSPFPCKPEQLVVWEPRGAQRRTARTSSSSWGAVRGAG RPGPAGAG, (SEQIDNO:154); CCDS58907.1, Del, A_7, ILPKSTWDLPRRRLCQTQANGFMKKSHIKWICSMKMVLVLVFMKMYAKQLVTSASGLLLLE FRTSMKSSS, (SEQIDNO:155); CCDS47741.1, Ins, A_7, TDGLHGRNGGARSHQGEWTVPPASEESLPFPPQYRQWTHRGRLGRKKQETSEAPVHPEVVV CAVSVADHE, (SEQIDNO:156); CCDS33489.1, Del, A_7, GHASCGRISWMGRRTGSCGFPWPARRGSPRPPTFFRFDRMCTQKSQILTFTTSWELLPEKTAT PRSARA, (SEQIDNO:158); CCDS14104.1, Del, A_7, HRPASSTGSAAWTWTGTAPCPCSSSSTSTRSSAEGWTAWPSRPCPSRTASARCWTWSSRGLK GRSRCRT, (SEQIDNO:159); CCDS 1764.1, Del, A_7, IRTAPDGMEMKWPKEQRDAKGAEMPAARGSKSWPLQLQKLAASCSSPRGTGQAPEGLLGG PQRERELLS, (SEQIDNO:160); CCDS33097.1, Del, A_7, LTNAMSVGRSSLRIHTLQDIGEFILERNLTSVMSVAKPLVKLQNLQGIREFIPERNHMSVGNPL VSVQA, (SEQIDNO:161); CCDS33425.1, Del, A_7, AGVRKKKEGPGAEAGGHQRKTHQRKRSTKEGLSSLTPSCPCTPLMCWTCPWTQTNPRTACA TRSPMGR, (SEQIDNO:163); CCDS31448.1, Del, A_7, FKNGWKMRNINLVQLQRAMVMPMENLQVFTVEIPIQNQPFIIQFRGNQFICHLPKKLRIYQEG RVKDL, (SEQIDNO:164); CCDS9890.1, Del, A_7, GSISIIRKQRGSMGRRVAAGLRQTPILKRTNLPEALEAVKRNLRNRIKEIMLQLILDIALFGLRT FRL, (SEQIDNO:165); CCDS9273.1, Del, A_7, DLEQDHVLPSTIRHPSPGLDHTQKQSILFPVPIGQCGGRGPAPGPLGGEPTPLRDGGKRGVCPL PTA, (SEQIDNO:167); CCDS47641.1, Del, A_7, DRERKKKRRNLVGIHLLLHQALILPAVLLILKMRIRNKENGERKRRTVHINLLKAPCQKLNQT VRIV, (SEQIDNO:168); CCDS8326.1, Del, A_7, LQNLIQWKICSSTSIQSHGKKILRICIWTPLGIEAGHTMTGSQKLTWIGSMMMPSVTVALPGIT RSI, (SEQIDNO: 172); CCDS3920.1, Del, A_7, LTRMLSLLTLMKQLCEQPKTSSPSSLKNVVVSKVKKITDHCLKILFKTFFQQSISLNGQLLNYS LVC, (SEQIDNO:173); CCDS3204.1, Del, A_7, GNWSIMLKRPMVKMMKILMIKIMMEKSKKFEMTTAPKAERNTTRRKKRRKKTGLQLLRNHI EELKC, (SEQIDNO:176); CCDS607.1, Del, A_7, HKRISSVNKLCTRQYWKRMLKILCTSTLCLRLDTSQSVIPKRKKTFIRFPPAKRRKVFMMCQK VNL, (SEQIDNO:177); CCDS2614.1, Ins, A_7, NKAHAAQWLPEVPGPQRQGAGSAADVQQILLCRDRSQCFLQEPQSHRGKSCPTGHQSHQPQ CQAAQ, (SEQIDNO:178); CCDS10987.1, Del, A_7, ASPSLRLSTVRSTSRLSTSSWWPWSLWSRTTSWFCSRRALTTLTHSCSSAMPAAFKRIRRWLE TS, (SEQIDNO:179); CCDS35049.1, Del, A_7, ERKEEKRRNSNRVKQMSSGILLLRKFPRNPPMLFVTLNMIFHCQTQYRRIHEKKIGKSGDKE MSS, (SEQIDNO:180); CCDS2702.1, Del, A_7, GRNQLIQKARIPLPIPPLLQNHLQKVNLNMREAEGGNIRGGQKLNVLKRGERKQAVQKSQGI NMQ, (SEQIDNO:181); CCDS10239.1, Del, A_7, NMIHLILPGMAELGFARADYQVAPPCLIKMEYLLIQLAANSWREPMEFSRETKISNPSLPFQST C, (SEQIDNO:182); CCDS31823.1, Ins, A_7, SLFNLLLTCDCCLYLLWKLHLHVCENISKGRSCFDKRSSYTQYLCRSYAESIYLHSKKPAGET SI, (SEQIDNO: 183); CCDS1618.1, Ins, A_7, GDKQRTLFLGITDSHQQYLPTYTLHVTTIFPPLEKTMELDSSLPEYNMFPRKGRGLPRTNFQS H, (SEQIDNO:184); CCDS6367.2, Del, A_7, KTTMKQLQCWSRRFHLNLKVRKKMKLTLAVLPTLRNLHCYPQLCLQGRLAARNANMNLEI LLGV, (SEQIDNO: 185); CCDS32009.1, Ins, A_7, NHLGPGGLSAGVAEGWGGPLQAAGAPAPRDHRESLPRAPERERVPEQHPRVPARLRGFPAA GDV, (SEQIDNO:186); CCDS6045.1, Del, A_7, QGPYADRRKMNVIFLRCALATRLPVLRTSSGSMDFLARTQKATVSWGNVQLVRISALNYLM MRQ, (SEQIDNO:187); CCDS2061.1, Del, A_7, SFGHILQGVKRNLSYFVIYQSHRNHISATEIDSHQNKSLSTCPSWVVTTYLMSNGTNNLRMEI H, (SEQIDNO:188); CCDS3411.2, Del, A_7, FHLSQPLILVLTKKELQRTWLIKKNLQLTLEVKDWKQPQSLKSSATSPVQSKKLKITQKSIII, (SEQIDNO:190); CCDS11213.1, Ins, A_7, ILPSSSAHVFWFCLWKLLWQLSILSFCFSTIPSRYAAYSGRKLIFPTIGSSQLSTSFQRFCQY, (SEQIDNO:191); CCDS34049.1, Del, A_7, ILTSPAIKSSASHGRDALRAPVFVNYRISAQRMALQCVQLTGEASQHTVNKRVWNVFIQGQS F, (SEQIDNO:192); CCDS3210.1, Del, A_7, KKRKKKMVKRKNPKRRKLQELLKRRKLRKNSNLSHMMIRFFWMEMRCMYGSMTQFTLKH LSWD, (SEQIDNO:194); CCDS32165.1, Del, A_7, MMTTFPHPRTKTLILPKSLWMTFSPMMKTTQGKESSSSTAVQPRLPSRAATRSPRGCGRSTT W, (SEQIDNO:195); CCDS41997.1, Ins, A_7, NSAEPCEWHVITTVPSEHIGQGRCCGALYPGSQCRKLSCAGGPYKAPVSQYCLKCCSWKIQIR , (SEQIDNO:196); CCDS41699.1, Del, A_7, SWKWLTKSRMKNEWYNLKRVLVPFLAPGARGLLCCLQRHLREVVAFWENSWASLLPHSSS FFN, (SEQIDNO:197); CCDS2751.1, Del, A_7, TFGEELSITNRQTGRLSWMEFFTMPPRTWFMRQLQRMWFLRPSMAIMAPSCVMGRRELARH TP, (SEQIDNO:198); CCDS9464.1, Del, A_7, ASQVCSVSLPRLPSFTIYFCMAIPSLDFSLMSSLTFIMRLVCTWKTMACMKSFRGLFWGCSW, (SEQIDNO:200); CCDS 11144.1, Ins, A_7, NASTYSGPGRTARRGGRGSAKAAAGKGDSRGQDQEDGRGDSASRGPKFQVHQRKETHGRS HC, (SEQIDNO:201); CCDS31499.1, Ins, A_7, NIICHVFTHIHCDNGGKPPHLGDYYWQPLLGLPNVLLPCLLVTYGCHIFHCHVTQIDDRLTL, (SEQIDNO:202); CCDS45246.1, Del, A_7, PSPSSSFRTEQTATRSWVWTRSQRRAKAKQALISKSSPHSHSLAALIRKVRNSNNSGTFSSR, (SEQIDNO:203); CCDS1870.1, Ins, A_7, WKRGSNNTQREVLEDIYPSRGILEPRAREAEPAVQPHPEHADQPDGGGGQALQYKPSELLRT, (SEQIDNO:205); CCDS42041.1, Del, A_7, KLPQRNKNLCLRKLQDLTWLKEKQKHQILKVNKIKLLLQKKKMDVMQIHLKAHQQQKVKK A, (SEQIDNO:206); CCDS44603.1, Del, A_7, LHHQAIVKSLERTRMTLPLILQCSPHPACTPLWTNTCCPERSGSVAQSHLLVSVHQSLPAF, (SEQIDNO:208); CCDS2430.2, Del, A_7, LITPSFAASTPKGRSSQAALPGSCGSSHLSRPRPTRWTCPYTSWDGTRPSSTSREWATTPI, (SEQIDNO:209); CCDS389.1, Ins, A_7, NPPEGANCLSEERVYSAILLHTVPHWIYSSTCPPTASSHQENLFKLLKRHFKSKGCDQCPV, (SEQIDNO:210); CCDS58232.1, Ins, A_7, NSYWGRGLQCPGWRGGYFGPSHCCLCEAVSSCSSLRPNRSANPNKIFVYLIGSSRERSAVP, (SEQIDNO:211); CCDS14104.1, Ins, A_7, NTDQHRVLVPLHGPGRGRRPVHVRARVLLRGAVPKAGQHGHRGPALPGLPLPDAGPGQAE D, (SEQIDNO:212); CCDS1180.3, Del, A_7, QPKKRLDPKGVRCPRNRLSLPLLQEPACPQPWAVPHLPRPHCQLHPTVLQLRTRKQWPNVR, (SEQIDNO:213); CCDS10159.1, Del, A_7, SERCLLVCLLLYMHHPQIQMISTVNLLVIHLLSHQPVCSLALSLCKMGPTILLTFGVHQMG, (SEQIDNO:215); CCDS46348.1, Del, A_7, CRFIGPMLRCSLPLLLNCHILVLEKEVSLGLDLQLTLSTLFTTEKKLLKPTFVIPCKSLK, (SEQIDNO:216); CCDS53826.1, Del, A_7, DKQPSNWKPTGVGPWLGRQSKGESGPCGLSESSLKDSSVATNPSVLTTRNLSCLCGRITS, (SEQIDNO:217); CCDS58928.1, Ins, A_7, NRPQDLGIYRRAAWKMAVHRDTINLFSSLSFAKYSPGDLYGKQSCCDVQLPRPCNSKESG, (SEQIDNO:218); CCDS2872.1, Del, A_7, CSINGSSKRTTRTRQTMQEAPDFLFLVKDQLLSRIAPSKLSCLGKLQSMLLDRPRLPKL, (SEQIDNO:219); CCDS1591.2, Ins, A_7, GSKGRRKETGPRNRYFQEKEEERTGGQEIQKEVRSQYFVKWTDWTGWKIQKWNTDSEPS, (SEQIDNO:221); CCDS9287.1, Del, A_7, KNLSNPRWNLNQVYLMILPFQRMTVKGYIPTAEKRNKTLKVQEREQGRTWGWSMALRSP, (SEQIDNO:222); CCDS46500.1, Ins, A_7, NQASCTNQKTQEEEKAWTSSTPKTHSTCSPPREPQHARQRLTASGGLSHPEPIHARAEC, (SEQIDNO:224); CCDS35493.1, Del, A_7, RRKLKKKMLQEKEKHYKLLKNGKRKRWNILKRKIERRENMKEQRNRKRRKLLPRKRKII, (SEQIDNO:225); CCDS31195.1, Del, A_7, SPSQAVSLGRSTHLSQIQPRPVNTYWTSAQPSMGLMHRWALGSLPMFYLTMISLCKWPI, (SEQIDNO:226); CCDS58290.1, Del, A_7, DLEQDHVLPSTIRHPSPGLDHTQKQSILFPVPIGQCGGRESQPPQGRCARSPVRAARQ, (SEQIDNO:229); CCDS5135.1, Del, A_7, EGRGKEKNLIKEKVKKQYLTAKVWNPAKKSQSNEKTNSSRRSEKSKINRNRYQSALYT, (SEQIDNO:230); CCDS 14649.1, Ins, A_7, NGDYVSIYCNFRGECSPGSHFAKRLCTVRKSMGNPPRGFTDRFSKTGRSAEPHSGRDT, (SEQIDNO:233); CCDS1235.1, Ins, A_7, NSGEFKTFNSAQAFIASSSTQDSNKLQCSSKNHHYSDSTNAYAIGKAATNYQFTTCTH, (SEQIDNO:234); CCDS54068.1, Del, A_7, PSLNLISHQSSVGCSKGIWRAPEMTLFSTPTFAASSPWASMSWVSSGCSLPLWSSSCC, (SEQIDNO:235); CCDS43472.1, Del, A_7, QEKLQREKENVSLFVVLASRNATAGNMYIITAMLTPAVRYGNTKSQSLTKSLVNSIKN, (SEQIDNO:236); CCDS3656.1, Ins, A_7, RAKFMYLFEGAQTVRNRDDCLDDNALRCPPQFHRWPGDWGFLHLVSPSGTQYFHSNPM, (SEQIDNO:237); CCDS7171.1, Del, A_7, RSCGTLFSGLKMSSTDGVMGRRCLLMNSLTKRKPTWKLSQWIKILLLPMINQQPQLEL, (SEQIDNO:238); CCDS47865.1, Del, A_7, TRRNRKNPRRKKSPRNPRPRKPLRFPKSQRKRKQKLPRQNPNPAKSQVIRNLTQKQVL, (SEQIDNO:239); CCDS53348.1, Del, A_7, EFSLSFYLYIMSMVFSKLVLRLGDHLFFCLKTTLENLPQKTIPSSELLTSLTGSGIG, (SEQIDNO:241); CCDS1829.1, Del, A_7, QDLIHMERISSPLVEFVKVLCTNQVLITARAVPTKKASVRCVEKRFWIPKTTSKHLS, (SEQIDNO:243); CCDS58960.1, Del, A_7, SHWGNHQDSYILGGVYFGISTVQLQRDVKHVNTQVKQKPSMITLLCHLGTLEVQGPH, (SEQIDNO:244); CCDS6435.1, Del, A_7, YLTACRGNIQITAMERSRTNVQSVGKSSGSARSLISIRESTRERNPLNALSVEKPSA, (SEQIDNO:245); CCDS56402.1, Del, A_7, FCQGKRKLNKRKKKLHSNFKKNCDGEEHSYMLGMSCLGSGDLHGNGHYMGNPLQLS, (SEQIDNO:247); CCDS4259.1, Del, A_7, LFTRQLISVRCLYPQLMVISILLWRSQLLALILKQARKRIRTKRKSLSGTTELLCL, (SEQIDNO:248); CCDS31658.1, Del, A_7, LLRRNEKNRKKKNTKLENKYFNKENRSLKKLLKNSSVPMFLFHSGGKQFPENQFLH, (SEQIDNO:249); CCDS32261.1, Del, A_7, LSTDWRLQPSRPQRQPHRPSPPPRMQLFPTRTLRPSSSWSRVARQWLITSLSWSRE, (SEQIDNO:250); CCDS12121.1, Del, A_7, SAGKSGTSSRRKKAGRRRRNISMAWRLGCQLALLRIRSYRGKSCISRSKTCPSWSN, (SEQIDNO:251); CCDS13571.1, Del, A_7, WQLKTSPQRLLEEVMPKKGPGPGLWVCIMAADCSAAHLSSAVTGWCPPHTACMGET, (SEQIDNO:253); CCDS3352.1, Ins, A_7, YRKLRLPVFATRSYRKIVCSSCVTERKCATQEPGQSYEGDFSDTSAGSTDAQDSKS, (SEQIDNO:254); CCDS10539.1, Del, A_7, APFSPKVWRTARGASLSCQTTPPITLSSTPTGMTNAWLLGDAPRTLTNTRCHPRR, (SEQIDNO:255); CCDS32595.1, Del, A_7, EPSPKSWLPSSLAPPSPMSTFPFNLEIHHLLPWDSILSLWITSTSGHKASISGAS, (SEQIDNO:257); CCDS34645.2, Del, A_7, FILERISTNVKSVEKPLTGPQTFLNLRKFILEKNPTNVKYVEKPFTNPQSLLNIR, (SEQIDNO:258); CCDS46227.1, Del, A_7, GKTRCLIFIDLQQEEAILGERSMPFIVVTQLLLMRNALKEGNQRAWQEQEIDVCL, (SEQIDNO:259); CCDS42127.1, Del, A_7, GPRTPQPSRSRTLGSVRPGRPGMLGTLAQWGRRMRNRQPWGRNGSGRAPENTMGR, (SEQIDNO:260); CCDS5043.1, Del, A_7, PQKMLKEGMALVYLREVNLIVMRKKKTLKMLRLQVVGKSPEVHPQFLKKSTVTLR, (SEQIDNO:263); CCDS30652.1, Ins, A_7, TTAAEAEAGISPAILCSRRQRQQGRVLRRPGQAGLPEPPEPVRWTVLTAPLLDTQ, (SEQIDNO:265); CCDS6160.1, Del, A_7, EKSVKEIKCFLTMNLNIAKVLLKTKVYFMYLTSLSKNPKIFMHRNLKQKWHLGI, (SEQIDNO:266); CCDS56403.1, Del, A_7, FCQGKRKLNKRKKKLHSNFKKNCDGEEHSYMLLMWSWIQTPLIPSSSCQRTGEV, (SEQIDNO:267); CCDS53435.1, Ins, A_7, IRGKCCSKQGETKSQVIAQRSHSSSSQNTLWGSSHYREGPSRGGSGWSGSCPQG, (SEQIDNO:268); CCDS6490.1, Del, A_7, LKDLIWFRSQVIMLDWDPCLPSFTPVPTSRLSAGLKKLSKKANRPFLSSILLFT, (SEQIDNO:269); CCDS 10932.1, Del, A_7, MFIVFSMSVTLRLNKMKKPKSLLLELDGKRQSKGGEGLLQLPASGRGRYQKRRG, (SEQIDNO:270); CCDS42801.1, Del, A_7, SVSRKAGLVSSTKPLVQLGFTLKRYRSFLNIEFLQTPKLTRKLSEWSRRKLLTC, (SEQIDNO:271); CCDS3158.2, Ins, A_7, TCFCKNGLNLHLVLFVLHLPAKYDLEQQGSNTIVCEKVCFLKGASGAEMASNGK, (SEQIDNO:272); CCDS2800.1, Ins, A_7, EARSPGDWVPGPPASGVSLTACEPPASQVPSGPLLTSLTPVPSPAPGHLLCFQ, (SEQIDNO:274); CCDS 1179.1, Del, A_7, HLKKRLEPKGVRCPKSRLGLPALQEPARPQPWAVPHLPRPHHQLHPTLPQLRA, (SEQIDNO:277); CCDS7452.1, Del, A_7, KGTLVGQHLRAFYLTVSTRKLWRTQKSQVSMNAPLRKMLSRRAKAGGSSPGRL, (SEQIDNO:278); CCDS 1807.1, Del, A_7, KKNHKDREKKKRNLILMIKVHKFEHHLLPSPLHNLSKYTDKLQKARMLLPPKA, (SEQIDNO:279); CCDS2382.1, Ins, A_7, NLPSQQWNGVSGSPLLVENASQLHCRKGISQLANPKWAYCHKGTSYSNWTSNG, (SEQIDNO:280); CCDS58395.1, Del, A_7, IAVSCAGRKWDLLGLNAGVEMFTVVYTVTQMYTIALTITKPMLLRKSEKKIQ, (SEQIDNO:282); CCDS3411.2, Del, A_7, NQQTKVKRSQTAMRKEKERKKRRKRLKRNLITQKRVKIHRKLKMKNKQRKKK, (SEQIDNO:283); CCDS5768.1, Ins, A_7, NRWTANHYCMHRKPSVPSKKFLEWSLEVRMEVYNHSFNHSSGWHLENSGSLL, (SEQIDNO:284); CCDS4597.1, Ins, A_7, QQPYQAWPQELGEQRYSGADQRYRCFWLLQTQQESGFRGRQTQGQKGWRSQA, (SEQIDNO:285); CCDS4056.1, Del, A_7, SHWGNHQDSYILGGVYFGISTVQLQRDVKHVNTQVKQKPSMITVLQQLPVQC, (SEQIDNO:286); CCDS33971.1, Del, A_7, TRPYKIYHQPTHLWEQETTFQLDQITALSTAVKPITSTANRCVYIHTLLCLA, (SEQIDNO:287); CCDS10504.1, Del, A_7, AQILSSAARASDAFVIKRCLDPKRLSPSSDSSAVSGCNPSCTPRSRFWSFW, (SEQIDNO:288); CCDS47388.1, Ins, A_7, DSVQKEKERARVERNGMEHNEARTKHKSEAPGGTQMEKYPVCISGRETFSL, (SEQIDNO:290); CCDS 1482.1, Del, A_7, FTTKQQLCLNAIRVFTSMAATQLSVTVTVLGIPQFQSVLKCCLHLVQNLQL, (SEQIDNO:291); CCDS47683.1, Ins, A_7, KEATVFDFLLLLLLLLFLADIPLVSTVRASQKELCFECQFCFELLSGGPSL, (SEQIDNO:292); CCDS 1825.1, Ins, A_7, KQRHVHRAGLLHEDEVHGHQQRPYCQPQVSHLEGLALHGPGESLQQLPSSQ, (SEQIDNO:294); CCDS41813.1, Del, A_7, LRMQRFFIICANSSSYRIEAIIFFHFCGNLLHPSLNRGLRSIITWICFGIS, (SEQIDNO:295); CCDS43183.1, Ins, A_7, NLPYCQLSTTGNDLTDEKASRFFTFPIITNRGNKRRNNCKSTPHFHGTCTR, (SEQIDNO:296); CCDS628.2, Ins, A_7, NPREVQPLLGCGQSEQASCRSILYGSPNLSPNCNSRLLYYPTFHDGIRWEL, (SEQIDNO:297); CCDS53588.1, Ins, A_7, NTLQISAIRPSGHPNKHKATAQEKPQESRRRGRIFSIQETNTISRQSHAHA, (SEQIDNO:298); CCDS43350.1, Ins, A_7, PSRPQQQAIQTQISFEKPRYPSKRRVMEAHSKRTCGSIRIVRRTIKRDSIH, (SEQIDNO:299); CCDS47517.1, Del, A_7, REKKRHCDRHLIKMIDLSKGRMLKILDWLLRFTKTCRKPALLEPFLILRWL, (SEQIDNO:300); CCDS30956.1, Del, A_7, TMSTFGSPQILVVLMNLLNLQSLKGISLVCCVNQVVFHPQISSGRRKALQC, (SEQIDNO:301); CCDS9140.1, Del, A_7, DIKQTGTRWNLCWELWNTNLVHKGTSPRNVLLQTTPQPSRLMSTSMKSLI, (SEQIDNO:302); CCDS5747.1, Del, A_7, EIRCVQAVVILCSELSSVHEVLSSCVALFKGAREHICLRETYRLISTIAI, (SEQIDNO:303); CCDS7086.1, Ins, A_7, ETSRGREKEARRAGQTSARRRSSCSDERKRRIHSASSGSLGTTSFKKGTS, (SEQIDNO:305); CCDS33931.1, Del, A_7, FILERNPTNVKNVAKPLDGPQIGVNIRKFIVQINPTNVKNVTKPLNNFRS, (SEQIDNO:306); CCDS2311.1, Ins, A_7, KPNCDCRLVQEICATKEVPADKEFRLSNSVLYPGLEGSKNSAGTEASKAL, (SEQIDNO:307); CCDS5135.1, Del, A_7, LIKEKVKKQYLTAKVWNPAKKSQSNEKTNSSRRSEKSKINRNRYQSALYT, (SEQIDNO:308); CCDS55861.1, Del, A_7, MWECKIVPKMGSLYQQTLFGILKKMELFFTHTQDCVLVLIGHRRADLMYK, (SEQIDNO:309); CCDS46935.1, Ins, A_7, NQEPKEETESNRTTERTSSNWKTARKKSVGENSERNSPSPGAGRFGIGYL, (SEQIDNO:310); CCDS2644.1, Ins, A_7, NYRLCHAGHHVWLVHYGDLICGILCEAKSGGCPETPRILEIPAKQAEESR, (SEQIDNO:311); CCDS4441.1, Del, A_7, QIRKFILRRKDMNVENVGKPFTRVRTLSITKEFTLARNPMNVRNVARPSQ, (SEQIDNO:312); CCDS2387.2, Ins, A_7, RFRNAEVHQTSGDELVACSLISVNQGSTNSDRGDVRRHPASCLGAPAQHG, (SEQIDNO:313); CCDS44335.1, Del, A_7, RKSWKMKSPVDTVLSKENQICKVKTILAPAPHALSLPRPQPFFRAIDKDR, (SEQIDNO:314); CCDS47741.1, Del, A_7, RWATWEKWRCKVPPGRVDSPSRLRRKPTFPTSVQAVDTSRETGPEETGNF, (SEQIDNO:315); CCDS389.1, Del, A_7, SSRRGKLLIRGKGLLSYSAPHCASLDIQFHLPAHRLLSPRKLVQVAQKTF, (SEQIDNO:316); CCDS42161.1, Del, A_7, TLVVKNDFLATVAVMENCFFGDKHFISSQNSWLMNLLVLKTLILSSAMSH, (SEQIDNO:317); CCDS46543.1, Del, A_7, YRKSLFQKSRRWTSGLWTKGRSALTLRVAIMKRPKNPIRQIRKKERKQIE, (SEQIDNO:318); CCDS34238.1, Ins, A_7, CNPALPVGICSQCPEPVHLCGPASTEQSPCGRHPSPESPTVHRCGPSAG, (SEQIDNO:319); CCDS55520.1, Del, A_7, GNGSGGIKKSSKKTISFLQALIFLGSHTRQTKVMHLPTESRTRLETMMK, (SEQIDNO:321); CCDS 10525.1, Del, A_7, GRRSISLLRSHQRKLRACGNWRVPLTPCSHSLALLLTTTCSRRPLPWTR, (SEQIDNO:322); CCDS43378.1, Ins, A_7, KTGSVRKANRMPKDVNIQVRKKQKHETRRKSKYNEDFERAWREDLTIKR, (SEQIDNO:323); CCDS164.1, Ins, A_7, LQIKERKISKLQVSSGQICKSEKCGCCCQSQGGCSTGRGEGIWGGGSLP, (SEQIDNO:325); CCDS56486.1, Ins, A_7, NPLGSMSPQGQVCPALCHLFSHGGICPCAAGLGTHQRAGGRRAASREGV, (SEQIDNO:326); CCDS47270.1, Del, A_7, NYAKRCGNLKKHFINKMEGMPRKRIVFQCLRSTESTRKLKPSLGFLKFL, (SEQIDNO:327); CCDS387.2, Del, A_7, CFIRAKLHIIRQDLLSSSAPHDASPDILHLPACHLLPRKPAQTARKTF, (SEQIDNO:328); CCDS7816.1, Del, A_7, FSMLTTILVSQLRGLLLMLDCYVILCVRSVWIPDLYSIDHCLCLVLYL, (SEQIDNO:329); CCDS41302.1, Del, A_7, FSRRGKLLIRGKDLLNFSAPYHASLNTFHLPVHQFLLRELVQTAQKTF, (SEQIDNO:330); CCDS34490.1, Del, A_7, LLLAIVQAIMSKKFLLKELKILRHQKLSDALPMVPQDQKATIFQIYHF, (SEQIDNO:332); CCDS48164.1, Del, A_7, LMELNLLHQKRLKKRKNFSHKVSQTGLNEILTSLLKLMRNMEEMTLIT, (SEQIDNO:333); CCDS33489.1, Ins, A_7, RVMLLADGSAGWGDGLEAAASRGLHAEAPHGRRPSSDSIVCVRRRAKY, (SEQIDNO:334); CCDS33125.1, Del, A_7, SILGKSPMNVTDVEKPSFRSQTFIAIRKLIAERGPIDVVNVEKPSSGN, (SEQIDNO:335); CCDS34681.1, Ins, A_7, TTPRGLAICRTKSSISRSLFLGLPPVLARKSRARSRFQTNRKVCFILK, (SEQIDNO:337); CCDS8511.1, Del, A_7, ALRNMNTVMKLLRKMRALWACPLSYKPIMLWKKWKNLFVRYGKVGGE, (SEQIDNO:338); CCDS54379.1, Del, A_7, KICCFNSNWMMLATKLTIKKKQYLIFKPDVMLEYKTFKLSAESTVFY, (SEQIDNO:339); CCDS43121.1, Del, A_7, KKMTQQYPLSLKSQVKTIIRIQNCLKKLQLNLQKVILLNYPHLTVRS, (SEQIDNO:340); CCDS 14482.1, Del, A_7, LLQKDRFREEVKSPVKWSKFQSLKGSLIPSRLYMMKGLSTSSPQLKN, (SEQIDNO:341); CCDS8336.2, Del, A_7, QRKKRKKRARNRNMKKTMSHLIAHQALKMSGLRLFHPRLLTRKKPGK, (SEQIDNO:343); CCDS 14448.1, Del, A_7, VKTEKEMEKMEKRKEKMKKRKKTEKKQEMEKRMKMEKRREIKKRGKM, (SEQIDNO:344); CCDS34343.1, Del, A_7, YGLAMAKPNLVPKLSWQTSNSPTGFSVQNLSVENGGSLPRKSSLLHR, (SEQIDNO:345); CCDS59085.1, Del, A_7, YREHRREWEIQLVNESNSVMTEYARVTFSTVVLMMSFLELEWILENV, (SEQIDNO:346); CCDS54597.1, Del, A_7, EEAMETHLIPDMMMEEGHQETLPEEWVESIICVALVPLQWLVDEEG, (SEQIDNO:348); CCDS 14406.1, Ins, A_7, GDPPLPNICCSAPGPTGTQETGHTDAKTAESGDPLGSREPNTSQTE, (SEQIDNO:349); CCDS2335.1, Del, A_7, HLYQPSIKLFKIQIMSSWSLSAISCLSSIVAQRFDLRGSHCSSCQN, (SEQIDNO:350); CCDS31131.1, Del, A_7, HWNSAYLGRTLLVTCILYHRWIVTSMCQSQRWLTSTTSRSSALMWT, (SEQIDNO:351); CCDS55872.1, Ins, A _7, KQPTRKPYHCWHKINQEWISEWGPVGYERRGICGESLYSGENKVIA, (SEQIDNO:352); CCDS5046.1, Del, A_7, KSLVKKPNYWYLQSLFLSLCLPNTTSEQCLIDGWVLRQYVLSTFNI, (SEQIDNO:353); CCDS4999.1, Del, A_7, LAGVRGLPKGGLPFSGPGSRIPTFRRAGHEEAWGTLPWTMTMLFWS, (SEQIDNO:354); CCDS 10622.1, Del, A_7, SNSTEKLGRKLEVQKIYQTQKNPLKNWSQHLAKLNKKKSKERSDEK, (SEQIDNO:356); CCDS53389.1, Ins, A_7, TEPCLSHGSQHSGHQEFKTSVLLGTSIVDQKQSIEEVEDTPKPTLV, (SEQIDNO:357); CCDS58089.1, Ins, A_7, WSCDYGRAEARLFPLSRGSCGSVLPEKHVGPAGRPPLCSGKGAHQD, (SEQIDNO:360); CCDS55733.1, Del, A _7, AFLVLKREFTTLNQLRMPPMPPFYYVTREGSCGFVRRPTVVQGMN, (SEQIDNO:361); CCDS2291.1, Del, A_7, EKSYVSIIIWRKPKNQKLAAKWKQRIVTLKMLGNIYWNQFQKSSI, (SEQIDNO:362); CCDS7375.1, Del, A_7, ELKQLHHPPCSLPQKAMIIPRTVSLVLTMQKTWILQRTCLVCLKV, (SEQIDNO:363); CCDS513.1, Del, A_7, FRRNMMKGKRMPKSAVSWRSSSSRASFLRASLQGRDSVAEQMAMC, (SEQIDNO:364); CCDS14214.1, Ins, A_7, KPTSCTCCPLDKFSRRQKGESWRYCVICHLSGWIKPHCKSEGLCA, (SEQIDNO:365); CCDS7789.1, Ins, A_7, RKKYQQRYNLQERFALKNLKCREKSISATMGSGQLYRRKSSSHKD, (SEQIDNO:367); CCDS53388.1, Del, A_7, RVQIRTFFLPTHPALSFSWEPTLRTPRIQDISSAWHKYRGSKTEY, (SEQIDNO:368); CCDS58928.1, Del, A_7, STPRSWHIQKGCMENGCSQRYDQSFLVVIFCKIQPWRSLWQTELL, (SEQIDNO:369); CCDS 10071.1, Del, A_7, DEEMKNFPLKNPLVAITTRPKSLPSFLTMHLPLALRKSTYPLSS, (SEQIDNO:371); CCDS34021.1, Del, A_7, ENSMQVPSSQMTASSGSVFKKIAIIPMPQQYIELKKQGGSGMWP, (SEQIDNO:372); CCDS43313.1, Del, A_7, FMYVSTVRNSLTILDILKNIFENIQVKNLLNVQIVMNDLLEIAL, (SEQIDNO:373); CCDS32186.1, Del, A_7, GLVEAYQ SARIRKKHLILLFRVLTMHHLLNLPAPFAVKWCLDMT, (SEQIDNO:374); CCDS43350.1, Del, A 7, KPTPTTGDPDTDLVREAPISKQKKSYGSTFKKNLWIHPDCPKNN, (SEQIDNO:375); CCDS31669.1, Del, A_7, LLKRNMKSSWMFAKIFNQFSVTSAWKNSWIQLFGLRSDWLIRAV, (SEQIDNO:376); CCDS9405.1, Ins, A_7, LVCFTDSRSDYSNYCWKHTRHHGSVPREKAAECHQLFPDVTCHS, (SEQIDNO:377); CCDS447.1, Ins, A_7, NGGEENTWNLRLIFRDWEDPDGGRGEQLLLECQFPSNNSVSGTC, (SEQIDNO:378); CCDS 10024.2, Ins, A_7, NLFLPYKGREGRENAPSPRMSEQSSPFTGHKHISNPRWQSPCSR, (SEQIDNO:379); CCDS4541.1, Del, A_7, QHSTCLPLEHFPLHLGILQSLKPVSLEILLFILEKQHTVGQQLL, (SEQIDNO:380); CCDS1882.1, Del, A_7, RFTPQIVMRFHILLIVLLLRMKNQQQILCWTCGLVKLLFLVLPV, (SEQIDNO:381); CCDS46418.1, Del, A_7, RKRSLNYSVRQKMSLSRTPSQQIIQMAVYKAKIIGEHHKTFTTN, (SEQIDNO:382); CCDS55959.1, Ins, A_7, RPGHRKYLTLEHSTHLCQPCDDSARAIAHPKRSPILRTSGSCFS, (SEQIDNO:383); CCDS54776.1, Del, A_7, SPVILKAAVIQIFKREGTMIYLLSVGTANLLRTFLVKEKLLVLT, (SEQIDNO:384); CCDS 10308.1, Ins, A_7, WDPLLDREKLLGSPVGNERVLPHRARKEHVWPGCLRLLPHPSGV, (SEQIDNO:386); CCDS42354.1, Del, A_7, ALTPTLMTPTRWQQNLFSNSTTRPSQWDNSLSLASMKSFLAYW, (SEQIDNO:387); CCDS9867.1, Del, A_7, CRMRWPFRWILKEKLNMMQLLDKDSQKTRSFIANTLTWFQRRL, (SEQIDNO:388); CCDS7258.1, Del, A_7, CSPSCPMRKKVPQETRRCPRLPGVASLRLQRSLLEIRKQRQHL, (SEQIDNO:389); CCDS6448.2, Del, A_7, DFIKIVILVLQRHFQQGKLLKKVDLKSHLGTLRKVSQNLGKRV, (SEQIDNO:390); CCDS44402.1, Ins, A_7, DRRSYRDLGSLCSGTNSWCQGRSGVHWSSPAWRCLFPRRLHQM, (SEQIDNO:391); CCDS14435.1, Del, A_7, ELENKMKKTQEMRKQKIKSILNQIQILKNLRSQDTDIGFCGTN, (SEQIDNO:393); CCDS5952.1, Del, A_7, GKRTILEMTQILKVFIVKNGSMSIKKAQRKGMAIAPWEKPLIG, (SEQIDNO:394); CCDS7430.1, Del, A_7, GQLKMTYCSKNHFRKKNMERWPINKLQQNCWIGKKQEIEGFIS, (SEQIDNO:395); CCDS47878.2, Del, A_7, GSCKSGSRIHERGRGKASSGRWVQHSLINGVRFAEPCLKQSRP, (SEQIDNO:396); CCDS2867.1, Del, A_7, HAPLLPPKVRYPHRCLQIDKGEKGSFAPFHFLTMKINLLHQRK, (SEQIDNO:397); CCDS2895.1, Ins, A_7, HLWNDKPPCSREDGVDHPSDCGISLDLRVPHPSHCCARLLERV, (SEQIDNO:398); CCDS46776.1, Del, A_7, KSQKLPTKMILQRSCLLRECIRRRHNLNTFFFVLIHILGQWSH, (SEQIDNO:399); CCDS31976.1, Ins, A_7, LCCQYSGCVHSHDRPDPCAGERTRPPSDNRLLRRNVGSETEHQ, (SEQIDNO:400); CCDS 1660.1, Del, A_7, LKEERIHMKTFSSHNPKSLMKEKILWDRTVFLQQTMFAIRIKK, (SEQIDNO:401); CCDS45430.1, Del, A_7, LLHQLILHQAPFQELARVLLVVLKRQSETLKLGKRCKRETPMQ, (SEQIDNO:402); CCDS31206.1, Ins, A_7, NLCCFKWTCTVSRYKIWCNIHFITWRKSAECKSRGSVHPDKWV, (SEQIDNO:403); CCDS30621.1, Ins, A_7, NPAEMRKEWVDGTDLWERVQWHLPALFSLLSQPRSSVSEERAR, (SEQIDNO:404); CCDS53942.1, Del, A_7, RHFLEVEGKLNKEIIHRLDSDAEDTVLPQKEDRICKRDQIISI, (SEQIDNO:406); CCDS9238.1, Del, A_7, RSHCLRTRNLKPCWFSKIGQTRALAMNAKRRNNRSILCLGRKV, (SEQIDNO:407); CCDS9190.1, Del, A_7, SIRTSPWDRNGSISSQRQRPRNSKFPQCESPTANQAGEAPHSV, (SEQIDNO:408); CCDS47860.1, Del, A_7, TSANIVIAGSTPERMSGDTWWCTLEERTSSVSIVHRDLGERIT, (SEQIDNO:410); CCDS1332.1, Ins, A_7, YWHKQDSKATRHKWFFRFFHTAQIGRILYLSYPVRAVSRREPV, (SEQIDNO:412); CCDS4814.1, Ins, A _7, CKSRLHQDPTACRRGVGVGFGAGIRTRGWAGSGPRPGSGAGA, (SEQIDNO:414); CCDS4413.1, Del, A_7, CRVNAVEELHSRRMSVRIVKNWVSCCYVRLSAVGLSTWSALD, (SEQIDNO:415); CCDS33361.1, Del, A 7, EIQLTMNDSKHKLESPALKQVSPASPPTQQPQTPQDSRQVLA, (SEQIDNO:416); CCDS14466.1, Del, A_7, ERKVTLMINLYHLSPHLNQIMKRMCKQKRREGVKSENKQRKK, (SEQIDNO:417); CCDS42016.1, Del, A _7, GKKWLSRNVWKLLVCQTKIIPLRRTQEDTERLPFYMEALLTL, (SEQIDNO:418); CCDS788.1, Del, A_7, GNIVPAKKNLESVVEAKNVPTNEITVIVRTSQTNMIVEGAKV, (SEQIDNO:419); CCDS12635.1, Del, A_7, HSNVRIVERSLYTGHTVKTNKKTTVEKIHPNVKTVGSATRGA, (SEQIDNO:420); CCDS10021.1, Ins, A_7, KFPACGQRCGGARQSGIPSGAVFCLCLPAQGLAIRRGCGPLQ, (SEQIDNO:421); CCDS46549.1, Del, A_7, KRRRRKRKNNPRKPKNKRKEQRRKIRKKTKNGKCHQVFFFLQ, (SEQIDNO:422); CCDS34918.1, Del, A_7, NQVLHLISGMQSALVMLEIMKASRLKLQMHISSRSILRKQLN, (SEQIDNO:424); CCDS45984.2, Del, A_7, PMNVSSVGKHSVFPVLFVDMKESTLQKNPMNVSSVGKHYLIL, (SEQIDNO:426); CCDS45998.1, Del, A_7, RKRRRRRRKTTVGKTALSQCLPIAPCSSCPRPTPFAACAITS, (SEQIDNO:428); CCDS2482.1, Del, A _7, RRRKWKWMRQRKRRKKRRKKNLSQTSSYWITQPELCLPSLRS, (SEQIDNO:429); CCDS34545.1, Del, A_7, SETQKYLKRMTKVSTTPMKSQSQTSLWIKRLTQRRLIVSKPP, (SEQIDNO:430); CCDS43426.1, Del, A_7, SLIKGEGQCLSVKLVETGIGNWKILKIIRNFTVLSYMDQKQR, (SEQIDNO:431); CCDS8518.1, Ins, A_7, TPPAFPESPAAVPPTTTGGPQTQLCIYTASHHSHSTGAQQAH, (SEQIDNO:432); CCDS7789.1, Ins, A_7, YQQRYNLQERFALKNLKCREKSISATMGSGQLYRRKSSSHKD, (SEQIDNO:433); CCDS32554.1, Ins, A_7, GEEDKAAEKGGGRWGAKASGTEVISNSASDLGPGGCGACVL, (SEQIDNO:434); CCDS53776.1, Del, A_7, GYRNLRKAIEKKEVWLVKRIIQTLTKRRKKKRVFRKEKTMN, (SEQIDNO:435); CCDS3920.1, Del, A_7, KGDLKHQNTGMTIGGILESHLQRKNLKCLNINKILNLTHLG, (SEQIDNO:436); CCDS45712.1, Ins, A_7, KQGALPCRSHHQLPGPLYLPGGPPGGLCPLSAAQARLHPLG, (SEQIDNO:437); CCDS47996.1, Ins, A_7, LRQVLGCGPPHIWGLRGGIKSSEPRDQRGGAVGGSWRTSKS, (SEQIDNO:438); CCDS748.1, Del, A_7, LTWNWSPLEIWSSWQRQLELTLEFRSSCHCSLCLLMLFLMI, (SEQIDNO:439); CCDS47986.1, Del, A_7, MTSKSIRAEPRGEGKVGHSKVSQTGKVSREKRKRKGSCFLF, (SEQIDNO:441); CCDS 10525.1, Ins, A_7, NACFPEVDSGSPSRRSKRRFQWWDPGSGKVADLAVPKALCS, (SEQIDNO:442); CCDS55691.1, Ins, A_7, NEATQELPADKWIQACPYGPELYQTHPITRSNGGQVGRKCT, (SEQIDNO:443); CCDS 1177.1, Ins, A_7, NKPGRSGSCGTCTHRKKQTDIGSKREDSCSSEKKNSKQRKD, (SEQIDNO:444); CCDS34777.1, Del, A_7, NLSSSWFKTMRADTAFSTTRPIVRMSRSPRCWSSFARLSLW, (SEQIDNO:445); CCDS58039.1, Ins, A_7, NNVSCYSGYTDTVLPCEGFKHQLEGEIQWKENHHHIRLFGI, (SEQIDNO:446); CCDS 14397.1, Ins, A_7, NPGTKFLYLPWPGLHSRILGLPAFQSAHYHCCWGTPSNSQD, (SEQIDNO:447); CCDS2702.1, Del, A_7, RKVIRALIQNTVIEVQKAHQGQGADLLGVDLIPDHIQDHVV, (SEQIDNO:449); CCDS35457.1, Del, A_7, RSIKWHCTISIQVFLRQWKCYHPKLEFGGWNALLASIYMLG, (SEQIDNO:450); CCDS9081.1, Ins, A_7, SSSPSTRSTSTTPELPSASPNSSSRWTESCCEQENQHAHIY, (SEQIDNO:452); CCDS 10932.1, Del, A_7, VCKSRSLFGATKTGPSPALIGARPLGIPVEGPTEGCPSQAP, (SEQIDNO:453); CCDS3883.1, Del, A_7, VLLLWIRGTEISLCGWRSTGPLWKKPWGFLQIPTNRVKVSS, (SEQIDNO:454); CCDS4289.1, Del, A_7, WTAAFTRSIQWWPSPAPSHTYQFVVLTTSPMGMNVTCVPRA, (SEQIDNO:455); CCDS43229.1, Del, A_7, ARAKALAWTTRIQTRASTMTWKLRGTHHTQRPICAKYFLI, (SEQIDNO:456); CCDS2716.1, Del, A_7, FTNVRSVGKHVVLIQRLWTIREFTLERSLMNVMSVEKLSS, (SEQIDNO:457); CCDS43658.1, Del, A_7, GGTGTDMTTQKLMMVPNQCKLELELSLRNYALESSQVPMK, (SEQIDNO:458); CCDS54415.1, Ins, A_7, HGGLFYQTEMGKHTRELLPGNPQHLPICLLGQKSLGKVCE, (SEQIDNO:460); CCDS33370.1, Del, A_7, IRLKGQAQLSMVKSSVPVYQRMATIQKSSQMEWKNTKPLV, (SEQIDNO:461); CCDS4999.1, Ins, A_7, IWPGSEDCRREAFLSVDPGQEYPHSEGLGTRRHGGRYLGL, (SEQIDNO:462); CCDS42761.1, Del, A 7, LKKGEKRKEKRKNREKLRRKLRGEKLEKKCWIIKENKKKN, (SEQIDNO:463); CCDS 13692.1, Del, A_7, LLRTGQFRGFGLILLHQESHSHRRASSPCRKELLSPSPRP, (SEQIDNO:464); CCDS2981.1, Del, A_7, LMSALSATRLSPPNRTTSSTCSYTQVRSPTNAASVGAPSP, (SEQIDNO:465); CCDS46164.1, Del, A 7, MREFILGRNRTDVMNVVRTSLEIQTWQIITESILERNRTD, (SEQIDNO:466); CCDS 1691.1, Ins, A_7, NDGQRHEAPPQELIFSYLIFNTNGCKTGICYGRGTTKAES, (SEQIDNO:467); CCDS 1290.1, Del, A_7, RESSEGIGQPSIAASCRLWSVSLSGHTILMLLCEKTLPAG, (SEQIDNO:468); CCDS31906.1, Del, A_7, SRIYPRCLRMPWSCLRCMPGNRGAEKTTLTLLKASEPYWS, (SEQIDNO:469); CCDS59076.1, Del, A_7, TTTTSLKEEEGIILITESRSLALLFQSLMILICAGTKEPF, (SEQIDNO:470); CCDS3843.1, Del, A_7, WCLARALKRICLQRIKKKTFLWRKVIILRIPRHFIQWKKN, (SEQIDNO:472); CCDS 10550.1, Del, A_7, YVQDQQKQIMKDHIIYVKMLLLRRNVDIQATARLLIAKRR, (SEQIDNO:473); CCDS47907.1, Del, A_7, AFFANQEISIVKTIVVCLKVGCVILKMTVVMAAMKKIAQ, (SEQIDNO:474); CCDS9400.1, Del, A_7, DRKRKALKKNVKNPKVILIFLMKKQPSKVRRKEAHGLPL, (SEQIDNO:475); CCDS41508.1, Del, A 7, DTEIFFHFNIMDIVAQMREQRSGMVQTKEQYHFCYDIVL, (SEQIDNO:476); CCDS3422.1, Ins, A_7, EDGCVGKALWKWGSGGLAERSPVCFWAELGTPIDGDASQ, (SEQIDNO:477); CCDS46557.1, Del, A_7, FWRRRKIMRNCSSHPNLGRRSDLSLSRHGRRRRTSFPRR, (SEQIDNO:478); CCDS3411.2, Del, A_7, IQGMLKRTPKRNSNMKKIPKKPLKQVSIVKRKKFLLQRS, (SEQIDNO:479); CCDS5776.1, Del, A_7, LFASQMPMYQEAIKVVYKNSARFRSDVWREHLCLTADPV, (SEQIDNO:480); CCDS44379.1, Del, A_7, LPYLPAKISSPHQKQRLKKAYFQMRRTLRICFLLKVRVS, (SEQIDNO:481); CCDS53528.1, Del, A_7, LPYLTAKISSPHQKQRLKKAYFQMRRTLRICFLLKVRVT, (SEQIDNO:482); CCDS2397.1, Del, A_7, MQVLSKTHMNLFPQVLLQMFLRGLKRLLQDLEKSKKRKL, (SEQIDNO:483); CCDS33592.1, Ins, A_7, NGSRAAVPVCGRPAERQCFPHKSAQHGDPGEAGAEQSRV, (SEQIDNO:484); CCDS46450.1, Ins, A_7, NYPRVCVSLPVSCDVCHICKISSKSCCWWYIFRPNCALG, (SEQIDNO:485); CCDS32792.2, Del, A_7, QKLVDMKVHWKTFQGLVCLILGIYLCPKNKLLKTFIRWT, (SEQIDNO:486); CCDS8438.1, Del, A_7, QPMLGKKKVYLKSHCWKYCRTDTKGKNRLWLLSKSFTSY, (SEQIDNO:487); CCDS47859.1, Ins, A_7, RRLHPQQLCGQTQHLRNRRVVFQGYNQEGRRKAAFGTRK, (SEQIDNO:488); CCDS46038.1, Del, A_7, SACTAQSLSHYTLTSAETVLTLYLPRSGTMQLFSTFRTF, (SEQIDNO:489); CCDS7354.1, Del, A_7, SLRGSLRRSRKPEINMASMITAQQSPVCCTSWTASPPPK, (SEQIDNO:490); CCDS 164.1, Del, A_7, SNWTDLILLPAPKTVRSLPVFLLGLAQGPAQTYKQDWEN, (SEQIDNO:491); CCDS56589.1, Ins, A_7, TPETISDQQPEISQARNERGHCECAAECPGENQGQDAKR, (SEQIDNO:492); CCDS58199.1, Ins, A_7, TPPAFPESPAAVPPTTTGGPQTQLCIYTASHHSHSTGIT, (SEQIDNO:493); CCDS727.1, Del, A_7, VRNTEMDTEWHLHVHFVNFEHLKKKILNCIWKVLHIRKH, (SEQIDNO:494); CCDS8341.1, Ins, A_7, ADTFFFPDELQIVICFPVLIQEIWKGDFKASITGTERM, (SEQIDNO:495); CCDS35348.1, Del, A_7, AMMYPPRKKNVSTGKRKRNLLKKGQKRKCFGMSLFLDF, (SEQIDNO:496); CCDS4124.1, Ins, A_7, DSDLLCCLPRDWTPVVWKLGYHELVEQYLAQRGFCILF, (SEQIDNO:497); CCDS55350.1, Del, A_7, EAMFSSRLRGLIPPKNGFWNWNLTWPRKTTFFWNRRNI, (SEQIDNO:498); CCDS 1508.1, Del, A_7, ELLLREVGRSRPRRLTSSMRNMRAWSKKTPCCGERSGS, (SEQIDNO:500); CCDS2635.1, Del, A_7, EPWRYHLPKKQFLFYIMFENLFRDSQTVGNVNLKPTSA, (SEQIDNO:501); CCDS55048.1, Del, A_7, GGRMLLEMMKRLVRKNLKKTMMILKTSLKMSFQKMRKR, (SEQIDNO:502); CCDS7433.1, Ins, A_7, IYDFQRKEKISIKNAEKVEESRRETRARASRGRSFREY, (SEQIDNO:503); CCDS53355.1, Ins, A_7, KKGKKIKEGKKKGKKRRGRRSKPTMPQAQRRADGSGRA, (SEQIDNO:504); CCDS46430.1, Del, A_7, KPLMPIKSRQKKKKKKKQDQSTKRLRNPFSVFLKIMRK, (SEQIDNO:505); CCDS46811.1, Ins, A_7, NKMDEHESRFWPPLLSRLGQPLCHARPREGRPVPVCGL, (SEQIDNO:506); CCDS54695.1, Ins, A_7, NLPYCQLSTTGNDLTDEKASRFFTFPIITNRGNKRRNN, (SEQIDNO:507); CCDS34357.1, Ins, A_7, NPQWGEASRVQGLWEGLQDQEPSLYASAYPHRGEALQM, (SEQIDNO:508); CCDS13115.2, Ins, A_7, NWSGGRGISSHSPRRSSGSVLCFSGTYPGDVRHGFRSS, (SEQIDNO:510); CCDS13085.1, Del, A_7, TDASATYPSAQPLWATHGKASGRATTAGVQMKTPRPRD, (SEQIDNO:511); CCDS34195.1, Del, A_7, THTRWGITTWDSWSVRMKANWIQAQQNKSLILSPSFTK, (SEQIDNO:512); CCDS4306.1, Del, A_7, TKKKKKRRRKQKRPQPKILSHRPRRKRRKRRRQQSRLY, (SEQIDNO:513); CCDS5283.1, Ins, A_7, WRLHAHEVSAAPVQARVVLELWLRVEPRLHGGPLVRRV, (SEQIDNO:516); CCDS44334.1, Ins, A_7, ACKSPSTQELSKASKFCSTRTSSNACKSSTYSEYRSN, (SEQIDNO:517); CCDS31735.1, Del, A_7, AGNFAIMEIQCPALRKTLPILFGSLRRQNMSLEMWCR, (SEQIDNO:518); CCDS3322.1, Del, A_7, DLNFFRVGRLLLRSVFHNRKTTVTVESLCSSTASASP, (SEQIDNO:519); CCDS34204.1, Del, A_7, DRLIHLRRMMMKKIMIMIKEVLVAKQLLMLAIRRMKK, (SEQIDNO:520); CCDS6264.1, Del, A_7, EERRSPRNISMKLGIVGHLYYLGGSVLALSLKHLTKK, (SEQIDNO:521); CCDS44157.1, Ins, A_7, GVVHHSPAGRHHASLSGRQSQAAELRVEQCHTTKEVY, (SEQIDNO:522); CCDS1763.1, Del, A_7, IRTAPDGMEMKWPKEQRLISKPLSLSSRRQHLPMESS, (SEQIDNO:523); CCDS9379.1, Del, A_7, KKLMSLFVKDFVMMSRVMSVGMYMDSCSVLIKNMMKL, (SEQIDNO:524); CCDS 11984.1, Del, A_7, LKNPDKRLTSGLNVNPKVKSRNSSARTLLMLRLCWYW, (SEQIDNO:525); CCDS30955.1, Del, A_7, LWRNIKTNVSSSLSIRAILKKIHLQQRLLNAGKNTMR, (SEQIDNO:526); CCDS14401.1, Del, A_7, NLGRIHLLNSGGVHSPLLKCVVKFRSQKILLQSRLKA, (SEQIDNO:527); CCDS47324.1, Del, A_7, NLGRIHLLNSGSVHSPFLRCMVKFWSQKIVLQNRLKA, (SEQIDNO:528); CCDS42411.1, Del, A_7, SKMINQPRKSMCQKRGTLKRNETRLKRKARNLLGRKK, (SEQIDNO:529); CCDS9270.1, Ins, A_7, SYQISEQGKKEKVDNYCCVSGSGCHNRSNYWLVSWQM, (SEQIDNO:530); CCDS6316.2, Del, A_7, TGGVIHFQPVMHYVEEMLEGLVEQSYHLVTRNFIQIL, (SEQIDNO:531); CCDS150.1, Del, A_7, DLRLRIMYTCGRCITQIWDGCALMPLIQRRETGCDM, (SEQIDNO:532); CCDS44878.1, Ins, A_7, GMAPDEGGKARTTEPETVAGRRDVEGRAKTGNSKAG, (SEQIDNO:533); CCDS48007.1, Ins, A_7, GNRAADAADRKPEGDRSSQGPAHQSAGKALRAPRNL, (SEQIDNO:534); CCDS1899.1, Del, A_7, GSPTPPQLLFGKMPCHWEPFPSILGISLISCKLNKS, (SEQIDNO:535); CCDS6342.1, Del, A_7, HLSSCTCLRVHLSGHSGHHQKSMTSWPKKAGLLEQT, (SEQIDNO:536); CCDS43349.1, Ins, A_7, ICLQRKKAVLCWKRQKTKSFGNCFRRGKKESLKRMP, (SEQIDNO:537); CCDS6082.1, Ins, A_7, KYHGTVTRKSLQFLTACYFGTRAGDSDCVIWQIGRS, (SEQIDNO:538); CCDS47226.1, Del, A_7, LIGFLKIKRSKLLVAKMFLCFSSELWGKFYIVKEHL, (SEQIDNO:539); CCDS8243.1, Del, A_7, LMKLLSRNKNIKKPTIAMLRTPAKKRVKGKMRTFYL, (SEQIDNO:540); CCDS53846.1, Del, A_7, LPNQAKSGLWPITELKIQMRGILGEMSRLPILPRVP, (SEQIDNO:541); CCDS4597.1, Del, A_7, LPRVQLRPKPLSPRRPSLSRGSRRLQRQRRQLRRKS, (SEQIDNO:542); CCDS702.1, Del, A_7, LSRNILEMSFCLFMTKTSNMDLTFILLQLKRAKKTI, (SEQIDNO:543); CCDS32797.1, Del, A_7, LTTLMNVVKLLTHSQSLLNIKEFILERAHKDVIKNV, (SEQIDNO:544); CCDS13443.1, Ins, A_7, NCFRYQQRADRLSTRRNAVLEGGLPAVVQLETKISP, (SEQIDNO:545); CCDS46943.1, Ins, A_7, NDIPTSVIQRLHHQRESQIVTLGSPLPCIYPIFFQI, (SEQIDNO:546); CCDS5773.1, Del, A_7, NLLNRLESLGKKGRILFSIQSTLYENFPLCKRLPYK, (SEQIDNO:547); CCDS41814.1, Del, A_7, NLLRYKRKITEKKRKEPQRSCSVQSQILLLLLWLIG, (SEQIDNO:548); CCDS3751.1, Ins, A_7, NRHMYTTNKRSTRARRKGRESSPGKTNGPGRGKKAK, (SEQIDNO:549); CCDS33215.1, Del, A_7, PPSLTSMQDHALLPQFLTINMATLHPGCSPEEPALS, (SEQIDNO:550); CCDS14180.2, Del, A_7, PSKEWTMRINSRKHSLPRCHPSPPLLLPRLITRGCP, (SEQIDNO:551); CCDS3572.1, Del, A_7, QELQESEKPEVVVMIAGILGAMIEVPVVHVELWVVE, (SEQIDNO:552); CCDS9600.1, Ins, A_7, QGSSQRDCCGPVPEVLPQAHGHSLLLLRNCRYWGQW, (SEQIDNO:553); CCDS3352.1, Del, A_7, QKIATSCFRHQKLPEDRLFFVCHRKKMCHPRTWPKL, (SEQIDNO:554); CCDS35339.1, Del, A_7, QLLFMTLNYRLQRPYRKLLMTWALMLLSIILTLSLF, (SEQIDNO:555); CCDS55946.1, Del, A_7, RAYKVCTIRYVALKLKLRDKRKCFEIMNPKSFIYSE, (SEQIDNO:556); CCDS3205.1, Ins, A_7, RKQRRIKTCFRWFLAACKTHSFLYGPYLQSREKKTN, (SEQIDNO:557); CCDS9269.1, Ins, A_7, SYQISEQGKKEIDVHYYLCNCFACDPWNYPSNNIVL, (SEQIDNO:561); CCDS1802.1, Del, A_7, VTMAMPSSQTALPPLHHLLLKHLLLTAQEGICHHHH, (SEQIDNO:562); CCDS2474.1, Del, A_7, ASQEGQPHLDTESKRSSKGWIRFLKRKMTQLVTPR, (SEQIDNO:563); CCDS6192.1, Del, A_7, CRKWFLLFRLQWTGTFGIKVQSSHHLIFLKGKQKL, (SEQIDNO:564); CCDS12934.1, Ins, A_7, DASRILSPYCIRKTGYAKTLFYVAASKTHKALRIQ, (SEQIDNO:565); CCDS10137.1, Del, A_7, DLISSNSRIISIQYLDLETSKKLSKKLKDSLKALN, (SEQIDNO:567); CCDS56401.1, Del, A_7, FCQGKRKLNKRKKKLHSNFKKNCDGEEHSYMLMST, (SEQIDNO:568); CCDS9681.1, Del, A_7, FLILLNKAPVQGHHQIHSKFPVLTSQPQRLYQKTQ, (SEQIDNO:569); CCDS 1304.1, Ins, A_7, GAEESGPFNRQVQLKVHASGMGRHLWNCPAFWSEV, (SEQIDNO:570); CCDS53790.1, Del, A_7, GELSSEVEDDEKMTGSQDLILQQLNQRLQHQSLTY, (SEQIDNO:571); CCDS43379.1, Del, A_7, GRETIIKTLTQRKKLPWKLKLKLPEKGPLSLWRLE, (SEQIDNO:572); CCDS47683.1, Del, A_7, GSHSLRLPPPPPPPPLPCRHPSRLHCQGLTKRTVF, (SEQIDNO:573); CCDS43614.1, Ins, A_7, HDSREDCRVPTYHPHICSYWRRNRRIKELYKKVTG, (SEQIDNO:574); CCDS6254.1, Del, A_7, IGMWIKSIGEYGSVHMYLQYALLPMWDCCLVLFVP, (SEQIDNO:575); CCDS32029.1, Ins, A_7, IITWALQGAVHIVCPHHCGHSVLWTLYFYLCLAIQ, (SEQIDNO:576); CCDS43157.1, Del, A_7, KKGMKNQPSLMGQGSQMHFPSSKQKIVITCFPRWT, (SEQIDNO:577); CCDS9247.1, Del, A_7, LKEKRNRIANLRISTLSEGAPGRAKPSCSLKKGKE, (SEQIDNO:578); CCDS55686.1, Del, A_7, LKSHFSSRYPSVYPVLIFRLQKGHCTSGITNIFLV, (SEQIDNO:579); CCDS54577.1, Del, A_7, LSKCGTVRTGTLWLFSPCHSPVIAWKPILQRMAHS, (SEQIDNO:580); CCDS45069.1, Ins, A_7, NHLGPGGLSAGVAEGWGGPLQAAGAPAPRDHRENV, (SEQIDNO:581); CCDS8327.1, Del, A_7, NITMLKLKTKFGSWQTLCKRSNVWQDKCFFKPFLT, (SEQIDNO:582); CCDS30621.1, Ins, A_7, NNSFLGYPFCQPASQGPETNTDGFFPTSQDGCNLN, (SEQIDNO:583); CCDS12991.1, Ins, A_7, NYKATSSKASNTCTDSSRLCYNNRKFPKPEDTVYI, (SEQIDNO:584); CCDS11772.1, Ins, A_7, PGSRCPGSEGKHAEPGWRSHRVLPRHHLSSFPIQS, (SEQIDNO:585); CCDS43327.1, Ins, A_7, PLIPDLPIDCCPYKQGQGHPPIPRDHSRDQSISLL, (SEQIDNO:586); CCDS43919.1, Del, A_7, PSKEWTMRINRKHSLPRCHPSPPLLLPRLITRGCP, (SEQIDNO:587); CCDS30769.2, Del, A_7, QILQIIKKWIFLLETVNVKRKLISVCIILMMKLMK, (SEQIDNO:588); CCDS46368.1, Del, A_7, QRLQTFILILDYSVGIIAMLACLFYPNLNPKCSLV, (SEQIDNO:589); CCDS31816.1, Ins, A_7, SIFYMFFPHGGRVHFLWQLHLHVCETLSKRKSVIK, (SEQIDNO:590); CCDS8438.1, Del, A_7, SSLWDYPPRKRTLIFNFTIKKEGNLIQNRSPTPSE, (SEQIDNO:592); CCDS59367.1, Del, A_7, TIKVWMSVRCTEEVIMDLTNVCQLPRAKYFYLINV, (SEQIDNO:593); CCDS 1336.1, Ins, A_7, VKTTTGKNQLTDRQFTKSLCKGHSQSTTTNQKNQG, (SEQIDNO:594); CCDS32097.1, Del, A_7, VPCPNYLLQKNVCQDALGGVLPVIAVDLALAVKNY, (SEQIDNO:595); CCDS46941.1, Del, A_7, VSEKMEFSTLVWIIHVHYSGAFRRKKLFCLHMEIV, (SEQIDNO:596); CCDS 13996.1, Del, A_7, WSKTRQQPLGTARRRGRSRRLLLVSAKSWKRRTRL, (SEQIDNO:597); CCDS53903.1, Del, A_7, ALKPINTSIYILRASSLWGAAWWSPRKSLACPMP, (SEQIDNO:598); CCDS3960.1, Del, A_7, DGAMTLKRGRFQNPSPSLMVQTFLGTKEQEYPQN, (SEQIDNO:599); CCDS41745.1, Ins, A_7, DYKRAGSYNIGVCEKKYARGRDSLELLPKELEVI, (SEQIDNO:600); CCDS3920.1, Del, A_7, ENKILTHRRLGVLQEVIDQLLRRRVLREMGQGQH, (SEQIDNO:601); CCDS42450.1, Del, A_7, ETWVNFHGQPQRTTKCSERQMRTRTMGPPLRTQR, (SEQIDNO:602); CCDS34743.1, Del, A_7, FEKQLQTFWAVDKGNLRKDEIPQMVLIYPWLQLI, (SEQIDNO:603); CCDS255.1, Ins, A_7, FQKRKGEGEDPTTISVTLQIKIPDAVPLSFSHST, (SEQIDNO:604); CCDS6353.1, Del, A_7, GEVISSLSWTVLSKMSMISISYWKKQKSRLSGRL, (SEQIDNO:605); CCDS11672.1, Del, A_7, GSVKRVWSRNVASRMPLSCQLCSSSTKSSSEPRS, (SEQIDNO:606); CCDS42772.1, Ins, A_7, HGGLFYQTEMGKHTRELLPEHSGAGEGKNSTFMQ, (SEQIDNO:607); CCDS41619.1, Del, A_7, HGRILALSFWIIIFMKKILIFPATGRFSLKFLMG, (SEQIDNO:608); CCDS5659.1, Del, A_7, HKRTRPLLSRPPGSQRTWTASNWCPWFRKPSPPS, (SEQIDNO:609); CCDS2221.2, Ins, A_7, ICREVFRHFGRSCGPTRTQQLFGHTIRTSERSEK, (SEQIDNO:610); CCDS4987.1, Ins, A_7, KDLKTPAKIRKLYLCILYTYTINTAKTQCCSDKR, (SEQIDNO:611); CCDS8438.1, Ins, A_7, KRGISSRTDLLPRQSNRQDVYSECQGNGKCCHRS, (SEQIDNO:612); CCDS53826.1, Ins, A_7, KTSSHQTGSPLAWGPGSEGNPKEKVGRADYQKVH, (SEQIDNO:613); CCDS45050.1, Ins, A_7, LCCCPRNFYRRTKIRYRDGVSSCWPGWSPTLDLK, (SEQIDNO:614); CCDS44737.1, Del, A_7, LSLLFPWASISDPFPLMFLSEGPSMSTKPFSIFF, (SEQIDNO:615); CCDS3873.1, Del, A_7, MVIKGCQKKYWRIASFKLYAFSLKRSPHSCQTLV, (SEQIDNO:616); CCDS54624.1, Del, A_7, NGRNYTRVNLVMTMKIPKIFKPSKKPRCIWEISI, (SEQIDNO:618); CCDS43472.1, Ins, A_7, NKRSCREKKRMSACSWCLQAGMQQLGICILLLQC, (SEQIDNO:619); CCDS1629.2, Ins, A_7, NRGSCTAERISFGFIFSVCHLTSCFKEQTKKHIQ, (SEQIDNO:620); CCDS3928.1, Ins, A_7, NRILSGTCSFRQPSRAGGYIDIPLLHSELERLLY, (SEQIDNO:621); CCDS1404.1, Ins, A_7, NTGGNGEIRCQQLLNLVPGFRMPVQIFIHLLPRN, (SEQIDNO:622); CCDS42626.1, Ins, A_7, NTKISQVWWCIPILPATQGAEVGEWLEPGRQRLQ, (SEQIDNO:623); CCDS55466.1, Del, A_7, PFIKQQTFLRCLCALLMVISTFLQKNQLPLLILI, (SEQIDNO:624); CCDS33750.1, Del, A_7, QRQLLQPESLNLMQSLKQPAQLQVHRNNLRGKSR, (SEQIDNO:625); CCDS14032.1, Del, A_7, RGKGGREGSLGPSRGSEKPNKQFPLWNPKNLRSN, (SEQIDNO:627); CCDS1387.1, Ins, A_7, RKLQSWRRVEILLQKKSYKSWIRLSSMLPIWVVT, (SEQIDNO:628); CCDS765.1, Del, A_7, SQRSGTLWLALLLHSVVTTGVIGDIMWAKNISGL, (SEQIDNO:629); CCDS54564.1, Del, A_7, STIRRLQSWLKNTRQNWKALSISRMPFGLKNSKS, (SEQIDNO:630); CCDS53687.1, Ins, A_7, TAFRPICKGLFATRQRQNGQEENTRSEENLESCV, (SEQIDNO:631); CCDS4012.1, Ins, A_7, TSCFKTKSTQEGRVCQERFCCCRKAKGEGENKSH, (SEQIDNO:632); CCDS7805.1, Ins, A_7, TTSTKSREEKGSQARTSASINEGKNKIQSHNFIK, (SEQIDNO:633); CCDS7352.1, Del, A_7, VAANGLKMSSRQHYHQKKSNGMEACGTHPTPRLS, (SEQIDNO:634); CCDS54956.1, Del, A_7, WRRKHGQAELRDARKHAVEVTLDPETAHPKLCVS, (SEQIDNO:635); CCDS55692.1, Del, A_7, AKINVELPWWKIFLKALTIQKMHHFTYHFSKAG, (SEQIDNO:636); CCDS8436.1, Del, A_7, CPLLLVYIMSTTGWLERAYGRLWRPTAIRQTLY, (SEQIDNO:637); CCDS59119.1, Ins, A_7, EQLPVSDGPTVWLPRTGVHPKYKQIINTSNFCF, (SEQIDNO:638); CCDS43328.1, Del, A_7, ESPLICFHLQVLLLLNLRILAAQQLRFLVISPY, (SEQIDNO:639); CCDS3123.1, Del, A_7, GLQTKRTLRIRKPRVLKPLQFRLASQILPTLSK, (SEQIDNO:640); CCDS42650.1, Del, A_7, HVVSHLLSSSFLSLAALYLELLFWLYLELTQSI, (SEQIDNO:641); CCDS43515.1, Ins, A_7, ISGRSRKERKAAGRLQGGGVADARLERDQPCSS, (SEQIDNO:643); CCDS35337.1, Del, A_7, LACQHLMIHLVKLVPTTKCQNQERKVHPARVGL, (SEQIDNO:644); CCDS33716.1, Del, A_7, LFPKKKCTNLSVPMMLTILIWKRVFILLLSDKK, (SEQIDNO:645); CCDS47878.2, Ins, A_7, NQRGGIRGHQTRKTPQKRGKNLICSFSVGQSCR, (SEQIDNO:648); CCDS33318.1, Ins, A_7, NTCFPRVIGYKYASSKSYLSILLESVSISTHQL, (SEQIDNO:649); CCDS5731.1, Del, A_7, PALSSLMKLMLLEGLVLMMVLEVTMKCREQCWN, (SEQIDNO:650); CCDS33125.1, Del, A_7, PILDKNLMDVLNAVKPSLTGHTLLGTRKEFTPD, (SEQIDNO:652); CCDS9719.1, Del, A_7, QTEVCWKRWDSLKVKLSHRKRTARTVLFARMGL, (SEQIDNO:653); CCDS10137.1, Ins, A_7, QTRKRTSGKAARGTERETEEGRTRTKSQKETRS, (SEQIDNO:654); CCDS5944.1, Del, A_7, SLKKSSQKSATASCAHTTPTLKSSLQKSISAWP, (SEQIDNO:656); CCDS55053.1, Ins, A_7, TCRSRTTQGKKTGKERKTCGTSKVTKERTLSSK, (SEQIDNO:657); CCDS56504.1, Del, A_7, TPMTPLRRFLDQGCSTTCSQMNSFEMYLNLDPQ, (SEQIDNO:659); CCDS34171.1, Del, A_7, VNQKKGNITKRKKRREKRKSILLHLIVLNSPES, (SEQIDNO:660); CCDS4993.1, Ins, A_7, AAAFRGGKEEFISIYGINCPRIIFRFTWAFTE, (SEQIDNO:662); CCDS32238.1, Ins, A_7, AAAGVGFYCRRNHNRRLCLNHKWPQFGSCPRK, (SEQIDNO:663); CCDS11195.2, Ins, A_7, AERLGLHQDSATHRNGDPEESQRQRLLLISGN, (SEQIDNO:664); CCDS53552.1, Ins, A_7, CYSSLSRGSLQNWTRNAEHFKKSCSYFASASK, (SEQIDNO:665); CCDS556.1, Ins, A_7, DPGVCDLYDGLCLWGPNSISAVHAHLSPGLYR, (SEQIDNO:666); CCDS47865.1, Ins, A_7, EKEKVKGKKRPEGTERTQGEKRAQGTQDPESP, (SEQIDNO:667); CCDS43993.1, Ins, A_7, EKKERKRESRPGGTGRATILFVTQTQDSKGKG, (SEQIDNO:668); CCDS46441.1, Del, A_7, FILEVQLKETVVTHLLEYSTLLVISSYPSWLW, (SEQIDNO:670); CCDS8552.1, Ins, A_7, GAYALMPAAGGQLWGGARVCGGGGRGGSALRQ, (SEQIDNO:671); CCDS5974.1, Del, A_7, HSLWKSLISNNRSLMKQHCTVLWPLCIPNVNK, (SEQIDNO:672); CCDS47573.1, Del, A_7, IIHKNSLPPPTPKDVENPANLLTLLVAKILAG, (SEQIDNO:673); CCDS43209.1, Ins, A_7, IWDGKECSDITGFPGGINWDSFSRKAKSVFGM, (SEQIDNO:674); CCDS44612.1, Del, A_7, IYRKSGTHQMRTAPSVWNASRPPQATRARSLR, (SEQIDNO:675); CCDS55053.1, Del, A_7, KYTKIRLKNKRNLKGKYKLKLHTKKKKKEKKK, (SEQIDNO:676); CCDS5091.2, Ins, A_7, NCDYALQMCPKSTTGSSVASSQRRIRTFQETA, (SEQIDNO:678); CCDS11827.1, Ins, A_7, NGFGGYFRTIECNDNRKQEKCENSERRSSKAG, (SEQIDNO:680); CCDS12504.1, Ins, A_7, NLQAACTPCSSPENSHWGVTVTTQPSQSPSPI, (SEQIDNO:681); CCDS31873.1, Ins, A_7, NMDGTNNNCNFLLYQESGNQENIFIHIARRIL, (SEQIDNO:682); CCDS45246.1, Ins, A_7, NQAHHLRFGQSKQQQGAGCGPGVRGGQRQNKP, (SEQIDNO:684); CCDS45823.1, Ins, A_7, NSFVCYRPENPEHSRGHPTVLFCNLLCGGFES, (SEQIDNO:685); CCDS55457.1, Del, A_7, PRENCRKPWNLNQSAGSKWSRHLSKPPLVTVA, (SEQIDNO:686); CCDS53435.1, Del, A_7, QRKVLLQAGRDQKPSYCPEEPQLFLSEHPLGI, (SEQIDNO:687); CCDS53348.1, Del, A_7, REDILKACKQMQMIIFLITRMEWKKYLVPSIP, (SEQIDNO:688); CCDS33425.1, Ins, A_7, RPGSERKKRVPGPRQEDIRGRHTKEKEAQRRV, (SEQIDNO:689); CCDS44154.2, Del, A_7, RRMDSSHLSLIIPSLYGWYQLCVSSMKLLAHS, (SEQIDNO:690); CCDS3422.1, Del, A_7, RWLCRQSSMEVGIRRPGRKESCLLLGRTWHAN, (SEQIDNO:691); CCDS33332.1, Ins, A_7, SEITKIMGWKINSVFLSSLSVYMLNCIFVVSS, (SEQIDNO:692); CCDS3892.1, Del, A_7, SGEILFQKRYLFLGGLLQLQITRTSGISLMKG, (SEQIDNO:693); CCDS1575.1, Del, A_7, SKRSLMQPSSLAFNTRTLSNSQRSLKAGLQIK, (SEQIDNO:694); CCDS32064.1, Del, A_7, TKGKELDMNFRKFQLTSHFLEDGVVISLAKPQ, (SEQIDNO:695); CCDS92.1, Del, A_7, VVKTVALGHLTIRNVASVDPGQTVLWMESLCL, (SEQIDNO:696); CCDS47253.2, Ins, A_7, YFILGQISTSRIKGNKYFKSFGIFLFKNVREL, (SEQIDNO:697); CCDS53355.1, Del, A_7, EGEEDQRRKEEGEEKKGKKIKTHHAPGSTAC, (SEQIDNO:698); CCDS8523.2, Del, A_7, EVCLRLMNKCCFMVPAVNLWKQSAFITLIGE, (SEQIDNO:699); CCDS6281.1, Del, A_7, FPPSAQFIMSCIIRFPVIRTARPKTYFQAYF, (SEQIDNO:700); CCDS55354.1, Del, A_7, GTMKTSTNCPMTTSADPRQRRCCSLTQGPRT, (SEQIDNO:701); CCDS55323.1, Del, A_7, HLWISLIFFMNSVNLSSPKARDWTVSELLKN, (SEQIDNO:702); CCDS55242.1, Ins, A_7, IETWCQRICKTSNDLQWCVFTMFFNPIYCWM, (SEQIDNO:703); CCDS8579.1, Ins, A_7, IYVRKRFSPIHELLHHLVLVLESLLGMLPGY, (SEQIDNO:705); CCDS53507.1, Del, A_7, KIQSLQTVVKVKSYQKILLLSLRRTKALKGG, (SEQIDNO:706); CCDS33493.1, Ins, A_7, KKCFPQQRLCIQYLNGTRGGAYEICVQSAES, (SEQIDNO:707); CCDS34633.1, Del, A_7, KLVTSFCKKLPCRAISYMRSSLTLASELRPG, (SEQIDNO:708); CCDS659.1, Ins, A_7, KTNKITVTRKTPQVIIWIIPFWFPFKFKKEI, (SEQIDNO:709); CCDS33830.1, Del, A_7, MTTKRLNKMVSNQKIKLIRPQPKFRLASVDT, (SEQIDNO:712); CCDS4138.1, Ins, A_7, NGIKGRGKWARSSQKETTCQRQAREQTRNEL, (SEQIDNO:713); CCDS34872.1, Ins, A_7, NKRPAHHRRWSETFWGYCVQVLWYDIYCFQP, (SEQIDNO:714); CCDS32404.1, Ins, A_7, NSDNVAGPAEPGAAEAGDYGGGIWARVKGEQ, (SEQIDNO:715); CCDS47904.1, Del, A_7, NVSCLERKRIKPQNFVKHLKSVGKNVYLAEG, (SEQIDNO:716); CCDS43689.1, Del, A_7, PEQLTLPISQQKPSPVLGKLEMVKAVQLRVA, (SEQIDNO:717); CCDS12504.1, Del, A_7, PSGSMHTLLITREFTLGSHCHHPTQSITKSY, (SEQIDNO:718); CCDS6795.1, Del, A_7, QTRKSKTRAGMRTMMKTKKGWKKKNRMKRKK, (SEQIDNO:719); CCDS56279.1, Del, A_7, QVNYQSVKLQAFSNLKIPLLCCFWLLQSSVF, (SEQIDNO:720); CCDS33213.1, Del, A_7, RVKTTFLNCCLQKSLLKFSVSWTFGVCAGLH, (SEQIDNO:721); CCDS3450.1, Del, A_7, SKEKEKLVHLKRNQNLKRAGRLPKGTVWQRQ, (SEQIDNO:722); CCDS3993.1, Del, A_7, SRLPHQSPGHLGLFLLHQVLRKLKQMLNNKL, (SEQIDNO:723); CCDS8439.1, Del, A_7, SSLWDYPPRKRTLIFNFTIKKEGNLIQNRSF, (SEQIDNO:724); CCDS5749.1, Ins, A_7, SSRKDGCYWCRSNRCRIGFSLAKTWCRCDSS, (SEQIDNO:725); CCDS14032.1, Ins, A_7, TEAKEGEKEAWGPAEEAKNQTSSSHCGTPRT, (SEQIDNO:726); CCDS8328.2, Del, A_7, TTTKKKQLRCWNTWAKMFFMVFLIIWQNTMF, (SEQIDNO:727); CCDS44174.1, Del, A_7, TVFHHIVNQSINFRKNAEKNDKKQQGMAGLV, (SEQIDNO:728); CCDS31627.1, Del, A_7, VNFKKSMNCWRKPGDKVYLLPNGTGQRLWSG, (SEQIDNO:729); CCDS5461.1, Del, A_7, YVWIMKRKAFQLQQFEKLKFSGSLPIRVLSI, (SEQIDNO:730); CCDS3424.1, Del, A_7, CWLFYRRFLFYPQSQYPWFLFLLKDYSTSL, (SEQIDNO:731); CCDS659.1, Del, A_7, DEQDHGHPKDATGHHLDHPILVPVQVQKRN, (SEQIDNO:732); CCDS 13029.2, Ins, A_7, EAHCHHAEQALAHGEEADRAQGGPGICGEV, (SEQIDNO:733); CCDS1836.1, Del, A_7, EGSYQILRVTLVALMWNLSQTLRRKEAVMK, (SEQIDNO:734); CCDS1296.2, Del, A_7, EKTPNQALKNQKRKWMLYHSLISTIMQVLL, (SEQIDNO:735); CCDS9464.1, Ins, A_7, GLHKSAAFHCPDFPVLPFISAWQFPHSTFP, (SEQIDNO:736); CCDS4092.1, Del, A_7, ILVDLMPERVQNMRGVMLFLSETEFTNSTD, (SEQIDNO:738); CCDS32708.1, Ins, A_7, IPLSQNSLPHDPQNTQGQKESKGQKEKLSE, (SEQIDNO:739); CCDS11504.2, Ins, A_7, IPLSQNSLPRDPQNNQSQKESKGQKEKLSE, (SEQIDNO:740); CCDS42353.1, Ins, A_7, IPLSQNSLPRDPQNTQSQKESKGQKEKLSE, (SEQIDNO:741); CCDS1765.1, Del, A_7, IRTAPDGMEMKWPKEQKERATEMGRNPALP, (SEQIDNO:742); CCDS4657.1, Ins, A_7, KHFLCGVCGSTFCICFLCRIRVSPTGSNGI, (SEQIDNO:743); CCDS6301.1, Ins, A_7, KIKAAAGRSQKTAESLSYKDSFLASSPEND, (SEQIDNO:744); CCDS10916.1, Ins, A_7, KKGPEHSSQEEKTRWPLIRRRGRGGCQFRI, (SEQIDNO:745); CCDS10620.1, Ins, A_7, KLLLWLVLLFRSLLFHHRRNCRSGCRAHLY, (SEQIDNO:746); CCDS 11260.1, Del, A_7, KLSTKYFLPSVIVNKNWRLMSAIKKPKGNS, (SEQIDNO:747); CCDS13115.2, Del, A_7, LEWWSWDFQPLPSQKLGLGFVFQRHIPGRC, (SEQIDNO:748); CCDS32918.1, Del, A_7, LTLERTHMNVRNVGKHLLLSVPCIDIKRLT, (SEQIDNO:749); CCDS45989.1, Del, A_7, LTLERTRMDVRNVGKHLLLSVPCIDIKRLT, (SEQIDNO:750); CCDS6223.1, Del, A_7, MDPFARRLQLKNGRLLIQADRELKELIFQQ, (SEQIDNO:751); CCDS46348.1, Ins, A_7, METNNIWHLFLPCNYSGEKEVWPPWLEYLL, (SEQIDNO:752); CCDS8336.2, Del, A_7, MSHLIAHQALKMSGLRLFHPRLLTRKKPGK, (SEQIDNO:753); CCDS 13288.1, Del, A_7, NGTTVLQSKIQLASEKHPKAESSFLKMTQC, (SEQIDNO:754); CCDS47256.1, Ins, A_7, NLYCLQRPILWMDKGRWCLQPFITQQQTDF, (SEQIDNO:755); CCDS31532.1, Ins, A_7, NNYLCWLFSTVFLLHCSCPCGNFYSCCDGL, (SEQIDNO:756); CCDS58082.1, Ins, A_7, NRRVSGIFSEAPGIQSRPREGQRNRPRFQQ, (SEQIDNO:757); CCDS7780.1, Ins, A_7, NTGRSPGNDCWGHRHHTHVAAHSPHHQGPQ, (SEQIDNO:758); CCDS34547.1, Del, A_7, PLMKSLRIRNLHYINLQKKQRLWRKMFILM, (SEQIDNO:759); CCDS31756.1, Del, A_7, QATSRRRLQTCFSNLVLRIQELRKEKSMMP, (SEQIDNO:760); CCDS43493.2, Del, A_7, QTLRKKINPRTTVVKKQIPKEPNQNSSATP, (SEQIDNO:761); CCDS34482.1, Del, A_7, RVYLLRITKKVEMITKTMKILEIQIAMMLY, (SEQIDNO:762); CCDS34743.1, Ins, A_7, SGAEQSASVLLWTRCTVRPDTTGYPTKICG, (SEQIDNO:763); CCDS3397.1, Del, A_7, SPRSSVWERKSRPQTSRPPQLRKMFPPAAI, (SEQIDNO:764); CCDS9247.1, Ins, A_7, SSRKNATESQTYGFLPCPKELQEEQSRAAV, (SEQIDNO:765); CCDS33391.1, Ins, A_7, TYCEPRGAAETGGVCDAGPRQLTGHVRNPV, (SEQIDNO:767); CCDS42016.1, Del, A_7, WVHSLPVKKKRRLISAKSRFKEQKQNSTHW, (SEQIDNO:768); CCDS34137.1, Del, A_7, ALLCLIALLSRKQTWKIRTCLRRVRQKCF, (SEQIDNO:769); CCDS43349.1, Ins, A_7, AVLCWKRQKTKSFGNCFRRGKKESLKRMP, (SEQIDNO:770); CCDS3252.1, Ins, A_7, CFCDDDDLQGVIPQPPNSLPRRQCGSGCG, (SEQIDNO:771); CCDS42478.1, Del, A_7, CRIRERSLGGRLRPPTMARVTPSQLASMP, (SEQIDNO:772); CCDS7584.1, Del, A_7, DVESQIHCRKERVRRDQITLLIQNLKQSI, (SEQIDNO:773); CCDS3205.1, Del, A_7, EATSNQDLLQMVPCSMQDPLLSLWTLFTE, (SEQIDNO:774); CCDS45003.1, Ins, A_7, EDRTTKGYYSQNSQSKEEICDKSMWPCNF, (SEQIDNO:775); CCDS9504.1, Ins, A_7, EIQFKHPSDLFLVWLHRFQGYRDAYVRFD, (SEQIDNO:776); CCDS41816.1, Ins, A_7, EYCETGKRATNKQGKNNIKRRCKPTANNK, (SEQIDNO:777); CCDS11928.1, Ins, A_7, FNCNFSIRKMSTRGCQESRVASVYRKGQS, (SEQIDNO:778); CCDS 14684.1, Del, A_7, GNGSGGIKKSSKKTISFLQALIFLGSHTR, (SEQIDNO:779); CCDS31867.1, Del, A_7, HSRAQNPGSLLLKNSLEFLIDPLMPSRTL, (SEQIDNO:780); CCDS47878.2, Ins, A_7, IHFCLSPFFYYKPHRTRSNLPRFMERFSC, (SEQIDNO:781); CCDS4140.1, Ins, A_7, KFRGRFGGSEGSDCPVGSLLSCSQKTVSR, (SEQIDNO:782); CCDS892.1, Del, A_7, KIWPNKFYQGLENLSPSSPWLTSESFLAS, (SEQIDNO:783); CCDS3227.1, Ins, A_7, KKKEDSTPFSLFRFLWNKQNCRWIQWNFL, (SEQIDNO:784); CCDS9363.1, Del, A_7, KNMKRLVRRRCPPLLKFYVRGFLQNSPCT, (SEQIDNO:786); CCDS34872.1, Del, A_7, LLLENRPWKMSRHWDAPKRVNQKSLKILM, (SEQIDNO:787); CCDS9391.1, Ins, A_7, LPSDITEDFECCPIPPCCQGQVSLYNVLQ, (SEQIDNO:789); CCDS2702.1, Del, A_7, LSIKRKGKSRNTAEDTNKQRREGFLYRLT, (SEQIDNO:790); CCDS31906.1, Ins, A_7, NQGFTLAVSVCPGAAYGVCLGTGVQKRQL, (SEQIDNO:791); CCDS418.1, Del, A_7, PIAKMIEGTGSLRKLSGSSVNPRLPQQLQ, (SEQIDNO:792); CCDS9014.2, Del, A_7, QEMVQKHLLSFFAICLEDFMQSQTEKRIL, (SEQIDNO:793); CCDS13265.1, Ins, A_7, QSCSNGKQFTKGKCWTYEALCGLITLQHN, (SEQIDNO:794); CCDS14426.2, Ins, A_7, SPSGVPSSMGRSPRDGYDLLLFSHCEYIH, (SEQIDNO:797); CCDS54029.1, Del, A_7, SQMLSPQRERKRRPSQLPQMPPTETSRPR, (SEQIDNO:798); CCDS32133.1, Ins, A_7, SRNSTEAIIIPTANRPSNGRNSADESRLL, (SEQIDNO:799); CCDS41612.1, Del, A_7, SRSSVRRMEKSFAGFVNCLRNTKVTKHSA, (SEQIDNO:800); CCDS10721.1, Ins, A_7, SSKNSKSVHGPLSTSAAFYRNSEQIYLFL, (SEQIDNO:801); CCDS13443.1, Del, A_7, SSKSRGICPWIACSAARPSHIQKTLINMS, (SEQIDNO:802); CCDS10622.1, Del, A_7, VILVPQETENLMITKPLMILNGQMKRQNL, (SEQIDNO:803); CCDS9032.1, Del, A_7, VLKGFWRWIQAMCKENTIFVLFILKKKTY, (SEQIDNO:804); CCDS53588.1, Del, A_7, YSANLRNQTQRTPQQTQSNGPREASRKQT, (SEQIDNO:805); CCDS9052.1, Del, A_7, DERRRRAKGLLQQGNRNLIKNQEPQWMK, (SEQIDNO:806); CCDS32178.1, Ins, A_7, DSEQNSELPQRVCGGRPAHLVHRQESSE, (SEQIDNO:807); CCDS41507.1, Del, A_7, DTEIFFHMIQHAFLLEKARTTSMLVILE, (SEQIDNO:808); CCDS41485.1, Del, A_7, DYQRLPSENRITWRRKSVFKICASICMR, (SEQIDNO:809); CCDS53732.1, Ins, A_7, EECTNVRGGATTGGQSQRQLSAATGQSP, (SEQIDNO:810); CCDS14435.1, Del, A_7, EFQIQKILTLLKMRNTAKKEWIIKGTKI, (SEQIDNO:811); CCDS1628.1, Del, A_7, FLEKEVQEAKKGLLRRYHQDFFQEILHL, (SEQIDNO:812); CCDS4382.1, Del, A_7, FLNAKPSLDKLEKRKANIKKNLLRKCRN, (SEQIDNO:813); CCDS643.1, Del, A_7, GKSTSIINTELLLSTSSITSTPMKTMGF, (SEQIDNO:814); CCDS34171.1, Del, A_7, GNITKRKKRREKRKSILLHLIVLNSPES, (SEQIDNO:815); CCDS33304.1, Ins, A_7, ILGQEKTEVARNGNERSSASRRTEEINR, (SEQIDNO:818); CCDS47878.2, Ins, A_7, KGRASLVPEYTSAGEERPVPGGGSSTVS, (SEQIDNO:819); CCDS5089.1, Del, A_7, KRLFSCALASLQELDDCSLAGLQIMCLV, (SEQIDNO:820); CCDS2635.1, Ins, A_7, KSPGGITCQRSNFYFILCSRICFEIPRL, (SEQIDNO:822); CCDS31650.1, Del, A_7, LHLLIGRNTRNSATLNYQVKIPMKQRCF, (SEQIDNO:823); CCDS9012.1, Del, A_7, LLRKNIRHSHHHNQKVRSIKNWLVVNTF, (SEQIDNO:824); CCDS126.1, Del, A_7, LNSRWETKACPFQLESQTEASGTTGHRD, (SEQIDNO:825); CCDS43328.1, Ins, A_7, LSAGRESTKGFKTCTSEGPIAKAKAKCR, (SEQIDNO:827); CCDS34547.1, Del, A_7, LSRNCLLLLPQRRDRSMWILKLRKSLML, (SEQIDNO:828); CCDS34698.1, Ins, A_7, NHHRRERSKGEGERIGKKLQSELQLHHP, (SEQIDNO:830); CCDS6673.1, Ins, A_7, NKEKKESSPIEPRDHNEPSISEHVCWNV, (SEQIDNO:832); CCDS3492.1, Ins, A_7, NKSSFECSSKDKERECSCQPCRPGQGKF, (SEQIDNO:833); CCDS31783.1, Ins, A_7, NPCGGWEGAGSSRSLHQSHYFRRIREGF, (SEQIDNO:834); CCDS12394.1, Ins, A_7, NPDLPHPGPKESAGDVHQAEVRADEEGL, (SEQIDNO:835); CCDS47495.1, Del, A_7, NQLLPVLWELCCRTGSWWPRMKSQPSQD, (SEQIDNO:836); CCDS8493.1, Ins, A_7, NRRRGFSGHKGKAVHQPPFPGCKGEDVH, (SEQIDNO:837); CCDS5832.1, Ins, A_7, NRSPGSDPFPYPEECDCHPQVCDTSTHC, (SEQIDNO:838); CCDS4255.1, Del, A_7, NSISKNFSPMKSILRQEMLEPFLENAPF, (SEQIDNO:839); CCDS10589.1, Ins, A_7, NYSTLQISQKGRIYSRAAKDYQWEDKKK, (SEQIDNO:840); CCDS45945.1, Del, A_7, PMNVIIAGNPSQVTPTFLCIRECIIGKC, (SEQIDNO:841); CCDS3146.1, Del, A_7, PVQCASKKLFLLKAIQTLTQTVVKKKMK, (SEQIDNO:842); CCDS34565.1, Del, A_7, QFQMWISISLTSALAVAMLSFQYLCPCL, (SEQIDNO:843); CCDS55597.1, Del, A_7, QKTSLWQSLKLIKNRTVKWRRVEEKIWI, (SEQIDNO:844); CCDS35457.1, Ins, A_7, RGGRKLGRLGKSNQANCREICMHHKDIS, (SEQIDNO:845); CCDS41963.1, Del, A_7, RISPLLTILEIFYLKKRKQLLSFQQICQ, (SEQIDNO:846); CCDS32554.1, Del, A_7, RRRQSGGKRGREMGGKSKWNRSHQQLCF, (SEQIDNO:848); CCDS32704.1, Del, A_7, SLILFPPLIIQRLTIHHLKKTFTMSMKR, (SEQIDNO:849); CCDS5944.1, Del, A_7, SQKSATASCAHTTPTLKSSLQKSISAWP, (SEQIDNO:850); CCDS33971.1, Ins, A_7, TPGRTRSTTSQHICGSRRQHFNWIRSLL, (SEQIDNO:853); CCDS751.1, Ins, A_7, TRVLYEFLQPGSQRYAAQPHGPLCIQLP, (SEQIDNO:854); CCDS47033.1, Ins, A_7, TWGEAGCCQRQNDAAGVSWDTADRCHED, (SEQIDNO:855); CCDS32528.1, Del, A_7, VVNLGHSCCLDPETRLLRCGMSVLACAL, (SEQIDNO:856); CCDS53775.1, Del, A_7, VWLVKRIIQTLTKRRKKKRVFRKEKTMN, (SEQIDNO:857); CCDS32077.1, Del, A_7, WKRFVVVTNHFWPQMTCRPNTCNLRKNL, (SEQIDNO:858); CCDS34480.1, Ins, A_7, WRGKLFKYTLQCNKNSRTYCKRNEDEDI, (SEQIDNO:859); CCDS9441.1, Del, A_7, EITLCKADQEKVPPLQRQKKIHFLKDL, (SEQIDNO:860); CCDS59367.1, Del, A_7, FILEENPTNVKNVAKLLTSSQLLLNIR, (SEQIDNO:861); CCDS32980.1, Del, A_7, FILERNPISVKNVAKALFCPLILLHIR, (SEQIDNO:862); CCDS43593.1, Del, A_7, FILERNPTNVRNVAKLLTNSQTLLDIR, (SEQIDNO:863); CCDS5528.2, Del, A_7, FILKRNLTNVKIVAKSLVYFQSLLNIR, (SEQIDNO:864); CCDS42292.1, Ins, A_7, FISLSVDYIGYTGKMSCWATKGHNEIR, (SEQIDNO:865); CCDS59106.1, Ins, A_7, FWIVCSGEEGWIEKKAAARGRATKSKN, (SEQIDNO:866); CCDS32493.1, Del, A_7, GIQASPLQEVTGPPRRCLTGLPPVVCK, (SEQIDNO:867); CCDS4922.2, Del, A_7, GKNQLKHVQAFLWKNSLRTQLVHLAFL, (SEQIDNO:868); CCDS3911.1, Del, A_7, GLSLSYGPVSPIIRRLWNIFVRNQEKI, (SEQIDNO:869); CCDS30690.1, Del, A_7, KCLKVSGQKRERERSSWHLVLTLQRRP, (SEQIDNO:870); CCDS11323.1, Ins, A_7, KEDDDFRNCFPRRRCYYTVDVICRSKD, (SEQIDNO:871); CCDS43993.1, Del, A_7, KKRKKKRKPTGRYRQSNHPLRHPDARQ, (SEQIDNO:872); CCDS14201.1, Ins, A_7, KLKFKFLYNTFFGNSHSLNKSKTPTSD, (SEQIDNO:873); CCDS45779.1, Del, A_7, KNCCLMMGMTSWPPWGLETAPKQRRGR, (SEQIDNO:874); CCDS7283.1, Ins, A_7, KPLTKGTCFGSNKGTGKPSSQRLQRYN, (SEQIDNO:875); CCDS7597.1, Del, A_7, KRRKGRKSQQMMSLGVGTPGAEGGSPY, (SEQIDNO:876); CCDS6343.1, Ins, A_7, KSKLQHISVGKFVCSNQPMYLSASQGP, (SEQIDNO:877); CCDS43042.1, Del, A_7, KTQIANAQDRRELEKIPTMSRRVKSSS, (SEQIDNO:879); CCDS41485.1, Ins, A_7, KTTRGCLQKTELLGGESLFSRFVLQFV, (SEQIDNO:880); CCDS58744.1, Del, A_7, LILEEKVVIKGIPLDHWTMKVSNKNLL, (SEQIDNO:881); CCDS46029.1, Del, A_7, LILERNHTNVKNVAKPSTGPHTLLHIR, (SEQIDNO:882); CCDS32971.1, Del, A_7, LIVEKSHMNVQGVVKPLGGAVPSEDMK, (SEQIDNO:883); CCDS10898.1, Del, A_7, LVLVVVQIQNGLGRGTNILKLIMKGRC, (SEQIDNO:884); CCDS6266.2, Del, A_7, LWKIYTVTIHHISLWPLNYGRLLKDII, (SEQIDNO:886); CCDS13359.1, Ins, A_7, MFRSQTRCMRNAKRNWPLPGLFSSLVV, (SEQIDNO:887); CCDS2221.2, Del, A_7, MQRSIPAFWKVLWTDPYSATIRTHHQD, (SEQIDNO:888); CCDS2872.1, Ins, A_7, NAASTAAASGPRERGKLCKRHQTSSFW, (SEQIDNO:889); CCDS12228.1, Ins, A_7, NGSRQNSHELQDPPSQVHSVRAVPGRP, (SEQIDNO:891); CCDS30628.1, Ins, A_7, NHGRIWNRDYVGETETRLQTRAKQSDK, (SEQIDNO:892); CCDS3211.1, Ins, A_7, NILIQAHCLFSQYLVTICTTSGNHCFT, (SEQIDNO:893); CCDS42650.1, Ins, A_7, NMLSPIFCHLPFYHWLHYIWNYSSGYI, (SEQIDNO:894); CCDS43729.1, Ins, A_7, NPTTKICAEYSARTDQCRGGGLLTLKY, (SEQIDNO:896); CCDS46285.1, Ins, A_7, NRERKLSGKQNDLTRSLPTKFSGAVRA, (SEQIDNO:897); CCDS56348.1, Ins, A_7, NRITEGPCKCTCCCTKRTTRTKEKGGL, (SEQIDNO:898); CCDS34913.1, Ins, A_7, NVSSTRVFLGHGGRCENSRHQKSQPDP, (SEQIDNO:899); CCDS53935.1, Del, A_7, PLRPSGESGLSDLGTSAGREALALRNN, (SEQIDNO:900); CCDS43650.1, Ins, A_7, PRLCCRFISAPSRPRGFKTTRIFWEVW, (SEQIDNO:901); CCDS34158.1, Ins, A_7, QARPKRGSGKNKEISKRNGNFRRPSSI, (SEQIDNO:902); CCDS43378.1, Ins, A_7, QKHETRRKSKYNEDFERAWREDLTIKR, (SEQIDNO:903); CCDS32064.1, Ins, A_7, QKHWIDHSIFCYLYSRGNISRVNKNAF, (SEQIDNO:904); CCDS7639.1, Del, A_7, QSKALQPLMTFLKGNISMVHTTTILYL, (SEQIDNO:905); CCDS46404.1, Del, A_7, RMMSESNLNIEEKKESFSSPDQLNWKI, (SEQIDNO:906); CCDS6726.1, Ins, A_7, RNTSQIRLPLYPARTPASGIQRNDEQQ, (SEQIDNO:907); CCDS 1937.1, Del, A_7, RQTRRGREKKGPTRTVMRSPSSLLPRL, (SEQIDNO:908); CCDS5039.1, Ins, A_7, SFQCRAAADLWNDGNSCEIMGTNLCHF, (SEQIDNO:909); CCDS31136.1, Ins, A_7, SISPASGLCRSALGLCQVSPAVGTEGF, (SEQIDNO:910); CCDS34509.1, Ins, A_7, SQYIAGGEIRTGCRGRNTVKSEQFSSE, (SEQIDNO:911); CCDS35201.1, Del, A_7, SRGPKRQRWRRRPVVNLVLCLHETVNT, (SEQIDNO:912); CCDS32135.1, Ins, A_7, SRNSTEAIIIPTANRPSNGRNSADESS, (SEQIDNO:913); CCDS30715.1, Del, A_7, SSANVIALICLSRLTQINLLEQTAGQI, (SEQIDNO:914); CCDS9458.1, Del, A_7, TLMGLLKWILKQIKILRGARALIISSK, (SEQIDNO:915); CCDS53935.1, Ins, A_7, VLYPAGQGASRDGALCTRFISTCSYQL, (SEQIDNO:916); CCDS55679.1, Ins, A_7, VSTVVGLTSLCTTCDEWTQRAGWSAGQ, (SEQIDNO:917); CCDS34162.1, Ins, A_7, WMAYTWKKWYRKHKSSRWIPWWKFPFS, (SEQIDNO:918); CCDS43501.1, Del, A_7, YRSAGIKNLAMEFWLHEWHQQQQVALH, (SEQIDNO:919); CCDS42697.1, Del, A_7, ARCFLIIELGGLIKLKLNRLNLINIF, (SEQIDNO:920); CCDS55872.1, Del, A_7, ATNSKTIPLLAQDQPGVDLGMGTSGL, (SEQIDNO:921); CCDS8552.1, Del, A_7, CMTCWGRTCCGGSKPMCSRTCPPRQN, (SEQIDNO:922); CCDS8963.1, Del, A_7, DILMKMLLRTLKNMKVWSINLLKNPL, (SEQIDNO:923); CCDS41816.1, Del, A_7, DVQKNWSSKKENMKIQITKLNWETLI, (SEQIDNO:924); CCDS4066.1, Del, A_7, EENCLRNRNKKLKMLLNYLIQTKMKQ, (SEQIDNO:925); CCDS31071.1, Del, A_7, EFLFVRSKAFFPRYLASSLVQTLFNG, (SEQIDNO:926); CCDS53334.1, Del, A_7, ETQKKHVPSVLLKKVMKNLKMMMMMI, (SEQIDNO:927); CCDS32178.1, Del, A_7, FGAKLRITSTCMRRKACAPCASPREF, (SEQIDNO:928); CCDS13315.1, Del, A_7, FIHSRRMSLASLWVRPYQSCRLEKWR, (SEQIDNO:929); CCDS44157.1, Del, A_7, GGAPLPSWSPPCQSLRQAVTSSRTSG, (SEQIDNO:930); CCDS7585.1, Del, A_7, GIYFMLQTPTITRLKLWIQKQKTVQH, (SEQIDNO:931); CCDS718.1, Del, A_7, GMTFVNRIRKHHQIVAQLYFRSFSVL, (SEQIDNO:932); CCDS58914.1, Ins, A_7, HRNVQCHDAVEENGQSSFITSPILHS, (SEQIDNO:933); CCDS6192.1, Ins, A_7, IAGNGFFYSGFNGQAPSGSKYRAVTI, (SEQIDNO:934); CCDS34472.1, Del, A_7, IHATLQSHLCTFQHLPVLLKQIQLLS, (SEQIDNO:935); CCDS2702.1, Ins, A_7, KEETNSFRRLGFLFQFLLFFRIIFRK, (SEQIDNO:937); CCDS2291.1, Ins, A_7, KKKVMSASSYGENQRIRNSLQNGSRE, (SEQIDNO:938); CCDS44826.1, Del, A_7, KRSCKMNRKTKGKGKMRNRNSIPRKD, (SEQIDNO:939); CCDS53502.1, Del, A_7, KTLQTSFQPVCLKHHSFLKDSYALCL, (SEQIDNO:940); CCDS59035.1, Del, A_7, KYTKIRLKNKRNLKGKYKLKKLDTAL, (SEQIDNO:941); CCDS44172.1, Del, A_7, LLKIWKHQNLQRAMKLQKLHLWVTGI, (SEQIDNO:943); CCDS41939.1, Del, A_7, LQQKYYSRLRNHWHWQVGLCQTGVNN, (SEQIDNO:944); CCDS3028.1, Ins, A_7, NEEERTGCPRGCPASAPTAPPVHHSP, (SEQIDNO:945); CCDS32946.1, Ins, A_7, NETSWYQRPVHRCSGEVRDSAGIFFL, (SEQIDNO:946); CCDS1779.1, Ins, A_7, NKTQRQPSNFRSVHTLEQGLWRYHCL, (SEQIDNO:947); CCDS34290.1, Ins, A_7, NPQADTQICGGWCGGEHHHLQDHQRR, (SEQIDNO:948); CCDS9411.1, Ins, A_7, NSLPQLWEWSTYCPCNNRRRSLYLGS, (SEQIDNO:949); CCDS4950.1, Del, A_7, PTGSRSGQIPMPNTSTARRTRMRSGT, (SEQIDNO:950); CCDS3974.1, Del, A_7, QFTITQSLANAQFSQQQMLLSNLLVK, (SEQIDNO:951); CCDS9946.1, Del, A_7, QKTLKTSVLISPMIFSLSQIQFIGKQ, (SEQIDNO:952); CCDS8585.1, Ins, A_7, RGECYADPPAVVGHPGCIPRHPGDER, (SEQIDNO:953); CCDS4289.1, Ins, A_7, SGLQHLQEVSSGGHPLPHHIPTSLWF, (SEQIDNO:955); CCDS31441.1, Del, A_7, TKMKVIMPYVTWKVVGKSSRRKKQRA, (SEQIDNO:956); CCDS47633.1, Del, A_7, TQRFWRINQDLEFQTVIPIPVCSMFR, (SEQIDNO:957); CCDS10352.2, Del, A_7, WIRKTKLESASFRYFFVWRCVCNALQ, (SEQIDNO:958); CCDS33524.1, Del, A_7, YQDQLLVQNLDIMISSQFQLEPPTSK, (SEQIDNO:959); CCDS10239.1, Ins, A_7, YRSYAPRHCSSSEKQQECIYLWDDWN, (SEQIDNO:960); CCDS1916.1, Ins, A_7, AVAASGGSDSTEEAREQPPGGDPTL, (SEQIDNO:961); CCDS13443.1, Ins, A_7, CHPNRGVYALGLHVLQPDLHTFRRP, (SEQIDNO:962); CCDS14010.1, Del, A_7, CLTRLYQSVLSMTTRIFLNKLLKID, (SEQIDNO:963); CCDS44937.1, Del, A_7, CSVRSIGRGFYLSLKFYYCFITQTY, (SEQIDNO:964); CCDS8788.1, Ins, A_7, DPCQPSPGPAEGTPRYSPAVDTGRV, (SEQIDNO:965); CCDS43513.2, Del, A_7, ECPSFPSISMSVLYIDTMFNGTGVF, (SEQIDNO:966); CCDS55323.1, Ins, A_7, EDNTRELWEYPQKRTMCCFQQSICI, (SEQIDNO:967); CCDS43313.1, Del, A_7, EGRSSTNARSATVCLTAGTSSKITW, (SEQIDNO:968); CCDS59135.1, Ins, A_7, ESPECCTKCFANQLKQFYKQRISSC, (SEQIDNO:969); CCDS58232.1, Del, A_7, FLLGPRPPMSWLERWIFWTVPLLPL, (SEQIDNO:970); CCDS34146.2, Ins, A_7, FNVKLHSSLYHSFFLHSSIYKMSFS, (SEQIDNO:971); CCDS53961.1, Del, A_7, FSSSMMKVFEGTWNRLNKEKKKLLS, (SEQIDNO:972); CCDS255.1, Ins, A_7, GGHEQSKFGGYKALDNKKSNGNPWV, (SEQIDNO:973); CCDS13288.1, Ins, A_7, GMERQYFSPKSNSPPRSIQKQSPAF, (SEQIDNO:974); CCDS43313.1, Del, A_7, GRLQKLQMLSLSHCHLQNQNLLKLR, (SEQIDNO:975); CCDS3091.1, Ins, A_7, HFSIPKNVDSAVWICFGSWMCSSYH, (SEQIDNO:976); CCDS30726.1, Ins, A_7, HHVGRTAWVFALVVVCILGPLLCSS, (SEQIDNO:977); CCDS54796.1, Del, A_7, HRMSRNGSRKGWLILLITLASAGQN, (SEQIDNO:978); CCDS13896.1, Del, A_7, HSQPLKTWAQHTPATLSSAQPRRDI, (SEQIDNO:979); CCDS5230.1, Ins, A_7, IKVQAGREKRRLHSWVWCRTFHSRH, (SEQIDNO:980); CCDS34101.1, Ins, A_7, KGGAISCLSCRGFCSFWCTNWRSSF, (SEQIDNO:981); CCDS3757.1, Ins, A_7, KKECRNYVAGCNDLPQTEWYSFHLL, (SEQIDNO:982); CCDS55311.1, Ins, A_7, KKRTCKSRTQPKSGFRGKARKAPKK, (SEQIDNO:983); CCDS3751.1, Del, A_7, KMMVKCLKKKRKVNLRKRVKKKLKS, (SEQIDNO:984); CCDS8676.1, Del, A_7, LCGAVRINRFFLLMTKLFDFGIMLL, (SEQIDNO:986); CCDS8019.1, Ins, A_7, LGNRGCCCLGLQRECSLRNLHRRYR, (SEQIDNO:987); CCDS3756.1, Del, A_7, LKILFNLCILMLLYIRKVMFTPVAK, (SEQIDNO:988); CCDS32710.1, Del, A_7, LMTCRVNLIKEKKILKRKFINLRKL, (SEQIDNO:989); CCDS42411.1, Del, A_7, LTTERKVEKRWIGNMTKKSLKKRGI, (SEQIDNO:990); CCDS7148.1, Del, A_7, LWNLHLAKGQFIKMQTAWKKKRRHL, (SEQIDNO:991); CCDS54777.1, Del, A_7, MNFLHHPDLYLESDEVAWLKMLCVN, (SEQIDNO:993); CCDS3761.1, Del, A_7, MRSRTYYPLAITDTVEQSKRRMKSY, (SEQIDNO:994); CCDS9793.1, Del, A_7, MTTVAGEKSWKNARVWSTSHFEAKE, (SEQIDNO:995); CCDS3194.1, Ins, A_7, NFLLSGTVNSEIWNASEYTSYQRDS, (SEQIDNO:996); CCDS10841.1, Ins, A_7, NKENQKEQTALYRGGQRCRCVCLRF, (SEQIDNO:998); CCDS9344.1, Ins, A_7, NRTCPFRLFVRRMLQFTGNKVLDRP, (SEQIDNO:999); CCDS41302.1, Ins, A_7, NSPEGANCLSEERICSTFLLHTMHH, (SEQIDNO:1000); CCDS5830.1, Ins, A_7, NTSGKRFYTQTARGSPASSNGKDTP, (SEQIDNO:1001); CCDS32787.1, Del, A_7, PGAAPSASALAFLMSAAASLGLLVR, (SEQIDNO:1002); CCDS3200.1, Del, A_7, PNLQWIFIKRFPYLIRTTLYFHPLE, (SEQIDNO:1003); CCDS9148.2, Del, A_7, QHQEGFILRRLSQTYLHWKTVAALQ, (SEQIDNO:1004); CCDS46549.1, Ins, A_7, QKEEEEKGKTTQESQKTKERNKGEK, (SEQIDNO:1005); CCDS54212.1, Ins, A_7, QPSIFPGIFIFSNLTFATLFHTIWE, (SEQIDNO:1006); CCDS30956.1, Del, A_7, QSADQCKEYLPMYCSILLEFPLQLE, (SEQIDNO:1007); CCDS55053.1, Del, A_7, RKRKKNMWNQQSHQRKNTQFQVTNK, (SEQIDNO:1008); CCDS10594.1, Del, A_7, RSFPPETKRIPLGSSWPSRRQSSHP, (SEQIDNO:1009); CCDS 14406.1, Del, A_7, RSTSTKHLLFSSRTHGNPGDRPHRC, (SEQIDNO:1010); CCDS42024.1, Del, A_7, SEKMRLSLVIRHKIGRFLITILKRI, (SEQIDNO:1011); CCDS31444.1, Del, A_7, SKAKILSSSEMGLGKLILCFPTLMM, (SEQIDNO:1012); CCDS2382.1, Del, A_7, SQVERSLILWLSMALFVPRTLHIKR, (SEQIDNO: 1013); CCDS4916.1, Del, A_7, SRSSISIFKFMMKILSMGPSCQSVF, (SEQIDNO:1014); CCDS11260.1, Del, A_7, SWKILVSKWFQKMAIYSYTLIKEVF, (SEQIDNO:1015); CCDS34723.1, Del, A_7, TKIFQKKKIHKIRILLWIVKERPTK, (SEQIDNO:1016); CCDS55733.1, Ins, A_7, TPENSLQQVAREHGSTPHRKDSLTR, (SEQIDNO:1017); CCDS1507.1, Ins, A_7, TQGSKELRAHQASAPEVCTDLCSRP, (SEQIDNO:1018); CCDS33125.1, Ins, A_7, WGHWRADLEAKGCERESRKRSPINQ, (SEQIDNO:1019); CCDS3877.1, Del, A_7, WKMRRLQFSHVHLNHPNQKQSISWM, (SEQIDNO:1020); CCDS6266.2, Del, A_7, WMTKHIVISRNWKIMLKTHRALFFT, (SEQIDNO:1021); CCDS41534.1, Del, A_7, YIMNKSKLFKNSATSLANQNLKLKT, (SEQIDNO:1022); CCDS 1377.1, Del, A_7, ANSKLSSSLLLRKHSCGQKHLTSC, (SEQIDNO:1023); CCDS 11512.1, Del, A_7, ATNIIAKLWRMMTLNLKDQKILGK, (SEQIDNO:1024); CCDS5622.1, Del, A_7, CMSSSPKWKIFSASWKRKDNKFIS, (SEQIDNO:1025); CCDS12495.1, Del, A_7, EEQPILKKNIHAMNVGNLSAGNQY, (SEQIDNO:1026); CCDS 13298.1, Del, A_7, GCCRLLTEMGKRKRKRRSHWMKAQ, (SEQIDNO:1027); CCDS34740.1, Del, A_7, GERNIQHVIAQNNLLSILMKILHK, (SEQIDNO: 1028); CCDS9148.2, Ins, A_7, GWQKCNWPGPGMYFKKSSTKKALS, (SEQIDNO:1029); CCDS44379.1, Ins, A_7, GYLIFQQKSQALIRNKDSKRLIFR, (SEQIDNO: 1030); CCDS53528.1, Ins, A_7, GYLILQQKSQALIRNKDSKRLIFR, (SEQIDNO:1031); CCDS35057.1, Del, A_7, HLWISLIFFMNSVNLSSPKASLLR, (SEQIDNO: 1033); CCDS1842.1, Ins, A_7, IAIKRNICQSPGGHVDLPQPLLCF, (SEQIDNO: 1034); CCDS34146.2, Del, A_7, IARRYLESFSVFSCFSGRLSVLFL, (SEQIDNO: 1035); CCDS2021.1, Del, A_7, ILKLTLKMILTLMYILEKQRLLLF, (SEQIDNO:1036); CCDS45021.1, Del, A_7, KAAQFLHSRRKFRKKCHVLICRWT, (SEQIDNO:1037); CCDS46063.1, Ins, A_7, KGEPFQGTSKTEQNLKGTSKTNQG, (SEQIDNO: 1038); CCDS33810.1, Ins, A_7, KHDCFNSYHSSLFGFVLKPKWRSD, (SEQIDNO:1039); CCDS43623.1, Ins, A_7, KKGTNQQASREERKGKIKSYQCRI, (SEQIDNO:1040); CCDS56537.1, Del, A_7, KKVKLLHENMDSSSWKRLLRLLPM, (SEQIDNO:1041); CCDS31658.1, Del, A_7, KKVKSLYMMILKLSKVSHKEVEQK, (SEQIDNO:1042); CCDS 10539.1, Ins, A_7, KLPFPPRSGGQQEEQVSLARPHLR, (SEQIDNO:1043); CCDS41963.1, Del, A_7, KSCVPGWCRWKTKFYRQRTLVLKK, (SEQIDNO:1044); CCDS5230.1, Del, A_7, KTLPLIQGKMLCPQGPTVRQNRHQ, (SEQIDNO:1045); CCDS3158.2, Del, A_7, LFLQKRSQSSSGSFCSSSPCQIRS, (SEQIDNO:1046); CCDS33545.1, Del, A_7, LKLPRLLPHTPPPAPSSSLSPLVS, (SEQIDNO: 1047); CCDS2259.1, Del, A_7, LLDKFWCLFYCTAPSIAPTCGQAF, (SEQIDNO:1048); CCDS41815.1, Del, A_7, LLGFQGPLGCLLQEAAARSREPLI, (SEQIDNO:1049); CCDS45820.1, Del, A_7, LLLGNQIHSILLGKITYYVCAMIS, (SEQIDNO: 1050); CCDS44402.1, Ins, A_7, LSTHKCFRTSNYVPFRSNKYGNHI, (SEQIDNO:1051); CCDS9767.1, Ins, A_7, LTGKETQKLYGRPCREKRNGIGFK, (SEQIDNO:1052); CCDS42805.1, Ins, A_7, NEREGEEKGKGQIESQGEKAQRGE, (SEQIDNO:1053); CCDS10622.1, Ins, A_7, NQTQQRNWEENWKYRKYIKHKRTL, (SEQIDNO:1054); CCDS30956.1, Ins, A_7, NSQQTSARNTYLCTAQYFWNFHSS, (SEQIDNO:1055); CCDS3123.1, Ins, A_7, NTTGMCSRRNYISPSTLVFNERVY, (SEQIDNO:1056); CCDS54834.1, Ins, A_7, PSSKAKSRGKELSHILCTAGRAGT, (SEQIDNO:1057); CCDS44337.1, Del, A_7, QDILQAKETEWHKVRAREEKLRKF, (SEQIDNO:1058); CCDS33391.1, Del, A_7, QKHFSILNLPQKQISHIVSQRRDI, (SEQIDNO:1059); CCDS 10606.1, Del, A_7, QNRASCLDAWANQKGELMYRLILK, (SEQIDNO:1060); CCDS34238.1, Del, A_7, QPSPASGYLQSMSGTGTSMWPSQH, (SEQIDNO:1061); CCDS93.1, Del, A_7, RDHMMWWFYQEVIWAHRIYLSLLL, (SEQIDNO:1062); CCDS55959.1, Ins, A_7, RGPETTIFLLLPISIISAANFMSF, (SEQIDNO:1063); CCDS32234.1, Del, A_7, RHFLEVEGKLNKEIIHRMKMGFKN, (SEQIDNO: 1064); CCDS5954.1, Ins, A_7, RKKDHFRNDTNTQKAWLLHLGRSL, (SEQIDNO:1066); CCDS10008.1, Ins, A_7, RLQVGRILASYEEFFLNGRRRYHV, (SEQIDNO:1067); CCDS41477.2, Del, A_7, RNGMRRKKVHPRLKDHFRKNLPKK, (SEQIDNO:1068); CCDS5930.1, Del, A_7, SIPVSLEAQVECSSHSSKQTKLTM, (SEQIDNO:1069); CCDS45727.1, Ins, A_7, SISAGTHVPGPPGQEKPVHRRQRG, (SEQIDNO: 1070); CCDS4662.1, Del, A_7, SKNMSGLRPRFLCRTRFFCWAPRS, (SEQIDNO:1071); CCDS55466.1, Del, A_7, SLMSKKWKKAALLSTLNLQTWKMK, (SEQIDNO:1072); CCDS33566.1, Del, A_7, SRYEWNLESLNGQNGKLAKPPQPS, (SEQIDNO: 1073); CCDS54820.1, Ins, A_7, STALSQSLCSTFGPGTRHQSSVPT, (SEQIDNO:1074); CCDS46543.1, Ins, A_7, STERAYSRRAGDGLPACGRRGGVL, (SEQIDNO:1075); CCDS3467.1, Del, A_7, STFQIIFQSANANGNLNCCRSMGY, (SEQIDNO:1076); CCDS54426.1, Del, A_7, STIYLRKKMKTLKPAGKILLFCKN, (SEQIDNO:1077); CCDS30628.1, Del, A_7, SWEDLEQRLRWRNRNPSPNQGQTK, (SEQIDNO:1078); CCDS54808.1, Del, A_7, TKISNGRPINRSSSILDRLQPHGG, (SEQIDNO: 1079); CCDS5734.1, Ins, A_7, TRKDKQSSPWAKTISTRQIISNII, (SEQIDNO:1080); CCDS31136.1, Del, A_7, DQPCIWPVQVSTWPVPSFSCSRD, (SEQIDNO:1081); CCDS8253.1, Del, A_7, EGKEKMRWKKSQQLCKKLTKRKF, (SEQIDNO:1083); CCDS9244.1, Del, A_7, EQRKPWKKRSYLKNCGSQVKRST, (SEQIDNO:1084); CCDS31816.1, Del, A_7, HFLHVLPTWWSCPFLMAAASSCM, (SEQIDNO:1085); CCDS34530.1, Del, A_7, HFSSFLVDPLTFRRKTMALAFLL, (SEQIDNO:1086); CCDS10624.2, Ins, A_7, KEKGKGNDERWKQTRRSVDKSIC, (SEQIDNO: 1088); CCDS32097.1, Ins, A_7, KFRVQIIYSKRTCVKTLWAEFYL, (SEQIDNO:1089); CCDS47331.1, Del, A_7, KLEKVVVQLTLLNMKIKNGEMKV, (SEQIDNO:1090); CCDS9407.1, Del, A_7, KVGSGFLGKNYLPWTSFSGDCVV, (SEQIDNO:1091); CCDS41485.1, Del, A_7, LLQESVIQTPKKTRISSVLTIMR, (SEQIDNO:1092); CCDS1802.1, Del, A_7, LQETMDQVIILHFRVHLPQLSGI, (SEQIDNO: 1093); CCDS8664.1, Del, A_7, LRLHLPRYLDFFMTKKMKAIWIP, (SEQIDNO:1094); CCDS 10245.2, Del, A_7, LTVALRQPLPVVATHLGIASTKN, (SEQIDNO:1095); CCDS 12228.1, Ins, A_7, NAPGEEEETEQESHLLGRRSRQD, (SEQIDNO: 1096); CCDS11963.1, Ins, A_7, NFYILRGKTEASYRYCFTWSNRS, (SEQIDNO:1097); CCDS3993.1, Ins, A_7, NLASHTKAPATSASSCCTRFFEN, (SEQIDNO:1098); CCDS1389.1, Ins, A_7, NSCTKERRVYADAGILYQTASIC, (SEQIDNO:1099); CCDS42690.1, Ins, A_7, NTTEVQSLSCENLHFQSKQVPED, (SEQIDNO:1100); CCDS4529.1, Ins, A_7, NVEGCIQSTSIFRSLEQPSWKSA, (SEQIDNO:1101); CCDS54212.1, Ins, A_7, PKGKDDDFHKGHKCKLISINRFY, (SEQIDNO:1102); CCDS8976.1, Del, A_7, PVKILNKTRMLQKNGLPLLSKVN, (SEQIDNO:1103); CCDS10180.1, Del, A_7, QLCQKRLYPPEIVTLLISGYLPS, (SEQIDNO:1105); CCDS35229.1, Del, A_7, QLLTHREMGKSRGPKCQSSQNDF, (SEQIDNO:1106); CCDS9056.1, Del, A_7, QRKSSKMMHWPLKSSFEKMTRDL, (SEQIDNO:1107); CCDS14435.1, Del, A_7, QRNLQVQDAFLPTVWALVRLYRW, (SEQIDNO: 1108); CCDS1113.2, Del, A_7, QRRKRTFANGIEKETSKLGKMMV, (SEQIDNO: 1109); CCDS47430.1, Del, A_7, QSQCWRRRCWTMMGVAWPPSLNP, (SEQIDNO:1110); CCDS2614.1, Del, A_7, QSTCCPVASGSSWSTTSRSWKCC, (SEQIDNO:1111); CCDS1605.1, Ins, A_7, QTQRRIPHSPSQIPVPLPEIWCT, (SEQIDNO: 1112); CCDS44945.1, Del, A_7, RNVCYTGRKREGYMEKLRFWLSV, (SEQIDNO:1113); CCDS47865.1, Ins, A_7, RPEGTERTQGEKRAQGTQDPESP, (SEQIDNO: 1114); CCDS54424.1, Del, A_7, RPLKLFLEKSHQLKYRRCLRKLW, (SEQIDNO: 1115); CCDS8585.1, Del, A_7, RRMLRGSSSGCGAPRMYPKTSRR, (SEQIDNO: 1116); CCDS55025.1, Ins, A_7, RSPRDAWFNGKQWLTRPAWNTGI, (SEQIDNO: 1117); CCDS3401.1, Del, A_7, SGRTVWIMVSPCSTRSEFLARAF, (SEQIDNO: 1118); CCDS1359.1, Del, A_7, SLSPPHPVLLSIPWTQIPQGCLP, (SEQIDNO: 1119); CCDS34206.1, Ins, A_7, SNMVCSFLLFNRTWITFMCFSIH, (SEQIDNO: 1120); CCDS44663.1, Del, A_7, STLCSRKSQIPSSPKWLSTGPRP, (SEQIDNO: 1121); CCDS679.1, Del, A_7, STRNTEVLFQTYLMEQSLVQCSV, (SEQIDNO: 1122); CCDS46935.1, Ins, A_7, SVGENSERNSPSPGAGRFGIGYL, (SEQIDNO: 1123); CCDS54288.1, Del, A_7, SVQKRVAAVRKSCRDGTVDPVGT, (SEQIDNO: 1124); CCDS32097.1, Del, A_7, SYLNILPALFDVLIVASVLKLKI, (SEQIDNO: 1125); CCDS58101.1, Ins, A_7, SYTKGQRQEEAGPPGECRPGLSP, (SEQIDNO: 1126); CCDS3424.1, Ins, A_7, TAGCFTGDSYFTHNPNIPGFFSC, (SEQIDNO: 1127); CCDS9072.1, Ins, A_7, TAPPEACHGPLFLSSAVSSIGCK, (SEQIDNO: 1128); CCDS58907.1, Ins, A_7, TFYPSLPGTFQEDVCVKRRPMAL, (SEQIDNO:1129); CCDS9822.2, Del, A_7, TKQMRVFSGWRYTKRSLKQCPNL, (SEQIDNO:1130); CCDS4330.1, Del, A_7, VLNWRKNGSLNLRQSPKRRKSPP, (SEQIDNO:1131); CCDS53868.1, Del, A_7, WNLFPRSLSLQKIMMDLNHPESI, (SEQIDNO:1132); CCDS10030.1, Del, A_7, WNLSNQVPPKMLPRKSWKTGWTA, (SEQIDNO:1133); CCDS41692.1, Del, A_7, WVKNLQKSQNLFCRYYFCGTSGY, (SEQIDNO:1134); CCDS45820.1, Ins, A_7, CCYLEIKYILFCWEKLLTTYVQ, (SEQIDNO:1135); CCDS32146.1, Ins, A_7, CWHLCSSSTLLSLCGRSKVSAR, (SEQIDNO:1136); CCDS31964.1, Del, A_7, DFKKTLEEKHLESISNTKPLSS, (SEQIDNO: 1137); CCDS895.1, Ins, A_7, DGAFGLLCWGNVCTRSRWFQSR, (SEQIDNO:1138); CCDS11923.2, Del, A_7, DSPLKRWWKRSCQRKPWLLESP, (SEQIDNO:1139); CCDS43513.2, Del, A_7, EKVNHLSIPIHKVTVQLLATLE, (SEQIDNO:1140); CCDS46393.1, Del, A_7, ELVSTGIMTFHLLSVDIYQEAT, (SEQIDNO:1141); CCDS35067.1, Del, A_7, FILEGNTLNVLNVEKLSQGNQH, (SEQIDNO:1143); CCDS6149.1, Del, A_7, FLERPLYQALNPSFMQEISKRN, (SEQIDNO:1144); CCDS3585.1, Del, A_7, FQEIVTSSSLRQAFHSALTSVQ, (SEQIDNO:1145); CCDS9498.1, Ins, A_7, GASRDEKESARRGTQREPPILR, (SEQIDNO:1146); CCDS34225.1, Del, A_7, GHAAALCLMPRSYMRMPKWQGR, (SEQIDNO:1147); CCDS31920.1, Del, A_7, GKCFGRARKKGTNPNLLKYGKN, (SEQIDNO: 1148); CCDS34908.1, Del, A_7, HLHRMLYMNRLQPSTQWYKITA, (SEQIDNO:1149); CCDS43423.1, Ins, A_7, IPFVRRHVSSYERPCKDTLDCG, (SEQIDNO:1152); CCDS9375.1, Del, A_7, KIVMEEVTQKHRKKKVFRNRIH, (SEQIDNO:1153); CCDS41715.1, Ins, A_7, KVTQKTEEKQKGSHRYNSRFLE, (SEQIDNO:1154); CCDS55861.1, Ins, A_7, LCGNAKLSQRWVPCTSKHYLAF, (SEQIDNO:1155); CCDS43209.1, Del, A_7, LGWKRVLRHHWIPRRNQLGFFQ, (SEQIDNO:1156); CCDS4912.1, Del, A_7, LPLVFMILLRKTTKLLTQISGN, (SEQIDNO:1158); CCDS6277.1, Del, A_7, LSVMELEIHVYHTLGSLQPQAL, (SEQIDNO:1159); CCDS43515.1, Del, A_7, LWKKQKREKSSWQTSGRWCRRC, (SEQIDNO:1160); CCDS14525.2, Ins, A_7, NDYYRGFIAQPRSHLELFHFQP, (SEQIDNO:1161); CCDS8597.1, Ins, A_7, NERVSRSQLCSRSQAWTPPQLL, (SEQIDNO:1162); CCDS54352.1, Ins, A_7, NGCYSVWKNTIFNCTRSFTQFP, (SEQIDNO:1163); CCDS45410.1, Ins, A_7, NGQEDPAGVQNQDAELRMEIQL, (SEQIDNO:1164); CCDS3954.1, Ins, A_7, NHADICYTAVETEQRKIQLPAQ, (SEQIDNO:1165); CCDS44852.1, Ins, A_7, NKCNRKHSEELIKRHADPEGSN, (SEQIDNO:1166); CCDS33307.1, Ins, A_7, NRIRENKCHCKCCQNTDTGCPE, (SEQIDNO:1167); CCDS43187.1, Ins, A_7, NRYFLYQSSKNKYLWTAQSCLL, (SEQIDNO:1168); CCDS53935.1, Del, A_7, PIPCGSGSIQRWGPMYKVYLNM, (SEQIDNO:1169); CCDS34725.1, Ins, A_7, PTEIGRASRKPLHCCSQKYNRN, (SEQIDNO:1170); CCDS6726.1, Del, A_7, QHFSNSATTLPSKDPGFGHPTQ, (SEQIDNO:1172); CCDS43284.2, Del, A_7, QSQQFLGVHIILICLQVAVLII, (SEQIDNO:1173); CCDS13117.1, Del, A_7, RKRKKTLFNILQTLLSKKNKGH, (SEQIDNO:1174); CCDS4518.1, Del, A_7, RVRLLFFMYIIMLLCLTSGGVS, (SEQIDNO:1175); CCDS47380.1, Del, A_7, SAIKQRKQRTMWSGKPSLWSSG, (SEQIDNO:1176); CCDS5929.1, Ins, A_7, SASANYLWLRANTKTSCNSQKD, (SEQIDNO:1177); CCDS30787.1, Del, A_7, SFEIQQNNFKNIIGKDYAINSI, (SEQIDNO: 1178); CCDS34207.1, Ins, A_7, SIFKYVYTCKDPPNTTSYLKEH, (SEQIDNO:1179); CCDS42395.1, Del, A_7, SNWQKRCVPQWIPRSARRRSGP, (SEQIDNO:1180); CCDS43309.1, Del, A_7, SRSALKKMLLHESKGRKDGGNC, (SEQIDNO:1181); CCDS7480.1, Del, A_7, SSQESLQWQTLQNMPVITFVLC, (SEQIDNO:1182); CCDS46935.1, Del, A_7, SWRKFRKKQPFSRSWKIWNWVF, (SEQIDNO:1183); CCDS11155.1, Del, A_7, TCNSRFKLKLTAWLMQRKGVTS, (SEQIDNO:1184); CCDS11156.1, Del, A_7, TCSSKFRLKPKAWLMQRKGVTS, (SEQIDNO:1185); CCDS55053.1, Ins, A_7, TGKERKTCGTSKVTKERTLSSK, (SEQIDNO:1186); CCDS12272.1, Del, A_7, TLERNPTNVNSVEKPLYITSLF, (SEQIDNO:1187); CCDS44926.1, Del, A_7, TLKNMPWLIKIFSKIKKAGIRF, (SEQIDNO:1188); CCDS31441.1, Del, A_7, TQKLKVSNQQLYKLFLKGKKRS, (SEQIDNO: 1189); CCDS31195.1, Ins, A_7, VHHHKQCHWEEAHICHRFSQDQ, (SEQIDNO: 1190); CCDS47161.1, Ins, A_7, WDAYGTAFKVFERFCIQHSGYQ, (SEQIDNO: 1192); CCDS4138.1, Del, A_7, WDQRKRKMGKVISKGNDLSKTG, (SEQIDNO: 1193); CCDS33943.1, Del, A_7, WTRSQKGKASLTIGGWRICCSL, (SEQIDNO: 1194); CCDS43242.1, Del, A_7, YVNMTACLLCTSAAWAQLLVKY, (SEQIDNO: 1195); CCDS3892.1, Ins, A_7, APARYYSRNVIYSSEDSYSSR, (SEQIDNO:1196); CCDS765.1, Ins, A_7, CPREVEPCGWHCYCTLLSPQV, (SEQIDNO: 1198); CCDS43419.1, Ins, A_7, CTQKTQPKEYKQETEKPANNS, (SEQIDNO:1199); CCDS47168.1, Del, A_7, CVMLQQDLKYTCPFQIEFPKL, (SEQIDNO: 1200); CCDS55918.1, Del, A_7, DCCHGNSRCRSSFGLSLGKPF, (SEQIDNO: 1201); CCDS41948.1, Del, A_7, DDFGSHQILITKMTTSRLAVR, (SEQIDNO: 1202); CCDS892.1, Ins, A_7, ERFGQTSSIRVWRICHQAHLG, (SEQIDNO:1203); CCDS1533.1, Del, A_7, GLPAPSVLLSWRWMKLNLGQL, (SEQIDNO: 1204); CCDS1767.2, Del, A_7, HIITTNLNAPKDLVWLYSVRI, (SEQIDNO: 1205); CCDS1113.2, Ins, A_7, HKGGRGPSQTESRKKHRSWER, (SEQIDNO: 1206); CCDS31166.1, Del, A_7, HLLLRRMCVAPITQVITSSNL, (SEQIDNO:1207); CCDS43341.1, Del, A_7, HRDCFSKISNLQKIKLEKQAS, (SEQIDNO: 1208); CCDS32146.1, Ins, A_7, ICNSDITLYVYRRGGPLCFAL, (SEQIDNO: 1209); CCDS55520.1, Ins, A_7, KGMGAEESRSPARRRSLFFRL, (SEQIDNO:1210); CCDS34204.1, Del, A_7, KLFLVREVQRGEKQELRRIKQ, (SEQIDNO:1211); CCDS34908.1, Del, A_7, KLKALSFFSAEEQTQISETST, (SEQIDNO:1212); CCDS12945.1, Ins, A_7, KLLQKFYGNKTQESLCRKEAF, (SEQIDNO:1213); CCDS34471.1, Ins, A_7, KPNLSHTSYLHGEHSPVPLDC, (SEQIDNO:1214); CCDS10860.1, Ins, A_7, KPVSTFYLYTSFDEARTQRRD, (SEQIDNO:1215); CCDS14466.1, Ins, A_7, KRKKEKEKEEILSVFIFFIKL, (SEQIDNO:1216); CCDS34330.1, Del, A_7, LGTRIQDLDIMRRRRCGQTPT, (SEQIDNO:1217); CCDS5750.1, Del, A_7, LKSQLMPEGKPKCYKMKQKLF, (SEQIDNO:1218); CCDS44410.1, Ins, A_7, LSGCLTGQCSQFSHVCCKYDV, (SEQIDNO:1219); CCDS10167.1, Del, A_7, LVSFIFRLIICSLNITEHEKL, (SEQIDNO:1220); CCDS47688.1, Del, A_7, MPVVSSTDDPLAVQFLASLTC, (SEQIDNO:1221); CCDS30888.1, Del, A_7, MQEWKRYLEASLKTPVSCVES, (SEQIDNO:1222); CCDS41500.1, Ins, A_7, NERDYILLGFYPSEIRITSAR, (SEQIDNO:1223); CCDS1197.1, Ins, A_7, NGEEGKTEAQYGGTEEGSGHG, (SEQIDNO:1224); CCDS53743.1, Ins, A_7, NGFLPECKRQQSIRTQLLVPK, (SEQIDNO:1225); CCDS3397.1, Ins, A_7, NHQDHQSGKEKAVHRRADLLS, (SEQIDNO:1226); CCDS8336.2, Del, A_7, NIYGIQSLHLLAVQSVSPFTS, (SEQIDNO: 1227); CCDS7587.1, Ins, A_7, NKNNSKLYFTRRIRHTFFRGI, (SEQIDNO:1228); CCDS47812.1, Ins, A_7, NPCQRSSAGAYGRDRKFEETT, (SEQIDNO:1229); CCDS229.2, Ins, A_7, NPEQYPGDAGADEPDSVCGGL, (SEQIDNO:1230); CCDS9504.1, Del, A_7, NPIQTSIRSFPGVAPQIPRIS, (SEQIDNO: 1231); CCDS58338.1, Ins, A_7, NRCGLQLAQKLPESPHNYPSD, (SEQIDNO: 1232); CCDS10606.1, Ins, A_7, NRIEPPASMLGQIKRENSCTD, (SEQIDNO: 1233); CCDS2384.1, Ins, A_7, NRSSGPLSLQEIHFKTCYQIY, (SEQIDNO:1234); CCDS33803.1, Ins, A_7, NYEEEKILSHSETNVIPSGNL, (SEQIDNO: 1235); CCDS9982.1, Ins, A_7, PLHPGSQEKSSDRGANSDHIY, (SEQIDNO:1236); CCDS31874.1, Ins, A_7, PYTTGASNSTNREAEESVRKS, (SEQIDNO:1237); CCDS43242.1, Ins, A_7, QDGWGVGSSAISGTPFERKGY, (SEQIDNO:1238); CCDS9819.1, Del, A_7, QRHSVRPRIRRTLSPVKNVAG, (SEQIDNO:1239); CCDS41815.1, Del, A_7, QRMQLPLLMWTTSQVRNCNNI, (SEQIDNO:1240); CCDS59105.1, Del, A_7, QTFCLIIPLYCKNQKTTKVLS, (SEQIDNO:1241); CCDS35085.1, Ins, A_7, RFKERQGYRERKRRKARCKER, (SEQIDNO:1242); CCDS5398.1, Del, A_7, RMERVKKLKRQSLKNLSWKKY, (SEQIDNO:1243); CCDS7405.1, Del, A_7, RTALITHTLCCWLTWAQVSAF, (SEQIDNO:1244); CCDS47495.1, Ins, A_7, RTNCYQCCGSFAAEQGPGGHE, (SEQIDNO:1245); CCDS34101.1, Ins, A_7, RYPPAYGPDGKPRPRFNNVQL, (SEQIDNO:1246); CCDS34225.1, Ins, A_7, SGRYKDEWSSPVIEYCLCCKG, (SEQIDNO:1247); CCDS3131.1, Del, A_7, SKRQTTKWSLQQMEFCGQIKD, (SEQIDNO:1248); CCDS10362.1, Del, A_7, SMIENRPDTRRLASTLDTLIM, (SEQIDNO:1249); CCDS2800.1, Del, A_7, SQIPWRLGTRPTCLRSLSHSL, (SEQIDNO: 1250); CCDS53353.1, Del, A_7, SRISGSKLAAIDPSPLTIYST, (SEQIDNO:1251); CCDS30942.1, Del, A_7, SRLIQFLYCVERKWKTQGGIT, (SEQIDNO:1252); CCDS41813.1, Ins, A_7, SSECRGFLSYVQILHLTESRR, (SEQIDNO:1253); CCDS644.1, Ins, A_7, TKTVSEGISFDISEKISNHGE, (SEQIDNO:1255); CCDS4541.1, Ins, A_7, TSIQLVCLWNTFPFTWEFFNP, (SEQIDNO:1256); CCDS47584.1, Ins, A_7, TSLFIKWLTYKLCQYLLERCN, (SEQIDNO:1257); CCDS6257.1, Del, A_7, VAAVAAATLGSRVPSTQTQLH, (SEQIDNO: 1258); CCDS1504.1, Del, A_7, VLNCFLWSLNGKILSSRKSKI, (SEQIDNO: 1259); CCDS41630.1, Del, A_7, VSAAYYFLNKLQIFCKSVMIL, (SEQIDNO:1261); CCDS43714.1, Del, A_7, VVEGGGYLSPIQLRFPKENLL, (SEQIDNO:1262); CCDS46028.1, Del, A_7, VVKVWMRVKYTKEVIMDLTNV, (SEQIDNO:1263); CCDS45410.1, Del, A_7, WPRGSSRCTKPRRGAQDGNPT, (SEQIDNO:1264); CCDS683.1, Del, A_7, YKMMLVFAFSLSQMMGQHPKG, (SEQIDNO:1265); CCDS35277.1, Del, A_7, YSLSKASAENFLSCGRPSEGC, (SEQIDNO:1266); CCDS3211.1, Del, A_7, YSYPSSLSVFSVPGYHLYYFR, (SEQIDNO:1267); CCDS2219.1, Del, A_7, ADPHLSSPPLMMNKSRSSQE, (SEQIDNO:1268); CCDS13585.1, Del, A_7, AFHHTVRKQTLNFHFWTRGI, (SEQIDNO:1269); CCDS12328.1, Del, A_7, AKPRNLLLKRRRKIIAATAM, (SEQIDNO:1270); CCDS3738.1, Del, A_7, CMRNLDNLLGKMQKKITGMG, (SEQIDNO:1272); CCDS34037.1, Del, A_7, CWILMRQSLFVGTFMDNSLI, (SEQIDNO:1273); CCDS34100.1, Del, A_7, DILRHMAQTANQDPASIMFN, (SEQIDNO:1274); CCDS44805.1, Del, A_7, DSLKVLLVFQLVQCLVQRWL, (SEQIDNO:1275); CCDS7805.1, Del, A_7, DVHQKQRRKRLPSQNVCLHQ, (SEQIDNO:1276); CCDS1767.2, Del, A_7, EDMLDWDCLHHHLYPVIQQG, (SEQIDNO:1277); CCDS11983.1, Ins, A_7, ETTESTSRCHCYRTTKCEME, (SEQIDNO:1278); CCDS11070.1, Ins, A_7, ETTRRSQLLSRREEKSAGNG, (SEQIDNO:1279); CCDS11870.2, Del, A_7, FCSIMTNKIRSNPIHLWYWT, (SEQIDNO:1280); CCDS9300.2, Del, A_7, GISLVLIQHYQCYRTPLFML, (SEQIDNO:1281); CCDS7258.1, Del, A_7, GNTANGPGIGRKSLMPMQVT, (SEQIDNO:1282); CCDS1838.1, Del, A_7, GVTAMHFIIPVLYDYKRVIL, (SEQIDNO:1283); CCDS32915.1, Del, A_7, GVTRTLNMSTKTPEEASGVS, (SEQIDNO:1284); CCDS4071.2, Ins, A_7, IEKEVRRRIVMFQGELPLCS, (SEQIDNO:1286); CCDS32967.1, Del, A_7, IFSTRMRRHVGTQTRAPPSG, (SEQIDNO:1287); CCDS55093.1, Del, A_7, IKPFSTNSVLRRTGSSIEEL, (SEQIDNO:1288); CCDS58957.1, Ins, A_7, IQAALDIACCRPAFRHAEWR, (SEQIDNO:1289); CCDS32073.1, Ins, A 7, ISSSVNDQPPCTKFIWNTKR, (SEQIDNO:1290); CCDS32686.1, Del, A_7, IVLPHLPTQPNACTGTSLRN, (SEQIDNO:1291); CCDS1336.1, Ins, A_7, KAKGKTESPRSQSDQAVRVI, (SEQIDNO:1292); CCDS907.1, Ins, A_7, KCIKCSPVIRLGTFISSKKI, (SEQIDNO:1293); CCDS53871.1, Del, A_7, KRKNKIERVAILIWKALMKI, (SEQIDNO:1294); CCDS5230.1, Ins, A_7, KRRCHSLGIIQKDGDAQEAC, (SEQIDNO: 1295); CCDS3598.1, Ins, A_7, KTYLRKELQDDQEEKGFTIN, (SEQIDNO:1296); CCDS47757.1, Del, A_7, KYKKFKELLMQKMKGLASYL, (SEQIDNO:1297); CCDS32146.1, Del, A_7, LASMFVLDSSLPMWKKQSQC, (SEQIDNO:1298); CCDS47063.1, Del, A 7, LFHLKGRRQQLKATKEYRSF, (SEQIDNO:1299); CCDS9477.1, Del, A_7, LGKNRRNLHLRKMNHILLMC, (SEQIDNO:1300); CCDS10540.1, Del, A_7, LICHQIKISLFLKVTMMMLC, (SEQIDNO:1301); CCDS899.1, Del, A_7, LQDLRLVLRFGKAFALSPNS, (SEQIDNO:1302); CCDS14237.2, Del, A_7, LQILKLILKRARLLEGVWWM, (SEQIDNO:1303); CCDS43349.1, Del, A_7, LSSKKKSCFMLEKTENKIVW, (SEQIDNO:1304); CCDS33356.1, Ins, A_7, NCAEGGKTVLWDRWCRSKLH, (SEQIDNO:1305); CCDS5605.1, Ins, A_7, NEDSENDWSINIVSILRLAG, (SEQIDNO:1306); CCDS11260.1, Ins, A_7, NHGKFWYPNGFKKWQFTVTH, (SEQIDNO:1307); CCDS11672.1, Del, A_7, NHIFDMNLLDMIEVGGNTGS, (SEQIDNO:1308); CCDS14246.1, Ins, A_7, NKEDFSVQKSPLNKSKDCQE, (SEQIDNO: 1309); CCDS3974.1, Ins, A_7, NSSLLRRAWPMLSFPSNRCS, (SEQIDNO:1310); CCDS7824.1, Ins, A_7, NTCITSYSYSEPFLFSTALF, (SEQIDNO: 1311); CCDS8772.1, Ins, A_7, NTETTGCHNYFKRQDHDTQP, (SEQIDNO:1312); CCDS8558.1, Del, A_7, PLKLRRQQQPQPHLRTLSNT, (SEQIDNO:1314); CCDS10654.1, Del, A_7, PPQPMGPPPECCSSWLRRIE, (SEQIDNO:1315); CCDS5063.1, Del, A_7, PSAMCQLQDCFQICTNPVIS, (SEQIDNO:1316); CCDS34698.1, Del, A_7, PSQEREVQGRRGKDWEEASV, (SEQIDNO:1317); CCDS33307.1, Del, A_7, QDSRKQVPLQMLPKYRHWMP, (SEQIDNO:1318); CCDS46851.1, Del, A_7, QNSYRTSQKRRGHWPVKFVT, (SEQIDNO:1319); CCDS56280.1, Del, A_7, QVNYQSVKLQAFSKLIFRMV, (SEQIDNO:1321); CCDS34171.1, Ins, A 7, RETSQKGKKEEKKGKAFFYT, (SEQIDNO:1322); CCDS1389.1, Del, A_7, RFRNSTRLPLLFRSIVKPLK, (SEQIDNO:1323); CCDS13092.1, Ins, A_7, RGVSGQIGNTCCRAFSGEDT, (SEQIDNO:1324); CCDS46430.1, Ins, A_7, RKGTSKAVAKEKLGSLKKHT, (SEQIDNO:1325); CCDS55858.1, Ins, A_7, RKRSLSTSSAGAYRSSEIKK, (SEQIDNO:1326); CCDS8884.1, Ins, A_7, SCGGTRKTRKRCSRRSRKRG, (SEQIDNO:1327); CCDS612.1, Ins, A 7, SKCSTYTIRNIQKAGSRSSC, (SEQIDNO:1328); CCDS390.1, Del, A_7, SKLQMIWAFYLKMMYHIFWG, (SEQIDNO:1329); CCDS43501.1, Del, A_7, SKRRKAIVTAQTIALIVIHH, (SEQIDNO:1330); CCDS31783.1, Del, A_7, SLRWVGRSWIFTVSTPESLL, (SEQIDNO:1331); CCDS13473.2, Del, A_7, SLSLPRWCATGSTRTCLPRP, (SEQIDNO:1332); CCDS6252.1, Del, A_7, SPQWVVHFHEDAAVHILPLE, (SEQIDNO:1333); CCDS9049.1, Del, A_7, SQILTSYIFGKQTAFLFASG, (SEQIDNO:1334); CCDS10159.1, Ins, A_7, SQKGASWFAFFCICTIPKFR, (SEQIDNO:1335); CCDS34501.1, Ins, A 7, SQSIWKRRCENELHRSDLYW, (SEQIDNO:1336); CCDS43162.1, Del, A_7, SREKALNSIYHSKKVMGFIK, (SEQIDNO:1337); CCDS47443.1, Ins, A_7, SSHCTAFFWCRGFRKIFGRH, (SEQIDNO:1338); CCDS3556.1, Del, A_7, SSWQVLFMNIQEDILSLLSQ, (SEQIDNO:1339); CCDS44525.1, Del, A_7, SWLKKNRGSCRSWRRMRGSS, (SEQIDNO:1340); CCDS389.1, Del, A_7, TFLNNLIRFLSLNQYGKILV, (SEQIDNO:1341); CCDS46028.1, Ins, A_7, TFQMYRMWQSFQPVLNPCYT, (SEQIDNO:1342); CCDS6080.1, Del, A_7, TFRCYKIITWIIFLSQKKMF, (SEQIDNO:1343); CCDS5746.1, Del, A_7, TMLALLNPSQGGFCLLNFHL, (SEQIDNO: 1344); CCDS10374.2, Ins, A_7, TRELQEKRGRNQTMVRESFS, (SEQIDNO: 1345); CCDS58012.1, Ins, A_7, TRVLYEFLQPGSQRVLPVCL, (SEQIDNO:1346); CCDS1177.1, Ins, A_7, VPRRCLSRQTNRTCQRYAIT, (SEQIDNO:1347); CCDS10620.1, Del, A_7, VTPMVGPFISEPSLSSSQKL, (SEQIDNO:1348); CCDS11103.1, Del, A_7, VTRRRSIRQTQISVTLGRSL, (SEQIDNO:1349); CCDS7258.1, Del, A_7, YRRASYPSTKFLLEKNSRRP, (SEQIDNO:1350); CCDS33737.1, Ins, A_7, ATAPRANQTTVPESITGPL, (SEQIDNO:1351); CCDS43729.1, Del, A_7, ATSMTHQDLKSASHTKSPN, (SEQIDNO:1352); CCDS6004.1, Del, A_7, AVMHNIRISKLEMKRISTS, (SEQIDNO:1353); CCDS43758.1, Del, A_7, CLQACNLSQILRLALQLCL, (SEQIDNO:1354); CCDS731.1, Ins, A_7, DFHERLCSRIIQKISYLHM, (SEQIDNO:1356); CCDS42670.1, Del, A_7, DFRNMNMFYIQELQALLLW, (SEQIDNO:1357); CCDS46222.1, Del, A_7, EKHKINSPRNLLLNESHLP, (SEQIDNO:1358); CCDS9379.1, Del, A_7, EMKKKKKPVALRKNLYLKN, (SEQIDNO:1359); CCDS9030.1, Del, A_7, ETIYLRQLSQFLSNPAMKS, (SEQIDNO:1360); CCDS2702.1, Del, A_7, EVKKNPSLNGNALIQKKEL, (SEQIDNO:1361); CCDS1278.1, Del, A_7, FHQQLNSAAPQLRKWLKRF, (SEQIDNO:1362); CCDS45827.1, Del, A_7, FIRKLRIIQLVIRKQLLKS, (SEQIDNO:1363); CCDS11963.1, Del, A_7, FLHLTRKDGSKLPLLFYLE, (SEQIDNO:1364); CCDS34283.1, Del, A_7, FLVSRTSLNKSYSYSRSHC, (SEQIDNO:1365); CCDS32519.2, Del, A_7, GRRRPRGLARGTINIRVRA, (SEQIDNO:1367); CCDS41329.1, Del, A_7, HIIQDWWTKIVSLMQLLSN, (SEQIDNO:1368); CCDS41953.1, Ins, A_7, IIIRICTTFEKSGGGTRGG, (SEQIDNO:1369); CCDS3131.1, Ins, A_7, IPKGKPQSGLCSRWNFVAR, (SEQIDNO:1370); CCDS53945.1, Del, A_7, ISLTSFLTGWMMYLHIQKL, (SEQIDNO:1371); CCDS43426.1, Del, A_7, KENIFVSIAIEHVQSLVCF, (SEQIDNO:1372); CCDS30920.1, Ins, A_7, KFSRCRLSTAATEPRGLCQ, (SEQIDNO:1373); CCDS58290.1, Ins, A_7, KISNKITFSQVPFVIQVQV, (SEQIDNO:1374); CCDS2194.1, Del, A_7, KLANMQNIPLQVYLCQNQI, (SEQIDNO:1375); CCDS4118.1, Ins, A_7, KRSAWGFEILEKGNEHEVQ, (SEQIDNO:1376); CCDS10932.1, Ins, A_7, KSANQGVYLVQQRLGHPRH, (SEQIDNO:1377); CCDS53626.1, Ins, A_7, KSFPRVQWHAQDKQIGIWK, (SEQIDNO:1378); CCDS46776.1, Ins, A_7, KSGFWKSLLISFIFSEVRR, (SEQIDNO:1379); CCDS9095.1, Del, A_7, LASLQNQKKNKKKLQTHLK, (SEQIDNO:1380); CCDS41470.1, Del, A_7, LGIRAGSLMLICKSLTEQF, (SEQIDNO:1381); CCDS54103.1, Del, A_7, LKKNSLGIIWPKSGLILTA, (SEQIDNO:1382); CCDS3756.1, Del, A_7, LKNFCRVLYILSKCSSTLL, (SEQIDNO:1383); CCDS14502.1, Del, A_7, LLYKLKLEQRGRLLVLLGL, (SEQIDNO:1384); CCDS10239.1, Del, A_7, LSFCNQHVEDSEQDKDLKL, (SEQIDNO:1386); CCDS8253.1, Del, A_7, LSKKKVIPSRKMSNPLKLR, (SEQIDNO:1387); CCDS10622.1, Del, A_7, LTMTPESIQVPNVEMKRMN, (SEQIDNO:1388); CCDS47584.1, Del, A_7, LTWSKWRNPFSPWKLPCIS, (SEQIDNO:1389); CCDS47542.1, Ins, A_7, MEPSTKPTTSGFCVHGRAE, (SEQIDNO:1390); CCDS43483.1, Ins, A_7, MFNPFRGFAKKLQTSYYFE, (SEQIDNO:1391); CCDS46227.1, Del, A_7, MKKTPNLQTMRTIRRTGNY, (SEQIDNO:1392); CCDS5447.1, Del, A_7, MPFLVKLISNRKCRNSITK, (SEQIDNO:1393); CCDS5931.1, Del, A_7, MRIKIMLTLLAHRLLLTQT, (SEQIDNO:1394); CCDS54757.1, Ins, A_7, NAKASLYLRWTACPGWAPQ, (SEQIDNO:1395); CCDS5452.1, Ins, A_7, NEKHEIRKDIGITSTKSLS, (SEQIDNO:1396); CCDS45822.1, Ins, A_7, NGKYSEVGELYLEITSCVE, (SEQIDNO:1397); CCDS4966.1, Ins, A_7, NITGSNFSTNFQALNSFFE, (SEQIDNO: 1398); CCDS53775.1, Ins, A_7, NKGSPNETNERRTRESQTD, (SEQIDNO:1399); CCDS4153.1, Ins, A_7, NKRLNGDRRKSQGSNNCIL, (SEQIDNO:1400); CCDS59214.1, Ins, A_7, NQEKLKEVSYTTPHFASCS, (SEQIDNO:1401); CCDS33993.1, Ins, A_7, NQQDRNRQLSKPLLRNTKK, (SEQIDNO:1402); CCDS10111.1, Ins, A_7, NSREDKRERTATEEKARRN, (SEQIDNO:1403); CCDS30978.1, Ins, A_7, NSSFATHCCQQRTIRGACR, (SEQIDNO:1404); CCDS59085.1, Ins, A_7, NTGSIGENGRYNSSTNQIQ, (SEQIDNO:1405); CCDS671.1, Ins, A_7, PYQHCLNNSTLPFESKSNG, (SEQIDNO:1406); CCDS8438.1, Ins, A_7, QAHFGITHPENGLLSSTSQ, (SEQIDNO:1407); CCDS13414.1, Del, A_7, QCISLLTLTLGCSPPITTT, (SEQIDNO:1408); CCDS3928.1, Del, A_7, QDTLRNLLLQTTLTCWWIH, (SEQIDNO:1409); CCDS47630.1, Del, A_7, QEYADMIESLRLMKLFDSV, (SEQIDNO:1410); CCDS30780.1, Del, A_7, QKLKSTGMLQAVLQITHSF, (SEQIDNO:1411); CCDS45021.1, Ins, A_7, QKQPNFCIPGGSSEKSVTF, (SEQIDNO:1412); CCDS3625.1, Ins, A_7, QRQYWFSPFGQENKSRAII, (SEQIDNO:1413); CCDS9265.1, Del, A_7, QSWAEFLRKPDLLSLSQSQ, (SEQIDNO:1414); CCDS58338.1, Del, A_7, QVWITTGSETTRIPTQLSQ, (SEQIDNO:1415); CCDS42207.1, Ins, A_7, RDSIQFRAEVDGGEMQSED, (SEQIDNO:1416); CCDS34171.1, Del, A_7, REKRKSILLHLIVLNSPES, (SEQIDNO:1417); CCDS53776.1, Ins, A_7, RFREVEKKRKEEEKKATET, (SEQIDNO:1418); CCDS6683.1, Del, A_7, RMISKSLTVKGLMELYHLR, (SEQIDNO:1419); CCDS4306.1, Ins, A_7, RQRKKRKEEESKKGLNQRF, (SEQIDNO:1420); CCDS34723.1, Ins, A_7, RQRYFKRKRYTKSEYYFGL, (SEQIDNO:1421); CCDS7142.1, Ins, A_7, RRGKSERGGRGYGGTKICW, (SEQIDNO:1422); CCDS43042.1, Ins, A_7, RRHRSQMHRTGENWKRFQL, (SEQIDNO:1423); CCDS34472.1, Ins, A_7, RYMLLSKAICVPFNTFQCF, (SEQIDNO:1424); CCDS42794.1, Ins, A_7, SKIKGGRFQAAHSSHSSDL, (SEQIDNO:1426); CCDS34374.1, Del, A_7, SKTNSRLIGKKMKTDSSKA, (SEQIDNO:1427); CCDS4117.1, Del, A_7, SQFRFGKLKSSKKGRKFSS, (SEQIDNO:1428); CCDS47157.1, Ins, A_7, SRKFKSQRIYDSGWPFWIL, (SEQIDNO:1429); CCDS3004.1, Del, A_7, SRWKKIMKFLLALNKIMMR, (SEQIDNO:1430); CCDS54694.1, Ins, A_7, TGFSGKPSPERMAKLGRLP, (SEQIDNO:1431); CCDS3444.1, Del, A_7, THPLLQACSPSACRCPCHQ, (SEQIDNO:1432); CCDS12635.1, Del, A_7, TVEKIHPNVKTVGSATRGA, (SEQIDNO:1433); CCDS1690.1, Del, A_7, VLTNLWNLNGWKDKKKYLG, (SEQIDNO:1434); CCDS43229.1, Ins, A_7, VPEQKPWPGQRVFKRGLLR, (SEQIDNO:1435); CCDS45840.1, Del, A_7, VQDLVTNVLPLEVTVLLGR, (SEQIDNO:1436); CCDS31466.1, Del, A_7, VTTSVWDGPARTNASNSAG, (SEQIDNO:1437); CCDS45703.2, Del, A_7, WRRQSLISRISHFQKLRHI, (SEQIDNO:1438); CCDS2110.1, Ins, A_7, YHGPVCGEESTEALSLFPQ, (SEQIDNO:1439); CCDS9255.2, Ins, A_7, YKSLPRTAVYYPGRQGSGL, (SEQIDNO:1440); CCDS59172.1, Ins, A_7, YSSLKGNYPAGERVCSNII, (SEQIDNO:1441); CCDS14498.1, Ins, A_7, YSSLKGNYPARERVCSNII, (SEQIDNO:1442); CCDS47170.1, Ins, A_7, YTTFFREATNDGSIRSTKK, (SEQIDNO:1443); CCDS47996.1, Del, A_7, AASSWLWASSYLGPSRWD, (SEQIDNO:1445); CCDS13662.1, Del, A_7, ASELRTMLKATGRNSVRN, (SEQIDNO:1446); CCDS5622.1, Ins, A_7, CFRKRKEAARTTEAIGGQ, (SEQIDNO:1447); CCDS3952.1, Del, A_7, CISFNTVLLVSLQNPVKK, (SEQIDNO:1448); CCDS42016.1, Del, A_7, CLCYFCTAIEMTTTESYF, (SEQIDNO:1449); CCDS42041.1, Del, A_7, DVMQIHLKAHQQQKVKKA, (SEQIDNO:1450); CCDS1838.1, Ins, A 7, EELRQCISSSQCCTITRE, (SEQIDNO:1451); CCDS33793.1, Del, A_7, EILAEYCVVGAVHAVAQN, (SEQIDNO: 1452); CCDS46894.1, Ins, A_7, ESFGFGKENRISNFSGVW, (SEQIDNO:1453); CCDS2305.1, Del, A_7, FLEKNPMSLLMTGGKRLP, (SEQIDNO:1455); CCDS46944.1, Del, A_7, FLKKIYGRNFISAVCIGH, (SEQIDNO:1456); CCDS4048.1, Del, A_7, FLLTVNGNVSIHPTKSQL, (SEQIDNO: 1457); CCDS6494.1, Del, A_7, GKRVAQRLYLKVFLAHHH, (SEQIDNO:1458); CCDS54890.1, Ins, A_7, GTAIRTSAEPARTAGLYP, (SEQIDNO:1459); CCDS11867.1, Del, A_7, HFWNSTKKRLRTMKIKCS, (SEQIDNO:1460); CCDS10142.1, Del, A_7, HILQKTSQVRATILLLIT, (SEQIDNO:1461); CCDS45672.1, Del, A_7, HKWLKFCLLRVVKESFHE, (SEQIDNO:1462); CCDS3123.1, Del, A_7, HNGYVLQKKLHFTFYTCL, (SEQIDNO:1463); CCDS34146.2, Del, A_7, HNLSHLKTSLRLNKANRN, (SEQIDNO:1464); CCDS5830.1, Del, A_7, HQRKEILYTDSTRQPGKL, (SEQIDNO:1465); CCDS11475.1, Ins, A_7, HRSHGRSCYYTPISSGKL, (SEQIDNO:1466); CCDS7602.1, Del, A_7, IAEDLINLTMGDLIALVT, (SEQIDNO:1467); CCDS34422.1, Ins, A_7, ILFQNPDSKMWRHCKVSE, (SEQIDNO:1468); CCDS41604.1, Ins, A_7, IPKMWRMWQNLYPVLTLY, (SEQIDNO:1469); CCDS1770.1, Del, A_7, IVLLSQHFHCLLVKNLKL, (SEQIDNO: 1470); CCDS9095.1, Ins, A_7, IWQVCKIKRKTRKNYRHI, (SEQIDNO:1471); CCDS6243.1, Ins, A_7, KAKEKKLQEFRCYQCETV, (SEQIDNO:1472); CCDS2749.2, Del, A_7, KIPILGRMKKFQIVLRLV, (SEQIDNO:1473); CCDS3171.1, Ins, A_7, KKNGKSLHKNRWPGCCSS, (SEQIDNO: 1474); CCDS42366.1, Ins, A_7, KNSKTLGCSGSSHLATIS, (SEQIDNO:1475); CCDS31023.1, Ins, A_7, KQESSHEWYSPCRRHGRY, (SEQIDNO:1476); CCDS8439.1, Ins, A_7, KRGISSRTGHFESKFSPT, (SEQIDNO:1477); CCDS3016.1, Ins, A_7, KRPLCITELSVHILHDYM, (SEQIDNO:1478); CCDS9052.1, Ins, A_7, KTKEEEEQRAFCSRGTGT, (SEQIDNO:1479); CCDS42292.1, Del, A_7, KTKNQWLASVSLRTASVQ, (SEQIDNO:1480); CCDS34470.1, Del, A_7, KVKSSVLQLKLLKKKMRI, (SEQIDNO:1481); CCDS14498.1, Del, A_7, LFPQRKLSSKRKSVFKHH, (SEQIDNO:1482); CCDS59172.1, Del, A_7, LFPQRKLSSRRKSVFKHH, (SEQIDNO:1483); CCDS46878.1, Del, A_7, LKAPMTYLTGTGQVGGKE, (SEQIDNO:1484); CCDS53745.1, Del, A_7, LKVFSFFTLHGGAWLLRL, (SEQIDNO:1485); CCDS47177.1, Del, A_7, LMWRREQRSGCRCWIQMT, (SEQIDNO:1486); CCDS2882.1, Del, A_7, LQFCKQRVKKIMKSGSVQ, (SEQIDNO:1488); CCDS44708.1, Ins, A_7, NCSFPAFYRKLKFSLQFI, (SEQIDNO:1490); CCDS46954.1, Ins, A_7, NDNGHSCKWKNDKQGANS, (SEQIDNO:1491); CCDS14435.1, Ins, A_7, NKEISRFRMHSCPLYGPW, (SEQIDNO:1493); CCDS59290.1, Ins, A_7, NKVFAVWGPLSFLLQLQG, (SEQIDNO:1494); CCDS3146.1, Ins, A_7, NLSSVQAKSCSFSRRFRL, (SEQIDNO:1495); CCDS44839.1, Ins, A_7, NMQCSRVCGNQTNYRCSK, (SEQIDNO:1496); CCDS8321.1, Ins, A_7, NPRNAEVPWVGGDREARH, (SEQIDNO:1497); CCDS691.1, Ins, A_7, NPWNRFIWKSHVGKTQSH, (SEQIDNO:1498); CCDS3228.1, Ins, A_7, NQGGYRGGGKQSFMCNDR, (SEQIDNO:1499); CCDS2528.1, Ins, A_7, NRKEQKIEVQAGQTSRLI, (SEQIDNO:1500); CCDS45262.1, Ins, A_7, NWRTRSGPDGEHIPRTDH, (SEQIDNO:1501); CCDS1113.2, Ins, A_7, NYQEKQWTINENNYPQNK, (SEQIDNO:1502); CCDS47155.1, Ins, A_7, PALPALSQGVYTSDRTDK, (SEQIDNO: 1503); CCDS9344.1, Del, A_7, QDLPLSSICQTNVTIYWQ, (SEQIDNO:1506); CCDS41764.1, Del, A_7, QFNLTMKEAGRFYGSSFI, (SEQIDNO:1507); CCDS53775.1, Ins, A_7, RFREVEKKRKEEEKKCGW, (SEQIDNO:1508); CCDS43513.2, Ins, A_7, RKKSTIYQSRFTRSLSSY, (SEQIDNO:1509); CCDS47238.1, Ins, A_7, RNLRRFRGNEHKLSSYFC, (SEQIDNO:1510); CCDS46789.2, Ins, A_7, RPITKKYSCPRVITEKSL, (SEQIDNO:1511); CCDS6860.1, Ins, A_7, RRKSRENGTRIGGTNQRT, (SEQIDNO:1512); CCDS58090.1, Del, A_7, RRQIFLYQVVLQVTFFPS, (SEQIDNO:1513); CCDS628.2, Del, A_7, SERSSTVTWMWSIGTSKL, (SEQIDNO:1514); CCDS34509.1, Del, A_7, SIHCRRRNQNWLQRKKYS, (SEQIDNO:1515); CCDS35325.1, Ins, A_7, SLSFPFRKQHPEEAKALR, (SEQIDNO:1516); CCDS33365.1, Ins, A_7, SSYVRCSRVSKSDSSNHL, (SEQIDNO:1517); CCDS9693.1, Del, A_7, STLKVMFLLLCQEAVLCV, (SEQIDNO:1518); CCDS47539.1, Del, A_7, STTNQHSTAIREFIGEAI, (SEQIDNO:1519); CCDS43976.1, Del, A_7, SVRMTSAWYPGMWRRQTR, (SEQIDNO:1520); CCDS44954.1, Del, A_7, TAFIWKIWPQTAELLLTS, (SEQIDNO:1521); CCDS45284.1, Del, A_7, TCFPPEEWKVTAQPIPGG, (SEQIDNO: 1522); CCDS4657.1, Del, A_7, AFLMWGVWFNFLHLFSL, (SEQIDNO:1524); CCDS3202.1, Del, A_7, AWKLTSRSPFFVWQLMM, (SEQIDNO:1525); CCDS31523.1, Del, A_7, CLGKIRLSIKYIFILKN, (SEQIDNO:1527); CCDS41774.1, Del, A_7, CRWFQTSPFSPCLLCTS, (SEQIDNO:1528); CCDS1605.1, Del, A_7, DSAKNSSQPIPDTSSSA, (SEQIDNO:1529); CCDS54321.1, Del, A_7, EHVAPGRMSASPQSPTQ, (SEQIDNO:1530); CCDS10916.1, Del, A_7, ERPRAFQPGREDKVASH, (SEQIDNO:1531); CCDS7281.1, Del, A_7, ERVSLSLFPVLERRGLR, (SEQIDNO:1532); CCDS31756.1, Ins, A_7, FRQLPEEGCKHASAIWF, (SEQIDNO:1533); CCDS11915.1, Ins, A_7, GERTSCWRCSRASTKAS, (SEQIDNO:1534); CCDS5106.1, Ins, A_7, GGRRGERGDTAQGPEDQ, (SEQIDNO:1535); CCDS34146.2, Del, A_7, GKEEELRKSCKKKDVRN, (SEQIDNO:1536); CCDS6249.1, Del, A_7, GKGMKKIKKCLHANQQE, (SEQIDNO:1537); CCDS1937.1, Del, A_7, GPTRTVMRSPSSLLPRL, (SEQIDNO:1538); CCDS47542.1, Del, A_7, GTFDETDHFWILCPWKS, (SEQIDNO:1539); CCDS8713.1, Ins, A_7, HRSTKNEKSCGVLDGSK, (SEQIDNO:1540); CCDS5369.1, Ins, A_7, IDLYLLSCINLGVRKSL, (SEQIDNO:1541); CCDS32685.1, Ins, A 7, IKCRCKALPSWPLSDWT, (SEQIDNO:1542); CCDS34531.1, Ins, A_7, IKSRVFGSFGYRQPKGM, (SEQIDNO:1543); CCDS53867.1, Del, A_7, IQSKMPRQQIMTAQWLL, (SEQIDNO:1544); CCDS58336.1, Ins, A_7, IVKSKDTFWTGISTSQI, (SEQIDNO:1545); CCDS31920.1, Ins, A_7, KENALAGQERRGQIPIC, (SEQIDNO:1546); CCDS2749.2, Ins, A_7, KFRVFKACRERNNSRSR, (SEQIDNO:1547); CCDS34100.1, Ins, A_7, KISSGIWPRRQTKTPLQ, (SEQIDNO:1548); CCDS4966.1, Del, A_7, KLIRSQRFRVAILGLNL, (SEQIDNO:1549); CCDS2235.1, Del, A_7, KNLIMNQALEQMKTKAD, (SEQIDNO:1550); CCDS46928.1, Ins, A_7, KQFGTLQRRIKANSRGT, (SEQIDNO:1551); CCDS47225.1, Del, A_7, KSRGKSQSPSLPVLFPS, (SEQIDNO:1552); CCDS33124.1, Ins, A_7, KTKSPAFNFEKQRVLVY, (SEQIDNO: 1553); CCDS30787.1, Ins, A_7, KVSLYYPEAEVFRSFMC, (SEQIDNO: 1554); CCDS46393.1, Del, A_7, LILGFTDCPIQSLIKWL, (SEQIDNO:1555); CCDS31686.1, Del, A_7, LKGHLLLRFSKPQRLKN, (SEQIDNO:1556); CCDS5622.1, Del, A_7, LKLTIKSYKRSMLAFSK, (SEQIDNO:1557); CCDS58737.1, Ins, A_7, NASRFIPSCNTYHKKQN, (SEQIDNO:1558); CCDS43157.1, Ins, A_7, NEESQLEEGARKQEPEP, (SEQIDNO:1559); CCDS4949.1, Ins, A_7, NEKKILFLGGMHEPSGG, (SEQIDNO:1560); CCDS11312.1, Ins, A_7, NKISSSYQNRKACGKQR, (SEQIDNO:1561); CCDS10137.1, Del, A_7, NKKKNFGKGSKRNRKRN, (SEQIDNO:1562); CCDS45436.1, Del, A_7, NPSLFMKKSLHHLIAPQ, (SEQIDNO:1564); CCDS44663.1, Ins, A_7, NQLCAPENHRSHPAQSG, (SEQIDNO:1565); CCDS6147.1, Ins, A_7, NQQKLSCKPADCLHGLV, (SEQIDNO:1566); CCDS33143.1, Ins, A_7, NSRSWNIFRFASFSRHN, (SEQIDNO:1567); CCDS3946.1, Del, A_7, PLKSFTILTVFYQTLKS, (SEQIDNO:1568); CCDS3457.1, Ins, A_7, PSSHRDWRQHTFSFTFL, (SEQIDNO:1569); CCDS10967.1, Del, A_7, QLFEKNRSLILKRKGLR, (SEQIDNO:1570); CCDS44334.1, Del, A_7, QLIVVKIESIMFHWKKL, (SEQIDNO:1571); CCDS6143.1, Del, A_7, QPLKTSSCLILYYQGLI, (SEQIDNO:1572); CCDS10841.1, Del, A_7, QRKPKRANCVIQRRAKM, (SEQIDNO:1573); CCDS5044.1, Del, A_7, QSLTSISATWLWLIWST, (SEQIDNO:1574); CCDS46445.1, Ins, A_7, QSRIKNNCRISSHCDEK, (SEQIDNO:1575); CCDS3892.1, Ins, A_7, RASDLVKRRYQRQHCEL, (SEQIDNO:1576); CCDS43145.1, Del, A_7, REERGAMQEPHSLLREK, (SEQIDNO:1577); CCDS45998.1, Ins, A_7, RGREEGGGGRRPWGRRP, (SEQIDNO: 1578); CCDS41770.1, Del, A 7, RMTSAVLLTVLIPCFPN, (SEQIDNO:1579); CCDS10363.1, Ins, A_7, RPNHKTSICHSRKDLCK, (SEQIDNO:1580); CCDS58066.1, Ins, A_7, RQRKGKTRKGTSIGRRE, (SEQIDNO:1581); CCDS31082.1, Del, A_7, SKRHLKSLTMSRIIQWM, (SEQIDNO:1582); CCDS34047.1, Del, A_7, SSKTIKSLWIERTLRTS, (SEQIDNO:1583); CCDS34034.1, Del, A_7, SVKDLSCSIQDKAFERS, (SEQIDNO:1584); CCDS32782.1, Ins, A_7, TDGREREPLDRPHRLRA, (SEQIDNO:1585); CCDS34725.1, Ins, A_7, TEAVGMQSNQRSRWATE, (SEQIDNO:1586); CCDS58275.1, Ins, A_7, TRKLMPQPEPEKFRDGK, (SEQIDNO:1588); CCDS6253.2, Ins, A_7, TTNTCIYETCIWLRRTL, (SEQIDNO:1589); CCDS2314.1, Del, A_7, TTVFYTLFPVLYDFSMS, (SEQIDNO:1590); CCDS34146.2, Ins, A_7, TWKCWACTSNQAGVCRV, (SEQIDNO:1591); CCDS42796.1, Del, A_7, VIFFSKPKRIKNLSLKT, (SEQIDNO:1592); CCDS41499.1, Del, A_7, WKTRLMYYKGSYLKQKK, (SEQIDNO:1594); CCDS54426.1, Del, A_7, WLIIYKKCKILALTEKI, (SEQIDNO: 1595); CCDS43553.1, Del, A_7, WPWSSLMDRQILVIKRN, (SEQIDNO:1596); CCDS10734.1, Del, A_7, YEHLVCQSRPIKSVSKR, (SEQIDNO:1597); CCDS2447.1, Ins, A_7, YTNPNLPSVQCDGTQPE, (SEQIDNO:1598); CCDS54313.1, Del, A_7, YTTLTVSGEMMKEIATK, (SEQIDNO: 1599); CCDS14426.2, Del, A_7, AKWSSKQHGSEPSRWV, (SEQIDNO:1600); CCDS42374.1, Del, A_7, AQLFIIMQRCLKWWNV, (SEQIDNO:1601); CCDS1554.1, Del, A_7, ARARSRRKVSTNLKRE, (SEQIDNO:1602); CCDS2146.1, Ins, A_7, ASTEKINFTACCSYAP, (SEQIDNO: 1603); CCDS5929.1, Del, A_7, CISKLSMAQSKYQNLM, (SEQIDNO:1604); CCDS34109.2, Del, A_7, CLEARAGMNSVKPKPF, (SEQIDNO:1605); CCDS14186.1, Del, A_7, CSNCWKKCQMEFHLRK, (SEQIDNO:1606); CCDS48200.1, Del, A_7, CTNVDIVTLRLQTHLF, (SEQIDNO:1607); CCDS55390.1, Del, A_7, CTSVDIVTLRLQIHLF, (SEQIDNO:1608); CCDS41644.1, Del, A_7, EGKTRQRRVRPKCHLW, (SEQIDNO:1609); CCDS5549.1, Del, A_7, EKGNGLLQNFCLTNMM, (SEQIDNO:1610); CCDS3227.1, Del, A_7, EKGRLYTLLVIQIPLE, (SEQIDNO:1611); CCDS13265.1, Del, A_7, ELQQWQTIYKREVLDL, (SEQIDNO:1612); CCDS1807.1, Ins, A_7, ERKTTRTERKKRGISF, (SEQIDNO:1613); CCDS3808.1, Del, A_7, ETFVMTQGDFMSALLS, (SEQIDNO:1614); CCDS56405.1, Del, A 7, FCQGKRSLNGKQEKLL, (SEQIDNO:1615); CCDS2259.1, Ins, A_7, FCWTNFGVCSIVQLPL, (SEQIDNO:1616); CCDS7148.1, Del, A_7, FIFFTTFMLVWLKRRN, (SEQIDNO:1617); CCDS31946.1, Ins, A_7, FNGYYENKRVFPEVWI, (SEQIDNO:1618); CCDS47904.1, Del, A_7, FQAVLQVFPGQIILAL, (SEQIDNO:1619); CCDS44410.1, Del, A_7, FRLSHRAVFPVQSCLL, (SEQIDNO:1620); CCDS9344.1, Del, A_7, FSMKQTLMNVKNLKTK, (SEQIDNO:1621); CCDS9755.1, Del, A_7, FTELKLQKNLRTQVIV, (SEQIDNO:1622); CCDS34884.1, Del, A_7, FYPIILEFTVIVAQEL, (SEQIDNO:1623); CCDS1937.1, Ins, A_7, GDRQEEEEKKRGPRGL, (SEQIDNO:1624); CCDS7095.1, Del, A_7, GENNLPIWNLRNFRKS, (SEQIDNO:1625); CCDS42127.1, Ins, A_7, GGPGPHSLLGPGPLVL, (SEQIDNO:1626); CCDS5034.1, Del, A_7, GKDRYCSNIDKHWLNS, (SEQIDNO:1627); CCDS12269.2, Del, A_7, GKIRKLKMSTKIPGET, (SEQIDNO:1628); CCDS42505.1, Del, A_7, GKTRALKIGTKIRGEF, (SEQIDNO:1629); CCDS58578.1, Ins, A_7, GLTDPSGIFRISNKTT, (SEQIDNO:1630); CCDS35457.1, Del, A_7, GRKKTWKAWEIKPSKL, (SEQIDNO:1631); CCDS45984.2, Del, A_7, GTTSTLKMSTKILGET, (SEQIDNO:1632); CCDS8311.1, Ins, A_7, ICSSSKLPTATSLHLV, (SEQIDNO:1633); CCDS47751.2, Del, A_7, IGKTCWRPDCTSLAEN, (SEQIDNO:1634); CCDS45107.1, Del, A_7, IGLTMLSGGKRRELGF, (SEQIDNO:1635); CCDS10654.1, Ins, A_7, IPPSQWGPPPSAAAAG, (SEQIDNO:1637); CCDS12639.1, Del, A_7, IQLQSRGFLMKNQLME, (SEQIDNO:1638); CCDS44954.1, Ins, A_7, IQRSFGRYGHKQPSFY, (SEQIDNO:1639); CCDS34743.1, Ins, A_7, ISKSSYRPFGPSTKEI, (SEQIDNO: 1640); CCDS34101.1, Del, A_7, ISSGIWPRRQTKTPLQ, (SEQIDNO:1641); CCDS44402.1, Del, A_7, IYAQMFQNFKLCALQK, (SEQIDNO: 1642); CCDS32133.1, Del, A_7, KELNRGNYYPDCKSTQ, (SEQIDNO: 1643); CCDS31027.1, Del, A_7, KKILLIGILRSSQSQK, (SEQIDNO:1644); CCDS45045.1, Del, A_7, KKMSWSLLLKRTFGMV, (SEQIDNO:1645); CCDS3920.1, Ins, A_7, KKTRFLPTGGWGCYRR, (SEQIDNO:1646); CCDS53824.1, Ins, A_7, KQNQYGCETRTILFEA, (SEQIDNO:1647); CCDS56056.1, Del, A_7, KSCQKHWRRWPLNIMN, (SEQIDNO: 1648); CCDS43426.1, Del, A_7, KSYRLVLKELRNPLKV, (SEQIDNO:1649); CCDS3738.1, Ins, A_7, KTSASPSEIEGISVCP, (SEQIDNO:1650); CCDS34506.1, Del, A_7, LDPKVPVLMYWELLLI, (SEQIDNO:1651); CCDS6953.1, Del, A_7, LKAVESPIFGVLSFSS, (SEQIDNO:1652); CCDS12414.2, Del, A_7, LLNIKNLANHFAYFQT, (SEQIDNO:1653); CCDS58923.1, Del, A_7, LNCGRPRKLLSIWGTT, (SEQIDNO:1654); CCDS2644.1, Del, A_7, LPIMPCWSPCLAGSLW, (SEQIDNO:1655); CCDS5228.1, Del, A_7, LPSSRFLQLSRQVTTT, (SEQIDNO:1656); CCDS55255.1, Del, A_7, LRLKTKQNQLPLILRR, (SEQIDNO:1657); CCDS5382.1, Del, A_7, LSKTQTLKSRYLHCRN, (SEQIDNO:1658); CCDS31066.1, Del, A_7, LTSRLRRTGRITWVRL, (SEQIDNO:1659); CCDS13715.1, Del, A_7, MACLKKKTRMLDLKTT, (SEQIDNO:1660); CCDS9543.1, Del, A_7, MPRLEASKMIQNIRSF, (SEQIDNO:1661); CCDS2194.1, Del, A_7, NDVRKKKNLFRRVHCI, (SEQIDNO:1662); CCDS9311.1, Ins, A_7, NERNAPKWSQSICWTI, (SEQIDNO:1663); CCDS4518.1, Ins, A_7, NESDYFSSCISSCFYV, (SEQIDNO:1664); CCDS33641.1, Ins, A 7, NETSLCEHRDTDPSGA, (SEQIDNO:1665); CCDS10142.1, Ins, A_7, NISSRKQARSEQLFFC, (SEQIDNO:1666); CCDS4278.1, Ins, A_7, NKRNSAGHYRSLTRNL, (SEQIDNO:1667); CCDS46851.1, Ins, A_7, NKTATGPHRREGDTGR, (SEQIDNO:1668); CCDS53353.1, Ins, A_7, NPESVEASWQRSTLHP, (SEQIDNO:1669); CCDS3467.1, Ins, A 7, NPHSRLFSRAQTPMAI, (SEQIDNO:1670); CCDS7480.1, Ins, A_7, NQARNLCNGRHSRTCQ, (SEQIDNO:1671); CCDS10265.1, Ins, A 7, NQGEGQRKQVPVCLDV, (SEQIDNO:1672); CCDS1377.1, Ins, A_7, NQITPKAVLKSKENIY, (SEQIDNO:1673); CCDS9014.2, Ins, A_7, NRRWCKNIYYLSLPFA, (SEQIDNO:1674); CCDS2261.1, Ins, A_7, NSHSFRKDLAPRPCSL, (SEQIDNO:1675); CCDS899.1, Ins, A_7, NSRTSVWYSDLERPLH, (SEQIDNO:1676); CCDS45279.1, Ins, A_7, NSSWKFFIWPGGPYDG, (SEQIDNO:1677); CCDS55317.1, Ins, A_7, NVCTPFYSYNQPKFSQ, (SEQIDNO:1678); CCDS3952.1, Ins, A_7, NVFHSTRCCWCHYKIQ, (SEQIDNO:1679); CCDS7273.1, Ins, A_7, NYSSNWSWGVCFMWNT, (SEQIDNO:1680); CCDS695.1, Ins, A_7, NYSTVHRKRFHRPDCY, (SEQIDNO:1681); CCDS5944.1, Ins, A_7, NYTVPQTRRLRKRICS, (SEQIDNO:1682); CCDS8253.1, Del, A_7, PKQTCLKGGQQEQGNV, (SEQIDNO:1683); CCDS58957.1, Del, A_7, PSRPRHCLLQAGFQTC, (SEQIDNO:1684); CCDS31347.1, Ins, A_7, QPCGGCIQLPHRQKDQ, (SEQIDNO:1687); CCDS14435.1, Del, A_7, QSLKSIKKSKAETEER, (SEQIDNO: 1688); CCDS54969.1, Del, A_7, QSTPSWITWMNRKEWN, (SEQIDNO: 1689); CCDS46299.1, Del, A_7, QVYLLLLNLDTHIVEI, (SEQIDNO:1690); CCDS1240.1, Ins, A_7, RECNLFSARNAPRHEF, (SEQIDNO:1691); CCDS34521.1, Del, A_7, RKKKRKKMKKKTILTT, (SEQIDNO:1692); CCDS45650.1, Ins, A_7, RNRKLHRKIAYIPLLL, (SEQIDNO:1693); CCDS53822.1, Ins, A_7, RRKDFGIVANLLSKVS, (SEQIDNO:1694); CCDS34204.1, Ins, A_7, RSSFWCGKFKEEKSKS, (SEQIDNO:1695); CCDS42415.1, Del, A_7, RSWSSPKSSSSSWWSR, (SEQIDNO:1696); CCDS8336.2, Ins, A_7, RTSTGYKVYICWQSRA, (SEQIDNO:1697); CCDS2382.1, Del, A_7, SAITAVEWSFRITATG, (SEQIDNO:1698); CCDS4993.1, Del, A_7, SCFQRRKRRIYQHLRY, (SEQIDNO:1699); CCDS30744.1, Ins, A_7, SFEEKQWGTKWILCGN, (SEQIDNO:1700); CCDS43930.1, Ins, A_7, SHQCAADRNGREQRAR, (SEQIDNO:1701); CCDS31878.1, Del, A_7, SQLLSKKYQQTQRILL, (SEQIDNO:1702); CCDS56590.1, Del, A_7, SRNHQRPAARNLTSEE, (SEQIDNO:1703); CCDS9344.1, Ins, A_7, SRRNYFNFKNNCQSRL, (SEQIDNO:1704); CCDS13029.2, Del, A_7, SSLPPCGASPGPWRRS, (SEQIDNO:1705); CCDS12436.1, Del, A_7, TAAVAAAYLAWATHLK, (SEQIDNO:1706); CCDS43523.1, Del, A_7, TAVTLMVTSMVPGATQ, (SEQIDNO:1707); CCDS34287.1, Ins, A_7, TCEDRCCEIHAKKPHE, (SEQIDNO:1708); CCDS1389.1, Ins, A_7, TDSGTAPGCHYYSEAL, (SEQIDNO:1709); CCDS43230.1, Ins, A_7, TEGVKEAQAPCPGSTT, (SEQIDNO:1710); CCDS9201.1, Ins, A_7, TETEAPVQHLSRPHQV, (SEQIDNO:1711); CCDS41499.1, Ins, A_7, TIYVRGFTGGYVTLSY, (SEQIDNO:1712); CCDS11684.1, Del, A_7, TMKWWELYLVILSHIA, (SEQIDNO:1713); CCDS54105.1, Ins, A_7, TTSSLLHSDCHQHSPG, (SEQIDNO: 1714); CCDS6195.1, Ins, A_7, TVSCYGPEDSCQENIW, (SEQIDNO:1715); CCDS5944.1, Ins, A_7, VHRNQPPHPVLIQHQH, (SEQIDNO:1716); CCDS4955.1, Del, A_7, VLHPGHFPLQVWLLTE, (SEQIDNO:1717); CCDS11374.1, Del, A_7, VMRRKWKSCNIQLGGT, (SEQIDNO:1718); CCDS5232.1, Ins, A_7, VRYQHAVSIKCCSLPR, (SEQIDNO:1719); CCDS32800.1, Del, A_7, VTIRIQKQKFRILKRI, (SEQIDNO:1720); CCDS34743.1, Del, A_7, WSRTVGQCPALDEMHC, (SEQIDNO:1722); CCDS44737.1, Ins, A 7, YCHCYFPGPAFQTLSH, (SEQIDNO:1723); CCDS59217.1, Del, A_7, YIKMLIGRSGRYIKKR, (SEQIDNO:1724); CCDS4348.1, Del, A_7, CRMKRNRKEEKRALD, (SEQIDNO:1727); CCDS9899.1, Del, A_7, EFINLKMIKWTLLKS, (SEQIDNO:1728); CCDS10168.2, Del, A_7, EIKTTEGKRSCLLKR, (SEQIDNO:1729); CCDS11995.1, Del, A_7, ENLKGESLCQNKEVK, (SEQIDNO:1730); CCDS12436.1, Ins, A_7, EQRQWRRLIWHGPHT, (SEQIDNO:1731); CCDS9493.1, Ins, A_7, ERRVQGRNRTICEQT, (SEQIDNO:1732); CCDS58395.1, Ins, A_7, ESLFHVQEESGTYWV, (SEQIDNO:1733); CCDS2557.1, Del, A_7, EWLILKGLEENLLGI, (SEQIDNO:1734); CCDS54424.1, Del, A_7, FLQKSLRWKNLSQPK, (SEQIDNO:1735); CCDS44689.1, Del, A_7, FRVLNLETLAIYLLQ, (SEQIDNO:1736); CCDS45663.1, Ins, A_7, GEEEKEGEEKEAQET, (SEQIDNO:1737); CCDS41715.1, Del, A_7, GHTKNRRKAKRKPQI, (SEQIDNO:1738); CCDS32600.1, Del, A_7, GKLGAIVPRVVKTLI, (SEQIDNO:1739); CCDS35215.2, Del, A_7, GKRRKILRTGRKLSV, (SEQIDNO:1740); CCDS56440.1, Del, A_7, GLLLANGSLLLLSLS, (SEQIDNO:1741); CCDS8300.1, Del, A_7, GLPQCGDSFLKRPLT, (SEQIDNO:1742); CCDS7128.1, Del, A 7, GMRLGSGTGMFTSAN, (SEQIDNO:1743); CCDS42035.1, Del, A_7, HLTLARMKHFIYFKH, (SEQIDNO:1746); CCDS5447.1, Del, A_7, HPSPRLLLQSDSSHL, (SEQIDNO:1747); CCDS54379.1, Del, A_7, KEEMLKRCTKKLGKS, (SEQIDNO:1748); CCDS1167.1, Ins, A_7, KEPEISDTCTESPCV, (SEQIDNO:1749); CCDS8530.1, Ins, A_7, KIPRFQGPHDGAENL, (SEQIDNO:1750); CCDS1332.1, Del, A_7, KKSHNRRFARSLIWW, (SEQIDNO:1751); CCDS33793.1, Ins, A_7, KKSLQSTVLLVLSML, (SEQIDNO:1752); CCDS59374.1, Ins, A 7, KQAQINDLSTSYTVL, (SEQIDNO:1753); CCDS46430.1, Del, A_7, KRNKQSSCEREIGKP, (SEQIDNO:1754); CCDS33893.1, Ins, A 7, KRVVFCPSEGDRHRL, (SEQIDNO:1755); CCDS47852.1, Ins, A_7, KTCRAEDGSGFATHP, (SEQIDNO: 1756); CCDS55858.1, Del, A_7, KTIIINFKCRSLQIF, (SEQIDNO:1757); CCDS9436.1, Ins, A_7, LCCCPRNFYRRTKIR, (SEQIDNO:1758); CCDS44279.1, Ins, A 7, LCRPGFQWRLYSLWK, (SEQIDNO:1759); CCDS3505.1, Del, A_7, LEYCQTTLKAKELFL, (SEQIDNO: 1760); CCDS33351.2, Del, A_7, LFKNTLRRKKISEEG, (SEQIDNO:1761); CCDS7103.1, Del, A_7, LLEKQLELLDLMFCD, (SEQIDNO:1762); CCDS34195.1, Ins, A_7, LLTPGGELPHGILGQ, (SEQIDNO:1763); CCDS2847.1, Del, A_7, LMRKRMTTPIEFLKL, (SEQIDNO:1764); CCDS44611.1, Del, A 7, LNRMKVPLMKLITRV, (SEQIDNO:1765); CCDS11681.1, Del, A_7, LPQEMSLFVLKKVKL, (SEQIDNO:1766); CCDS6947.1, Del, A_7, LSLNSKKNVKTRRIL, (SEQIDNO:1767); CCDS10176.1, Del, A_7, LSNEYIKNLIQLERF, (SEQIDNO: 1768); CCDS58709.1, Del, A_7, LTFPQKTFIRTSTGC, (SEQIDNO:1769); CCDS6518.1, Ins, A_7, LYLDSSRYQERCYYT, (SEQIDNO:1770); CCDS3087.1, Ins, A_7, MHRHHKTMHKEKIRE, (SEQIDNO:1773); CCDS2290.1, Del, A_7, MKVLYPKQWSTCLAG, (SEQIDNO:1774); CCDS8342.1, Ins, A_7, NDQSCRSKKSNEEKF, (SEQIDNO:1776); CCDS35409.1, Ins, A_7, NEISIFRKHKDNKNG, (SEQIDNO:1777); CCDS41765.1, Ins, A_7, NFKFGKRVGCLSEGS, (SEQIDNO:1778); CCDS53846.1, Ins, A_7, NFQIKQNPVSGRLQN, (SEQIDNO:1779); CCDS5776.1, Ins, A_7, NFSHPKCQCIRKPSR, (SEQIDNO:1780); CCDS47155.1, Ins, A_7, NGGTGEGLWPLLGGQ, (SEQIDNO:1781); CCDS34680.1, Del, A 7, NILLIWASHMDQLFK, (SEQIDNO:1782); CCDS41619.1, Ins, A_7, NMEESWHCLFGLSYL, (SEQIDNO:1783); CCDS56400.1, Ins, A_7, NPVLDSWRGVFVRYQ, (SEQIDNO:1784); CCDS2234.1, Ins, A_7, NQSSKRCFRRQTPRT, (SEQIDNO:1785); CCDS41887.1, Ins, A_7, NSCISTKVAAVEGRD, (SEQIDNO:1786); CCDS13665.1, Ins, A_7, NTELGKTTEVELQSL, (SEQIDNO:1787); CCDS41961.1, Ins, A 7, NTPCKDQQTKNQRER, (SEQIDNO:1788); CCDS33310.1, Ins, A_7, NYQGDDESVRYRQFS, (SEQIDNO:1789); CCDS13697.1, Ins, A 7, PGKERARIRAAENGL, (SEQIDNO:1790); CCDS2130.1, Del, A_7, PKDKSCLIRLCTTWT, (SEQIDNO:1791); CCDS3198.1, Del, A_7, PRFWWVLIKMKGQLF, (SEQIDNO:1792); CCDS1508.1, Ins, A_7, PSCCSEKSEEADPEG, (SEQIDNO:1793); CCDS47630.1, Del, A_7, QVCILTKSQNWKWKA, (SEQIDNO:1795); CCDS43313.1, Ins, A_7, RKEEALRMPGLQQCV, (SEQIDNO:1796); CCDS4844.1, Ins, A_7, RKEESSGGSPLGWHG, (SEQIDNO:1797); CCDS11494.1, Del, A_7, RKKKAVRQIQPRISL, (SEQIDNO:1798); CCDS32234.1, Ins, A_7, RKREGNCKTKTTHST, (SEQIDNO: 1799); CCDS46338.1, Del, A_7, RMRIWMRLKPTFQSL, (SEQIDNO:1800); CCDS9201.1, Del, A_7, RNRSSCSAPQSSTPS, (SEQIDNO:1801); CCDS5549.1, Ins, A_7, RRKEMGSSKISASQI, (SEQIDNO:1802); CCDS43242.1, Del, A_7, RWLGSWKFCDLRNAV, (SEQIDNO:1803); CCDS3882.1, Del, A_7, SCRKLIRLSWSYSIH, (SEQIDNO:1805); CCDS31874.1, Ins, A_7, SDLDRKLAEKRTRLY, (SEQIDNO:1806); CCDS56144.1, Ins, A_7, SGTINDWSKRREINN, (SEQIDNO:1807); CCDS1917.1, Ins, A_7, SIRRCSTTIWAWDRI, (SEQIDNO:1808); CCDS645.1, Del, A_7, SISLTKASILKDQWK, (SEQIDNO:1809); CCDS6766.1, Del, A_7, SNTCFRSLHCAGLSL, (SEQIDNO:1810); CCDS54856.1, Ins, A_7, SREKRSCLRVGPTSC, (SEQIDNO:1811); CCDS14399.1, Del, A_7, SRFQLTRSQMLSCIH, (SEQIDNO:1812); CCDS33506.1, Del, A_7, SRPLRDFTSRARPRR, (SEQIDNO:1813); CCDS3228.1, Del, A_7, SRRLPRRWQTKFHVQ, (SEQIDNO:1814); CCDS35201.1, Ins, A_7, TAGDQNGRDGGGGQW, (SEQIDNO:1816); CCDS2707.1, Ins, A_7, TGAEGIWIPFSARGP, (SEQIDNO:1817); CCDS5734.1, Del, A_7, TKRQAIISLGQNHFH, (SEQIDNO: 1818); CCDS33205.1, Del, A_7, TLLASLSRRHLTQLV, (SEQIDNO:1819); CCDS2135.1, Ins, A_7, TRTTYSWSSLCAPPT, (SEQIDNO:1820); CCDS46919.1, Ins, A_7, TVHISVIISESSRTY, (SEQIDNO:1821); CCDS42366.1, Del, A 7, VLLENLVYRNFGSGA, (SEQIDNO:1822); CCDS44942.1, Del, A_7, VLSLARVSPVNTELN, (SEQIDNO:1823); CCDS47218.1, Del, A_7, VLYRMMLLQCQGKHL, (SEQIDNO:1824); CCDS47445.1, Del, A_7, VRKQLTTVNCSASTV, (SEQIDNO:1825); CCDS47573.1, Ins, A_7, VSFTKTVFLHLLQKM, (SEQIDNO:1826); CCDS6049.1, Ins, A_7, WKSHTILQCGQLCRN, (SEQIDNO:1827); CCDS31071.1, Ins, A_7, WNSYSYGQKLSFQDT, (SEQIDNO:1828); CCDS4072.1, Ins, A_7, YCTKDHSEGSSPFSR, (SEQIDNO:1829); CCDS3096.1, Del, A_7, YFGMNLKMSPSRTLH, (SEQIDNO:1830); CCDS54820.1, Del, A_7, YGPQPVAMQHFRTRD, (SEQIDNO:1832); CCDS33332.1, Del, A_7, YLKKSWKKKLTRRLN, (SEQIDNO:1833); CCDS3200.1, Ins, A_7, YRICSGSLSRGFLIS, (SEQIDNO:1834); CCDS14501.1, Del, A_7, YSVPKLFQYLWVSLT, (SEQIDNO:1835); CCDS9508.1, Ins, A_7, AESCPKDEESYWNY, (SEQIDNO:1837); CCDS5011.1, Ins, A_7, AFSASIPNAEDHGI, (SEQIDNO:1838); CCDS46809.1, Ins, A_7, CGGVPEVPRVDDTL, (SEQIDNO:1839); CCDS1842.1, Del, A 7, CHQEKHLSISWRPR, (SEQIDNO:1840); CCDS9416.2, Del, A_7, CLCLVNHTKIQQKL, (SEQIDNO:1841); CCDS34913.1, Del, A_7, CLINTCFPWPWRKM, (SEQIDNO:1842); CCDS3127.1, Del, A_7, CLQLQNSLSPPHSL, (SEQIDNO:1843); CCDS46348.1, Del, A 7, CSLLILICDQLFLK, (SEQIDNO:1844); CCDS3973.2, Del, A_7, DITITLELRTIWIV, (SEQIDNO:1845); CCDS43655.2, Del, A_7, DLKRMMMRNQENVS, (SEQIDNO:1846); CCDS42791.1, Ins, A_7, EGEDYRRQNFRFEE, (SEQIDNO:1847); CCDS8438.1, Del, A_7, EGNLIQNRSPTPSE, (SEQIDNO:1848); CCDS54659.1, Del, A_7, EKEYGRKTLKKKVT, (SEQIDNO:1849); CCDS10517.1, Ins, A_7, ELWGPRKGEPNENG, (SEQIDNO:1850); CCDS43658.1, Del, A_7, EWQQPNNVLGRSLS, (SEQIDNO:1851); CCDS11625.1, Ins, A_7, FEDVNSWPNESPRN, (SEQIDNO:1852); CCDS3591.1, Del, A_7, FLLVAFLQIHLKRK, (SEQIDNO:1853); CCDS34494.2, Del, A_7, FRIWSDKLRKWKGF, (SEQIDNO:1854); CCDS44337.1, Ins, A_7, FRTFYKQRRQSGTK, (SEQIDNO:1855); CCDS43758.1, Del, A_7, FYKTSNLEIPFMML, (SEQIDNO:1856); CCDS13900.2, Del, A_7, GASPATCLPCRGSS, (SEQIDNO:1857); CCDS47037.1, Del, A_7, GGRMRNIFTPAGHK, (SEQIDNO:1858); CCDS9372.1, Del, A 7, GKIYSQGKDLIFLT, (SEQIDNO:1859); CCDS6243.1, Del, A_7, GKRKKATRIQVLSV, (SEQIDNO:1860); CCDS41958.1, Ins, A_7, GKRMYGWNFRVRGG, (SEQIDNO:1861); CCDS31735.1, Ins, A 7, GLETSLSWRSNALP, (SEQIDNO:1862); CCDS6839.1, Del, A_7, GLLHILEQHGNLED, (SEQIDNO:1863); CCDS2955.1, Ins, A_7, GNSSFKPRKTGAVQ, (SEQIDNO: 1864); CCDS5046.1, Ins, A_7, GWRRFRSFRRTYVL, (SEQIDNO: 1865); CCDS30975.1, Ins, A_7, HLRGPCLQSRGDRI, (SEQIDNO:1866); CCDS41766.2, Del, A_7, HNYRRLNLSKSQSL, (SEQIDNO:1867); CCDS47539.1, Ins, A_7, ILPQISIQQPSENS, (SEQIDNO:1869); CCDS58039.1, Ins, A_7, IQEHCSTKRIRGHQ, (SEQIDNO:1870); CCDS34158.1, Del, A_7, IRNTSQFRLEHMLI, (SEQIDNO:1871); CCDS2749.2, Del, A_7, ISKIEGLLKKGGRK, (SEQIDNO:1872); CCDS11423.1, Del, A_7, ISSLIGMMGPFWGL, (SEQIDNO:1873); CCDS43341.1, Ins, A_7, ITEIVFQKYQIYKK, (SEQIDNO:1874); CCDS3593.1, Ins, A_7, IVRKQIPSNWFWEV, (SEQIDNO:1875); CCDS47859.1, Del, A_7, KASSPATMWPNSTP, (SEQIDNO:1876); CCDS7585.1, Ins, A_7, KEFTLCCRLLQSQD, (SEQIDNO: 1877); CCDS7258.1, Ins, A_7, KETPQTVPGSEEKV, (SEQIDNO: 1878); CCDS47063.1, Ins, A_7, KFFNIVFRNSKTKK, (SEQIDNO:1879); CCDS5034.1, Ins, A 7, KGKTDTVPTSTSTG, (SEQIDNO:1880); CCDS4224.1, Ins, A_7, KGQCGDNSQHQSEK, (SEQIDNO:1881); CCDS46392.1, Del, A_7, KILKLVTRMANRRK, (SEQIDNO:1882); CCDS11270.1, Ins, A 7, KKGCKLSNKASSHR, (SEQIDNO:1883); CCDS6286.1, Ins, A_7, KKNFNRLPVSEEKR, (SEQIDNO:1884); CCDS3356.1, Ins, A_7, KKPHKEDTGPYRRC, (SEQIDNO:1885); CCDS8740.1, Del, A_7, KKRKLLNQKKKLRK, (SEQIDNO:1886); CCDS987.1, Ins, A_7, KKSRGPPSQLHSRG, (SEQIDNO:1887); CCDS1518.1, Ins, A_7, KKVENNQTRTKNPR, (SEQIDNO:1888); CCDS7775.1, Ins, A_7, KMDNGERNTKEDYF, (SEQIDNO:1889); CCDS4835.1, Ins, A_7, KSCRACPEPQLHVR, (SEQIDNO:1890); CCDS46430.1, Ins, A 7, KTAIRVSFCFRTFF, (SEQIDNO:1891); CCDS660.1, Ins, A_7, KTNKITVTRKPGDW, (SEQIDNO:1892); CCDS1533.1, Ins, A_7, KVYQHQVFCFHGDG, (SEQIDNO:1893); CCDS4955.1, Ins, A_7, LCCILGIFHCKCGC, (SEQIDNO: 1894); CCDS58101.1, Del, A_7, LHQRTASGRSGPSW, (SEQIDNO:1895); CCDS31757.1, Del, A_7, LIKPDSWKTVCLTG, (SEQIDNO:1896); CCDS33770.2, Del, A_7, LKWFHLSQLKINLS, (SEQIDNO:1897); CCDS8652.1, Del, A_7, LLGPLTQIHQILLS, (SEQIDNO:1898); CCDS31976.1, Del, A_7, LLPILWVCTFSRPA, (SEQIDNO:1899); CCDS30956.1, Ins, A_7, LQCQHLGPPKYWWF, (SEQIDNO:1901); CCDS46928.1, Del, A_7, LQISLPMKDHHLFL, (SEQIDNO:1902); CCDS42794.1, Del, A_7, LRISGAQLRDFLVT, (SEQIDNO:1903); CCDS55971.1, Del, A_7, LVLSTKKIMTAKDL, (SEQIDNO:1904); CCDS6872.1, Del, A_7, LVMTLPRQRVTGRA, (SEQIDNO:1905); CCDS34146.2, Del, A_7, MEVLGLHLKPSRCV, (SEQIDNO:1906); CCDS34042.1, Del, A_7, MSMRHRKKCLKWSN, (SEQIDNO:1907); CCDS7799.2, Ins, A_7, NADCFTRTCYKSNC, (SEQIDNO:1908); CCDS33716.1, Ins, A_7, NCSPKKNAQICQFQ, (SEQIDNO:1909); CCDS1121.1, Ins, A_7, NDTQAWASYPRRQM, (SEQIDNO:1910); CCDS8342.1, Ins, A_7, NIERFARSSVPFGD, (SEQIDNO: 1911); CCDS3505.1, Ins, A_7, NIRNRRMGGVFRIL, (SEQIDNO:1912); CCDS58490.1, Ins, A_7, NIVGSNTTTENGIV, (SEQIDNO:1913); CCDS2222.1, Ins, A_7, NLLFSKKIWFIFFY, (SEQIDNO:1914); CCDS34333.1, Del, A_7, NMVFEKEENIYITL, (SEQIDNO:1915); CCDS7123.1, Ins, A_7, NPEGCSDHLQEGRR, (SEQIDNO: 1917); CCDS53528.1, Ins, A_7, NQAVRAKCWEPVWG, (SEQIDNO: 1918); CCDS9492.1, Ins, A_7, NQLYHPLFNKFGDF, (SEQIDNO:1919); CCDS7383.1, Ins, A_7, NSAFIQSTFFISEG, (SEQIDNO:1920); CCDS4562.1, Ins, A_7, NSHYSPPPAASDPQ, (SEQIDNO:1921); CCDS1628.1, Ins, A_7, NSWKKKSKKQKRDC, (SEQIDNO:1922); CCDS43426.1, Ins, A_7, NTKIPTEKSSSGKT, (SEQIDNO:1923); CCDS45057.1, Ins, A 7, NVSRQPAASYLHNK, (SEQIDNO:1924); CCDS2664.1, Ins, A_7, NWGPRETERIHCLP, (SEQIDNO:1925); CCDS33264.1, Del, A_7, PIKKNPSMKSFIFV, (SEQIDNO:1926); CCDS6597.1, Ins, A_7, PKVHTQTTGLPDDF, (SEQIDNO:1927); CCDS45984.2, Del, A_7, PMNVSSVGKHYLIL, (SEQIDNO:1928); CCDS34290.1, Del, A_7, PSSGHANLWWMVWR, (SEQIDNO:1929); CCDS41302.1, Del, A_7, PWKNSESMKKVKCI, (SEQIDNO:1930); CCDS43729.1, Del, A_7, QENPLPTNTSKLTK, (SEQIDNO:1931); CCDS2251.1, Ins, A_7, REINHSKQLPQKFC, (SEQIDNO:1932); CCDS41470.1, Del, A_7, RKFWMTQLKIFVLC, (SEQIDNO:1933); CCDS58996.1, Ins, A_7, RKRSHSQYHLSPPG, (SEQIDNO:1934); CCDS5611.1, Del, A_7, SCLFQSPSPTLQMN, (SEQIDNO: 1936); CCDS8336.2, Ins, A_7, SKERKEKKEQETEI, (SEQIDNO:1937); CCDS4966.1, Ins, A_7, SKSCCRSSSQTFRQ, (SEQIDNO:1938); CCDS45399.1, Del, A_7, SLNQRSYARPSCQP, (SEQIDNO:1939); CCDS41630.1, Ins, A 7, SSLRLDKVQSHMSI, (SEQIDNO:1940); CCDS9244.1, Del, A_7, SYLKNCGSQVKRST, (SEQIDNO:1941); CCDS56533.1, Del, A 7, SYMCWRRSSILNKK, (SEQIDNO:1942); CCDS13571.1, Ins, A 7, TGSSRHHPKDCWRK, (SEQIDNO:1943); CCDS9697.1, Del, A_7, TGWQPLFLFIIVVL, (SEQIDNO:1944); CCDS41292.1, Ins, A_7, TIKEGRKTYNCRGG, (SEQIDNO:1945); CCDS14501.1, Ins, A_7, TIQCQSSFNICGSL, (SEQIDNO:1946); CCDS54838.1, Ins, A_7, TQSSTTSYQSYPVE, (SEQIDNO:1948); CCDS11194.1, Del, A_7, TRPRGSPTCCQTVA, (SEQIDNO:1949); CCDS34478.1, Del, A_7, TRQFRGWISMISHH, (SEQIDNO:1950); CCDS4098.1, Ins, A_7, TRRLFGARESWETW, (SEQIDNO:1951); CCDS5510.1, Del, A_7, TTNLSVHQGLHMVV, (SEQIDNO:1952); CCDS35118.1, Ins, A_7, VGTNQKGFSSSRRK, (SEQIDNO:1953); CCDS47885.1, Ins, A 7, VKKQYPVERCRCWL, (SEQIDNO:1954); CCDS6343.1, Del, A_7, VKTTTYFSWKICMQ, (SEQIDNO:1955); CCDS4973.1, Del, A_7, VPALKKLRSPLWIF, (SEQIDNO:1956); CCDS10171.1, Del, A_7, VQKHSGKFRISLSF, (SEQIDNO:1957); CCDS8019.1, Del, A_7, WEPWVLLPWTAKGM, (SEQIDNO:1958); CCDS54564.1, Del, A_7, WKKLSRKQRRCKKR, (SEQIDNO:1959); CCDS42686.1, Del, A_7, WLILMVKNLDVKRS, (SEQIDNO:1960); CCDS33167.1, Ins, A_7, YAPFKRWIENDFDL, (SEQIDNO:1961); CCDS14186.1, Ins, A_7, YARIAGRNAKWSST, (SEQIDNO:1962); CCDS14782.1, Ins, A 7, YEYYKGFVGSSCRS, (SEQIDNO:1963); CCDS34086.1, Ins, A_7, YHQRPGRGIHKDSG, (SEQIDNO:1964); CCDS34209.1, Ins, A_7, YNKAECGAKVPCGL, (SEQIDNO:1965); CCDS5611.1, Ins, A_7, APVYFRAHPPPFK, (SEQIDNO:1966); CCDS4959.1, Del, A_7, AQPKTVPSNQILR, (SEQIDNO:1967); CCDS32710.1, Ins, A_7, ASQCCSRNGTGKV, (SEQIDNO:1968); CCDS47973.1, Del, A_7, ASQHQSLLRAKKQ, (SEQIDNO: 1969); CCDS41655.1, Ins, A_7, CESVTTGTAQTGH, (SEQIDNO:1970); CCDS43349.1, Del, A_7, CFMLEKTENKIVW, (SEQIDNO:1971); CCDS31064.1, Del, A_7, CKRETIFVITRLI, (SEQIDNO:1972); CCDS7434.1, Del, A_7, CTASTRIQRCLSS, (SEQIDNO:1973); CCDS41485.1, Ins, A_7, CTCTNTDRRCKYC, (SEQIDNO:1974); CCDS2620.2, Ins, A_7, DEKIKRRWNRRRK, (SEQIDNO:1975); CCDS58308.1, Del, A_7, DLLISIQMLPLLF, (SEQIDNO:1976); CCDS2010.1, Del, A_7, EEAKELKRRKERI, (SEQIDNO:1977); CCDS5230.1, Del, A_7, EKVSLPGHHSKRW, (SEQIDNO:1978); CCDS33493.1, Del, A_7, EMLPPTKTVHSIP, (SEQIDNO:1979); CCDS3707.1, Del, A_7, EQKTEWEWKLQIE, (SEQIDNO:1980); CCDS33924.1, Del, A_7, FLKWLRLWLMVQT, (SEQIDNO:1981); CCDS47457.1, Del, A_7, GKLKKLNQHQQLS, (SEQIDNO:1982); CCDS9344.1, Del, A_7, GLEKEYLMVNQKE, (SEQIDNO:1983); CCDS7259.1, Del, A_7, GLPETEKMPQLMP, (SEQIDNO:1984); CCDS47466.2, Del, A_7, GNSKMKPGNILPL, (SEQIDNO:1985); CCDS34101.1, Del, A_7, GRCYQLPQLQGFL, (SEQIDNO:1986); CCDS35493.1, Ins, A_7, GSFFQAKGKRKNK, (SEQIDNO:1987); CCDS5933.1, Del, A_7, IFLLLEKVGLNFV, (SEQIDNO:1988); CCDS34384.1, Ins, A_7, IKDHIFLCDSSRE, (SEQIDNO:1989); CCDS6766.1, Ins, A_7, IQIPVSEVYTVLD, (SEQIDNO:1990); CCDS46038.1, Ins, A_7, IQPVLHKACHITH, (SEQIDNO:1991); CCDS10390.1, Del, A_7, IQVTVKAAIIMAQ, (SEQIDNO:1992); CCDS1332.1, Ins, A_7, IRLLKCDRTCWSS, (SEQIDNO:1993); CCDS34158.1, Ins, A_7, ISGIHPSSDWSTC, (SEQIDNO:1994); CCDS35344.1, Del, A_7, ISVQIVTLQLTRK, (SEQIDNO:1995); CCDS9477.1, Ins, A_7, IWERTEGTSTCEK, (SEQIDNO:1996); CCDS54749.1, Del, A_7, KASNMKLQILLYS, (SEQIDNO:1997); CCDS10033.1, Ins, A_7, KDCKGDDCSQILW, (SEQIDNO:1998); CCDS13562.1, Ins, A_7, KDSSDKGHTMPGR, (SEQIDNO:1999); CCDS59248.1, Ins, A_7, KDSSDKGRTMPGR, (SEQIDNO:2000); CCDS32519.2, Ins, A_7, KEEEDQGVWQGGR, (SEQIDNO:2001); CCDS714.1, Ins, A_7, KFGLCRQHFEKIK, (SEQIDNO:2002); CCDS44196.1, Ins, A_7, KHNGIWFYYYWWR, (SEQIDNO:2003); CCDS9493.1, Ins, A_7, KHVIELSTSLDCG, (SEQIDNO:2004); CCDS42422.1, Del, A_7, KIFYSILRDLLGS, (SEQIDNO:2005); CCDS4500.1, Ins, A_7, KKGEDSQDEKRQI, (SEQIDNO:2006); CCDS34235.1, Ins, A_7, KKGKKESLPRQDD, (SEQIDNO:2007); CCDS47693.1, Del, A_7, KKGKRSRNSQTVF, (SEQIDNO:2008); CCDS6243.1, Ins, A_7, KLQEFRCYQCETV, (SEQIDNO:2009); CCDS2179.1, Ins, A_7, KNHRESYSSTHTL, (SEQIDNO:2010); CCDS46063.1, Del, A_7, KNNLSYKNKEERA, (SEQIDNO:2011); CCDS1554.1, Ins, A_7, KQGPGQGGRYQQI, (SEQIDNO:2012); CCDS35049.1, Ins, A_7, KREKKKKEGTATE, (SEQIDNO:2013); CCDS58294.1, Ins, A_7, KRSRGKRKTCQNS, (SEQIDNO:2014); CCDS6368.1, Del, A_7, KTTMKQLQCWSRY, (SEQIDNO:2015); CCDS31089.1, Del, A_7, LILEKSPINVKKC, (SEQIDNO:2016); CCDS5458.2, Del, A_7, LKDIRFWILAWHI, (SEQIDNO:2017); CCDS13231.1, Del, A_7, LKRYLILMKLKKV, (SEQIDNO:2018); CCDS32879.1, Del, A_7, LKTLRRKKKTRMS, (SEQIDNO:2019); CCDS9308.2, Del, A_7, LLYTIIHLFCLKK, (SEQIDNO:2020); CCDS41470.1, Del, A_7, LNYLRKGNLNLNQ, (SEQIDNO:2022); CCDS12991.1, Del, A_7, LQSHLVQSQQHLH, (SEQIDNO:2023); CCDS9270.1, Del, A_7, LSNIRARQEGKSG, (SEQIDNO:2024); CCDS34487.1, Del, A_7, LVWMMKKSLISSG, (SEQIDNO:2026); CCDS 11260.1, Ins, A_7, MFCPCFSLFKFSL, (SEQIDNO:2027); CCDS387.2, Del, A_7, MQNLILHLQRKKD, (SEQIDNO:2028); CCDS41878.1, Ins, A_7, NARHRTGGEIRYG, (SEQIDNO:2029); CCDS54540.1, Ins, A_7, NCCLLFNDFVYIP, (SEQIDNO:2030); CCDS1703.1, Ins, A_7, NDFQFPCGSLRLS, (SEQIDNO:2031); CCDS42906.1, Ins, A_7, NESYFMDNEEKSG, (SEQIDNO:2032); CCDS34146.2, Ins, A_7, NFMVSSTIKSKSS, (SEQIDNO:2033); CCDS42164.1, Ins, A_7, NFSFSGIKYNRFT, (SEQIDNO:2034); CCDS47329.1, Ins, A_7, NFTQEPFISGDYS, (SEQIDNO:2035); CCDS53868.1, Ins, A_7, NGICFLGVCHSRR, (SEQIDNO:2036); CCDS54426.1, Ins, A_7, NGLSFTKNARYWL, (SEQIDNO:2037); CCDS42016.1, Ins, A_7, NGSIPYQSRKRED, (SEQIDNO:2038); CCDS2045.1, Ins, A_7, NHWFTKKDSESFE, (SEQIDNO:2039); CCDS34614.1, Ins, A_7, NKCRELCTIVEFC, (SEQIDNO:2040); CCDS3572.1, Ins, A_7, NKSCKRARNQRWW, (SEQIDNO:2041); CCDS34640.1, Ins, A_7, NNRTEKESLWRFR, (SEQIDNO:2042); CCDS32273.1, Ins, A_7, NPKSTCSRQ1WEP, (SEQIDNO:2043); CCDS8703.1, Ins, A_7, NQQRRKDSWLCEN, (SEQIDNO:2044); CCDS53783.1, Ins, A_7, NQRGKHKKQDKIL, (SEQIDNO:2045); CCDS41816.1, Ins, A_7, NRFHCASSLIQKQ, (SEQIDNO:2046); CCDS46501.1, Ins, A_7, NRPMEGRGYWPEK, (SEQIDNO:2047); CCDS 12994.1, Ins, A_7, NSEFCGGGNPASL, (SEQIDNO:2048); CCDS2039.1, Ins, A_7, NSKKWAHFTVNFQ, (SEQIDNO:2049); CCDS6195.1, Ins, A_7, NSSQQKRAKTYYG, (SEQIDNO:2050); CCDS7258.1, Ins, A_7, NTGEQATRLPSFC, (SEQIDNO:2051); CCDS47170.1, Ins, A_7, NTPASKRKKLTWK, (SEQIDNO:2052); CCDS41329.1, Ins, A_7, NTSFKTGGQRLCP, (SEQIDNO:2053); CCDS32981.1, Ins, A_7, NTYWRETTELECV, (SEQIDNO:2054); CCDS5228.1, Ins, A_7, PCLQAGSCNYPGR, (SEQIDNO:2055); CCDS35325.1, Del, A_7, PFLPIQKAASGRG, (SEQIDNO:2056); CCDS47878.2, Del, A_7, PLLFIPIFLLQTS, (SEQIDNO:2057); CCDS32787.1, Ins, A_7, PPGLLRVLLLWRF, (SEQIDNO:2058); CCDS4153.1, Del, A_7, QETQWRQKKIPRF, (SEQIDNO:2059); CCDS41508.1, Ins, A_7, QIPRYSSISTSWI, (SEQIDNO:2060); CCDS2384.1, Del, A_7, QKFRPTLPPGNSF, (SEQIDNO:2061); CCDS34482.1, Del, A_7, QKKQLEDSSWLIK, (SEQIDNO:2062); CCDS3573.2, Del, A_7, RKNQRCLTFQPFT, (SEQIDNO:2063); CCDS5190.1, Ins, A_7, RKSKKKSKGNRVH, (SEQIDNO:2064); CCDS43767.1, Del, A_7, RNFYSLEHFLKLP, (SEQIDNO:2065); CCDS43658.1, Ins, A_7, RNGNSQTTSWEDP, (SEQIDNO:2066); CCDS8767.1, Ins, A_7, RRHDVPQDLHTEA, (SEQIDNO:2067); CCDS9375.1, Ins, A_7, RSFKGQMGRRHNL, (SEQIDNO:2068); CCDS55466.1, Del, A_7, RSSFPYRNSCSVF, (SEQIDNO:2069); CCDS41948.1, Ins, A_7, RTILDHTRSLSQR, (SEQIDNO:2070); CCDS42721.1, Ins, A_7, RTRSLCKNRTYPW, (SEQIDNO:2071); CCDS31444.1, Ins, A_7, SAKQRFYLLPRWD, (SEQIDNO:2072); CCDS35344.1, Ins, A_7, SAYYEAPQRGSYV, (SEQIDNO:2073); CCDS5414.1, Ins, A_7, SEEGLIINRFQHY, (SEQIDNO:2074); CCDS53790.1, Ins, A_7, SENSLQRSKTTRR, (SEQIDNO:2075); CCDS3877.1, Ins, A_7, SGRCEDCNSHMSI, (SEQIDNO:2076); CCDS9111.1, Del, A_7, SLKLILAKFRPKK, (SEQIDNO:2077); CCDS47812.1, Del, A_7, SLPEILSRSLWKR, (SEQIDNO:2078); CCDS4643.1, Ins, A_7, SPIWKIFQEPKTC, (SEQIDNO:2079); CCDS34470.1, Ins, A_7, SRIKMYNICSGFN, (SEQIDNO:2080); CCDS10265.1, Del, A_7, SRRRTKKTSSCVS, (SEQIDNO:2081); CCDS12394.1, Del, A_7, SRSPASWTKGIGR, (SEQIDNO:2082); CCDS34116.1, Del, A_7, SSCLLLASCLSNL, (SEQIDNO:2083); CCDS4000.1, Ins, A_7, SYKREFPFQQPLA, (SEQIDNO:2084); CCDS679.1, Ins, A_7, TAQEIQKCYFRHI, (SEQIDNO:2085); CCDS46789.2, Del, A_7, TDYQEIFMSQSYH, (SEQIDNO:2086); CCDS33304.1, Del, A_7, TGPRKNRSGKKWK, (SEQIDNO:2087); CCDS47696.1, Ins, A_7, TKPKWAFSLDWLR, (SEQIDNO:2088); CCDS2225.1, Ins, A_7, TKSSAACLWSEKD, (SEQIDNO:2089); CCDS41816.1, Ins, A_7, TMFRRIGQARKKI, (SEQIDNO:2090); CCDS13665.1, Del, A_7, TPGMHHSLILKSI, (SEQIDNO:2091); CCDS35344.1, Ins, A_7, TYEDPYWKEDLPM, (SEQIDNO:2092); CCDS4692.2, Ins, A_7, TYNASTQEQNKLS, (SEQIDNO:2093); CCDS41963.1, Ins, A_7, VLFGCPVNKTGSG, (SEQIDNO:2094); CCDS3583.2, Del, A_7, VLKKKHQMRKKMK, (SEQIDNO:2095); CCDS8391.1, Ins, A_7, VRWGNAKNTASLL, (SEQIDNO:2096); CCDS31423.1, Ins, A_7, VTFQTPEAAPLRP, (SEQIDNO:2097); CCDS895.1, Del, A_7, WGIWPALLGECLH, (SEQIDNO:2098); CCDS33592.1, Del, A_7, WQQSCSSSLWTSC, (SEQIDNO:2099); CCDS56144.1, Ins, A_7, WVSSLTVHREDKG, (SEQIDNO:2100); CCDS447.1, Del, A_7, WWRRKYLEFTSYL, (SEQIDNO:2101); CCDS30960.1, Del, A_7, YCSVLNVIKHLTE, (SEQIDNO:2102); CCDS34158.1, Ins, A_7, YGSKYERVDFTIC, (SEQIDNO:2103); CCDS13433.1, Del, A_7, YLVTLGWNTVKNI, (SEQIDNO:2106); CCDS1404.1, Del, A_7, YWRKWRNQMPTTS, (SEQIDNO:2107); CCDS14065.1, Del, A_7, AHNQILMMLEGL, (SEQIDNO:2108); CCDS47704.1, Ins, A_7, ASERGSKGAVWG, (SEQIDNO:2110); CCDS47161.1, Del, A_7, CIELNILISWRI, (SEQIDNO:2111); CCDS6462.1, Del, A_7, DDPTWHCLRNVA, (SEQIDNO:2112); CCDS1803.1, Del, A_7, DLLLKNYYSILL, (SEQIDNO:2115); CCDS6301.1, Del, A_7, DQSSRWEKSKDG, (SEQIDNO:2116); CCDS4959.1, Ins, A_7, EHSQRLYLQTRF, (SEQIDNO:2117); CCDS6049.1, Del, A_7, EITHHPPVWAAL, (SEQIDNO:2118); CCDS4500.1, Del, A_7, ERRRQPRRKKAN, (SEQIDNO:2119); CCDS10180.1, Ins, A_7, FNSAKSDCILQR, (SEQIDNO:2120); CCDS47443.1, Del, A_7, FPLYSLLLVQRI, (SEQIDNO:2121); CCDS7148.1, Ins, A_7, FSYFLLHLCWFG, (SEQIDNO:2122); CCDS1840.1, Del, A_7, GLIFLTTIFISV, (SEQIDNO:2124); CCDS45672.1, Del, A_7, GLPQKELKGIQL, (SEQIDNO:2125); CCDS42172.1, Ins, A_7, GPQSYSMDCDHS, (SEQIDNO:2126); CCDS43789.1, Ins, A_7, GSQAYSPSIKNK, (SEQIDNO:2127); CCDS43100.1, Ins, A_7, GSRTSSKIKKGG, (SEQIDNO:2128); CCDS454.1, Del, A_7, HINRLRSVCHLI, (SEQIDNO:2130); CCDS4966.1, Del, A_7, HLGRLYLLLKQE, (SEQIDNO:2131); CCDS9719.1, Ins, A_7, HRQKFVGKGGTL, (SEQIDNO:2132); CCDS31064.1, Ins, A_7, IARERLFSLLQG, (SEQIDNO:2133); CCDS30888.1, Ins, A_7, ICKSGKDTWRHH, (SEQIDNO:2134); CCDS44689.1, Del, A_7, IFRIRPMAKMMM, (SEQIDNO:2135); CCDS10020.1, Del, A_7, IGYLTGFCSPNA, (SEQIDNO:2136); CCDS58322.1, Del, A_7, ILLSLLCRYYPV, (SEQIDNO:2138); CCDS7883.1, Ins, A_7, IWRHSRVCPGLY, (SEQIDNO:2139); CCDS1917.1, Del, A_7, KLFRKYCHQLVL, (SEQIDNO:2141); CCDS3707.1, Ins, A_7, KNRKRSGSGNFK, (SEQIDNO:2143); CCDS42697.1, Ins, A_7, KQGAFSSSSWEV, (SEQIDNO:2144); CCDS9244.1, Ins, A_7, KSKGNPGKKEAT, (SEQIDNO:2145); CCDS834.1, Del, A_7, KSQKTLLWMKKP, (SEQIDNO:2146); CCDS47641.1, Ins, A_7, KTEKEKRKEEIW, (SEQIDNO:2147); CCDS3625.1, Del, A_7, KTILVFTIWARE, (SEQIDNO:2148); CCDS34633.1, Ins, A_7, KTQHPPMVSCDG, (SEQIDNO:2149); CCDS3004.1, Del, A_7, LCLHSKMTETDY, (SEQIDNO:2150); CCDS695.1, Del, A_7, LFHSASQEISQT, (SEQIDNO:2151); CCDS34001.1, Del, A_7, LFSDKINPSGGL, (SEQIDNO:2152); CCDS3193.1, Del, A_7, LGGDMRFLHFSY, (SEQIDNO:2153); CCDS33179.1, Del, A_7, LKKEAVVRSFKS, (SEQIDNO:2154); CCDS43729.1, Ins, A_7, LKKTPSLQTHQS, (SEQIDNO:2155); CCDS5018.1, Del, A_7, LLKIPRTLKIRI, (SEQIDNO:2156); CCDS43068.2, Del, A_7, LLWLADCVGVTR, (SEQIDNO:2157); CCDS43505.1, Del, A_7, LNRLPQRRTMKI, (SEQIDNO:2158); CCDS46450.1, Del, A_7, LPQSMCFTASQL, (SEQIDNO:2159); CCDS41745.1, Del, A_7, LQKSWFIQHWSV, (SEQIDNO:2160); CCDS10024.2, Del, A_7, LRKRKLPQKLNA, (SEQIDNO:2161); CCDS53460.1, Del, A_7, LSQLRPRLSLYS, (SEQIDNO:2162); CCDS781.1, Del, A_7, MIYLLLKLMHPI, (SEQIDNO:2164); CCDS45497.1, Ins, A_7, NAFTIEAEGRGE, (SEQIDNO:2165); CCDS42656.1, Ins, A_7, NCRSGKHFFQML, (SEQIDNO:2166); CCDS13692.1, Ins, A_7, NCSEPASSEVLA, (SEQIDNO:2167); CCDS10594.1, Ins, A_7, NEVFLQKQRGFH, (SEQIDNO:2168); CCDS54765.1, Ins, A_7, NGEVTGWRRWDK, (SEQIDNO:2169); CCDS47430.1, Ins, A_7, NKANAGDGGAGQ, (SEQIDNO:2170); CCDS43629.1, Ins, A_7, NKKYHLYVPTHN, (SEQIDNO:2171); CCDS1327.1, Ins, A_7, NKPEKIPSCHRW, (SEQIDNO:2172); CCDS42794.1, Ins, A_7, NPRWLEPLSSSL, (SEQIDNO:2174); CCDS41899.1, Ins, A_7, NQIRSHEQLSCQ, (SEQIDNO:2175); CCDS2381.1, Ins, A_7, NQWRFCGRDARR, (SEQIDNO:2176); CCDS35086.1, Ins, A_7, NSCTENYRKKGG, (SEQIDNO:2177); CCDS43758.1, Ins, A_7, NSTKPATSKSHL, (SEQIDNO:2178); CCDS9188.1, Ins, A_7, NTFPRDTEWTLE, (SEQIDNO:2179); CCDS2176.2, Ins, A_7, NTYEMPKDFIWH, (SEQIDNO:2180); CCDS54426.1, Ins, A_7, NVAKITTTENVF, (SEQIDNO:2181); CCDS32537.1, Del, A_7, PFSVQMTSGSMG, (SEQIDNO:2182); CCDS164.1, Del, A_7, PNQREEDLETPG, (SEQIDNO:2184); CCDS45069.1, Del, A_7, PSRTRRAFCRRR, (SEQIDNO:2185); CCDS47256.1, Del, A_7, PVLPPETHIVDG, (SEQIDNO:2186); CCDS58711.1, Del, A_7, QDVMSFSYRNLF, (SEQIDNO:2187); CCDS30621.1, Del, A_7, QFLPGLPFLPAS, (SEQIDNO:2188); CCDS1703.1, Ins, A_7, QKQCIRFCHQIL, (SEQIDNO:2189); CCDS9697.1, Ins, A_7, QLDGSPYFSSLS, (SEQIDNO:2190); CCDS46894.1, Del, A_7, QLMSKLIIGTTQ, (SEQIDNO:2191); CCDS7281.1, Del, A_7, QNLNHPCQSEAF, (SEQIDNO:2192); CCDS13817.1, Del, A_7, QNLTLRITDTRL, (SEQIDNO:2193); CCDS56056.1, Ins, A_7, QNLVRSTGDAGH, (SEQIDNO:2194); CCDS11870.2, Ins, A_7, QRKFKENTGTTK, (SEQIDNO:2195); CCDS14246.1, Del, A_7, QRRFFCSKESPQ, (SEQIDNO:2196); CCDS5230.1, Del, A_7, QSPSWKRKAKTP, (SEQIDNO:2197); CCDS8391.1, Del, A_7, QVGECQKHSKPF, (SEQIDNO:2198); CCDS43750.1, Del, A_7, QWKQKLSNLLLL, (SEQIDNO:2199); CCDS3894.1, Ins, A_7, RAFDHFQRGHQR, (SEQIDNO:2200); CCDS11838.1, Del, A_7, RFEVVLGTFHLM, (SEQIDNO:2201); CCDS42207.1, Del, A_7, RFHSVQSRSGWR, (SEQIDNO:2202); CCDS1620.1, Ins, A_7, RHTSKEQGSWAI, (SEQIDNO:2203); CCDS53335.1, Ins, A_7, RITFSRKIETNF, (SEQIDNO:2204); CCDS42805.1, Del, A_7, RKRRRRKRKRAN, (SEQIDNO:2205); CCDS46292.1, Ins, A_7, RLYQKVDRTQTT, (SEQIDNO:2206); CCDS47203.1, Del, A_7, RRLQFFVIPFAI, (SEQIDNO:2207); CCDS2402.1, Ins, A_7, RRYTKRNSLLLK, (SEQIDNO:2208); CCDS3484.1, Ins, A_7, RSQSKQVWRYLG, (SEQIDNO:2209); CCDS31906.1, Del, A_7, SGTKSSRKSMNS, (SEQIDNO:2210); CCDS44196.1, Del, A_7, SKEWLRNLFHPK, (SEQIDNO:2211); CCDS10748.1, Del, A_7, SLREKKKISLGF, (SEQIDNO:2212); CCDS8966.1, Del, A_7, SNNTDLKLAFKY, (SEQIDNO:2213); CCDS53502.1, Ins, A_7, SRPCKHPFSLSV, (SEQIDNO:2214); CCDS47155.1, Del, A_7, SSASAFSRSLHF, (SEQIDNO:2215); CCDS6346.2, Del, A_7, SSEGKQPVIFYQ, (SEQIDNO:2216); CCDS10042.1, Del, A_7, SWPSLSVLGVKN, (SEQIDNO:2217); CCDS55059.1, Ins, A_7, TGPGQPLSHWRL, (SEQIDNO:2218); CCDS7490.1, Ins, A_7, TGSQVQEAGEAM, (SEQIDNO:2219); CCDS7588.1, Ins, A_7, TKQVGREFLVHK, (SEQIDNO:2220); CCDS1332.1, Ins, A_7, TKRATTEDLQEA, (SEQIDNO:2221); CCDS10558.1, Ins, A_7, TPISSSHTVFWK, (SEQIDNO:2222); CCDS43615.1, Ins, A_7, TTNAEGICSKNY, (SEQIDNO:2223); CCDS8655.1, Ins, A_7, TTYNNSNSWSTD, (SEQIDNO:2224); CCDS7417.1, Ins, A_7, TVYCSECQLQRH, (SEQIDNO:2225); CCDS 12098.1, Del, A_7, VKAMALKTTTKI, (SEQIDNO:2226); CCDS4516.1, Del, A_7, VTGQKDTSWHPL, (SEQIDNO:2227); CCDS3302.2, Ins, A_7, WDHHLHHSGCRE, (SEQIDNO:2228); CCDS31882.1, Ins, A_7, WISAVPHRKLQH, (SEQIDNO:2229); CCDS53868.1, Ins, A_7, WRCKNFLDGARR, (SEQIDNO:2230); CCDS32981.1, Del, A_7, YILERNYRTGMC, (SEQIDNO:2232); CCDS8324.1, Del, A_7, YLNWVFQKKLRR, (SEQIDNO:2233); CCDS47904.1, Ins, A_7, YSKQCFRFFQGK, (SEQIDNO:2234); CCDS 11870.2, Ins, A_7, AERRKRSSREG, (SEQIDNO:2235); CCDS34471.1, Del, A_7, AKPLTHVVFAW, (SEQIDNO:2237); CCDS1825.1, Del, A_7, AKTCPQSGTSS, (SEQIDNO:2238); CCDS54212.1, Del, A_7, ALHLPWYLHLQ, (SEQIDNO:2239); CCDS32742.1, Del, A_7, APRGRALWPRR, (SEQIDNO:2241); CCDS10860.1, Del, A_7, ASLNVLLIHQF, (SEQIDNO:2242); CCDS9010.1, Del, A_7, CIMDWTRKISD, (SEQIDNO:2243); CCDS11144.1, Del, A_7, CKHIFRTWKNS, (SEQIDNO:2244); CCDS54258.1, Del, A_7, CQLIENVPLLL, (SEQIDNO:2245); CCDS45057.1, Del, A_7, CQQTTSCKLSS, (SEQIDNO:2246); CCDS7799.2, Del, A_7, CRLFYKDMLQE, (SEQIDNO:2247); CCDS34206.1, Del, A_7, CTTIVLALKKD, (SEQIDNO:2248); CCDS660.1, Del, A_7, DEQDHGHPKAR, (SEQIDNO:2249); CCDS47238.1, Ins, A_7, DRKSTSFRSNC, (SEQIDNO:2250); CCDS3882.1, Del, A_7, DSPSLTTFWNN, (SEQIDNO:2251); CCDS8530.1, Del, A_7, DTQIPRTSRWC, (SEQIDNO:2252); CCDS43167.1, Del, A_7, EAFTLTMVGFS, (SEQIDNO:2253); CCDS9776.2, Del, A_7, ECIWPQMTLLY, (SEQIDNO:2254); CCDS34171.1, Ins, A_7, EEKKGKAFFYT, (SEQIDNO:2255); CCDS47517.1, Ins, A_7, EERKRGIATGI, (SEQIDNO:2256); CCDS6365.1, Ins, A_7, EESETTQDFDH, (SEQIDNO:2257); CCDS34235.1, Del, A_7, EGEKRKSPQTR, (SEQIDNO:2258); CCDS42654.1, Del, A_7, EIKTLKYPLRK, (SEQIDNO:2259); CCDS43226.1, Ins, A_7, EKEKRKEEQVR, (SEQIDNO:2260); CCDS58294.1, Del, A_7, EKQRKKKNVSE, (SEQIDNO:2261); CCDS5749.1, Del, A_7, FQKRWLLLVQE, (SEQIDNO:2262); CCDS7383.1, Del, A_7, FSFYTVNILYI, (SEQIDNO:2263); CCDS54695.1, Del, A_7, FTLLSTVNHWE, (SEQIDNO:2264); CCDS47253.2, Del, A_7, FYTWTNQYLQN, (SEQIDNO:2265); CCDS47630.1, Ins, A_7, GDQEKNKSMQT, (SEQIDNO:2266); CCDS34099.1, Ins, A_7, GFVEVAFWRLH, (SEQIDNO:2267); CCDS6047.1, Del, A_7, GPHFMRTSLRQ, (SEQIDNO:2268); CCDS4125.1, Ins, A_7, HQDDTVFWTQF, (SEQIDNO:2269); CCDS31499.1, Del, A_7, HYLSCFYSYTL, (SEQIDNO:2270); CCDS7804.1, Ins, A_7, IDLCHRSFYGN, (SEQIDNO:2271); CCDS8725.2, Ins, A_7, IEVSRGNLSLQ, (SEQIDNO:2272); CCDS35411.1, Ins, A_7, IKQVISPGKGT, (SEQIDNO:2273); CCDS10753.1, Del, A_7, ILSFLPIKSGI, (SEQIDNO:2274); CCDS1180.3, Ins, A_7, INQRKGWTQRE, (SEQIDNO:2275); CCDS33205.1, Ins, A_7, IRCWPHYQDVI, (SEQIDNO:2276); CCDS32800.1, Ins, A_7, IRSKGDYKITG, (SEQIDNO:2277); CCDS32070.1, Del, A_7, ISLKKKIITVL, (SEQIDNO:2278); CCDS45081.1, Ins, A_7, IYRGPGYKDHR, (SEQIDNO:2279); CCDS45485.1, Ins, A_7, KARKEEECRRC, (SEQIDNO:2280); CCDS43313.1, Ins, A_7, KEDCRNFKCYH, (SEQIDNO:2281); CCDS31393.1, Del, A_7, KIINLNTATGL, (SEQIDNO:2282); CCDS14667.1, Ins, A_7, KKATGSINRTG, (SEQIDNO:2283); CCDS44159.1, Del, A_7, KKNQILLRDYR, (SEQIDNO:2284); CCDS6264.1, Ins, A_7, KKRGGHQETSV, (SEQIDNO:2285); CCDS35493.1, Del, A_7, KLFSSKRKKKK, (SEQIDNO:2286); CCDS41499.1, Del, A_7, KMVLILLKVLH, (SEQIDNO:2287); CCDS2146.1, Ins, A_7, KQICTRSENQH, (SEQIDNO:2288); CCDS 1377.1, Ins, A_7, KQTASFHQAFS, (SEQIDNO:2289); CCDS33143.1, Ins, A_7, KRNGGESTETI, (SEQIDNO:2291); CCDS7554.1, Del, A_7, KSKLPTMLTQA, (SEQIDNO:2292); CCDS31477.1, Ins, A_7, KTGENKEQSQM, (SEQIDNO:2293); CCDS59106.1, Del, A_7, LDCMFRRRGMD, (SEQIDNO:2294); CCDS53775.1, Del, A_7, LGLNMKRNSKP, (SEQIDNO:2295); CCDS47033.1, Del, A_7, LGRSWLLPKTE, (SEQIDNO:2296); CCDS6080.1, Del, A_7, LIEEFTHLCSL, (SEQIDNO:2297); CCDS2308.1, Del, A_7, LILEEKVVIKG, (SEQIDNO:2298); CCDS6277.1, Del, A_7, LISHLMKWAHP, (SEQIDNO:2299); CCDS7281.1, Del, A_7, LKITYRVWLHD, (SEQIDNO:2300); CCDS55894.1, Del, A_7, LKTLCGSTGWP, (SEQIDNO:2301); CCDS790.1, Del, A_7, LKTTTRLMKRA, (SEQIDNO:2302); CCDS47907.1, Ins, A_7, LLFLPTRKFPL, (SEQIDNO:2303); CCDS54426.1, Del, A_7, LLMRDPHKEKK, (SEQIDNO:2304); CCDS8298.1, Del, A_7, LMKKMMKSVRL, (SEQIDNO:2305); CCDS9677.1, Del, A_7, LNKFSNCLKKS, (SEQIDNO:2306); CCDS722.1, Del, A_7, LPNLKHCLMMS, (SEQIDNO:2307); CCDS55698.1, Del, A_7, LPPMIPFVCWE, (SEQIDNO:2308); CCDS5865.1, Del, A_7, LPRFYIYLAWM, (SEQIDNO:2309); CCDS9269.1, Del, A_7, LSNIRARQEGN, (SEQIDNO:2310); CCDS47388.1, Del, A_7, LSSERKRESKS, (SEQIDNO:2311); CCDS11260.1, Del, A_7, LTHPLKMYLEK, (SEQIDNO:2312); CCDS5754.1, Ins, A_7, MCKCHHQRFAP, (SEQIDNO:2313); CCDS2017.2, Ins, A_7, MGCPGNQAGGD, (SEQIDNO:2314); CCDS7259.1, Ins, A_7, MGYQRRKKCHN, (SEQIDNO:2315); CCDS31623.1, Del, A_7, MQLLTENLQLA, (SEQIDNO:2317); CCDS4488.1, Del, A_7, MVKMLVFILKL, (SEQIDNO:2318); CCDS33011.1, Ins, A_7, NAEDITKCPDL, (SEQIDNO:2319); CCDS46935.1, Ins, A_7, NAKLVSILVRR, (SEQIDNO:2320); CCDS 10096.1, Del, A_7, NAQKLWKLKPV, (SEQIDNO:2321); CCDS34671.1, Ins, A_7, NCLGTRSQNAK, (SEQIDNO:2323); CCDS3591.1, Ins, A_7, NFCWWPFSRYT, (SEQIDNO:2324); CCDS33943.1, Ins, A_7, NGPDLRKGKRR, (SEQIDNO:2325); CCDS56533.1, Ins, A_7, NHTCAGEGPVS, (SEQIDNO:2326); CCDS4041.1, Ins, A_7, NIPSTKACTTA, (SEQIDNO:2327); CCDS31636.1, Ins, A_7, NKPVNSSCQII, (SEQIDNO:2328); CCDS33125.1, Ins, A_7, NPYWTKTLWMF, (SEQIDNO:2329); CCDS55457.1, Ins, A_7, NQEKIAGSLGI, (SEQIDNO:2330); CCDS4916.1, Ins, A_7, NQGVPSVFSSL, (SEQIDNO:2331); CCDS42654.1, Ins, A_7, NQRSTDEGRRL, (SEQIDNO:2332); CCDS6795.1, Ins, A_7, NRLGRARQEQG, (SEQIDNO:2333); CCDS53335.1, Ins, A_7, NRRRASKKESN, (SEQIDNO:2335); CCDS31627.1, Ins, A_7, NSNRWSAGHKP, (SEQIDNO:2336); CCDS5013.1, Del, A_7, NSPYLLYGRLG, (SEQIDNO:2337); CCDS9867.1, Ins, A_7, NVECAGHSGGF, (SEQIDNO:2338); CCDS55264.1, Ins, A_7, NVHKDRLLCLI, (SEQIDNO:2339); CCDS46894.1, Ins, A_7, NYGCQEWPDNE, (SEQIDNO:2340); CCDS214.1, Ins, A_7, NYKNSGSKPGR, (SEQIDNO:2341); CCDS13482.1, Ins, A_7, NYNKSSKEKSV, (SEQIDNO:2342); CCDS11983.1, Del, A_7, NYRINFKVPLL, (SEQIDNO:2343); CCDS11996.2, Ins, A_7, PGITEPSTCLL, (SEQIDNO:2344); CCDS2045.1, Del, A_7, PLVHQKGFRVL, (SEQIDNO:2345); CCDS13117.1, Del, A_7, PRLKKKSIQKI, (SEQIDNO:2346); CCDS1336.1, Ins, A_7, PTGSPAEVITK, (SEQIDNO:2347); CCDS3751.1, Del, A_7, PTYVYHKQKEY, (SEQIDNO:2348); CCDS10091.1, Del, A_7, PWSGTLEKEIV, (SEQIDNO:2349); CCDS8990.1, Del, A_7, QALRLQSLRRD, (SEQIDNO:2350); CCDS55946.1, Ins, A_7, QEHTKFAQSDM, (SEQIDNO:2351); CCDS47558.1, Del, A_7, QFLKERKTVDR, (SEQIDNO:2352); CCDS41764.1, Del, A_7, QKNSLKFSMIE, (SEQIDNO:2353); CCDS55350.1, Ins, A_7, QKPCSPADSEA, (SEQIDNO:2354); CCDS42164.1, Del, A_7, RKLAFPQFSSC, (SEQIDNO:2355); CCDS2957.1, Ins, A_7, RKQKSSEQSNT, (SEQIDNO:2356); CCDS34021.1, Ins, A_7, RKTPCKCQVHR, (SEQIDNO:2357); CCDS1069.1, Ins, A_7, RLVHTPKRRRL, (SEQIDNO:2358); CCDS31778.2, Ins, A_7, RNQCARNKNCY, (SEQIDNO:2359); CCDS4912.1, Ins, A_7, RQTAFEKEKRI, (SEQIDNO:2360); CCDS41631.1, Ins, A_7, RRKCCWFSDTT, (SEQIDNO:2361); CCDS42791.1, Del, A_7, RRRLSKTKLSV, (SEQIDNO:2362); CCDS621.1, Ins, A_7, RRSKKCGRITY, (SEQIDNO:2363); CCDS42041.1, Ins, A_7, RSSRKGTRTCV, (SEQIDNO:2364); CCDS55317.1, Del, A_7, RVHTFLQLQST, (SEQIDNO:2365); CCDS31425.1, Ins, A_7, RWSPAYDWLFS, (SEQIDNO:2366); CCDS8323.1, Del, A_7, SEKCRSSLDWR, (SEQIDNO:2367); CCDS214.1, Ins, A_7, SESCGRKWRRS, (SEQIDNO:2368); CCDS8321.1, Del, A_7, SKECRSSLGWR, (SEQIDNO:2369); CCDS34075.1, Del, A_7, SLTKRQKRIIV, (SEQIDNO:2370); CCDS13093.1, Ins, A_7, SNFRKTVVLPG, (SEQIDNO:2371); CCDS8438.1, Ins, A_7, SNLCWERRKFT, (SEQIDNO:2372); CCDS46790.1, Ins, A_7, SNRLSCPQRSC, (SEQIDNO:2373); CCDS14197.1, Del, A_7, SQALMLGSFMP, (SEQIDNO:2374); CCDS14482.1, Ins, A_7, SSSRKTDSEKR, (SEQIDNO:2375); CCDS9492.1, Del, A_7, STLPPSIQQIW, (SEQIDNO:2376); CCDS41774.1, Del, A_7, SVMSIFQVQPP, (SEQIDNO:2377); CCDS4597.1, Del, A_7, TAVSSLASRAW, (SEQIDNO:2378); CCDS54238.1, Ins, A_7, TFQMYRMWQSF, (SEQIDNO:2379); CCDS11838.1, Ins, A_7, TGSKWCLALFI, (SEQIDNO:2380); CCDS3525.1, Ins, A_7, TIHIRSQYSAV, (SEQIDNO:2381); CCDS7597.1, Ins, A_7, TKEEKEERASK, (SEQIDNO:2382); CCDS47634.1, Del, A_7, TLNQSPEHATR, (SEQIDNO:2383); CCDS6339.1, Ins, A_7, TNNTLVPDGFL, (SEQIDNO:2384); CCDS33744.1, Del, A_7, TRGCCSLPRKN, (SEQIDNO:2385); CCDS44756.1, Ins, A_7, TRSFETFSETS, (SEQIDNO:2386); CCDS33092.1, Ins, A_7, TSDNPFRRQTV, (SEQIDNO:2387); CCDS33093.2, Ins, A_7, TSDNPLRRETM, (SEQIDNO:2388); CCDS33094.1, Ins, A_7, TSDNSLRRETM, (SEQIDNO:2389); CCDS41477.2, Ins, A_7, VGRQGKRSSEC, (SEQIDNO:2390); CCDS6489.1, Del, A_7, VIHFKKSKIIW, (SEQIDNO:2391); CCDS33812.1, Del, A_7, VKGLPRPLKFC, (SEQIDNO:2392); CCDS43277.1, Del, A_7, VKPTLWEEGTS, (SEQIDNO:2394); CCDS55104.1, Del, A_7, VLLLISRIQKG, (SEQIDNO:2395); CCDS34993.1, Del, A_7, VLNLCLLPFLK, (SEQIDNO:2396); CCDS53626.1, Del, A_7, VLPQSSVACPG, (SEQIDNO:2397); CCDS43758.1, Ins, A_7, VVCRLATSHKF, (SEQIDNO:2398); CCDS55991.1, Ins, A_7, VWTQVECVRSI, (SEQIDNO:2399); CCDS45822.1, Del, A_7, WKIFRSWGIIP, (SEQIDNO:2400); CCDS7490.1, Del, A_7, WKPSTRSWRSD, (SEQIDNO:2401); CCDS45703.2, Del, A_7, YPVLTTLQFQG, (SEQIDNO:2402); CCDS9308.2, Ins, A_7, YYFTLSSTYFA, (SEQIDNO:2403); CCDS42360.1, Del, A_7, AERMRPKVQS, (SEQIDNO:2404); CCDS1069.1, Del, A_7, AGTHPQKKTA, (SEQIDNO:2405); CCDS43767.1, Ins, A_7, AQDVEPNHFR, (SEQIDNO:2406); CCDS34993.1, Del, A_7, AQPGSGLETC, (SEQIDNO:2407); CCDS5667.1, Del, A_7, ASVRKHIVGT, (SEQIDNO:2408); CCDS47630.1, Ins, A_7, ATPRRKKANP, (SEQIDNO:2409); CCDS 14214.1, Del, A_7, AYLMYMLPSG, (SEQIDNO:2410); CCDS12934.1, Del, A_7, CFQNPLSLLH, (SEQIDNO:2411); CCDS33273.1, Ins, A_7, CKVIWGLGSQ, (SEQIDNO:2412); CCDS2294.1, Del, A_7, CVQEIMQTGL, (SEQIDNO:2413); CCDS722.1, Ins, A_7, EERGTSESRS, (SEQIDNO:2414); CCDS53867.1, Ins, A_7, EFKARCQDNR, (SEQIDNO:2415); CCDS8439.1, Del, A_7, EGNLIQNRSF, (SEQIDNO:2416); CCDS42308.1, Del, A_7, EGRPGENQSP, (SEQIDNO:2417); CCDS748.1, Del, A_7, EGWKRRLSKK, (SEQIDNO:2418); CCDS46127.1, Ins, A_7, EHNRGYSYVR, (SEQIDNO:2419); CCDS7273.1, Del, A_7, EKILIQLKML, (SEQIDNO:2420); CCDS55311.1, Del, A_7, EKNVQKQNTT, (SEQIDNO:2421); CCDS43426.1, Ins, A_7, EVSSRARDNV, (SEQIDNO:2422); CCDS31774.1, Del, A_7, FFWLEPLMAS, (SEQIDNO:2423); CCDS9681.1, Ins, A_7, FLGTNETYRN, (SEQIDNO:2424); CCDS46419.1, Del, A_7, FLKMEPVSHF, (SEQIDNO:2425); CCDS14205.1, Del, A_7, FLVTVGGTLR, (SEQIDNO:2426); CCDS41887.1, Del, A_7, FMHLNKGGSS, (SEQIDNO:2427); CCDS702.1, Del, A_7, FVSKKRKKWN, (SEQIDNO:2428); CCDS34330.1, Ins, A_7, FWGPGFKIWT, (SEQIDNO:2429); CCDS56144.1, Del, A_7, GFFPHCPQRR, (SEQIDNO:2430); CCDS4895.2, Ins, A_7, GKGCERSKGG, (SEQIDNO:2431); CCDS31477.1, Del, A_7, GKREKRPWSL, (SEQIDNO:2432); CCDS42039.1, Ins, A_7, GNSNLTGKTH, (SEQIDNO:2433); CCDS46063.1, Del, A_7, GRAFPRNQQN, (SEQIDNO:2434); CCDS43505.1, Ins, A_7, GSTDSHRGGQ, (SEQIDNO:2435); CCDS7422.1, Ins, A_7, GTRWLSQYTV, (SEQIDNO:2436); CCDS388.1, Ins, A_7, HERCKRQIGK, (SEQIDNO:2437); CCDS1629.2, Del, A_7, HPIRTSLKMN, (SEQIDNO:2438); CCDS14259.1, Del, A_7, HQRTSTCHTL, (SEQIDNO:2439); CCDS2447.1, Del, A_7, HQSEPTKCAM, (SEQIDNO:2440); CCDS9767.1, Del, A_7, HWKRNPKAIW, (SEQIDNO:2441); CCDS47264.1, Del, A_7, IQSLMDCWQI, (SEQIDNO:2442); CCDS47167.1, Ins, A_7, IRAKVIPVKF, (SEQIDNO:2443); CCDS45485.1, Ins, A_7, IRRRYRREAI, (SEQIDNO:2444); CCDS31814.1, Ins, A_7, ISREEKYYSL, (SEQIDNO:2445); CCDS44612.1, Ins, A_7, ISTESPAPTR, (SEQIDNO:2446); CCDS34149.1, Del, A_7, KALKRQMNIS, (SEQIDNO:2447); CCDS10171.1, Ins, A_7, KCKNTLGNSE, (SEQIDNO:2448); CCDS31275.1, Del, A_7, KCMISIKKEV, (SEQIDNO:2449); CCDS46941.1, Ins, A_7, KCQRRWSFQH, (SEQIDNO:2450); CCDS43027.1, Ins, A_7, KEIRSFHPVF, (SEQIDNO:2451); CCDS9372.1, Ins, A_7, KEKYIHREKT, (SEQIDNO:2452); CCDS7095.1, Ins, A_7, KERTTCLSGI, (SEQIDNO:2453); CCDS6597.1, Del, A_7, KGAYPNHRSA, (SEQIDNO:2454); CCDS2402.1, Del, A_7, KIYKKKQFIA, (SEQIDNO:2455); CCDS 13482.1, Ins, A_7, KLQKTEDYLC, (SEQIDNO:2456); CCDS34146.2, Del, A_7, KLSRRRGQKL, (SEQIDNO:2457); CCDS35009.1, Ins, A_7, KQGSLWAGRR, (SEQIDNO:2458); CCDS2219.1, Ins, A_7, KQILIFHLLH, (SEQIDNO:2459); CCDS14161.1, Del, A_7, KRIMFLNIFY, (SEQIDNO:2460); CCDS9233.1, Ins, A_7, KRIQVHKRRR, (SEQIDNO:2461); CCDS260.1, Ins, A_7, KSIWTVDTYL, (SEQIDNO:2462); CCDS 13117.1, Ins, A_7, KTEKETEGSC, (SEQIDNO:2463); CCDS32008.1, Del, A_7, KTPFHRIFYL, (SEQIDNO:2464); CCDS43171.1, Del, A_7, LEVCCYPLNN, (SEQIDNO:2465); CCDS55633.1, Del, A_7, LHLLNLKNQV, (SEQIDNO:2466); CCDS2235.1, Del, A_7, LIEIKVPSQK, (SEQIDNO:2467); CCDS33268.1, Del, A_7, LIVCRKSFYS, (SEQIDNO:2468); CCDS42656.1, Del, A_7, LKKNGIGYYY, (SEQIDNO:2469); CCDS8967.1, Del, A_7, LKLRRNIPGI, (SEQIDNO:2470); CCDS6526.1, Del, A_7, LKSWRSIDYF, (SEQIDNO:2471); CCDS55053.1, Del, A_7, LNIEKENLHL, (SEQIDNO:2472); CCDS8713.1, Del, A_7, LNQPKMNWHL, (SEQIDNO:2473); CCDS10589.1, Del, A_7, LQHLTNIPER, (SEQIDNO:2474); CCDS54426.1, Del, A_7, LRMLPLKQIA, (SEQIDNO:2475); CCDS8579.1, Del, A_7, LRQKEIFTHP, (SEQIDNO:2476); CCDS33924.1, Del, A_7, LSLKVLKKRG, (SEQIDNO:2477); CCDS1765.1, Ins, A_7, LSVQPQMGWK, (SEQIDNO:2478); CCDS10333.1, Del, A_7, LVACPDLRRQ, (SEQIDNO:2479); CCDS3702.1, Del, A_7, MRQNLQKHLS, (SEQIDNO:2480); CCDS31121.1, Ins, A_7, NAERAETSIL, (SEQIDNO:2481); CCDS11681.1, Ins, A_7, NCHKKCLFLC, (SEQIDNO:2482); CCDS30628.1, Ins, A_7, NEPRLYKECF, (SEQIDNO:2483); CCDS7973.1, Ins, A_7, NESKKWGNII, (SEQIDNO:2484); CCDS34047.1, Ins, A_7, NHQRPSRVSG, (SEQIDNO:2486); CCDS11867.1, Ins, A_7, NISGILRRKD, (SEQIDNO:2487); CCDS43284.2, Ins, A_7, NNHSNFLVST, (SEQIDNO:2488); CCDS42788.1, Ins, A_7, NNKLCKVLCP, (SEQIDNO:2489); CCDS3836.1, Ins, A_7, NNKLFQRGSL, (SEQIDNO:2490); CCDS33566.1, Ins, A_7, NQDTSGTWNL, (SEQIDNO:2491); CCDS34097.1, Ins, A_7, NQKCGSKPNT, (SEQIDNO:2492); CCDS58191.1, Ins, A_7, NRFCSSSSAA, (SEQIDNO:2493); CCDS33750.1, Ins, A_7, NRGSCYNPKA, (SEQIDNO:2494); CCDS34451.1, Ins, A_7, NRMPWQSTEI, (SEQIDNO:2495); CCDS45989.1, Ins, A_7, NSHWREPVWM, (SEQIDNO:2497); CCDS32981.1, Ins, A_7, NSYWRATLHM, (SEQIDNO:2498); CCDS59371.1, Ins, A_7, NSYWREILQM, (SEQIDNO:2499); CCDS33931.1, Ins, A_7, NSYWRETLQM, (SEQIDNO:2500); CCDS42537.1, Ins, A_7, NSYWRGTLQI, (SEQIDNO:2501); CCDS59367.1, Ins, A_7, NSYWRKTLQM, (SEQIDNO:2502); CCDS34530.1, Ins, A_7, NTSAVSWWIH, (SEQIDNO:2503); CCDS10550.1, Ins, A_7, NTSKTNKNKL, (SEQIDNO:2504); CCDS10111.1, Ins, A_7, NYQFTDCALQ, (SEQIDNO:2505); CCDS9982.1, Del, A_7, PASGKPGEEQ, (SEQIDNO:2506); CCDS4061.1, Del, A_7, PMEATRVECF, (SEQIDNO:2508); CCDS4648.1, Ins, A_7, PQRQGCSACV, (SEQIDNO:2509); CCDS44154.2, Ins, A_7, QEGWIQVISA, (SEQIDNO:2510); CCDS47583.1, Ins, A_7, QFTYQFFQTH, (SEQIDNO:2511); CCDS1336.1, Del, A_7, QNNNWKKSAY, (SEQIDNO:2512); CCDS45284.1, Ins, A_7, QPVSPQKSGK, (SEQIDNO:2513); CCDS46424.1, Ins, A_7, QRENDWSPQH, (SEQIDNO:2514); CCDS46285.1, Del, A_7, QRKETQRKAE, (SEQIDNO:2515); CCDS34204.1, Ins, A_7, QRTRRDSVFN, (SEQIDNO:2516); CCDS58039.1, Del, A_7, QTRETMTPRA, (SEQIDNO:2517); CCDS34777.1, Ins, A_7, QTSQAVGSRL, (SEQIDNO:2518); CCDS331.1, Del, A_7, QWSLKHRRKQ, (SEQIDNO:2519); CCDS31906.1, Ins, A_7, RAAQNRQGNP, (SEQIDNO:2520); CCDS33332.1, Del, A_7, RDYKNYGLED, (SEQIDNO:2521); CCDS9287.1, Ins, A_7, RKICRIPGGI, (SEQIDNO:2522); CCDS53348.1, Ins, A_7, RNSVFPFIYI, (SEQIDNO:2523); CCDS34478.1, Ins, A_7, RPVNSEGGSQ, (SEQIDNO:2524); CCDS43628.1, Ins, A_7, RRNEQNKNIS, (SEQIDNO:2525); CCDS55850.1, Ins, A_7, RSPCPFKNGG, (SEQIDNO:2526); CCDS31282.1, Del, A_7, RTQHPWITPS, (SEQIDNO:2527); CCDS32493.1, Ins, A_7, RVFRQALFRR, (SEQIDNO:2528); CCDS8703.1, Del, A_7, SAKKKRLLAV, (SEQIDNO:2529); CCDS8300.1, Ins, A_7, SAYHSAETPF, (SEQIDNO:2530); CCDS53552.1, Ins, A_7, SCSYFASASK, (SEQIDNO:2531); CCDS2194.1, Ins, A_7, SFNFIKENIN, (SEQIDNO:2532); CCDS31963.1, Ins, A_7, SGRCYNRNKY, (SEQIDNO:2533); CCDS33360.1, Del, A_7, SILKQCCCLI, (SEQIDNO:2534); CCDS12212.1, Ins, A_7, SIQRCDAGEL, (SEQIDNO:2535); CCDS34158.1, Del, A_7, SKAKKRLRKK, (SEQIDNO:2536); CCDS3424.1, Del, A_7, SKHFIVKVQK, (SEQIDNO:2537); CCDS43423.1, Del, A_7, SLCQKACVIL, (SEQIDNO:2538); CCDS13672.1, Ins, A_7, SLFLAASRAE, (SEQIDNO:2539); CCDS3829.1, Del, A_7, SQLRKKWTAS, (SEQIDNO:2540); CCDS6045.1, Ins, A_7, SRVHMQTGER, (SEQIDNO:2541); CCDS34089.1, Del, A_7, STTMMIMKKS, (SEQIDNO:2542); CCDS43187.1, Del, A_7, SVFSVSVLKE, (SEQIDNO:2543); CCDS8748.2, Ins, A_7, TAERNKRQEK, (SEQIDNO:2544); CCDS3981.1, Del, A_7, TEFTVQSIIL, (SEQIDNO:2545); CCDS5933.1, Ins, A_7, TFFYYWRKWG, (SEQIDNO:2547); CCDS54956.1, Ins, A_7, TGEESTDRQN, (SEQIDNO:2548); CCDS54379.1, Ins, A_7, TKYVASTATG, (SEQIDNO:2549); CCDS3006.1, Ins, A_7, TLLKRMHFDY, (SEQIDNO:2550); CCDS35329.1, Del, A_7, TLSDILVNKN, (SEQIDNO:2551); CCDS6848.2, Ins, A_7, TNGISLQHED, (SEQIDNO:2552); CCDS4569.1, Ins, A_7, TQNCKAQESS, (SEQIDNO:2553); CCDS4255.1, Ins, A_7, TTRFRKTSVL, (SEQIDNO:2554); CCDS6223.1, Ins, A_7, TWTHLPEDCN, (SEQIDNO:2555); CCDS1515.1, Del, A_7, VILQVRSTAC, (SEQIDNO:2557); CCDS41710.1, Del, A_7, VNFVKILMIQ, (SEQIDNO:2558); CCDS31448.1, Ins, A_7, VSRMVGKCET, (SEQIDNO:2559); CCDS6149.1, Ins, A_7, VSWRGLYTKP, (SEQIDNO:2560); CCDS2561.2, Del, A_7, VVTKWKKMNF, (SEQIDNO:2561); CCDS33946.1, Del, A_7, VVVVKNGRRY, (SEQIDNO:2562); CCDS45081.1, Del, A_7, WHPNRKQLLK, (SEQIDNO:2564); CCDS32447.1, Ins, A_7, WRPFLFSESR, (SEQIDNO:2565); CCDS9188.1, Del, A_7, YFSKRHGMDP, (SEQIDNO:2566); CCDS5091.2, Del, A_7, YLTFPIIYLR, (SEQIDNO:2567); CCDS43767.1, Del, A_7, AIQRQSQMR, (SEQIDNO:2568); CCDS46928.1, Del, A_7, AIWNSSKKN, (SEQIDNO:2569); CCDS53868.1, Del, A_7, AMQKLSGWS, (SEQIDNO:2570); CCDS41423.1, Ins, A_7, ASSCQARER, (SEQIDNO:2571); CCDS6243.1, Del, A_7, ATRIQVLSV, (SEQIDNO:2572); CCDS10734.1, Del, A_7, CLSQCQNML, (SEQIDNO:2573); CCDS43278.1, Del, A_7, DIQRKMLVN, (SEQIDNO:2574); CCDS34633.1, Del, A_7, DSASPNGIV, (SEQIDNO:2575); CCDS2225.1, Del, A_7, EELCCVPLE, (SEQIDNO:2576); CCDS14336.1, Del, A_7, EKPGICPGP, (SEQIDNO:2578); CCDS6748.1, Del, A_7, ENANMIFKK, (SEQIDNO:2579); CCDS41850.1, Ins, A_7, ENSYIYKML, (SEQIDNO:2580); CCDS47270.1, Ins, A_7, ETTQNVAGI, (SEQIDNO:2581); CCDS34283.1, Del, A_7, FSRYPYQMM, (SEQIDNO:2582); CCDS33447.1, Del, A_7, FWTLNKGNF, (SEQIDNO:2583); CCDS6969.1, Del, A_7, GFGKARRGK, (SEQIDNO:2584); CCDS1113.2, Del, A_7, GGILLSMFL, (SEQIDNO:2585); CCDS47757.1, Ins, A_7, GNTRNSKSY, (SEQIDNO:2586); CCDS3302.2, Del, A_7, GPSSPSFWV, (SEQIDNO:2587); CCDS45045.1, Ins, A_7, GRKCPGVCF, (SEQIDNO:2588); CCDS13651.1, Del, A_7, GRRKTLKQK, (SEQIDNO:2589); CCDS34613.1, Ins, A_7, GSFLYFSSE, (SEQIDNO:2590); CCDS42016.1, Ins, A_7, GVCAISVQL, (SEQIDNO:2591); CCDS47630.1, Del, A_7, HHLLRIANL, (SEQIDNO:2592); CCDS58179.1, Del, A_7, HLKKVEKLP, (SEQIDNO:2593); CCDS7824.1, Del, A_7, HLYYQLLLF, (SEQIDNO:2594); CCDS41485.1, Del, A_7, HMYQYRQKV, (SEQIDNO:2595); CCDS46339.1, Ins, A_7, HQLADRHCH, (SEQIDNO:2596); CCDS13900.2, Del, A_7, HVKKTYADL, (SEQIDNO:2597); CCDS46226.1, Del, A_7, HVQSVTSVP, (SEQIDNO:2598); CCDS10112.1, Del, A_7, IFLKKRKEL, (SEQIDNO:2599); CCDS1479.1, Ins, A_7, ILLQSNSYV, (SEQIDNO:2600); CCDS43121.1, Del, A_7, ILRSHFKCP, (SEQIDNO:2601); CCDS44410.1, Del, A_7, ILTRAGSVM, (SEQIDNO:2602); CCDS14439.1, Ins, A_7, INEKKSVKS, (SEQIDNO:2603); CCDS1504.1, Ins, A_7, KCSIVFCGL, (SEQIDNO:2604); CCDS46251.1, Ins, A_7, KEIRSCRNE, (SEQIDNO:2605); CCDS5955.1, Ins, A_7, KETEKETAN, (SEQIDNO:2606); CCDS47168.1, Ins, A_7, KHKEKVLQS, (SEQIDNO:2607); CCDS58275.1, Del, A_7, KKAHAPART, (SEQIDNO:2608); CCDS9344.1, Del, A_7, KKKLLQFQK, (SEQIDNO:2609); CCDS31150.1, Ins, A_7, KKMLTLRYL, (SEQIDNO:2610); CCDS42654.1, Ins, A_7, KKSKLSNIH, (SEQIDNO:2611); CCDS47157.1, Del, A_7, KKVQESKDL, (SEQIDNO:2612); CCDS2474.1, Ins, A_7, KPPRRDSLI, (SEQIDNO:2613); CCDS3806.1, Ins, A_7, KQDINHVLP, (SEQIDNO:2614); CCDS43378.1, Ins, A_7, KRRREMERL, (SEQIDNO:2615); CCDS11643.1, Ins, A_7, KSREGANHE, (SEQIDNO:2616); CCDS3509.1, Ins, A_7, KYKEENSEK, (SEQIDNO:2617); CCDS4562.1, Del, A_7, LALFPATCS, (SEQIDNO:2618); CCDS1332.1, Del, A_7, LAQTGLKSN, (SEQIDNO:2619); CCDS8271.1, Ins, A_7, LERYAHANK, (SEQIDNO:2620); CCDS45081.1, Ins, A_7, LGTQTGNNY, (SEQIDNO:2622); CCDS31858.1, Del, A_7, LKRTLIRQL, (SEQIDNO:2623); CCDS45280.1, Del, A_7, LLLCNNALS, (SEQIDNO:2624); CCDS7407.1, Del, A_7, LLLMFKYSM, (SEQIDNO:2625); CCDS55261.1, Del, A_7, LNLFYEQPN, (SEQIDNO:2626); CCDS31439.1, Ins, A_7, LPKFEGSDL, (SEQIDNO:2627); CCDS33527.2, Del, A_7, LRCSFYTRN, (SEQIDNO:2628); CCDS8342.1, Del, A_7, LRQSIGRKD, (SEQIDNO:2629); CCDS55894.1, Del, A_7, LSIACLGLE, (SEQIDNO:2630); CCDS34671.1, Del, A_7, LSWNKKSKR, (SEQIDNO:2631); CCDS46894.1, Del, A_7, LWMPRMARQ, (SEQIDNO:2632); CCDS56579.1, Del, A_7, LWPMKGERK, (SEQIDNO:2633); CCDS31650.1, Del, A_7, LWQERKWRN, (SEQIDNO:2634); CCDS 10722.1, Del, A_7, LYLIDCVNN, (SEQIDNO:2635); CCDS47329.1, Del, A_7, LYPRAIYLG, (SEQIDNO:2636); CCDS41537.1, Ins, A_7, MGREVRSAN, (SEQIDNO:2637); CCDS44337.1, Del, A_7, MKLEHQGAA, (SEQIDNO:2638); CCDS31060.1, Ins, A_7, NAERAFFRL, (SEQIDNO:2639); CCDS45285.1, Ins, A_7, NAVKEDGST, (SEQIDNO:2640); CCDS1802.1, Ins, A_7, NCKRQWTRS, (SEQIDNO:2641); CCDS9694.1, Ins, A_7, NERKIQRPG, (SEQIDNO:2643); CCDS41577.1, Ins, A_7, NFLSKSKIM, (SEQIDNO:2644); CCDS13996.1, Ins, A_7, NGAKQDSSH, (SEQIDNO:2645); CCDS3628.1, Del, A_7, NGLTGSFKS, (SEQIDNO:2646); CCDS46789.2, Ins, A_7, NKRSFFDND, (SEQIDNO:2647); CCDS6063.1, Del, A_7, NLYYVQLQL, (SEQIDNO:2648); CCDS6781.3, Ins, A_7, NNYKNGDPD, (SEQIDNO:2649); CCDS34937.1, Ins, A_7, NPTDEWRRS, (SEQIDNO:2650); CCDS47044.1, Ins, A_7, NRKPMCYGL, (SEQIDNO:2651); CCDS30769.2, Ins, A_7, NRRDRCKGT, (SEQIDNO:2652); CCDS42411.1, Ins, A_7, NRRKTQITV, (SEQIDNO:2653); CCDS55597.1, Ins, A_7, NRRQVFGKA, (SEQIDNO:2654); CCDS6281.1, Ins, A_7, NSLRVLNSS, (SEQIDNO:2655); CCDS14205.1, Ins, A_7, NSWLPLGGH, (SEQIDNO:2656); CCDS13315.1, Ins, A_7, NSYTQGECP, (SEQIDNO:2657); CCDS14538.1, Ins, A_7, NVPCPSLEC, (SEQIDNO:2658); CCDS31523.1, Ins, A_7, NVWEKSGYQ, (SEQIDNO:2659); CCDS47115.1, Ins, A_7, NWESVFHYK, (SEQIDNO:2660); CCDS55843.1, Ins, A_7, NWSKSTKTN, (SEQIDNO:2661); CCDS41939.1, Ins, A_7, NYNKNITAD, (SEQIDNO:2662); CCDS47415.1, Ins, A_7, NYSASVTGS, (SEQIDNO:2663); CCDS 13568.1, Del, A_7, PALREYTMM, (SEQIDNO:2664); CCDS53783.1, Del, A_7, PERKTQKTG, (SEQIDNO:2665); CCDS45149.1, Del, A_7, PKLRYLGEY, (SEQIDNO:2666); CCDS8788.1, Del, A_7, PLPAFPGTS, (SEQIDNO:2667); CCDS12635.1, Ins, A_7, PQWRKSIQM, (SEQIDNO:2668); CCDS59452.1, Ins, A_7, PRNQLDSRL, (SEQIDNO:2669); CCDS9390.2, Ins, A_7, QKGSVQFEE, (SEQIDNO:2670); CCDS47885.1, Del, A_7, QKTVSSGKV, (SEQIDNO:2671); CCDS46785.1, Ins, A_7, QRFMAGPCM, (SEQIDNO:2672); CCDS6673.1, Del, A_7, QRKKRKFAH, (SEQIDNO:2673); CCDS14622.1, Del, A_7, QRPVKMFLR, (SEQIDNO:2674); CCDS9967.1, Del, A_7, QSLRTSAKS, (SEQIDNO:2675); CCDS5611.1, Ins, A_7, QTISEISEN, (SEQIDNO:2676); CCDS2397.1, Ins, A_7, RCKCSARLI, (SEQIDNO:2677); CCDS9543.1, Ins, A_7, RCQDWKHRR, (SEQIDNO:2678); CCDS11915.1, Del, A_7, REDFLLEMF, (SEQIDNO:2679); CCDS12639.1, Ins, A_7, RFNFKAEDF, (SEQIDNO:2680); CCDS41644.1, Ins, A_7, RKERQGKEE, (SEQIDNO:2681); CCDS5135.1, Ins, A_7, RKGEEKKKT, (SEQIDNO:2682); CCDS53507.1, Ins, A_7, RKSSRYKQL, (SEQIDNO:2683); CCDS834.1, Ins, A_7, RNPRKLCCG, (SEQIDNO:2684); CCDS33619.1, Del, A_7, RNSQKNWRK, (SEQIDNO:2685); CCDS43655.2, Del, A_7, RNVIKILFL, (SEQIDNO:2686); CCDS41878.1, Del, A_7, RPSQNRRRN, (SEQIDNO:2687); CCDS53427.1, Ins, A_7, RQEIQEKYE, (SEQIDNO:2688); CCDS54808.1, Ins, A_7, RQRFQMEDQ, (SEQIDNO:2689); CCDS7452.1, Ins, A_7, RRAPWWDNT, (SEQIDNO:2690); CCDS54659.1, Ins, A_7, RRRNMEGRL, (SEQIDNO:2692); CCDS3633.1, Del, A_7, RRSKITKII, (SEQIDNO:2693); CCDS46776.1, Ins, A_7, RRVRNCQQK, (SEQIDNO:2694); CCDS8293.1, Ins, A_7, RTLEKNFNV, (SEQIDNO:2695); CCDS10932.1, Ins, A_7, SCLLSSRCQ, (SEQIDNO:2696); CCDS55871.1, Del, A_7, SGRLNLLMF, (SEQIDNO:2697); CCDS 12272.1, Ins, A_7, SHWRETLQM, (SEQIDNO:2698); CCDS4966.1, Ins, A_7, SIWEDCIYC, (SEQIDNO:2699); CCDS2234.1, Del, A_7, SLSKSMAIM, (SEQIDNO:2700); CCDS10967.1, Ins, A_7, SNYSKRTGA, (SEQIDNO:2701); CCDS34086.1, Del, A_7, SPEAWKRNS, (SEQIDNO:2702); CCDS9291.1, Ins, A_7, SPQSCPKGE, (SEQIDNO:2703); CCDS43426.1, Ins, A_7, SSESTCPCR, (SEQIDNO:2704); CCDS3519.1, Del, A_7, STIMAPLKF, (SEQIDNO:2706); CCDS33993.1, Del, A_7, STRQKQTAI, (SEQIDNO:2707); CCDS32708.1, Del, A_7, TAFTKLAPA, (SEQIDNO:2708); CCDS 11504.2, Del, A_7, TAFTKLAPT, (SEQIDNO:2709); CCDS58090.1, Ins, A_7, TEGKYFCTK, (SEQIDNO:2710); CCDS10317.1, Ins, A_7, THRGGEGSG, (SEQIDNO:2711); CCDS2061.1, Ins, A_7, TPLDIFYKE, (SEQIDNO:2712); CCDS46445.1, Ins, A_7, TRKEKRRDA, (SEQIDNO:2713); CCDS55959.1, Del, A_7, TRPQKIPHP, (SEQIDNO:2714); CCDS5419.1, Del, A_7, TSRSSAMNA, (SEQIDNO:2715); CCDS47230.1, Del, A_7, VALMSSTII, (SEQIDNO:2716); CCDS2209.2, Ins, A_7, VDRYFQSEL, (SEQIDNO:2717); CCDS260.1, Del, A_7, VHMDCGYLS, (SEQIDNO:2718); CCDS2867.1, Ins, A_7, VMLLFSHPK, (SEQIDNO:2719); CCDS41878.1, Del, A_7, VTRETTLIL, (SEQIDNO:2720); CCDS6158.1, Ins, A_7, WWCCYYSLL, (SEQIDNO:2721); CCDS46097.1, Del, A_7, YFQKMIFLK, (SEQIDNO:2722); CCDS43720.1, Del, A_7, YQKYLFQLK, (SEQIDNO:2723); CCDS47955.1, Ins, A_7, YRKFQKASL, (SEQIDNO:2724); CCDS42690.1, Del, A_7, YYRSTIIVV, (SEQIDNO:2725); CCDS7880.1, Ins, A_7, AHPFKTTL, (SEQIDNO:2727); CCDS1038.1, Del, A_7, AMKKATKS, (SEQIDNO:2728); CCDS31636.1, Del, A_7, AMLVHHLR, (SEQIDNO:2729); CCDS32064.1, Ins, A_7, AQRERSWT, (SEQIDNO:2730); CCDS55218.1, Ins, A_7, AREVRTSH, (SEQIDNO:2731); CCDS34283.1, Ins, A_7, ASAGTLIR, (SEQIDNO:2732); CCDS42705.1, Del, A_7, CIMWTLTM, (SEQIDNO:2733); CCDS2201.1, Ins, A_7, CK1RVFSK, (SEQIDNO:2734); CCDS34106.1, Del, A_7, CLKKLQSY, (SEQIDNO:2735); CCDS55123.1, Del, A_7, CLKLFLSS, (SEQIDNO:2736); CCDS11028.1, Del, A_7, CQKICHMK, (SEQIDNO:2737); CCDS10151.1, Del, A_7, CSLSHQEK, (SEQIDNO:2738); CCDS56403.1, Ins, A_7, DSVRGKES, (SEQIDNO:2739); CCDS56405.1, Ins, A_7, DSVRGKGV, (SEQIDNO:2740); CCDS34206.1, Ins, A_7, DVLQLFLH, (SEQIDNO:2741); CCDS44172.1, Ins, A_7, EHNRRPIQ, (SEQIDNO:2742); CCDS5795.1, Ins, A_7, EHTGSTEK, (SEQIDNO:2743); CCDS43278.1, Ins, A_7, EIFKERCL, (SEQIDNO:2744); CCDS33282.2, Del, A_7, EKKYLITS, (SEQIDNO:2745); CCDS32234.1, Del, A_7, EKRRKLQN, (SEQIDNO:2746); CCDS42796.1, Ins, A_7, ERKIKETV, (SEQIDNO:2747); CCDS32755.1, Del, A_7, ESRRHSGQ, (SEQIDNO:2748); CCDS5055.1, Ins, A_7, ESSDDSWN, (SEQIDNO:2749); CCDS6462.1, Ins, A_7, ETTLRGTV, (SEQIDNO:2750); CCDS3091.1, Del, A_7, FFNPEKCG, (SEQIDNO:2751); CCDS2057.1, Ins, A_7, FQNLLSYH, (SEQIDNO:2752); CCDS43720.1, Ins, A_7, FTRNTSFS, (SEQIDNO:2754); CCDS35373.1, Ins, A_7, GDGRNSTR, (SEQIDNO:2755); CCDS2482.1, Ins, A_7, GGGENGSG, (SEQIDNO:2756); CCDS47324.1, Ins, A_7, GISGESTS, (SEQIDNO:2757); CCDS14401.1, Ins, A_7, GIWGESTS, (SEQIDNO:2758); CCDS56130.1, Del, A_7, GRRSFLVL, (SEQIDNO:2759); CCDS58490.1, Del, A_7, HCWFQHHD, (SEQIDNO:2760); CCDS12345.1, Ins, A_7, HESRLQQR, (SEQIDNO:2761); CCDS13568.1, Ins, A_7, HQPSGSTQ, (SEQIDNO:2762); CCDS10050.1, Ins, A_7, IFIFDCCF, (SEQIDNO:2763); CCDS2882.1, Ins, A_7, IFNFASRE, (SEQIDNO:2764); CCDS5011.1, Del, A_7, IFSKYSKC, (SEQIDNO:2765); CCDS46368.1, Ins, A_7, IKDCKHLF, (SEQIDNO:2766); CCDS6346.2, Ins, A_7, ILQKENSP, (SEQIDNO:2767); CCDS7428.1, Del, A_7, ILRLNDKP, (SEQIDNO:2768); CCDS32265.1, Del, A_7, IMKMHKYL, (SEQIDNO:2769); CCDS34565.1, Ins, A_7, INSRCGSQ, (SEQIDNO:2771); CCDS10501.1, Ins, A_7, IQSELPPY, (SEQIDNO:2772); CCDS3846.1, Ins, A_7, IRVVIESN, (SEQIDNO:2773); CCDS32280.1, Ins, A_7, ISRNGKRT, (SEQIDNO:2774); CCDS3505.1, Ins, A_7, IWSIVRQL, (SEQIDNO:2775); CCDS55698.1, Ins, A_7, KAEYYYCG, (SEQIDNO:2776); CCDS31167.1, Del, A_7, KARKLMRK, (SEQIDNO:2777); CCDS1387.1, Del, A_7, KATKLETC, (SEQIDNO:2778); CCDS10307.1, Ins, A_7, KCCEAQAL, (SEQIDNO:2779); CCDS41963.1, Ins, A_7, KCFARSLF, (SEQIDNO:2780); CCDS3123.1, Ins, A_7, KGCKQKEL, (SEQIDNO:2781); CCDS34225.1, Ins, A_7, KGTPQLSA, (SEQIDNO:2782); CCDS30974.1, Ins, A_7, KHQIRESE, (SEQIDNO:2783); CCDS13662.1, Ins, A_7, KHQSYELC, (SEQIDNO:2784); CCDS46785.1, Del, A_7, KIHGWTMY, (SEQIDNO:2785); CCDS387.2, Ins, A_7, KIRFFPDL, (SEQIDNO:2786); CCDS47625.1, Del, A_7, KIRKFIPT, (SEQIDNO:2787); CCDS54597.1, Ins, A_7, KKKLWKLI, (SEQIDNO:2788); CCDS33524.1, Ins, A_7, KKLEVSVP, (SEQIDNO:2789); CCDS1322.1, Del, A_7, KKRTRIIM, (SEQIDNO:2790); CCDS34204.1, Del, A_7, KNQERFSI, (SEQIDNO:2791); CCDS14435.1, Ins, A_7, KNSKYKRF, (SEQIDNO:2792); CCDS46393.1, Ins, A_7, KNWFLQGL, (SEQIDNO:2793); CCDS47973.1, Ins, A_7, KQASTSHC, (SEQIDNO:2794); CCDS12328.1, Ins, A_7, KQSQGTSS, (SEQIDNO:2795); CCDS1296.2, Ins, A_7, KRRPQTRP, (SEQIDNO:2796); CCDS46919.1, Del, A_7, KSNDLEYT, (SEQIDNO:2797); CCDS47238.1, Del, A_7, KSQAIQRK, (SEQIDNO:2798); CCDS2135.1, Ins, A_7, KTKKQQSV, (SEQIDNO:2799); CCDS702.1, Ins, A_7, KTKTSISC, (SEQIDNO:2800); CCDS2561.2, Ins, A_7, KWLPNGKR, (SEQIDNO:2801); CCDS33318.1, Del, A_7, LFRMEAII, (SEQIDNO:2802); CCDS34740.1, Ins, A_7, LGKEISNM, (SEQIDNO:2803); CCDS3520.1, Del, A_7, LKRLYIKV, (SEQIDNO:2804); CCDS14781.1, Del, A_7, LNFLWVVS, (SEQIDNO:2805); CCDS41577.1, Del, A_7, LPFKEQNH, (SEQIDNO:2806); CCDS7485.1, Ins, A_7, LPNARKID, (SEQIDNO:2807); CCDS1113.2, Del, A_7, LPRETMDN, (SEQIDNO:2808); CCDS53552.1, Del, A_7, LQLLCKCQ, (SEQIDNO:2809); CCDS3981.1, Ins, A_7, LQNSLFSP, (SEQIDNO:2810); CCDS13303.1, Del, A_7, LSLRFETR, (SEQIDNO:2811); CCDS34506.1, Ins, A_7, LWIQRFQY, (SEQIDNO:2812); CCDS53502.1, Del, A_7, LYRIKKLV, (SEQIDNO:2813); CCDS6160.1, Ins, A_7, MHQKSSDF, (SEQIDNO:2814); CCDS55937.1, Ins, A_7, MVYTHVNY, (SEQIDNO:2815); CCDS34312.1, Del, A_7, MWKNLTGT, (SEQIDNO:2816); CCDS46348.1, Ins, A_7, NADSSALC, (SEQIDNO:2817); CCDS46938.1, Ins, A_7, NAERTRNI, (SEQIDNO:2818); CCDS8436.1, Ins, A_7, NALCFLCI, (SEQIDNO:2819); CCDS45280.1, Ins, A_7, NCCYATMH, (SEQIDNO:2820); CCDS31826.2, Ins, A_7, NCISFKKV, (SEQIDNO:2821); CCDS702.1, Ins, A_7, NCPGISWK, (SEQIDNO:2822); CCDS45277.1, Ins, A_7, NDGKASQN, (SEQIDNO:2823); CCDS43789.1, Ins, A_7, NEEKQGRL, (SEQIDNO:2824); CCDS8609.1, Ins, A_7, NEQTTKHQ, (SEQIDNO:2825); CCDS46557.1, Ins, A_7, NFGDEERL, (SEQIDNO:2826); CCDS45985.1, Ins, A_7, NFSWSKIM, (SEQIDNO:2827); CCDS43337.1, Ins, A_7, NGGIGKKR, (SEQIDNO:2828); CCDS46152.1, Ins, A_7, NHGVVQRL, (SEQIDNO:2829); CCDS31821.1, Ins, A_7, NIIIEQAI, (SEQIDNO:2830); CCDS9684.1, Del, A_7, NIKCLQRN, (SEQIDNO:2831); CCDS45703.2, Ins, A_7, NKAAKWIL, (SEQIDNO:2832); CCDS14435.1, Del, A_7, NLQTLRKK, (SEQIDNO:2833); CCDS31177.1, Ins, A_7, NNSWKGKA, (SEQIDNO:2834); CCDS41302.1, Ins, A_7, NPGKIQKA, (SEQIDNO:2835); CCDS11180.1, Ins, A_7, NPHWRKTL, (SEQIDNO:2836); CCDS7269.1, Ins, A_7, NPSIASHE, (SEQIDNO:2838); CCDS33125.1, Ins, A_7, NPYWGKAL, (SEQIDNO:2839); CCDS41815.1, Ins, A_7, NRGCNCHF, (SEQIDNO:2840); CCDS34681.1, Del, A_7, NSERSCNL, (SEQIDNO:2841); CCDS34494.2, Ins, A_7, NSGSGATS, (SEQIDNO:2842); CCDS32918.1, Ins, A_7, NSHWREPI, (SEQIDNO:2843); CCDS1660.1, Ins, A_7, NSKKKEYT, (SEQIDNO:2844); CCDS43157.1, Ins, A_7, NSKWRLEA, (SEQIDNO:2845); CCDS43171.1, Ins, A_7, NSKYVAIL, (SEQIDNO:2846); CCDS7639.1, Ins, A_7, NSPRPSNL, (SEQIDNO:2847); CCDS7548.1, Ins, A_7, NSSCESNI, (SEQIDNO:2848); CCDS7256.1, Ins, A_7, NSSRRSTR, (SEQIDNO:2849); CCDS35067.1, Ins, A_7, NSYWRETL, (SEQIDNO:2850); CCDS31089.1, Ins, A_7, NSYWRKAL, (SEQIDNO:2851); CCDS55970.1, Ins, A_7, NTSQWPDE, (SEQIDNO:2852); CCDS34908.1, Ins, A_7, NTYTGCYI, (SEQIDNO:2853); CCDS32208.1, Del, A_7, NVLRLTSC, (SEQIDNO:2854); CCDS34109.2, Ins, A_7, NVWRPERV, (SEQIDNO:2855); CCDS34613.1, Ins, A_7, NWKRHGHN, (SEQIDNO:2856); CCDS10239.1, Ins, A_7, NYPFAINM, (SEQIDNO:2857); CCDS31823.1, Del, A_7, PFQPAPHM, (SEQIDNO:2858); CCDS35086.1, Del, A_7, PLRKVRRL, (SEQIDNO:2859); CCDS41470.1, Ins, A_7, PWESGQAV, (SEQIDNO:2860); CCDS47955.1, Del, A_7, QEVSKSKF, (SEQIDNO:2861); CCDS42411.1, Del, A_7, QFRLLVLL, (SEQIDNO:2862); CCDS2545.1, Ins, A_7, QGGGGEVY, (SEQIDNO:2863); CCDS10239.1, Del, A_7, QIICAQTL, (SEQIDNO:2864); CCDS1853.1, Del, A_7, QIMYMKLY, (SEQIDNO:2865); CCDS5232.1, Del, A_7, QIPACCLH, (SEQIDNO:2866); CCDS41507.1, Ins, A_7, QIPRYSSI, (SEQIDNO:2867); CCDS861.1, Del, A_7, QIRKNSLL, (SEQIDNO:2868); CCDS7658.1, Ins, A_7, QISQHPSQ, (SEQIDNO:2869); CCDS8493.1, Del, A_7, QKKRLLGA, (SEQIDNO:2870); CCDS33267.1, Del, A_7, QNCLTSWK, (SEQIDNO:2871); CCDS2922.1, Del, A_7, RFQWPLRP, (SEQIDNO:2872); CCDS 14448.1, Ins, A_7, RGATEYCL, (SEQIDNO:2873); CCDS2751.1, Ins, A_7, RHSERSCQ, (SEQIDNO:2874); CCDS7554.1, Ins, A_7, RKASCLQC, (SEQIDNO:2875); CCDS3210.1, Ins, A_7, RKREKKRW, (SEQIDNO:2876); CCDS54624.1, Ins, A_7, RMGGTIQE, (SEQIDNO:2877); CCDS10096.1, Ins, A_7, RMLRSYGS, (SEQIDNO:2878); CCDS1336.1, Del, A_7, RQKKPLYL, (SEQIDNO:2880); CCDS8328.2, Ins, A_7, RQPQKKNS, (SEQIDNO:2881); CCDS46495.1, Ins, A_7, RQSYERKI, (SEQIDNO:2882); CCDS43767.1, Ins, A_7, RQYRGKAR, (SEQIDNO:2883); CCDS2749.2, Ins, A_7, RRFPYWEG, (SEQIDNO:2884); CCDS43513.2, Ins, A_7, RSVHPFHL, (SEQIDNO:2885); CCDS4200.2, Del, A_7, RTMKQLIA, (SEQIDNO:2886); CCDS59357.1, Del, A_7, RTWLPTSS, (SEQIDNO:2887); CCDS4117.1, Ins, A_7, RVNSDLEN, (SEQIDNO:2888); CCDS11614.1, Ins, A_7, RWTHADNS, (SEQIDNO:2889); CCDS42029.1, Del, A_7, RYLRMMTS, (SEQIDNO:2890); CCDS2938.1, Ins, A_7, SFIHEEDC, (SEQIDNO:2891); CCDS10748.1, Del, A_7, SGPALNSP, (SEQIDNO:2892); CCDS 13039.1, Ins, A_7, SICKHLAL, (SEQIDNO:2893); CCDS1852.1, Ins, A_7, SIRNKGRN, (SEQIDNO:2894); CCDS34501.1, Del, A_7, SKHMEKKV, (SEQIDNO:2895); CCDS54447.1, Del, A_7, SMETKLII, (SEQIDNO:2896); CCDS33770.2, Ins, A_7, SPVEERAR, (SEQIDNO:2897); CCDS34283.1, Ins, A_7, SSSFLGHH, (SEQIDNO:2898); CCDS9148.2, Ins, A_7, SSTKKALS, (SEQIDNO:2899); CCDS32238.1, Del, A_7, SSWSWILL, (SEQIDNO:2900); CCDS43309.1, Ins, A_7, STKIHIGY, (SEQIDNO:2901); CCDS42400.1, Ins, A_7, SVPEAATR, (SEQIDNO:2903); CCDS34293.1, Del, A_7, SWLPELVG, (SEQIDNO:2904); CCDS 11069.2, Ins, A_7, TAEGSLWT, (SEQIDNO:2905); CCDS5279.1, Del, A_7, TAVTLMVM, (SEQIDNO:2906); CCDS874.1, Del, A_7, TLLHGKNV, (SEQIDNO:2907); CCDS2234.1, Ins, A_7, TPYQSLWP, (SEQIDNO:2908); CCDS42366.1, Ins, A_7, TQGRRWDI, (SEQIDNO:2909); CCDS58846.1, Del, A_7, TRRTLTRW, (SEQIDNO:2910); CCDS42172.1, Del, A_7, TTVILHGL, (SEQIDNO:2911); CCDS10034.1, Ins, A_7, TVPWKWGL, (SEQIDNO:2912); CCDS1604.1, Del, A_7, TVYIILRM, (SEQIDNO:2913); CCDS47878.2, Del, A_7, TWKQATLN, (SEQIDNO:2914); CCDS55971.1, Ins, A_7, TWYYQRRK, (SEQIDNO:2915); CCDS12273.1, Ins, A_7, TYENAQSI, (SEQIDNO:2916); CCDS58578.1, Del, A_7, TYRPEWNL, (SEQIDNO:2917); CCDS44172.1, Del, A_7, VFMNKTLM, (SEQIDNO:2918); CCDS55858.1, Ins, A_7, VKEFRRFS, (SEQIDNO:2919); CCDS2758.1, Del, A_7, VKRNRIVK, (SEQIDNO:2920); CCDS11260.1, Del, A_7, VLPLFLIV, (SEQIDNO:2921); CCDS5394.1, Del, A_7, VMSMEMIL, (SEQIDNO:2922); CCDS4993.1, Del, A_7, VNNFFKKL, (SEQIDNO:2923); CCDS2207.1, Ins, A_7, VRLLEESI, (SEQIDNO:2924); CCDS46584.1, Ins, A_7, VSNCNTKH, (SEQIDNO:2925); CCDS54404.1, Del, A_7, VTELAVAQ, (SEQIDNO:2926); CCDS45149.1, Del, A_7, VTKLLQSK, (SEQIDNO:2927); CCDS54694.1, Del, A_7, WLLRETLP, (SEQIDNO:2928); CCDS14649.1, Del, A_7, WRLCIHLL, (SEQIDNO:2929); CCDS6479.1, Del, A_7, YGDSKLVR, (SEQIDNO:2930); CCDS3556.1, Ins, A_7, YLLGKFCS, (SEQIDNO:2931); CCDS10167.1, Ins, A_7, YLSALYSD, (SEQIDNO:2932); CCDS3198.1, Ins, A_7, YSSLWWKS, (SEQIDNO:2933); CCDS41953.1, Del, A_7, YYQNLHNL, (SEQIDNO:2934); CCDS43277.1, Del, A_8, FFLCLKKEVWNVTRNRLKIQYQMHLRSFLLLLLLHRVLQGKAILWLPVVLWIIFQILVTVKFL HDPVLLAIRLLTQCQSHCTMRGARGILSASWKQT, (SEQIDNO:2935); CCDS55581.1, Del, A_8, DSIVKPRDFLIRRMISQDLGSTMLFTSHRCPTVSHCPRKELACFPQCAPDWTPSFLNTLQRMH TLSHRILFPRETLVIRVPACSSCQAL, (SEQIDNO:2936); CCDS11861.1, Del, A_8, LILIQHGGIFTFRFWVEKLSWNICKNLSLYLDKFVQRLLYVYIIETNVFVLNLFHVPVNQIFMM AFYLKYTEKAWVMELEWLIQQQCYQ, (SEQIDNO:2937); CCDS6948.1, Del, A_8, RKSPITRNLRQWPCLRASRVHTLKDHRWAVRLGLWKAVQLLKGSRNPTVQRRSLRKGSLRL SKGHECLVMIFQCPVRTLRAHSLIQ, (SEQIDNO:2938); CCDS7430.1, Del, A_8, EKIKPKKTVKSHHIKNPDYLLQKRPPRKKIKDIHLQRNLKILYLETLMRKKVLQNLNFGRVHY QRQMKNHRKKNLMSIFRICFY, (SEQIDNO:2939); CCDS42721.1, Del, A_8, RKDRRAKNRVLMIMIAKNWKIKIQNQKRLQNRKWKCTLGVMMMMILTNFLKKLKGKLKN QIRSGMGQRRMRITVKKLKSVQE, (SEQIDNO:2941); CCDS47535.1, Del, A_8, RRRKRRDIQENQRTLLKIQNCTYPGSPAWRLSPSSGEPRVAFLSLVARLWKASDLSVRKRNT YGWKAGMTGVVSLSMARVI, (SEQIDNO:2942); CCDS47492.1, Del, A_8, IPRLLVSRPPSLHPSQQAETRPERLLAPLIQKKQLALKHQLRHPLHPPHLVPKLQRRQFLAKQG QWGPPRAREKLTSSQ, (SEQIDNO:2943); CCDS43354.1, Del, A_8, TKDLIAHSLVTVMAEKNFVKRRRNILMIAQKKGFTSSLPCLKIVIAVTTSSLGKSDGHERRKV VVILQLRNKNH, (SEQIDNO:2945); CCDS786.1, Ins, A_8, IRTMVEAAFGRRHCWCCLSNKHCPFGPSENHDAGSRFKIRQNEHIWWLSTDGKRRRYPLALE GKWYKRHQNCS, (SEQIDNO:2946); CCDS 1146.1, Del, A_8, RKRKGGRKRRKKLQHLKGMMQMRSTQNMLSRTSEKARRRKGDIKKERSQMKERVQLKGM RRRTLQEQVGLGN, (SEQIDNO:2947); CCDS34854.1, Del, A_8, SLPQKVLRRKLTEHIQRPSVVLEVLLTHFWTFLMIQMLLCTKVDSWLGKFMQIWMERRLQEE NEDGKPFMLY, (SEQIDNO:2948); CCDS3986.1, Del, A_8, ILLYWSFWNDCRMGLLVSMELQKNPLGYLERASFVVMKMKLTLPLYIALCVQLICALSVLK LLILQRH, (SEQIDNO:2950); CCDS55244.1, Del, A_8, MLQKQCCYNLVIKHPLIFRINLRRQGKTKDGVVQSLGFLNQQIILQKEFFICLLMFIKRWKPA ATK, (SEQIDNO:2951); CCDS11877.1, Del, A_8, SMEIAKFPFPLKIIEKCILMKTAIALKMRLTGRILVMIPTDQQNRFPLLKRALLEKEKISPPNMM K, (SEQIDNO:2952); CCDS53456.1, Ins, A_8, KELASKFLQQSVQYKKGAQVSFLILGYRGDCYTRLFSPLIPQTTAWFYRDCFTLHQVLHLVL RGH, (SEQIDNO:2953); CCDS47176.1, Del, A_8, IRLLRNRLKRQNVKHLCLRLWTLPLKTSVTKLKLKRKGGACRTTASCTASSVSCCQMFPRGT HP, (SEQIDNO:2955); CCDS5024.1, Del, A_8, RSLLYSRMCLVQIPTHTWEPDYFVSLPMMFSWATQSFPVGAVFLTRPAIPCCSCTSHSNPWSQ S, (SEQIDNO:2956); CCDS879.1, Del, A_8, TRKHKHFYWKHLKFIGNWILKQFLHKLYLEISHQLIMAYPKIKETICGHLPKILYLHHCQRHIQ , (SEQIDNO:2957); CCDS2557.1, Del, A_8, SLLFKLVGILLSDANQMLFPGQLSLGKEERRPLDKAKEYFSWRMAASRYIILPGQMLDHIHA, (SEQIDNO:2958); CCDS42281.1, Del, A_8, TYMEMHYGHQAPQVAMLPKLLCRQMLPTLSTLQQRHPQGPCPPPVTPLRVQSVLLLGVPQT A, (SEQIDNO:2959); CCDS55009.1, Del, A_8, QFEERERGKNESARNPGISPGAFPVGLAQSGESICLYKIRRRVNVPAKTQTRVARRGSLS, (SEQIDNO:2960); CCDS46919.1, Del, A_8, SSKDKNLALLREHLVGGRLFYMWISLEKQASNAFFSQEEQRCYLVILYLYLKRPHIFFLT, (SEQIDNO:2961); CCDS44839.1, Del, A_8, SEKDIMQLMLISILGRSGALLQHFRISSFLRPSLIYIFLLITQHNLFILCHVLIILSKI, (SEQIDNO:2962); CCDS45847.1, Del, A_8, CYLHHWNYLSFLKGQIIREMSFHLLNYRTSNISHQWIRLHFQKALEIKHISKGVHRVG, (SEQIDNO:2963); CCDS45057.1, Del, A_8, FMRNFLNMLCFENILLHLHQIMKFLLRQPGQGIWKLRLIQPSLWLSLWIRPNLLLFHI, (SEQIDNO:2964); CCDS1838.1, Del, A_8, QRLQSPHTPLVFSFIWPLSTLQINVCLSKKFIAGFWTIFHILLLHQQAGRILFDIICP, (SEQIDNO:2965); CCDS12661.1, Ins, A_8, KSAAERTRGSRACGDRAHSGDTGASGSAVPVHHQEEEEAQRERNLRARRQDSEAGTD, (SEQIDNO:2966); CCDS4488.1, Del, A_8, RSINTEVNIRNINIPQKKTRIKNINISINIRNTKEKRLLMLLIKRVCLQQKELNLMI, (SEQIDNO:2967); CCDS11885.1, Del, A_8, DLRKNKEQPAKTGPSQKRTGRRGGSIKVLIPTMEHRTGFTLAATGTEITSICLTFI, (SEQIDNO:2968); CCDS46063.1, Del, A_8, RKSCLCGRHLWHCTMFCGCCSSDIQKTALLGWNSSTSSWTCTNLSPPESRSCFRSC, (SEQIDNO:2969); CCDS41355.1, Del, A_8, SKQQGVPQVSLVEIEFISVKEAAVLVEHAELILQQNCMEKIVNSFLIKYKQKKHP, (SEQIDNO:2971); CCDS55008.1, Del, A_8, QFEERERGKNESARNPVPVGLAQSGESICLYKIRRRVNVPAKTQTRVARRGSLS, (SEQIDNO:2972); CCDS41941.1, Del, A_8, RRNNTRFQQMTNYCMILKKITEIRPGLMHREGVTMVWDHRDHVNNSLFQIVMLS, (SEQIDNO:2973); CCDS34535.1, Del, A_8, TNSTRFLTSETYLLNRENQKKQLQVALNRSHLEQMKTNTKEEMTRHLTLLVKRS, (SEQIDNO:2974); CCDS56156.1, Ins, A_8, KQHPRASCHRNSLVKCNEHCGMEQKRGACSRASHQALEAIQPSTCCLYYSRSV, (SEQIDNO:2975); CCDS33301.1, Del, A_8, TALKYIFVAVRAICVMKSFLIFRRWKSHSPLQIQLHLSHPITTSCSIPWCHLC, (SEQIDNO:2976); CCDS34865.1, Del, A_8, TRTSLHQATRKHIIIPLERRSPQAHKLWMIVIIKDIFLMKRFLTLASAHVGV, (SEQIDNO:2978); CCDS110.1, Ins, A_8, STKDVQPASSQPASEQHPLTVHIPYCFSTTRLPNTAQVLAAAVLPSAVYAV, (SEQIDNO:2979); CCDS2738.1, Del, A_8, STRPSGSFLSSWRCFSFSGHPTMWLSFSLPINPSYLEMTVSGASIWTWSCW, (SEQIDNO:2980); CCDS34116.1, Del, A_8, ILFHYPHFWKDFLATSSPTRRRRQRRTVTCQIMVMMWMGGKTPWLNHVSC, (SEQIDNO:2981); CCDS34882.1, Del, A_8, PQQRAPLVELKILARGSHSSRPGSHPRRHIQSQLSQALELGQPSTDFIL, (SEQIDNO:2982); CCDS41745.1, Del, A_8, ICPWKRQPGAVAQRIGSHLVPTATLFLLNQHLGKTVRRTVLEWRLTCW, (SEQIDNO:2983); CCDS781.1, Ins, A_8, RTWLFNSRKWNCTKPWADFSFKFMPQVYVPTTFQHNPSKHCKCLGKRA, (SEQIDNO:2984); CCDS42031.1, Del, A_8, TRRKKRSKRRGKSSRRMKEGRRRRRMPRRRRRGKIPNLSSRRFPSQT, (SEQIDNO:2985); CCDS5999.1, Del, A -8, MKREKLKEMKKSVQCCVFNFMDTILLLIVYLKQMLRNLSPEAIGIH, (SEQIDNO:2986); CCDS43512.1, Del, A_8, QLALKHQLRHPLHPPHLVPKLQRRQFLAKQGQWGPPRAREKLTSSQ, (SEQIDNO:2987); CCDS11459.2, Del, A_8, STTKCQSGTAETYNSWKVKNLQLEPRRVTSQMNRQVKDMTAILSQS, (SEQIDNO:2988); CCDS8652.1, Del, A_8, TTLAFQKSLPWPKLLPKTRSMTHLTEKPRKKSLLGPLTQIHQILLS, (SEQIDNO:2990); CCDS44410.1, Del, A_8, KTKNLPLKTKLKKKENKTTLNLQMAEHHLLCPRIMNKAQPYGIC, (SEQIDNO:2992); CCDS33736.1, Ins, A_8, KVRGPGHLHDRGAEEILQCHEEVGLQEAPEAHPTAPEQVPGFCL, (SEQIDNO:2993); CCDS45798.1, Del, A_8, MDLDGMLTVIGQGLVGMTPRDLGTVAGATAQIQRPIQVQTGGRL, (SEQIDNO:2994); CCDS6948.1, Del, A_8, RKSPVTGNMRHWPCLKDRKQAERPGLICRNPSLLWAWMMKLHNY, (SEQIDNO:2996); CCDS42472.1, Del, A_8, RRRHQPPTPTPRPIRTGPSLSRWPWRGRRPPPPLPPPPPTPMCL, (SEQIDNO:2997); CCDS55045.1, Del, A_8, TLNWKREPRLSYLKTMYSSLFWSQFPFLQCCHHHHLHHHYHHVL, (SEQIDNO:2998); CCDS5434.1, Ins, A_8, KDNKAAIGKGQVQETGSERAREEQDARPQRRSGQLKKSGPLLF, (SEQIDNO:2999); CCDS43384.1, Ins, A_8, KQGCLSQLQQHHRQHSGKLQLTQHCFSKPRLCKDGEQRRLFRA, (SEQIDNO:3000); CCDS47093.1, Del, A_8, PSLALMCFLRRRSGLHPWTCFVQLTETSLQERLPHIVVVTLRQ, (SEQIDNO:3001); CCDS42183.1, Del, A_8, PTCMIIREFILEKNPILVRNVGKTSAEVQLLLNTREFILEINS, (SEQIDNO:3002); CCDS42411.1, Del, A_8, QNWKKTSKMINQPRKSMCQKRGTLKRNETRLKRKARNLLGRKK, (SEQIDNO:3003); CCDS32146.1, Del, A_8, REPLVLPPSKRSLLSVGNLIGLPIMPSRCENSSLGGMLEMSMH, (SEQIDNO:3004); CCDS5343.1, Ins, A_8, RRVHFQTARGLFSSSHNREQASQPKDSRTKKEPSRTEKGYAVF, (SEQIDNO:3005); CCDS7944.1, Ins, A_8, KFDAEVKVNRRYLRKGRICFYSAKAANLFQKLREFQFFTNEK, (SEQIDNO:3006); CCDS1807.1, Del, A_8, RNLILMIKVHKFEHHLLPSPLHNLSKYTDKLQKARMLLPPKA, (SEQIDNO:3007); CCDS6367.2, Del, A_8, MKLTLAVLPTLRNLHCYPQLCLQGRLAARNANMNLEILLGV, (SEQIDNO:3008); CCDS8336.2, Del, A_8, RARNRNMKKTMSHLIAHQALKMSGLRLFHPRLLTRKKPGK, (SEQIDN0: 3011); CCDS43379.1, Del, A_8, SLRKILDVLSSPPVTGKNKENLKNISETNISQPKLTSGRF, (SEQIDNO:3012); CCDS33203.1, Del, A_8, IMKLSNRDKMRKGWYRALLQYLVKTTNGSEKVKKRLENF, (SEQIDNO:3013); CCDS33197.2, Del, A_8, PYPISEGLTMTTGRNINSNSLMTNCLFSPIFLCHHAIMH, (SEQIDNO:3014); CCDS31158.1, Ins, A_8, SPISGGHCNLWRCIFSSASIFLQGQHGYLFQSILHARRV, (SEQIDNO:3016); CCDS35317.1, Del, A_8, TCSKATFGTSLSPFATPTRSGTCCWAWTAPTAWTVAWTP, (SEQIDNO:3017); CCDS35003.1, Ins, A_8, KGQDERTREASLRWWRVVFYAHTSGSHRQRWRSYSCRI, (SEQIDNO:3019); CCDS2738.1, Ins, A_8, KVQGHPAHFCHHGGVFHFLDTLQCGYPSLFLSIHLIWK, (SEQIDNO:3020); CCDS41335.1, Del, A_8, LLKNSLQRLFVLELGVSLIQMTCRGWHTFWSTWYSWVV, (SEQIDNO:3021); CCDS5089.1, Ins, A_8, NFQFCHLQGDSLCSVGSWNTTCTFWILCALCSLDETCK, (SEQIDNO:3022); CCDS46501.1, Del, A_8, RLPGLTCKVRRTLHQLKLCQRVMILSVVFQILMTCQWP, (SEQIDNO:3023); CCDS55008.1, Ins, A_8, ISSRKGKGAKTKAQEIPSLWALLRAEKAFVCTRSADV, (SEQIDNO:3026); CCDS7281.1, Ins, A_8, KEKQPTTKDNQGSQQKCGISQDFKPWKIKAINSRKRC, (SEQIDNO:3027); CCDS9393.1, Del, A_8, RKERNQFFTFFVIPVIVVLKIKKSMTNTCLNIQNALN, (SEQIDNO:3029); CCDS7484.1, Ins, A_8, KEVWDQKRCSKILVDEGSVQNFLHGAPEIIPTEASE, (SEQIDNO:3030); CCDS44912.1, Del, A_8, LQNASLKLTREAVRNPAQKNPQPIYHHWIPREPTRS, (SEQIDNO:3032); CCDS42869.1, Del, A_8, SWHCGCRRQRRAWRLPTPSAHRWRRPSCGYRQSQRM, (SEQIDNO:3033); CCDS47833.1, Ins, A_8, KFPDPGDKCHPRGPGGPHPQKEEEDLRVPHCLCPE, (SEQIDNO:3034); CCDS8062.1, Ins, A_8, KGQCCEARQGCHSPPPSPAPFCSGLGLCPRSTLPS, (SEQIDNO:3035); CCDS35317.1, Ins, A_8, KLAQRPLSELHSLLLRHLHGLGLAAGHGPPRQPGL, (SEQIDNO:3036); CCDS4261.1, Ins, A_8, KSHLLSTSFSNPGFCGVCGHSVRSNHIQSLQVEAV, (SEQIDNO:3037); CCDS32064.1, Ins, A_8, KYDLGRSTCRTTFPDPYSCLDQSKPKCVHLPRTLG, (SEQIDNO:3038); CCDS13134.1, Ins, A_8, MFTGNLSRSRARALPRSFLPSGFLQSEIPHAACAV, (SEQIDNO:3039); CCDS10111.1, Ins, A_8, RGSRSKTRGKNFRKEKNSREDKRERTATEEKARRN, (SEQIDNO:3040); CCDS58259.1, Del, A_8, IAQLMKDQRIIKVPQIVCGLRIVLRKKRTVFLRQ, (SEQIDNO:3041); CCDS10168.2, Del, A_8, IRVIEVARHKPLSKKKRKDRLRNLKKKKARILRK, (SEQIDNO:3042); CCDS31000.1, Del, A_8, ISFRVLASSWYASLPSPSCNLWMPSTKIRLFTAI, (SEQIDNO:3043); CCDS5434.1, Del, A_8, RQQSCDWKGSSSGDRKRTRARGTGCTASTTLWTT, (SEQIDNO:3044); CCDS56348.1, Del, A_8, CGKSIWWLKELRVLMFLHLWDSMKQVALHQSNR, (SEQIDNO:3045); CCDS56566.1, Del, A_8, IITVRGSITTMKGTDQGACLVTDQGLHLPGLTG, (SEQIDNO:3046); CCDS30860.1, Del, A_8, KEKDIHLLIEKKNMEKTIITQVKKRKTRLKILD, (SEQIDNO:3047); CCDS9097.1, Ins, A_8, KFKTKTKSWPLCCCNRHSIVFLEWKRWLQKSTE, (SEQIDNO:3048); CCDS7391.1, Ins, A_8, KGLSSTHQGAQAVQYMQTCVGKRHKNNCVGSEV, (SEQIDNO:3049); CCDS43346.1, Ins, A_8, KPVNWTNSAICNKWEIVSMGTDQASHCRCATTL, (SEQIDNO:3050); CCDS4306.1, Del, A_8, KRRRKQKRPQPKILSHRPRRKRRKRRRQQSRLY, (SEQIDNO:3051); CCDS2557.1, Ins, A_8, KVFCSSWWGYCYRMQTKCFSQGSYLLEKRNGDP, (SEQIDNO:3052); CCDS47329.1, Ins, A_8, KVHDRAAQKARAMERGIREQQPVLNLRDTTTSL, (SEQIDNO:3053); CCDS 13177.1, Ins, A_8, MLEQIRALQETMQRWRSSERYMQKSSSLLHSIQ, (SEQIDNO:3054); CCDS4168.1, Del, A_8, MLKSRQGTSGSTLTWAATWISTLLGLPSGVLWC, (SEQIDNO:3055); CCDS58899.1, Ins, A_8, NANQDFTFYPECDCNSKNYRACPPKDQVRGSVL, (SEQIDNO:3056); CCDS6551.2, Del, A_8, SQKSQHHCALRKIEWATKLSLGGKAGPQAQEKA, (SEQIDNO:3057); CCDS5343.1, Del, A_8, TCPFPDCERPFLFVTQQRTSLTAQRLQNQEGAL, (SEQIDNO:3058); CCDS43556.1, Del, A_8, WILVNLFSGTSKAPFLCGGIDQTSQPSAGPRGT, (SEQIDNO:3059); CCDS53936.1, Del, A_8, KHKYSWICFLTESGMEHFSTPVREDPWIWWTL, (SEQIDNO:3061); CCDS7434.1, Ins, A_8, KRSQGAAFFTNDKHVGHFDGLLPSQRFQLQQA, (SEQIDNO:3062); CCDS11965.1, Del, A_8, MILFQLTFSCCLALSLTASAMWKQQNWMEKPI, (SEQIDNO:3063); CCDS45016.1, Del, A_8, REPRERLYQDTSLMMIVLTIQNAAFLSNIRMA, (SEQIDNO:3064); CCDS53722.1, Del, A_8, RGPRRKVLMVLWLPANQSLQKSQVFQWVLRTE, (SEQIDNO:3065); CCDS5636.1, Ins, A_8, SKRKLFLAFWLTVETRLFAVIHMFFGIFSGCI, (SEQIDNO:3066); CCDS813.1, Del, A_8, TRTREVSPRHSTLTASRRSSTASTSWEGWPPG, (SEQIDNO:3067); CCDS53450.1, Del, A_8, YFVELMISMIQRVQQDQQIGSIKVDYHPHGFL, (SEQIDNO:3068); CCDS33309.1, Del, A_8, ALYVKWRQIFTLQRRCQKKDQVTASFLLRLY, (SEQIDNO:3069); CCDS7148.1, Del, A_8, IPSSSCALTLQMNKFSIIIINMCLHGNRMNT, (SEQIDNO:3070); CCDS9097.1, Del, A_8, IQDQDQELATVLLQSALDCIFGVEEMATKKH, (SEQIDNO:3071); CCDS35325.1, Ins, A_8, KGQDERTREASFRWWRDVFYAHTSGPHRQRW, (SEQIDNO:3072); CCDS47911.1, Del, A_8, LRVQLIFQLLPMDFKPQQGMLLVIIVYRHQL, (SEQIDNO:3073); CCDS34195.1, Del, A_8, RATFLLALWECSLPQARLCLIVSRTLLLVQS, (SEQIDNO:3074); CCDS30576.1, Del, A_8, SSRADGLLCSSKSTTEVIRNVAKDGRLAGRL, (SEQIDNO:3075); CCDS43384.1, Del, A_8, TRMFIPAAAAPPPAQWETPAHTTLLLKAPTL, (SEQIDNO:3076); CCDS43993.1, Ins, A_8, KERKRESRPGGTGRATILFVTQTQDSKGKG, (SEQIDNO:3077); CCDS32017.1, Ins, A_8, KHLLCWMFSPVLFLLLFGYIRMLAFDCDGL, (SEQIDNO:3078); CCDS55010.1, Del, A_8, QFEERERGKNESARNPGISPGACTLVPAAV, (SEQIDNO:3079); CCDS31590.1, Del, A_8, RASVSFWTLLPTTGVRTRGSPLRLTLWPPR, (SEQIDNO:3080); CCDS8875.1, Del, A_8, RESRAMISLGALREDWNQKRSLGQQIPVVI, (SEQIDNO:3081); CCDS3453.1, Del, A_8, RNSYRMDQTFKILYLVILQTGAPGMNIKET, (SEQIDNO:3082); CCDS58167.1, Del, A_8, RSQNHQKRKVLGTILVHNHIIDYAWVICHL, (SEQIDNO:3083); CCDS31426.1, Del, A_8, TSQRIPCSLQKKMHLKYGLFSTFYLRTSIH, (SEQIDNO:3084); CCDS7450.1, Del, A_8, VSVITMKLWEKHFKGLNWNLVVWILNLKMM, (SEQIDNO:3085); CCDS2702.1, Del, A_8, IAFHIKSIAAALKRHFTVNMSKVETGLHV, (SEQIDNO:3087); CCDS55049.1, Ins, A_8, KHPAAAAERGRVCGSSTGASAHPSGGSLV, (SEQIDNO:3088); CCDS11684.1, Del, A_8, MFNPSYGFQASGLQHTGVDRLWWTFHYTS, (SEQIDNO:3089); CCDS3006.1, Del, A_8, QELKEAVKFSIVKNNSNNKCGIQPGLPIL, (SEQIDNO:3090); CCDS31177.1, Del, A_8, RIYHLNICCLRRKFYYQEANLKRRNFKLK, (SEQIDNO:3091); CCDS41943.1, Ins, A_8, SKTESSEFSRTTNYSSFILDQPYPSFALT, (SEQIDNO:3092); CCDS12962.2, Del, A_8, WMTMQTIIKTRSSTLERNRTEAMPREHLL, (SEQIDNO:3094); CCDS54746.1, Del, A_8, IEALTALFTCRTASISVNISTLMSILEA, (SEQIDNO:3095); CCDS7434.1, Del, A_8, IKRRMRMKTPPLLISVWKIFRKIKIRIV, (SEQIDNO:3096); CCDS8293.1, Ins, A_8, KEKEEVPANRKTTGKTRRKEKSYILTNR, (SEQIDNO:3097); CCDS34116.1, Ins, A_8, KFFFIILIFGKTSSRLLLPPGGGDRGGQ, (SEQIDNO:3098); CCDS5024.1, Ins, A_8, KGHCCIQGCVWFKFQPIHGNQTISYHSL, (SEQIDNO:3099); CCDS9344.1, Del, A_8, LSINNYRFQMKFYFRFTSHGSPFTSANF, (SEQIDNO:3101); CCDS46302.1, Del, A_8, QEPQKKGNQLKFLLSRDNSMQLLEPYCC, (SEQIDNO:3102); CCDS44756.1, Del, A_8, RDQCFQMMEGKAFLPERKCFPGNQHPLG, (SEQIDNO:3103); CCDS8048.1, Ins, A_8, RQNKYMETRNATVTKTPFNSYNVVTCTM, (SEQIDNO:3104); CCDS6940.1, Del, A_8, SFLVLRFMSQIILSEFWMCCGLVPTSSP, (SEQIDNO:3105); CCDS4035.1, Del, A_8, TGTKHESGLLKTSQKLSLKKMTQGPCLG, (SEQIDNO:3106); CCDS33143.1, Del, A_8, ALQVFSSTGLKRTLIERVSMDTAFRES, (SEQIDNO:3108); CCDS58143.1, Del, A_8, GRISTWKPEMQQLLKHHLIVITSLLVL, (SEQIDNO:3109); CCDS59367.1, Del, A_8, IILDTNSTNVKNVAKLLTSPQSLLPIR, (SEQIDNO:3111); CCDS47036.1, Ins, A_8, IKNSGSFWFVESSRGQGHHTVQRPHSS, (SEQIDNO:3112); CCDS35139.1, Ins, A_8, ISQREASNFWEAVDIICTPRTVPSAAE, (SEQIDNO:3113); CCDS34933.1, Del, A_8, KGKNSPCCQKKLRVQNQGHLLMILLHN, (SEQIDNO:3114); CCDS9016.1, Del, A_8, LLQKEIPLLQLSLLTDSKRNHGHPQLT, (SEQIDNO:3115); CCDS31448.1, Del, A_8, MLKNVRGRRKKRKKKNNSKLKYRRKRK, (SEQIDNO:3116); CCDS32488.1, Del, A_8, MLTALPPAMTAMCGYGIRGNPVQQWNI, (SEQIDNO:3117); CCDS53898.1, Ins, A_8, MYSRGRGANGKRKGRISNKKTRVDCTD, (SEQIDNO:3118); CCDS786.1, Del, A_8, PDNGGGSFWQEALLVLSLEQALPLWTV, (SEQIDNO:3119); CCDS3452.1, Del, A_8, RGSGLTNGGVTERNWLPFGLFVVMYRT, (SEQIDNO:3120); CCDS6367.2, Del, A_8, RKRNLSNMTIQTMKTVPSNFWKGALLH, (SEQIDNO:3121); CCDS32319.1, Del, A_8, RRTKPAALSPVMVPRQKYQQRVKRSSA, (SEQIDNO:3122); CCDS13102.1, Del, A_8, EIKVLKCLPSWKPKDLNNSPSLQWNL, (SEQIDNO:3123); CCDS41715.1, Ins, A_8, KAEKKVTQKTEEKQKGSHRYNSRFLE, (SEQIDNO:3124); CCDS34565.1, Ins, A_8, KCTVLIHVRLSGWIFAKKINSRCGSQ, (SEQIDNO:3125); CCDS33941.1, Del, A_8, LVIPKLIILMEKTLTGCLRALKMNRF, (SEQIDNO:3126); CCDS33796.1, Del, A_8, SLKRAEAKPSPHAAPICFLSHCTTEL, (SEQIDNO:3127); CCDS14635.1, Del, A_8, ARQSLKGQAEDSSKSTLEKILNQMS, (SEQIDNO:3128); CCDS30948.1, Del, A_8, IWWIVQNSFLLPFRMSSFPKKFFFL, (SEQIDNO:3129); CCDS47299.1, Ins, A_8, KASGKEGEGYEEMRFFSDMSSSRAP, (SEQIDNO:3130); CCDS1616.1, Ins, A_8, KEAQAAVPAETVGHFSQARTRAGLV, (SEQIDNO:3131); CCDS9933.1, Ins, A_8, KGKKETCGPRRKFTICSTRKCSILR, (SEQIDNO:3132); CCDS54094.1, Del, A_8, KKKRKMKRKKMKKKTPKKIPRKRTR, (SEQIDNO:3133); CCDS30603.1, Ins, A_8, KSASTESNSSEKQICKAEGRLEKCL, (SEQIDNO:3134); CCDS45277.1, Del, A_8, LTPVEKLRRKISELLLGNNMQPNSI, (SEQIDNO:3135); CCDS45264.1, Ins, A_8, NFAYCPRCGHDPGCPEIWTLPGYGD, (SEQIDNO:3136); CCDS3701.2, Ins, A_8, NNIPNKIRVRNGNRQWSTSKHWNEK, (SEQIDNO:3137); CCDS47202.1, Ins, A_8, NPGAADRPRRELLLQGNSAANKDLL, (SEQIDNO:3138); CCDS54586.1, Del, A_8, PPVKESQVARRKCLKEMARRKKTEE, (SEQIDNO:3139); CCDS41346.1, Del, A_8, RFQMQPLSLMPAHWSICLLRDSMIW, (SEQIDNO:3140); CCDS43993.1, Del, A_8, RKKKRKPTGRYRQSNHPLRHPDARQ, (SEQIDNO:3141); CCDS8293.1, Del, A_8, RKRGGTSQQEDHGEDQKEGEILYTH, (SEQIDNO:3142); CCDS59269.1, Ins, A_8, RNGNRIRLFGHHKHYKGSRGADQLI, (SEQIDNO:3143); CCDS10145.1, Del, A_8, GKYLSPNRNLYRLLQKKKCLLIQN, (SEQIDNO:3144); CCDS9957.1, Del, A_8, ILTMRQWLKNRSLERTHLLIIQNI, (SEQIDNO:3145); CCDS 1279.1, Ins, A_8, KARRADERKTDRRTKKARGMFILP, (SEQIDNO:3146); CCDS9246.1, Ins, A_8, KEEKYRSRFYSKCLISIRSWIKTT, (SEQIDNO:3147); CCDS5142.2, Ins, A_8, KEKRLSTQLFPVHSNHQQRDYKRN, (SEQIDNO:3148); CCDS6673.1, Ins, A_8, KESSPIEPRDHNEPSISEHVCWNV, (SEQIDNO:3149); CCDS58144.1, Ins, A_8, KGQCCEARQPQAPLPTLHPAPPAP, (SEQIDNO:3150); CCDS7895.1, Ins, A_8, KPVYSSFHECLHNERSRCSDSYPL, (SEQIDNO:3151); CCDS34983.1, Ins, A_8, NHANFNFGMCNYIPPCLQWNIFSI, (SEQIDNO:3153); CCDS1616.1, Del, A_8, RGASSSTSRDSRSFFTSAHPSRPC, (SEQIDNO:3154); CCDS47527.1, Del, A_8, RLFWMRRLWCFRQLSGDISRGQSS, (SEQIDNO:3155); CCDS14214.1, Del, A_8, RSTLLWLLSQRRMGIMSPNRSSKD, (SEQIDNO:3156); CCDS3738.1, Del, A_8, SGEKFFRISKKKPKKTTNLVSCMD, (SEQIDNO:3157); CCDS9137.1, Del, A_8, SKKPPQSKRTNGMKMNMKGPEGRR, (SEQIDNO:3158); CCDS43278.1, Del, A_8, SSIHIVEKQLKLRERPTNKKKRKN, (SEQIDNO:3159); CCDS54447.1, Ins, A_8, TAGSFEKHDGSWRICLPSKHLRGR, (SEQIDNO:3160); CCDS44065.1, Ins, A_8, TEDHTKREQEIQISSQSAHCDSGR, (SEQIDNO:3161); CCDS33353.1, Del, A_8, TILKNFLSVMVKMEDYPFLVWRNF, (SEQIDNO:3162); CCDS5436.1, Ins, A_8, TKEERYTGAAHPTFHTRCVFRTFN, (SEQIDNO:3163); CCDS47161.1, Del, A_8, TQRRLKNWKEKKCIELNILISWRI, (SEQIDNO:3164); CCDS32017.1, Del, A_8, TSPLLDVFSSFISSSLWVHQNACF, (SEQIDNO:3165); CCDS11623.1, Del, A_8, FFMATGQLLFLIRLNLAAHSQCP, (SEQIDNO:3166); CCDS5419.1, Del, A_8, FGRSLNLTVKWLVTSGKRVTSIR, (SEQIDNO:3167); CCDS6787.1, Del, A_8, IICIKNMFLQRKGFLNLSHVEKF, (SEQIDNO:3168); CCDS13117.1, Ins, A_8, KHCSTYYRLFSRRKTKDIRLRHL, (SEQIDNO:3170); CCDS55862.1, Ins, A_8, KHHESFPLSCPPPHRCCLPCLLF, (SEQIDNO:3171); CCDS9341.1, Ins, A_8, KHLKAGCRSYTGAPQCPPPSSAG, (SEQIDNO:3172); CCDS43027.1, Ins, A_8, KYSGRAQTSKIREGRGRTLPESP, (SEQIDNO:3174); CCDS10180.1, Del, A_8, LVCSALISLTLKGNLFTLLTLLM, (SEQIDNO:3175); CCDS3639.1, Del, A_8, MFLIQRELLKTLNMIQVLMSVVH, (SEQIDNO:3176); CCDS12021.1, Del, A_8, RFGMKVLPWVLTRLTNHPSWNSS, (SEQIDNO:3177); CCDS30729.1, Del, A_8, RKTVLKNQKVSLLEISSKPRSKT, (SEQIDNO:3178); CCDS45114.1, Ins, A_8, RLRRINGRGSQIRSRNAFNRSEE, (SEQIDNO:3179); CCDS2231.1, Del, A_8, SPFGSSCLQTSNNNMEKVTLKKV, (SEQIDNO:3180); CCDS44512.1, Del, A_8, STSASCSSSFSLVMTLTLDTWRL, (SEQIDNO:3181); CCDS54430.1, Ins, A_8, TRFKQLAFRKRHFKFKDYFKSNN, (SEQIDNO:3182); CCDS704.1, Del, A_8, YSVEKTLKKFLVEHQVEKGLENC, (SEQIDNO:3183); CCDS1113.2, Del, A_8, EKEENLGGLKWWQEAHAGLQKG, (SEQIDNO:3184); CCDS11906.1, Del, A_8, HGILSFLMLQKIFQLFLWHVQN, (SEQIDNO:3185); CCDS44948.1, Del, A_8, IAQLMKDQRIIKVAPLVEDKMI, (SEQIDNO:3186); CCDS47757.1, Del, A_8, IPSRHMFSVTKIMLKETFKRRK, (SEQIDNO:3187); CCDS1336.1, Del, A_8, IYETKERMIKCPRSGRNSVVCF, (SEQIDNO:3188); CCDS43264.1, Ins, A_8, KEEETAESSLRFNLPFRCYLWI, (SEQIDNO:3189); CCDS10010.1, Ins, A_8, KGPPSTCQERLYESRSRWPCIS, (SEQIDNO:3190); CCDS11787.1, Ins, A_8, KRLCGVLCPMVWSLQTVGSHLG, (SEQIDNO:3191); CCDS5049.1, Ins, A_8, KRSIGKGIFYLVSATIPSRHCL, (SEQIDNO:3192); CCDS45016.1, Ins, A_8, KSFNDGKQSVSSIPSVFLVWNR, (SEQIDNO:3193); CCDS4203.1, Ins, A_8, KVFFWPRKAQEGLMFKEDSLSV, (SEQIDNO:3194); CCDS5436.1, Del, A_8, QGGKIHRRCTSHLSYQVCFQNI, (SEQIDNO:3195); CCDS47483.1, Del, A_8, QKRRLKQLQIIMKMLMVMVFMK, (SEQIDNO:3196); CCDS53341.1, Ins, A_8, RKEKICRSSTWPIYKKNDSKNC, (SEQIDNO:3197); CCDS7281.1, Del, A_8, RKTANYERQPREPTKMWHQSRL, (SEQIDNO:3198); CCDS11255.1, Del, A_8, RRKIIPNCFWGKTESPSIFTTC, (SEQIDNO:3199); CCDS3503.1, Ins, A_8, RSQTKIHRSPSVLCHLLFRTTS, (SEQIDNO:3200); CCDS6209.1, Ins, A_8, RTNERRTEARGRDYARARRRRV, (SEQIDNO:3201); CCDS43336.1, Del, A_8, SLTKLNKVRKKQKTDLMFKKKL, (SEQIDNO:3202); CCDS42794.1, Del, A_8, SNCQLVTAVPNWNLFSTVLLHL, (SEQIDNO:3203); CCDS2874.1, Del, A_8, SVSRPEACGIRTSLLVRRMRRQ, (SEQIDNO:3204); CCDS30978.1, Ins, A_8, TSAAGSRETKKQKGYNLHQAKD, (SEQIDNO:3205); CCDS54419.1, Del, A_8, VLTFWMMTQNKRLSKLWTMKMS, (SEQIDNO:3206); CCDS3108.1, Del, A_8, HLNQILEQKVSTAASYWESDL, (SEQIDNO:3207); CCDS53450.1, Del, A_8, ILKKSSTSSLIESLFPFLDPL, (SEQIDNO:3208); CCDS35069.1, Ins, A_8, KDSQNCEDAQAIQNPQAPEAP, (SEQIDNO:3209); CCDS2477.1, Ins, A_8, KEVQGSQCTQEASFGLFPVLL, (SEQIDNO:3210); CCDS8652.1, Ins, A_8, KLRWHFRNPFHGQNYYQRLDL, (SEQIDNO:3211); CCDS9088.1, Ins, A_8, KVFPRSSRRRRVIEDTIGILH, (SEQIDNO:3212); CCDS11512.1, Del, A_8, LGERVYKYNSTVKELRLSLLF, (SEQIDNO:3213); CCDS34226.1, Del, A_8, LQIRLCLLHFLVRLPRQRLLS, (SEQIDNO:3214); CCDS8680.1, Del, A_8, MLLVQDGMKHSHLLFMSQNWH, (SEQIDNO:3215); CCDS46722.1, Del, A_8, MLTQTDSAWDLEIAEVLFHIQ, (SEQIDNO:3216); CCDS43270.1, Del, A_8, MQKKKSSREIRKRRRMMMMRR, (SEQIDNO:3217); CCDS7414.1, Ins, A_8, NHYSERLRATDDKHRKAKSGG, (SEQIDNO:3218); CCDS10145.1, Ins, A_8, RENIYPQTETCTDFYRRKSVS, (SEQIDNO:3219); CCDS7136.1, Del, A_8, RLAARVWMYQNSVLQKKMKDC, (SEQIDNO:3220); CCDS43264.1, Del, A_8, RRRNSRKQSQVQLAIQVLLVD, (SEQIDNO:3221); CCDS5005.1, Del, A_8, TEAFAFLNMKITKQLPRQGVG, (SEQIDNO:3222); CCDS1917.1, Del, A_8, VTWMKRRRRNLILRKPEWIFK, (SEQIDNO:3223); CCDS31612.1, Del, A_8, GKLSPRTAVGAARNIRSSSF, (SEQIDNO:3224); CCDS43556.1, Ins, A_8, IGSWSTCSQEPQKPHSFVGA, (SEQIDNO:3225); CCDS33107.1, Ins, A_8, KCCCNGPRACREQNSEQGGL, (SEQIDNO:3226); CCDS42621.1, Ins, A_8, KCCCNGPRACREQNSQQRGL, (SEQIDNO:3227); CCDS6463.1, Ins, A_8, KEASLPGPDCSIKLYPHLPV, (SEQIDNO:3228); CCDS42472.1, Ins, A_8, KEGAISLRLRLQGRFGRGQA, (SEQIDNO:3229); CCDS34195.1, Ins, A_8, KGQHFYWHCGSAACHRRGCV, (SEQIDNO:3230); CCDS32126.1, Ins, A_8, KSTATRGDWSSATTGDQPTS, (SEQIDNO:3231); CCDS43434.1, Del, A_8, MQVRKTSLFYLDFLIGLSWK, (SEQIDNO:3232); CCDS4567.1, Ins, A_8, NAWCWFADLFPSHPLYTTGC, (SEQIDNO:3233); CCDS7391.1, Del, A_8, RAFIHTPRSPGCSVYANMCW, (SEQIDNO:3234); CCDS5142.2, Del, A_8, REKIINPTISCPFQSPTKRL, (SEQIDNO:3235); CCDS879.1, Del, A_8, REKWPLNLILIQIVQKLLIF, (SEQIDNO:3236); CCDS30763.2, Del, A_8, RKPLLKCIKLRYKSCKKQLK, (SEQIDNO:3237); CCDS2217.1, Ins, A_8, SCKRRKSQRTCLCRTFEEVQ, (SEQIDNO:3238); CCDS33182.1, Del, A_8, SINIQNSQFLNVGIQKNIRE, (SEQIDNO:3239); CCDS 11284.2, Del, A_8, SRTSQSWKAGKSWKIMRRNR, (SEQIDNO:3240); CCDS46292.1, Del, A_8, STVREALSPVQHGKCRKRRN, (SEQIDNO:3241); CCDS59367.1, Ins, A_8, TFQMQRMWQIILHASTSSST, (SEQIDNO:3242); CCDS2231.1, Ins, A_8, TRHLGAAVSRHQTTIWRRLH, (SEQIDNO:3243); CCDS30978.1, Del, A_8, IGSRKQRDKKAKRIQLASS, (SEQIDNO:3244); CCDS 13629.1, Del, A_8, IKDSQKNLSQRRNLMMMGT, (SEQIDNO:3245); CCDS41826.1, Del, A_8, IKLELPLLMMALPWVWLTF, (SEQIDNO:3246); CCDS45717.1, Ins, A_8, KDADSYIEFGYSWKNHHLV, (SEQIDNO:3247); CCDS33301.1, Ins, A_8, KEACFKRLLAETCWNGNAL, (SEQIDNO:3248); CCDS8902.1, Ins, A_8, KEGLQDCRECHQWGNIRRK, (SEQIDNO:3249); CCDS32308.1, Ins, A_8, KEGRRSEISVFQGRKSSWE, (SEQIDNO:3250); CCDS56334.1, Ins, A_8, KPILQKKATKRFLFYLSKL, (SEQIDNO:3251); CCDS3842.1, Ins, A_8, KSCNNFISFRNTRWPAAGL, (SEQIDNO:3252); CCDS54647.1, Del, A_8, KSSGLACLPILLRNVRIWR, (SEQIDNO:3253); CCDS41745.1, Ins, A_8, KYARGRDSLELLPKELEVI, (SEQIDNO:3254); CCDS12963.1, Del, A_8, LGKSLHFVIIREFTKERAY, (SEQIDNO:3255); CCDS42033.1, Del, A_8, LNVTCFVIFRRVRNELQKW, (SEQIDNO:3256); CCDS7179.1, Del, A_8, MTWQICTSYSVLCPLVYLI, (SEQIDNO:3257); CCDS2304.1, Ins, A_8, RLHGSHNTKQTKVPQCTDS, (SEQIDNO:3258); CCDS44547.1, Del, A_8, RSIFRGHRSWDLPIIVNLS, (SEQIDNO:3259); CCDS41715.1, Del, A_8, SRKKGHTKNRRKAKRKPQI, (SEQIDNO:3260); CCDS47917.1, Del, A_8, SWCLCCRLIAMKEMTWTKI, (SEQIDNO:3261); CCDS56334.1, Del, A_8, AYPSEKSNKKISVLSFET, (SEQIDNO:3262); CCDS8740.1, Del, A_8, GNPVQESPQHQMIQDAQG, (SEQIDNO:3263); CCDS30576.1, Ins, A_8, IPAEPTGCCAHPKVLPKL, (SEQIDNO:3264); CCDS58104.1, Del, A_8, IQASTSPSSQHGCMRDLP, (SEQIDNO:3265); CCDS6467.1, Ins, A_8, KCHCYRELYLSNREDFEV, (SEQIDNO:3267); CCDS7136.1, Ins, A_8, KDWQQECGCIKTRCFRKK, (SEQIDNO:3268); CCDS32174.1, Ins, A_8, KGPPSTCQERLYESSGSW, (SEQIDNO:3269); CCDS1521.1, Ins, A_8, KQWEDPTSPANVIALLQT, (SEQIDNO:3270); CCDS 11459.2, Ins, A_8, KVQPNASQAQQKPTTHGR, (SEQIDNO:3271); CCDS55123.1, Ins, A_8, MLFRTENSLWRESASFNL, (SEQIDNO:3273); CCDS239.2, Del, A_8, MTLKALVKTWTQFRNYPR, (SEQIDNO:3274); CCDS42277.1, Ins, A_8, NSRICDLSAIPKQWNYQP, (SEQIDNO:3275); CCDS34218.1, Ins, A_8, NWMWQFWRITIREKFIHK, (SEQIDNO:3276); CCDS6274.1, Del, A_8, RKRRSKVKITLLHRTQKN, (SEQIDNO:3277); CCDS7944.1, Del, A_8, RSHFRLGNSEMSSIRKKI, (SEQIDNO:3278); CCDS32234.1, Del, A_8, YRKLYQKQLEKRIKHLCS, (SEQIDNO:3279); CCDS 13177.1, Del, A_8, AGTDQGTAGNNAKMEKQ, (SEQIDNO:3280); CCDS31668.1, Del, A_8, IIVVENYIGIISCQMEL, (SEQIDNO:3281); CCDS3189.1, Ins, A_8, IWQDSRNIWKIGGLRSH, (SEQIDNO:3282); CCDS621.1, Ins, A_8, KCRSYWTSWRTKEQSRL, (SEQIDNO:3283); CCDS3503.1, Del, A_8, KPNKNTQITFSLMSFTL, (SEQIDNO:3284); CCDS1202.1, Ins, A_8, KPVPWCGGIGCERNNWG, (SEQIDNO:3285); CCDS13704.1, Ins, A_8, KQNQHPSPNLYCGQNKK, (SEQIDNO:3286); CCDS10131.1, Ins, A_8, NEERTKNSPLSSYGNNL, (SEQIDNO:3287); CCDS6184.1, Ins, A_8, NLGSIKGISDGENRTWL, (SEQIDNO:3288); CCDS58128.1, Ins, A_8, NPSPAEGASLWEEGFII, (SEQIDNO:3289); CCDS44708.1, Ins, A_8, NSTDWGWHCSSIMPCNF, (SEQIDNO:3290); CCDS3754.1, Del, A_8, RLVRGRRLRTAENVKNN, (SEQIDNO:3291); CCDS42031.1, Ins, A_8, RQGEKRDQKGEERAQEG, (SEQIDNO:3292); CCDS2194.1, Del, A_8, RQTLRTLSLRGMVPRTL, (SEQIDNO:3293); CCDS47443.1, Ins, A_8, RSCNFLSRTVGCFPSSC, (SEQIDNO:3294); CCDS13651.1, Ins, A_8, RSKKVSTRKNGGGLKRK, (SEQIDNO:3295); CCDS2191.1, Ins, A_8, SCRECHQTKETLASKED, (SEQIDNO:3296); CCDS1404.1, Del, A_8, SDQNLFTEIILNPPKHQ, (SEQIDNO:3297); CCDS10374.2, Ins, A_8, SKSQKTCPQKNSAQTSC, (SEQIDNO:3298); CCDS13117.1, Del, A_8, TLFNILQTLLSKKNKGH, (SEQIDNO:3299); CCDS59367.1, Del, A_8, FSNAKNVANHFACFHI, (SEQIDNO:3300); CCDS34472.1, Del, A_8, IPWMNLEPRLLNCCAL, (SEQIDNO:3301); CCDS3627.1, Del, A_8, IPWMTFQRVCGKEEAS, (SEQIDNO:3302); CCDS41623.1, Ins, A_8, ITTRNSSIRASWRIEV, (SEQIDNO:3303); CCDS2518.1, Ins, A_8, KAPPLRPRLRLRGRLR, (SEQIDNO:3304); CCDS12904.1, Ins, A_8, KCCGNGPRVCRKQNSE, (SEQIDNO:3305); CCDS43270.1, Ins, A_8, KCRKRKAAEKSEKEEG, (SEQIDNO:3306); CCDS44547.1, Ins, A_8, KEAYSAATAAGISQLL, (SEQIDNO:3307); CCDS47535.1, Ins, A_8, KEEEKEETFKKIRGLC, (SEQIDNO:3308); CCDS3645.1, Ins, A_8, KEIFTYEINHPGSWHI, (SEQIDNO:3309); CCDS 1146.1, Ins, A_8, KEKEKEAERGGRSYSI, (SEQIDNO:3310); CCDS4235.1, Ins, A_8, KGRTTAGSEQQDLEHR, (SEQIDNO:3311); CCDS3587.1, Ins, A_8, KTSSKAYRYKLLQLGR, (SEQIDNO:3313); CCDS2874.1, Ins, A_8, KVCQGRKPVGSEHHCL, (SEQIDNO:3314); CCDS34480.1, Del, A_8, LGNMMFLKPMVKFRIC, (SEQIDNO:3315); CCDS58214.1, Del, A_8, LKTFHMKCFLNLSQME, (SEQIDNO:3316); CCDS55599.1, Del, A_8, LLKNSNCRACFCCGQS, (SEQIDNO:3317); CCDS6827.1, Del, A_8, LWLIVLTQLVLNLVQE, (SEQIDNO:3318); CCDS643.1, Del, A_8, MPERLPNLKLGDMYFL, (SEQIDNO:3319); CCDS3457.1, Del, A_8, MSPPFMPSRVLFMSRR, (SEQIDNO:3320); CCDS44185.1, Ins, A_8, NIFSNLLWKYTWNGDL, (SEQIDNO:3321); CCDS5622.1, Del, A_8, REKRQAVNMMCQHTMI, (SEQIDNO:3322); CCDS34679.1, Del, A_8, RERKFYWKRNFKATVK, (SEQIDNO:3323); CCDS43379.1, Del, A_8, RESTLGNFWMKLLQLP, (SEQIDNO:3324); CCDS58166.1, Del, A_8, RSQNHQKSPQALAMPW, (SEQIDNO:3325); CCDS1414.2, Del, A_8, RVGSMSFEVPSTKRSV, (SEQIDNO:3326); CCDS33237.1, Del, A_8, SSHMHNTSPCQMMSHL, (SEQIDNO:3327); CCDS5091.2, Del, A_8, FRRDFRKFSNRMISL, (SEQIDNO:3328); CCDS12495.1, Del, A_8, IHAMNVGNLSAGNQY, (SEQIDNO:3329); CCDS14214.1, Ins, A_8, IKEGAPRSPRKTEKG, (SEQIDNO:3330); CCDS44153.1, Ins, A_8, KAQGTDEFQELCRRT, (SEQIDNO:3331); CCDS31590.1, Ins, A_8, KEHPCHSGPYSQLPG, (SEQIDNO:3332); CCDS34679.1, Ins, A_8, KEKESFIGNETSRQQ, (SEQIDNO:3333); CCDS8302.1, Ins, A_8, KESHDCGGVSPFGKQ, (SEQIDNO:3334); CCDS41329.1, Ins, A_8, KFVMLFTWFSDSRRR, (SEQIDNO:3335); CCDS31426.1, Ins, A_8, KPHKESPAHYRRRCI, (SEQIDNO:3336); CCDS47630.1, Ins, A_8, KVNSCISSDYSYTSV, (SEQIDNO:3337); CCDS30948.1, Ins, A_8, KYGGSFKTPSYFPSE, (SEQIDNO:3338); CCDS9300.2, Del, A_8, LKPYRSVLVLIKMFF, (SEQIDNO:3339); CCDS2217.1, Del, A_8, LQKKEIAKNVFVQNI, (SEQIDNO:3340); CCDS3212.2, Ins, A_8, NRSYLCNESCEKRAC, (SEQIDNO:3342); CCDS5000.2, Ins, A_8, REYFLGLSWPSPEES, (SEQIDNO:3343); CCDS58956.1, Del, A_8, RMILHFWKKKNAVIT, (SEQIDNO:3344); CCDS4678.1, Del, A_8, RPENSQINSSLCNEA, (SEQIDNO:3345); CCDS8902.1, Del, A_8, RRPPRLQRMPPVGKH, (SEQIDNO:3346); CCDS14008.1, Del, A_8, RSVKRCKRSLPKGIV, (SEQIDNO:3347); CCDS54103.1, Del, A_8, SLGIIWPKSGLILTA, (SEQIDNO:3348); CCDS31612.1, Ins, A_8, TESSAPGQPWGQQEI, (SEQIDNO:3349); CCDS43119.1, Ins, A_8, TKSTRKQEQRFRKRD, (SEQIDNO:3350); CCDS54426.1, Del, A_8, TQLLLFIRQMYRIMV, (SEQIDNO:3351); CCDS3998.1, Del, A_8, TWRKLADSLVCSWIT, (SEQIDNO:3352); CCDS6354.1, Del, A_8, CWSQILTALWRGRV, (SEQIDNO:3354); CCDS31774.1, Del, A_8, GNFQRTTCHSILSS, (SEQIDNO:3355); CCDS33960.1, Del, A_8, IICIHIELLFLPLP, (SEQIDNO:3356); CCDS47846.1, Ins, A_8, ISETSLWQWQIGGK, (SEQIDNO:3357); CCDS43434.1, Ins, A_8, KCKFGRLLYSTWIF, (SEQIDNO:3358); CCDS43744.1, Ins, A_8, KCNYGIIRNEKTAS, (SEQIDNO:3359); CCDS1584.1, Ins, A_8, KDVCIEAYTSYSVS, (SEQIDNO:3360); CCDS5388.1, Ins, A_8, KEKDDVSHNQKCRI, (SEQIDNO:3361); CCDS32146.1, Ins, A_8, KENLWSCHPQKEVY, (SEQIDNO:3362); CCDS58104.1, Ins, A_8, KFRPAPPHLPSMGV, (SEQIDNO:3363); CCDS6538.1, Ins, A_8, KFWSKLWDSKETRL, (SEQIDNO:3364); CCDS45016.1, Ins, A_8, KGSQEKGCIRIPVS, (SEQIDNO:3365); CCDS42281.1, Ins, A_8, KHTWKCITDIRLLK, (SEQIDNO:3366); CCDS2889.1, Ins, A_8, KKDFSSKEVKTICS, (SEQIDNO:3367); CCDS4528.1, Del, A_8, KMPRGLKDGGWSER, (SEQIDNO:3368); CCDS33191.1, Ins, A_8, KNRIKMYKLLFNYV, (SEQIDNO:3369); CCDS34865.1, Ins, A_8, KQEPPCTRLHGNIL, (SEQIDNO:3370); CCDS5787.1, Ins, A_8, KSPTNYKYNIHTDD, (SEQIDNO:3371); CCDS44512.1, Ins, A_8, KVRLQVALHLSPWS, (SEQIDNO:3372); CCDS34498.1, Ins, A_8, KYRVNSTIGTSSTV, (SEQIDNO:3373); CCDS44708.1, Del, A_8, LNRLGLALLQHHAL, (SEQIDNO:3375); CCDS11885.1, Del, A_8, MIRKIQKRRRTANR, (SEQIDNO:3376); CCDS10558.1, Del, A_8, MKMACGKRSLLQEV, (SEQIDNO:3377); CCDS12392.1, Ins, A_8, NTHRESYYSQNSGH, (SEQIDNO:3378); CCDS47945.1, Del, A_8, QIQFVTLGKSSVQT, (SEQIDNO:3379); CCDS41346.1, Del, A_8, QQLKWTPHILKSAS, (SEQIDNO:3380); CCDS33506.1, Ins, A_8, RDHSEEGSGGPCAE, (SEQIDNO:3381); CCDS4306.1, Ins, A_8, RKEEESKKGLNQRF, (SEQIDNO:3382); CCDS34606.1, Del, A_8, RKENTVKRPNLPHL, (SEQIDNO:3383); CCDS54605.1, Ins, A_8, RKLYAHKTRNRKFK, (SEQIDNO:3384); CCDS10131.1, Del, A_8, RRKNKKQPTFFLWQ, (SEQIDNO:3385); CCDS4203.1, Del, A_8, SIFLAKESSGRAYV, (SEQIDNO:3386); CCDS33770.2, Ins, A_8, SKEADGIASFGRKK, (SEQIDNO:3387); CCDS3411.2, Ins, A_8, SSLKQKEKKRFKEC, (SEQIDNO:3388); CCDS14682.1, Ins, A_8, TFRYKGKQHPFFST, (SEQIDNO:3389); CCDS 13704.1, Del, A_8, TKPTSFPEPLLWTK, (SEQIDNO:3390); CCDS3673.1, Del, A_8, TKSGKKQELTFLLL, (SEQIDNO:3391); CCDS6848.2, Del, A_8, TVLSLVTISRFVLS, (SEQIDNO:3392); CCDS4244.1, Del, A_8, CICSSIGRPGICC, (SEQIDNO:3393); CCDS32528.1, Del, A_8, GHLLLDYKRRLWN, (SEQIDNO:3394); CCDS110.1, Del, A_8, HQRCAASQQSASF, (SEQIDNO:3395); CCDS761.1, Ins, A_8, IAWSPWHENFRSR, (SEQIDNO:3396); CCDS33814.1, Ins, A_8, IKGGKGGNVPAKF, (SEQIDNO:3397); CCDS33894.1, Del, A_8, IPSLNAQWMFLRI, (SEQIDNO:3398); CCDS34901.1, Ins, A_8, ISFGCESTLRSSH, (SEQIDNO:3399); CCDS6018.1, Ins, A_8, KAGKRRRGNKSQR, (SEQIDNO:3401); CCDS3457.1, Ins, A_8, KCRHLLCPAGYSS, (SEQIDNO:3402); CCDS2234.1, Ins, A_8, KGGSARQCGSCYT, (SEQIDNO:3403); CCDS54426.1, Ins, A_8, KLSFCCLSGRCTG, (SEQIDNO:3404); CCDS813.1, Ins, A_8, KQGPGRSHPGTVH, (SEQIDNO:3405); CCDS6149.1, Ins, A_8, KSNPGQVCTPNFL, (SEQIDNO:3406); CCDS58398.1, Ins, A_8, KSWSEPVPKCAIQ, (SEQIDNO:3407); CCDS3189.1, Del, A_8, LAGFQEYMEDWGT, (SEQIDNO:3408); CCDS33909.1, Del, A_8, MKSIWRANSSQSK, (SEQIDNO:3409); CCDS13482.1, Ins, A_8, NKFSGTYTRNTKQ, (SEQIDNO:3410); CCDS30715.1, Ins, A_8, NSKLLYFCGQSEY, (SEQIDNO:3411); CCDS9435.1, Del, A_8, RCSLMEQIPHLKS, (SEQIDNO:3412); CCDS13651.1, Ins, A_8, RNPRKTKITEEEN, (SEQIDNO:3413); CCDS43525.1, Del, A_8, RRSKIEASPIGSI, (SEQIDNO:3414); CCDS7484.1, Del, A_8, RSLGPKKMFQNPG, (SEQIDNO:3415); CCDS53456.1, Del, A_8, RVGFEVPSTKRSV, (SEQIDNO:3416); CCDS46451.1, Del, A_8, SGSQSGTKMKATH, (SEQIDNO:3417); CCDS46613.1, Del, A_8, SPSLQTQWRLKRS, (SEQIDNO:3418); CCDS9137.1, Ins, A_8, TARSHRRAREQTV, (SEQIDNO:3419); CCDS8937.2, Ins, A_8, TGGRICKQKQVYI, (SEQIDNO:3420); CCDS7146.1, Del, A_8, TLIGTCPKSLLCH, (SEQIDNO:3421); CCDS5048.1, Del, A_8, TLNWKRGPSVVPW, (SEQIDNO:3422); CCDS55858.1, Del, A_8, TMSLSNILMKFRL, (SEQIDNO:3423); CCDS6082.1, Del, A_8, WKQLHSSGNVLNG, (SEQIDNO:3424); CCDS41623.1, Del, A_8, YNKEFLHQGQLEN, (SEQIDNO:3425); CCDS14469.1, Del, A_8, GLNKMNSGWSLK, (SEQIDNO:3426); CCDS3740.1, Ins, A_8, IGGLSPGHRGRN, (SEQIDNO:3427); CCDS7283.1, Ins, A_8, IKASIIFRYFYS, (SEQIDNO:3428); CCDS5091.2, Ins, A_8, ISEETSGNSQTE, (SEQIDNO:3429); CCDS5787.1, Del, A_8, ISNKLQIQHPHR, (SEQIDNO:3430); CCDS7142.1, Del, A_8, IVIILVLDLDLP, (SEQIDNO:3431); CCDS33237.1, Ins, A_8, KAAICTTPALVK, (SEQIDNO:3432); CCDS55244.1, Ins, A_8, KCCRSNAVTTWL, (SEQIDNO:3433); CCDS3719.1, Ins, A_8, KCFVCSLLLHSK, (SEQIDNO:3434); CCDS9393.1, Ins, A_8, KEKKGTSFSLFL, (SEQIDNO:3436); CCDS58166.1, Ins, A_8, KEVKTTRKVHKP, (SEQIDNO:3437); CCDS8929.1, Ins, A_8, KGESLLGGDCAV, (SEQIDNO:3438); CCDS13416.1, Ins, A_8, KGGDDSRGKVSV, (SEQIDNO:3439); CCDS54426.1, Ins, A_8, KGVPKDRDCEGS, (SEQIDNO:3440); CCDS3504.1, Del, A_8, KLINLNLFMKHY, (SEQIDNO:3441); CCDS32123.1, Ins, A_8, KLQNEYKLWARE, (SEQIDNO:3442); CCDS47028.1, Del, A_8, LIWQVFLKMILG, (SEQIDNO:3444); CCDS54773.1, Del, A_8, LPRNLFQMSTSF, (SEQIDNO:3445); CCDS2191.1, Del, A_8, LQRMSSDKRNTG, (SEQIDNO:3446); CCDS8740.1, Ins, A_8, METQFRSPLNIR, (SEQIDNO:3447); CCDS2237.1, Del, A_8, MKKENKIKWKLN, (SEQIDNO:3448); CCDS41355.1, Ins, A_8, NRSNKVFRRYLW, (SEQIDNO:3449); CCDS44989.1, Ins, A_8, NSLLPAATEDLK, (SEQIDNO:3450); CCDS58480.1, Del, A_8, PPLRFNLILMMY, (SEQIDNO:3451); CCDS8740.1, Del, A_8, RKLLNQKKKLRK, (SEQIDNO:3453); CCDS11592.1, Del, A_8, RLRIPSLRKYRQ, (SEQIDNO:3454); CCDS44770.1, Del, A_8, RLRKKRKRRRRK, (SEQIDNO:3455); CCDS879.1, Ins, A_8, RQENTNIFIGNT, (SEQIDNO:3456); CCDS34933.1, Ins, A_8, RRGRTARAARRN, (SEQIDNO:3457); CCDS6367.2, Ins, A_8, SEIGRQKLNSIW, (SEQIDNO:3458); CCDS9088.1, Del, A_8, SISKIFSTKKSH, (SEQIDNO:3459); CCDS55053.1, Del, A_8, SREKLLKPNKGL, (SEQIDNO:3460); CCDS3982.2, Ins, A_8, SRKCSKTSRKKK, (SEQIDNO:3461); CCDS6184.1, Del, A_8, SRKHQRHFRWRK, (SEQIDNO:3462); CCDS31654.1, Ins, A_8, SSGVPSEAGYSI, (SEQIDNO:3463); CCDS45847.1, Ins, A_8, SVTFTIGIICLF, (SEQIDNO:3464); CCDS11512.1, Ins, A_8, SWEKEFTSTTAQ, (SEQIDNO:3465); CCDS2924.1, Del, A_8, TKKWRKTVMAAT, (SEQIDNO:3466); CCDS47867.1, Del, A_8, TLVMVATVKLFL, (SEQIDNO:3467); CCDS1765.1, Del, A_8, TTGLVNFQLVSF, (SEQIDNO:3468); CCDS34948.1, Del, A_8, FLFHNVVLLEE, (SEQIDNO:3469); CCDS6082.1, Ins, A_8, IGNNCTAAEMS, (SEQIDNO:3470); CCDS13458.1, Del, A_8, ITFSCVMQPKS, (SEQIDNO:3471); CCDS9016.1, Ins, A_8, IYSRKKSHYSS, (SEQIDNO:3472); CCDS31309.1, Ins, A_8, KAEAKEVLCLL, (SEQIDNO:3473); CCDS32319.1, Ins, A_8, KEEQNQPLSAR, (SEQIDNO:3474); CCDS42721.1, Ins, A_8, KERTEGQKTEF, (SEQIDNO:3475); CCDS58167.1, Ins, A_8, KEVKTTRKEKF, (SEQIDNO:3476); CCDS46032.1, Ins, A_8, KLFPKSDTEKI, (SEQIDNO:3477); CCDS46031.1, Ins, A_8, KLFPKSDTENI, (SEQIDNO:3478); CCDS9684.1, Ins, A_8, KSGSSATGMDD, (SEQIDNO:3479); CCDS7632.1, Ins, A_8, KSKIGVLHTCL, (SEQIDNO:3480); CCDS44730.1, Ins, A_8, KSVNSDGHEMH, (SEQIDNO:3481); CCDS31879.1, Del, A_8, LINPDKKIKMI, (SEQIDNO:3482); CCDS9340.1, Del, A_8, LTSLQKLKSPK, (SEQIDNO:3483); CCDS35419.1, Del, A_8, MMRCRRHRTGS, (SEQIDNO:3485); CCDS7659.1, Del, A_8, MNLPFGSFMSQ, (SEQIDNO:3486); CCDS6467.1, Del, A_8, MSLLQRTVSQQ, (SEQIDNO:3487); CCDS5147.1, Del, A_8, MWRRSIGSGSQ, (SEQIDNO:3488); CCDS56026.1, Ins, A_8, NEGLSTEKTAE, (SEQIDNO:3489); CCDS2045.1, Ins, A_8, NENRQVCTCCN, (SEQIDNO:3490); CCDS41943.1, Del, A_8, QDRIFRVFQNH, (SEQIDNO:3491); CCDS10010.1, Del, A_8, RASFDLPGKAL, (SEQIDNO:3492); CCDS5000.2, Del, A_8, RILLGTFLAIP, (SEQIDNO:3493); CCDS41630.1, Ins, A_8, RRLIYRGNNYR, (SEQIDNO:3494); CCDS32270.1, Del, A_8, RRNLQRNLKVL, (SEQIDNO:3495); CCDS6463.1, Del, A_8, RSQPSWSRLFH, (SEQIDNO:3496); CCDS34925.1, Del, A_8, SFSKQWQNFTF, (SEQIDNO:3497); CCDS95.1, Del, A_8, SLVMKGQIPVR, (SEQIDNO:3498); CCDS3740.1, Ins, A_8, SSGRRTRKDLP, (SEQIDNO:3499); CCDS11623.1, Ins, A_8, SSLWLLVSFYS, (SEQIDNO:3500); CCDS9911.2, Del, A_8, SYALSNKNLLI, (SEQIDNO:3501); CCDS10112.1, Del, A_8, SYMKHTLGLNF, (SEQIDNO:3502); CCDS1404.1, Ins, A_8, TATKIYSQRSY, (SEQIDNO:3503); CCDS45002.1, Ins, A_8, TEAKAPEVNTS, (SEQIDNO:3505); CCDS32973.1, Ins, A_8, TFQMHRMWQSF, (SEQIDNO:3506); CCDS47639.1, Del, A_8, THPCTMLLPQG, (SEQIDNO:3507); CCDS5049.1, Del, A_8, TLNWKRNLLFG, (SEQIDNO:3508); CCDS11928.1, Del, A_8, YLEIILFHSMR, (SEQIDNO:3509); CCDS5987.2, Del, A_8, YMMKKLLNSHT, (SEQIDNO:3510); CCDS32306.1, Del, A_8, ASSPGHPLGP, (SEQIDNO:3511); CCDS7434.1, Del, A_8, EVTRCCFFHK, (SEQIDNO:3512); CCDS44989.1, Del, A_8, FFTTCGNRRS, (SEQIDNO:3513); CCDS30715.1, Del, A_8, FQAPLLLWTK, (SEQIDNO:3514); CCDS34061.1, Del, A_8, IAMTMDQYPF, (SEQIDNO:3515); CCDS34721.1, Del, A_8, IEITPQRSKA, (SEQIDNO:3516); CCDS55858.1, Del, A_8, IKALLTLNSL, (SEQIDNO:3517); CCDS33179.1, Ins, A_8, IKGNISKSST, (SEQIDNO:3518); CCDS46302.1, Ins, A_8, IRSHRRKETS, (SEQIDNO:3519); CCDS1838.1, Ins, A_8, ISDFKAPILL, (SEQIDNO:3520); CCDS31209.1, Ins, A_8, KANSHCEHHL, (SEQIDNO:3521); CCDS46613.1, Ins, A_8, KAQAYKPSGD, (SEQIDNO:3522); CCDS6551.2, Ins, A_8, KARRVSTIVL, (SEQIDNO:3523); CCDS6948.1, Ins, A_8, KEEKVQSPGI, (SEQIDNO:3525); CCDS41941.1, Ins, A_8, KEETTQDSNK, (SEQIDNO:3526); CCDS46348.1, Ins, A_8, KFCHIIYSVS, (SEQIDNO:3527); CCDS4035.1, Ins, A_8, KLGQSTRAVY, (SEQIDNO:3528); CCDS33143.1, Ins, A_8, KPFKFSVQLD, (SEQIDNO:3529); CCDS13651.1, Ins, A_8, KQISRSSQSL, (SEQIDNO:3530); CCDS55045.1, Ins, A_8, KRSIGKESQD, (SEQIDNO:3531); CCDS5048.1, Ins, A_8, KRSIGKEVLL, (SEQIDNO:3532); CCDS34218.1, Del, A_8, LDVAILENYD, (SEQIDNO:3533); CCDS30745.1, Del, A_8, LGNHWILLVS, (SEQIDNO:3534); CCDS45842.1, Del, A_8, LLPSFKMKEL, (SEQIDNO:3535); CCDS42801.1, Del, A_8, LWTIRNYLLH, (SEQIDNO:3536); CCDS11870.2, Ins, A_8, MGNSFSKENP, (SEQIDNO:3537); CCDS11928.1, Ins, A_8, NIWRSSYSTV, (SEQIDNO:3538); CCDS34022.1, Ins, A_8, NLGQSEDCKI, (SEQIDNO:3539); CCDS33197.2, Ins, A_8, NLIQFPKDSP, (SEQIDNO:3540); CCDS55858.1, Ins, A_8, NNYAGNEGIK, (SEQIDNO:3541); CCDS46919.1, Ins, A_8, NPAKTRIWHC, (SEQIDNO:3542); CCDS9344.1, Ins, A_8, NSVSTTTGFR, (SEQIDNO:3543); CCDS41623.1, Del, A_8, NSWSQIAGRH, (SEQIDNO:3544); CCDS9344.1, Ins, A_8, NTERPKIRTN, (SEQIDNO:3545); CCDS11319.1, Ins, A_8, NYKRPWINQP, (SEQIDNO:3546); CCDS31023.1, Del, A_8, PRHWISCLKK, (SEQIDNO:3547); CCDS13433.1, Del, A_8, RLPCKVTESS, (SEQIDNO:3548); CCDS53936.1, Ins, A_8, RNTNTPGSAS, (SEQIDNO:3549); CCDS7414.1, Del, A_8, SLFRETQSNR, (SEQIDNO:3550); CCDS3920.1, Del, A_8, SLILSFQRVK, (SEQIDNO:3551); CCDS3740.1, Ins, A_8, SQSKDQYNGT, (SEQIDNO:3552); CCDS31309.1, Del, A_8, SRGQRSLVSA, (SEQIDNO:3553); CCDS1202.1, Del, A_8, TCPLVRWNRM, (SEQIDNO:3554); CCDS4041.1, Ins, A_8, TSYRHENACF, (SEQIDNO:3555); CCDS56156.1, Del, A_8, TTSQSQLSSE, (SEQIDNO:3556); CCDS45820.1, Del, A_8, VPLTKLSEVF, (SEQIDNO:3557); CCDS58899.1, Del, A_8, CKPRFHFLP, (SEQIDNO:3558); CCDS6149.1, Del, A_8, IESWTSLHP, (SEQIDNO:3559); CCDS34948.1, Ins, A_8, IFFSTMWFS, (SEQIDNO:3560); CCDS761.1, Del, A_8, IFSLIMKSQ, (SEQIDNO:3561); CCDS42411.1, Ins, A_8, IKIGKKHQR, (SEQIDNO:3562); CCDS41329.1, Del, A_8, IRHAFYLVL, (SEQIDNO:3563); CCDS42721.1, Del, A_8, IRKTSQVLT, (SEQIDNO:3564); CCDS3189.1, Ins, A_8, KAEIARRKR, (SEQIDNO:3566); CCDS41346.1, Ins, A_8, KDSRCNPSP, (SEQIDNO:3567); CCDS58.1, Ins, A_8, KEASSEVHS, (SEQIDNO:3568); CCDS35069.1, Ins, A_8, KECQEEADS, (SEQIDNO:3569); CCDS6948.1, Ins, A_8, KEKVQSPGI, (SEQIDNO:3570); CCDS2881.1, Ins, A_8, KFQHKSAGI, (SEQIDNO:3572); CCDS1113.2, Ins, A_8, KKRKKTSVD, (SEQIDNO:3573); CCDS3673.1, Ins, A_8, KPNLERSKS, (SEQIDNO:3575); CCDS32062.1, Ins, A_8, KYDRKFLFV, (SEQIDNO:3576); CCDS34233.1, Ins, A_8, KYKQQNSTG, (SEQIDNO:3577); CCDS47757.1, Ins, A_8, KYQAGICSV, (SEQIDNO:3578); CCDS45264.1, Del, A_8, LCLLSEMWP, (SEQIDNO:3579); CCDS54564.1, Del, A_8, LRLAVLVNN, (SEQIDNO:3580); CCDS3719.1, Del, A_8, MFCLQFVIA, (SEQIDNO:3581); CCDS34681.1, Del, A_8, MKSKTSCVV, (SEQIDNO:3582); CCDS7588.1, Del, A_8, QHLLEVNPY, (SEQIDNO:3583); CCDS2482.1, Del, A_8, QLTRDWKAS, (SEQIDNO:3584); CCDS8354.1, Del, A_8, RGTKSMKVT, (SEQIDNO:3585); CCDS9933.1, Del, A_8, RKKGNMWTT, (SEQIDNO:3586); CCDS30860.1, Ins, A_8, RKKRIFTYS, (SEQIDNO:3587); CCDS1685.1, Del, A_8, SKIKSPFTH, (SEQIDNO:3588); CCDS43267.1, Del, A_8, SLITQMMKH, (SEQIDNO:3589); CCDS42721.1, Del, A_8, SRTLMKMIS, (SEQIDNO:3590); CCDS7736.1, Del, A_8, SSSWTKTMG, (SEQIDNO:3591); CCDS47299.1, Del, A_8, SVWQRRRRI, (SEQIDNO:3592); CCDS2431.2, Ins, A_8, TFNMPYKSQ, (SEQIDNO:3593); CCDS1521.1, Del, A_8, TVGRPHISC, (SEQIDNO:3595); CCDS3740.1, Del, A_8, WRPFPWAQR, (SEQIDNO:3596); CCDS33175.2, Del, A_8, WVNTPRRRL, (SEQIDNO:3597); CCDS3587.1, Del, A_8, DFFQGLQI, (SEQIDNO:3598); CCDS41736.1, Del, A_8, DPAGKLKK, (SEQIDNO:3599); CCDS43482.1, Del, A_8, FHIKVATQ, (SEQIDNO:3600); CCDS5089.1, Del, A_8, FSILPSSR, (SEQIDNO:3601); CCDS14439.1, Del, A_8, ICQVIKRL, (SEQIDNO:3602); CCDS47751.2, Ins, A_8, IKLGPEML, (SEQIDNO:3603); CCDS1614.1, Ins, A_8, ISPTSQYN, (SEQIDNO:3604); CCDS2881.1, Del, A_8, ISTQICWN, (SEQIDNO:3605); CCDS54430.1, Del, A_8, IVVPKRNI, (SEQIDNO:3606); CCDS3468.1, Ins, A_8, KAEENWQR, (SEQIDNO:3607); CCDS643.1, Ins, A_8, KCQKGSPT, (SEQIDNO:3608); CCDS3754.1, Ins, A_8, KDWCEEEG, (SEQIDNO:3609); CCDS32270.1, Ins, A_8, KEGIFKGT, (SEQIDNO:3610); CCDS14214.1, Ins, A_8, KEVRCSGC, (SEQIDNO:3611); CCDS7148.1, Ins, A_8, KFLRAAVH, (SEQIDNO:3613); CCDS53722.1, Ins, A_8, KGGQEGRF, (SEQIDNO:3614); CCDS2702.1, Ins, A_8, KNFERESF, (SEQIDNO:3615); CCDS2924.1, Ins, A_8, KPKNGERQ, (SEQIDNO:3616); CCDS32306.1, Ins, A_8, KPLHQAIL, (SEQIDNO:3617); CCDS42721.1, Ins, A_8, KSEKQARS, (SEQIDNO:3618); CCDS47169.1, Ins, A_8, KSRQSIPP, (SEQIDNO:3619); CCDS8318.1, Ins, A_8, KYFDIQNI, (SEQIDNO:3620); CCDS3785.1, Del, A_8, LKLAEVGS, (SEQIDNO:3621); CCDS9884.1, Del, A_8, LVLLNWLP, (SEQIDNO:3622); CCDS9246.1, Del, A_8, MEDMIWES, (SEQIDNO:3623); CCDS45026.1, Del, A_8, MKKRIMVH, (SEQIDNO:3624); CCDS31023.1, Ins, A_8, NHGTGSVV, (SEQIDNO:3625); CCDS47917.1, Ins, A_8, NPGVSAAG, (SEQIDNO:3626); CCDS44839.1, Ins, A_8, NQRKISCS, (SEQIDNO:3627); CCDS54426.1, Ins, A_8, NTLRTECC, (SEQIDNO:3628); CCDS54773.1, Ins, A_8, NYQETYSR, (SEQIDNO:3629); CCDS1336.1, Del, A_8, QLLNLIAL, (SEQIDNO:3630); CCDS42686.1, Del, A_8, QTLQEFVI, (SEQIDNO:3632); CCDS46422.1, Ins, A_8, REYGHSCS, (SEQIDNO:3633); CCDS46286.1, Del, A_8, RNWLRSWL, (SEQIDNO:3634); CCDS42443.1, Del, A_8, RRMVLLKS, (SEQIDNO:3635); CCDS6940.1, Ins, A_8, SHSLSSVL, (SEQIDNO:3636); CCDS34882.1, Ins, A_8, SHSRGLRW, (SEQIDNO:3637); CCDS3445.1, Ins, A_8, SILGMAQG, (SEQIDNO:3638); CCDS31050.1, Del, A_8, SKMTQIIQ, (SEQIDNO:3639); CCDS56348.1, Ins, A_8, SVGRAFGG, (SEQIDNO:3640); CCDS6018.1, Del, A_8, SWKKKKRK, (SEQIDNO:3641); CCDS54586.1, Ins, A_8, THQSRKVK, (SEQIDNO:3642); CCDS55593.1, Del, A_8, WNLLFLRR, (SEQIDNO:3644); CCDS9344.1, Del, A_8, YRKTKNQN, (SEQIDNO:3645); CCDS4681.1, Del, A_9, KYCHLRLPSQAPLKGKRAQNQAQPNLLPQNQHHLLRQWTQTVQAPRFPLLKSRSSWIQPLW SQELWMPSQWRVLEILTN, (SEQIDNO:3646); CCDS6913.1, Ins, A_9, KMEGSQADEKTGAAAGTGGTGKAPGRGGGSGREGGPRAALHTERSPAGLHPGQCSVAGAS HLLGRSDCQSLCHLLCG, (SEQIDNO:3647); CCDS41878.1, Del, A_9, NLDLQHQRRRKKKKDKVEIRNRSPKANSTECQGEHSREQNLLNLVQLNPHSPHHRKDEEDH QKRHHHHNQKKMSV, (SEQIDNO:3648); CCDS30603.1, Del, A_9, KFLIHLRKVDTHHLQVVTKTVTATTADGQRMRRLKCKACRLRWARPEPAAQAPPKSHFPPH PSGPSRTSSLQLR, (SEQIDNO:3649); CCDS32004.1, Del, A_9, NYGHCNSPLAFLTQLLPRPTSNPWWMTRRDPFCGGNLISTKLENFVSGISAGTERRQMNLCFL Y, (SEQIDNO:3652); CCDS43378.1, Ins, A_9, KTGSNEVTTRYEEKKTGSVRKANRMPKDVNIQVRKKQKHETRRKSKYNEDFERAWREDLTI KR, (SEQIDNO:3653); CCDS6573.1, Del, A_9, MCSSLALPTGLTSLILPSSDLAVLISSSTSHFLMRSPVLPSSRLTCASPQLPRMWTWSSWLK, (SEQIDNO:3654); CCDS34225.1, Del, A_9, VIAILSSMCQEILNRHHRRKEVRKLKQILFQVEETKSGRFWIKHDLVSCLQSYLVMEA, (SEQIDNO:3656); CCDS517.1, Del, A_9, KQWSSVTSLEWTAQERGCSSWLMKQTWMKSWSLRDPSYRSILEYVSTRVLWMPTSTV, (SEQIDNO:3657); CCDS1207.1, Del, A_9, ALRSMPKSRNQVLFRRNLTPSQLRTLAPPYMLLPQSLSQSLSRKQIPSQSMLV, (SEQIDNO:3658); CCDS734.1, Del, A_9, TSRILNLQVLKKILRSFMKLYHSLVMCLRLRTKLEKALSALFIWPQHSYK, (SEQIDNO:3660); CCDS2256.1, Del, A_9, YKGRKERKTSVQGITTVILKRRTSLRRESFMKNFLAVTITGKKPRKSPGS, (SEQIDNO:3661); CCDS13289.1, Ins, A_9, KVICTFYPTDRTEIFTRKRSSHYSCCISHPKCEPVTEYCGWSEKCTK, (SEQIDNO:3663); CCDS11614.1, Ins, A_9, KRSLSLSVQANYTSSSTTLTRKSRYFFNRYISKQKLANIILELQFS, (SEQIDNO:3664); CCDS35176.2, Del, A_9, PGLRPSQHLLLYITLRVVKWIPALATASAWPAPSAKTVWHPTLLI, (SEQIDNO:3665); CCDS1113.2, Del, A_9, QRKVVKNPSQPVPLRNDGITNGNDSTRSCSISLKKSLEKMPLM, (SEQIDNO:3666); CCDS1551.1, Del, A 9, GRRKRSEGGVKRKKENVCKRRKRNVGEKKRKGFDGRKRKGDG, (SEQIDNO:3667); CCDS41878.1, Ins, A_9, KIWTSSTRGGGRRRKTKWKYGTEVQKQTAPSVKESTAESRIS, (SEQIDNO:3668); CCDS 11681.1, Ins, A_9, KSSFPATDFRPLPFCILVWPSTGGCFPVLFDPPANANNGLYF, (SEQIDNO:3669); CCDS8395.1, Del, A_9, NGIWEVMPRTLEGCISVKDHRTSQNHSKCITECVRTALN, (SEQIDNO:3670); CCDS43689.1, Del, A_9, NVNVCSPKILILKHQKMIRDFRRNLRKWLKSYKGKKQI, (SEQIDNO:3672); CCDS8723.1, Ins, A_9, KLESCGRCKGWTQSKDYIETKESENSIWEQDKKQPDQ, (SEQIDNO:3673); CCDS58290.1, Del, A_9, NQESALTMKMMMMKKMGITFIPLSLPPRSVTALKS, (SEQIDNO:3674); CCDS46450.1, Del, A_9, KKQTRRRKLLLLCKKNQILKKKGEKLKHCFKAWG, (SEQIDNO:3675); CCDS55048.1, Ins, A_9, KRRRRSKKSHRRGIETRRRKSKATGTYESEGKRS, (SEQIDNO:3676); CCDS43758.1, Ins, A_9, KIGSPRRRKIWKGWNQTICKYFSACYQNCFTQ, (SEQIDNO:3677); CCDS3368.1, Del, A_9, NIRKLIWTKQRSFLSLFPKLRATEQMTNVGC, (SEQIDNO:3679); CCDS8300.1, Del, A_9, WKKTQADCFFGLLKKIGLPQCGDSFLKRPLT, (SEQIDNO:3680); CCDS5061.1, Ins, A_9, KETFKKKTYTCAITTVKATETKAGKWSRWE, (SEQIDNO:3681); CCDS9151.1, Del, A_9, SQTKNRFSQKCRRPITPGNCRNRFTFWKPV, (SEQIDNO:3682); CCDS11681.1, Del, A_9, KLIPSYGFQASTLLHTGLAKHWWMFPCTF, (SEQIDNO:3683); CCDS58167.1, Del, A_9, CKSWKQNSMKKNRKGNACKLRQLSCRLV, (SEQIDNO:3684); CCDS7417.1, Del, A_9, TGMEILLWILLKMEIQIFKICLGEMQLC, (SEQIDNO:3685); CCDS33716.1, Del, A_9, CTNLSVPMMLTILIWKRVFILLLSDKK, (SEQIDNO:3686); CCDS58577.1, Del, A_9, GQAFGSFSADFSAPQVTRLRSLNHLLI, (SEQIDNO:3687); CCDS2054.1, Ins, A_9, KRRDHSCDHFPPQDHISFSGVKTDNPV, (SEQIDNO:3688); CCDS34139.1, Ins, A_9, KRSCIESLTKYQQQQQFYSWDSKSATL, (SEQIDNO:3689); CCDS10987.1, Ins, A_9, KTEKQEKQHGRSIGKRTRRYRSHPRED, (SEQIDNO:3690); CCDS35457.1, Ins, A_9, NSGRNRKEGNINPRECSFASDTYSDWH, (SEQIDNO:3691); CCDS3171.1, Del, A_9, CLESGTDHILTEILSISCKKMNQMEL, (SEQIDNO:3692); CCDS10124.1, Ins, A_9, KSRTLGNCGPYCTSKAYNNQPTMETH, (SEQIDNO:3693); CCDS34872.1, Ins, A_9, NFCDKASAKVLGPRREIENWTTECKQ, (SEQIDNO:3694); CCDS2316.1, Del, A_9, LQKKAPQKRIKLLRMLPALYLMACQ, (SEQIDNO:3695); CCDS5931.1, Del, A_9, RNKKTKLWFSLINIHHRKNPLLPMR, (SEQIDNO:3697); CCDS 13482.1, Del, A_9, TIKISQIQNQSVNKNFHIHLKRTYQ, (SEQIDNO:3698); CCDS32123.1, Del, A_9, KNLAIVEEEMFLVMGELNYVPLAL, (SEQIDNO:3699); CCDS34487.1, Ins, A_9, KTAGRGSRKAEAYSRRNGKGKKKT, (SEQIDNO:3700); CCDS9971.1, Del, A_9, LCTICTSYVSPWIIFTEMEYFTEM, (SEQIDNO:3701); CCDS34734.2, Del, A_9, NPRRKTWKMRKKYAQKPSQKNHLY, (SEQIDNO:3702); CCDS43133.1, Ins, A_9, NYCLFQRESAWWKQPGNSCSGDNF, (SEQIDNO:3703); CCDS53578.1, Ins, A_9, KCVRYIQGEGSCLSPPSSDGSLL, (SEQIDNO:3704); CCDS11672.1, Ins, A_9, KREETGRRRNDAASDMGKIHISS, (SEQIDNO:3705); CCDS3418.1, Ins, A_9, KSEEWTSQQSGLRTKGHSGQHPD, (SEQIDNO:3706); CCDS9462.1, Ins, A_9, KTGGIETSAGCKEKETRNFRKAH, (SEQIDNO:3707); CCDS5098.1, Del, A_9, NVLCKQQNGLPAPEHVGWEYLTG, (SEQIDNO:3708); CCDS9087.1, Ins, A_9, NYDGNSAARDSKCSEVSSIHVIL, (SEQIDNO:3709); CCDS3091.1, Ins, A_9, KRESFLWNHGYPTSTNRRPNDQ, (SEQIDNO:3710); CCDS11974.1, Del, A_9, KRNSRRATPTSKGSLRRKYLRV, (SEQIDNO:3711); CCDS13482.1, Ins, A_9, NERKVKRERIYQCSRILDKPNQ, (SEQIDNO:3712); CCDS2222.1, Del, A_9, NMEHRLTMDSALSLTKREGPET, (SEQIDNO:3713); CCDS5098.1, Ins, A_9, KMSCASNKMDSLLQNMWDGNI, (SEQIDNO:3715); CCDS3436.1, Del, A_9, KNKWNAMVVLNKSLNRVHLLG, (SEQIDNO:3716); CCDS43133.1, Del, A_9, LLFIPTGISLVETAREQLFRG, (SEQIDNO:3717); CCDS10063.1, Del, A_9, LLQPCLKALSVPLWKMPSRAS, (SEQIDNO:3718); CCDS9474.1, Ins, A_9, NPGEICPLHRANHCTQIEHHY, (SEQIDNO:3719); CCDS10154.1, Del, A_9, FREKRNYVKKKSKLKKKEKK, (SEQIDNO:3720); CCDS9300.2, Ins, A_9, KILLFQSIEHTCLKCFKRGC, (SEQIDNO:3721); CCDS34260.1, Del, A_9, KLLKKSSQLRRSWRNVSRIS, (SEQIDNO:3722); CCDS11974.1, Ins, A_9, KRGIPEGPPRPPRAACGGNI, (SEQIDNO:3723); CCDS56247.1, Ins, A_9, KRWSEKIAHVWHRDSGVSTA, (SEQIDNO:3724); CCDS43493.2, Del, A_9, KSATTTTVILEIIIMDRVIP, (SEQIDNO:3725); CCDS46547.1, Ins, A_9, KTRLFARGTVLLERRRYYWC, (SEQIDNO:3726); CCDS58578.1, Ins, A_9, KVKHLAVCANSFQPTFQLLK, (SEQIDNO:3727); CCDS3091.1, Del, A_9, KGELSLEPWIPYLHQQKTQ, (SEQIDNO:3728); CCDS9944.2, Ins, A_9, KIRLSEQELSEFSLSSSEY, (SEQIDNO:3729); CCDS3179.1, Ins, A_9, KPQQSESICHGNWKEDSSP, (SEQIDNO:3730); CCDS46450.1, Ins, A_9, KRNRPEEGSCCSCARRIRS, (SEQIDNO:3731); CCDS58294.1, Del, A_9, LTDFFVLWKASGTIQFNCK, (SEQIDNO:3732); CCDS731.1, Ins, A_9, KKAKNGCKQDNSLYRGLL, (SEQIDNO:3733); CCDS1207.1, Ins, A_9, KPYDLCPSPETRSSSEET, (SEQIDNO:3734); CCDS6343.1, Ins, A_9, KRRAQRRQSDSSYSVIEG, (SEQIDNO:3735); CCDS58191.1, Ins, A_9, NIPQPLEKNRFCSSSSAA, (SEQIDNO:3736); CCDS3630.1, Ins, A_9, NNSDHMWRLPFSCTLKRW, (SEQIDNO:3737); CCDS35176.2, Ins, A_9, NQACVPANTFCSTSRCEL, (SEQIDNO:3738); CCDS41630.1, Del, A_9, ILFHVRNIVYGIQVMLC, (SEQIDNO:3739); CCDS180.1, Ins, A_9, KTAENKEHSVKQDIERT, (SEQIDNO:3740); CCDS10374.2, Ins, A_9, KTEEEREQGEQGETNEF, (SEQIDNO:3741); CCDS2936.1, Del, A_9, NILNSLPSSMEIAVLQE, (SEQIDNO:3742); CCDS41816.1, Del, A_9, FQKNGALRIFLLLSKC, (SEQIDNO:3743); CCDS10124.1, Del, A_9, IQNLGKLWTLLYLKSL, (SEQIDNO:3744); CCDS11672.1, Del, A_9, KRRNRKKKKRCSKRHG, (SEQIDNO:3745); CCDS58577.1, Ins, A_9, KVKHLAVFQPTFQLLK, (SEQIDNO:3746); CCDS5627.1, Del, A_9, LGQIIMKKMRRPVCYK, (SEQIDNO:3747); CCDS41534.1, Ins, A_9, NGRLLTLLNYSEGSLE, (SEQIDNO:3748); CCDS10987.1, Del, A_9, NRKTRKAAREKHRKTD, (SEQIDNO:3749); CCDS41350.1, Del, A_9, NSRKKLLEDMKQNGFT, (SEQIDNO:3750); CCDS7352.1, Del, A_9, SNGMEACGTHPTPRLS, (SEQIDNO:3751); CCDS7352.1, Ins, A_9, KATEWRHAELTQHLA, (SEQIDNO:3752); CCDS11990.1, Ins, A_9, KENGIQLEQLGRNTL, (SEQIDNO:3753); CCDS56554.1, Ins, A_9, KIQGDFEALRCEGCD, (SEQIDNO:3754); CCDS54773.1, Del, A_9, LMLLRLNLRKMCGPF, (SEQIDNO:3755); CCDS34028.1, Del, A_9, LMNILKRVPKSLGLT, (SEQIDNO:3756); CCDS5411.1, Del, A_9, LTETVYSTTQQIHSS, (SEQIDNO:3757); CCDS10363.1, Del, A_9, MKALISQEMFSQALL, (SEQIDNO:3758); CCDS9151.1, Ins, A_9, NRKPKTDFRKNAGGQ, (SEQIDNO:3759); CCDS1589.1, Ins, A_9, KAQLHFAAESIIII, (SEQIDNO:3760); CCDS33771.1, Ins, A_9, KRIQLYLYEAMCRK, (SEQIDNO:3761); CCDS46495.1, Del, A_9, KRRKWQIRHVLKNL, (SEQIDNO:3762); CCDS9684.1, Ins, A_9, KSHQQVHGDFRKYI, (SEQIDNO:3763); CCDS31756.1, Ins, A_9, KSPDKENKAVFRGF, (SEQIDNO:3764); CCDS1852.1, Ins, A_9, NRRKEGPNSNREEY, (SEQIDNO:3765); CCDS10374.2, Del, A_9, NRRRKRTRRTRRNK, (SEQIDNO:3766); CCDS10154.1, Ins, A_9, NSERREIMSKRKAN, (SEQIDNO:3767); CCDS2256.1, Ins, A_9, NTKEEKKEKQVFRA, (SEQIDNO:3768); CCDS552.2, Del, A_9, RKMQSLNHPSRLTN, (SEQIDNO:3769); CCDS33451.1, Del, A_9, IASTFYLDGNVSS, (SEQIDNO:3771); CCDS55048.1, Del, A_9, KKKKKQEKPQKRN, (SEQIDNO:3772); CCDS9544.1, Del, A 9, KRQINRRKLQRKK, (SEQIDNO:3773); CCDS53961.1, Ins, A_9, KSQKDKSPDELQG, (SEQIDNO:3774); CCDS4681.1, Ins, A_9, KSTVTYGCQAKPL, (SEQIDNO:3775); CCDS8300.1, Ins, A_9, NGKRPKQIASLGC, (SEQIDNO:3776); CCDS53775.1, Ins, A_9, NSRAFSCNKEKGI, (SEQIDNO:3777); CCDS34233.1, Del, A_9, RKSGVMKCWDLCP, (SEQIDNO:3778); CCDS46450.1, Del, A_9, GEKLKHCFKAWG, (SEQIDNO:3779); CCDS41878.1, Ins, A_9, KCPCRTLQTSSY, (SEQIDNO:3780); CCDS32004.1, Ins, A_9, KITDIATHPWLS, (SEQIDNO:3781); CCDS2710.2, Del, A_9, KMEVPSCCLDHC, (SEQIDNO:3782); CCDS879.1, Ins, A_9, KRYSRKQATECL, (SEQIDNO:3783); CCDS34139.1, Ins, A_9, KSKSRFASRSKA, (SEQIDNO:3784); CCDS34872.1, Del, A_9, LLRQGKCQSAWS, (SEQIDNO:3785); CCDS34470.1, Ins, A_9, NDKSLCHCDRWT, (SEQIDNO:3786); CCDS35118.1, Ins, A_9, NKCSTNSTIRTA, (SEQIDNO:3787); CCDS6573.1, Ins, A_9, KCVHHWRYQPA, (SEQIDNO:3788); CCDS5931.1, Ins, A_9, KGTRKQNSGSL, (SEQIDNO:3789); CCDS1787.1, Del, A_9, KMLNQKCLAPF, (SEQIDNO:3790); CCDS5593.1, Del, A_9, KPGTSLQCYLN, (SEQIDNO:3791); CCDS7417.1, Ins, A_9, KQGWKYSFGSC, (SEQIDNO:3792); CCDS46495.1, Ins, A_9, KREGNGRSDMY, (SEQIDNO:3793); CCDS46547.1, Del, A_9, NKIICKGHRIT, (SEQIDNO:3794); CCDS11685.1, Ins, A_9, NKRIFKDNGTS, (SEQIDNO:3795); CCDS44556.1, Del, A_9, NQYQRSLQCWP, (SEQIDNO:3796); CCDS4246.1, Del, A_9, NWISKKLNLTM, (SEQIDNO:3797); CCDS42794.1, Del, A_9, ILAMTLTLNG, (SEQIDNO:3798); CCDS45704.1, Ins, A_9, KDTERGAKAI, (SEQIDNO:3799); CCDS1551.1, Ins, A_9, KEGGRGAKAA, (SEQIDNO:3800); CCDS3436.1, Ins, A_9, KRTNGTRWLF, (SEQIDNO:3801); CCDS5593.1, Ins, A_9, KSLVPHCNVT, (SEQIDNO:3802); CCDS2316.1, Ins, A_9, NFRRRHLRRE, (SEQIDNO:3803); CCDS2955.1, Ins, A_9, NKEEIAVHYL, (SEQIDNO:3804); CCDS47335.1, Del, A_9, NKVRQNFKRN, (SEQIDNO:3805); CCDS9474.1, Del, A_9, SGRNLPTAPC, (SEQIDNO:3806); CCDS47457.1, Del, A_9, VLTKQKVLYK, (SEQIDNO:3807); CCDS34734.2, Ins, A_9, KIPEEKLGR, (SEQIDNO:3809); CCDS11614.1, Del, A_9, KKPLTFSPS, (SEQIDNO:3810); CCDS56248.1, Del, A_9, KMEITMMIS, (SEQIDNO:3811); CCDS2710.2, Ins, A_9, KRWRCPVVV, (SEQIDNO:3812); CCDS389.1, Ins, A_9, KTYCYHKYK, (SEQIDNO:3813); CCDS58167.1, Ins, A_9, NARVGSKTP, (SEQIDNO:3814); CCDS41445.2, Ins, A_9, NEAFSHGAI, (SEQIDNO:3815); CCDS34487.1, Del, A_9, NSRKRKQKG, (SEQIDNO:3816); CCDS9971.1, Ins, A_9, NYALYVPVM, (SEQIDNO:3817); CCDS9684.1, Del, A_9, VPSASPWGL, (SEQIDNO:3818); CCDS13289.1, Del, A_9, GHLHLLPH, (SEQIDNO:3819); CCDS486.2, Ins, A_9, KCPPAALV, (SEQIDNO:3820); CCDS6343.1, Del, A_9, KKSTEKTK, (SEQIDNO:3822); CCDS41630.1, Ins, A_9, KSCSMSEI, (SEQIDNO:3823); CCDS43493.2, Ins, A_9, KSLLLLRR, (SEQIDNO:3824); CCDS11874.1, Ins, A_9, KTSENTPF, (SEQIDNO:3825); CCDS14131.1, Ins, A_9, KTTKKAET, (SEQIDNO:3826); CCDS879.1, Del, A_9, KVQQKASN, (SEQIDNO:3827); CCDS53577.1, Ins, A_9, KVRSLHTR, (SEQIDNO:3828); CCDS33716.1, Ins, A_9, NAQICQFQ, (SEQIDNO:3829); CCDS13093.1, Del, A_9, NMKPSLLL, (SEQIDNO:3830); CCDS47839.1, Ins, A_9, NTDWNKTL, (SEQIDNO:3831); CCDS13548.1, Del, C_10, LRSPQTSPLRLRTRWVWSGPQAGVSAARRPRWRCSTCRR, (SEQIDNO:3832); CCDS55731.1, Ins, C_12, PPYPPSWPLAEGAGLLLLCKRHCGFAFQHRNREKRFNVQMLFPIIHGCKS, (SEQIDNO:3833); CCDS55731.1, Del, C_12, PVPPELAPGRRSWFVVIMQAALWVCVSA, (SEQIDNO:3834); CCDS41411.1, Ins, C_7, LGVAGRLLGTSSGSHHLQGTPVPKPGPSPASPASRPPSWLGFGPAPAPTPACPSPKEEEPTWTA AHSGGAVRGGPGCQPSGWRGGPSREGAGRAGRPGGP, (SEQIDNO:3835); CCDS43088.1, Ins, C_7, PAASQGKQGRDVRRALTGSCGLAAAEPEQARPGQAGPEGPDWGEAAAEAAGRRRSSPRCFL QKSRPHSSGSAWRRCVTSGSGSTHGRSGRDRLPARGPV, (SEQIDNO:3836); CCDS8162.1, Del, C_7, RSCRRRPSCHRCTAWCCQSGTCSGRSRPSWPSCPIPRRDPRPGHRSASPGPKPSLRPSSWQEC DEAPSLRCLGLWCSPRRSPDHRGGNPRPAARLSMGS, (SEQIDNO:3837); CCDS46143.1, Del, C_7, SCSPRSSAMPPVPLPAPPKSASTSLSQPPAMGHPSHLHRHPRLHHPCPCSSRPTSAAMAWSPRP PAPACDPRRAWIRQAPCRNCSGLWSRCPRRLGILA, (SEQIDNO:3838); CCDS398.1, Del, C_7, CSRCSRHLAGLALALWGNQSSSWPITLRWTSLRSTCTTTRWTSSRISVPVESTGKWWNTWSS ISSLRSLVIASLCMMERRTFTLSQHCPLATNGSTLR, (SEQIDNO:3839); CCDS7667.2, Del, C_7, LPAGFSPPRPPAPSHPRPSCRRCPPEACPRTLPTSKAPPSLTRCGSGTRTSCGCPSRRPGPSSAR GGVCPATATRGASSSAPSACGRARCSVRSARAR, (SEQIDNO:3840); CCDS42092.1, Del, C_7, PARQARAQDRWRTQRPGPASSPGSRSPSHVRHLHHGWASVPGLPKVAMAPGTPGIRGPHAP PTWLPARPSRLPGSAAAPTLPRALARLPPAVGGCV, (SEQIDNO:3842); CCDS7845.1, Ins, C_7, TPAPGAGGGGGSPAGGAPLCPHHRPLQCRDWEDRLLHCHQHLLPAAAAGGCGGHPEDHVP APSGQGRHDPDMRAVPVCAPRHEPLRKAAVPPVPRM, (SEQIDNO:3843); CCDS47583.1, Del, C_7, CLVKKTVHLPIFPNSLAMVIRMSGCSSSSELTQTRGPRRPPGHQCYLSPHETWKVPRMQPGSR MQRLLKVLRTPQSLLKAQKNLYLHQHQQSSN, (SEQIDNO:3844); CCDS45544.1, Del, C_7, GPLGSRQLPAACSRPSPSPTARTNPGRHHQSSGRQSRATHRGRTDWARPSPRGDPCSGPPRGA QLSTVLNLPSHTPTGRPRPRVTSSASSSGRD, (SEQIDNO:3845); CCDS32732.1, Del, C_7, IAPSCLWQRGKTSSPFPRRPSTTSCSASCSSSASPSAPASSALASHCRASVTAPGTATSPTAPPS CCPATTTGLQPGLRTLPMDCCRLRSSRPP, (SEQIDNO:3846); CCDS35368.1, Del, C_7, RAAAAAAGGSPCRHLRAAEWGPYFLPRPPPPLPHPPHRPPPRPHRRAPGRWWCQQRCAAF A SMCSTVTCMDTSSPGPPDSPTSPMPLKIAVFPQ, (SEQIDNO:3848); CCDS11409.1, Ins, C_7, RLCPQSRGRGLHALPLQWLLDGRHPLRFQLLPQPHLQYLYRAGLHHPRDGPGAAGCLHGGT PENYHPPAPRLWGEWNWRQDPWLCRANLQRPCH, (SEQIDNO:3849); CCDS4856.1, Del, C_7, SPTIPCPTDSLLCSHLGETALPTRRPPAPTPCTDTESASGQAVRPCVKTWASLSNTSTQSTPWM TGVQPSAGYRCRWCSSWRSSSPRRASGCRP, (SEQIDNO:3850); CCDS55892.1, Del, C_7, SRSPKSMSPPGRTQLPPSQLPQPHRHRKTCSRRATPLSSLAAAPGARAGARHPPPTRRQRSLCS SQPSQPRPRSCLGTPLAGLPACPSPCPPVR, (SEQIDNO:3851); CCDS32435.1, Del, C_7, VGPLGRPSSRFHPRRRQPTACRMLYMHRGRRAEGHTHGRPTTCPCQWQPSPCPPPQSRERRP GCHPGKKQSWQRARPSRQQAPWAVCSPCWSRR, (SEQIDNO:3852); CCDS9278.1, Del, C_7, CSQRGQLGPFMNCPARNRRNTRREGWRITAGKPTRRSTSPRWKSVSPPSASGKTPSTWTPCV PSGTGVTSSKGSTRCGKRSSRRPWRWATRLR, (SEQIDNO:3853); CCDS10544.1, Ins, C_7, RGPGRAGQAGLGAGPQTSKYPTGGPVPIRRREDLGDGQRLSPTPTAGRRVRAGLPQLPP AML PGGPVAGSEWRNQGQGAPAVPSIDPKEEEAL, (SEQIDNO:3854); CCDS8421.1, Del, C_7, TSSPRTMSWLCCLGQIMASAVEASQPPTWPSMPRRTPVGPVSSPARQEGVRVCSGCVTCGET APMAAMTTAAAPCSHPQSWPVSLSRWRCASV, (SEQIDNO:3856); CCDS54707.1, Ins, C_7, ARVPVPDVTAAAAAAAAAAESGAAGRPGPRRARRECRPRAEPPMRGLPRLRVHAARPAGPA EDSLQPGRADGDPTAAGRAPCPVCLLASGAL, (SEQIDNO:3857); CCDS7667.2, Ins, C_7, SCPRGSAPRDHPPRATPAPRAAAARRKPAPVPSLLRRRPLPSPAVAPGHVQAVGAPAAAQDH QAHAAASVPQPRPGAPHPQHHPPAAAPGAL, (SEQIDNO:3859); CCDS12922.1, Ins, C_7, PAATAAPALSAGNFPEAAGPGGCVPGPDAGRLDGPLLPEPGPAAGGCPQDPRLSLPPCLPAG KWLYHPALHALLHGDQRGQDRVHSCLEKE, (SEQIDNO:3861); CCDS6380.1, Del, C_7, PGMGRSPPLPPWWAAWTPTPIATAPPCACSSTGRRSYKTWPPWSASSSSSSTSPRASSPPASSS TATVSLKASSSRFSTTSCWPSVRPVSS, (SEQIDNO:3862); CCDS8931.1, Ins, C_7, PGTEDSDQVPGWSSIPVGLEVPGGPSQASAGQGRHGDREAGAGAECASGSWGWSRKHWRN HQQHCALGEQHSWELLLCPVQEPASQEDQAV, (SEQIDNO:3863); CCDS3707.1, Ins, C_7, RSQPGIHPAESCGHRLLRGDVLPAGACVRGNSRSIHRVSHSSAQCCCPCSRGTSHGLKVPLLL WCQRFGHGFLLHAGGNHYSCHNSGICVG, (SEQIDNO:3864); CCDS42103.1, Ins, C_7, PDAHQNPTRLSPGPWGTSWSSPSYGQGEAHPAAAAADRAPHVTPLPASVTDAPRLCLRAAP ARGGRGGAGCPGACAPTRADGCAHTVPAP, (SEQIDNO:3866); CCDS34562.1, Ins, C_7, PPLLLPLHCLHPHHQAPNSRSEQHERLCQLPISRQRAAALHHEAHRGRPGGGRPVLHALPGVP GRACAHPQRAAHQQAAARVRHHGPADR, (SEQIDNO:3867); CCDS32755.1, Del, C_7, RAPLRRGKRPLWESRRGWAQRRASHSTSGRPLRLPTGRGRGRRRSARRTQLPAPCRRGRHR DSLGAPCTGGRARHSCPLSDGRKMAHSHR, (SEQIDNO:3869); CCDS12235.1, Ins, C_7, APRGPPVLGGGNVVAGGLHPRRAFSSLRGPGLPGAGGPDAECDSHEPRGHANGHSHSHGAR GSGGCPGDRLQRDPRGRETGGPGELDGL, (SEQIDNO:3870); CCDS42824.1, Ins, C_7, PGPANPSAAPHTLCSDHSVPPAVRPRPGPLAGPCRAAFRAQAPRCCLCQGGLPTSGSPREARA LSRGSPGASRESPSSHGAPQARQQSQ, (SEQIDNO:3873); CCDS3883.1, Ins, C_7, RAGLLCSLEPGRGRQDVRYVHPRALPASPWRGPRGRPQRAQQAAPRRGRRGVRARSGADPQ DEGAGEPQERRRERLGVEPRRPRQGRAR, (SEQIDNO:3874); CCDS13132.1, Del, C_7, RTAAATAAAAAAAAAAARGSLEEQRAARPRTPPRAKPPSPATPRAPMDTWRPSSARSPDRKS SSPQARAATRWFTAVTCVARASVCSAC, (SEQIDNO:3875); CCDS42972.1, Del, C_7, APQAPQAHVATLGFQVPRERASGASPAHLDLRDPPASATRGARALPAPQAPQGPLHFLALTG RLSAFPALRAPLGPLGPLEPWAPPQG, (SEQIDNO:3876); CCDS34863.1, Del, C_7, GGFRQWWCTCRMSGPSCLLWRSGRPCASRKLQRQLPQPRRHKGKRSLLNRHLMPWSKQQT LLDGTQKLQRPPHSRKRTLISQRPLHPP, (SEQIDNO:3877); CCDS47839.1, Ins, C_7, QHGCTCHHLHPLQPCSAGRMEAESKRSCREHHANQGKSEGETPAPGNPWVLGSHHRAEWD PRLSWTQLPAGGIPGQEEAHLYPQERSD, (SEQIDNO:3878); CCDS55791.1, Ins, C_7, GCGGRGGGGGKQRGWGSRGSGRRLSSLLVVRLVFVFVIVLSLFRAGPAPGGPGLRRGAAGL GRHRHQPAPVPGRVWGERRGRRQRRG, (SEQIDNO:3880); CCDS3037.1, Ins, C_7, HAGGHRQRWGGTTAVLHGAPAAPGCLPRAGGAEPVADPEQPGDGEDPGGAEPHPETHPDP HGQGGQPAEAVGHHNDDVHHQGGGHSS, (SEQIDNO:3881); CCDS35368.1, Ins, C_7, PGLRRRRRGDRPVATSELRSGDPTFYPGRRHLFLTLLIVRRLVLIAGLPEDGGVSRDVLLLLRC ALLSPVWIPAAPDPPIHQRALCP, (SEQIDNO:3882); CCDS10978.1, Del, C_7, AVGRRCWPRRWPRRLRCPSWRWPAQSSWRSLEASALPVCGASLRKPEPGPPASSTSMRSTR WARSAPPPCPASPTRRRSRRSTSFW, (SEQIDNO:3884); CCDS59410.1, Del, C_7, PAQCPQPQPYPHSSTPSPCLCLCPSGASRRRGQRRGHQMRRMGRPLRPTWTASRRACARWC CERPRTPRSSGTCHGRGLTPATSRS, (SEQIDNO:3885); CCDS41858.2, Del, C_7, SRSPKSMSPPGRTQLPPSQLPQPHRHRKTCSRRATPLSSLAAAPGARAGARHPPPTRRPSQPRP RSCLGTPLAGLPACPSPCPPVR, (SEQIDNO:3887); CCDS7357.1, Del, C_7, SSPTPRTCPTTWPPSRNPSATPCRKLCHTLAALTSPTPPYNKVCTYHTPAASQGLHYITVGLLL LLLPSLPGSRHRPLPAAIHTAT, (SEQIDNO:3888); CCDS24.1, Ins, C_7, PGLSGQRLQPQRRPRPRPHTGARPQRHQCHRPREPSPLLPAAGLRAGHAAALHPATGHCPPG RPQAPRRLRILGPEVGKVKGEGC, (SEQIDNO:3889); CCDS12716.1, Ins, C_7, PGPLIAGSQNGGVSQDVEPQGAPRLGPAVPRALHSLLQGAAAPPPNTGIPLESGREGGFGGVL GPRGLLHPVPLPPNPGPAAGLI, (SEQIDNO:3890); CCDS10440.2, Del, C_7, RPPARRRRRPRRRAAPRTSTTSSGPACGRRARGPGRILRALWGAATPVARGPAEKELKVVDH RLEMCLGLPLLPLQGARAPPPGT, (SEQIDNO:3891); CCDS55757.1, Ins, C_7, SAHPWGPVQRVRAADPEYSGLERVSDIWGAHLVRDQRVRGLLLRWGAVLCSQDAAEVSVS KKVRSLQDCGAHALHRAAGEDKFPL, (SEQIDNO:3892); CCDS55640.1, Ins, C_7, SPRCHLGPGHQAGPGLHRPPSPWCHLGPGQQARLGLHRPSSPQCHLGLRLCIRLSFYSGAQRH LCQGYHNPSQQEHSILNSQPPL, (SEQIDNO:3893); CCDS41932.1, Ins, C_7, GGAASASPTQPREEAPSPPTSSPPPSSPVTVLGQGDSDLPGEIPGTTANQSPLLGMSYGAAARE DHQRPCGVVQNPNSLWLATP, (SEQIDNO:3894); CCDS55892.1, Del, C_7, GRTQLPPSQLPQPHRHRKTCSRRATPLSSLAAAPGARAGARHPPPTRRQRSLCSSQPSQPRPRS CLGTPLAGLPACPSPCPPVR, (SEQIDNO:3895); CCDS1687.1, Del, C_7, GRTSGRSLSCCPRPRCRPAVASRSTAPSPRAGSRRCCLRTSCGAARPRRTRSAWGDWVASPPT RSSSRTACGAASPPARSWSAP, (SEQIDNO:3896); CCDS9989.2, Del, C_7, HLLYHHPCQAPVSSCPHHLHHSRAWDARPHPHPCCLVWAGALLHPHLHYCPAPAAPPWREA WRRSSWPRWTMAWAQHGSPAIGG, (SEQIDNO:3897); CCDS47235.1, Del, C_7, HRSGHS SLRHPPTLHPEDWAPPPAQLRERGPVSCSVVEPPCRIIRAGTRRARRRIQRRSLGRLW SAAGSSVSGQTTFMGGRIVG, (SEQIDNO:3898); CCDS7918.1, Ins, C_7, SAHPWGPVQRVRAADPEYSGLERVSDIWGAHLVRDQRVRGLLLRWGAVLCSQDAEVSVSK KVRSLQDCGAHALHRAAGEDKFPL, (SEQIDNO:3900); CCDS56415.1, Del, C_7, SHFLRSQWKFREQKELPLSLSGRMLPQPLEQQQKRPCPEVHLLLLRGAPGMNRMELQLRQNL GQLQSPQNGSLLSSRTFRASGR, (SEQIDNO:3901); CCDS8097.1, Del, C_7, GLPLQSTPLRRLPLQLLLLPPPPASLAQRPRPQPQEPPHQPLKWKGLQLLSQWAQRRCLRMES PMQQSSAGAACRSWSLLLPT, (SEQIDNO:3902); CCDS31863.1, Ins, C_7, GRGQQRRRWEAPRGGSGHPGGRRCQRAQQQPPGRRAGRTCRPRSRGRELRGRLRHADAGG PRPAAVGGRAPSRLCLGCTAAPA, (SEQIDNO:3903); CCDS46741.1, Del, C_7, LTVLQNHPSLPRPIPAPPTLCPQTRWPHLRGHQGILAASHILETALAAIFQGGAALLVLLALPC RDPQTRQPQIQFLSPAQEL, (SEQIDNO:3904); CCDS33592.1, Ins, C_7, NPGCTLWPHQGPCQRPQTGSSSLPTQWGKSHSIPKFKIRKIPDCFSRSRTFLDRVYSPFRSDPA KIGRGTTRFYFKEILPPDD, (SEQIDNO:3905); CCDS55892.1, Ins, C_7, PGGRSSHQASSPSPTATAKPAAGERRPSAAWQQPPGQEQEPGTPRRQGGREACVLPSLRSRGP EAAWGPPLLDFRPALPRAPP, (SEQIDNO:3906); CCDS45544.1, Ins, C_7, PLPPHPLPARASQGLPFSRRWAGSRGCLPVPGGDPIPPRGPRGGSGRAAGLPPCAASVPHTPLL PQQRQPGPAGRAADLQAV, (SEQIDNO:3909); CCDS30652.1, Del, C_7, PPGPCFPPGPLPAHLTRPSPPTPSPRTHAHPHAGTPAPGAHQSLPRGTTRCPHGQPVLSHRPAA PHRSETAAAPDAAGTTGG, (SEQIDNO:3910); CCDS2446.1, Ins, C_7, QPEEPAQYGTALTHPHARDLLHSLLPAQVQEQPLPGGQGSSHHRGLWHPHLHPGDGPGGLL HHRHLHAEADSAYRALSDLSR, (SEQIDNO:3911); CCDS45960.1, Ins, C_7, SHAAAQWASPVHGAVAARPADPGPEEARHPGRLTPGRPPAWGPASQPPPRGAACRRGPAPG AAAPAREATTPAAIRAWPCWG, (SEQIDNO:3913); CCDS44299.1, Del, C_7, AMEAPRGTCSPAPRACLWASLVKGTLSPKPSRMLGSCCTATVGPWMAPPTPPSVRTSSAWT HPQPRSGWRTAVCTHWRKPC, (SEQIDNO:3915); CCDS46579.1, Del, C_7, GGGTIQPQATGLLKPKISQNMIPQMARVTGVSGLSAASPAGTATRNGPGLVATRALQQNRGP VTVQTAQELKTLLGQLPPK, (SEQIDNO:3916); CCDS8078.1, Ins, C_7, HGRRGLLHHLSPGHGPHHPPVTPQTRSPAQPQDRWGHGRPWGAVCPLRPGPQPAGGQNEPP RPASALAGEPALGARGPHVL, (SEQIDNO:3917); CCDS13819.1, Ins, C_7, PVHRGARGPGPAHGLRRLQRAVRALQPAQHRQDRRRAEPLLQQAAVRPAGRAPAHQPGQG LHQLLRHERGQCGLEQLHLRL, (SEQIDNO:3918); CCDS31269.1, Del, C_7, AESEPHRPWDASSSSTPPSKPTVMKVTSSIQSGWRRVQQGGWASACAGAQSMAWASSSAK WRKAAVQSGLACAWGTRSRR, (SEQIDNO:3919); CCDS32758.1, Ins, C_7, QRNDAGPQRGCDGQRDWGQDEDSQEQEQERTHRDRDEQIHGERHAGGEDQVRRGGRGGG SLPQPQEAGSRRAPPSCRQDF, (SEQIDNO:3923); CCDS58876.1, Del, C_7, SLMTSTFTSSHFKWILLLTILLRGARPVCHLQARGPAPALPSQFSSTPGSSSQTVARHQLFVVM MKMLHHCQPNLLIFIH, (SEQIDNO:3924); CCDS32510.1, Del, C_7, ASRRPSWPAAPPAPPGPHSAPRPPLPVAAASAPARPPPPDATRPGHRPAGDCGCRWTRFPAPV PPRPQTSQPALLRARR, (SEQIDNO:3925); CCDS12652.2, Del, C_7, CSPPSPQNRSRTNGSVTTSATEAWCRTTLPASQRSSACTRSTLMSCTEASRDPAKMRRRSWQ RRRLPSVIPTRTARWPR, (SEQIDNO:3926); CCDS42417.1, Del, C_7, GSRAWTRGAARLPSPSSPTSRWTAGCRRRTRSGAVARRAARPSRAPAAPAARGLGSACSPLP SGAAASRSPRRARRLRG, (SEQIDNO:3927); CCDS7532.1, Del, C_7, GWLRPPCPPGPCPALMPRPPPPPRLPPLPPTSIGTRVTRAWPACGSKPNSTPPSATPLCTGRPRQ PTLVRASTPWKGPY, (SEQIDNO:3928); CCDS11098.1, Del, C_7, PGVRRTSRCWQSKSWALSNGSTSGMVTDSSTGMTPRKMSLFTRQLLKETTPGSFCAALEMG RLWNLMSWKERRAQKPLM, (SEQIDNO:3929); CCDS12922.1, Ins, C_7, PRPLGPSAGLALGPALWAALRGACGPSSPGPSPTSYPSPCWDPRPHPDPEAGPRLRPLLPTQAP TAGGQPRLHRPGCRR, (SEQIDNO:3930); CCDS12013.1, Del, C_7, RAGRPSSTTASRAGRPSPRPAPCRSMSARTPARSRSAAPSAAGPSPLRATSRCTWGHTCGITPP RDAAAACLWRTPWLS, (SEQIDNO:3931); CCDS53685.1, Del, C_7, RTTMRPSLASTILQASSTMRPKASWRRTETPCMGTLSSWSTPPGTSSSSRSSRPMSPWAPRPGS ARPHLAASSSGHWSC, (SEQIDNO:3932); CCDS4404.1, Del, C_7, SPPTWRPWGSLCLCRPSTLGCPSSGSTTTWTCVGTSPPWAASPTPPLRLAQPFPRRCPCTTCPT IRCTRSCPLVCHILT, (SEQIDNO:3933); CCDS53377.1, Ins, C_7, AAGPAAAARGAPGRPDGGPRSADAPAPHRPQRGERRQALRSLQRGRAAPEGQQQHHGVCS RAHLRARGRDRGQARLQD, (SEQIDNO:3934); CCDS41580.1, Del, C_7, ALWMARRGHTRWPAAPRATPSPWSPSPRAVMGALPSTAAPRSRAGRQRSGPATWRCCGGH SPCPLVAPARTGCPRVNC, (SEQIDNO:3935); CCDS44254.1, Del, C_7, CMSSSGCALDSCFPSSSSSASTLPELTLITWDESGCRRGPLSRLRVSGWKTRAGTSAACSSWTS TSLKTILLTAPGCI, (SEQIDNO:3936); CCDS43002.1, Ins, C_7, LSCALRVLQAWLEPLAGDHHLQQVPGKIHLHHAARCHPLRIPGQWESLRVEEEHLNCSQHH RGQALDRPVRGHRDCEP, (SEQIDNO:3938); CCDS41800.1, Ins, C_7, PAPTSSSSPSGGGRRAGEHLPHLSGGDPECQDIGEVPAFILRGLHHPGSAGEKGLPHVRPLLW AAGGQPAPEWADAGL, (SEQIDNO:3939); CCDS54831.1, Ins, C_7, RGLHHQRAQLPAQHGDRRTAGERGVGAAAAPRGRRRAGSPAGTLRALCAGGSQLRLPGVR AAAVPARRCHSGPAPATR, (SEQIDNO:3940); CCDS13952.2, Del, C_7, RSPAPAPPLQPRPCPLALAALGPPKPCPDVWLAAASEPPQCHPRYPPHPLSLPGAKAGVHQPP PARPPQVQPPPAQSL, (SEQIDNO:3941); CCDS58874.1, Del, C_7, SLMTSTFTSSHFKWILLLTILLRGARPVCHLQARGPAPALPSQFSSTPGSSSQTVARHQLLMM KMLHHCQPNLLIFIH, (SEQIDNO:3942); CCDS4739.1, Del, C_7, TTPLPALLPGHPHPAALPCPACHPTLTCRRLASRAHFPRRRPLPPPRPRRRQPRLPRPLASWCP RTRGLWARCRWGAT, (SEQIDNO:3943); CCDS11563.1, Del, C_7, ARQPSRPTGRTPTTRLMAPVGSVMSCSLLTQPSPASACTMPPLLHPQWAPHSAGLSPGACRP ACTCISMTTISRATW, (SEQIDNO:3944); CCDS13949.1, Ins, C_7, PEDGISPCTLPGSTGHLPHHQERHLPAPAQPGRLRQPRGWQAQLRQQPHQLHGRPLQLRPGL WVLHHLPPAPLGKAA, (SEQIDNO:3946); CCDS2405.1, Ins, C_7, QPPGLRAGQGAGLRLLHDLRPGRGAVVRRLHRALRPGAALPPPAGRGEAAARPAARPRGLP QRKELPRASQDRERLP, (SEQIDNO:3947); CCDS45864.1, Ins, C_7, TAEWQQRQLSGHAGHGHLGGQHLRFLQWCHLGPAAGKAGLHPVPPQSPTHAQAAPATHRP RRVWQREAGRWGWHQPE, (SEQIDNO:3949); CCDS9607.1, Del, C_7, TSRCPMTLLSTLTRKILNVKRSQGSSPPTVVACPRSSSTAPPMWPPSSTVWLSRPSGSRAPAKP RSTRCCWCSLTVW, (SEQIDNO:3951); CCDS6982.1, Del, C_7, AWSALRVPREKRAQWVSVATLGPLDPPVNRGFRALLEKKGRRVTQALQASLGKMALQDYV VSLGTEGFLVQWELLD, (SEQIDNO:3952); CCDS30652.1, Del, C_7, GLRVTPSTPCGALIGQRSCVAGGKPDGDARQTPDPRAAYCPCLCPRGWWLPWPNPHHHRLL HPLPCQAQARSQSQS, (SEQIDNO:3953); CCDS12065.1, Del, C_7, HPHRSPPTSCPPLLEAFPLPRASLRATVPRHSSVLATGLHLHRQISLPLRGFLLHQPLPGQHLW LSHRLRLRLPRT, (SEQIDNO:3955); CCDS45544.1, Del, C_7, TPSPPTTSQSQPRPSLFPQMGWEPRVPSSAWRRPHSPTRAPRWVGEGCRASPVRRLRTPHTTS PSAAPAWTSWTCC, (SEQIDNO:3958); CCDS7013.1, Del, C_7, APWSCYSPASPRPSWGPSWKPSTCAPPAETSSAGPSRRSVATGTAPSAWPASSALGLRTVLPV FTRAYMKKAFLF, (SEQIDNO:3960); CCDS41858.2, Ins, C_7, PGGRSSHQASSPSPTATAKPAAGERRPSAAWQQPPGQEQEPGTPRRQGGLRSRGPEAAWGPP LLDFRPALPRAPP, (SEQIDNO:3961); CCDS11367.1, Ins, C_7, RGLRGAGARAGLRLLRHLRPGLGDALRGVHPPLRLGPALLPAPRGGEAPAHTDARARRVHG AGGDRGHPGKPAAL, (SEQIDNO:3962); CCDS7957.1, Ins, C_7, GAPGPRPARAPRGRLPGVSARGTHAAGQPRPRQQLLQPPVRGGRGRGAPSRSLHPLPRSACR RLAGAPGRGRAY, (SEQIDNO:3964); CCDS55351.1, Del, C_7, GGIVPTRVPRSHLCLSHPLPKQRDPDRQPRKKRRNLRRVNPGSFVGYLERKRQKLICQMTRT NRLFGMKRKTSG, (SEQIDNO:3965); CCDS43537.1, Ins, C_7, GTHAEHLQRHQGDSEPPGHRAGRHPQLLPRLPALHAAGHARGAQARHPPQRRLRREQEEPE PGEADADPLGGPV, (SEQIDNO:3966); CCDS14081.1, Del, C_7, RQTSFCTLTWQPPRQVCLGFSKSTAPGGTTRGRMCSRGQACWHTHTSSWRRPLGSWPSTGT HTGSWPASWGPQV, (SEQIDNO:3967); CCDS9266.1, Del, C_7, RWLPGGGSPWTSRRGPPWSSTTPCTQGVREGTASGKTRGEAMGSGQATVTSMSPGPPCRGS GASSFIRHTGNPP, (SEQIDNO:3968); CCDS59054.1, Del, C_7, SMVIRMSGCSSSSELTQTRGPRRPPGHQCYLSPHETWKVPRMQPGSRMQRLLKVLRTPQAQK NLYLHQHQQSSN, (SEQIDNO:3969); CCDS47213.1, Ins, C_7, PAAAPAAARGCPRPLRLERGHRPRPASRLLGHRALPVLSRHGPHLLRGGDRPPARPEEIQDVL ALLVPGTQAF, (SEQIDNO:3974); CCDS7922.1, Ins, C_7, PAQPAVWSLAAAPPGEESRAGAHSHCTQGDCCLLTGPRSHHRGHLPEVGQHPSGPGSAAEV QHGAACGFRPVQ, (SEQIDNO:3975); CCDS44659.1, Del, C_7, AQMPPPMPALGSSGPHPPVPPTHPNMEGLCLPRACLSGACRTLPETLRTAPRKSSLMPTKASR TVRRSSPRR, (SEQIDNO:3978); CCDS44904.1, Ins, C_7, EGRDHPVPAQAVQLSGHLCRWSGLYHSRTVCHSTARFDQAAPVGNATVSFSHDAWQHRIQC RFGCICSDYFS, (SEQIDNO:3979); CCDS45510.1, Del, C_7, HCLWLWCRPSWKGKGASAPRGPGMPNSLNQGIPGRYLTGGLWAWKAGTGAQRVCCLRCC FSPLKKVWNLQGL, (SEQIDNO:3980); CCDS9577.1, Ins, C_7, PLSPAPPAHARHRARLPRLGPKQQLQPRSLATAIWLPASRGPTYPHTALTPVTLSGHRCHAEV LRLASNRQS, (SEQIDNO:3981); CCDS941.1, Ins, C_7, PRASIRISHGSPRSYASAWYAWTSSTDAPPWIHWPSTTPTLWLPAGASPSTQTHSPATSSPSRP TSRPSPSV, (SEQIDNO:3982); CCDS45890.1, Ins, C_7, QAALGPRGGRGTGRAGPLHVGTEPGLCGPPPDPTEPPAACQAGSREQEPAEGSHPARDPDRE CRGLAVRGGH, (SEQIDNO:3983); CCDS276.1, Del, C_7, RRPLRSWTPRTRGAHHCLWPHCLPGPHLKTSLPLTCLQTPVPDPALPGQTLPPLALSPCPSPRN PQPYSQQG, (SEQIDNO:3984); CCDS34625.1, Ins, C_7, SSAGHKLVNREPGSWAACTCGVPAPPGGGWAKEGASEPSMDACLCLPAPYAPHTPQHWPLR GSGDTGRGSRL, (SEQIDNO:3985); CCDS31621.1, Ins, C_7, LPGEDPGHGLPGRLRPAHGLHPPGRQEIQVCLPQLGLAGGGQGRPSHTWPRALPPRLASQGC PVDAPDCVL, (SEQIDNO:3986); CCDS1646.1, Ins, C_7, LPRLCEVQKHQRRLPVSLRGPLRVRRRWENLRRLREAPSGDLDLHVAGCSADRRLRRSHLD GDLQVDTHWH, (SEQIDNO:3987); CCDS43446.1, Del, C_7, PGLAAPIAWATGPTCVMWPCGPSRSLASSLGSTPNCMPSPGVMTGGSAWKTTAAKVLWSIM SDSQEVTGWN, (SEQIDNO:3988); CCDS32513.1, Del, C_7, PRPSVPLTAPPRTLWHKLRPQTVPAPRTTLRPPVPPPQPKALLAASSRKPQNQNPRPKPRRPPE WRRSLSA, (SEQIDNO:3989); CCDS12328.1, Ins, C_7, PSAPRPAATPAAACQASASHPAPPFTPAPQVGPLLNRSPPRSPLPAYDTFPPDVTVPEPDPPVST PAKRPA, (SEQIDNO:3990); CCDS12136.1, Ins, C_7, TALPTASSLSTVTALPAVPSLPYGLTRTPSEPRAATPHYPPRTDGTAGAEQPHVEPERVPGAR GQDAGGRM, (SEQIDNO:3991); CCDS42402.1, Del, C_7, AATHQPWQGRCSGPLRGELMPGGSCWRLGGLCTAKWPGQYGPTWASCPLAQQCLAHTQFL RALGSPWGRD, (SEQIDNO:3992); CCDS984.1, Ins, C_7, CPAHSSPLGRSLQVLRGSREAPPVPGPARGLPGPRPEKGGRREAPVVPEASPRRALLPPGRPER RPFQLL, (SEQIDNO:3994); CCDS4267.1, Del, C_7, NTPASSYLTAASPSRPPARPRSCRTHPSTVTMTVAWRSLRRRPASHPQGLDSVPWPCLRITMS APPLMAA, (SEQIDNO:3996); CCDS55185.1, Ins, C_7, PAVRALQLRGHPGRDPALRVRASQYLRHLRPDRAHRGFGAAEPDPGARHLHRGLVCEQPGV DAEPLRTGA, (SEQIDNO:3997); CCDS7537.1, Del, C_7, PSLRSFPRDCTPSTESAAAFTLTSRTRSRLPLSSSTGWVAQTPWTMLACTGMWGALLLTSPST GTTSASA, (SEQIDNO:3998); CCDS45960.1, Del, C_7, PVASANGAAVGVAGAPCLTSIQARPLPTSPAACRLASSRATSVRQSALPRWGPEKGSWASAP GLMVTATC, (SEQIDNO:3999); CCDS2773.1, Ins, C_7, RAFRPCRGAWKAWYSRAPRQGWGCGRGRKARREGRTGRERRTWRTGQRWPSWTPWNPW APRTPWPQGVCG, (SEQIDNO:4000); CCDS14374.1, Del, C_7, APPALVAPRGLLCPALHLVKPRVLHQNLDPTSSSFSKWCRPWLEQMLHSCRIQKSDFSNNWN SSTQWGS, (SEQIDNO:4002); CCDS33428.1, Ins, C_7, HLLPSPARGDRRGQRHLHLQLLQHIGELRAKLVPHEPQQPDGQAGRLPRGPQPARPGLPLPC HTTAQRA, (SEQIDNO:4003); CCDS59192.1, Ins, C_7, PTISSSTTGARSFLSWTRTWGLPQCLWNAFLPKSAGVSVSTYHSAYAATAASSQEREKNYKN SGSKPGR, (SEQIDNO:4006); CCDS46614.1, Del, C_7, RCGRPALTPRRCLPPHPRPACLATSTRPWSSWGPASRARGETRLQNPSCWFRPLGPSRWCLPF PSAASQ, (SEQIDNO:4007); CCDS2615.1, Ins, C_7, RYLPPQLPCAEEGAEAPSDPDRRNHNGGEAGCGARLGHQRGGWLPPLLQRPWRGLLCLCGH HARHQVSV, (SEQIDNO:4008); CCDS4457.1, Del, C_7, RYMRRRPPPPASTRVTAARPSPARPTGCPCLSASSGTGGPGHPARCLPSVVSGGGSSKTSCHS AVTGGR, (SEQIDNO:4009); CCDS8260.1, Del, C_7, SQVRQKHLDGAVLSRDRQSVKIADLSLPPSPDATPSLQWTTADFTELLPVRPTSTHSVVERVQ AGLLNP, (SEQIDNO:4010); CCDS34342.1, Ins, C_7, TSPAEPVRPHFQFSAEHRPHRLSSGHPRPPPPPPDDDPTHAHPGAPLPGRHAIRRLRQPLCPSG GHQES, (SEQIDNO:4011); CCDS12196.1, Ins, C_7, RLPLLAGHGDLQRCRRMPVQPVCEWRLSEPGRLLHLQMWPWQPAGPLWYLLSRQHQGHLL AEDPGEPL, (SEQIDNO:4015); CCDS47428.1, Del, C_7, AQRTQRLQHLWTGGTPARWKSCGTSWQPISVAPGERTDSSVTGQAQQWPLRARRARRAPA CLRRRLS, (SEQIDNO:4016); CCDS53997.1, Del, C_7, ASPLKRDPQQGQFIPSQPPTLPWTLRKQPQGPSCSRGSQRAVRGTSPSQGILGSTGCSLGSRLH SWC, (SEQIDNO:4017); CCDS13243.1, Del, C_7, LRGHAQMCRSHGTKGLTCGGMSSCKRQGMSVSPSGVMPRTHSSSCTPVAPQANPRVWFTQL GATCSM, (SEQIDNO:4018); CCDS31473.1, Ins, C_7, PASRCHPCGPAVHGPAADVGSPVQALAWPHEPGLVPDRRRSSAVPAFRRGLTSACCPAGRGL PCGVP, (SEQIDNO:4019); CCDS2754.1, Ins, C_7, PTHPALPRSRSRPSASPLRGSAFPQPWAPSASPSPTGIWSCPFYQTHGPPGSPSYHSRALVCWP VHP, (SEQIDNO:4020); CCDS44581.1, Del, C_7, QRCHRHRAPWLIRTLPMSPAQAVPPRTPRNAQPQSCPPQRPPRPPCPPLPCSRGPSTQPPDGPR SRL, (SEQIDNO:4021); CCDS13495.1, Del, C_7, SVRSTRKVSTMTSCSMAPSTPSSSSGAKAWRRSPEAPSPWSCSNIMKPTPGPTPPAASTTSSSG SCG, (SEQIDNO:4022); CCDS42217.1, Ins, C_7, TPAGSRCQVGAGFACSPGVQPGGSKPQGEAREALSYDPVPGAAAGGGVGGRHPPALHLTLEI PCGHG, (SEQIDNO:4023); CCDS9577.1, Del, C_7, VVSCSPCSRSAPRSPPSARPKAAASAPKPGYSNMATCLPGTYVPTHSAHPSHSQRPSLPCRSFT ACK, (SEQIDNO:4024); CCDS9629.1, Del, C_7, WARGDWGKNWTQRMPRHAAVWPWESPLPMALHLSVFPDLHPLGLACIRHRRDQPPHLAPG RASPPGR, (SEQIDNO:4025); CCDS11409.1, Ins, C_7, QPQPPPAPPAAVAATGGAGRGEGAGPRQPGRGPPGRCGHREVPHPQGLSPGSADGGFCALPL QRHF, (SEQIDNO:4029); CCDS13819.1, Del, C_7, STSRCTWSRTSSWPSAAPASRARSAACTASARSAARRTAPTASCCAACWPSACASARTGSTS TITT, (SEQIDNO:4032); CCDS12584.1, Del, C_7, TRLTFRERTSTSPATRPLTHPRSILGVSMGYRSNTHKFSLSPKSRQIITGPMPVLSLTWLLAAIIP , (SEQIDNO:4033); CCDS6882.1, Del, C_7, TTAASGPASSACPTTTTSPRGRTNVSPTSRMSTVARATTTASSPRKGSSHRGPSCMASPSPTPP GP, (SEQIDNO:4034); CCDS41266.1, Ins, C_7, TVALLQAGPVAAVLFNFPPAQNLAARPPGEQQLNGNDVEGVSSALRFGKRVALGPGRRSQH RPARR, (SEQIDNO:4035); CCDS46224.1, Ins, C_7, ASSTECWQRSPDPHAAPTRCQDAQRNGSLEPAEQACPAWDLTDQAPTSAPTAAQPPPAEEAC AGG, (SEQIDNO:4037); CCDS45660.1, Ins, C_7, HAVCGRCGVPAGVPDPGGRGRRGQRGPALLQRGAGAHVRAAAHGDRGPESGGAQRAPEG GRRPQA, (SEQIDNO:4038); CCDS9256.1, Ins, C_7, HRLRPKRRAAQPGPRKAAQGPRRGGTSDPPRHPAAGPARGEDPGGRPGERRQGGEEALRDG QNCV, (SEQIDNO:4039); CCDS56148.1, Del, C_7, LLRILRLRRHLPPAAGLPVTGSPRSFTDTITYRDSRFLRPSKRPSWYNILTVNRPVVILARTQTT, (SEQIDNO:4040); CCDS6879.1, Del, C_7, LTCGVLGASWQRCGPAAPSCRATRSSTNSPSSVSSAAPSPLRCGQTWTTMSCTKSWSWSRAR SGR, (SEQIDNO:4041); CCDS9835.1, Ins, C_7, PAVGLVCWERLVRGGSGTLPGSVGPQLLPGAGEAGGRRVRVPQRGGAHVQPILCLPAALHR LLRR, (SEQIDNO:4042); CCDS41411.1, Del, C_7, RGRRAAPRHLLGKSPPSRNSGPEAGPQPRQSRLETPQLAGLWSRPGPHPRLSLPEGRRTDLDC SP, (SEQIDNO:4045); CCDS9999.1, Ins, C_7, RLEGQHLRRRQEQQLPAQPLCEWWHLRGQRGLLLLHLPGRLGGSYLHSQYQRLQPSALLQW WHLC, (SEQIDNO:4047); CCDS42560.1, Del, C_7, SAAAPPLRATLRFYRSRGFRNPQSPWQGQGAWGGAAAATRITGAPGHPRPSPCPPASCASPR PAR, (SEQIDNO:4048); CCDS55640.1, Ins, C_7, SPWCHLGPGQQARLGLHRPSSPQCHLGLRLCIRLSFYSGAQRHLCQGYHNPSQQEHSILNSQP PL, (SEQIDNO:4049); CCDS41247.1, Del, C_7, TRPRTPERSASGVRWRPCGTWAWPSSPVPSPRSSPQCPSSSASSPHLPSSARLWHSTRACPSST R, (SEQIDNO:4050); CCDS32782.1, Del, C_7, ALLAHQERWCPWPCRMSQPQHRRTMAARLTGRTSQTNATIFQLRKKFLRMQSFSVKTSLHIL FS, (SEQIDNO:4052); CCDS3352.1, Del, C_7, CQLLGALTTSTGTKWMTQTSSRSEVTPSLVAVRPSPQKALRPGWASQRLNSCMLGLPRRSQV PV, (SEQIDNO:4053); CCDS46076.1, Del, C_7, EASSCMAHLAVGRPCWQRRWHITQQLHSSGSWARSLYRSIWVRAPAWSGMCSAWPRRMH LPSSS, (SEQIDNO:4055); CCDS12022.1, Ins, C_7, PDPGRARRQPLPGQWRGPGAAAAAGPGPGRRPPAAAQLPAGRPPSQPGAAARRRGGARARG HAL, (SEQIDNO:4057); CCDS47048.1, Ins, C_7, RARPGRRRRFVVVAVLHARHIPGPLEDSGAAAARVPRRLLVVFVRRCRQVWPRRGGGRRHR RHG, (SEQIDNO:4058); CCDS1053.1, Del, C_7, TLWTLCSSTPTCLRTCHLMPGPDSSGSRSLWPPQRPSNSRKFGWQMRASTPAPCLTHCTFWT RL, (SEQIDNO:4060); CCDS55444.1, Del, C_7, ALLHPREPWTCLIPLLAWKKTLESWMEPLSCWGWGGCSIKPPLPRRWTTVLREPWHGMQEI RP, (SEQIDNO:4061); CCDS6714.1, Del, C_7, EKACGHPPTDRAETLLKFLLKGILALAIGPAGALAGPVLTTLGTQRPLPWAAPCPATASPSPL, (SEQIDNO:4062); CCDS2800.1, Del, C_7, LHMQPSLPWQGLKDLGSLVSLCCTGVSPALPQTCHCKRRSSGRPAVVASRSGTPCHACGMP VS, (SEQIDNO:4064); CCDS12022.1, Del, C_7, RPRARPQAASPGSMARAWRSGCSGTWPWTAASSGCSAPCGPTPVTAWRCRQAAWRSTGPR SRS, (SEQIDNO:4065); CCDS44903.1, Ins, C_7, EGRDHPVPAQAVQLSGHLCRWSGLYHSRTVCHSTARFDQAAPVGNATVSFSHDAWQHRIQ WH, (SEQIDNO:4066); CCDS10283.1, Del, C_7, FALRPSTVPFLTRAVPMKPSGRCLLSSPRMTSSRCWTHCLSTRACSPTSLTSSIYKKAPSPR, (SEQIDNO:4067); CCDS6916.1, Del, C_7, PVGPAKLGCSMTTRQPTAVSWPCWLMSSSLSTACLAWTLTGSLAREATRRARSLSPTWNCS A, (SEQIDNO:4068); CCDS47428.1, Ins, C_7, QPREHSVFSTCGLEGPQPDGKAAERAGSLSLWLQERGPIPQSQARPNSGPSEQGGQEGPPPA, (SEQIDNO:4069); CCDS30880.1, Del, C_7, RPTPPPSSTLPPGKAASSISMTTCLATLQVPQLQVGLAEAAGTDRPAPWCTGGTATPSRRSP, (SEQIDNO:4070); CCDS54978.1, Del, C_7, APAAWETHQNEVPSAPLCQSRSCCVPCASAPHTLPPAFCWLLTLGAAQLNVVAPGGLPQVW, (SEQIDNO:4071); CCDS3883.1, Del, C_7, CWSPLQPRARQRQTRCQVRPPPGPPCLPLARPPRPAPARPAGGPPARTPRGPSEKRSRSPR, (SEQIDNO:4072); CCDS58443.1, Del, C_7, KARCPRVGCLHSQLPHPHPTPTSDTPKVSSLARRLDFQEEPMTGFFNLIPPSSSPSTPRGT, (SEQIDNO:4074); CCDS30625.1, Ins, C_7, PGGDTSPGVHPGFPGPDSDLHLRGHWRPHPHHQLEAQLGPHPLSSQGDSDQRGWPWHTDHP , (SEQIDNO:4075); CCDS6575.1, Ins, C_7, PSPGPGLWGIHGAASGCSRAGCFRARAAALEACDSAGEGWGRRSKDQDCPHSLLRPPHFSS, (SEQIDNO:4076); CCDS14033.1, Del, C_7, RGAALAISMGVRVMWASFKHSTLALAVCHIISRITLGLSLQGVLSTNSRLPASSSSSKSSS, (SEQIDNO:4077); CCDS6357.1, Ins, C_7, RLALQPAAPACRSGGRRRLRERAGAQPHRRLPAAAPSRARACVPGAVHPHWTRAALQGVSH , (SEQIDNO:4078); CCDS9121.1, Del, C_7, SPAVSGDSQTPFCLPLRMKLAAWSTWRIRRGRLCWRKLLHLQRCTGRPDSPSKVPDSVRRM, (SEQIDNO:4079); CCDS13464.1, Del, C_7, AVTRASAPRATAAAGAWRGRRPPTARSSATTRGPPSSTSRAVGRPPCWRGPGSTTHTSRP, (SEQIDNO:4081); CCDS5715.1, Del, C_7, GCFWPCLWNSLLRFCPASCSGCYSWTIPPTGSPFSCTTTRSSMNPTSLTPGRSSRTTSQL, (SEQIDNO:4082); CCDS47339.1, Ins, C_7, PETDGRPPAPTSRPESLATAPTPDQPRRTQQKQKPQNGKAPCSFNRQKHETSPRSFISSV, (SEQIDNO:4083); CCDS44755.1, Ins, C_7, PRPPGAFRVLEEGRCKTQGRGRKDHDPWREADDVTYTQERCRHVCVRSLQHGGRTGECGS, (SEQIDNO:4085); CCDS54719.1, Ins, C_7, RGPSDLQREGMHVPEVFPARIGGQQRAPVPGPPKLWGLRHRNQEWPGPGSLGQALALGLF, (SEQIDNO:4086); CCDS3355.1, Ins, C_7, RRGGGEEVPGAAGAGRAEALLPWLPPAMDRHQDRGTHALAHPQRPQPDPPGAQAVSPDLR, (SEQIDNO:4087); CCDS1862.1, Del, C_7, SLLCNPPLLPPSPRSSPSHASSAARRSNFRATWWCTGAATRARSPTSATCATTRAPRPAS, (SEQIDNO:4088); CCDS13442.1, Ins, C_7, SVLAVSERGGRNDPAGCPADHPRADGRSHSAPDSHPHGLRWPPGRLWQSNFHYQFGYYLH, (SEQIDNO:4090); CCDS6940.1, Ins, C_7, GVREAHGAPCDILCNHHLSSTTSSHQHFLNCRFWQSASHQCRILWGHQFWWDISKCWQD, (SEQIDNO:4091); CCDS5700.1, Ins, C_7, HEERSQLSADGPGRREQRPRPSSRELCGSPPAQPGPDPHQPKQEEVPLSTCAGCCVPGL, (SEQIDNO:4092); CCDS5280.1, Ins, C_7, PAALDPRELAVLGLAGALPFLPVPGQHDQERVFPDAGQLHPRLLCGLRGSSMDDWCDGN, (SEQIDNO:4093); CCDS7916.1, Ins, C_7, PERPRPHHRSRHLHRQPLPPQHSHPDGTSGWSPCCPAWGSGPLASRPNPLPGRCATRGH, (SEQIDNO:4094); CCDS12326.1, Ins, C_7, RSPRPGASALSSRGLPPWPQSGTVGVQEEQEAPREGGAGAAGAPGAGQEADAGLPGPPG, (SEQIDNO:4095); CCDS10206.1, Del, C_7, APQLPMNWSPLCTPIPRTGPRHPQTWRTGLKCTALWVAVFLKAGVTPKERSPGLNQAG, (SEQIDNO:4097); CCDS32853.1, Ins, C_7, CCWPIPHDSGLQPSACGPEQPHPAAPSTSARLSFSRGSTSSVCESSCVALESASRTCQ, (SEQIDNO:4098); CCDS35317.1, Del, C_7, CGPLTPFPVPRPSSPPPKKTCSKATFGTSLSPFATPTRSGTCCWAWTAPTAWTVAWTP, (SEQIDNO:4099); CCDS58453.1, Ins, C_7, PGPPGAELPGGALPISPAHLSPPGGYPPGGQLLGVGLERTPGPSPCGCCCLHLDTTNL, (SEQIDNO:4101); CCDS13060.1, Ins, C_7, RQAAAAPQGPCCGHFPAIRGWLQHLWGLFPTHEGHQSPQLAACGD SQPFAGRGGLVPL, (SEQIDNO:4103); CCDS12481.1, Del, C_7, ADFTEPGQAISPHPTLELSPATSNPHPLGPQIWPPGLPPSHMLPSPHLATRVSRLTC, (SEQIDNO:4104); CCDS8992.2, Ins, C_7, QGLTHLEEILLPRRKPHAALQHGRAVHHQGDQPDKHFTEPDPTVQCENFPHQNATGT, (SEQIDNO:4105); CCDS10440.2, Ins, C_7, RAPRRAAGAVPAAGQPPGPAPLPPVQRAAAEQEAQEGSSERCGERQRRWLGAPRKRS, (SEQIDNO:4106); CCDS8078.1, Del, C_7, RTTRTFTPPFPWSRTAPPSCHPANPLPGPTSGQMGPRAPLGCCLPPPPRPPTCRRAK, (SEQIDNO:4107); CCDS276.1, Del, C_7, PERKTATVRPKQRTRTMRLGPTQPRPALLKLGVPSMETHHLQPPPHSAAHGPPLAL, (SEQIDN0:4110); CCDS58150.1, Ins, C_7, PRSCRRRPSCHRCTAWCCQSGTCSGRSRPSWPSCPIPRRDPRPGHRSASPGPKPSL, (SEQIDN0:4111); CCDS8930.1, Del, C_7, QGEESLRGLGLGGWQQVGLGVVQTDWSQRWYAWWWKVWRGSSGASQGGMLGRSHPC, (SEQIDNO:4112); CCDS9274.1, Del, C_7, RAPAPGCSQRAPLALPALLPATWCLGPAARPQPLSSLLQDTAAALPTPLLRTWRGL, (SEQIDNO:4113); CCDS46529.1, Del, C_7, RGCFATSLRPSPTCFLTDPRCWYFPPPPCIAPPTPTSPRLPLWRSRSFPCWKTCLT, (SEQIDNO:4114); CCDS41281.1, Del, C_7, RPWRFTGWSTGRRRSRRRSGSTACAWAPLCGGGPSAAAKKSMSSAPRTTPWQQPWL, (SEQIDNO:4115); CCDS35172.1, Del, C_7, WASMASSRSPPTPQETWLPSPSTSAPSAGTAPQASTMECTAARGARASSSGRCART, (SEQIDNO:4116); CCDS47213.1, Del, C_7, CRRPRRRPGLPAAATPRAGPPAASGIAATRTPSATCTVAPWTAPPTRWRPATGPA, (SEQIDNO:4118); CCDS34579.1, Ins, C_7, EPCGEGPPPARRRECRGVHRGGRRVLRQEGDSQGLQNHDCAGQGHLQERALRSPG, (SEQIDNO:4119); CCDS30833.1, Del, C_7, FWGLLLLHLSTSSLHHFLPRHLDGPLLQNLPFRVLGSLQTIKHPSPWPPQPCWEM, (SEQIDNO:4120); CCDS45921.1, Del, C_7, GQRPSPRPPLAPACVPRAGQRWPRDPWPRRPYARGLLSHRQQAADPSGGNQPNLN, (SEQIDNO:4121); CCDS10055.2, Ins, C_7, HAGGHQLRFCTEGFLGALEPAGGGAPGGGLGLCPVEAGAGADRPSPPRPAQRRTG, (SEQIDNO:4122); CCDS1912.1, Ins, C_7, HQRAPFPVAADEVSHWGRHDKKGPWRCLQPAVRLLCHREGRAGHEGSSWGGGAHL, (SEQIDNO:4123); CCDS3863.1, Ins, C_7, PLRSLHLPRLGQRAGLGHRHILHGHGAHLCGLQVLQPAWVLSRETGLRHCTREGP, (SEQIDN0:4125); CCDS6412.1, Del, C_7, PWEVAPRCGRPKLNGATRSGCACRVRSHRHPSVPCPLPSSGPWRPRSVRCGGQPG, (SEQIDNO:4126); CCDS53984.1, Ins, C_7, QSLPSLLSRHQDAEWQHSPNGRLCPALPAGHRLQHGVGPGGCIPAPRPVAGAGEE, (SEQIDNO:4127); CCDS8162.1, Ins, C_7, RAAAGGGLHVTGAQRGAVRAGPAAGDQGPAGPAARFRAGTPAPATGPPRRGPSHL, (SEQIDNO:4128); CCDS7916.1, Del, C_7, RAAPPPPQIEAPPPTALAAAAQPPRWHLRVVPLLPRLGVGATRIETESTTRPMCD, (SEQIDNO:4129); CCDS2283.1, Del, C_7, TCRRPALPSLYTPTPTSRLGCPVRLTVPWTAPMSSTLSLRRTPTAQRWSPSLKAL, (SEQIDNO:4130); CCDS34961.1, Del, C_7, TPMDSHRSSQDKTLTHPSMETTRRRVPHPTMTTRTSLPPTGMTRASDRPSSARCS, (SEQIDN0:4131); CCDS9592.1, Del, C_7, ELRALGLVREPPAAKRRRRSRDWSRVTRGTAPLRTRSWKLSKLESGRWRKKLRS, (SEQIDNO:4133); CCDS32928.1, Del, C_7, KSTTPRARQPWWPGIPCRATTRCGVLATRATWQPQALRGQGPMGTGRCSWSPVS, (SEQIDNO:4134); CCDS14306.1, Ins, C_7, NLTCAGNYTPDISQYTDWESQDLGARQRISGGLRISGPRRGEPTRRGSWRSGHG, (SEQIDNO:4135); CCDS12394.1, Ins, C_7, PVSIKSERLSPAPGGPGDFPKTFPYPLLLARSLAEPLRPGPALRRLPLADGWPR, (SEQIDN0:4139); CCDS7845.1, Del, C_7, SCTWCGRWRRQPSRRGPTVPPSSSTAVQGLGGPAASLPPASAASSCGRRVWWTS, (SEQIDNO:4141); CCDS32760.1, Del, C_7, TAASIAPLTRPSPRNSARPAAVATSLQSRGCREPRWGSPGASSHPATPATSWRT, (SEQIDNO:4142); CCDS5928.1, Del, C_7, TQGRGSARLPSRARPGLHWHHRHTMLPPKPRRWRRPWDPRKPACWRSWSSRTKQ, (SEQIDNO:4144); CCDS2529.1, Del, C_7, WETLSTWRPSERWSCRSPASLPRMWCWCHQASMPWRATPPLLGATTSPPDASGT, (SEQIDNO:4145); CCDS54507.1, Del, C_7, ARPLPPPQILERKRKGRPLGLLVAPRPAPRSWPRKLRRPGLGVLGTQAKELWH, (SEQIDNO:4146); CCDS11120.1, Del, C_7, ILGRGACESGPCCCWGFWGWCLGSAWSLSTGTRRIRGSRQRVVMCCTLRSGTG, (SEQIDNO:4147); CCDS32423.1, Del, C_7, KARCPRVGCLHSQLPHPHPTPTSDTPKVSSLARRLDFQEEPMTGFLPFHPPGN, (SEQIDNO:4148); CCDS4716.1, Ins, C_7, PHSSTCENRAPEASKGGRAQDSRGPGPGTEPALCGSGPSSPQQAPPAVHSGPC, (SEQIDNO:4149); CCDS835.1, Ins, C_7, PSAPGGPGVESSPKCARLHGTAVGGCAVPVRWGASPRGLQPEWALLGRLPLAF, (SEQIDNO:4150); CCDS46024.1, Del, C_7, SASSLSASLRPPGAPATFLRPSPIPCSSPGPWQSLCGLGPPCAGCPWPTAGPG, (SEQIDNO:4151); CCDS59451.1, Ins, C_7, HLSGATSHSSSTTACGRQALPVCGSGEGLQPCSQRDHRSEHSCAGSAWASMF, (SEQIDNO:4155); CCDS34406.1, Del, C_7, IAPLVKTEPQRPVKEEEPKIPGDLDQEPSLLYADLDHLALSRPRRLSTADPA, (SEQIDNO:4156); CCDS4805.1, Del, C_7, LSYSFFTNVVPARWTCCVPSCSSLSSQTSVSCGSAPGCWPFHLISASAMLLC, (SEQIDNO:4157); CCDS33068.1, Ins, C_7, PLPLPPPYHWPGVRELPPVPSRLALAACHAPAPPPLPTLLLQPARPTLPVQL, (SEQIDNO:4158); CCDS56095.1, Ins, C_7, PQFHPLCHSPTPGHHQQHKPQPSRQPLGYRLVRPEPQHGPWPLVEPCPLPAL, (SEQIDNO:4159); CCDS47954.1, Ins, C_7, PSPHTVGRHQGLARCHWTEPSFSADVSSGNTAGPGSWEFCSPWLPHQRSPCP, (SEQIDNO:4160); CCDS58032.1, Ins, C_7, PSRPLPPDPVSKRLQSQGPAKPPPPRKPLPADPQGRCPSGDLPGPGAGIPPL, (SEQIDNO:4161); CCDS8986.2, Del, C_7, FHHIATDVGPFVRIGFLKIKGKIKGKSLRKPNWKTQHKLKRALMFLIVKKL, (SEQIDNO:4163); CCDS46055.1, Del, C_7, NPQRWRSHLSCDLPLPPPHLFPRSQHQSPLHHVPQLLHLPQFHSLRRDGPS, (SEQIDNO:4165); CCDS45010.1, Ins, C_7, PRRRPRAPHRASPAPPPPRVQLAGGGGHRRLRHRQLQHPGGPGEGYGPEA T, (SEQIDNO:4166); CCDS32744.1, Ins, C_7, TLCQLLPRDPTLSLPCLPRWGANPSEGVWPSPSPRAPARQAHRLQGEALCL, (SEQIDNO:4168); CCDS56415.1, Del, C_7, VLGGLGPLFMTGMPTAMSWLEPSIFLVTALLWMFTSTWNRPRFRVSPGYGW, (SEQIDNO:4169); CCDS11367.1, Del, C_7, WAARSWCESRAAAVAPLAPWAWGCPAGCTPPVAARACAATRPEGWRSPCTH, (SEQIDNO:4170); CCDS3883.1, Del, C_7, AAPCRRGAPSGAPSPPPPPADPAPSPSRGTATPSSGRHPATRPLARILTA, (SEQIDNO:4171); CCDS41800.1, Del, C_7, CPHLFLLAFGRRQKSRRAPAPSVWGRSRMPRHWRSAGIHSARAASPGLCR, (SEQIDNO:4172); CCDS27.1, Del, C_7, CRSSRTWASPCSLSAPAEATSWSCQPLPQPLPRSTCTLWTWMTCTSLSKS, (SEQIDNO:4173); CCDS59221.1, Del, C_7, DPPAPRVPGAGAWSPTAPSRGARAPPSPTSSWWPWRTSSGPRATCQCASA, (SEQIDNO:4174); CCDS3444.1, Del, C_7, KHCCKRITLRSSCSLGRDLYLWNPRILKGSGGYTDVIMMDATKCTLKAPT, (SEQIDNO:4175); CCDS3565.1, Del, C_7, KTRRKVKILQINPKERKREAKMEKIEETERRKIHVMQNFKISAFTENANI, (SEQIDNO:4176); CCDS11834.1, Del, C_7, LHRRAAHSRVPSPRSWGPRLGPAGRIGGNFRGPHPRAHRVQLLGADSWKQ, (SEQIDNO:4177); CCDS1085.1, Del, C_7, PGRCRLTLCPRDSSLRGQPSPHPQGSHCLPTPRAGAHRVTCPAQGLESRP, (SEQIDNO:4178); CCDS46446.1, Ins, C_7, PHICISRCCLPVHGYSQQRPVHCHQRREWLWEDRKRPPDCSAFDFLGKGQ, (SEQIDNO:4179); CCDS12642.2, Ins, C_7, PRPGPAGGALLPRSLTAFVPHSGHGLPVSSGEPAYTPIPHDVPHGTPPFC, (SEQIDNO:4180); CCDS55403.1, Del, C_7, SNRIRLHRLILPMKIPRQLPQMSKVKVMPHHSLKMRNLHFHILTWPSWMT, (SEQIDNO:4181); CCDS7353.2, Ins, C_7, SQAEQLQDCGRCQQEDPGATRCLHQKVPAGAWGALVWWEPAWGVCGRGGG, (SEQIDNO:4182); CCDS3483.1, Del, C_7, ARTGTHTPRPPRARARRAAAGSSAAGAAFPPPPPARRARPRCPSRAAGG, (SEQIDNO:4183); CCDS46133.1, Ins, C_7, GGRGLRGLPPRPRVGRPAAPQQPAVPGAPRGQVPRLPPAPPGPPAPHRE, (SEQIDNO:4184); CCDS8132.1, Del, C_7, GKTSTPLTSISPRWPSLLTCSWLGWHWAFRKGSPRRCWACVQAQRWCGW, (SEQIDNO:4186); CCDS10641.1, Del, C_7, KARCPRVGCLHSQLPHPHPTPTSDTPKVSSLARRLDFQEEPMTGFVSSH, (SEQIDNO:4188); CCDS31340.1, Ins, C_7, PGPLRARGHPLLLGLYAGAGGNPAGLLHRRGHTPGEEVHTVCGGQLEQV, (SEQIDNO:4190); CCDS7725.1, Ins, C_7, PVLHSRLRHGLLQPVGPLHPLQTAGHRLLDPPQVQQRQVQHLREEWYLV, (SEQIDNO:4191); CCDS9989.2, Ins, C_7, RGGRHGGGHRGPGGPWLGLSMGPQPSAGEPTHTAHEEAELAEAAIQRGT, (SEQIDNO:4192); CCDS30673.1, Del, C_7, RKSPLPLKQRAEDPPHQPWAHGMPDLLEGAASHLQQQCQPPTALPPSKR, (SEQIDNO:4193); CCDS10746.1, Ins, C_7, RLSFPRSGTQSSSGPPPHPPAPGQTGSVGPGHVLLRVLSPAAPGSTGQP, (SEQIDNO:4194); CCDS55757.1, Del, C_7, RPPLGPRAASQSGRSGVQWTGASQRHMGSTSGSRPTCPGTSATLGSSTV, (SEQIDNO:4195); CCDS45890.1, Del, C_7, SCPRAPRRARDRPRWAPPRRNRTWPLRPPARPHGAPGCLPSWQPRARAC, (SEQIDNO:4196); CCDS9327.1, Del, C_7, SPTTPRWRTFTTTSRLSSRSPTRPPGPSRREPSRASWRSPTASSCLSHG, (SEQIDNO:4197); CCDS12326.1, Del, C_7, VPTAWGLCSVLQGPSSLASKRHSRGPRRAGGPPGRRGWGRRGPGGGARS, (SEQIDNO:4198); CCDS7529.1, Ins, C_7, KKDACPSHSRGGLPMECQAPSGHHHQTCLVLGPSCPSAPMHSCLPGAA, (SEQIDNO:4199); CCDS12172.1, Del, C_7, LAPRQTAAATAHHRTPRRALKSLPQKRARTPPFTRSLMRISRRNPHPP, (SEQIDNO:4200); CCDS59482.1, Del, C_7, LGAGEAASLTPWCPFTLKSPAMSSPEHTWSFPTATRTGLCPTTVTTFT, (SEQIDNO:4201); CCDS34863.1, Ins, C_7, PAASDSGGVPAGCLDHHAYFGGVGGPVPAESCRGSSPNPGGTRGNGAY, (SEQIDNO:4203); CCDS54954.1, Del, C_7, PPASLCRLAACCVTGNAKAGKKALLKMDWCCRVRSCPLTSCQSSSRIS, (SEQIDNO:4204); CCDS58757.1, Ins, C_7, PVQFSRKGLRVPEQPAVPRSPGTPCGNRQREGRGEGLPPRGRPGHLSR, (SEQIDNO:4205); CCDS3883.1, Ins, C_7, QQPPAEGGLLLELHPRLPRQPTRLLHLPGGQRLPPAADTPPRGPWQGY, (SEQIDNO:4207); CCDS58553.1, Ins, C_7, QTNRHQVPCPWCPWTVAPTALSPGRATEFPEILWHLPAPPWPWVPRGT, (SEQIDNO:4208); CCDS12136.1, Del, C_7, RPPHSQQPFHSHCPSRSPQPSLRPHPHPLRAQGCNPSLSTTHRWYSWG, (SEQIDNO:4209); CCDS13465.1, Ins, C_7, SPTDHPGLHRLEICSARPEQVPRARRCDQKLQRCFCTRAVLAQAGRAL, (SEQIDNO:4210); CCDS53377 .1, Del, C_7, SRPRRRRPRRPRPPRRRPPQRRRPSPPPPPKGRATPSSALSTKRPSCA, (SEQIDNO:4211); CCDS1911.1, Ins, C_7, TAAPSASCLLFLGPAPGSACRLRTGFLGLRAIQLARTESGQRQQLQES, (SEQIDNO:4212); CCDS45010.1, Del, C_7, EAPPPRPAPRVPRAAAAASPARRRRRSSTASPSPASAPWRPWRRIWP, (SEQIDNO:4213); CCDS31328.1, Del, C_7, GMPYHQSAPHILTGATSQHQPWPSTSRSCMTSQPEMPTSYRCSRMRS, (SEQIDNO:4214); CCDS46133.1, Del, C_7, RKRPAGTPPTPTSRPASRPPAACGPWCPQRPGPPAPSCPTGTSCAAS, (SEQIDNO:4220); CCDS11693.2, Del, C_7, RPKEVAETLSFRIPGTQSLQAVTVVGAAAAAAAASIARTGPAGRKAA, (SEQIDNO:4221); CCDS45999.1, Del, C_7, RPPRIALSSPTPAAPRPIQLRLPGNPTTPQWTSPQPAHPPSTTPSYK, (SEQIDNO:4222); CCDS35440.1, Ins, C_7, APVPQPEQQRHRQRHLPDHACHQVSYLFSVFLQSREPAPQRGDVPL, (SEQIDNO:4224); CCDS31863.1, Del, C_7, EGAAATPLGSPPRGLWAPRRPPLPTSSTTTSRAARRPHLPSPVPGA, (SEQIDNO:4225); CCDS4512.1, Del, C_7, IISQFTLALPMWLYQESLPTLFCMTHGLLICSSGPRTLSVLMSISG, (SEQIDNO:4227); CCDS 12328.1, Del, C_7, IRPQASSHPRRSLPSLSKSSSTTIHPGTTSRTPTQPVTSAKPPPRL, (SEQIDNO:4228); CCDS45176.2, Ins, C_7, PHLQPPASTGPGRYPGRTHGLPLPGHPPDLEGDGDGPGGPGGPTPL, (SEQIDNO:4229); CCDS12575.1, Ins, C_7, PSASARQPPSSHPLSHPRGMHQQPGPLILDPLASCGDAGGSAPGPP, (SEQIDNO:4230); CCDS30649.1, Ins, C_7, QRWGPSAVPGLQLRGHRPDGRRRQASCPAGTPALGGTATPWEEPGF, (SEQIDNO:4232); CCDS58576.1, Del, C_7, RAKVLGPRPALVSASGGGLRPATPRIPWPLAPLTAWLPRPPQRKMC, (SEQIDNO:4233); CCDS34004.1, Ins, C_7, RGGGGRRRRWRQQQSSLGLVSSWDGASLRPAPEEEAAAAAAPQGQA, (SEQIDNO:4234); CCDS42929.1, Ins, C 7, RKCPSHSNITRKHINILVHTFQGSLEWNSPGVQSHLLGQPHGRRAG, (SEQIDNO:4235); CCDS8023.2, Ins, C_7, RTATHVHPFLGEPRHPDGDRGGAQVIPWTPWPEQWTPSPSGGQRRA, (SEQIDNO:4236); CCDS14053.1, Ins, C_7, RVQRGALHRWGSEQQLALCRLPLHHHGVALPWDSGHLPPEHLPQPA, (SEQIDNO:4237); CCDS46332.1, Del, C_7, SPSRSSITASLAAGLWWASVPSAPWRASCVTPTRRRVHPHRVAQTM, (SEQIDNO:4238); CCDS45225.1, Del, C_7, TMVQGPAGHPLAMAQGPAGHPPTMVQGPAGLPLAMAQVTHPLVHIT, (SEQIDNO:4239); CCDS12922.1, Del, C_7, TPAGPLSRTGTGPGAMGCLTWCVWTFVAWPQPHQLPQPLLGPQAPS, (SEQIDNO:4240); CCDS8859 .1, Ins, C_7, EGRDHPVPAQAVQLSGHLCRWSGQVQHRQRQCELSPHHPVHVPQP, (SEQIDNO:4242); CCDS8213.1, Del, C_7, GDCPRTMAGPSASLHPASGRASRKTWQRRLRACRAGGPAGWARQA, (SEQIDNO:4243); CCDS 12328.1, Ins, C_7, PATTPTRSSSPARTEPPTHHRGHPTACEDKEGSEEESRHHHPHHH, (SEQIDNO:4245); CCDS45010.1, Ins, C_7, PGDGGRAPHAPRAAAEPDYSAGGPRAGRGARLLPVPKSPGGGGAV, (SEQIDNO:4246); CCDS55589.1, Ins, C_7, PPGRAAHSGPGGAPLPTTPRETETEQGWLGPWRHPEYPVLPDGAG, (SEQIDNO:4247); CCDS46055.1, Ins, C_7, QTPKGGGLTCPATSHYHLPTCSPGASTSPLSTTCPNSSIYPSSTP, (SEQIDNO:4248); CCDS42523.1, Ins, C_7, RARPGPRARLPSGIPARLPRALQEAFGESEHPPAAALHGGPSAVL, (SEQIDNO:4249); CCDS12160.1, Del, C_7, RSHPRPTARCQAGGAPSPAASLRRSSRRAPTTRPTRMSWWSYTGG, (SEQIDNO:4250); CCDS46835.1, Ins, C_7, SPPCPDPAQVSSPPYPSLPHKPPVRSGRQQLYAVRQCQQRRVLLE, (SEQIDNO:4251); CCDS5565.1, Del, C_7, WAAASEMALPSVDPTWISENPQKRKEHRDHDSSLNLEQSRRPSIK, (SEQIDNO:4252); CCDS5336.2, Del, C_7, GAAAASCQSPPSPRETTPSSDATRRTRPIAAQSKKTMTAAGLTP, (SEQIDNO:4253); CCDS12716.1, Del, C_7, GPSHRWQPKWGSIARCGTPGSPATGPSSTASASFPSPRSSCSAS, (SEQIDNO:4254); CCDS3253.1, Ins, C_7, HADDAPAARGHGAPRSPSGPRPGLSAPGTDRQQTVLPHGHGKPQ, (SEQIDNO:4255); CCDS8860.1, Del, C_7, HQDGVAVARPVTARPAPRGAVGTCLGFVQLCHPMNLEDQEAQGA, (SEQIDNO:4256); CCDS42665.1, Del, C_7, KRVALLTMWTCMDMLPKGAASCTETALVMRAPRWKTCYIQSSSP, (SEQIDNO:4257); CCDS46186.1, Ins, C_7, PDHRRGEEPRALGASPKPECPGPHASSDTQWLCPGRACIPRLPI, (SEQIDNO:4258); CCDS7626.1, Ins, C_7, PGGELGSRPGAHGTHPGPRSAGNPILPRGRLDKRSCIRVAPTIF, (SEQIDNO:4259); CCDS10639.1, Ins, C_7, PRRGAPEWGACTLSFHTLTLPLHRTPPSSISSLPAAPLPPPGEL,(SEQIDNO:4260); CCDS45451.1, Ins, C_7, PRRGAPEWGACTLSFHTLTLPLHRTPPSSPSTPRGTEDCPGRDL, (SEQIDNO:4261); CCDS42824.1, Ins, C_7, QESGHGHRPGLPDVHSAAPGAGLGPVDGTGHRPAGARVGSHRAA, (SEQIDNO:4262); CCDS47339.1, Del, C_7, RDRWPPSRPHQPAGITRHCPHPRPTTKNPAEAETTKRQSSLLLQ, (SEQIDNO:4263); CCDS4774.1, Ins, C_7, RGSRTEQLPKPGPRRGWGPRWPGRGRGAGGRGGGRRRGPCVRLG, (SEQIDNO:4264); CCDS7477.1, Ins, C_7, RVPSPGGSLLGSVAPSLPFLPGWRQETLACRQSCRFEGKDPDST, (SEQIDNO:4265); CCDS8944.1, Ins, C_7, YPHSFKFCAAGCHPGSPLGDTPPALCPECPPTWTTYLQCHHGNY, (SEQIDNO:4266); CCDS32391.1, Del, C_7, AMRRPSSRHAAHRPLCQRFPTLTVSRWMTSLISSLRVERSPSP, (SEQIDNO:4267); CCDS45960.1, Ins, C_7, AQWLRRTEQRWEWRGPPVSLLFRLAHFLLHQRPAGWPQAEPPQ, (SEQIDNO:4269); CCDS 12601.1, Del, C_7, AWSTLWPPAQPHLQPPFCPRARQPLPLPPQPRLALSPAPQQVP, (SEQIDNO:4270); CCDS45660.1, Del, C_7, CCLRTMRSTRGSTGPWGAGAAGAAGAGASPTWGWCTRPSGGTR, (SEQIDNO:4271); CCDS44482.1, Del, C_7, HLSQVKGPVNWKDAQVPRLRFSLPVALDAGKNTLTVKNKPNQL, (SEQIDNO:4272); CCDS10639.1, Del, C_7, KARCPRVGCLHSQLPHPHPTPTSDTPKFNLIPPSSSPSTPRGT, (SEQIDNO:4273); CCDS 10457.1, Ins, C_7, KGTQVPGAAVTDALQPNQRPGDGGRHAATAGAGPDGGQHPADR, (SEQIDNO:4274); CCDS45937.1, Del, C_7, NLRLHPHPGTLAVHTCMGTRLATCMTTTLNISRHSRYWTQESS, (SEQIDNO:4275); CCDS5715.1, Ins, C_7, PGVSGRVCGTAYSVSAPLPAAAATPGLSPRQGHPFPAQQRGLP, (SEQIDNO:4277); CCDS42522.1, Ins, C_7, PISSHLHQRPDAGLGHVPVLLEVPEPSSSDTPRLCGPGHPSRL, (SEQIDNO:4278); CCDS4856.1, Ins, C_7, PLPPYPAQRTAYCAHISERQLFPRGDPRLPPPVRTRRVQVARL, (SEQIDNO:4279); CCDS8384.1, Del, C_7, PPAPPLLSHPPPPALPLRPPAPSLHEPGAHTPKWGAPGTRFSR, (SEQIDNO:4280); CCDS941.1, Ins, C_7, PWIHWPSTTPTLWLPAGASPSTQTHSPATSSPSRPTSRPSPSV, (SEQIDNO:4282); CCDS42153.1, Ins, C_7, QASGGQHSPWRVALAGQCEEARSPHLQRLPGGRHLGPHCCPLL, (SEQIDNO:4283); CCDS42516.1, Del, C_7, RHHLSSSWATSTPSNAGSVRWNSRAPSPSRKSGCGTYSGTSWR, (SEQIDNO:4284); CCDS 10978.1, Ins, C_7, RLWEDAAGQGGGHGGSGALPGDGRPRVRGGHWRPRRCPCAEPL, (SEQIDNO:4285); CCDS10055.2, Del, C_7, RRWPSAQILHGRVLGSPGAGRWGSPRRRAGTMPSGSRSGSRST, (SEQIDNO:4287); CCDS54035.1, Del, C_7, SSSSSSSNHRCSSLPRRQQPSRHPHHSSHCNSSSNARTKTVRK, (SEQIDNO:4288); CCDS47263.2, Del, C_7, TVSPNSLSTNISTAHIPPLHLRTSARSKFTGLCRPLTSLASTP, (SEQIDNO:4289); CCDS5091.2, Del, C 7, VGKKLWQLCWIMTCLRLFTRNHFAVILLMYQKSPGRLVDGSSW, (SEQIDNO:4290); CCDS8946.1, Del, C_7, AKVKAVMVASLSPTIGLWSSKKYPVRTLLTCIATSPTITSTL, (SEQIDNO:4292); CCDS48066.1, Del, C_7, ALRHHWPASSSCRMQTGAACWHTSTASTTRAGSEKQPRWARR, (SEQIDNO:4293); CCDS31272.1, Del, C_7, AQVLSPQSRPPHLHLGWAQGRALGWLWGEQPSTPRQTWTRCP, (SEQIDNO:4294); CCDS44512.1, Ins, C_7, CPRGPPTLLRRQRAARGRVLRLGLCGRASRSWLRRQLCQVCL, (SEQIDNO:4295); CCDS45590.1, Del, C_7, CVTCTPCEPSSSFLGTLRPGSSPRSGLRSSLTSLTHVSSRLT, (SEQIDNO:4296); CCDS 10679.1, Del, C_7, DQWCPDGSHLGQPRQLPRDRRPRTSHISALIAARGLCSAAAS, (SEQIDNO:4297); CCDS53661.1, Del, C_7, GCPRSPGPKEASSRALRLHPGLGRGCWLPGRRRPRPWARWGP, (SEQIDNO:4298); CCDS31173.1, Del, C_7, GRRCWTMHPACRWSWTMHPCRRRKPPNPRPSAGTGTAVTPTA, (SEQIDNO:4299); CCDS8083.1, Del, C_7, GSLLGLSLAQPEALKVAARLRCQHPQHHHRRRVQCSLSRVRR, (SEQIDNO:4300); CCDS27.1, Ins, C_7, HAGAQGPGRHRVHCQHRPRQLPGAVSRCLSPCREAPALCGRG, (SEQIDNO:4301); CCDS594.1, Del, C_7, LSTVTTHWSSGWTLCWALPKRASNWTSRTSRQWAPPWTSCGS, (SEQIDNO:4302); CCDS941.1, Del, C_7, MDTLALHDPHPMATSGGLSLHPDPLPGHQFPLEAHFEALSLS, (SEQIDNO:4303); CCDS59192.1, Del, C_7, NNIQFNHRGQVLFILDQDLGTSPMLMERLFTQVSRCISQHLS, (SEQIDNO:4304); CCDS41281.1, Ins, C_7, PGHGGSQAGAPAGGGAEGGAAAQPAHGLLCAEADLPQQRRRA, (SEQIDNO:4305); CCDS59002.1, Ins, C_7, PLQQPGKLTRTRSPPPLCARAGAAAFLAPLPPTPYRPPSVGC, (SEQIDNO:4306); CCDS14023.1, Del, C_7, PLTQGPRLASLPTPLTLSQEALYWMMQRSKMSQTLLLWRWAW, (SEQIDNO:4307); CCDS7725.1, Del, C_7, SASQSSSTRAPPTCGSPPSTANCWTSLAGSTTSTTATSPAPT, (SEQIDN0:4310); CCDS34063.1, Del, C_7, SSSAPARPYSPSECWTPMTMCPLSTNPSTLCPYQRTLPQALS, (SEQIDN0:4311); CCDS42268.1, Ins, C_7, SSSPRRLGFPPVSTPKSQVTGQEVHGALRDARPAEFQERLAD, (SEQIDNO:4312); CCDS6067.1, Del, C_7, TPRPAPSIWGMLLVLPKLIMALRPILTLSCWTNQLHEKERTL, (SEQIDNO:4313); CCDS45171.1, Del, C_7, WPALMACRRPGAVVESARQPWSPPPRARWGAPQAPQLQPPSS, (SEQIDNO:4314); CCDS30833.1, Ins, C_7, CFGVCCCFTCPPQVSITSCPVTWMDLFSKTSPSESWDPSKP, (SEQIDNO:4315); CCDS54035.1, Ins, C_7, PAAAAAAATTGAAASPAASSPAATHTTAPTATAAATQGQRQ, (SEQIDNO:4316); CCDS2.2, Ins, C_7, PGAPGLRTPHPVPGLCPASPPEGGSRPCLSAAQRVQGDDGG, (SEQIDNO:4318); CCDS5404.1, Ins, C_7, PGYLRAAPRLLPCVPAQLRTPATPTEALHGGRGGRRGHPRL, (SEQIDNO:4319); CCDS32552.1, Ins, C_7, PPCPTTPPPSRGLLGASGLPLFCGHSGFSHPSHSPNPNHQQ, (SEQIDNO:4320); CCDS59406.1, Ins, C_7, PSPLTPAPTYPIAPSLAGSGPGHRPVAAGRVLSLRGTARCS, (SEQIDNO:4322); CCDS54626.1, Del, C_7, QAHPQVPALAPQAWWPARRGPLTSAPSAQQSLTKSSSSTTT, (SEQIDNO:4323); CCDS5812.1, Del, C_7, RAVETLRLQLPPRSGPRSPARARGPPPLSAPPPGRCLRRRP, (SEQIDNO:4325); CCDS55757.1, Del, C_7, RSLMRWRKTPAPPAGRPVCTKQRPWASAPSATTSWRPGPRS, (SEQIDNO:4326); CCDS6981.1, Ins, C_7, RVFCEVLPERQIHPGRHAGQHSEALGLQQGEVPEDVHWPQE, (SEQIDNO:4327); CCDS12190.1, Ins, C_7, SHREGRAQRTEIALASSWDLSPEPVPGAPGASGSGSHPCQV, (SEQIDNO:4328); CCDS11765.1, Del, C 7, ALGGACTGTWGPRGEGPPSSPGSLWPESWGTRRKSPGAPP, (SEQIDNO:4329); CCDS9239.1, Del, C_7, AWASPLSKDSLRSSCPHIRHLSLRDPRPGSLHTSRVSSSS, (SEQIDNO:4330); CCDS11136.1, Del, C_7, HPCQPLWTWSSNGCWARCLGRSSSCRGSPPCCSLWPCPQG, (SEQIDNO:4331); CCDS46143.1, Ins, C_7, LPPWPPRPTTTSSPPPNSVQPAGFLFRCRRRCRAVLPTAL, (SEQIDNO:4332); CCDS46134.1, Ins, C_7, NSAWHLCHPKPARRSPACQQPGPYCPRPAAAASPPPVPAA, (SEQIDNO:4333); CCDS 13305.1, Ins, C_7, PCPPETENHRHCQQVPEQAAGARVLYFTFPGGWTQELQFL, (SEQIDNO:4334); CCDS 13254.1, Ins, C_7, PGPERLQARGSQLWWGGHQCQCQGKGRHRADRRPDTAQEG, (SEQIDNO:4335); CCDS41977.1, Del, C_7, PLPTLTCPTMPGRTPTANPPTPSAASYLWPSRTLQPSACQ, (SEQIDNO:4336); CCDS56094.1, Ins, C_7, PQFHPLCHSPTPGHHQQHKPQPSRQPLGYRLVRPEPQHGV, (SEQIDNO:4337); CCDS 12597.1, Del, C_7, PSGSGGVGAGPVVVPDGGGEGPQPHPSSSQSPPDLPLKAK, (SEQIDNO:4338); CCDS32409.1, Ins, C_7, PTSWCPLSWGQQPHQHSTNSFQDSNRLQFQGFRTASQHLS, (SEQIDNO:4339); CCDS54003.1, Ins, C_7, PVCVPRVQGDLLHNDAAASARALTPGAPALPLHPVRQELL, (SEQIDNO:4340); CCDS5873.2, Ins, C_7, QAALQRGGQVDGSCRGLPLPAWIPTSTRRQGLPSLPTGAL, (SEQIDNO:4341); CCDS4269.1, Del, C_7, SQVPAWSRSTSWTPMTIALRLLRVHWHWKSKKMLHLVRFS, (SEQIDNO:4343); CCDS33602.1, Del, C_7, SRPRSPASPAHSPTPTSALALPHLPVASCPAPHRSPPRAQ, (SEQIDNO:4344); CCDS8932.1, Del, C_7, TSSPVPVAAASPSPGRVIWMTTVGTALMSLLRVPIPPASP, (SEQIDNO:4345); CCDS8992.2, Del, C 7, GPHPPGRNPASQKEAPRCTSAWESCPPSGRPARQALYRA, (SEQIDNO:4346); CCDS59410.1, Ins, C_7, HLLSAPSLSPTHTAVRQVPACVCARLGLQGEEDRGAVIR, (SEQIDNO:4347); CCDS299.1, Ins, C_7, LPRAREPTGCHVSGGYVQGPSANAQLSVGRGPSLSPPNF, (SEQIDNO:4348); CCDS54212.1, Ins, C_7, LSPSCDFTSHLWPGDNHRYLGYKHRTWNQFIFKFEHHLP, (SEQIDNO:4349); CCDS13305.1, Del, C_7, MSAGNRKSPTLSTSTGTSSWSQSFILHFSRRLDSRTAVL, (SEQIDNO:4350); CCDS13789.1, Ins, C_7, PQPAAVHGLCHPSSVSGARGWAQGGPRPPGAPPSQRLRR, (SEQIDNO:4351); CCDS 12603 .1, Ins, C_7, PRPGQVRAGQPQFQGGLGVPDKILFPLGLRPTGLPFPLP, (SEQIDNO:4352); CCDS33116.3, Ins, C 7, PVPGARQGLRVRRLPAAAPPAPQPARASRPAAPAPARGA, (SEQIDNO:4353); CCDS30977.1, Ins, C_7, QKAQDFLRISVGSSSRCHAYHPGDCSHHLPGPVLLSPCW, (SEQIDNO:4354); CCDS6954.1, Ins, C_7, RDAPVRGGEPHLRVDAEHAGATPDPPARRSHPLHDPALA, (SEQIDNO:4355); CCDS33927.1, Ins, C_7, RPPERGTEHLPAPPASRRGEKGASTSPTSACLLHSETNL, (SEQIDNO:4356); CCDS8844.1, Ins, C_7, RQPRWVSPHSCAFPDALACGQPRAASELTYTCFPPGCLL, (SEQIDNO:4357); CCDS31651.1, Del, C 7, RTGTVKVGLQGDKGLFRNLALERLLKQLVCPEIGNPFLS, (SEQIDNO:4358); CCDS2434.1, Ins, C_7, RWSEMLPWRTSGDTEPRDSEWWPHCSARHPVTSTLPCWK, (SEQIDNO:4359); CCDS9256.1, Del, C_7, SPPTEAASSTARPAKSRTRPTSGWHIGSTTPPRRRPSPR, (SEQIDNO:4360); CCDS1684.1, Del, C_7, VAPPARRAPAATTTTCCTSWSSTRLSFPPFGARNASFPK, (SEQIDNO:4361); CCDS54132.1, Del, C_7, CSWTPPQQRTPVQLSAVAALSPGCPSRWSAYWPQSWCC, (SEQIDNO:4362); CCDS10857.1, Del, C_7, EPSPRWAPPEPTPCRAPTLPRPHSVVPAPRACKSRHGP, (SEQIDNO:4363); CCDS41693.1, Ins, C_7, HGGLSGQGGLELGDQLPGAAGGAGPLHGVPVPGLPGSV, (SEQIDNO:4364); CCDS 1151.1, Ins, C_7, HTSGTLSCCRCRDQSPGCSSLSAPDTGWEETGSRAPAG, (SEQIDNO:4365); CCDS12328.1, Del, C_7, KCCWRMKSHLPHPSPPCRCSCTCSSCRRCSPLRRYSLP, (SEQIDNO:4366); CCDS 1742.1, Del, C_7, KISLPKPWSGSKPLCMLWARSYRSQDPSICSSLPRMTS, (SEQIDNO:4367); CCDS32166.1, Ins, C_7, LHPETEPAGAPPPRGQAPEDLEARVVPRTPSGDRPLHC, (SEQIDNO:4368); CCDS 11637.1, Del, C_7, PATTSTGGIFEPSIRGSVLLLPETKPTLMLELSFMFQM, (SEQIDNO:4369); CCDS45967.1, Ins, C_7, PGYECPDAVEPAEARAAVQAGVPDWARPCPHLYHVCGG, (SEQIDNO:4371); CCDS3279.1, Del, C 7, QTPPPKLGQEDLSNIFLTWMIKIPRKRALRRPFLCIWT, (SEQIDNO:4372); CCDS323.1, Ins, C_7, SRCHPQKQEGEGLTQVPQHRTRLGADTRPGRRGPAGAG, (SEQIDNO:4373); CCDS32553.2, Del, C_7, AATSSRRRRRRRHRCPRRRRPRRQIRMTFGCCRQHWV, (SEQIDNO:4374); CCDS8662.1, Del, C_7, ALALLSSSWALPTRWPSRMPTRKMISTISPWYPGWNW, (SEQIDNO:4375); CCDS2407.1, Ins, C_7, GSGQDTPVCSGPETSQPSGHPVAPHRSYQKTDPARGR, (SEQIDNO:4376); CCDS34407.1, Del, C_7, IAPLVKTEPQRPVKEEEPKIPGDLDQEPVRAWGWEGD, (SEQIDNO:4377); CCDS31272.1, Ins, C_7, ISTWGGLKAGLWGGCGASSQVLRDRPGHGAPEVLPRD, (SEQIDNO:4378); CCDS42518.1, Del, C_7, MASTGSPHTCGDRAHPTSTTMTPAWSPMCPTSISLLG, (SEQIDNO:4380); CCDS 12597.1, Ins, C_7, NQAAAAESGPAQWWCQTAAERGPSRTPAAVRARQTFL, (SEQIDNO:4381); CCDS4778.1, Ins, C_7, PAEPLWCLLTLGPSFSCSHLWPQASALGGSRCAGQMD, (SEQIDNO:4382); CCDS5336.2, Ins, C_7, PEPQQHHVNHRRAPGKRLHRQTLQGGRAPSQHSRRRQ, (SEQIDNO:4384); CCDS33592.1, Del, C_7, PGMYPLATPGTLPEAADWQQQPPHTVGEEPLHPQIQD, (SEQIDNO:4385); CCDS56182.1, Del, C_7, PGPRHPLLIVSTQALGWPGLKPRRACQKQSTITNMGT, (SEQIDNO:4386); CCDS1446.1, Ins, C_7, PHLCLPLWQPKECDAWQEPPDFCSPGGLPAPHCCQWP, (SEQIDNO:4387); CCDS2670.2, Ins, C_7, PLPRHLPGPAGDLPGESWAPWKGAVSIHHKAFPSARH, (SEQIDNO:4388); CCDS2283.1, Ins, C_7, PPADGQRFLPCTPLLLPVAWAAQSALRYHGQLPCLPR, (SEQIDNO:4389); CCDS45805.1, Del, C_7, PSTPLRHRTASLPSTPRPHRTPRRTTPARPRSPQSMA, (SEQIDNO:4391); CCDS9819.1, Ins, C_7, QPRYEPRTAQGPSAGLSPAASATSGPDPLPPPLPPPL, (SEQIDNO:4392); CCDS7532.1, Ins, C 7, RAGSGRRVLRGRVLPLCLGRRRRRGCRLFPLRLSGPV, (SEQIDNO:4393); CCDS2.2, Del, C_7, RGPRAPDPPPRPGTLPGEPPGRGVPALPQRGPASPRR, (SEQIDNO:4394); CCDS8423.1, Ins, C_7, RRPLSRPRQGAAAALPSLPVPAALHLRGAPAQQSQEF, (SEQIDNO:4396); CCDS58538.1, Del, C_7, SIPTPPGFHLQWWSQIPAASVHSPTCLQVNSVLYKPG, (SEQIDNO:4397); CCDS6020.1, Ins, C_7, TIPRGVGGQPVAWNHLSGLGPQNHWNPPDQPAPFPPP, (SEQIDNO:4398); CCDS42179.1, Del, C_7, WWAAMASACSLRTASSIPRRWTTSGTQHPQPVPSPKK, (SEQIDNO:4400); CCDS3444.1, Ins, C_7, ASIVARESPFGHRAAWEETFTCGIPGYSKEAEDTQM, (SEQIDNO:4401); CCDS45001.1, Ins, C_7, HPPGAGPGPGDPGAPARRGAPGVHRVQLRRGPHIDI, (SEQIDNO:4402); CCDS31272.1, Del, C_7, HPVLLPHPGHPLHPLGSPHPGPRALWSSSALWRRPV, (SEQIDNO:4403); CCDS46741.1, Ins, C_7, LSRFFRIIPLFHVLFQPRPPCVHRLVGPIYGDTRVF, (SEQIDNO:4404); CCDS41425.1, Del, C_7, PASVVTPAPESLTESCRWLAGRAVSTLSDAMEASTH, (SEQIDNO:4405); CCDS44115.1, Ins, C_7, PGTEADAGRTLVPTHPNNAFKSGWEDHGNAAGDRQL, (SEQIDNO:4406); CCDS7094.1, Ins, C_7, PPGPPKPGHVYPGGAAAADERAPDREPPAERSHEAK, (SEQIDNO:4407); CCDS47263.2, Ins, C_7, PRCPPTLSLRTFQQPTSHPCTCGHQPEASSQASADP, (SEQIDNO:4408); CCDS33768.1, Ins, C_7, PRGCARWAGVAAAAGLGACLQPLPGIAAAPWVGAPD, (SEQIDNO:4409); CCDS 10985.1, Ins, C_7, PRLPCCLQGPTGLIPGLAGRRGVGGRQPVWGAVTRP, (SEQIDNO:4410); CCDS12035.1, Ins, C_7, PTAAAAAAAARAPPGPRARAPPAPAPGRGVAPGGGG, (SEQIDN0:4411); CCDS10526.1, Del, C 7, QTLRPWLWLTAWAASTGACGRRQLGANARCSSGMRC, (SEQIDNO:4412); CCDS44659.1, Ins, C_7, RPRCLPRCQLWEAVVLILPFLLLIPTWRGCVSPELV, (SEQIDNO:4413); CCDS7918.1, Del, C_7, RSLMRWRKTGRPVCTKQRPWASAPSATTSWRPGPRS, (SEQIDNO:4414); CCDS55351.1, Ins, C_7, RVGSCRLGSHAATSVSLTRSRNKETRTGSQERNEGT, (SEQIDNO:4415); CCDS56414.1, Del, C_7, SHFLRSQWKFREQKELPLSLRNGSLLSSRTFRASGR, (SEQIDNO:4416); CCDS33022.2, Del, C_7, SPSHHLPQPGGSAMPRRLTSTWAGVGGCGQPRPRSG, (SEQIDNO:4417); CCDS41295.1, Ins, C_7, SRCHPQKQEGEGHEGRCLPPRSWRPLLPRCLRAEPS, (SEQIDNO:4418); CCDS47149.1, Del, C_7, SRSSSLIQSFLQKTHGTCSRRGCTRCTGATIPCTWK, (SEQIDNO:4419); CCDS1742.1, Ins, C_7, TRYHCQNPGADQSHCACCGPGATGHRTLQSAAHCQG, (SEQIDNO:4421); CCDS33546.1, Del, C_7, WLTGLPLLYNLCLHLLILQPHCQRVLPLVDLVQGHN, (SEQIDNO:4422); CCDS54467.1, Del, C_7, APTQQLLPPTSTTRTCGRPSTSRSSCHNGTCATFW, (SEQIDNO:4423); CCDS6357.1, Del, C_7, ARPAARSPRLPLRGPAEAPGARRGPAASPTTCCCP, (SEQIDNO:4424); CCDS516.1, Del, C_7, CLVPTSIQPLMSPLGPLLPLALATSVPGDQVQPLG, (SEQIDNO:4425); CCDS45451.1, Del, C_7, KARCPRVGCLHSQLPHPHPTPTSDTPKLPFHPPGN, (SEQIDNO:4427); CCDS32561.1, Del, C_7, LPRLGRVVPLHPLQTSPVTGDCSRPRPRWMRWWTS, (SEQIDNO:4428); CCDS54686.1, Del, C_7, MPALQPPSVSHWQRATTVTGPWRSCCTPVSPISHT, (SEQIDNO:4429); CCDS 11120.1, Ins, C_7, PFWAGGRASRGPAAAGGFGAGVWAQPGACLLELGE, (SEQIDNO:443 1); CCDS46736.1, Ins, C_7, PSGLPSISRHPVLPRPGPLPEPDLLPAGHSLRRWL, (SEQIDNO:4432); CCDS34342.1, Del, C_7, QPSRTSTSTFPVLRRTPAAPPLLRPSPSTSTPTRR, (SEQIDNO:4434); CCDS42271.1, Ins, C_7, RGALFLPGGVGFGLCVPLGTTAHPVAPQPVCPRHG, (SEQIDNO:4435); CCDS3037.1, Del, C_7, RRRTPTAMGRNHSCTSRSPSSSWMSSESWRSRTCR, (SEQIDNO:4437); CCDS8548.1, Del, C_7, SGCLRNTGCLSDCGRQSQKALSTQTHSGSHSKRWQ, (SEQIDNO:4438); CCDS9586.1, Del, C_7, SSHRAYTSSPWWRQLDLAHWWAGSGPRTQTWGTTP, (SEQIDNO:4439); CCDS31452.1, Ins, C_7, TYADTHEQSANGHLGHHFNRTHFPWCVSSSSSSRK, (SEQIDNO:4440); CCDS8861.1, Del, C_7, WNCSPLSPLSSLSATPLVLCRSWWCRKAGGCRSTQ, (SEQIDNO:4441); CCDS44612.1, Del, C_7, AFRDRNTRILGRVSAPEASHDTVTFRTARKGEKF, (SEQIDNO:4442); CCDS46711.1, Del, C_7, AGHRCPWAWAPWRPRRNRSPESWSRETPPARGRD, (SEQIDNO:4443); CCDS42714.1, Del, C_7, ARMSHRRSWKAPGCRTSSQGGSQEALGKARCLTL, (SEQIDNO:4444); CCDS6811.1, Del, C_7, GHQSPVTCWSMNQWMAQSRKSLWVQIPPPTAWQT, (SEQIDNO:4446); CCDS3080.1, Del, C_7, GWMPRCTWAMSMSLPSGIYGKAHAQKPQQMNASL, (SEQIDNO:4447); CCDS2430.2, Ins, C_7, HDRVLLRRHQRHNHLPPAHQCRACRGRLRCLPRA, (SEQIDNO:4448); CCDS33033.1, Ins, C_7, LSRSPWRAQALAGAPDHSLTFNLLAPAHHCPVHY, (SEQIDNO:4450); CCDS771.1, Ins, C_7, LSSIQGCFLWKLHLQSHHSRLFAGNQKGITTWIF, (SEQIDNO:4451); CCDS8860.1, Ins, C_7, LTRTESPWQDPSPQGQRQGELWGPAWASYSCATP, (SEQIDNO:4452); CCDS9636.1, Ins, C_7, PDAGRPGRRRRQGPPAAAAPAAATATTGRRPGRG, (SEQIDNO:4453); CCDS9274.1, Ins, C_7, PGPPHQDVLSGPHWLCQLFCPPPGAWALQRGPSP, (SEQIDNO:4454); CCDS8521.2, Del, C_7, PSPPSPSSLLSPCNASIMCPLNPAAQDGARCPSC, (SEQIDNO:4455); CCDS4266.1, Ins, C_7, PTTPTPPHGHAGAAWTQGQGVCCLEPWRAGTVQE, (SEQIDNO:4456); CCDS14680.1, Ins, C_7, PVSNLSAFSQWKPLDKTVSKRICQLLQRGDSSPR, (SEQIDNO:4457); CCDS58497.1, Ins, C_7, QHRRPALGPRAQQAAPKQAGPTNRGDQELAHGRD, (SEQIDNO:4458); CCDS43375.1, Ins, C_7, QIPQQAGRPALSAQHTPAPTPPLPRSHLDRGVGV, (SEQIDNO:4459); CCDS31340.1, Del, C_7, RAPQGPRSPSTSGSLRWCWRKSGRASSQTRTHTW, (SEQIDNO:4460); CCDS11177.1, Del, C_7, TSRGSSLQASSWKMAALFLTTTSRKSRPCTWSCA, (SEQIDNO:4462); CCDS7700.1, Ins, C_7, AAEPGLACGEGGHQEGQRPSPAGLSPWSPHPET, (SEQIDNO:4463); CCDS6541.1, Del, C_7, ASSPSLLVGANRSSAMGRTGGGCQWWHHFWVPI, (SEQIDNO:4464); CCDS45135.1, Del, C_7, EIRQLSSKSLWRVGPKTRVLNSSKPPLSQIPLR, (SEQIDNO:4465); CCDS3760.1, Ins, C_7, GEKVEALCMEEEMVRVTQWPFNWRSRCFGILQK, (SEQIDNO:4467); CCDS11558.1, Del, C_7, GPAKLMRPSFASWGTPSNGTRGWSCSSPLRSSG, (SEQIDNO:4468); CCDS 11098.1, Ins, C_7, GPESGGQAGAGNPSPGHCQMVQRPEWLRIHQQE, (SEQIDNO:4469); CCDS2791.1, Ins, C_7, HAHLPGLWHSGTPRWAPAGVGGLWPGWTAPGHC, (SEQIDNO:4470); CCDS8343.1, Del, C_7, HLHHHQSFLQQKTNMMNTMRIMLKLVLNYQMKG, (SEQIDNO:4471); CCDS3370.2, Del, C_7, HRAAPATVRPGTQAARRPPGVPLAGWARDGGAW, (SEQIDNO:4472); CCDS13418.1, Del, C_7, KASLGAPVRSSARGQDRRKTTLKAPLYPPPLSS, (SEQIDNO:4473); CCDS 12601.1, Ins, C_7, KPGLHCGHQHNPTCSHHSAQGPASPCHCHPSPD, (SEQIDNO:4474); CCDS45376.1, Ins, C_7, LHLGGTPCGGRGLCAALRGRGRQHHTEAQDPVL, (SEQIDNO:4475); CCDS7357.1, Ins, C_7, PALPPPGHAQQHGRPRETPQPPHAGNYATRWQL, (SEQIDNO:4476); CCDS44236.1, Del, C_7, PGRCRLTLCPRDSRLSWLTDPIPLPALCPLTRW, (SEQIDNO:4477); CCDS1155.1, Ins, C_7, PLPLWDEPPRLCCRQEEEPPMDQAAPSGCGTHL, (SEQIDNO:4478); CCDS32861.1, Ins, C_7, QAQRLHHGQVAPGLRGLHRRRLQTADRGKALRH, (SEQIDNO:4480); CCDS8421.1, Ins, C_7, QHPLRVAYPGGHRPCNTAQDRSPQHRECGLLPF, (SEQIDNO:4481); CCDS43002.1, Del, C_7, QLRTTCSPSMAGASGRRSPSPASSGENTSPSCR, (SEQIDNO:4482); CCDS58576.1, Ins, C_7, REPRSSDPAQPWYLPPEEGSVQRLQGSLGLWPP, (SEQIDNO:4483); CCDS54954.1, Ins, C_7, RLQPACADLLPAVSPGTQRLGRRPFSKWTGVAG, (SEQIDNO:4484); CCDS34137.1, Ins, C_7, RNGDLYCVPHQGAGGHRDCGPEFWEHPCFRGLW, (SEQIDNO:4485); CCDS45930.1, Ins, C_7, RPLPDVPAGPSLAQELQGENDHEDISSAQLGVP, (SEQIDNO:4486); CCDS44233.1, Del, C_7, RSCLPHQLHPWGPSCLLCLGTIVPLPCAPSSPG, (SEQIDNO:4487); CCDS858.1, Ins, C_7, RWRLGLGSGNWSFHFHRLLLCISQINYCLLQRD, (SEQIDNO:4488); CCDS56095.1, Del, C_7, SIPSPLSLPHPRPPPTAQTPALKAATRLSACQA, (SEQIDNO:4489); CCDS 13254.1, Del, C_7, WPGTSSGQGVTAMVGGPPMPMPGQREAPGRQEA, (SEQIDNO:4490); CCDS54626.1, Ins, C_7, CRHTPRCPRWPPRRGGLHGGDHLRLLRLPSKV, (SEQIDNO:4491); CCDS2062.1, Del, C_7, HPTAEKRAPRRQAYYSSPFSLKTSLAQRGKTV, (SEQIDNO:4493); CCDS45128.1, Ins, C_7, HQPTRSLPAVGTGWTTVLFFCFLCYQGANVAV, (SEQIDNO:4494); CCDS13197.1, Ins, C_7, LDSRCKAGGGSGLETSSQASGPGGGESSEVPM, (SEQIDNO:4495); CCDS42714.1, Ins, C_7, LPGCPTEDHGKHRGAEPPLREGPRRHWGRPGA, (SEQIDNO:4496); CCDS58503.1, Ins, C_7, PATPTTLRCHTTPYSPHWATNSLPSCASEGGA, (SEQIDNO:4497); CCDS9819.1, Ins, C_7, PAVHQSEGGAVPRARGSRRGSLHLSACGTAPS, (SEQIDNO:4498); CCDS42587.1, Ins, C_7, PETLGHRWPAAGPQPAPRVPPPLRGPRVASAA, (SEQIDNO:4499); CCDS47428.1, Ins, C_7, PPSPSTPTSPSCTSFALCSEGSPSTCWVYKNP, (SEQIDNO:4500); CCDS55907.1, Ins, C_7, PTPRSALAGVWGEKPPGGSHLTNARSTSQTEA, (SEQIDNO:4501); CCDS361.1, Ins, C_7, QGDPQEEEEILFQEAFQIERPVLQEKSEGGWG, (SEQIDNO:4502); CCDS32883.1, Ins, C_7, QVLVVRSICHRAAKDRPAGSGSQGCCLPSFHQ, (SEQIDNO:4505); CCDS46911.1, Ins, C_7, RDTRLQKKDSSSWQKGTYRPTGNSRHPRTRWT, (SEQIDNO:4506); CCDS43212.1, Ins, C_7, RGGPVHTLAAQQGVTPEFRGDGAPGHQRCGDR, (SEQIDNO:4507); CCDS32414.1, Del, C_7, RGRHLLLTAPGAPRGAPAGPVRMGRPTPRAPR, (SEQIDNO:4508); CCDS44115.1, Del, C_7, RNRSRCWENACSHSSKQCIQIWLGRSRECCWR, (SEQIDNO:4509); CCDS44613.1, Del, C_7, RVQKMIGTFLNEHYQGGQLHLSLAVLLLELSP, (SEQIDNO:4510); CCDS10410.1, Del, C_7, SCSHRCLRLPSRHPLPAAGEPPRARAGLGPPA, (SEQIDN0:4511); CCDS56581.1, Del, C_7, SHCQLCSPVARLTWRRPGPKEAISASTRRQLS, (SEQIDNO:4512); CCDS5187.1, Del, C_7, SSVAGPPPVIILAMPSATATATNAFSSSRCMA, (SEQIDNO:4513); CCDS41294.1, Del, C_7, CLLIYLQSLHAPLHSLLRLFKLCQKLSFHHP, (SEQIDNO:4515); CCDS11253.1, Del, C_7, CPLILSHQAPNPVARLKSQISVQIALTFLVP, (SEQIDNO:4516); CCDS42519.1, Del, C_7, EQRREKGLSSPKHGFGIVSTSVTHLVRGHSV, (SEQIDNO:4517); CCDS58033.1, Del, C_7, IHHLPGTIPLSSGARMCLLSQAPPAGSGGRR, (SEQIDNO:4518); CCDS11439.1, Del, C_7, ILRTPLVLAPRGAHRLAPKNPPRARGPREPL, (SEQIDNO:4519); CCDS 12326.1, Ins, C_7, LPGRKPVCKWRSLHPAALPGGCLPVPAWLGG, (SEQIDNO:4520); CCDS34863.1, Ins, C_7, PRTCCHRTPWLCKPVPPWDCGGSEAKGKDLF, (SEQIDNO:4522); CCDS45128.1, Del, C_7, PTHPEPASCGNWMDDSSFLLFPVLPRGQRCC, (SEQIDNO:4523); CCDS6882.1, Ins, C_7, QLLQPAAPPPAHALPLLRAQGAGRMCHLHPE, (SEQIDNO:4524); CCDS7626.1, Del, C_7, RGRTWQQTWGSRHSSWTKISRESHPAQGKAG, (SEQIDNO:4525); CCDS12143.1, Ins, C_7, RHVPVQPVRVSADRPADTPAPSPSKDLLSHP, (SEQIDNO:4526); CCDS34004.1, Del, C_7, RRRRSAAALAAAAELARPRLLVGWCESATCS, (SEQIDNO:4527); CCDS58103.1, Del, C_7, RTAGTAAATLPPASWLRGPSVRTVPAARSAR, (SEQIDNO:4528); CCDS7332.1, Del, C_7, SIFSTWIIPANVWMLMTALSYPWALMNSWPL, (SEQIDNO:4529); CCDS30673.1, Del, C_7, SQDLLQKVTLPPHHHQCQPWSPTLPRTPLLP, (SEQIDNO:4530); CCDS41878.1, Del, C_7, VKPNMPFIVSMRYFLVKRPSLHRYLSLCIRA, (SEQIDNO:4531); CCDS43544.1, Ins, C_7, WKATQPWGLPGWPPAHGLVLLAHASQHSTWP, (SEQIDNO:4532); CCDS44755.1, Del, C_7, AATRSLPCPGGRTVQDSRKRKEGSRSVEGS, (SEQIDNO:4533); CCDS45523.1, Del, C_7, EKGLRGRSWDPESYVTVRGRASRQERAPVW, (SEQIDNO:4534); CCDS31328.1, Ins, C_7, GGCPTTSQLPTYSQGLPANTSHGQVRQDPV, (SEQIDNO:4535); CCDS10544.1, Del, C_7, GPWQSWPSWPGSWAADIKVPYRRTSSHPQT, (SEQIDNO:4536); CCDS8844.1, Ins, C_7, GTLSRASTTPHALCQQRLRPFLHADHRAGC, (SEQIDNO:4537); CCDS58245.1, Del, C_7, IAALSLMNYILGLSRTSSLKLPKSQNLKSA, (SEQIDNO:4538); CCDS31272.1, Del, C_7, LHLGWAQGRALGWLWGEQPSTPRQTWTRCP, (SEQIDNO:4539); CCDS42514.1, Ins, C_7, LPEPPSPGPLQPKRKPQWPEQPQSRSREHQ, (SEQIDNO:4540); CCDS4665.1, Del, C_7, LTRPWSSRHSASCHRNSLSPSQFSPAWALS, (SEQIDNO:4541); CCDS45958.1, Del, C_7, NPFPNLARPGGASPMERLSVQETLLPQPPK, (SEQIDNO:4542); CCDS59351.1, Ins, C_7, PDSCSRGGSSLLLGAPNPIPAWTPERVCQQ, (SEQIDNO:4543); CCDS47526.1, Ins, C_7, PEASPSHPGSSEKPQPVPPPRAPLVPQPKP, (SEQIDNO:4544); CCDS34637.1, Ins, C_7, PRWGPRVAAELGSHEGGAEGQHHGRHRGPA, (SEQIDNO:4546); CCDS10679.1, Ins, C_7, PTSGAPTAAISGSHGSYPGTEGPGQAIYLH, (SEQIDNO:4547); CCDS41580.1, Ins, C_7, QLSGWPGGDTHAGLLHPGRPLPHGHPARGQ, (SEQIDNO:4548); CCDS45403.1, Ins, C_7, QQRGLQPPGCGRCPDGSAQPRAQVQTSLLL, (SEQIDNO:4549); CCDS12929.1, Ins, C_7, QVRAGAPTGAGAGGDSPSRQQWRERPPPDR, (SEQIDNO:4550); CCDS12090.2, Del, C_7, RRWCGRARAAGARARASAPSWLTRMARKWP, (SEQIDNO:4551); CCDS48197.1, Del, C_7, RSHLTSAVPSASIRPLIWTPCRYMSWSALS, (SEQIDNO:4552); CCDS58110.1, Del, C_7, SAGVLRGSKRTGPACMPTKRRTVMKSTWRS, (SEQIDNO:4553); CCDS32732.1, Del, C_7, SPLHPHPGEKWREGHRAPSSTGFSVSLRPL, (SEQIDNO:4554); CCDS8521.2, Del, C_7, SPSSLLSPCNASIMCPLNPAAQDGARCPSC, (SEQIDNO:4555); CCDS56148.1, Ins, C_7, SSSASSASAATSPLRRDCLSRGAREVLRIR, (SEQIDNO:4556); CCDS55185.1, Del, C_7, SSSRSPTPRPSGPRSSASSPCIPISTPSTT, (SEQIDNO:4557); CCDS13948.1, Del, C_7, VPAPSSAGSVRKTPHPLRDPSAACQTSQGV, (SEQIDNO:4559); CCDS58576.1, Ins, C_7, WWRRVCAQLLPCRLPLRASPLPGAPSQVPI, (SEQIDNO:4560); CCDS12665.1, Ins, C_7, YSCPPGSTEVGKIPEKQRCPQPLTRSRYGE, (SEQIDNO:4561); CCDS58553.1, Del, C_7, DKSPSSPLPLVPLDSRPYSPFPGQSNGIS, (SEQIDNO:4562); CCDS31473.1, Del, C_7, GLTMSPLWPSCPWTGCRCWKPCAGTGLAP, (SEQIDNO:4563); CCDS3324.1, Del, C_7, GTSYTSQAWGHQWRWWTWGGLRTGPCAKP, (SEQIDNO:4564); CCDS992.1, Del, C_7, MSVLSVGATSCKPIFRPISGRPVCTSLAV, (SEQIDNO:4566); CCDS11784.1, Ins, C_7, PAAAPRGPATAGTGAAGTGQRGPAHFIRL, (SEQIDNO:4567); CCDS46075.1, Ins, C_7, PAPIPWSSPITNLTTTPGADGCEPGGRPC, (SEQIDNO:4568); CCDS8378.1, Ins, C_7, PEPLGSSEGPGHCVRGCAQRQRQRLCVPV, (SEQIDNO:4569); CCDS46136.1, Del, C_7, PPTPTPPTLSTRRCPRPACCLSSAPAAPP, (SEQIDNO:4570); CCDS10276.1, Ins, C_7, STAPLCPGVPVSSTEGGRKATQLSTNACH, (SEQIDNO:4574); CCDS8002.1, Ins, C_7, TDGLLYLCSSEMLFPRPVVRRRGCVQWGW, (SEQIDNO:4575); CCDS2670.2, Ins, C_7, TDPGPAAGGGLLHPGPGWLQDLCVAGTHV, (SEQIDNO:4576); CCDS9409.1, Del, C_7, TPSRWTARTQMMWYQLAKEEPGVGACALD, (SEQIDNO:4577); CCDS31143.1, Del, C_7, AACWAAPPPASDASPGPRPGPAQKAGSV, (SEQIDNO:4578); CCDS45510.1, Ins, C_7, ATACGCGAGHPGRERELQPRGAQECPTA, (SEQIDNO:4579); CCDS32598.1, Del, C_7, AVLLPKCRVSSGAAGTRNRTKSQVRRQP, (SEQIDNO:4580); CCDS31796.2, Del, C_7, CHLASCPQCFHQWGRHHHSHRYQEWYLR, (SEQIDNO:4581); CCDS11637.1, Ins, C_7, FPLQLRLVVSSNQVSGDLSSCYPKRNPL, (SEQIDNO:4582); CCDS9266.1, Ins, C_7, HGGCREEEVRGPAGGDPRGALLHRAPRG, (SEQIDNO:4583); CCDS59406.1, Del, C_7, KPSHPCPHLPHRPQPGRLGAWAPPGCSW, (SEQIDNO:4584); CCDS42268.1, Del, C_7, LLSQTPRLPPCLHTEEPSHWTGGPRSPP, (SEQIDNO:4585); CCDS53578.1, Ins, C_7, LRLHCQHSSLQGHEKEPLLTKSWGALVY, (SEQIDNO:4586); CCDS3104.1, Ins, C_7, PGGPRASGFQLSRPVRSRICCRLPATDE, (SEQIDNO:4588); CCDS46085.1, Ins, C_7, PGPEGGLPSGNPWSDPQDCRGRHWRCLR, (SEQIDNO:4589); CCDS44578.1, Del, C_7, PMPPPPRRPLPPPPRAAQRTVTRGKRLN, (SEQIDNO:4590); CCDS45399.1, Del, C_7, PRPSPLLQRWKRLGRSSVRPSSSSTCTG, (SEQIDNO:4591); CCDS58443.1, Ins, C_7, PRRGAPEWGACTLSFHTLTLPLHRTPPR, (SEQIDNO:4592); CCDS32054.1, Ins, C_7, QDHTSRTWSTQYASTWDSSGEWDGHPPG, (SEQIDNO:4593); CCDS54706.1, Del, C_7, RKAWAPPPCTRSPASRSSDRCPWSPTRP, (SEQIDNO:4595); CCDS450.1, Ins, C_7, RNSRAPRLARRQRRQGLPREDRAPRQSG, (SEQIDNO:4596); CCDS54914.1, Del, C_7, RRTSWPSALAYLKVQTPQKRNSSQNQNT, (SEQIDNO:4597); CCDS47494.1, Del, C_7, SLLRISALLPQTLQLSSSALELTCPSLP, (SEQIDNO:4599); CCDS11784.1, Del, C_7, SSSPPWPSNRRHRGRRHRAARPSSFHST, (SEQIDNO:4600); CCDS346.2, Del, C_7, STAARPARVLSCRLSSAVWLPARWKASG, (SEQIDNO:4601); CCDS276.1, Ins, C_7, TDAPSGPGLPAPEEPITVSGPTVSQGPI, (SEQIDNO:4602); CCDS14010.1, Del, C_7, TPVLPQGCSLSLLHTTFPHRQVPHILDW, (SEQIDNO:4603); CCDS44281.1, Del, C_7, VWRHLRRSWRNSLGCSTMEGRRKPWSGP, (SEQIDNO:4604); CCDS44650.1, Del, C_7, WHTPHLRTRQAMVTNPSHQALAGGRGPP, (SEQIDNO:4605); CCDS14003.1, Del, C_7, YAASPLPIAAPARAPLGPVGWHVRTAPH, (SEQIDNO:4606); CCDS7083.1, Del, C_7, AACWAAPPPASDASPGPRPGPAQAGSV, (SEQIDNO:4607); CCDS14091.2, Del, C_7, AGSTGASRPLRTRRTRSQCRRSWSPAR, (SEQIDNO:4608); CCDS11420.1, Del, C_7, GALLPGSPLSFSFPHWEPLDLRTSVPG, (SEQIDNO:4609); CCDS7045.1, Ins, C_7, HRQLQHPGLRQVQRDLRSGLPARAHGV, (SEQIDNO:4610); CCDS32732.1, Ins, C_7, LLPSTLTPGRSGERATEPPAAPAFPCP, (SEQIDNO:4611); CCDS398.1, Ins, C_7, PAAGVPGTSPAWHWHCGETNQAPGQLL, (SEQIDNO:4612); CCDS2781.2, Ins, C_7, PALQLHDSAVLFEQRHWWGRDCFPCSR, (SEQIDNO:4613); CCDS45980.1, Ins, C_7, PAPHRRKWKRRRRSGTSTTRCSWTWSM, (SEQIDNO:4614); CCDS45979.1, Ins, C_7, PAPHRRNPESRSAGRPGPPQSSPACLC, (SEQIDNO:4615); CCDS32041.1, Ins, C_7, PPWEQHSHPGQSRDLRAIPTESCSSPE, (SEQIDNO:4616); CCDS42665.1, Ins, C_7, QREWRCLLCGPAWTCFQKGLLHVRKQL, (SEQIDNO:4617); CCDS45871.1, Ins, C_7, RMEEGGSDPKIWAKCWQERCLLLQSKW, (SEQIDNO:4618); CCDS8917.1, Del, C_7, RPPGACWALLSVGSSPPMLAEASTQTS, (SEQIDNO:4619); CCDS33876.1, Ins, C_7, RPWHEPRAAGLLRFSFLCTAWSPGLWT, (SEQIDNO:4620); CCDS45176.2, Del, C_7, TSPATRFYRTWPLPGPHAWTSTPRTPT, (SEQIDNO:4621); CCDS8666.1, Ins, C_7, WAVPTSSLAKPWGFKCPTQLDSATQGG, (SEQIDNO:4622); CCDS55513.1, Ins, C_7, WHTGIHYVTTRFHPICGIKEKRVPLSC, (SEQIDNO:4623); CCDS9989.2, Del, C_7, WREAWRRSSWPRWTMAWAQHGSPAIGG, (SEQIDNO:4624); CCDS45637.1, Ins, C_7, ECDRVYLNYPPFNGGHSAAQPASGPE, (SEQIDNO:4625); CCDS9592.1, Ins, C_7, GSSGPWAWFGSPRQPRGGGGAGTGRG, (SEQIDNO:4626); CCDS47235.1, Ins, C_7, HTGAGTLRFAIPRPFTPRTGRLLRRS, (SEQIDNO:4627); CCDS48134.1, Del, C_7, LWCCIVFSGSGPSGMRICCGPRCWKP, (SEQIDNO:4628); CCDS44254.1, Ins, C_7, PACHRVAALWIPASHLHPVRPLLSPN, (SEQIDNO:4629); CCDS33927.1, Ins, C_7, PASRRGEKGASTSPTSACLLHSETNL, (SEQIDNO:4630); CCDS33602.1, Ins, C_7, PAVPAARPAQLTAQLQRQLWPCPISQ, (SEQIDNO:4631); CCDS41290.1, Del, C_7, PGPLKIRASASRGDTAVREASSLLKS, (SEQIDNO:4632); CCDS1075.1, Ins, C_7, PGSWLPTPRQHELSQPTLQPASGSKL, (SEQIDNO:4633); CCDS31013.1, Ins, C_7, PWWGPVRDGLCAQGRKPHPPGHGLLH, (SEQIDNO:4634); CCDS32928.1, Ins, C_7, RNLLHLAPASPGGPESPAGLLPGAAS, (SEQIDNO:4635); CCDS42523.1, Del, C_7, RPPRSPGPAPLGDPRAAPPGSSGSFW, (SEQIDNO:4636); CCDS42103.1, Del, C_7, RRPPKPDQALPRPLGDLMVQPQLRPR, (SEQIDNO:4637); CCDS6870.1, Del, C_7, SGLSRAFTWPCCGSPASTSPSSSASP, (SEQIDNO:4638); CCDS12328.1, Del, C_7, SHNPHPLQLPSPYRATHPSSRPPHSL, (SEQIDNO:4639); CCDS12013.1, Ins, C_7, TAPDAQAAQLPVVREDLLLGQRPADP, (SEQIDNO:4640); CCDS994.1, Del, C_7, VVLRLGNLPPFFRGSEVPFPRPCRTK, (SEQIDNO:4642); CCDS59420.1, Del, C_7, WQTSTLALITSNIRENVLWDFWDHGG, (SEQIDNO:4643); CCDS45376.1, Del, C_7, ASRWNPLRRTRALRGPPRQRAAAPH, (SEQIDNO:4644); CCDS463.1, Ins, C_7, CASPEKPLNAFCRLHYQHPPPPLPR, (SEQIDNO:4645); CCDS9392.1, Ins, C_7, CHHPATGHHTAPRPASIAGLRTNRI, (SEQIDNO:4646); CCDS941.1, Del, C_7, EGLHSDLPWVTQVLCLRMVCVDLLH, (SEQIDNO:4647); CCDS12793.1, Del, C_7, KTGVSQAPQASCPTSTPTPRPGYPP, (SEQIDNO:4648); CCDS44216.1, Ins, C_7, LGSLTQRHHQPEWYNLTESPARLSA, (SEQIDNO:4649); CCDS5721.1, Del, C_7, LLSCPATRGSRLPILMVHTSSHQRG, (SEQIDNO:4650); CCDS13906.1, Ins, C_7, LLTSSFCAPRTQQLHALHWNRRVFL, (SEQIDNO:4651); CCDS4317.1, Ins, C_7, LLVSNHQGEQRLCLPSDPGWMAGDC, (SEQIDNO:4652); CCDS 13498.1, Ins, C_7, LTPAAPLPWWLEGHHREATGRPRGG, (SEQIDNO:4653); CCDS46143.1, Ins, C_7, PAAPLGPQPCPQSLSQRLQSRRPPP, (SEQIDNO:4654); CCDS 1547.1, Ins, C_7, PAARRPYPEALADGARLARLFLRVL, (SEQIDNO:4655); CCDS46659.1, Ins, C_7, PGACSAPQRGGAEVVCGERGGDSLC, (SEQIDNO:4656); CCDS45225.1, Ins, C_7, PPWSRALRASPWPWPRSPTPWSTSL, (SEQIDNO:4657); CCDS5696.1, Del, C_7, PRPPCSPAPPRTRPCLTPWCTRRSR, (SEQIDNO:4658); CCDS45937.1, Ins, C_7, PTSASTPILEPWLYTRVWVRVWPHA, (SEQIDNO:4659); CCDS6415.1, Ins, C_7, QLSVVSPWPPLFLGPHQHPEGFPGI, (SEQIDNO:4660); CCDS10666.1, Del, C_7, QPSLGPPRLRMVRRWTAMTRMMPPP, (SEQIDNO:4661); CCDS10857.1, Ins, C_7, QSPHHAGRPQSPHHAGHLRSLDHTQ, (SEQIDNO:4662); CCDS32553.2, Ins, C_7, RLPPLPAAAAAAATAAPAAAAPAAR, (SEQIDNO:4663); CCDS 10677.1, Ins, C_7, RRGRQRRLQKHLLRHHARRRQGPGV, (SEQIDNO:4665); CCDS9756.1, Del, C_7, RYHVRIFLVSQGLNHLNLTKMKSTF, (SEQIDNO:4666); CCDS9746.1, Del, C_7, SKLTEIEMQYLKFKQKRNPLDFLFL, (SEQIDNO:4667); CCDS13098.1, Del, C_7, SPQKHLRICTSLGLWLIKPSSMQVG, (SEQIDNO:4668); CCDS47048.1, Del, C_7, GPAGPPPPLRRRRRPARPPHPRAT, (SEQIDNO:4669); CCDS5809.1, Del, C_7, IPYQMWLKCSGRSCRLTDRLFVDG, (SEQIDNO:4670); CCDS7101.1, Ins, C_7, KQPPPHPNLADWRSNRKLTHSGWV, (SEQIDNO:4671); CCDS32598.1, Ins, C_7, KQYCCQSAECHQAPPAPETGRRAK, (SEQIDNO:4672); CCDS6575.1, Del, C_7, LLKLTWIMWSLKSTDTCRRSSGAG, (SEQIDNO:4673); CCDS46221.1, Del, C_7, MTPGPGAGPAACSSCAPALCAAAA, (SEQIDNO:4674); CCDS117.2, Ins, C_7, PAICAPGQNALQELLLEDLLLLQI, (SEQIDNO:4675); CCDS7929.1, Ins, C_7, PNPAPAQPGTTGHDREARRCPATV, (SEQIDNO:4677); CCDS2531.1, Ins, C_7, PPGDLHRCARSRGPEADSEERRST, (SEQIDNO:4678); CCDS9392.1, Del, C_7, PPPSHRAPHSPPPSQHRRAQDQPH, (SEQIDNO:4679); CCDS3020.1, Del, C_7, PQSPRGKSSRQACCTWWGTTSGRP, (SEQIDNO:4680); CCDS45277.1, Ins, C_7, PVWGAERSQHSHGAGQAPAALPLL, (SEQIDNO:4681); CCDS8662.1, Ins, C_7, QHWHCCHPRGHFRRGGHQGCPRER, (SEQIDNO:4682); CCDS54035.1, Ins, C_7, RGSFPRQRPCQRIPQTRRTEKHPP, (SEQIDNO:4683); CCDS33928.1, Ins, C_7, RPPERGTEHLPAPPASRRGEKTNL, (SEQIDNO:4684); CCDS403.1, Ins, C_7, RPPWSSWLRQTWFGWASWGPRRQG, (SEQIDNO:4685); CCDS32744.1, Del, C_7, SLPATSEGSHTQPPLSPPMGCKSL, (SEQIDNO:4686); CCDS32861.1, Del, C_7, SSTPSSWTSRSWTTRSPSTPTANC, (SEQIDNO:4687); CCDS34390.1, Del, C_7, TLILTDFATWSQQTISGMQGTSKQ, (SEQIDNO:4688); CCDS45225.1, Del, C_7, TMVQGPAGLPLAMAQVTHPLVHIT, (SEQIDNO:4689); CCDS8421.1, Del, C_7, TPTACGISRWPQTMQYSSRSKPSA, (SEQIDNO:4690); CCDS 10677.1, Del, C_7, TWAATSTTKTSATSSRTATPRTRS, (SEQIDNO:4691); CCDS2030.1, Del, C_7, VSVYSGNLLSFSRRGMKSSVRLKN, (SEQIDNO:4692); CCDS34562.1, Del, C_7, ASAPTSPLPSSPPSSPQFQIRTT, (SEQIDNO:4693); CCDS1646.1, Del, C_7, ATPLRGAETPKAASSVSARTPTS, (SEQIDNO:4694); CCDS1661.1, Del, C_7, FLHGMLAKFRQPPLLTPCGRQTL, (SEQIDNO:4695); CCDS2027.1, Del, C_7, GNLSPWLSRSPRPRWSLWAWRCS, (SEQIDNO:4696); CCDS11218.1, Del, C_7, GTWTPGPSSPLETETLRWRLMTW, (SEQIDNO:4697); CCDS43455.1, Ins, C_7, HLSNISRPHPHHSGHGTCCAEEE, (SEQIDNO:4698); CCDS8844.1, Del, C_7, HPLQSLHHPPCSLSAATQAIPPR, (SEQIDNO:4699); CCDS55088.1, Ins, C_7, HTIQHASVPTASCISKLNSYTET, (SEQIDNO:4700); CCDS45696.1, Ins, C_7, HWRRGVPYSLQDSGDAHCPRVLT, (SEQIDNO:4701); CCDS44925.2, Del, C_7, IFPFLWALILHLYIRVLLVLSNF, (SEQIDNO:4702); CCDS47954.1, Del, C_7, KSPHSWKTSGPCSLPLDRTFLQC, (SEQIDNO:4703); CCDS 11627.2, Ins, C_7, LQGASQRPGQEGQVYPADGHCSR, (SEQIDNO:4704); CCDS2934.1, Del, C_7, MPTGLRKARMEGQPMRCLHLPPS, (SEQIDNO:4705); CCDS4266.1, Del, C_7, NHPDPSARACWSCVDTRPRCLLP, (SEQIDNO:4706); CCDS47918.1, Del, C_7, NQKIRSSSRPGGAEKEPIFQKQA, (SEQIDNO:4707); CCDS 10630.1, Ins, C_7, PELPPPVGGHSSATFEPWTPGQL, (SEQIDNO:4708); CCDS47413.1, Ins, C_7, PGRQERLRGPPSLSFCHRSDRNR, (SEQIDNO:4709); CCDS3607.1, Del, C_7, PPRPRRRPPRRPPRLWAPTTQAA, (SEQIDNO:4710); CCDS31269.1, Ins, C_7, PRNPSLIAHGTRHPHQLPHRSQQ, (SEQIDNO:4711); CCDS10972.1, Del, C_7, PSRHTHIERTGLRRCPTAVFMAA, (SEQIDNO:4713); CCDS42824.1, Ins, C_7, RAGVGDHAQKATPQWGALILLGF, (SEQIDNO:4714); CCDS42525.1, Ins, C_7, REPWPAIDGDRHRLGAGSAAVPH, (SEQIDNO:4715); CCDS47347.1, Del, C_7, RLLARRTAGGQCTMNWRSAGGPS, (SEQIDNO:4716); CCDS12065.1, Ins, C_7, RRRQRLWTRAEPQRARVPPLPTL, (SEQIDNO:4717); CCDS13670.1, Del, C_7, RSYRFSLTLEAVTLPWQEPRTPT, (SEQIDNO:4718); CCDS11785.1, Del, C_7, RYSSWSRTSPASLSWKSQTSTSS, (SEQIDNO:4719); CCDS117.2, Del, C_7, SNLRAGTECPAGTSSGRPSPPAN, (SEQIDNO:4720); CCDS34434.2, Ins, C_7, SPTTPTPAADYGERRVPKHRRPL, (SEQIDNO:4721); CCDS5689.1, Del, C_7, TRCLPATVEPRRPGKRRAGCGRW, (SEQIDNO:4722); CCDS47839.1, Del, C_7, TRVHVSSPPSSATLLCWENGSRV, (SEQIDNO:4723); CCDS30673.1, Ins, C_7, TTTNVSPGPRHSPGHPSCHEECH, (SEQIDNO:4724); CCDS55589.1, Del, C_7, TWKSCTLRPRRGSAPYNTKRNRN, (SEQIDNO:4725); CCDS45544.1, Del, C_7, YVSSSSQGLEWLRVKMASWACRS, (SEQIDNO:4726); CCDS 12196.1, Del, C_7, ASTSGRTRRSAKMSTNACPARV, (SEQIDNO:4727); CCDS 10630.1, Del, C_7, GATSASGWSFLRHFRALDPRPA, (SEQIDNO:4728); CCDS44233.1, Ins, C_7, GGAAFPISFIPGAHPASSAWGR, (SEQIDNO:4729); CCDS7537.1, Ins, C_7, GLRFALSPGTARHLRRVPPPLP, (SEQIDNO:4730); CCDS1821.1, Ins, C_7, GRVVHVPAAHPQLLAGAGGQRL, (SEQIDNO:4731); CCDS58097.1, Del, C_7, HLSQVKGPVNWKDAQVPRLRFN, (SEQIDNO:4733); CCDS33007.1, Ins, C_7, HPQWNHIPQIRRLQRLLAGQGD, (SEQIDNO:4734); CCDS12090.2, Ins, C_7, LDAGVEGPEQLGHGRELPHRPG, (SEQIDNO:4735); CCDS32548.1, Ins, C_7, LGGQRMPAAHHQESPVRSPESG, (SEQIDNO:4736); CCDS11733.2, Ins, C_7, LPCPRLRHPPSCLPALQAGPAD, (SEQIDNO:4737); CCDS32409.1, Del, C_7, NLLVSTQLGTAASPTQHQQLPR, (SEQIDNO:4738); CCDS3541.1, Del, C_7, NSQILTSHLNIQIKIAVWSTLP, (SEQIDNO:4739); CCDS12065.1, Del, C_7, PAAAALDEGRTTTCKGSTPTDA, (SEQIDNO:4740); CCDS44112.1, Ins, C_7, PAGCLRQRRLSKGCWRWSCGAL, (SEQIDNO:4741); CCDS8944.1, Ins, C_7, PALCPECPPTWTTYLQCHHGNY, (SEQIDNO:4742); CCDS12160.1, Ins, C_7, PEATPAQQQGVRPEEPRVLQQA, (SEQIDNO:4743); CCDS13401.1, Ins, C_7, PGTAEHCAHRLWAEFHFSTSRG, (SEQIDNO:4744); CCDS45171.1, Del, C_7, PHPRGSPAWSRGAARPRPLRML, (SEQIDNO:4746); CCDS32581.1, Ins, C_7, PPQAALVGGRLEGHSGQQAALL, (SEQIDNO:4747); CCDS45671.1, Del, C_7, QAAVAPASAPAPTEAARPPTPR, (SEQIDNO:4748); CCDS13418.1, Ins, C_7, QKPPWEHQSEVQHGDKIGEKPL, (SEQIDNO:4749); CCDS42519.1, Ins, C_7, QNREEKKDFLPRNMDLALSQHQ, (SEQIDNO:4750); CCDS44299.1, Ins, C_7, QPWRPHAGPAVQPPEPASGLPW, (SEQIDNO:4751); CCDS6556.1, Ins, C_7, QVREPHVHVLPGHVLHRLPHSV, (SEQIDNO:4752); CCDS 14649.1, Ins, C_7, RGFTDRFSKTGRSAEPHSGRDT, (SEQIDNO:4753); CCDS9991.1, Del, C_7, RRSWWRVPTPPSSTLTSGPTPL, (SEQIDNO:4754); CCDS42972.1, Ins, C_7, RTPGTCGLRVGAGRIRPERAGT, (SEQIDNO:4755); CCDS628.2, Ins, C_7, SDCCPTKSQVPPLPVTPPYTSS, (SEQIDNO:4756); CCDS5702.1, Del, C_7, TGRRRRSYPRSPSPGITTCVMR, (SEQIDNO:4757); CCDS32581.1, Del, C_7, AAGCTRGRTTGRPFWTASGPA, (SEQIDNO:4758); CCDS414.1, Del, C_7, ARAIATRPGGWNRMGEPRGFC, (SEQIDNO:4759); CCDS2446.1, Del, C_7, ARGTSPIRHCSHSSSCSGPSS, (SEQIDNO:4760); CCDS56236.1, Del, C_7, CPQWPGQRRLSAGSGVLTAGC, (SEQIDNO:4761); CCDS44578.1, Ins, C_7, CQCRHLHAGPFPLLPELHSEL, (SEQIDNO:4762); CCDS30880.1, Ins, C_7, FVQLHRHPPPFLQARQPAQSQ, (SEQIDNO:4763); CCDS10206.1, Ins, C_7, HRAQRGDRGVSDPVQQQPHAA, (SEQIDNO:4764); CCDS4832.1, Ins, C_7, NDCPGENPPRKIPSTSLQPEH, (SEQIDNO:4765); CCDS2062.1, Ins, C_7, NTPQQRKGHPDVRLTTAAPSL, (SEQIDNO:4766); CCDS3324.1, Ins, C_7, PAPPTPPRPGGTSGGGGHGGD, (SEQIDNO:4767); CCDS 13227.1, Ins, C_7, PEEGSPPQLRQWWAQGADPLA, (SEQIDNO:4768); CCDS41837.1, Del, C_7, PKCRVPAPLLTWLEPCTPVPR, (SEQIDNO:4769); CCDS42518.1, Ins, C_7, PWLQRAAPTHAATGPTPHQPR, (SEQIDNO:4770); CCDS46682.1, Ins, C_7, QGRGFPGGPCGAVSNRRDCRG, (SEQIDNO:4771); CCDS31013.1, Del, C_7, QPACLSSAPSTLRIVTGPSTT, (SEQIDNO:4772); CCDS2063.1, Ins, C_7, QPGRCVSLSGHFHNVSKQATL, (SEQIDNO:4773); CCDS32414.1, Ins, C_7, RGAGTSFSQPPGHQEARQLGQ, (SEQIDNO:4774); CCDS45766.1, Del, C_7, RPKEVAETLSFRIPGTQRQYL, (SEQIDNO:4775); CCDS11253.1, Ins, C_7, RTFICPQGTRNEQRQREIQWV, (SEQIDNO:4776); CCDS44112.1, Del, C_7, SRLSPPEAAFQRMLAMELRSP, (SEQIDNO:4778); CCDS41346.1, Ins, C_7, VDRPQHTEWLSWSNLYRIRHQ, (SEQIDNO:4779); CCDS9999.1, Del, C_7, AGRAAPAPSPRTAAACPTPV, (SEQIDNO:4780); CCDS32045.1, Del, C_7, GVRAQPSVEWQKVARQLACN, (SEQIDNO:4782); CCDS54132.1, Ins, C_7, HAAGLHLSRGHQSSSQQWQL, (SEQIDNO:4783); CCDS8162.1, Ins, C_7, HRGAPQAPTRLHLLPGHPLL, (SEQIDNO:4784); CCDS47396.1, Ins, C_7, HTRLGQTHQHPGLQWGLYGA, (SEQIDNO:4785); CCDS45544.1, Del, C_7, KMSACLSPASSLAHSWMPGV, (SEQIDNO:4786); CCDS34137.1, Del, C_7, KWRSVLCTSPRSWGTQRLWA, (SEQIDNO:4787); CCDS14078.1, Del, C_7, LIHCYMALCSGPRPRCRPHQ, (SEQIDNO:4788); CCDS8260.1, Ins, C_7, QAKSGRNTSMGQSSAETANQ, (SEQIDNO:4789); CCDS6026.1, Ins, C_7, QQELHLAAGGPHPVPHLPAV, (SEQIDNO:4790); CCDS1051.1, Ins, C_7, QYLHPHRVLSPWEPAGHSGE, (SEQIDNO:4791); CCDS377.1, Del, C_7, SPNAAAKRSRRRTRGVRLSL, (SEQIDNO:4792); CCDS33007.1, Del, C_7, SPVEPHSANPTSSETSCWPR, (SEQIDNO:4793); CCDS33055.1, Ins, C_7, TQAEKRFPYTEKARPMEISV, (SEQIDNO:4794); CCDS42723.1, Del, C_7, TSPAPRLNRHPLTSGSWING, (SEQIDNO:4795); CCDS12603.1, Del, C_7, APGPSTCGATSVPRRTGCS, (SEQIDNO:4796); CCDS11409.1, Del, C_7, AVSAEPGPGTSCATTTMAP, (SEQIDNO:4798); CCDS617.2, Ins, C_7, CPCSLTCCPAYCSQQGPWF, (SEQIDNO:4799); CCDS14033.1, Ins, C_7, LAEQDRERQPQHRAVGAGV, (SEQIDNO:4800); CCDS725.1, Ins, C_7, LDVWAPKSGRAVPSWLSKS, (SEQIDNO:4801); CCDS54507.1, Ins, C_7, LQDLSPRHRYWKGKERGDL, (SEQIDNO:4802); CCDS 10278.1, Del, C_7, LRRPARSWRPCSSTGRARS, (SEQIDNO:4803); CCDS730.1, Ins, C_7, PAAGLGRLGPQDFHVFFFN, (SEQIDNO:4804); CCDS44228.1, Ins, C_7, PAGGLLRGSWACRERSTHP, (SEQIDNO:4805); CCDS50.1, Ins, C_7, PATVRGLPDQQIQEGDLQV, (SEQIDNO:4806); CCDS42516.1, Ins, C_7, PDITCQVRGQHLHPQMQVL, (SEQIDNO:4807); CCDS5213.1, Ins, C_7, PEATLLYPNSTGNRGANRR, (SEQIDNO:4808); CCDS54907.1, Ins, C_7, PEVTHCDGSLSYRFCLPRR, (SEQIDNO:4809); CCDS12896.1, Ins, C_7, PGPLPPQRGPRAERRHRPL, (SEQIDNO:4810); CCDS33858.1, Ins, C_7, PHYHTQAPQAPRLFPKRGW, (SEQIDNO:4811); CCDS7591.1, Ins, C_7, PLFCIKERWTKTFDFDEER, (SEQIDNO:4812); CCDS6424.1, Ins, C_7, PRGLPRRPGGGGEPGAAEP, (SEQIDNO:4813); CCDS11608.1, Ins, C_7, QGTSEGGRESSGPLHSAAG, (SEQIDNO:4814); CCDS7736.1, Ins, C_7, QRGRGPHHPALRQWRLRRP, (SEQIDNO:4815); CCDS58434.1, Del, C_7, RLLLPRMSRRCLPSGSLET, (SEQIDNO:4816); CCDS11177.1, Ins, C_7, RPAEAHLCRQAAGRWPHSF, (SEQIDNO:4817); CCDS42139.1, Del, C_7, SERKWSWMTLPGSWSMRRT, (SEQIDNO:4818); CCDS43455.1, Del, C_7, SIQHLQAPSPSLWPWNMLC, (SEQIDNO:4819); CCDS8162.1, Del, C_7, SRGPPGPNEAPPPSWTPAS, (SEQIDNO:4820); CCDS42248.1, Del, C_7, VQPALTPRPATGKSTSTSC, (SEQIDNO:4822); CCDS44306.1, Del, C_7, VSVSRSPQRVSPMNTEGRP, (SEQIDNO:4823); CCDS7967.1, Del, C_7, YIWALSISSTSMIKPLMRN, (SEQIDNO:4824); CCDS14616.1, Del, C_7, APLRLWSWCGTSQTYFGS, (SEQIDNO:4825); CCDS44963.1, Del, C_7, AQPGVSIRFVHRPFLGRW, (SEQIDNO:4826); CCDS14033.1, Del, C_7, CRTRPRKAASAQSSRSGG, (SEQIDNO:4828); CCDS3369.1, Del, C_7, GTASPWCWASTSSTARRT, (SEQIDNO:4829); CCDS54060.1, Ins, C_7, HFLRRTQAEHTVLLHRRV, (SEQIDNO:4830); CCDS30673.1, Del, C_7, HHQCQPWSPTLPRTPLLP, (SEQIDNO:4831); CCDS10687.1, Ins, C_7, NEAGVEDRHLRGRAVWGC, (SEQIDNO:4832); CCDS34063.1, Ins, C_7, PAAAHRHGPTHHPSAGLQ, (SEQIDNO:4833); CCDS33022.2, Ins, C_7, PAPATTSPSQAAQLCHDG, (SEQIDNO:4834); CCDS53838.1, Ins, C_7, PATPTPTSPTCRAHQAAL, (SEQIDNO:4835); CCDS7373.1, Ins, C_7, PGPGLLPLSMQLQPPYHG, (SEQIDNO:4836); CCDS59018.1, Ins, C_7, PGRQERLRGPPSLSDRNR, (SEQIDNO:4837); CCDS6067.1, Ins, C_7, PPPGQPQVFGGCFWFSPN, (SEQIDNO:4838); CCDS54707.1, Del, C_7, PRAGPRCHRRRRRCCCCC, (SEQIDNO:4839); CCDS6578.1, Ins, C_7, QCLPCSYPCCQPYPAGQL, (SEQIDNO:4840); CCDS54772.1, Ins, C_7, QGSASRPLSAPLWRGFLL, (SEQIDNO:4841); CCDS45958.1, Ins, C_7, QTPFQTSHAQEEQVRWRG, (SEQIDNO:4842); CCDS9261.1, Ins, C_7, RAAPRAAGTGGQGHVEGS, (SEQIDNO:4843); CCDS32548.1, Del, C_7, RGTAHARCAPSRESSSES, (SEQIDNO:4844); CCDS47413.1, Del, C_7, RKTRAAPRTPLPFFLSQV, (SEQIDNO:4845); CCDS34455.1, Ins, C_7, RLPHNPSPFSALPEGEQP, (SEQIDNO:4846); CCDS12584.1, Ins, C_7, RLVLPFGSEPQPLLPLGL, (SEQIDNO:4847); CCDS46055.1, Del, C_7, RPLPGNGVRKAQNGWCRH, (SEQIDNO:4848); CCDS6607.1, Ins, C_7, RWTGQLLSTDADRDGAWE, (SEQIDNO:4849); CCDS54686.1, Ins, C_7, SCQLCSHHLCHTGRGPPL, (SEQIDNO:4850); CCDS46075.1, Del, C_7, SPDTLVVPHHQPHHHPRS, (SEQIDNO:4851); CCDS4798.1, Ins, C_7, TPATSHLQYGLHITEVSG, (SEQIDNO:4852); CCDS45399.1, Ins, C_7, TPGPAPSCSGGSGSADRA, (SEQIDNO:4853); CCDS44020.1, Del, C_7, VRPTRRAPLTRPPLSLPT, (SEQIDNO:4855); CCDS 14374.1, Ins, C_7, WLHRLWWPHGAYCVQRCT, (SEQIDNO:4856); CCDS56236.1, Ins, C_7, AAHSGQDRGGCRQGRVC, (SEQIDNO:4857); CCDS55513.1, Del, C_7, AHRNTLCYDKIPSNLRN, (SEQIDNO:4858); CCDS30649.1, Del, C_7, ALGPISCSGASASWPPA, (SEQIDNO:4859); CCDS54060.1, Del, C_7, FPSQNSSGTHCTSPPTC, (SEQIDNO:4860); CCDS33500.1, Del, C_7, HRAAPLSPQGPRRPPRS, (SEQIDNO:4861); CCDS2971.1, Del, C_7, KLPTGPPSPRPPRPQCT, (SEQIDNO:4862); CCDS9635.1, Del, C_7, KPAAPRGPIRAKTASIF, (SEQIDNO:4863); CCDS7743.1, Del, C_7, MKSFWLPDSLRMEPRAW, (SEQIDNO:4864); CCDS3279.1, Ins, C_7, NRLPLQSWAKRICPISS, (SEQIDNO:4865); CCDS8944.1, Del, C_7, NSLALCWVPACWVTCLH, (SEQIDNO:4866); CCDS5320.1, Ins, C_7, PAGEGPAHPTGAAAALL, (SEQIDNO:4867); CCDS42563.1, Del, C_7, PAQLSPLTTSTPCWGIS, (SEQIDNO:4868); CCDS32742.1, Ins, C_7, PLLLPLPMQWPPEPQPV, (SEQIDNO:4869); CCDS58446.1, Del, C_7, PMGPPPECCSSWLRRIE, (SEQIDNO:4870); CCDS53553.1, Del, C_7, PPTARTRCRWNHRSCQA, (SEQIDNO:4871); CCDS8932.1, Ins, C_7, QPVLLCQWPLHPHLLDV, (SEQIDNO:4872); CCDS1195.1, Ins, C_7, RAPRPPGPNLTPTHDGA, (SEQIDNO:4873); CCDS34971.1, Del, C_7, RPASSWCSPSRSVPSAS, (SEQIDNO:4874); CCDS361.1, Del, C_7, RRPPRRRRNSLSRSLSN, (SEQIDNO:4875); CCDS45930.1, Del, C_7, SATGRSCGTKSRPRTSR, (SEQIDNO:4876); CCDS 13465.1, Del, C_7, SHRPPRASSAGDLQCQA, (SEQIDNO:4877); CCDS42153.1, Del, C_7, SLRRATQSLASGPGRPV, (SEQIDNO:4878); CCDS53277.1, Del, C_7, SRTPLCSGRGLSSRNFC, (SEQIDNO:4879); CCDS730.1, Del, C_7, SSWARPPRPPGLPRLLF, (SEQIDNO:4880); CCDS41294.1, Ins, C_7, TAYSYTSRASTHPSIPC, (SEQIDNO:4881); CCDS7357.1, Del, C_7, TCRAIVTMTSCLYLRTT, (SEQIDNO:4882); CCDS31911.1, Del, C_7, VISLCQSWMNKSHSHTA, (SEQIDNO:4883); CCDS456.1, Del, C_7, AASAWVPRPGTPPARS, (SEQIDNO:4884); CCDS2405.1, Del, C_7, APWAASWSRSRAAAAA, (SEQIDNO:4885); CCDS56256.1, Ins, C_7, CGSASPHLQQHGGGGL, (SEQIDNO:4886); CCDS42972.1, Del, C_7, DHQGSFRLTFFSWRLK, (SEQIDNO:4888); CCDS42972.1, Del, C_7, DPRDLWSTCRSRTDPS, (SEQIDNO:4889); CCDS 1138.1, Del, C_7, GSPTPTPLSASGLRQW, (SEQIDNO:4890); CCDS3352.1, Ins, C_7, HASFSGLLPPRLGQNG, (SEQIDNO:4891); CCDS10324.1, Ins, C_7, HVSNKPGGDCPVQCSV, (SEQIDNO:4892); CCDS5702.1, Ins, C_7, KLEEEGEATPGLQVLA, (SEQIDNO:4893); CCDS53257.1, Ins, C_7, PAPGHQHSRLPPRASD, (SEQIDNO:4895); CCDS11218.1, Ins, C_7, PEPGLPDLHHHWRQKL, (SEQIDNO:4896); CCDS9121.1, Ins, C_7, PPLLFPATLRPPSAFP, (SEQIDNO:4897); CCDS10081.1, Ins, C_7, PPRAPLAQEVPAQGRL, (SEQIDNO:4898); CCDS7332.1, Ins, C_7, PVFSAPGSYRQTCGCL, (SEQIDNO:4899); CCDS13098.1, Ins, C_7, PVPRSTCGYVPASVSG, (SEQIDNO:4900); CCDS48130.1, Ins, C_7, QCKPQGQAKPDQHRGL, (SEQIDNO:4901); CCDS54092.1, Ins, C_7, QHPHSRACCCKVQILG, (SEQIDNO:4902); CCDS6714.1, Ins, C_7, QRRLVATPLQTAQRRF, (SEQIDNO:4903); CCDS47583.1, Ins, C_7, RAWSKKQFTYQFFQTH, (SEQIDNO:4904); CCDS2030.1, Ins, C_7, RCRCIQETSCLSQEGG, (SEQIDNO:4905); CCDS13227.1, Del, C_7, RGGQPPAVAAVVGARC, (SEQIDNO:4906); CCDS58103.1, Ins, C_7, RGLPEPLLQLYHLPAG, (SEQIDNO:4907); CCDS 11563.1, Ins, C_7, RHASPQGLLGEHLRRG, (SEQIDNO:4908); CCDS45769.1, Del, C_7, RIRVSSRAPRPPWCAE, (SEQIDNO:4909); CCDS32782.1, Ins, C_7, RPSWPIRSGGAPGPAE, (SEQIDNO:4910); CCDS6982.1, Ins, C_7, RRGRPSGSHGRNGPNG, (SEQIDNO:4911); CCDS43446.1, Ins, C_7, RRGWLHLSLGPQGRPV, (SEQIDNO:4912); CCDS11439.1, Ins, C_7, RSSGLRWSWPREEPTA, (SEQIDNO:4913); CCDS12652.2, Ins, C_7, SAHHHLPRTGVGRTEV, (SEQIDNO:4914); CCDS8403.1, Ins, C_7, SPPAATHGPSQRAEEI, (SEQIDNO:4915); CCDS11627.2, Del, C_7, SRCESAAWTRRPSISC, (SEQIDNO:4916); CCDS13839.1, Del, C_7, TMATLTPSSSTTHSGS, (SEQIDNO:4917); CCDS6424.1, Del, C_7, TRAPAQTWWRRRARSC, (SEQIDNO:4918); CCDS6575.1, Del, C_7, VSGSWSLGHPWCCLGL, (SEQIDNO:4919); CCDS1661.1, Ins, C_7, ASSTECWQSSDSLLC, (SEQIDNO:4920); CCDS189.1, Del, C_7, CWTSHLMQMTRPWLN, (SEQIDNO:4921); CCDS31596.1, Del, C_7, GPQERARAGRTMRAQ, (SEQIDNO:4922); CCDS32375.1, Ins, C_7, HDLLPSPAPTQEPLL, (SEQIDNO:4923); CCDS3217.1, Ins, C_7, HHQSTSSNTVRRKFM, (SEQIDNO:4924); CCDS5309.2, Del, C_7, HPGKWAEGGKTAKRD, (SEQIDNO:4925); CCDS30652.1, Ins, C_7, HPPAPASHPAPCQPT, (SEQIDNO:4926); CCDS12035.1, Ins, C_7, PAPAPGRGVAPGGGG, (SEQIDNO:4927); CCDS44327.1, Del, C_7, PDPGLCAEFPDRLKC, (SEQIDNO:4928); CCDS7771.1, Ins, C_7, PEGPGPAGRDTAGHQ, (SEQIDNO:4929); CCDS30559.1, Del, C_7, PGSRAHWPPADAATR, (SEQIDNO:4930); CCDS14306.1, Del, C_7, PHLCRELHPRYLPVH, (SEQIDNO:4931); CCDS 1172.2, Ins, C_7, PHSHLDQKGLKYGPE, (SEQIDNO:4932); CCDS42402.1, Ins, C_7, PLPPTSPGRDVAQDP, (SEQIDNO:4933); CCDS59451.1, Del, C_7, PLRGHLPQFLHHRVW, (SEQIDNO:4934); CCDS44963.1, Ins, C_7, PPNPAFPYGLFTGPF, (SEQIDNO:4935); CCDS41878.1, Ins, C_7, PSSQICHSLYPCDIF, (SEQIDNO:4936); CCDS9991.1, Ins, C_7, QEDPGGGCQRRPPRH, (SEQIDNO:4937); CCDS12921.1, Ins, C_7, QGQEAPGHQRDPSAL, (SEQIDNO:4938); CCDS11157.1, Ins, C_7, QVRQDRRHGHADAPE, (SEQIDNO:4939); CCDS43212.1, Del, C_7, RRPCTYSGSTTRSHS, (SEQIDNO:4941); CCDS5696.1, Del, C_7, RTRPCLTPWCTRRSR, (SEQIDNO:4942); CCDS45820.1, Ins, C_7, RWRKIFKDGRAYIKH, (SEQIDNO:4943); CCDS48134.1, Ins, C_7, SCGAASCSPGAGPLG, (SEQIDNO:4944); CCDS46987.1, Ins, C_7, SFSLCTSQGFPFDPK, (SEQIDNO:4945); CCDS5721.1, Ins, C_7, SFSVAPQPGGAGFQY, (SEQIDNO:4946); CCDS33407.1, Del, C_7, SWSCTRWSLSSSVMK, (SEQIDNO:4948); CCDS4317.1, Del, C_7, TRFESPRGTTALPSE, (SEQIDNO:4949); CCDS10081.1, Del, C_7, TSCPPRTGSPCPRPT, (SEQIDNO:4950); CCDS58576.1, Del, C_7, VAQSLCTASTMPTAT, (SEQIDNO:4952); CCDS32555.1, Del, C_7, VLFKADQSESSLSSG, (SEQIDNO:4953); CCDS48130.1, Del, C_7, VQTPRPSQAGSAPWS, (SEQIDNO:4954); CCDS56256.1, Del, C_7, WIGQPPPTATWRRWI, (SEQIDNO:4955); CCDS41618.1, Del, C_7, AMKQIHPLLLPKNS, (SEQIDNO:4956); CCDS 12922.1, Del, C_7, CGDSSTCAQRWKLS, (SEQIDNO:4957); CCDS4457.1, Ins, C_7, DLEHHGGVTRHRHR, (SEQIDNO:4959); CCDS12227.1, Del, C_7, DPMWPPPLSVTMSL, (SEQIDNO:4960); CCDS10013.1, Ins, C_7, GCILPFLACDWLGC, (SEQIDNO:4961); CCDS10098.1, Del, C_7, GDFVLSRSCSLVGS, (SEQIDNO:4962); CCDS46682.1, Del, C_7, GERIPWRSLWSSFE, (SEQIDNO:4963); CCDS13197.1, Del, C_7, GFKMQSRRRKWAGN, (SEQIDNO:4964); CCDS11194.1, Del, C_7, GLASPLAACRLTSP, (SEQIDNO:4965); CCDS53257.1, Del, C_7, GPRPPAQPPPPSGL, (SEQIDNO:4966); CCDS3748.1, Del, C_7, GRVGLKPLSGTRWS, (SEQIDNO:4967); CCDS 1137.1, Ins, C_7, HRGEQRARSKSPPH, (SEQIDNO:4968); CCDS45930.1, Del, C_7, KTLNVSACAPRPFW, (SEQIDNO:4969); CCDS58599.1, Ins, C_7, LLLQERHQPDRRCG, (SEQIDNO:4970); CCDS48064.1, Ins, C_7, LLPLPQLLLLCHLL, (SEQIDNO:4971); CCDS55952.1, Ins, C_7, LVHSWAEEAADEAA, (SEQIDNO:4972); CCDS47266.1, Ins, C_7, NGDCHGCPEGRQSL, (SEQIDNO:4973); CCDS8403.1, Ins, C_7, PAATHGPSQRAEEI, (SEQIDNO:4974); CCDS57999.1, Ins, C_7, PAEPLCLLSFGHLS, (SEQIDNO:4975); CCDS33692.1, Ins, C_7, PAPHHEFPASAQAG, (SEQIDNO:4976); CCDS8083.1, Ins, C_7, PEASWDCRWHSPRP, (SEQIDNO:4977); CCDS47347.1, Ins, C_7, PGSWRAGQRAVSAQ, (SEQIDNO:4978); CCDS8882.1, Del, C_7, PLPKMNTTRMGIWI, (SEQIDNO:4979); CCDS2446.1, Ins, C_7, PLRDPTEGKVLHWK, (SEQIDNO:4980); CCDS33033.1, Del, C_7, QPLPVEGTGPGRGS, (SEQIDNO:4981); CCDS34961.1, Ins, C_7, QPLWTATGLPRTRP, (SEQIDNO:4982); CCDS918.1, Del, C_7, RMLGLALPWELFLI, (SEQIDNO:4983); CCDS13505.1, Ins, C_7, RPPRAAREARPGRH, (SEQIDNO:4984); CCDS54831.1, Del, C_7, RPSPPACAATCPTR, (SEQIDNO:4985); CCDS41266.1, Del, C_7, RRAPPGWACGRGAF, (SEQIDNO:4986); CCDS12921.1, Del, C_7, RTRSSWPPTGPLCP, (SEQIDNO:4987); CCDS9261.1, Del, C_7, SRPPCSWNWRTRTR, (SEQIDNO:4988); CCDS42217.1, Del, C_7, TCWLEVPSGGRLCL, (SEQIDNO:4989); CCDS32041.1, Del, C_7, TLGAALPPWPVEGS, (SEQIDNO:4990); CCDS34455.1, Del, C_7, TPPQPQSVLCATGG, (SEQIDNO:4991); CCDS10804.1, Del, C_7, TTRCTSTATRAPSP, (SEQIDNO:4992); CCDS45403.1, Del, C_7, AAWPAASRVWTMS, (SEQIDNO:4993); CCDS58503.1, Del, C_7, CHPHHPQVPHHPL, (SEQIDNO:4994); CCDS10324.1, Del, C_7, CIQQARWRLSSPM, (SEQIDNO:4995); CCDS42144.1, Ins, C_7, CPGRRPPATAHLL, (SEQIDNO:4996); CCDS8666.1, Del, C_7, GCSHQLPCQTLGF, (SEQIDNO:4997); CCDS1195.1, Del, C_7, GPEAARPQPHPHA, (SEQIDNO:4998); CCDS43537.1, Del, C_7, HPCRASPAASGRQ, (SEQIDNO:4999); CCDS4026.1, Del, C_7, ITFRYLLAVLILF, (SEQIDNO:5000); CCDS44306.1, Ins, C_7, LCQCQDLPKGCPQ, (SEQIDNO:5001); CCDS45696.1, Del, C_7, LETWSTLQPSGQW, (SEQIDNO:5002); CCDS870.1, Ins, C_7, LRLDSRHLHRPGS, (SEQIDNO:5003); CCDS2615.1, Del, C_7, LSSSPTSLCRGRC, (SEQIDNO:5004); CCDS55442.1, Del, C_7, MTRCASLHCPTTP, (SEQIDNO:5005); CCDS5910.1, Del, C_7, PAGWPQAAPRPSA, (SEQIDNO:5007); CCDS42031.1, Ins, C_7, PCSYPEQRPKPPS, (SEQIDNO:5008); CCDS918.1, Ins, C_7, PGCSVWLCHGSSS, (SEQIDNO:5010); CCDS6950.1, Ins, C_7, PGRRPPTRRAASV, (SEQIDNO:5011); CCDS41788.1, Ins, C_7, PKTQTESKASEYS, (SEQIDNO:5012); CCDS1053.1, Ins, C_7, PRFGPYAALHPRV, (SEQIDNO:5013); CCDS44281.1, Ins, C_7, QCGDTCADRGEIH, (SEQIDNO:5014); CCDS46579.1, Ins, C_7, QGVGPYSPRPPDF, (SEQIDNO:5015); CCDS47358.1, Ins, C_7, QHPSYTSRGLPGL, (SEQIDNO:5016); CCDS33407.1, Ins, C_7, RAGPVLAGLCPLL, (SEQIDNO:5017); CCDS6954.1, Del, C_7, RCPSTGRRTTSSS, (SEQIDNO:5018); CCDS9925.1, Ins, C_7, RGQVQQTLCLLND, (SEQIDNO:5019); CCDS9060.1, Ins, C_7, RGRSQDCDQVCDQ, (SEQIDNO:5020); CCDS59018.1, Del, C_7, RKTRAAPRTPLPV, (SEQIDNO:5021); CCDS2369.1, Del, C_7, RMKDPRVLVPPIS, (SEQIDNO:5022); CCDS6982.1, Ins, C_7, RRAWPRGSRWSPW, (SEQIDNO:5023); CCDS48064.1, Del, C_7, SAAATAAAAVSSP, (SEQIDNO:5024); CCDS14680.1, Del, C_7, SIESISFFSMETS, (SEQIDNO:5025); CCDS44477.1, Del, C_7, SKLTSKTKLAKKC, (SEQIDNO:5026); CCDS54003.1, Del, C_7, SLCAESARRPSPQ, (SEQIDNO:5027); CCDS11629.1, Ins, C_7, SLRPANAEPLLLQ, (SEQIDNO:5028); CCDS1172.2, Del, C_7, SLSPGPKRTQIWS, (SEQIDNO:5029); CCDS12929.1, Del, C_7, SQGRSPHWCRCRG, (SEQIDNO:5030); CCDS9510.1, Del, C_7, SQKLPAWPARRRA, (SEQIDNO:5031); CCDS58446.1, Ins, C_7, SQWGPPPSAAAAG, (SEQIDNO:5032); CCDS11524.1, Ins, C_7, SQWRLNQRGHRSD, (SEQIDNO:5033); CCDS8384.1, Ins, C_7, SRHSPPSPRPHPC, (SEQIDNO:5034); CCDS48189.1, Del, C_7, STTRATLSSSARS, (SEQIDNO:5035); CCDS34637.1, Del, C_7, TMGPSGRSRTWFA, (SEQIDNO:5036); CCDS6412.1, Ins, C_7, TPGRWRPGADGQS, (SEQIDNO:5037); CCDS44216.1, Del, C_7, WVPHTETPSAGVV, (SEQIDNO:5038); CCDS12378.1, Del, C_7, APSRPPTTPTPP, (SEQIDNO:5039); CCDS47534.1, Del, C_7, AREGRAATVAES, (SEQIDNO:5040); CCDS53984.1, Del, C_7, EPALSALKAPRR, (SEQIDNO:5041); CCDS725.1, Del, C_7, GCMGSEIWKSCT, (SEQIDNO:5042); CCDS8861.1, Ins, C_7, HGTAAPCLPSAV, (SEQIDNO:5043); CCDS45544.1, Ins, C_7, HTSAPAPRAWSG, (SEQIDNO:5044); CCDS13347.1, Del, C_7, ISVRWMASASVT, (SEQIDNO:5045); CCDS7929.1, Del, C_7, KSCPSSARNNWA, (SEQIDNO:5046); CCDS34063.1, Ins, C_7, LFPGARPDGGNL, (SEQIDNO:5047); CCDS8517.1, Del, C_7, LNHRKGSSVQNP, (SEQIDNO:5048); CCDS32732.1, Del, C_7, PAPATQAPCMMT, (SEQIDNO:5049); CCDS 12366.1, Ins, C_7, PAPIFLLWGPLG, (SEQIDNO:5050); CCDS56581.1, Ins, C_7, PATASCAAQWPD, (SEQIDNO:5051); CCDS34779.1, Del, C_7, PLLVLTPPGGLL, (SEQIDNO:5052); CCDS8403.1, Del, C_7, PPSSHPRPLPAG, (SEQIDNO:5053); CCDS4206.1, Ins, C_7, PRTPLRLPSGVL, (SEQIDNO:5054); CCDS30855.1, Del, C_7, PVLAPIMSRGRA, (SEQIDNO:5056); CCDS2800.1, Ins, C_7, PYTCSLHCHGRA, (SEQIDNO:5057); CCDS11816.1, Ins, C_7, QKLHQKQLQNFL, (SEQIDNO:5058); CCDS53578.1, Del, C_7, QTSLSALKSSGT, (SEQIDNO:5059); CCDS 13938.1, Del, C_7, RAVCPRESLRRP, (SEQIDNO:5060); CCDS747.1, Del, C_7, REHSAARKFYKT, (SEQIDNO:5061); CCDS31596.1, Ins, C_7, RGRRRGPGRGGR, (SEQIDNO:5062); CCDS58101.1, Ins, C_7, RRPEPAPSWLRA, (SEQIDNO:5063); CCDS7331.1, Ins, C_7, SNGSQDPAPYDS, (SEQIDNO:5064); CCDS32883.1, Del, C_7, SPRCPFHMSSCR, (SEQIDNO:5065); CCDS42144.1, Del, C_7, SRETPPSHSPPA, (SEQIDNO:5066); CCDS31013.1, Ins, C_7, SSRHASVQHLPL, (SEQIDNO:5067); CCDS9989.2, Ins, C_7, STSSTTTPARLL, (SEQIDNO:5068); CCDS12601.1, Ins, C_7, TPRSLHSCHRQV, (SEQIDNO:5069); CCDS53685.1, Del, C_7, TSLPLLTTATST, (SEQIDNO:5070); CCDS7512.1, Del, C_7, VPLLEHTPTSPD, (SEQIDNO:5071); CCDS6950.1, Del, C_7, WPASSHAPRSLG, (SEQIDNO:5072); CCDS55444.1, Ins, C_7, WPFSILGSPGPA, (SEQIDNO:5073); CCDS45997.1, Ins, C_7, WTWGCRWARHRL, (SEQIDNO:5074); CCDS44581.1, Ins, C_7, ASAATATAPRG, (SEQIDNO:5075); CCDS42843.1, Ins, C_7, CGGLSPCDWQL, (SEQIDNO:5076); CCDS6916.1, Ins, C_7, CQWDPQSSGAL, (SEQIDNO:5077); CCDS5873.2, Del, C_7, GCTATGRASGW, (SEQIDNO:5078); CCDS42824.1, Del, C_7, GVWTWPSTRTP, (SEQIDNO:5079); CCDS11816.1, Del, C_7, KTAPETAAKFL, (SEQIDNO:5081); CCDS46134.1, Del, C_7, LCLASLSSKTS, (SEQIDNO:5082); CCDS42868.2, Del, C_7, LRGHAQMCRVN, (SEQIDNO:5083); CCDS4956.1, Ins, C_7, LSQLVICFGAE, (SEQIDNO:5084); CCDS9278.1, Ins, C_7, LVPRGASSGLS, (SEQIDNO:5085); CCDS55892.1, Del, C_7, MRRPPPSAPMT, (SEQIDNO:5086); CCDS33928.1, Ins, C_7, PASRRGEKTNL, (SEQIDNO:5087); CCDS33075.1, Del, C_7, PCWSRCSLRQM, (SEQIDNO:5088); CCDS11120.1, Ins, C_7, PEPSKHLLQGM, (SEQIDNO:5089); CCDS14033.1, Ins, C_7, PGEQLWQSVWE, (SEQIDNO:5090); CCDS6811.1, Ins, C_7, PGISHRLPAGL, (SEQIDNO:5091); CCDS31723.1, Ins, C_7, PGQRFCHALCD, (SEQIDNO:5092); CCDS10743.1, Ins, C_7, PHLQSHSISLP, (SEQIDNO:5093); CCDS8896.1, Ins, C_7, PLHQFLWLLEL, (SEQIDNO:5094); CCDS617.2, Del, C_7, PLQPYLLPCLL, (SEQIDNO:5095); CCDS53277.1, Ins, C_7, PPGPRCVQEGA, (SEQIDNO:5096); CCDS12431.1, Del, C_7, PQPPGCCPWPA, (SEQIDNO:5097); CCDS56602.1, Ins, C_7, PQRDPSAGEGK, (SEQIDNO:5098); CCDS12601.1, Del, C_7, PQVPPQLPQAG, (SEQIDNO:5099); CCDS42179.1, Ins, C_7, RGGQRWLLHVL, (SEQIDNO:5100); CCDS12896.1, Del, C_7, RPAAAPTRTSS, (SEQIDNO:5101); CCDS9409.1, Ins, C_7, RRRRGGLQGPR, (SEQIDNO:5102); CCDS1547.1, Del, C_7, SCPQAIPRSPC, (SEQIDNO:5103); CCDS13442.1, Del, C_7, SPRGVRARRAQ, (SEQIDNO:5104); CCDS10693.1, Del, C_7, SSASAQMPLTP, (SEQIDNO:5105); CCDS14053.1, Del, C_7, SSEGSATSMGL, (SEQIDNO:5106); CCDS32742.1, Del, C_7, TSATSPNAVAA, (SEQIDNO:5107); CCDS5794.1, Del, C_7, TSSLTMESSGH, (SEQIDNO:5108); CCDS55791.1, Ins, C_7, VFCLFLVKHFC, (SEQIDNO:5109); CCDS2407.1, Del, C_7, VWPRHPCLLWA, (SEQIDNO:5110); CCDS8756.2, Del, C_7, WGILSTWLLSG, (SEQIDNO:5111); CCDS45778.2, Del, C_7, AQWAPAASPA, (SEQIDNO:5112); CCDS41837.1, Ins, C_7, CQSAECQPHC, (SEQIDNO:5113); CCDS8023.2, Del, C_7, DCHPRAPLPW, (SEQIDNO:5114); CCDS55462.1, Ins, C_7, GLRDRLGAGV, (SEQIDNO:5117); CCDS12700.1, Del, C_7, GPVMAWTSRM, (SEQIDNO:5118); CCDS54474.1, Ins, C_7, HEAGNGCQAL, (SEQIDNO:5119); CCDS8452.1, Ins, C_7, HSGGGHTETD, (SEQIDNO:5120); CCDS13911.1, Del, C_7, IKASSMPQTP, (SEQIDNO:5121); CCDS8548.1, Ins, C_7, LPAASGTQVV, (SEQIDNO:5123); CCDS8521.2, Ins, C_7, LPAPPARRPC, (SEQIDNO:5124); CCDS32732.1, Ins, C_7, LQPQPRRLPA, (SEQIDNO:5125); CCDS11253.1, Del, C_7, NIHLSSRNQK, (SEQIDNO:5126); CCDS346.2, Ins, C_7, PARRQGLRGS, (SEQIDNO:5127); CCDS13734.1, Ins, C_7, PASCYIRGAE, (SEQIDNO:5128); CCDS516.1, Ins, C_7, PAWCLHPSSP, (SEQIDNO:5129); CCDS54603.1, Del, C_7, PGALLNPSRG, (SEQIDNO:5131); CCDS6419.1, Del, C_7, PPPPLKSASA, (SEQIDNO:5132); CCDS9189.1, Del, C_7, PSLGSPGKCV, (SEQIDNO:5134); CCDS4112.1, Ins, C_7, PTASRDAGPV, (SEQIDNO:5135); CCDS7607.1, Del, C_7, PWRSCPAPAT, (SEQIDNO:5136); CCDS12581.1, Ins, C_7, QAAAAVFISG, (SEQIDNO:5137); CCDS13948.1, Ins, C_7, QCRLHPPPAA, (SEQIDNO:5138); CCDS3080.1, Ins, C_7, QDGCQDVPGL, (SEQIDNO:5139); CCDS4668.1, Ins, C_7, QDTRDPPPCL, (SEQIDNO:5140); CCDS34373.1, Ins, C_7, QDTYDPPPHL, (SEQIDNO:5141); CCDS14091.2, Ins, C_7, QLGAPGPAAP, (SEQIDNO:5142); CCDS 10206.1, Del, C_7, QSPTGRSWSI, (SEQIDNO:5143); CCDS11120.1, Del, C_7, RALQTSTTRY, (SEQIDNO:5144); CCDS53981.1, Del, C_7, RLRSRPTTGT, (SEQIDNO:5145); CCDS9925.1, Del, C_7, RSSSTNPLSS, (SEQIDNO:5146); CCDS12227.1, Ins, C_7, RTQCGHHHCQ, (SEQIDNO:5147); CCDS1075.1, Del, C_7, RVLATHPQAT, (SEQIDNO:5149); CCDS 10457.1, Del, C_7, RYAGTRCCCH, (SEQIDNO:5150); CCDS53838.1, Del, C_7, SHPHPHLPHL, (SEQIDNO:5151); CCDS8403.1, Del, C_7, SSHPRPLPAG, (SEQIDNO:5152); CCDS11157.1, Del, C_7, SSTGSKTWPC, (SEQIDNO:5153); CCDS47684.1, Del, C_7, TIKITSTFIS, (SEQIDNO:5154); CCDS34129.1, Del, C_7, TPRTGGPRCW, (SEQIDNO:5155); CCDS2670.2, Del, C_7, TSSPSSRASW, (SEQIDNO:5156); CCDS56415.1, Ins, C_7, WYSGAWGLCS, (SEQIDNO:5157); CCDS54466.1, Del, C_7, CPSCMPSGT, (SEQIDNO:5158); CCDS45997.1, Del, C_7, DLGLPLGQA, (SEQIDNO:5160); CCDS7801.1, Ins, C_7, EWKCSPLSL, (SEQIDNO:5161); CCDS32872.1, Ins, C_7, GLRRRERHI, (SEQIDNO:5162); CCDS12684.1, Del, C_7, GTNPRLMEL, (SEQIDNO:5163); CCDS31007.1, Del, C_7, HVKRHAVNL, (SEQIDNO:5164); CCDS1943.1, Del, C_7, IRRVSPGQG, (SEQIDNO:5166); CCDS13305.1, Del, C_7, ITGGTGLLL, (SEQIDNO:5167); CCDS31683.1, Del, C_7, KVRWPWVPH, (SEQIDNO:5168); CCDS31882.1, Del, C_7, LEHLNHSHL, (SEQIDNO:5169); CCDS46221.1, Ins, C_7, LLLCHDPPQ, (SEQIDNO:5170); CCDS54370.1, Del, C_7, NATSACQDP, (SEQIDNO:5171); CCDS12780.1, Del, C_7, NRGAQPLNN, (SEQIDNO:5173); CCDS4112.1, Del, C_7, NSLQRRGPS, (SEQIDNO:5174); CCDS8097.1, Ins, C_7, PACHFSQLH, (SEQIDNO:5175); CCDS12190.1, Del, C_7, PPRRPSTAN, (SEQIDNO:5176); CCDS55403.1, Ins, C_7, PPTESDFIA, (SEQIDNO:5177); CCDS47534.1, Ins, C_7, PQEREERPQ, (SEQIDNO:5178); CCDS55220.1, Del, C_7, PSHQATRLW, (SEQIDNO:5179); CCDS5187.1, Ins, C_7, QAALPGPRL, (SEQIDNO:5180); CCDS54914.1, Ins, C_7, QDGPHGLQP, (SEQIDNO:5181); CCDS3541.1, Ins, C_7, QIPKSSPAT, (SEQIDNO:5182); CCDS41295.1, Del, C_7, QMPSPKARR, (SEQIDNO:5183); CCDS7331.1, Del, C_7, QWLPRPRPL, (SEQIDNO:5184); CCDS59376.1, Ins, C_7, QWPCGAGHE, (SEQIDNO:5185); CCDS13938.1, Ins, C_7, REPCVPGKV, (SEQIDNO:5186); CCDS11316.1, Ins, C_7, RNERGGRVV, (SEQIDNO:5187); CCDS8002.1, Del, C_7, RRIAVLVFQ, (SEQIDNO:5188); CCDS31723.1, Del, C_7, RSEVLSCPL, (SEQIDNO:5189); CCDS59482.1, Ins, C_7, RSGPARLPV, (SEQIDNO:5190); CCDS7628.1, Ins, C_7, SEGDATGAR, (SEQIDNO:5191); CCDS45001.1, Del, C_7, SSRCWAGAW, (SEQIDNO:5192); CCDS1912.1, Del, C_7, STCSLPCRG, (SEQIDNO:5193); CCDS2632.1, Del, C_7, TIIKGKRRT, (SEQIDNO:5194); CCDS7739.1, Del, C_7, TPRLQDLGH, (SEQIDNO:5195); CCDS10693.1, Ins, C_7, TQAHLPKCP, (SEQIDNO:5196); CCDS4956.1, Del, C_7, VATGHLLRS, (SEQIDNO:5198); CCDS992.1, Del, C_7, VQPNGLGGN, (SEQIDNO:5199); CCDS8896.1, Del, C_7, APPISLAS, (SEQIDNO:5200); CCDS59420.1, Ins, C_7, CGKPLHLH, (SEQIDNO:5201); CCDS5863.1, Ins, C_7, CLITWLTN, (SEQIDNO:5202); CCDS8221.1, Del, C_7, EALTGQGM, (SEQIDNO:5203); CCDS42139.1, Ins, C_7, EARGNGPG, (SEQIDNO:5204); CCDS42292.1, Del, C_7, FADKPRPN, (SEQIDNO:5205); CCDS11841.1, Del, C_7, FYLSRVYP, (SEQIDNO:5206); CCDS34437.1, Ins, C_7, GAPYSETH, (SEQIDNO:5207); CCDS13734.1, Del, C_7, GFLLHQRR, (SEQIDNO:5208); CCDS1687.1, Ins, C_7, GGGHLEEV, (SEQIDNO:5209); CCDS7771.1, Del, C_7, GGPRPCWT, (SEQIDNO:5210); CCDS13418.1, Del, C_7, GLLRNTQQ, (SEQIDNO:5211); CCDS1753.1, Ins, C_7, HHPWEPDL, (SEQIDNO:5212); CCDS1151.1, Del, C_7, HLRHPLLL, (SEQIDNO:5213); CCDS46055.1, Ins, C_7, KDPCPETG, (SEQIDNO:5214); CCDS10013.1, Del, C_7, LHPPFPCL, (SEQIDNO:5215); CCDS858.1, Del, C_7, MEAGAGQW, (SEQIDNO:5216); CCDS45820.1, Del, C_7, MEKDFQRW, (SEQIDNO:5217); CCDS4913.1, Del, C_7, PAACSPAK, (SEQIDNO:5218); CCDS34327.1, Ins, C_7, PCDRHLSK, (SEQIDNO:5219); CCDS46732.1, Ins, C_7, PDPALQPG, (SEQIDNO:5220); CCDS14081.1, Ins, C_7, PDRRPSAH, (SEQIDNO:5221); CCDS12173.1, Ins, C_7, PDTLLRTA, (SEQIDNO:5222); CCDS9756.1, Ins, C_7, PDTMSGYF, (SEQIDNO:5223); CCDS1138.1, Ins, C_7, PGHPPRPH, (SEQIDNO:5224); CCDS41687.1, Ins, C_7, PPAGEPAH, (SEQIDNO:5225); CCDS32872.1, Del, C_7, PPTARATH, (SEQIDNO:5226); CCDS33428.1, Del, C_7, PSPQPCSW, (SEQIDNO:5227); CCDS1263.1, Ins, C_7, QGRPGPEW, (SEQIDNO:5228); CCDS32760.1, Ins, C_7, QQPLRLRP, (SEQIDNO:5229); CCDS7728.1, Ins, C_7, RDGQQSEV, (SEQIDNO:5231); CCDS10554.1, Del, C_7, RISGNIRL, (SEQIDNO:5232); CCDS33469.1, Del, C_7, RPASQQVS, (SEQIDNO:5233); CCDS42972.1, Ins, C_7, RTTRAVSV, (SEQIDNO:5234); CCDS46119.1, Ins, C_7, SGAHRGEA, (SEQIDNO:5235); CCDS14003.1, Ins, C_7, STQPLHYQ, (SEQIDNO:5237); CCDS33378.1, Ins, C_7, TGDPKLPW, (SEQIDNO:5238); CCDS10976.1, Del, C_7, TGPPSTSS, (SEQIDNO:5239); CCDS276.1, Ins, C_7, TPRGRLLQ, (SEQIDNO:5240); CCDS44492.1, Ins, C_7, TPRRGASV, (SEQIDNO:5241); CCDS56182.1, Ins, C_7, TQAPATRY, (SEQIDNO:5242); CCDS7801.1, Del, C_7, VEVLPTFF, (SEQIDNO:5243); CCDS44753.1, Ins, C_7, WIFEATSP, (SEQIDNO:5244); CCDS43896.1, Del, C_8, CRPRVTPGPPPCRPRVTPGPPPCRPRVTPGPPPCRPRVTPGPPPCRPRVTPGPPPCRPRVTPAPPP CRPRVTPGPPPCRPRVTPAPPPCRPRVTPGPPP, (SEQIDNO:5245); CCDS54609.1, Del, C_8, QSSPFLARSWNTMQWNNKKMAMTVIAGAHHCQYKLTYTKVWKMNWKKMMIGSQHLLFE AWLLLLLSPLDSSPLQLLLHPHGKRCNPCCRLTWMS, (SEQIDNO:5248); CCDS43896.1, Del, C_8, CRPRVTPGPPPCRPRVTPGPPPCRPRVTPGPPPCRPRVTPGPPPCRPRVTPAPPPCRPRVTPGPPP CRPRVTPAPPPCRPRVTPGPPP, (SEQIDNO:5249); CCDS10689.2, Del, C_8, HHLVPLFIWTPWRKSGSGSGLNAWNGFSNLVRLMGPWHLCMGLKSWISVPCPNLLPAPSAL VLLAPATPPFGLIPRLPTGLYCFPSSD, (SEQIDNO:5250); CCDS46136.1, Ins, C_8, RGGHGPVNASSAPDTSPPRHPRRWVSQQRQRLWRQFRVGGGFPPPPPSRPPAVLLPLLRLAC AGDPGATRPGPRRPARRPRGEL, (SEQIDNO:5251); CCDS46136.1, Del, C_8, RGTRTRQRLQRPRHLPTPPPPEMGFPATTAALEAVSSGRRISPSSPLQAPRCASPASPSRLRWR PRGHPPGPPTPGPQAPWRI, (SEQIDNO:5253); CCDS33928.1, Del, C_8, RPPMTTWPRMRMTLRSAPSTAPSWARGSLPGPARARPTTPLCLSRRGRPLPWRTTCSSRPRV GASRPAPHRPQRSSRRYSRSA, (SEQIDNO:5254); CCDS47878.2, Ins, C_8, PTSSTFCSSTGPTAGFSGPAALSFHYDATCATPCASSPACPAAATDGRTLCRPHPTLPAAARIR SQLLKTFSVQTPTDKNYR, (SEQIDNO:5255); CCDS55835.1, Ins, C_8, PWTPWPLTVPQPTNSSLNDARGSARQRAPRRGGPKPCHCGSCSGQPPAQCQPTARPRHPPAS RPHSPEPRHGHPCATSTV, (SEQIDNO:5256); CCDS43896.1, Del, C_8, CRPRVTPGPPPCRPRVTPGPPPCRPRVTPGPPPCRPRVTPAPPPCRPRVTPGPPPCRPRVTPAPPP CRPRVTPGPPP, (SEQIDNO:5257); CCDS7529.1, Ins, C_8, PTNGAPSVWYSWCLCRASHLQCALGTPSCPSLTLQFHMYLWALGMGPRASTCSILVYCSPTS FASSLHWESCSPT, (SEQIDNO:5258); CCDS41693.1, Ins, C_8, HQPVHRRPGLQGSWWAEQSGGKDREGGRREGGENGEGRSGGGGGKHQRGRREGERGGGG RGRREGGEGDRKGG, (SEQIDNO:5259); CCDS44622.1, Ins, C_8, QHPQCPDAEWNDQTGAWDRDSPASAPHSSPIATLASPGSALPGPWFLASQHCPCPDTALAPA RCPLPRPPAGQ, (SEQIDNO:5260); CCDS45775.1, Del, C_8, RRGTSQRAQSGPLSFVLHFLAKDPHPMFLCTPRAHPLARPQGHLLAHPGPSPTWPGPLPLPQT RLDLSAGPTA, (SEQIDNO:5261); CCDS43374.1, Ins, C_8, PTSSLAWRSRNATSSSPSSWWSWNPSTSSISRRPWHSSTSTRNGYASTSPIWIWSSCSPSSAIWI NPQKAL, (SEQIDNO:5262); CCDS33051.1, Ins, C_8, PTPRFAPFGGPSCSARSRGRTTPCTPSPGSTGPWWWGSWGPRPGRSYCWSQTQESQQAGGGL RGAHSPQS, (SEQIDNO:5263); CCDS 11528.1, Ins, C_8, PTPISGRHAPGGRLPALSLLAAVPGQRCPTCQEQGAQRQLHEAGSGPRAPVGPAWLTPAQCR TYQCH, (SEQIDNO:5264); CCDS43896.1, Del, C_8, CRPRVTPGPPPCRPRVTPGPPPCRPRVTPAPPPCRPRVTPGPPPCRPRVTPAPPPCRPRVTPGPPP , (SEQIDNO:5265); CCDS470.1, Ins, C_8, RRPRPQRRRHQARHYGQRRRERWPGRPHIGGACRRGQEGHRNEGFGNSKMVQCKERIWFH QQE, (SEQIDNO:5267); CCDS46143.1, Del, C_8, HPVRPPPPNVRAWVGQQPPMPLGRPLGLEGQGARLIPPVSLKGFWDPRPTGKGLEGGRHRT, (SEQIDNO:5268); CCDS56415.1, Del, C_8, QLPMQRHLPLVLGRPHPWLRLLPMSSPQLRGLPRQVQLPRQPPATRGSSGFPTRVWNPWS, (SEQIDNO:5269); CCDS12027.1, Del, C_8, PAPRRILLRYQARASTTARTQTPTASACLPSPCWGGPGPRDPWRRPPALAPTAQSRSP, (SEQIDNO:5271); CCDS8908.1, Ins, C_8, PWTPWPLTVPQPTNSSLNDARGSARQRAPRRGGPRHPPASRPHSPEPRHGHPCATSTV, (SEQIDNO:5272); CCDS33016.1, Del, C_8, HLTTKGEGWVHQWGVTVGAQVATAPSMGTPHPLPHHPSMALTPTALCSWSMAWINLR, (SEQIDNO:5273); CCDS30551.1, Ins, C_8, RRPPPGSQQYRTQPDHPSALITTSDHCQRPQDHRVARADGAPHGTLPCTQPGGVRLW, (SEQIDNO:5274); CCDS46158.1, Del, C_8, LRPGPGSGFSPPPPWPAWPSSAEGCSPRAWSSTLLPTTTQCVKVTTPPSAASSTST, (SEQIDNO:5275); CCDS33016.1, Ins, C_8, PTSLRREKDGSTSGGSPSGPKSLRPPVWAPPTPSPTTRVWPSRRQPCAHGLWLGSI, (SEQIDNO:5276); CCDS53986.1, Ins, C_8, PVPPLQCLLSCLGPSPDDASSTTFPATSAAAATADSIPKGPPAGPRVHQALKSSTH, (SEQIDNO:5277); CCDS43896.1, Del, C_8, CRPRVTPGPPPCRPRVTPAPPPCRPRVTPGPPPCRPRVTPAPPPCRPRVTPGPPP, (SEQIDNO:5278); CCDS12921.1, Ins, C_8, PEACGFSHAEPSREAATRAEVHGSPEHHRCRGWWLPCTHPTGLGDGPAQPEIP, (SEQIDNO:5279); CCDS6980.1, Ins, C_8, PGGLQPQQARPQDQPAAVHAECGGEDALETPVRLALLPARGRNQIEPAGLS, (SEQIDNO:5280); CCDS9209.1, Del, C_8, QGQARDLRCPLTAPLACLHLPSPPVRSTVCAMDSLRPVRLQKYPQAAAVP, (SEQIDNO:5281); CCDS3368.1, Del, C_8, GRSLPAGPSALPSPPSPSRRRAPPRSGRGSLRKWRPGASRRWRMSTWPS, (SEQIDNO:5282); CCDS42139.1, Del, C_8, GAPRSPSSVAGSSSATWQSGAMWVQPPWEQLPGGSCTLRMGLMSTTAS, (SEQIDNO:5283); CCDS5254.1, Ins, C_8, PSNRFNGEKVGFRCVHLLRHPWTVFNFADGKTSTRSTSATSQGSDWNK, (SEQIDNO:5284); CCDS12057.2, Del, C_8, LGGPGSALQAPWTRARLPPAARPLPEADSLGPPLQHGPEEGSRERLG, (SEQIDNO:5285); CCDS 12310.1, Del, C_8, LPQPCGPASCSTPSWPMAVPLAASRASPTSRSCMARSPRPSACQLPR, (SEQIDNO:5286); CCDS470.1, Del, C_8, PPPPSAPPTPSPALRAAAQGAVARAASHRRRLPAGTRRSSQRRFWEQ, (SEQIDNO:5287); CCDS1157.1, Del, C_8, RKLPQMPPSSMTKHLSGCRARGTEGEKKSTLWRSKVMTSSVGPSNG, (SEQIDNO:5289); CCDS2232.1, Del, C_8, HAGACTEEIAILMRLTSPNASVLAATPENIVKWRFQKASLQEQPQ, (SEQIDNO:5290); CCDS43896.1, Del, C_8, CRPRVTPAPPPCRPRVTPGPPPCRPRVTPAPPPCRPRVTPGPPP, (SEQIDNO:5291); CCDS3368.1, Ins, C_8, RAEVSQRALLHSPVPRLPRAGGHRPDLEEAVSGSGGRGHPDGGG, (SEQIDNO:5292); CCDS 14732.1, Del, C_8, QSAMSIMTRRSLWRAARRRQTAWMSWSMRRWSCIKAATGTSWA, (SEQIDNO:5293); CCDS588.2, Ins, C_8, HEWSSPAPSPLATPLPTLGTPKTGRYGSRLFPLLQSLLPQLL, (SEQIDNO:5294); CCDS45847.1, Del, C_8, HHLRYLHLSLMQKSHLIKNNLQLPWKPLRDLVGHSPRAYVAI, (SEQIDNO:5295); CCDS 10834.1, Del, C_8, LPHFHLPHPSKAPSHHLHLYLWLPLFPIQCLTAQPSPLRGRQ, (SEQIDNO:5296); CCDS46158.1, Ins, C_8, PCARGPAPASRRRRPGRLGRHQPRAALPEPGVQLSCRQLHSV, (SEQIDNO:5297); CCDS8403.1, Ins, C_8, PTTTAGLRARDQQQPAQPDAPELSRCPEPYGHEPARPAAPVP, (SEQIDNO:5298); CCDS11673.1, Ins, C_8, QHQRPGPGGARRRGRQDGGRGRRRPPGPDHRGPEGRQQSGVR, (SEQIDNO:5299); CCDS33508.1, Ins, C_8, PWGEAAGGSLHLVLRPLQQVGGCPGRTAPEKDGAEAPPCVQ, (SEQIDNO:5300); CCDS1701.1, Del, C_8, ARSASCCAQWQARCRARWHCGGWASAGRAEGALRQRSARC, (SEQIDNO:5301); CCDS9955.1, Del, C_8, HPQSHLHPLGLPHLPHPHCQPEEPRGPATTRAPCQDWPLP, (SEQIDNO:5302); CCDS11528.1, Del, C_8, NPHFRPPRTWRATTSAQLARCSPWATLPHMPRARSSTAAA, (SEQIDNO:5303); CCDS 14359.1, Ins, C_8, PTGAHPPSTITPTACRPPSGQGPGSQGRGQPQFCSSILTD, (SEQIDNO:5304); CCDS32502.1, Del, C_8, RRPATPPRPSRRGPEAEAEAAPASPLRARPAPRPTAPST, (SEQIDNO:5305); CCDS35096.1, Del, C_8, HNPRHQTSVLSFTTANTVPTAIGQLWECLSTTRKDTQK, (SEQIDNO:5306); CCDS42139.1, Ins, C_8, PEPQGAPHQWLALLPLHGNRGLCGCSHRGSSCLVVPVR, (SEQIDNO:5308); CCDS8038.2, Ins, C_8, PSDPRPSPSRHHVLPGSDPGVSVCLQVAADPSEWNPDK, (SEQIDNO:5309); CCDS32579.1, Del, C_8, TCSPQSLLSSWRSTQPHVPPSTLWGVRMTSLSASTSLW, (SEQIDNO:5310); CCDS30551.1, Del, C_8, APSPWLPAVPHTARPPLRPHHDLGPLPASPGPPCGPC, (SEQIDNO:5311); CCDS58532.1, Del, C_8, HPAARSSSCCPASPHSPGASPMSLSWGTALMTSPR, (SEQIDNO:5312); CCDS 1402.1, Ins, C_8, PSASPGHDFQEGAGGVSRGLPLAEDLLGVLAVFG, (SEQIDNO:5313); CCDS43896.1, Del, C_8, CRPRVTPGPPPCRPRVTPAPPPCRPRVTPGPPP, (SEQIDNO:5314); CCDS42144.1, Ins, C_8, LPRAGPGLPGGGRPHELLPATSGSRPEPPAGRG, (SEQIDNO:5315); CCDS13888.1, Del, C_8, VAEEAAASRWKQSQQSLSLQQWKRPMGLSRPE, (SEQIDNO:5318); CCDS33055.1, Ins, C_8, PSPAPSARLPPTQAEKRFPYTEKARPMEISV, (SEQIDNO:5319); CCDS 10834.1, Ins, C_8, PSPTSTSPTHQRPLPTTSTSTSGCPSSPFSA, (SEQIDNO:5320); CCDS45604.1, Del, C_8, QQVPGAAAPAAAALMKTGPSWMEAWNQMGPT, (SEQIDNO:5321); CCDS1731.1, Del, C_8, AFSGRILHQPEMAAMRLMPKFLNSTNSWWT, (SEQIDNO:5322); CCDS53838.1, Del, C_8, HHHHPHRSLASPCPHRCPHRRPHPLQPTLL, (SEQIDNO:5323); CCDS33055.1, Del, C_8, QPRSLRTTPPHTSGEKVSLHGKGAADGNLR, (SEQIDNO:5324); CCDS9318.1, Ins, C_8, PASPSGPRGVPGLCTDGSSRLRDAPSADN, (SEQIDNO:5325); CCDS56415.1, Ins, C_8, PNSQCRGTSPWSWAGLIRGSVFYQCRVLS, (SEQIDNO:5326); CCDS53838.1, Ins, C_8, PPPPPAAPAAARPFGREEEPPLEKLQVDD, (SEQIDNO:5327); CCDS33508.1, Del, C_8, VGRSGWGVPASCTAAPPTSRWMSWQDCS, (SEQIDNO:5328); CCDS33074.1, Del, C_8, PPPGWLPRLTRERAAAALRAVGTQISI, (SEQIDNO:5329); CCDS1756.1, Ins, C_8, QRTPWGHGFAGAPFPISKPHGPPAGQL, (SEQIDNO:5330); CCDS9318.1, Del, C_8, RLPLRPQRGPRPLHRWLQQTTGCSQRR, (SEQIDNO:5331); CCDS58489.1, Del, C_8, SLFLPGSTPSLHFLCWSLRSSGGCRSA, (SEQIDNO:5332); CCDS32579.1, Ins, C_8, PRALLRVCCHRGGPRSRTFHPPPCGV, (SEQIDNO:5333); CCDS53838.1, Del, C_8, TTTTSSPRRSPALRARGGTTIGEVTS, (SEQIDNO:5334); CCDS47878.2, Del, C_8, HLLHLLLLHRSNCRFLWTCRSFLPL, (SEQIDNO:5335); CCDS11137.1, Del, C_8, LTTGHLLAPKICHLHPGAWVTLNSW, (SEQIDNO:5336); CCDS9209.1, Ins, C_8, PRARPGTCAARSQLPWPASTCPLPQ, (SEQIDNO:5337); CCDS11936.1, Ins, C_8, PSLLQIPDGFSQTNCRLSRCCAFLR, (SEQIDNO:5338); CCDS45465.1, Del, C_8, RRTCLGPPNERKGRQGMSLPPQGKR, (SEQIDNO:5339); CCDS33940.1, Ins, C_8, PRAREAEGEEAAAEGLAESHRGQI, (SEQIDNO:5340); CCDS41575.1, Ins, C_8, PTRSQGVPVLHQEPAQRRPPPAAP, (SEQIDNO:5341); CCDS12035.1, Ins, C_8, PGPRARAPPAPAPGRGVAPGGGG, (SEQIDNO:5342); CCDS33940.1, Del, C_8, QSQGSRRGRGGGGGAGGESQRAN, (SEQIDNO:5343); CCDS6980.1, Del, C_8, RRSPTPASPAARPTSCSTCRMWW, (SEQIDNO:5344); CCDS41693.1, Del, C_8, PACTSKAWTPRKLVGRAVRRKG, (SEQIDNO:5345); CCDS4328.1, Del, C_8, PQPGNQRSNRSLPGTTRMRHRV, (SEQIDNO:5346); CCDS54609.1, Ins, C_8, PSPAPSWHGAGTLCSGTTRKWL, (SEQIDNO:5347); CCDS58532.1, Ins, C_8, PTQPQGLPAAAQLPHTHPEQVP, (SEQIDNO:5348); CCDS10689.2, Ins, C_8, PTTSFPFLSGLPGGKAEAAAV, (SEQIDNO:5349); CCDS8403.1, Del, C_8, HHHRRAPGQGSATASPARCT, (SEQIDNO:5350); CCDS2702.1, Del, C_8, LQDGSLDRNLGSPLMSEFRK, (SEQIDNO:5351); CCDS34708.1, Ins, C_8, PGWHLPRQGQHAEPRPRPW, (SEQIDNO:5352); CCDS 13202.1, Del, C_8, TALGATIPSRPRSAAALRT, (SEQIDNO:5353); CCDS2359.1, Del, C_8, HLLLSLHPSLPKLPQNPL, (SEQIDNO:5354); CCDS4328.1, Ins, C_8, HPSLATRDQTGACLARPG, (SEQIDNO:5355); CCDS14239.1, Del, C_8, HQMKCLTAVDCLQAFWDM, (SEQIDNO:5356); CCDS59406.1, Ins, C_8, PCLLPGQVLGGPELGPKW, (SEQIDNO:5357); CCDS11107.1, Ins, C_8, PCRGDGPVPCPRRPAPAQ, (SEQIDNO:5358); CCDS58489.1, Ins, C_8, PVSSFQDRLPPCISSAGA, (SEQIDNO:5359); CCDS8908.1, Del, C_8, LDPMAPHRSPTNKLLPQ, (SEQIDNO:5360); CCDS13202.1, Ins, C_8, LQLWELRFHQDRGQRLP, (SEQIDNO:5361); CCDS45465.1, Ins, C_8, PGGRAWAPQTKGKGGRE, (SEQIDNO:5362); CCDS1756.1, Del, C_8, ADSVGTWLRWCPFPHI, (SEQIDNO:5363); CCDS11816.1, Ins, C_8, PEPPQKLHQKQLQNFL, (SEQIDNO:5364); CCDS45539.1, Ins, C_8, RGDHRSNQNREWCLEE, (SEQIDNO:5365); CCDS44440.1, Del, C_8, TLMMSNGCWGWQQSWE, (SEQIDNO:5366); CCDS53986.1, Del, C_8, CAPAPVPAQLPRALT, (SEQIDNO:5367); CCDS33637.1, Del, C_8, GPQERARAARMMRAQ, (SEQIDNO:5368); CCDS1731.1, Ins, C_8, LHSPEESSISPKWRP, (SEQIDNO:5369); CCDS2702.1, Ins, C_8, PFKMEAWTETLEALL, (SEQIDNO:5370); CCDS11816.1, Del, C_8, RAPPKTAPETAAKFL, (SEQIDNO:5371); CCDS1943.1, Ins, C_8, PLYCGPRPPLDCFQ, (SEQIDNO:5372); CCDS45604.1, Ins, C_8, PSKCQAPPLPPPPH, (SEQIDNO:5373); CCDS59317.1, Ins, C_8, PSLLQIPDGFSQTN, (SEQIDNO:5374); CCDS42144.1, Del, C_8, SPCWAWTPGGWAAS, (SEQIDNO:5375); CCDS1153.1, Del, C_8, STGRLTARRMPPCL, (SEQIDNO:5376); CCDS46137.1, Del, C_8, HTSWNFRPLNWRS, (SEQIDNO:5377); CCDS45775.1, Ins, C_8, PGEEPPRGHRAAP, (SEQIDNO:5378); CCDS10967.1, Ins, C_8, QEADAKRQRQWQW, (SEQIDNO:5379); CCDS2232.1, Ins, C_8, PMQVHARRKLLF, (SEQIDNO:5380); CCDS58106.1, Ins, C_8, PTSARTKLGCWA, (SEQIDNO:5381); CCDS43896.1, Del, C_8, CRPRVTPGPPP, (SEQIDNO:5382); CCDS59406.1, Del, C_8, MPSSWSSSGRT, (SEQIDNO:5383); CCDS1157.1, Ins, C_8, PGNCPRCLLHR, (SEQIDNO:5384); CCDS33637.1, Ins, C_8, RGRRRGPGLRG, (SEQIDNO:5385); CCDS10413.1, Del, C_8, RTARGVTARSP, (SEQIDNO:5386); CCDS4827.1, Ins, C_8, PAVSSPYSCV, (SEQIDNO:5387); CCDS35096.1, Ins, C_8, PTTPATRPQY, (SEQIDNO:5388); CCDS45847.1, Ins, C_8, PTTSATSTSP, (SEQIDNO:5389); CCDS32879.1, Ins, C_8, PVPPLHPPLL, (SEQIDNO:5390); CCDS33074.1, Ins, C_8, HPHRAGCHV, (SEQIDNO:5391); CCDS46134.1, Del, C_8, HRLLGPSRW, (SEQIDNO:5392); CCDS1943.1, Del, C_8, LILWPQAAP, (SEQIDNO:5393); CCDS8932.1, Ins, C_8, PRCSPAWNL, (SEQIDNO:5394); CCDS13213.2, Ins, C_8, PSIYQRQKT, (SEQIDNO:5395); CCDS46137.1, Ins, C_8, PTQVGTSDP, (SEQIDNO:5396); CCDS32879.1, Del, C_8, RTPTSPAAT, (SEQIDNO:5397); CCDS12921.1, Del, C_8, GSVWILPC, (SEQIDNO:5398); CCDS54073.1, Del, C_8, LHLCQPLR, (SEQIDNO:5399); CCDS11423.1, Ins, C_8, PGEGHHHQ, (SEQIDNO:5400); CCDS12027.1, Ins, C_8, RPPPAGSC, (SEQIDNO:5401); CCDS9239.1, Ins, C_9, PTTPGACGTLSHPVYCGHEFGTGRAVPRITVPAPAPPRGPPHSARILFAVRVPISATSPSETPAP APSTPAGFHPPV, (SEQIDNO:5402); CCDS10383.1, Ins, C_9, PWAWRHTLPGCQCRTCGLREPALPQGSAQLPGPAVPGTRPAEPQEERPADSRGG, (SEQIDNO:5403); CCDS45938.1, Del, C_9, LPGGIASRFPASWPRTPAACHPSLRTGT, (SEQIDNO:5404); CCDS12767.1, Del, C_9, PAVFASSRPTSPASCRRPPSSWWR, (SEQIDNO:5405); CCDS43896.1, Del, C_9, PCRPRVTPAPPPCRPRVTPGPPP, (SEQIDNO:5406); CCDS13085.1, Ins, C_9, PHPRNKQGNFRGRPLLCS, (SEQIDNO:5407); CCDS10383.1, Del, C_9, LGLEAHTARVPV, (SEQIDNO:5409); CCDS58446.1, Del, C_9, ECCSSWLRRIE, (SEQIDNO:5410); CCDS9239.1, Del, C_9, HHPRCLRDA, (SEQIDNO:5411); CCDS2221.2, Ins, G_7, GTASAFSVPAFRNSCSHPPTPHHCSWLHLPGPWFDTYCTVGSDKQFSKFSQKESFLHGRARR YGLQLWKLQLWPTEIYTESYVQNARQQHFKHKSGEWL, (SEQIDNO:5414); CCDS8078.1, Ins, G_7, VPAPPPGPGWAPAAASTAAAATSAAAVAAVAAVAAGGGRGGPGLLQPQHHPPRPPLPQQAR HRAHRHQPHALPHPRRPRRDHIHLWEGGSAHHLHVAPQ, (SEQIDNO:5415); CCDS7694.1, Del, G_7, VRRRRRPAQTCCGQIPGIPFSSLHAWNLRAWRTCWSPAPTPPPTWSRTSRSPDTRACNLCTCP SFIPTTTLASPTWRRTTSASTASLRCIWRGLGSINT, (SEQIDNO:5416); CCDS58996.1, Ins, G_7, AAAAGGPAAAGGAAELQEETGRGTPGGEAAVAPQEDERGGWPRRRPWQEGPGRERSAERT REEGSAGAQSGEESAEAGHGREEHARQTSTWQGGQRLL, (SEQIDNO:5417); CCDS8891.1, Ins, G_7, GALQPASPVRGARAGRLPRVRLAAAQPRAAPREHQVADLEQTAAQGGAQHQPRGGEAAAA QGAAAAADPGPTRLPGRRAAARGLPGGGRVPRHHRDRH, (SEQIDNO:5418); CCDS10624.2, Del, G_7, GKMILLLQEWSRAISGGIAKRRRLHGMTRKRINRDGVMDKNQAKGGLFLPVITGEKLQGITI GVRPIRNPAQEVVTVTGPFPVGTNLVKLVLSLGETT, (SEQIDNO:5419); CCDS6988.1, Del, G_7, SGARETRGPRAPPGACPACPLRASRRCCPPSSSTLERVSPGGAGRLASSAPTWGCLCRAPPSRP SGRPRPDPALSLEAHTCWQRRTAAPGQPGRHRQG, (SEQIDNO:5421); CCDS7694.1, Ins, G_7, ASAAGGDQRRHAAARSQGYLFPHSTHGIFEPGERAGALRLRPHLRGQGLPDRQIPGPAICVPV LRLSQRLHSPHPHGDGQQVLLPRVSAVSGEVWVL, (SEQIDNO:5422); CCDS45634.1, Del, G_7, LVWAQPGMALWHGPLAPYPLPAPCPALWMHPQQTVAAGWVPTGESGPPSAKGSYWSWRG RLQHGPTPTSAPMSTWPGSLAFLRPRYRCGSRSAGPK, (SEQIDNO:5424); CCDS10912.1, Del, G_7, CPLASTPPPPGAPATPRGRPAAEGLRPTPPMPMAMVTRRRAAVTIETGCCTWAPEPRWRMLY LCTSHPGGSARIVVSSAPFAPSLWLLTRWKCTL, (SEQIDNO:5425); CCDS53777.1, Del, G_7, AVRHPGLAGAGARAGGRRAAAGAAGGDPGLGSSPGGGARRRCWRSSGRCRARCSSRRAGT RRSSPNTWLTSATSSATSVAATAGMESISHSLLC, (SEQIDNO:5426); CCDS34507.1, Ins, G_7, GPGLPLAGAALPRALQRRGLLPGLQCLWLCGQAQAGVAAPAAHPQLCGGLLPLGEGHGCV AGRVPAPAERLLQPLLGPAGNGGQPAQRPGRQLL, (SEQIDNO:5427); CCDS12519.1, Ins, G 7, PHAQVHGPSVPHPAQPAAPESQGPHLPHRWLPRVPDSRGAAGPLGFPAQPCAPAPAAARRPL APQRPPRRDPPLLPASRPLPGGAEHRPRRRRG, (SEQIDNO:5428); CCDS6434.1, Del, G 7, TLHHPTRMAVLRTGQINPPTPGLGSTAPPQGFCRKTWAGLWGAQPPATGACAARRSDTTRCF SGTRSSTPAPSPSSAQSAGRPSSKAPSCCGTS, (SEQIDNO:5429); CCDS32498.1, Del, G_7, STITLGFGWMLILGKDTAEPSPRAPRTTARSCRLRRTSSLIRWRCGRLETPQRSSWPRATRAS WMRTLRPRPCWRSVGIRATAKGSGKSRTMN, (SEQIDNO:5431); CCDS10750.1, Ins, G_7, VSTGGSGRAFRPAALSGRRRRRRSQTGLSAAGQVPGLDQPAVSRPTARPPPAPALPAGLCPSA PAGTSRSRGPPGCRRRLRSASGARRRNRSL, (SEQIDNO:5432); CCDS1951.1, Del, G_7, QCCCCCGAGVGVLRPSSPPSLARRPLLPGVSTTSSQMWWRRQHLPWSISRSWTGTLSWAARS LSRTAQDSWWLPMGSLSPTPMWWLIGAESV, (SEQIDNO:5433); CCDS13710.1, Ins, G_7, GQELQHEAAAEEHFPQRPGQGGSDHRGLDHHRGVPHRRHEAGRRGGAGLQPEQQLQGRRA HHHRSRHLGHCPPPRGPDPSHGQLPRRVHTG, (SEQIDNO:5436); CCDS956.1, Ins, G_7, GRLGGRQRRRHPPGRATFGYGEGGLGPARDRGRGGRRGDWRKRRRKPPVHPHARLPEGRA AAAHWRRGPRRHRDPREAAFLRAHPPRGAA, (SEQIDNO:5437); CCDS46366.2, Del, G_7, QEATSTTWTTYCRRRGNRPWSRSERGCFCRSVSISTPWILMKRKCHSHPSTGCWWKSTQCPC RPSRRSIQVRLCFCPGVTPCHASWTPHS, (SEQIDNO:5438); CCDS12673.1, Del, G_7, RRWRRRRRPKRRRRKRASSCRLCRRPRVRRRRRPGPGRAQRLRELRARDPRASPGRPPRPLP NRPRASASRPSRWRASARRCSRRATPAA, (SEQIDNO:5439); CCDS31847.1, Del, G_7, GEAAAAGRPSLAAPPGTATATRRSTTGRSRGPGAWPATRSRGPDPCRRPRRRCRPRARWTPC RSAVAWTADSEPLSAGSASRARSRGSS, (SEQIDNO:5440); CCDS32830.1, Del, G_7, TGGRRMTRRTMTTTKMMMMRRTKRRRRKRRRMTMMTRRTLLTKKTCLTPRTSAATKALP RQTISQRRPIQTPPGTSLTPSRLAVLAIWG, (SEQIDNO:5441); CCDS30670.1, Del, G_7, TPPVARMGRSPAAGCGAAATSKPWGMRRAETQTAAPRHLPKQSPDASPPVAPPAWTRPGST AVSHPGSTPGAPSLATAVPSPLDSSAMS, (SEQIDNO:5442); CCDS53777.1, Ins, G_7, LRSDTQGWPGRGRARGAAGRPLGPRAGTRASGPRLEAAREGAAGAALGAAGRGAAHGAQ VPGEVHPIHGSRPQLRRRPRWRPPQGWRA, (SEQIDNO:5444); CCDS780.2, Del, G_7, LVHLGPKVRVVIQVLRALEASRVPLVQRENLEKGVVQVQMEEEECQENLGQREIEGLMDFR VCQVTKVTGVNEVLKVLQVLLVMME, (SEQIDNO:5446); CCDS32830.1, Del, G_7, RRMTRRTMTTTKMMMMRRTKRRRRKRRRMTMMTRRTLLTKKTCLTPRTSAATKALPRQTI SQRRPIQTPPGTSLTPSRLAVLAIWG, (SEQIDNO:5447); CCDS 11607.1, Ins, G_7, SLRAHPWLSAPGCNCAPSLPDFSPLHPQCGISLVPRGTPLDLWTSRPGQEAATRNPRPLLLKFT ASVVVPDSSPAPGTTSTWGGAF, (SEQIDNO:5448); CCDS46134.1, Ins, G_7, GPCRPADLHQPGAPHQPRCVCAGQWAGPIWDPHCPQPRPRPGTHGRHRPPSSASSREQGFCQ QPPDPECGQGRFLRAREALRAAV, (SEQIDNO:5449); CCDS41781.2, Ins, G_7, DTGGSGLGHKQPEAAGTWTYGGSRGPSLLLSLHGGPGGPGACLTKELPHQSGAGQEPAGRG PVPPGTPYPCQSANLSPKPGQAQ, (SEQIDNO:5451); CCDS9548.1, Ins, G_7, GCGCGPAGQYGGAVGLARRGTAGCLPCPPWLCCCCAFPACGLPVTDGWRGWQGSGVVRV SGSAPWAPGFPFSLSCPLCGTQPRW, (SEQIDNO:5452); CCDS7644.1, Del, G_7, RCPGRGPAASATEANCSMVTWLLWALRLLCRQHPPELETRLAVPPAHHLSTSWRRQPPILPL AWAPMPHTSPPTPGTALPPLAP, (SEQIDNO:5453); CCDS7916.1, Del, G_7, PRPRPPPPRSWGCTSLRLPISGSPMTSAWRSLRMKTSRRSLMSVASAYSAKTPCPYGPRAPGC SLRAAAARGAGCRPRCCRWT, (SEQIDNO:5455); CCDS46218.1, Ins, G_7, RGAPGAGGRTGAAAAGGGTRPGAAEAPLGGAQGPAAPRAGLRPGPRGGGGAGAFAGAASG NCSSRSEDEGQVSKTPYRPSLF, (SEQIDNO:5457); CCDS10389.1, Del, G_7, RTPASCVRTLTWTWQWSVPIRECSSTKASVARQPPGCSWRSRSTLSLSGGAWSMPRNGPWE TPSMSKQNRGLRLIKSSSTKS, (SEQIDNO:5458); CCDS32107.1, Del, G_7, RILSSPSGCGCVVAVWRSSPAAGWAMSSGNGTPTTSLRVMPSPTSGILSALQKCGWMNTSNT TMRPGPRPSGRPSAVWLRG, (SEQIDNO:5459); CCDS13.1, Del, G_7, AGARPPGNSATTTCPTRWAAATCSRPTWCTTCASAATTCAPGTARTCLWAPGWRRWTSSGS TTRASTPNTGPAAAATSTW, (SEQIDNO:5460); CCDS47165.1, Del, G_7, RVAVKKAGRAQPVIREPATPAVPSTGPARMASVNAARAGMESTALSRVVLVCATAMEDVP WTKMAGIVCASLDGEEQAVT, (SEQIDNO:5461); CCDS12474.1, Ins, G_7, VCFPAFSLVGQHLLVLPRGLPCPSLHRTEPNQRLCLALSCGLPLLGDSAHLLDHHLPDLPAPL GCPWGLPLLHHTPGHPL, (SEQIDNO:5462); CCDS34561.1, Ins, G_7, GGDTAPSAAPTCPVHLQSVQWHRAQLTARSLPGPYRQRLPVGLPVLLQPPEPTRQRPRPHRL RQLGLHGGRGPVPALSA, (SEQIDNO:5463); CCDS11091.1, Del, G_7, QGARGGPRSGPPSPSPAVAVSLSPPAEGAAFGGVGKQVGLAMGALLHPEAQLGVPLISEPTQ GSIPMDRPLAWPSPTTP, (SEQIDNO:5464); CCDS8932.1, Del, G_7, RSTGLTGEQTHWLRPTSGPATMSPWYRGPTPSPLTCRCTTPPASPWLPIPVRPMGARAPAPTC VSSTTTGPCPAPAPTS, (SEQIDNO:5465); CCDS42992.1, Del, G_7, CRWRCLGRSEAMSWHTSGMGGCPGLASSSPASSWPARASPWARAWRQPWKTSGPSSSSSLS CVRCSAGIERCFGRGRD, (SEQIDNO:5467); CCDS58065.1, Del, G_7, PLSGTWGTLRAPSSFHPLVATQALLSQRGHPRTALGGSQPLSATPSRVLPPRRAAAPTQQLPH ALLAPSLQDLAKRPP, (SEQIDNO:5468); CCDS13149.1, Del, G_7, RTRLTLTGTRSSGTRASPSAVCLCCWTCPSRSFPAASPSGLRAPVGAQAPPEVTLRASCASSAS KACAGLWPWGAQVR, (SEQIDNO:5469); CCDS48044.1, Del, G_7, SWGPAASTAAVSAAAVAGACESLRGQRTAPEPSPDGELPVRPIARAQSMKNFPSAAGRGAPR TGMKARSWRGRRAP, (SEQIDNO:5472); CCDS5707.1, Ins, G_7, AVCAGPRHPGRPRLPALGPCTAAVWGMAQPGGPCPGEGFCGSNPLTLRAPGGSASAANLRP RWHEAQHVGPRFLR, (SEQIDNO:5473); CCDS53264.1, Ins, G_7, GPRALAAAVQATGLGPPRQRGADGGRGGRLLPGAGGRRRELHQPPRSEPAGPGRRGSRAQG PSGLRPALPSARAG, (SEQIDNO:5474); CCDS59068.1, Ins, G_7, AVCAGPRHPGRPRLPALGPCTAAVWGMAQPGGPCPGEGFCGSNPLTLRAPGGSASAANLRP RVWDAITLGIQVM, (SEQIDNO:5475); CCDS 1942.2, Ins, G_7, RTGRAAGRAGCGSGPHGALLQRSTGFHPFLPTWRPRPHGSVSAAIPLRPAAALLCPRLSPSAP LRLLPHRPGTL, (SEQIDNO:5477); CCDS58679.1, Del, G_7, TWRSSSTRREARSQGLQQPRPRNLSPSTSCRSILLRLSTTRAATTADSSSSMWPVVWSIFSSLS LGPVWHPEGT, (SEQIDNO:5478); CCDS31576.1, Del, G_7, PSSLDRSQSPITMVTNTWLLPLLSSSKARLCATIEWTLMAQDTCWETWKAGSSCCFWRRRNR WMAPSLSRISV, (SEQIDNO:5480); CCDS58680.1, Del, G_7, TWRSSSTRREARSQGLQPRPRNLSPSTSCRSILLRLSTTRAATTADSSSSMWPVVWSIFSSLSL GPVWHPEGT, (SEQIDNO:5482); CCDS 10047.2, Del, G_7, EEEVGEVVGEGEEGGGEEEEVVEEVSKAGEIMVEEGVMVEEGAMVEEAMEIHMEEVVVVV VVVVEEVDIEDT, (SEQIDNO:5483); CCDS13024.2, Ins, G_7, GPGSAVAAPPAARSHGNGEGRRPQRYTKPGAEKAKPSAAGAQHEAEAGGGGPGHGSGGGR GPGRGLPGAATA, (SEQIDNO:5484); CCDS 10746.1, Ins, G_7, VPKDHYRYVPADSAAFSAACHPPRLSFPRSGTQSSSGPPPHPPAPGQTGSVGPGHVLLRVLSP AAPGSTGQP, (SEQIDNO:5486); CCDS43859.1, Ins, G_7, PGRRRLLLGAARRARGQRVPGGRRAPAHRRRGRAHLRRRRRQGHPLLPRLPARRDRSERGP AETCQRPGFV, (SEQIDNO:5488); CCDS59069.1, Ins, G_7, AVCAGPRHPGRPRLPALGPCTAAVWGMAQPGGPCPGEGFCGSNPLTLRAPGGSASAANLRP RVKLASPAV, (SEQIDNO:5489); CCDS12645.1, Ins, G_7, DTAGAAASGWGVLGLHPAEGEEEEEEPWRSRRRSQWRRGILRSESSGVGKWGPRLLDTSSP WSHGTRWQG, (SEQIDNO:5490); CCDS872.1, Ins, G_7, GGRGLLGLGPGCFISCGLSGHWSCRGRSIDAGSSSSLGSRSGSLGRAASWTASLAPGHLRRVS AELAEPA, (SEQIDNO:5491); CCDS5873.2, Ins, G_7, GLPGGSCGHLCGSCRARGGWSRGPGRRQPPQAALQRGGQVDGSCRGLPLPAWIPTSTRRQG LPSLPTGAL, (SEQIDNO:5492); CCDS44230.1, Del, G_7, SWRVMQEVNPPNQHQTGAPRGHFIGLMMYGLWTPILEPERTMILIPRFPRRVLARFYSPSPNP ISRASL, (SEQIDNO:5493); CCDS33469.1, Del, G_7, CLASPRTPSWALPVSLRASSWQARPPLGGGRGVAGGNPSRKRMQWQLILVQRNWRQALRV SYPCRKS, (SEQIDNO:5494); CCDS4080.1, Ins, G_7, DRGPAGGAALWAVVRTAGAGQHHRTACEAHRDGDPGARDLPEPQEFNSFSTFNPVPRTPRA CQNSQK, (SEQIDNO:5495); CCDS9585.1, Ins, G_7, AVRQHYGDCHAQRCQRQPPQVPTEPIPVLRGGDSWTWHTGGPAPGPGPRPGGQRPDGIQHP GWGGV, (SEQIDNO:5497); CCDS2897.1, Ins, G_7, GPSVRRQRLVQNQLWRVLQGRAGPGAVRAARGQGHQAAGHQPGCGAAGQRLALLLQLPG LDRVPAV, (SEQIDNO:5498); CCDS31847.1, Ins, G_7, PGRRLRLGALRWLLHPGRRRRRGGVLRVGAAGPGPGRRQGAAGRTPAAARAAAAAPGRAG RPVAQQ, (SEQIDNO:5499); CCDS46134.1, Del, G_7, PLPPRRPPPAWGPSPAPLRLCWSVGRPHLGPPLPPATPPPRHPWPPQASLLCFQPRTRLLPATS RP, (SEQIDNO:5500); CCDS35004.1, Del, G_7, RGDREATGSGWVPGCAPFGQAGSRASRSPRPSVVRLPLGVHLPGVLPAGMTDPPGAPPPALA GRG, (SEQIDNO:5503); CCDS46218.1, Del, G_7, RSPGSRRTDRRCCGWWWNSSRSCGSTTRRSTRACSSSGGTAPWAARRRRGWGLRRSSEWK LFLEI, (SEQIDNO:5504); CCDS33807.1, Ins, G_7, AAAMAAGSGGRGTPAAGRGCHIPVESAATAPGGQRPGRRQRPEAQQRTEDPGGQGQSGPGQ RTP, (SEQIDNO:5505); CCDS32873.2, Del, G_7, PTRMWMMSPRLQPWPGPRSPCRQMSPRARGIVGESRLIRGPRWTAATPRDSGDRTACEPMN GKP, (SEQIDNO:5507); CCDS2479.1, Del, G_7, QWAACATCRWAWSCCSWASCSPLSTSTDTSSLRSWPEITSSAVVCCMRTPCPPRSGLRWSW KRM, (SEQIDNO:5508); CCDS33104.1, Del, G_7, RPASSRCTTPSPRRRAAASRSRSPGRPPRPRPRHPRPLRPPPGPWPRRPPPPTPTRSTGKPRLC, (SEQIDNO:5509); CCDS5715.1, Del, G_7, SLPPGCFWPCLWNSLLRFCPASCSGCYSWTIPPTGSPFSCTTTRSSMNPTSLTPGRSSRTTSQL, (SEQIDNO:5510); CCDS11693.2, Del, G_7, AEQTPEVVVVRVRLRGTRHRLRPPERLREPRPEGVGVAAAETQPPLFPLFAERRRVASIRQLP, (SEQIDNO:5511); CCDS12196.1, Ins, G_7, AGGRHGWPRVRGHPRAQHLLWGHREGLLCPPLPWHCHQVRVLLCQSGPRFWGALPALSCQ RLR, (SEQIDNO:5512); CCDS58679.1, Ins, G_7, GPGGAAARGGKPGARDSSSPDPGIFHRPLPAARFCSDSPPQELLQRPIPAPLCGLWYGPSSHP, (SEQIDNO:5513); CCDS1810.1, Del, G_7, PLEGPATPPLPRWCLPSWHQSWPWSLSWAWWGTVWPSSSSASTRGPGPPTRCSWSAWWPL TSS, (SEQIDNO:5514); CCDS41842.1, Del, G_7, ALPTGCGSCPPCPEASPLCSTRRCSTTGSPPPSNTSLIPGTRMSGKAPTGPPQSGEPLASRS, (SEQIDNO:5517); CCDS55182.1, Del, G_7, ALQTGPGSCPPSRAASLLFSIRRWSTISCPRPSATSQTPGRGVPPRAPASPGRRPLKKWPTP, (SEQIDNO:5518); CCDS59246.1, Del, G_7, GGHVSGLLSPWKWQWLQGVCAREEGAATESPARTTMKARSTEGLWLAHKHAWCTRQREQ PRR, (SEQIDNO:5519); CCDS59279.1, Del, G_7, MEETVVVAVATVETEVEAMEETGVVAAMEETEVGATEETEVAMEAKWEEETTTEMISATD HT, (SEQIDNO:5521); CCDS59399.1, Del, G_7, PAVAVVGRWRSAARPSAACTTRPRCCDTATMTICRARAVWKWTTACRRWSSGCSYRKTSW RS, (SEQIDNO:5522); CCDS 10452.1, Del, G_7, PGALVALGWGTAVASAEVSAVASGAGVAAVDGAGAEAAELAEARPRIRSGCPSPSWAAWS RT, (SEQIDNO:5523); CCDS41865.1, Del, G_7, SGRGRRRRSSLSCQGGWLSLTVSEGMSPKTWFLSRTSLTSILTCRSSCGHTASRPTFQSRQL, (SEQIDNO:5525); CCDS30556.1, Ins, G_7, AVPPGRGRGGWPPQPDTAQQPCVHQVLLKRPALGTGHENGRGGDQQQVGSAARAAPGLRP L, (SEQIDNO:5526); CCDS 11420.1, Del, G_7, LAASSSTTASPRPSGTALSSLPPSTLRSPSPTMSVSRVTMTPPSLRDTPLSATSPWKCSSS, (SEQIDNO:5528); CCDS5690.1, Ins, G_7, PQRCWGDHAGCVAPRTWHPAMGPCSPGPLGNPEQHLSHPGRLWGQQPLRQHHLLLQVCVL P, (SEQIDNO:5529); CCDS14077.1, Del, G_7, RSALGTFAAATPPAAAPSPTAPGTATGRSCTALPSRSESTASWFDSLHEELKRIRSQTHTA, (SEQIDNO:5530); CCDS10785.1, Del, G_7, GALRAVVQSSPPTLRCSAAATTSPTLLFSCNSPQPWSLQSCWHLLRTSPSWAAASPSWPR, (SEQIDNO:5531); CCDS 11086.1, Ins, G_7, SPAGRGCGLWAERGELRPAGFRCTPRAGGRRRCGAGHRAGDDADEPRGAPGPSPVRLPKG, (SEQIDNO:5532); CCDS10908.1, Del, G_7, SRSSATLPGRRRRRWLRRRRQPISVAPAGPPRRPNQKPNPPGGARCVIMRPTWPGTSAFT, (SEQIDNO:5533); CCDS1810.1, Ins, G_7, VLWRALPPHLFLAGVCLPGTNPGPGVCPGPGGEQFGPLHLLHPHAALDLQHGVPGQPGGR, (SEQIDNO:5534); CCDS6988.1, Ins, G_7, QAGWPLQPPPGAASAGPLLLGLQGGPGRTQPCLWKPTLAGKEGRRPLANQEGTGRAEFA, (SEQIDNO:5535); CCDS6025.1, Del, G_7, RGRGCWRSPRSASASCCKSALRGPPRRPPARPAALAPGTPPSAWTQRRCPGTCPRRSSP, (SEQIDNO:5536); CCDS58850.1, Del, G_7, SSWMPRAPRPLRPSIPPMEVSSARYPWPKSPTSTRQWPQPRMPLRMDGGGRSVRGTGAG, (SEQIDNO:5537); CCDS13145.1, Del, G_7, TPARSESGECFSPRRRPTSWSGAFGSSGTCRRPSANTWPASSASRPRRSRSGSRTTATR, (SEQIDNO:5538); CCDS43896.1, Ins, G_7, GPQQHHALRGVCWRCQRLSADPLPLQQGPHPASHQPERRGPESLGHPEKPTLLGQKGG, (SEQIDNO:5540); CCDS6097.2, Ins, G_7, GRPQPPCPAHLSECEERGIGGLPHQAAQLRGPDLHSLPEEGGRGAGHTARKPWPEPPT, (SEQIDNO:5541); CCDS45544.1, Del, G_7, PEAPSTRAAPPSLRAARAHQRTGRQHPPPAKHSSSQCTQGRRWGAWHPGSWPVAQRMG, (SEQIDNO:5542); CCDS32996.1, Del, G_7, SSGSGDSGVLLRASQRRQRQGRKRTESGTNMRRASGQESGTLRAPRSAQQRQSRITAP, (SEQIDNO:5543); CCDS47173.1, Del, G_7, NRFRLCITILPGMKMSWSSEKVMSLMSWKSVMTAGLWGPQEEPNSLVLSPETTSRGC, (SEQIDNO:5545); CCDS12794.1, Del, G_7, SGLTSRPMITSSRLLEMASASACPVSRPWMSLRLQGPSGSSVTSSWGRMWPSSTAGT, (SEQIDNO:5546); CCDS1733.1, Ins, G_7, AAGQCERGAGGAVGSVGGAGGRGHAGMRAALLWGPWGAGLSSQRDPQCLGAQGAGQ, (SEQIDNO:5547); CCDS46136.1, Ins, G_7, GTCLAAGVGPPGPPRQPLQPFLRGAVHAQRAACHQRPQPLREQRVLHPLGGARLPS, (SEQIDNO:5548); CCDS55182.1, Ins, G_7, LPCRLGLDRAPHLGQPHSCFPSGDGQLFPVPGLPLPARPLEGECRQGHRHHQEEDL, (SEQIDNO:5549); CCDS34222.1, Del, G_7, LPVNVHLVSHSITLLVSTTTNVGLNLRFVEQRESVKTLQAVSAVNAKEGSLLMPPD, (SEQIDNO:5550); CCDS43859.1, Del, G_7, RPPPPPPRRCPPRPGAACSRRAAGPCSPAARPCTSPPPAPPRPPSTAASSCSTGPK, (SEQIDNO:5551); CCDS3359.2, Del, G_7, AACASASSVRPSRRRRAASSTTASWSASLTRPSAACGSTRARSCSTPCWRRCASP, (SEQIDNO:5552); CCDS10047.2, Del, G_7, EEEVVEEVSKAGEIMVEEGVMVEEGAMVEEAMEIHMEEVVVVVVVVVEEVDIEDT, (SEQIDNO:5553); CCDS 1955.1, Ins, G_7, GPLCCHCEKLPGDGANYHQSSGSCRGLDSDRYLAFLAERPSLCRSRGLEPSRMGG, (SEQIDNO:5554); CCDS6988.1, Del, G_7, GRLASSAPTWGCLCRAPPSRPSGRPRPDPALSLEAHTCWQRRTAAPGQPGRHRQG, (SEQIDNO:5555); CCDS45938.1, Ins, G_7, LSEGPSPPQPLRPPAATPPNHSALWKEPAPSPQQETPAGRGNFRAPRGGDTPTEP, (SEQIDNO:5556); CCDS59279.1, Ins, G_7, LWRRPWWWQWLRWRPKWRLWRRQEWWRLWRRPRWGLRRRPRWLWRQNGRKKRLQK, (SEQIDNO:5557); CCDS5194.1, Del, G_7, PWRRRPVAGRRRRARRAPEARRAPEARGLAPRTLRPPRGLRPRAMPKTKPHVPLP, (SEQIDNO:5558); CCDS32871.1, Del, G_7, LPQWRKRPVSLRRRHLGRLMTCWKATWAFGTPSWRPPWWRRPRRQRAPRSLHAV, (SEQIDNO:5560); CCDS41590.1, Del, G_7, QPCSRSRSSSRLISPTRRVGRRRRRTASTPSPSPCSQAPAVASRGWWRPSRPSC, (SEQIDNO:5561); CCDS9991.1, Del, G_7, TTPATRWWWMGKSTTSTCCPAASSTPRPCPSLATGWSSTCQACLRKQRRMKRKA, (SEQIDNO:5562); CCDS31598.1, Ins, G_7, AHGLRTQLPGEQLLAPRRLPPLQSPDPGHLQAGPQMPSLWSELPQAVQGSPVS, (SEQIDNO:5563); CCDS2164.1, Ins, G_7, GDSLLSTQQAGAVGTPGHHRPARRREQGPRRGHTRDWHTVQGCLSRGRDTRSL, (SEQIDNO:5565); CCDS34507.1, Del, G_7, TWPAACWCSSSPSSSATWTSPGAAVPVALRPSASWSRCTCSSSATMWRSTTPR, (SEQIDNO:5567); CCDS11439.1, Del, G_7, CHPAWLSPISACPRLLPYPFHVLALEVTFPPGTAMPQMQLQALAMWEKGWDK, (SEQIDNO:5568); CCDS7296.1, Del, G_7, RNPRLPNHGRPKSPNQRTAIKKELQIPPLKMGQGMDSPFCLLRALFWDRSCP, (SEQIDNO:5569); CCDS 14077.1, Ins, G_7, EEALWAHLQQPPLLLLHRLQQLLELRRGGPAQPCHRGQSPLPAGSTLCTRN, (SEQIDNO:5571); CCDS12604.2, Del, G_7, LTVACRSAQPTVAATAPAPRPWDHAAVSLASWDVPVTCTCGRTRGLGGGTT, (SEQIDNO:5572); CCDS6714.1, Ins, G_7, AAPCCRAGPGLSAPAQLLRRRLRSGADFQGEGYWPESAAVAESEVSETLI, (SEQIDNO:5573); CCDS54360.1, Del, G_7, CQKIKLALSSPPRCGLLCSTMATWAASGICSSMAKAKISGKWLKFKVLLE, (SEQIDNO:5575); CCDS35004.1, Ins, G_7, GAETARRPGAAGFPAARPSARPGAAPVGAPGPAWSASLWASTCPEYCQRA, (SEQIDNO:5576); CCDS43789.1, Ins, G_7, GSSGSCSPPQRAHGVQTARGHLGRGGDGDPDAAAGGRAEQPKGGVGPVSG, (SEQIDNO:5577); CCDS45544.1, Del, G_7, SCQHLRPAGTLPAPSSCWPVLLPGHLWKRQMASKPRQILGLRIPRWLPRL, (SEQIDNO:5578); CCDS5941.2, Del, G_7, SSSPHICQRGPTHTRLSLQGSKASKEFTRRRRPSCTDEAEEWPVPWAGGA, (SEQIDNO:5579); CCDS 11486.1, Del, G_7, YPSWEAQEPLRSRNPAVWLGRAPGERSESSSWTQGSARSHQGARLSIWPR, (SEQIDNO:5580); CCDS42397.1, Del, G_7, TMCLCPTSSREAGRTLLSRSPGRGQSQSWGSNELSRGARGRTMPSPTGT, (SEQIDNO:5583); CCDS6607.1, Ins, G_7, GFCEWTATPGCSPPEDSGTCSSRCQALRHLQAASGQPWLCQQNSWQVL, (SEQIDNO:5584); CCDS6996.2, Ins, G_7, GLAPHRHGPHQELRRGGIEARPAWRRWQPPENGAYKPVLLGLRDSTGL, (SEQIDNO:5585); CCDS9602.1, Ins, G_7, GPGATCLLCAPPLQCPGRRHFPATTGAGGSLVHWGVWGPPAGRELRGD, (SEQIDNO:5586); CCDS31754.1, Ins, G_7, GSARPDWAGRRGGSLTLTPRRGGGAQLLGRRDMAGAEAEAGRPHDRLD, (SEQIDNO:5587); CCDS7694.1, Del, G_7, KTGSSWTGSCRQGWRWSGSDCGISITTTTPRGACGASAAGRALARGRL, (SEQIDNO:5588); CCDS56255.1, Ins, G_7, PGGPGCTRCSGWCKGCRADRSNPSGFNPTRRCSPPPDRPGGGPGCGEG, (SEQIDNO:5589); CCDS33764.1, Ins, G_7, VQGQRRHRHALRLPTLRLFPAVPPVSTKDIGQARRRTHGDSHRQRHCL, (SEQIDNO:5590); CCDS5715.1, Ins, G_7, AASPPGVSGRVCGTAYSVSAPLPAAAATPGLSPRQGHPFPAQQRGLP, (SEQIDNO:5591); CCDS10452.1, Ins, G_7, ARGPWWPWDGEPRWLPRRFRQWHPGPGSRPWTGPGPRPRSSRRQGRG, (SEQIDNO:5592); CCDS6919.1, Ins, G_7, EATVHEPVLSDLVLLPSAGPQAGHSQQLQQDQEASHAHHRGTQLPVF, (SEQIDNO:5594); CCDS6204.1, Ins, G_7, GHLHQCLCDYTHVLPVTVLHAHREVCAQSQCLHQQRELLFPLVAGHA, (SEQIDNO:5595); CCDS45072.1, Del, G_7, GSATKMSTNAARRTGAASRSATTSRVASTVPATAASSSPLMAGPAKT, (SEQIDNO:5596); CCDS46339.1, Del, G_7, LWLLSPSRKRRRGSSPPGRHWLCPQTRRSPCPPMPTRRSLAPTAAGS, (SEQIDNO:5597); CCDS46539.1, Del, G_7, PCRRPPTAPASRPPAWPSRRPPAGPCKPPEPGNQWKEPGRGQASPDC, (SEQIDNO:5598); CCDS6996.2, Del, G_7, SCSPQARTASGAAEGWDRGQASMAEVAAARKWRLQASSSWPQGQHRP, (SEQIDNO:5600); CCDS7957.1, Del, G_7, TGVRTSEGSRCAPALPARRPRTPEALRSRPGSPALCFASCSWCPTTS, (SEQIDNO:5601); CCDS30901.1, Del, G_7, TRLSPMWAVLPRCSFLPHGPVLTASFWLPWALTDMWPSVLHSTMPAT, (SEQIDNO:5602); CCDS2832.1, Ins, G_7, AAGAQEVRSDRRLPVVQAGAGPGCERQSAGVLPSAHWTEVCPEAPV, (SEQIDNO:5603); CCDS53360.1, Del, G_7, GARVSTLPPRRRSQRWGLSRKIGCRSPGVVSLAVAWPPDASQGSRS, (SEQIDNO:5605); CCDS41741.1, Del, G_7, GSGSARLPSRRTPPCRRVRGPASPMGMRRRAQPSASTRRCSACSTS, (SEQIDNO:5606); CCDS46546.1, Del, G_7, PRKMILSQLHHSMTRFWRWSPCSAACRWPSLESSGYSIIGSPSVAR, (SEQIDNO:5608); CCDS 10864.1, Ins, G_7, RGQDPARPGFRRQQPDPSHRAPEDPGAAAVSCLHQLAAALQLHMVT, (SEQIDNO:5609); CCDS 10912.1, Ins, G 7, GALWPLHPRLPGHRRLREGARRRRVCVRLHLCPWQWLPGDGRRSP, (SEQIDNO:5612); CCDS4748.1, Ins, G_7, GPGRPREARHRGHSAADNDHHRPEPGRGPGQETRPKLPPNEACSL, (SEQIDNO:5613); CCDS 12474.1, Del, G_7, LLSCLFSGWSTSSGSSERPSLSQPTQNRAKSKAMSGAQLWASSSG, (SEQIDNO:5614); CCDS46136.1, Del, G_7, RPRRKGSLGRGLPPSHPTQERPGHLQTRVRSHSRGPGRSRPSWFK, (SEQIDNO:5617); CCDS9830.1, Del, G_7, SCHFSSSSSGSRRSPAQPTMPGSMSCWALAPARPCPPGRCWAWRS, (SEQIDNO:5618); CCDS46196.1, Ins, G_7, GAGCPRRCQIRAWCRARVDGRCGVWRRRTGPPRLPPKPLGPEQL, (SEQIDNO:5621); CCDS55562.1, Ins, G 7, GLHPHHRPEGGVCRWNEQVGTLLPGEHDRVCGKESARRPCHRQR, (SEQIDNO:5622); CCDS33159.2, Del, G_7, GPKKGARHRWPVPAPLLSASASTSLPSPSSCPHTLFRKNPLWRC, (SEQIDNO:5623); CCDS10363.1, Del, G_7, LPHVERYLPKRNPPASLDPVQPHILLKRHQEPIANWGLWLHRSL, (SEQIDNO:5624); CCDS9548.1, Del, G_7, LWLWAGWTVWWSCGPGEKGHGWLPSLPTMALLLLRFSCMRVASY, (SEQIDNO:5625); CCDS 13024.2, Del, G 7, PGQRRCCSPRRPQPWKRGRTAPAEVHKARSGKGEAQCRGRPARS, (SEQIDNO:5626); CCDS9991.1, Ins, G_7, QQRRPHGGGGWERVRLPPAAQRHHQHQGRVLHWQRGGHPLARLV, (SEQIDNO:5627); CCDS 10904.1, Del, G_7, TSRPCTSTSTTSLLPMALLCVKRGRRCTKAKATAVSAAVSTEQI, (SEQIDNO:5628); CCDS33415.1, Ins, G_7, EREGETERQGPRARPGQREGQGQTESEKRGALLGPGQGEEQRA, (SEQIDNO:5629); CCDS47200.1, Del, G 7, HGAKVRKRKRETRIQPPRNRSHCLPCLPWVHLHQNPTDHQMLT, (SEQIDNO:5630); CCDS 10670.1, Ins, G_7, HGLLPLPRDHIHLRDSLPEPTDHTTEDCSQSLCQRLPGERQKL, (SEQIDNO:5631); CCDS30556.1, Del, G_7, PWPALAWCAWAWSASASSCSLASPALPDAWPSSPCPTSRSRAA, (SEQIDNO:5632); CCDS48074.1, Del, G_7, RVRIQPLVLESRVKIQHPVRACRVQIHHWVLTSLLRAFRSRPR, (SEQIDNO:5633); CCDS 11962.1, Del, G_7, TLSHQIKSSFGQKMGKVIFTNYLPVAFQLVIHSAVMWGRQLKI, (SEQIDNO:5634); CCDS6876.1, Del, G_7, TSQRFSSTALACAPSRIQFPMGLAGLSQCPPPLSEMTLSFRLV, (SEQIDNO:5635); CCDS31763.1, Del, G_7, VSTSWGPTAGAARARTCSPCSPTTPSPASGSTSRRTRPNRVLT, (SEQIDNO:5636); CCDS7163.1, Del, G_7, WWCTRGGGKRKKKMCKVSGGCTACVERCPWKGICWKWPVTVAA, (SEQIDNO:5637); CCDS3936.1, Del, G_7, ALSVYQFFIPQREVKISTIILPSNKPFKPLTKRILVLLGSIK, (SEQIDNO:5638); CCDS47200.1, Ins, G_7, AMGPKSGKGKGRQEFSHPETEAIASLVYLGSTSTKTQQTTKC, (SEQIDNO:5639); CCDS8190.1, Del, G_7, GAWELGGLARWGPLCTHPQCQVLLLSSSTTSSSSSSSSSSLV, (SEQIDNO:5640); CCDS13064.1, Del, G_7, KERNWGRKRRWRRRVMLIVMKKRRKMRRAPRRAWRLRTGPRE, (SEQIDNO:5641); CCDS12398.1, Del, G 7, PGVQGAAVMPTGACATTGIAITPRNATTPSASRPTCRGACSS, (SEQIDNO:5642); CCDS31763.1, Ins, G_7, VSPRAGAQPQEQPERGHAHRAVLQHRHPPVALPQGEHVQIGS, (SEQIDNO:5643); CCDS45544.1, Ins, G_7, AAASISVLQGPSRPPAAAGLFSCLGTSGRGRWRPSHDRYWG, (SEQIDNO:5644); CCDS33807.1, Del, G_7, CRDGSWLWRSGHPCCWARVPYTCGVGSNGAGRPEAGATPAA, (SEQIDNO:5645); CCDS4155.1, Ins, G 7, GYWAESTACLHRSEEHGCHLAGQPADPSPVALHPGHSLCTV, (SEQIDNO:5646); CCDS53272.1, Del, G_7, PEPLEPMTSQGAHTTSCAGRRRRSSRRHISPWGTGATRTCS, (SEQIDNO:5647); CCDS54254.1, Del, G_7, SSTEGPCTEMEGKEGRGLVPHPLTPLPAGPSTLRARPEATA, (SEQIDNO:5648); CCDS22.1, Ins, G 7, ATRGTPCPGAGCRPPGIDRQPPVLPEGYGEPLRVLRGHHV, (SEQIDNO:5649); CCDS6714.1, Del, G_7, CAVLPRRTGTICTGPATATPPSLWSRFPRGRLLAGKRRCG, (SEQIDNO:5650); CCDS3476.1, Del, G 7, CPLSLWKRLKVFSRDGLSEDQVLHRLTVGKSHLLEFQTPL, (SEQIDNO:5651); CCDS48044.1, Ins, G_7, GPGGPQHLLQQSAQRPWPGPARVCAARGLPQSQAPTESCQ, (SEQIDNO:5652); CCDS46136.1, Del, G_7, HLPGSRRRPPRPPTPTPPTLSTRRCPRPACCLSSAPAAPP, (SEQIDNO:5653); CCDS5699.2, Del, G_7, KGLGDRRAGAGRGQSGWSGEARDGSCKNSPGCVDGRRGSG, (SEQIDNO:5654); CCDS42402.1, Del, G 7, LCTAKWPGQYGPTWASCPLAQQCLAHTQFLRALGSPWGRD, (SEQIDNO:5655); CCDS33908.1, Del, G_7, LKNAAASRSMESTQRSPITWTGCGSRWAYHKVLWSPRWNG, (SEQIDNO:5656); CCDS46366.2, Ins, G_7, LRKLLQQPGLLTAGEEGTGPGAGAREAASAGVSQSQLLGS, (SEQIDNO:5657); CCDS32325.2, Ins, G_7, PAAPEWPASRVLCASTSYSPPGGCPRPCAGHGAACLPPWR, (SEQIDNO:5658); CCDS8839.1, Del, G 7, PPRVDSAQMWAIAPSREGQSLRAPPSCGRPLRSRRPARGI, (SEQIDNO:5659); CCDS 11662.1, Ins, G_7, QQRAPPHHPPCPPVHPGPCDASPDPTQHAGSAGGGLSQAS, (SEQIDNO:5661); CCDS46136.1, Del, G_7, RREPQGLGGWTSGVGAEPQWARGRPPAAAPEAAWTQRPDG, (SEQIDNO:5662); CCDS2493.1, Del, G_7, SMTAQGTCCTGGRRPPTAASCERLSASSVSMTTSLSTTSG, (SEQIDNO:5663); CCDS43896.1, Del, G_7, TPTTSRSSGSLLEVPASLCRPSPPTTRASSGEPSARAAWP, (SEQIDNO:5664); CCDS2885.1, Del, G 7, TTFQRAPLKFKLALKRVCRKSVLGALGSMVGLSGGQRTSW, (SEQIDNO:5665); CCDS8937.2, Del, G_7, TTVGLLRMAATRTSLPCTPLWLCSPAPWLPKMPPFVSTTP, (SEQIDNO:5666); CCDS4442.1, Ins, G_7, GLYCPVYQDSQGTKARRVQQGDQETHGDAQRPGHHHLCQ, (SEQIDNO:5667); CCDS33768.1, Ins, G 7, GVPAGHVCPWLQWPVLQRVHGGLWAHRAGPALPPACPLC, (SEQIDNO:5668); CCDS47911.1, Ins, G_7, KISPRRRISCCYYKRTDHSAGSKHPYFENVAYSGWDSNI, (SEQIDNO:5669); CCDS 11607.1, Del, G_7, PPSPPLALGPRMQLCTQLARFFPITPPVWHILGPQRHTP, (SEQIDNO:5670); CCDS13874.1, Ins, G 7, PRPAGPIPCAEGLHAVPARGGLLPGPGAHCRCLAHAYAC, (SEQIDNO:5671); CCDS3679.1, Ins, G_7, GPGTLRHGRDDPLQGRGRSSEGKDLRRDQSSRRGRRFS, (SEQIDNO:5673); CCDS 10077.1, Del, G_7, TLQRLTTSGLMGKMEYPSYTPAASSSCSLWQSPRTTER, (SEQIDNO:5675); CCDS11420.1, Ins, G 7, VSLPPPPLQRLQGHLGRPYPPCHLLRCGHRPLQCLFLG, (SEQIDNO:5676); CCDS11675.1, Del, G_7, YLSAPGWWPPPSHKRFLICTSPSASSLVWDTALVFSQL, (SEQIDNO:5677); CCDS33460.1, Ins, G_7, ALKLRGSGLPARRGAGAQDGVHEVQVPPGRRQYILKN, (SEQIDNO:5678); CCDS1825.1, Ins, G 7, GPTWWQHLTFDVETDEEPQGWELPFDAGQATERKCTQ, (SEQIDNO:5679); CCDS13086.1, Ins, G_7, GRPGRSSADHWHSGVPTRILPPAHRLLCIQRLPWLLL, (SEQIDNO:5680); CCDS34710.1, Ins, G_7, GRRCQVQGLAGSLRRVRHGSLCPGIGPSLLGHSKKPQ, (SEQIDNO:5681); CCDS41842.1, Ins, G 7, LPCRLGVDRAPHVRKHHPCVPPGDAQLPAHPLLRIPA, (SEQIDNO:5682); CCDS55911.1, Del, G_7,PSSAQALAAAACPRGASSPMPLTRQPCMQPATRWSLS, (SEQIDNO:5683); CCDS34137.1, Ins, G 7, QEEENPPGSCAGCGLHLGDRAEEEQPSREEWEGPTAG, (SEQIDNO:5684); CCDS9636.1, Del, G_7, RRRRGRARAARTGRGARRARRRTASTRSRRSATTRSS, (SEQIDNO:5685); CCDS2164.1, Del, G_7, SLQVPQNAAAASWPVRASSVASQKWGPLGQPWLSPIC, (SEQIDNO:5686); CCDS7037.2, Del, G 7, TCLPWKGHPIPRHCHGGPVREARSWAFPAWPQRMRSL, (SEQIDNO:5687); CCDS31045.1, Del, G_7, AETTLSTAASSSRNSWADGQARICEPVWTSTTTSWV, (SEQIDNO:5688); CCDS59246.1, Ins, G_7, AGGMFLACCHHGNGSGCRVCVQGRRVLPQRAQPGPP, (SEQIDNO:5689); CCDS30556.1, Del, G_7, CSPWARPRRLASAAGHGPAALCAPGSPQTACSGHWP, (SEQIDNO:5690); CCDS2862.1, Ins, G_7, GHCSYKQHSAQHSLQCDFLHCYSQQYSRPSPVLAQQ, (SEQIDNO:5691); CCDS6762.1, Ins, G 7, LRLLAGCGGAGNHQGADGHREENWCLYATDALSLLR, (SEQIDNO:5692); CCDS8115.1, Ins, G_7, RAQAGAAPRATAAARDPHGAQAARWRFCVGGPGDQS, (SEQIDNO:5695); CCDS10511.1, Ins, G_7, GPAPACLLLPYPSQGQVPLARPAPAQAAGVSLGQV, (SEQIDNO:5697); CCDS58996.1, Del, G 7, GSCGRACSCGRGCGTPRGDWKRHTRRRSRSRPPRR, (SEQIDNO:5698); CCDS31754.1, Del, G_7, KRSSRLGWASGRITYSHSPPRGWCSAAWPPRHGWC, (SEQIDNO:5699); CCDS44714.1, Del, G_7, PLGRGSLEGAQSPRECTVLSWSASGLGSLSAANTT, (SEQIDNO:5700); CCDS56255.1, Del, G 7, WRPRLHEVQWVVQRLPCRQVQPLWIQPHQEVLPTP, (SEQIDNO:5701); CCDS13804.1, Ins, G 7, AEVRAQKVDPLLRGRHSHHLLCGAQRLRPETLRG, (SEQIDNO:5703); CCDS2445.1, Ins, G_7, ALWAVWVALAHGCHGPLRHPCQCVDSDAYCHRAW, (SEQIDNO:5704); CCDS11417.1, Ins, G_7, GRERGGSGSTWPEPAAARQSEEGASPGAAARQEA, (SEQIDNO:5705); CCDS 12421.2, Ins, G_7, LSQHPCRLPTSQHDEVVPGLVGEEHAPETHVWQQ, (SEQIDNO:5706); CCDS33655.1, Del, G_7, QGPHRPPVALAEDAGLSSRATCYSPLRVARAGPH, (SEQIDNO:5707); CCDS11675.1, Ins, G_7, ATCQHRDGGRLLLTRGFSYVRRHRHHLWSGILL, (SEQIDNO:5708); CCDS 12712.2, Ins, G_7, GHSQCIRRAGIFRGTRFRRHLLRRESNSFLLCF, (SEQIDNO:5709); CCDS35277.1, Del, G_7, HPGASPLRGVWNTVSRSQCLRLKMEARQLCPRR, (SEQIDNO:5711); CCDS2221.2, Del, G_7, HRLRLQRTCLQEFLLPPPYPPPLFLATPTRAMV, (SEQIDNO:5712); CCDS54254.1, Ins, G_7, RAPPRVLVQKWRAKRGGGWSPTPLPHSQLVPPL, (SEQIDNO:5713); CCDS7048.1, Ins, G_7, RGPGLPGGAPQRCDRGQSPGQPATGAVGLAAQG, (SEQIDNO:5714); CCDS6444.1, Del, G_7, RSRGRRSRLRPHPPAPVPESAALPRAAARSRAS, (SEQIDNO:5715); CCDS43090.1, Del, G_7, WRPRLHEWVVQRLPCRQVQPLWIQPHQEVLPTP, (SEQIDNO:5716); CCDS59442.1, Ins, G_7, ALQAAAQTLRETDPHIQTGQTASCPFGDMAWV, (SEQIDNO:5717); CCDS14240.1, Ins, G_7, ALWRRGRSGVYALPRLAEPAFRHGPGTLPALG, (SEQIDNO:5718); CCDS12741.1, Ins, G_7, GPAGHRHLLCPVHLHGLWRAEAREDFCRGKAS, (SEQIDNO:5721); CCDS9911.2, Del, G_7, PTPARRTKKQSSATYVSLVSSAISLLVNSWRD, (SEQIDNO:5722); CCDS44091.1, Del, G_7, TPLSISRPTSRARQSCCLPGLTHPAHQPLGSM, (SEQIDNO:5724); CCDS58829.1, Del, G_7, YPAHSPWCGPSPGSILRIMGAGTPSTPHCGGS, (SEQIDNO:5725); CCDS30569.2, Ins, G_7, AAAAAAAGDRCPGGLGCPLRRPQREAAATPC, (SEQIDNO:5726); CCDS45171.1, Ins, G_7, APGGGSLGRQQSRLLHQLLAQRGQRLHCRQP, (SEQIDNO:5727); CCDS 12519.1, Del, G_7, GGLQGHGAQRGGAARPSARSFCPSSSATGGA, (SEQIDNO:5728); CCDS12342.1, Ins, G_7, GPFHVDQTQQPVSGCHIQEERVRVAGLFFPL, (SEQIDNO:5729); CCDS59399.1, Ins, G_7, GRRWLWWGDGGARPGLLRPVRHVLAAAILQR, (SEQIDNO:5730); CCDS53807.1, Ins, G_7, LLQGAEALQSPVHAGGSHAQTHWREATQVHV, (SEQIDNO:5731); CCDS46736.1, Ins, G 7, PAPGGGDRGRSPGCPRLPHRNGRQVAPWGGA, (SEQIDNO:5732); CCDS 10488.1, Del, G_7, PRAPAITWPATQHLPLPSLGGPLSTLPRITS, (SEQIDNO:5734); CCDS42296.1, Del, G_7, QATGWRSCWTFSRMSTCPSGERQRKRHLKQE, (SEQIDNO:5735); CCDS41570.1, Del, G_7, SCWRCPRSPWTGKAPRRGSRWGRSHRRRLMG, (SEQIDNO:5736); CCDS34531.1, Ins, G_7, TGSEARNRSEKDNRGIHVSERLTWKRIFPTC, (SEQIDNO:5737); CCDS 10056.1, Del, G_7, WRSAWAAPCTTSATCCWSPASPRARPSMPAT, (SEQIDNO:5738); CCDS42402.1, Ins, G_7, GSALPNGLGNTGPRGRRALWPSSVLPTLSS, (SEQIDNO:5739); CCDS 13676.1, Del, G_7, PGPPTPRLEKLSPGPRLGTRGSVSSSWKHL, (SEQIDNO:5740); CCDS45666.1, Ins, G_7, PNSVFCLWKTLSGAYKSPTKRGKRERSSLL, (SEQIDNO:5741); CCDS3925.1, Del, G_7, RDASAPWAKVVRAAQKILLSSILSSLATPM, (SEQIDNO:5742); CCDS8562.1, Del, G_7, RPSPCLSWSSRIVAPGHALSCRTRRRWSSK, (SEQIDNO:5743); CCDS8891.1, Del, G_7, SAPAGQPRPWRPSRTAAPCAFGGSTAASCA, (SEQIDNO:5744); CCDS 13253.2, Del, G_7, SFQMIMGWPLPSGEPSSTGNVKTLDILNCW, (SEQIDNO:5745); CCDS43842.1, Del, G_7, TRKASAASCASGVTTSTHRPASAWTSMHQT, (SEQIDNO:5746); CCDS30639.1, Del, G_7, VAGTASCLRTGQTRGRRCWRGCCSASSTWA, (SEQIDNO:5747); CCDS13316.1, Ins, G_7, VGSGRLWDHAGRARAEPRGAPSNIGWGAKP, (SEQIDNO:5748); CCDS41570.1, Ins, G_7, AAAGGARGLRGQERPQGGAPDGAAATAGG, (SEQIDNO:5749); CCDS42519.1, Ins, G 7, ASEHQVMDLHQQDGRARGQCTHGQDPLLG, (SEQIDNO:5751); CCDS11417.1, Ins, G_7, GSGSTWPEPAAARQSEEGASPGAAARQEA, (SEQIDNO:5752); CCDS33768.1, Ins, G_7, GVLRPWAVPGQVPGQRGVPLPRGLPWNGL, (SEQIDNO:5753); CCDS956.1, Del, G 7, PAWGPAAAAPPAREGDFWLRRRRPRPGER, (SEQIDNO:5754); CCDS45666.1, Del, G_7, QLSLLLMENSIGGLQESHQEGEEGEEFLT, (SEQIDNO:5755); CCDS42230.1, Del, G_7, SPASPAPTARASSTSPRASLLWLASRPSS, (SEQIDNO:5756); CCDS53798.1, Ins, G_7,SWPPALWAPAGTGTASQGLPVPLLDPVHQ, (SEQIDNO:5758); CCDS8776.1, Del, G_7, AATTLTSAASSAGSSWTPGRRGGTCASS, (SEQIDNO:5759); CCDS12673.1, Ins, G_7, GGGGGGGGDRRGGGGSAPALADFAGGRG, (SEQIDNO:5761); CCDS920.2, Ins, G_7, GLEGGAWRTPALRSLFDWLSVRHHFPGL, (SEQIDNO:5762); CCDS31898.1, Ins, G_7, GSGHREGAYCQQRDCRREVHALHPQVGL, (SEQIDNO:5763); CCDS4711.1, Del, G_7, GSQEWGWDPGVRAVPIPSPLSSRGTRKD, (SEQIDNO:5764); CCDS7694.1, Ins, G_7, GWEAATELHPSDTPMAGGVQGAGEPARV, (SEQIDNO:5765); CCDS 12444.1, Ins, G_7, LTAVGPAQSRPAPGPNADPGPAAAAAAQ, (SEQIDNO:5766); CCDS 13804.1, Del, G_7, RGQSAKSGSTASRASQPSSSVWSSAATT, (SEQIDNO:5767); CCDS12915.1, Ins, G_7, AGVREQCAAAASDIGCRGAAAGQRHST, (SEQIDNO:5768); CCDS 14409.1, Del, G_7, CSIKPPLPRRWTTVLREPWHGMQEIRP, (SEQIDNO:5769); CCDS 13160.1, Ins, G_7, GELLLRRRGGPHHMYQVPAQLGHLCLP, (SEQIDNO:5770); CCDS41561.1, Ins, G_7, GVCERPAPTRRGEAAHRGAGPPGCAAL, (SEQIDNO:5772); CCDS 12927.1, Del, G_7, LEPLGRPCPAPPAAASSVSPTTSPGTG, (SEQIDNO:5773); CCDS46200.1, Ins, G_7, PSTHGCCRPHGCPHLGPLGRPPCPSSC, (SEQIDNO:5774); CCDS12793.1, Ins, G_7, SGWAGDHGAASSASPRRALPVGSGGGD, (SEQIDNO:5775); CCDS31948.1, Del, G_7, SLSSCGWAPFSVGLHMGFSTPATSLHP, (SEQIDNO:5776); CCDS2420.1, Del, G_7, SLTLRLSETDTSSAFSSLLPLRNLSQL, (SEQIDNO:5777); CCDS9338.1, Del, G_7, STSSVTASCASTGSSTASAATSRGMWN, (SEQIDNO:5778); CCDS2891.1, Del, G_7, TSPQLETLKAITLLIEMDLFSDMSSTS, (SEQIDNO:5779); CCDS11129.1, Ins, G_7, ALCQGHVPGRGRLCWGDGTGSVGAHL, (SEQIDNO:5780); CCDS45937.1, Ins, G_7, ATEHGPGGRLPLPVAGQRHQRAQDCS, (SEQIDNO:5781); CCDS3846.1, Del, G_7, FCPLGCCVHPPPPSALLAMKLLTAAT, (SEQIDNO:5783); CCDS45604.1, Ins, G_7, GCGAGTARRGARLQRGLLQGLCEGCP, (SEQIDNO:5784); CCDS7030.1, Del, G_7, ISEPPATSSSPTGTWTPGQGAGFGGT, (SEQIDNO:5786); CCDS43015.1, Del, G_7, LPGSTRRAGGSQRPGRRSPLMSSPEN, (SEQIDNO:5787); CCDS30670.1, Ins, G_7, LPHRWQGWGDPLPQDAERQLHQSHGG, (SEQIDNO:5788); CCDS48016.1, Del, G_7, LPRSTACLSLSSPSFCPLSPAPPVTL, (SEQIDNO:5789); CCDS45638.1, Del, G_7, LTHCPWPTSLSCCGAKSSAAPPTKRP, (SEQIDNO:5790); CCDS47911.1, Del, G_7, NLPQKKDLLLLLQKDRPFCWIKAPLF, (SEQIDNO:5791); CCDS45072.1, Ins, G_7, PALRQRCQRMQPGERGLPPDLPQQAG, (SEQIDNO:5792); CCDS59086.1, Ins, G_7, PGPHTHLGILLYLSPFCSTCPEDQLP, (SEQIDNO:5793); CCDS33764.1, Del, G_7, PRAETAQACSTPSHPATFPSSTSSQH, (SEQIDNO:5794); CCDS33655.1, Ins, G_7, PRDPTDPQWHWPKMLVCPQGQPAILL, (SEQIDNO:5795); CCDS12698.1, Del, G_7, PTSSGCAVALSTSGRAAELAWAVLET, (SEQIDNO:5797); CCDS834.1, Del, G_7, PWCGHWTWMTSGARSVALALSPLSTY, (SEQIDNO:5798); CCDS4080.1, Del, G_7, QGACGRSSAMGCRADGWGRTTSPYCM, (SEQIDNO:5799); CCDS5332.1, Del, G_7, RNSSKFRDSATKGFSLLTSHLVKGTW, (SEQIDNO:5800); CCDS7940.1, Del, G_7, RRMIWTSLPNLPDSSSALTQRQTNAM, (SEQIDNO:5801); CCDS55698.1, Del, G_7, SRPELMPHTFSKSPSTSGICSPTWST, (SEQIDNO:5802); CCDS12712.2, Del, G_7, TFPVYQKSRNLQRDKIQETFAQKRIK, (SEQIDNO:5803); CCDS325.1, Ins, G_7, CPAHPQDPNPQVPQQEAALRPMPVL, (SEQIDNO:5804); CCDS55596.1, Ins, G_7, DAGARLQGCAPSLPAALPRGRHPLL, (SEQIDNO:5805); CCDS5689.1, Ins, G_7, GIYWFSHPNHPHSLPLSPGPAWTSI, (SEQIDNO:5807); CCDS31785.1, Ins, G_7, GRATCGAPRHARHYPAATGGRGLPV, (SEQIDNO:5808); CCDS7916.1, Ins, G_7, GRVPARRLPVPGAAHRFASQFQAHP, (SEQIDNO:5809); CCDS12793.1, Del, G_7, RVGWGSWGCLLCQPPASLARWLRRR, (SEQIDNO:5811); CCDS5194.1, Del, G_7, TSTPPCSSFGGWTSPNSSLTSPGIS, (SEQIDNO:5812); CCDS14729.1, Ins, G_7, WATDPCQGHRPRRGAAAPTVCVAGG, (SEQIDNO:5813); CCDS31598.1, Del, G_7, AWASYTTSRRATPCAPSPAATAKP, (SEQIDNO:5814); CCDS44155.1, Ins, G_7, GDVCGNAGPETSVAERELERDLLG, (SEQIDNO:5815); CCDS1089.1, Ins, G_7, GHSQPPLSLAITAASDSSSSANSV, (SEQIDNO:5817); CCDS32006.1, Ins, G_7, GRAGGEEQASHSALELVGFLPFVL, (SEQIDNO:5818); CCDS58996.1, Ins, G_7, GVQVHWARRQWPQGQWQVEPKVMH, (SEQIDNO:5819); CCDS5689.1, Del, G_7, HLLVFTPKPSSFSPSQPWPCLDLD, (SEQIDNO:5820); CCDS44442.1, Ins, G_7, LCHLRVWRQPHQPLLGDQRHTLLH, (SEQIDNO:5821); CCDS6578.1, Del, G_7, RHPPGSTRYPSFPLRLVPLSLIGP, (SEQIDNO:5822); CCDS44647.1, Del, G_7, RPASGAQAGAGRWPASASRWSLRR, (SEQIDNO:5823); CCDS9830.1, Ins, G_7, RRATSAVPPAVPGDLPLNLLCLEA, (SEQIDNO:5824); CCDS2442.1, Del, G_7, SAVCSPITTTPTVPQEVSLKILLA, (SEQIDNO:5825); CCDS325.1, Del, G_7, SGSSTRPKSPGPPAGSSTQAHASV, (SEQIDNO:5826); CCDS31785.1, Del, G_7, TGHLWSPSTCPTLPCCNWRARPPC, (SEQIDNO:5827); CCDS5194.1, Ins, G_7, TLRHPRAVPSAAGPLRTPHSHPQA, (SEQIDNO:5828); CCDS9548.1, Del, G_7, WQLAGPMLAGWRSLGSWFCRMYGI, (SEQIDNO:5829); CCDS33606.1, Ins, G_7, ADRGCGPGRRRAVQGSPERGLRP, (SEQIDNO:5830); CCDS14350.1, Ins, G_7, EPGNRFSGCTVEQFWWRGGPRNL, (SEQIDNO:5831); CCDS34219.1, Ins, G_7, GQWEREAGQPQCQRTKDTPKLCC, (SEQIDNO:5832); CCDS53798.1, Del, G_7, LAACSLGPCWYWNCIPRAPCATT, (SEQIDNO:5833); CCDS59157.1, Del, G_7, LAPAPVRTPRVLGEQVQGAPSPW, (SEQIDNO:5834); CCDS4748.1, Ins, G_7, PGIGEWGAWGPWRASRWRRPRWG, (SEQIDNO:5835); CCDS34420.1, Ins, G_7, RGLFSLPEPLGSRSRRRPDPSRP, (SEQIDNO:5837); CCDS10750.1, Del, G_7, RRRTRGARAWLTTTRTRRSIWRT, (SEQIDNO:5838); CCDS2445.1, Del, G_7, SVGCLGCPGSRLPRSAPSPMSMR, (SEQIDNO:5839); CCDS10488.1, Ins, G_7, VPGPQPLHGPLLNICPCHLSGAL, (SEQIDNO:5840); CCDS 14076.1, Ins, G_7, DAHQRRHPEHEQRTQGQGEGRL, (SEQIDNO:5841); CCDS54978.1, Ins, G_7, GEFLIPQYRQPRLQAGRTGNKE, (SEQIDNO:5842); CCDS44618.1, Ins, G_7, GLGGGDRACRLRVCQHTGPAGV, (SEQIDNO:5843); CCDS55911.1, Ins, G_7, GLLQPKPWQQQPVLVELLLRCL, (SEQIDNO:5844); CCDS30901.1, Ins, G_7, GPGYLLCGLCCPDVLFCLMGLY, (SEQIDNO:5845); CCDS9277.1, Ins, G_7, GPQPLGLERAVHLDSQTQPGVP, (SEQIDNO:5846); CCDS3066.1, Del, G_7, LWDPTNGSTLADEKMRLHGQSM, (SEQIDNO:5847); CCDS12326.1, Del, G_7, LWSQCQLKRMRQMTHQLASSPT, (SEQIDNO:5848); CCDS54060.1, Del, G_7, MASPRLHLRDWLLGWKQGKAWR, (SEQIDNO:5849); CCDS53360.1, Ins, G_7, PGPEFQHCPPEGGARDGVCPGK, (SEQIDNO:5850); CCDS46932.1, Del, G_7, PSFPLLFLASWLTSCYFFLEEK, (SEQIDNO:5851); CCDS43361.1, Del, G_7, RPPSPLSRPGSGPPTHRFQPLS, (SEQIDNO:5852); CCDS3607.1, Del, G_7, SHPSAAPRSSSRPPPHTASTIS, (SEQIDNO:5853); CCDS11662.1, Del, G_7, TAGTPAPPPVPTCSPRTLRCLP, (SEQIDNO:5854); CCDS10047.2, Ins, G_7, WRRRLGRWWGRGRRVGGRRRRW, (SEQIDNO:5855); CCDS43361.1, Ins, G 7, AGLRARSAARGAVRRPTASSP, (SEQIDNO:5856); CCDS33460.1, Del, G_7, AQAARIRAARETRSGRPGWGA, (SEQIDNO:5857); CCDS1001.1, Ins, G_7, GGRSGRGAQGGPHPGGEPREC, (SEQIDNO:5858); CCDS41237.1, Ins, G_7, GPLPRGHGAVPVSAGEDWGRL, (SEQIDNO:5859); CCDS47741.1, Ins, G_7, GWSNSCNVSRLHFRFEAKRNH, (SEQIDNO:5861); CCDS43015.1, Ins, G_7, HFQGVQGELGAARGLGGEAHS, (SEQIDNO:5862); CCDS53948.1, Del, G_7, HPNLSTPTSHTLILRLTCCPP, (SEQIDNO:5863); CCDS1924.1, Ins, G_7, LLLPLQDSGGPGARRAGGDHL, (SEQIDNO:5864); CCDS45604.1, Del, G_7, MWSRDCPSRCAAPTGPSTRAL, (SEQIDNO:5865); CCDS6025.1, Ins, G_7, PGAGAAGALRARLLPHAASQR, (SEQIDNO:5866); CCDS862.1, Del, G_7, RSGHLNVCLWVKWLRRSERCA, (SEQIDNO:5867); CCDS32172.1, Del, G_7, SFILSYRWLSLFDFLSVGPMS, (SEQIDNO:5868); CCDS53264.1, Del, G_7, TQGPCSCCPGYRPRASPAARS, (SEQIDNO:5869); CCDS5848.1, Del, G_7, TQGRARAPATATCLHPLTIGP, (SEQIDNO:5870); CCDS10904.1, Ins, G_7, VHPGLVLLLPLPLCCLWLSCV, (SEQIDNO:5871); CCDS10864.1, Del, G_7, WPRPCASWLQETTTGSQSSGP, (SEQIDNO:5872); CCDS42253.1, Del, G_7, WSEKPSQMQMTLAYSSRWTWM, (SEQIDNO:5873); CCDS7296.1, Ins, G_7, EEIQGCQTTDVQKVQTKGQR, (SEQIDNO:5874); CCDS44474.1, Ins, G_7, GAAGERTDPGQAAGVRAEHQ, (SEQIDNO:5875); CCDS13316.1, Del, G_7, GEWAALGPCWKSACRASRSA, (SEQIDNO:5876); CCDS4328.1, Ins, G_7, GPHRQPHLACQDERRTGQGH, (SEQIDNO:5877); CCDS11091.1, Ins, G_7, GRAHGEARGAGPRVQVRQWQ, (SEQIDNO:5878); CCDS34424.1, Ins, G_7, GRRARGGRRGWRRCELRLRP, (SEQIDNO:5879); CCDS32172.1, Ins, G_7, LHSFYHTGGSHCSTSFLWAQ, (SEQIDNO:5880); CCDS30852.1, Del, G_7, LLTGPAWSLTLSTRAPGCPC, (SEQIDNO:5881); CCDS5873.2, Del, G_7, PPWWQLWAPVWLMQSQRRME, (SEQIDNO:5882); CCDS4442.1, Del, G_7, SVLPSLSRFPGNQSQESSAR, (SEQIDNO:5883); CCDS10953.1, Del, G_7, TWGLLTLVYPWKCPCGWRLT, (SEQIDNO:5884); CCDS9472.1, Ins, G_7, AAAAGCGPHVLLVAAGQPG, (SEQIDNO:5885); CCDS11592.1, Ins, G_7, ALAPRRERAPCERQRLPQS, (SEQIDNO:5886); CCDS14350.1, Del, G_7, AWKSIFRLYSRAVLVERGP, (SEQIDNO:5887); CCDS8954.1, Del, G_7, CRGYTSCRCRAPRTSGRCG, (SEQIDNO:5888); CCDS6578.1, Ins, G_7, GDIPRGLPGIHLFPSGWYL, (SEQIDNO:5890); CCDS9889.1, Ins, G_7, GGSLCNHEQQVGRDQWGQV, (SEQIDNO:5891); CCDS7037.2, Ins, G_7, GHACLGRGTPYPATATAAP, (SEQIDNO:5892); CCDS2442.1, Ins, G_7, GQQSAPQSPPPPRSPRRSL, (SEQIDNO:5893); CCDS44195.1, Del, G_7, HVNPKERKASPRSWAAGSP, (SEQIDNO:5894); CCDS7644.1, Ins, G_7, PAAPGAVQQLQPPKRTAPW, (SEQIDNO:5895); CCDS37.1, Del, G_7, TSSSPAWTPPRPSRSSVRK, (SEQIDNO:5896); CCDS41741.1, Ins, G_7, AEAGAQGCRRGGHLPVGE, (SEQIDNO:5897); CCDS12519.1, Del, G_7, ARPSARSFCPSSSATGGA, (SEQIDNO:5898); CCDS32069.1, Ins, G_7, CFYYQSWPQSSILYTTLC, (SEQIDNO:5899); CCDS1719.1, Ins, G_7, ECQDHHGSHTGASFSQLR, (SEQIDNO:5900); CCDS13075.1, Del, G_7, GGMRMSSGRWWWSARTVS, (SEQIDNO:5901); CCDS31829.1, Ins, G_7, GLPHQVPENATAHHEPDL, (SEQIDNO:5902); CCDS9911.2, Ins, G_7, IPHLPEGRKSNRVQLMSV, (SEQIDNO:5903); CCDS 13149.1, Del, G_7, MAPSSAAADQDSGCWMTW, (SEQIDNO:5904); CCDS33646.1, Del, G_7, MFVLNPCLPSIMLTSHNM, (SEQIDNO:5905); CCDS3702.1, Del, G_7, RAPQKKVRSISLTFTEIP, (SEQIDNO:5906); CCDS48016.1, Ins, G_7, RCQGAQPAFLCPPPLSAP, (SEQIDNO:5907); CCDS 12604.2, Del, G_7, RMGVAAAWRVDSAAPVMG, (SEQIDNO:5908); CCDS45376.1, Del, G_7, TLQPRGSAGARPPAGLST, (SEQIDNO:5909); CCDS42817.1, Del, G_7, VAGEENEGPPRPSLMCPR, (SEQIDNO:5910); CCDS41865.1, Ins, G_7, VREEEGAEGPAFPAKVAG, (SEQIDNO:5911); CCDS41243.1, Ins, G_7, VVLPTPHRPDQGGGSGRR, (SEQIDNO:5912); CCDS7029.1, Ins, G_7, APECLPPVWGQPLPEES, (SEQIDNO:5913); CCDS1924.1, Del, G_7, APSSPRLGWSRSTAGGR, (SEQIDNO:5914); CCDS7163.1, Ins, G_7, DGGALGEAGRGRRKCAK, (SEQIDNO:5915); CCDS12804.1, Ins, G_7, DWCCAPDSVPDCGRLGV, (SEQIDNO:5916); CCDS55440.1, Del, G_7, FLPPVSWPRASACVPSP, (SEQIDNO:5917); CCDS9769.1, Del, G_7, FWINLASRVISTTPATA, (SEQIDNO:5918); CCDS37.1, Ins, G_7, GHLHHQRGRRHDLRGAL, (SEQIDNO:5919); CCDS5848.1, Ins, G_7, GLRGEPEPLLLPHVCTL, (SEQIDNO:5920); CCDS12645.1, Del, G_7, HCWCCCFWLGGPWPSSC, (SEQIDNO:5921); CCDS32169.1, Ins, G_7, LPAPSQAAADELDRRPG, (SEQIDNO:5922); CCDS44037.2, Del, G_7, LSPPDRLGLWLPRGPAT, (SEQIDNO:5923); CCDS32830.1, Del, G_7, LWDPSRRRTTTVPISTS, (SEQIDNO:5924); CCDS3846.1, Ins, G_7, PFALWDAVCILHHQVHC, (SEQIDNO:5925); CCDS13254.1, Del, G_7, PPMPMPGQREAPGRQEA, (SEQIDNO:5926); CCDS43789.1, Del, G_7, QLRLMLPSSARPWGPDG, (SEQIDNO:5927); CCDS11139.1, Ins, G_7, RALCLAPPLHPSLPAAL, (SEQIDNO:5928); CCDS5332.1, Ins, G_7, REIQANSEIQPPRGSAY, (SEQIDNO:5929); CCDS58996.1, Del, G_7, RPSPLGPPSMASRSVAG, (SEQIDNO:5930); CCDS34137.1, Del, G_7, RRGKPTRVLCWMWAASG, (SEQIDNO:5931); CCDS13048.1, Ins, G_7, APVRQAPYDFASYPIF, (SEQIDNO:5932); CCDS33367.1, Ins, G_7, CRCQSRLFETGHRFVL, (SEQIDNO:5933); CCDS45376.1, Ins, G_7, GPCSQGGALGPGLLPG, (SEQIDNO:5934); CCDS53970.1, Ins, G_7, GRLPLPPGTHPLLADL, (SEQIDNO:5935); CCDS58829.1, Ins, G_7, GTQHIHHGVDHRQDPF, (SEQIDNO:5936); CCDS13149.1, Ins, G_7, GWLLPLRLPTRIPAAG, (SEQIDNO:5937); CCDS22.1, Del, G_7, HPGDPLSGSWLPSPRN, (SEQIDNO:5938); CCDS31898.1, Del, G_7, IGSSRRCLLPTTGLPP, (SEQIDNO:5939); CCDS42175.1, Del, G_7, MARPGWPWPQTSVTSA, (SEQIDNO:5940); CCDS12604.2, Ins, G_7, PGWGWPLHGGWTQRPP, (SEQIDNO:5941); CCDS4748.1, Del, G_7, SREAEGSKTSGTFCSR, (SEQIDNO:5942); CCDS5780.1, Del, G_7, APMMSSMACGSAESS, (SEQIDNO:5943); CCDS8146.1, Ins, G_7, AWHRAGGGALPTADP, (SEQIDNO:5944); CCDS3819.1, Del, G_7, CRRAPGPRAWGGRAR, (SEQIDNO:5945); CCDS3066.1, Ins, G_7, GCGTPQTAAPWPTRR, (SEQIDNO:5946); CCDS12418.2, Ins, G_7, GCRGAVRCLDDKWTV, (SEQIDNO:5947); CCDS2164.1, Ins, G_7, GLCKCPRMPLQPPGP, (SEQIDNO:5948); CCDS13064.1, Ins, G_7, GRRGIGGGRGGGGGG, (SEQIDNO:5949); CCDS 13438.1, Ins, G_7, GSYPPGFLGGHLRCE, (SEQIDNO:5950); CCDS59423.1, Ins, G_7, HQGRPRHPGGAAPDT, (SEQIDNO:5951); CCDS44442.1, Del, G_7, LSPTCVEAASSALAG, (SEQIDNO:5952); CCDS46831.1, Del, G_7, MSWMSRASRKTLRKS, (SEQIDNO:5953); CCDS55562.1, Del, G_7, PPSTSPPRGWGLQVE, (SEQIDNO:5954); CCDS1947.1, Del, G_7, RLQCLGPRVWAAPEA, (SEQIDNO:5955); CCDS1001.1, Del, G_7, RPIGPGCARGTASWR, (SEQIDNO:5956); CCDS11129.1, Del, G_7, SLPRACPWAWKALLG, (SEQIDNO:5957); CCDS6762.1, Del, G_7, SPTCRMWWSRQSSGC, (SEQIDNO:5958); CCDS53455.1, Del, G_7, WHLREVGTHFWEAEG, (SEQIDNO:5959); CCDS11327.1, Del, G_7, WQAVGACPSMGSLGG, (SEQIDNO:5960); CCDS12933.1, Ins, G_7, ADPGPWQPSVGCAD, (SEQIDNO:5961); CCDS9196.1, Ins, G_7, AVGEHHGGGREAIF, (SEQIDNO:5962); CCDS42296.1, Ins, G_7, DRQRAGDHAGHSAG, (SEQIDNO:5963); CCDS3936.1, Ins, G_7, ELFQCTNFLFLKEK, (SEQIDNO:5964); CCDS31576.1, Ins, G_7, GHHHWTGVNHLSQW, (SEQIDNO:5965); CCDS2891.1, Ins, G_7, GLPHSSRPSRQLLY, (SEQIDNO:5966); CCDS8115.1, Del, G_7, GTGRSCSESYSGCT, (SEQIDNO:5967); CCDS10359.1, Del, G_7, LSSGSPSSAKTSHA, (SEQIDNO:5968); CCDS33606.1, Del, G_7, RSGLRPWQAARGPR, (SEQIDNO:5969); CCDS33469.1, Ins, G_7, SVWLPLGLPAGHCQ, (SEQIDNO:5970); CCDS1089.1, Del, G_7, TLPAPSVSCYYCCF, (SEQIDNO:5971); CCDS42817.1, Ins, G_7, TWRGRKTRDPPDPA, (SEQIDNO:5972); CCDS48074.1, Ins, G_7, AVCGFSLWCWRAV, (SEQIDNO:5973); CCDS5911.1, Ins, G_7, AVGGEPVQWPLQP, (SEQIDNO:5974); CCDS11065.1, Ins, G_7, ELTHSHDCSPEPC, (SEQIDNO:5975); CCDS13438.1, Del, G_7, FLPSRFPWGPPPL, (SEQIDNO:5976); CCDS42175.1, Ins, G_7, GWRGRAGPGPKRV, (SEQIDNO:5977); CCDS34424.1, Del, G_7, KESPGREKGMEKM, (SEQIDNO:5978); CCDS5313.1, Ins, G_7, PFLQPGPELLHTP, (SEQIDNO:5979); CCDS53455.1, Del, G_7, PLKGLGRLLGSST, (SEQIDNO:5980); CCDS 14699.1, Ins, G_7, QDHSPAREHPLPP, (SEQIDNO:5981); CCDS53970.1, Del, G_7, SSSTTARDPPATR, (SEQIDNO:5982); CCDS34710.1, Del, G_7, TQMPGPRPCWESA, (SEQIDNO:5983); CCDS10670.1, Del, G_7, WPPSASPRPHSSP, (SEQIDNO:5984); CCDS13742.1, Ins, G_7, AAGAEHQGGAGQ, (SEQIDNO:5985); CCDS11069.2, Del, G_7, ANQSWSLLRTMR, (SEQIDNO:5986); CCDS1942.2, Ins, G_7, ARGSWIPPFGYV, (SEQIDNO:5987); CCDS41590.1, Ins, G_7, ASRVPEAGQVPG, (SEQIDNO:5988); CCDS42992.1, Ins, G_7, AVGGGAWGDPRL, (SEQIDNO:5989); CCDS42512.1, Del, G_7, CSRLTGCWGQPS, (SEQIDNO:5990); CCDS46932.1, Ins, G_7, DPHSPCFFWLPG, (SEQIDNO:5991); CCDS8562.1, Ins, G_7, EDPLRVSAGAPG, (SEQIDNO:5992); CCDS8839.1, Ins, G_7, GHQGWIQHKCGL, (SEQIDNO:5993); CCDS2870.1, Ins, G_7, GPDVPHPGQRPL, (SEQIDNO:5994); CCDS918.1, Ins, G_7, GPQCPMCQGPLR, (SEQIDNO:5995); CCDS53948.1, Ins, G_7, GTPTSQPPQVTP, (SEQIDNO:5996); CCDS12421.2, Del, G_7, IPASMPPTNFPT, (SEQIDNO:5997); CCDS8146.1, Del, G_7, LAPCRGWCASYS, (SEQIDNO:5998); CCDS4216.1, Del, G_7, MRMMMRRTRTSC, (SEQIDNO:5999); CCDS5379.1, Ins, G_7, QQRSRKAIFQFF, (SEQIDNO:6000); CCDS12287.1, Del, G_7, RLRSWGRRRGAS, (SEQIDNO:6001); CCDS59423.1, Del, G_7, SGPPTPSWGGCT, (SEQIDNO:6002); CCDS4337.1, Del, G_7, VTHPLYCLIVGN, (SEQIDNO:6003); CCDS59086.1, Del, G_7, WASHSSRDSSVP, (SEQIDNO:6004); CCDS7048.1, Del, G_7, WPRASRWCTSTL, (SEQIDNO:6005); CCDS32498.1, Ins, G_7, AAQLLWALGGC, (SEQIDNO:6006); CCDS44729.1, Ins, G_7, AHFKRRVSIQL, (SEQIDNO:6007); CCDS6434.1, Ins, G_7, APCTTPPAWRS, (SEQIDNO:6008); CCDS45371.1, Ins, G_7, APHEAHYLHLQ, (SEQIDNO:6009); CCDS720.1, Ins, G_7, AQYLLRSKKED, (SEQIDNO:6010); CCDS10055.2, Ins, G_7, DGCDHTSTPPA, (SEQIDNO:6011); CCDS10750.1, Ins, G_7, GGGGHGGRGPG, (SEQIDNO:6012); CCDS34420.1, Del, G_7, GPILIAGASRE, (SEQIDNO:6013); CCDS32996.1, Ins, G_7, GPLAAETQASC, (SEQIDNO:6014); CCDS58850.1, Ins, G_7, GVRGCRGRQDL, (SEQIDNO:6015); CCDS736.1, Ins, G_7, HDCLANCHLLS, (SEQIDNO:6016); CCDS8031.1, Ins, G_7, HLAPTPLLFAG, (SEQIDNO:6017); CCDS3925.1, Ins, G_7, LAMPVHPGQRW, (SEQIDNO:6018); CCDS4711.1, Ins, G_7, LGARNGAGTPG, (SEQIDNO:6019); CCDS59442.1, Del, G_7, PAGSGSDTQRD, (SEQIDNO:6020); CCDS11417.1, Del, G_7, PGAGGIRLDLA, (SEQIDNO:6021); CCDS6097.2, Del, G_7, PPSAPMPSTSQ, (SEQIDNO:6022); CCDS1942.2, Del, G_7, PRVLDPPFWLR, (SEQIDNO:6023); CCDS11069.2, Ins, G_7, QPTRAGHSSGP, (SEQIDNO:6024); CCDS12915.1, Del, G_7, RSSRAVCGGRI, (SEQIDNO:6025); CCDS33679.1, Del, G_7, SSSKCTLAALR, (SEQIDNO:6026); CCDS4328.1, Del, G_7, TAQATTPRVPR, (SEQIDNO:6027); CCDS45572.1, Del, G_7, TWRPTWRPPTT, (SEQIDNO:6028); CCDS41781.2, Del, G_7, YGGQWPWAQTA, (SEQIDNO:6029); CCDS56184.1, Ins, G_7, ALSDMPGSLP, (SEQIDNO:6030); CCDS10389.1, Ins, G_7, EEPLHRVCGR, (SEQIDNO:6031); CCDS12131.1, Ins, G_7, GGQDCAPRAL, (SEQIDNO:6032); CCDS44438.1, Ins, G_7, GRAEETVTGV, (SEQIDNO:6033); CCDS12421.2, Del, G_7, LISSNPWKTQ, (SEQIDNO:6034); CCDS11486.1, Ins, G_7, LTPAGRPRSP, (SEQIDNO:6035); CCDS6402.1, Ins, G_7, PASCPAAACP, (SEQIDNO:6036); CCDS 11086.1, Del, G_7, PCWPRLWPMG, (SEQIDNO:6037); CCDS42253.1, Ins, G_7, PGPRSPHRCR, (SEQIDNO:6038); CCDS6876.1, Ins, G_7, PLLRGSALQP, (SEQIDNO:6039); CCDS10746.1, Del, G_7, PQRPLQICTC, (SEQIDNO:6040); CCDS12698.1, Ins, G_7, PRLRAGALWP, (SEQIDNO:6041); CCDS918.1, Del, G_7, PTMPHVPRAT, (SEQIDNO:6042); CCDS7494.1, Ins, G_7, RCAQTREKTL, (SEQIDNO:6043); CCDS3506.1, Del, G_7, CQYLRCGVK, (SEQIDNO:6046); CCDS14350.1, Ins, G_7, DPRILHARC, (SEQIDNO:6047); CCDS30852.1, Ins, G_7, DSSRAPPGA, (SEQIDNO:6048); CCDS5708.1, Del, G_7, FALEAWTPR, (SEQIDNO:6049); CCDS44752.1, Del, G_7, FSLPTTELC, (SEQIDNO:6050); CCDS55799.1, Del, G_7, FWLSLLYWP, (SEQIDNO:6051); CCDS32871.1, Ins, G_7, GCHSGGSAQ, (SEQIDNO:6053); CCDS56144.1, Ins, G_7, GCQDCEIHV, (SEQIDNO:6054); CCDS3324.1, Del, G_7, LRTGPCAKP, (SEQIDNO:6057); CCDS7494.1, Del, G_7, LRTNPGENP, (SEQIDNO:6058); CCDS32169.1, Del, G_7, PCPQSSGGG, (SEQIDNO:6059); CCDS34238.1, Del, G_7, PQWCGWVRY, (SEQIDNO:6060); CCDS12131.1, Del, G_7, RTGLRPPCP, (SEQIDNO:6061); CCDS13874.1, Del, G_7, TASRTYSVC, (SEQIDNO:6063); CCDS1825.1, Del, G_7, THLVAAPHI, (SEQIDNO:6064); CCDS3679.1, Del, G_7, TRNSAPRTR, (SEQIDNO:6065); CCDS14199.1, Ins, G_7, AQGTGGGD, (SEQIDNO:6066); CCDS780.2, Ins, G_7, AWFIWGQR, (SEQIDNO:6067); CCDS12398.1, Ins, G_7, DRECRGRR, (SEQIDNO:6068); CCDS9376.1, Del, G_7, ETLLLELS, (SEQIDNO:6069); CCDS2832.1, Del, G_7, GGSPRSTQ, (SEQIDNO:6070); CCDS3506.1, Ins, G_7, GVSTCAVE, (SEQIDNO:6071); CCDS54060.1, Ins, G_7, GWRLPDST, (SEQIDNO:6072); CCDS32879.1, Ins, G_7, LRLRQEAE, (SEQIDNO:6073); CCDS47741.1, Del, G_7, MEQFLQCI, (SEQIDNO:6074); CCDS12286.1, Del, G_7, MGFLTSIT, (SEQIDNO:6075); CCDS30639.1, Ins, G_7, RWPAPQAA, (SEQIDNO:6076); CCDS2885.1, Ins, G_7, TPHSKEPL, (SEQIDNO:6078); CCDS11532.1, Del, G_8, AWRARARPASTRPAMGSSRVPSTCTARPLSRTSPGCVPATPPRRRAPRSRGTRTWRPRVTSGS TPGCEAQELTANEAARPTPATRPWSWRKNFTTIAT, (SEQIDNO:6079); CCDS3665.2, Del, G_8, VAAAAAAAEAGAGVVGAAGNPPPPPPPPPPPPPRRSSPPAVAAAAAAAAAGPACTTETTPRG WGKLAARQSRRCKWQILISSPPYPLNTADLWRGNA, (SEQIDNO:6080); CCDS14315.3, Ins, G_8, GTCPECSPSAAPCTALRCPSGPALSQRPPGGPGRPLPPGAGGHSDGGGGGGGGRTVGGCPVP TAGCLRSPALFSVPCCLQGQQPPQQLQHGRGVL, (SEQIDNO:6081); CCDS 1069.1, Ins, G_8, GPGAGALPEARAEAARVHLPPRPRALPHCQRLPGRAELRVPAGWGRRGRGGGRARTASPPT GVGAWGSCPHPLQHHGVIPCCAPHLVGGV, (SEQIDNO:6082); CCDS53377.1, Ins, G_8, GRRPRAAGRPPAAGPAAAARGAPGRPDGGPRSADAPAPHRPQRGERRQALRSLQRGRAAPE GQQQHHGVCSRAHLRARGRDRGQARLQD, (SEQIDNO:6083); CCDS5040.1, Ins, G_8, GRGRRRGRRRRLPVVHQPPGPAGHQALGGGAAGRPRRRAARARRPAAAASAAATATAAAT AATAAAAAATAAAASGAPRR, (SEQIDNO:6084); CCDS3665.2, Ins, G_8, GWRRRRRRRRRGRGWWGRQEILLRRRLLLRLLLLGDPPRRRWRRRRRRRQQDQHAPPKRLP EAGESWLPARAVVANGKY, (SEQIDNO:6085); CCDS14729.1, Ins, G_8, GVPLAPGESHRGARRGQGAVQQPAGARASKGQGHSGNLPHPSAVHEGHAAEVCGRHLPGH SQREPAHPHRRQVPV, (SEQIDNO:6086); CCDS44063.1, Ins, G_8, GAVRWLPESQHHGSAPRLPGHSLRPALPGAKCRERGRDGRVDAASLPGCVQRGHPPGRSSQ PLHTLLPGPHG, (SEQIDNO:6087); CCDS10511.1, Ins, G_8, GHGPWAARPRPASQSGQEPAAALALWGWRTGLACGLPPCWRSWWQGRGGEGAWVGAHQ GPGHGGPQDAP, (SEQIDNO:6088); CCDS1076.1, Ins, G_8, GGRHEASGRSRSRGCSTHCTQCPDPQPLGSYIACRTACWGRSRSPQTDATSTTLSSRQRAF, (SEQIDNO:6089); CCDS30852.1, Del, G_8, VAPRCPRSAVDISPGPTSPSLASCASVAIGKLALLGASAPCGAAGAREAGRFAVLGTMVMK, (SEQIDNO:6090); CCDS44234.1, Ins, G_8, GGRHEASGRSRSRGCSTHCTQCPDPQPLGSYIACRTACWGRSRSPQTDATSTTLSRQRAF, (SEQIDNO:6091); CCDS30852.1, Ins, G_8, GWLLGAPGALWTSPPAQHHPVLPAAPLWPLGSWLSLEPVLRAVRPGPEKPAGSLCWEQW, (SEQIDNO:6093); CCDS1953.1, Ins, G_8, GPCSGELCARPCLRGIQWPEEATTVEASGGGPCPSKGRRPPWRGPGRSPEGQHMGHSL, (SEQIDNO:6094); CCDS45544.1, Ins, G_8, AAALRVPRRAEAGSAGTEGPGPRGHTAQQTAQGGHARQCPWARRGQASSPGKQPHPE, (SEQIDNO:6095); CCDS13201.1, Del, G_8, VARVEVAAGPPMREVAEAAAVVMVVRPVATLSPGEARAPLESVRQIYSEHNYCRLIL, (SEQIDNO:6096); CCDS33088.1, Ins, G_8, VPAGEASVRAASAEVGDGAGGPVRPCALLLLLPLEILVFLSPTLRLLVPGRGDPILR, (SEQIDNO:6097); CCDS8.1, Ins, G_8, GPGQAGKGRSSARPGGPPVDKPRIPQATEGPENGRVFPRDLPPGPQTRERGLFHLV, (SEQIDNO:6098); CCDS8077.1, Del, G_8, PGEEEPAMCTSQQKARRSLWRPSKAMSSSATTCHTTPSRAALMRSHWPSAPCNATA, (SEQIDNO:6099); CCDS9962.1, Del, G_8, TGTGSGCSCVGPPAALSPSRVPGTPWSRAPWRPPRKCILPPASAPSRPFRGLVARG, (SEQIDNO:6100); CCDS45544.1, Del, G_8, GSPSRSAACGGRERRDRRPGPSRAHCPANGAGWPCPAVPLGPARTGLLPGEAAPS, (SEQIDNO:6102); CCDS6895.1, Del, G_8, RPPCPPGRGLPLTLSALPLRCPRAPTAPRPGSPADRVRQVHPVLRAPGPPSTS, (SEQIDNO:6103); CCDS4774.1, Del, G_8, AALTSSAISGPGWTPRPPTFLSPWPSPAIPLLTPRMSWCSSLTTWPNS, (SEQIDNO:6104); CCDS34709.1, Del, G_8, VGAQLMAPLSPGATLTSTSCSTMSCLSRPGWAALQRLTWVCRRP, (SEQIDNO:6105); CCDS14315.3, Del, G_8, DLPRMLPISSPLHRPQMPFWTCTFPATTWGTWETPSTWGWRTF, (SEQIDNO:6106); CCDS42560.1, Ins, G 8, GPPGALRHPRGTPPGPENNLGVYTEARLGLQQGESHLSPLEPG, (SEQIDNO:6107); CCDS53999.1, Del, G_8, PADPWGLGVQPACSELPCRWRRPSWSPACWGSCCCPSWPC, (SEQIDNO:6108); CCDS43882.1, Del, G_8, RPPCPPGRGLPLTLSALPLRCPRAPTAPRPGSPESLSVTP, (SEQIDNO:6109); CCDS42894.1, Del, G_8, TRGTGWPLAGQQPGSCPSQHQGLPGRLPPPRPLQRVRHT, (SEQIDNO:6110); CCDS 14752.1, Ins, G_8, GPGQQALPCLRGDRHHAYPPGCAPGDWGGVRGRSEPSL, (SEQIDNO:6111); CCDS 11765.1, Ins, G 8, GPVPGHGGPGGKALPHRQDPCGQNPGGPGGRVLEPLPD, (SEQIDNO:6112); CCDS11765.1, Del, G_8, ACTGTWGPRGEGPPSSPGSLWPESWGTRRKSPGAPP, (SEQIDNO:6114); CCDS11672.1, Ins, G 8, ARRRGRQDGGRGRRRPPGPDHRGPEGRQQSGVR, (SEQIDNO:6115); CCDS12742.1, Ins, G_8, GGTRAGPGPGASGCVHQEAERVSQAHRGRTGQ, (SEQIDNO:6116); CCDS12424.1, Ins, G 8, GLRPQEGPGRHPFRRRAAGGCGPSRVRGEAEL, (SEQIDNO:6117); CCDS8.1, Del, G 8, SRPGWKRTQQRPPWRTSRGQAQDTSGHRGS, (SEQIDNO:6119); CCDS11532.1, Ins, G_8, GHGGPERGRRLRGRLWARAEFLQHALRAL, (SEQIDNO:6120); CCDS8947.1, Ins, G_8, AQRGSLCLSPCDPKSAGKMRLLLRPGGT, (SEQIDNO:6122); CCDS8077.1, Ins, G_8, GRERRSRRCAPANKRQGGVCGDLQRQ, (SEQIDNO:6123); CCDS5273.1, Del, G 8, TLAIRFISAPQPSSSTVTAAPSGQYF, (SEQIDNO:6124); CCDS54834.1, Ins, G_8, GLLHAVQEKLEEALVCPPPVQADVL, (SEQIDNO:6125); CCDS12037.1, Ins, G_8, APARRPDAAVPPVRPLPARPGLL, (SEQIDNO:6126); CCDS8950.1, Del, G_8, ASPSPSRNKSELLTSPRPLTLQS, (SEQIDNO:6127); CCDS42560.1, Del, G_8, PARGTTSSTGNASGTRKQPWSVH, (SEQIDNO:6128); CCDS42594.1, Ins, G_8, GRVAHSPASCCARARATATGGL, (SEQIDNO:6129); CCDS12037.1, Del, G_8, PGQAAGRGGTTCPPTSCPSWSP, (SEQIDNO:6130); CCDS54834.1, Del, G_8, APPRCPGEIGRSAGLSSASPS, (SEQIDNO:6132); CCDS42594.1, Del, G_8, PCCSQPCVLLRQGPRHRHRRP, (SEQIDNO:6133); CCDS10524.1, Del, G_8, AFWKPPSQGISSCLMTGCW, (SEQIDNO:6134); CCDS14544.1, Del, G_8, AKAQEVGKDPMVMVNVENL, (SEQIDNO:6135); CCDS12742.1, Del, G_8, RHPSWPWTRCLRMRPPRS, (SEQIDNO:6136); CCDS44327.1, Del, G_8, VLQVHCLVTVTGYSLCLR, (SEQIDNO:6137); CCDS47669.1, Ins, G_8, GCPGHLQEECAESHRAG, (SEQIDNO:6138); CCDS42144.1, Ins, G_8, GRPPCPGRRPPATAHLL, (SEQIDNO:6139); CCDS9962.1, Ins, G_8, GRGQARGAAVWDRQQP, (SEQIDNO:6140); CCDS42144.1, Del, G_8, PPPLSRETPPSHSPPA, (SEQIDNO:6141); CCDS34709.1, Ins, G_8, GWGPNLWPPSALGPP, (SEQIDNO:6142); CCDS33088.1, Del, G_8, PCRRSQCTSCKCRSR, (SEQIDNO:6143); CCDS10524.1, Ins, G_8, ALSGSPRLRESAAV, (SEQIDNO:6144); CCDS8950.1, Ins, G_8, GPPPPLPETSPSY, (SEQIDNO:6145); CCDS2463.1, Ins, G_8, APPPRQLHPPHG, (SEQIDNO:6146); CCDS8944.1, Del, G_8, ELLHPSQRLLVP, (SEQIDNO:6147); CCDS43882.1, Ins, G_8, GAPRALQAGGFP, (SEQIDNO:6148); CCDS14544.1, Ins, G_8, GQRLRKWERIRW, (SEQIDNO:6149); CCDS13201.1, Ins, G_8, GWPGWRWRRGHR, (SEQIDNO:6150); CCDS42395.1, Del, G_8, KKWGPWSLKSKG, (SEQIDNO:6151); CCDS14752.1, Del, G_8, PWATGPSVPSW, (SEQIDNO:6152); CCDS12340.1, Ins, G_8, AECRGGEPGM, (SEQIDNO:6153); CCDS5693.1, Del, G_8, AGARGEIAVM, (SEQIDNO:6154); CCDS8944.1, Ins, G_8, GSSSTPLRGF, (SEQIDNO:6155); CCDS8957.1, Del, G_8, MLPSLTCPWP, (SEQIDNO:6156); CCDS8947.1, Del, G_8, TKGVAVPVPV, (SEQIDNO:6158); CCDS44327.1, Ins, G_8, GFYKCTAW, (SEQIDNO:6159); CCDS42894.1, Ins, G_8, GQGGQDGH, (SEQIDNO:6160); CCDS42395.1, Ins, G_8, GRSGAPGA, (SEQIDNO:6161); CCDS7472.1, Del, G_9, ACGRPRWRCCWGRCTGRRRAARSTTTMAGRPSRCTAAPTPSRRSALTSLPTCRSATRWATS ACGCPTCWSTRAWPK, (SEQIDNO:6162); CCDS6426.1, Ins, G_9, GDAGCGRPPEPAGGGQAAAATEGGCLSLGLGCRQLGLLPGPAYTAPPVGV, (SEQIDNO:6163); CCDS32912.1, Del, G_9, ARPARPCPTGHSLVQMEPLTTARPTSSSTTCPRT, (SEQIDNO:6165); CCDS6802.1, Ins, G_9, GVSLLLDLSLVCLSPGLHPRSS, (SEQIDNO:6166); CCDS32912.1, Ins, G_9, GPGRPGPAQRATPWYKWSH, (SEQIDNO:6167); CCDS8024.1, Ins, G_9, GAPGRRPRASPGFAWESG, (SEQIDNO:6168); CCDS9603.1, Ins, G_9, GRRTGRGTSWL, (SEQIDNO:6169); CCDS6426.1, Del, G_9, RCWVWQAS, (SEQIDNO:6171); CCDS31397.1, Del, T_10, LAMLSPSLTSLPAPPLYPMHSASSGSVSKKLTSMLAWPRCSLFMGSQVWSLGCSCSWL, (SEQIDNO:6172); CCDS3504.1, Del, T_10, WDEKTVRSNVMANVLTLNLCNRLLKSFSKWSLVQLHGIIRKN, (SEQIDNO:6174); CCDS2119.1, Ins, T_10, FLLCVVFGKFVISRSTFRHTSCYS, (SEQIDNO:6175); CCDS2119.1, Del, T_10, FALCGFWQICHIKKHFQTHKLL, (SEQIDNO:6176); CCDS12317.1, Ins, T_10, FHFIWMPGQFTLDRDGL, (SEQIDNO:6177); CCDS3504.1, Ins, T_10, FGTKRQSDQTSWQTS, (SEQIDNO:6178); CCDS58774.1, Ins, T_10, FCVCLCVCVFYFTG, (SEQIDNO:6180); CCDS59050.1, Del, T_10, FLQKQITLLKMKK, (SEQIDNO:6181); CCDS12317.1, Del, T_10, SLHLDAWTIYS, (SEQIDNO:6182); CCDS31397.1, Ins, T_10, FWPCSLPH, (SEQIDNO:6183); CCDS45955.1, Del, T_11, FSSKGVRATQSHRISQVSQNSSSWDSQRIQNCSRSSLGCSCPCTWSRCWGTCSSSWLSALTPT STPPCTSSSPTCPWLTSVSPPPRSPR, (SEQIDNO:6184); CCDS45955.1, Ins, T_11, FFPQKVSELHRATESHRCLRIPPPGTLRGSRTAAGPRWAVPVHVPGHGAGEPAHHPGCQL, (SEQIDNO:6185); CCDS58832.1, Del, T_13, FLRWSLTLSPSWSAVVQSRLTPGFK, (SEQIDNO:6186); CCDS45501.1, Ins, T_13, FFSVIFSTRCL, (SEQIDNO:6187); CCDS45501.1, Del, T_13, FFCYILNTMF, (SEQIDNO:6188); CCDS53350.1, Del, T_7, ASGELTCHGCLCFVPFCWSSSFSLWFPIGFFTGSAFWTLGTGITRALCNMQSPLWMPSSSSITW PSSCWSSGSCSPCSRCRWSAPPMASPASTAWDT, (SEQIDNO:6189); CCDS48169.1, Ins, T_7, AEVSVYEAERASGGQAKASGTTQISEGKAPSDPFPEPGSGPEPESESESESEPKSECQSEPDSES DAESDSEFEPEGEPGKPEAELRQGAE, (SEQIDNO:6190); CCDS11266.1, Del, T_7, QRQRQLQQRSSHSLPYSHHQCHRYWLILKTQQGILWVNQHNSATLFLDNQYHLPKSLLDNQ HQLSIPLLKNQKHILLLDNHLPTTPNNQHQ, (SEQIDNO:6191); CCDS3793.1, Del, T_7, YGTLYPKSSIFKKLLPIPLALERLLILLQVTKTSALWNLSGTKVFISTIALCWTVSFLESHVAQR ILHMSSLNMEIVLLLIMVKLSKQREK, (SEQIDNO:6192); CCDS30955.1, Del, T_7, MTFFGETGMMKKVVRIILHLLKKELICGVIYRMEQYLVLSHNVLKKLWRNIKTNVSSSLSIRA ILKKIHLQQRLLNAGKNTMR, (SEQIDNO:6193); CCDS35089.1, Ins, T_7, LLYVHPLHHLANEFSHRKTQGIFHVLSSPDCGDHILRYHLLYVCETEVSRPDWGRKIASIRQA HFSVLWGSDTHAESYTL, (SEQIDNO:6194); CCDS47187.1, Del, T_7, MKIFKLQLTSSNFPLLFCHQCPRPLRCHHLTRVPYRLHLHLKPTLENTQIPAIMTQSSLEILLSV FQKMIQLNHRIILAH, (SEQIDNO:6195); CCDS5370.1, Del, T_7, PLHLSACKTTGEVPPRSSGQPGCFHLFLDWAKVKTIDVHLQMQHPVPGALRWVQNVDGVFK RISFQVDQEVNVVILFPI, (SEQIDNO:6196); CCDS7831.1, Ins, T_7, MCGSLCFQPGPAGQDPLWIPEDAADQAVRDHPAHSAGLPPLRPALRHSGGPNLQDAPEFGSL ILSCLSGLHVPVLSKQ, (SEQIDNO:6197); CCDS32595.1, Del, T_7, GSRMSVLSFSNTSKQWKLITCGFPANSLAMIFHTMWHCALEKCVSERTYRKFLTCLPHSPRA LGWIKRNCPSAHPY, (SEQIDNO:6199); CCDS2035.1, Ins, T_7, HQPCYPDRHIYWIFSWKYLMVGCSWLLYLCNFPGIQCIAIFEKYSNSSVSICTSDSALRAFPGT GMELHPYSLFFL, (SEQIDNO:6200); CCDS2300.1, Del, T_7, VAVACFGILVIWARVSASLQLAPKHLDWQISGKKFQHLDFSELILSLLSFPDALFYNCILGNIF LGRILLKKMLIA, (SEQIDNO:6204); CCDS13643.1, Ins, T_7, WQAADGDGSSHCSRDIESHCRENRFLSEGGSCALESAEQRDPGASRHLRRIVLPAALPGPYG QHLRGHHLPCPPGP, (SEQIDNO:6205); CCDS5104.1, Del, T_7, LIQFWDWKIQKTPWFIHGLVRESVLTALLQFQVLKQSQNYLWTTNLQDSLQAIQKQLATIQIL HWSYQVMRH, (SEQIDNO:6206); CCDS57972.1, Del, T_7, VPMIPTMTWPHSSSTRRSLPSSSSSTRMVLSAPSLWPCSSFSISCLHVGLTAFLFQVAFLCLLCC VELLLDV, (SEQIDNO:6207); CCDS55189.1, Ins, T_7, QVQKRLPYSRFHDSHLPCQFNMEWDTDRMYTSCLQTARNPGTRGCESHRSSYFRLHLSVHL PSRLFPRRGI, (SEQIDNO:6208); CCDS55741.1, Del, T_7, IYCTFYLSSQRGKQLQRMGNAILPCLETPTGKLVTLKRLTQFMTFQTVSSVGKKQSWWITLT QKNFLLKK, (SEQIDNO:6209); CCDS31915.1, Del, T_7, QTIQPPLLLCLRFRESLWMETKHPDLECRHRYKVKSLQQTQKMMMTTMMRMMMMKKKTQ RIGTPGSPRRE, (SEQIDNO:6210); CCDS43483.1, Del, T_7, RKFPLKKLMADLLPVQEPMKRASKEVVGKRKTKLKLYHLNLKYNLEANKNFSFLMKKKKL EKTTPSSLAQ, (SEQIDNO:6211); CCDS389.1, Del, T_7, IKQLIKLKKHYIPIYHKPQKQTLGIPATHLPIKNPLVRNWGIPLHQILELKKNLWMMSMTKQW HHSRDY, (SEQIDNO:6212); CCDS4822.1, Del, T_7, KALTGNISERDLLQYLLKSKALMIPQTSMSFQNLIFLSQQWPQVIILRLTTRTKTGSSSITRTSA LRA, (SEQIDNO:6213); CCDS58048.1, Del, T_7, PFCTAGSMPLLRCYALVTGCSIRIGGTPRHTPTIIEPGMWWSMTGYITMLTRTFSGFSPRDSNL LPC, (SEQIDNO:6215); CCDS7113.1, Del, T_7, LTCILEKEPVGGPSTSPKAKSFSSTSLSLKLEVLPTVKQIMLRLVAVPFWVLLKIKSIAVQTYLH L, (SEQIDNO:6216); CCDS5843.1, Ins, T_7, FQCCCHLWYGYCSLLFASDHHDCAILAHIPSQQEQDKEGQEGACCQPRPRFSKSGTRKDSEA KQ, (SEQIDNO:6217); CCDS5108.1, Del, T_7, KTRGQILFPCLRLLPGRLLQSCILSTKASNTIAPSTEWWTMVCNLALRMMRRQWSLWVLPTI C, (SEQIDNO:6218); CCDS41446.1, Del, T_7, MMKTEQFQVLQLLWCHIVLMDLLKQFIRRRLLVSLDSGLGHLKICHKQVLVTLILASLLLKE V, (SEQIDNO:6219); CCDS6160.1, Del, T_7, LGLPANLRFVLIILWNFSVPGKQVFMILKGSHLALLNRYLVVHLCCRNSRRKDKISVMLVL, (SEQIDNO:6220); CCDS7450.1, Ins, T_7, CYSSSKSCWYNTWPKSVRSAQLSLSCQCCASSYTGEKMVHGACGYCLPGWNFTFWFNLY, (SEQIDNO:6222); CCDS7847.1, Ins, T_7, IHGSLWVQSGPAGQDPLWLQGSATDQAVPDHPAHSAGVPPLRPALWHSVVPNIMDLEGF, (SEQIDNO:6224); CCDS4935.1, Del, T_7, MQATLCARQETWPQDPTTCQFFIQEMGMLVLVMFPDTSTLCPKCFIIFLRISAYMVEAS, (SEQIDNO:6225); CCDS59415.1, Ins, T_7, FLNPFRRVGLGHLHWSHRQLPGPPLWGLALFLVLHPGLGGSAHDVLRRDFLHVRLPGA, (SEQIDNO:6226); CCDS4872.1, Ins, T_7, PSRLRPAAGTRGAGAAAGGLGRAAPGAAAQEAFRFQDPGKGCCFVYQSRRGSWHPPGS, (SEQIDNO:6227); CCDS10129.2, Ins, T_7, PSSYWDSCWCQYLRRFGGSPRCHPQRNHAGHFHHHCCLLRGCNLCRGLCGPRCHREHE, (SEQIDNO:6228); CCDS4658.1, Ins, T_7, SGKPFLSGYFLFFSDTAQAARKPRVQSQGYIFSRLYHPATLLPLFGKHRGHFTGYHGL, (SEQIDNO:6229); CCDS6649.1, Del, T_7, CSFLPTLYSTDVLKLININFILGSISLAITGDQLIRFDPSFSLMLTPTVFLLVLTFL, (SEQIDNO:6231); CCDS55504.1, Ins, T_7, SKSWVYPSIRFSWNSNFLFRYWVNNVWLCNADEGNGTTCRRFLLYRLPTVWCHCISN, (SEQIDNO:6232); CCDS42173.1, Ins, T_7, GDRRRAPQHESSDRCALHGPVQGHQSPVQLLQPQVPPACQPALLDLPGLSDDSPTQ, (SEQIDNO:6233); CCDS31098.1, Ins, T_7, SDTGALCFHCVLHIENPFHLWPEKDLFHMWLPPGCCHSLLRDHDLHVCVSQSPPVA, (SEQIDNO:6234); CCDS3569.1, Del, T_7, HEGPESILGFQTDLSSLQGVTTVFHSSHFIGHTVTLLIGISPEEDSREEAHLRKA, (SEQIDNO:6235); CCDS2368.1, Ins, T_7, PDSDQPGPLYPLDPSCLISSASNPQELSDCHYIHLAFGFSNWRSCPVLPGHCGVQ, (SEQIDNO:6236); CCDS34081.1, Del, T_7, GKLFRRSKFEAKPTSGPWQPGRNITTKLIQRPLRSSLGSRKTPPSLPFLGEEEL, (SEQIDNO:6238); CCDS56396.1, Del, T_7, QNSSFSGNWWETQDPKREIMSLQGLKLKKTLPPLHTESDPLAKGGHLLMKATDP, (SEQIDNO:6239); CCDS6491.3, Del, T_7, KLLGNFLCLSTNFLCLDHILFPLKSTFHILCKTSLFLHHKDRESLSSCQSMMH, (SEQIDNO:6240); CCDS 12954.1, Del, T_7, TLESALEKGFMNLANVGKPAAVSAPLFSCKESTLEKGLMSAVNVGNLLAKPLI, (SEQIDNO:6241); CCDS1476.1, Del, T_7, GVRAVLWLRGEFLLQMQSTVQSFLQWTIAYLSQRRWKDRFWGLTFVSRATTW, (SEQIDNO:6242); CCDS7179.1, Del, T_7, KIMFGICIREFWSQKNKYLLCIIGTGKNTARVQTIWTAYIGISTPSLLKRIN, (SEQIDNO:6243); CCDS528.1, Del, T_7, TMVPYELQEILGTSPPCSAMILWTLVSLREHMLESQGTHLVGIALPEVMMA, (SEQIDNO:6245); CCDS7393.1, Del, T_7, VTLLYVNTVTLAPNVNMESSRNAHSPATPSAKRKDPDLTWGGFVFFFCQFH, (SEQIDNO:6246); CCDS31815.1, Del, T_7, WPFIQSLCWATFLLSQLPVWISHFKHPCTSFFKICHFLKYVSPWLWCQKCL, (SEQIDNO:6247); CCDS31816.1, Ins, T_7, HLHVEHHWESNHHHSHPTGFASQDTHVFLPPKFLIFRSLIHNCLHSQISC, (SEQIDNO:6248); CCDS6195.1, Del, T_7, NHSAGLTLYKRRFHHHLILMWLDQMISETLTQHLQKKQFHILCVYLLTIL, (SEQIDNO:6251); CCDS43455.1, Del, T 7, IMPFRWIPACLSLIPSLPRDLRLPGISGLSTLPRSPWTLREQKTSKAIP, (SEQIDNO:6253); CCDS1285.1, Del, T_7, PTPFCTTLRNFFLSSLILAVLCTNCIFRYTASFLQAFMLPGFLKHTKRK, (SEQIDNO:6254); CCDS9303.1, Ins, T_7, SASRQTTSGLPFHYPWRQTKGRWRLSTPSCSSYAESSPFLKEFSNFANS, (SEQIDNO:6255); CCDS44470.1, Ins, T_7, DNTRFPYVCLSLCPVSCPCVKVAVSDDVSSYRLYCVSADFKYIDGNNN, (SEQIDNO:6256); CCDS7113.1, Del, T_7, RMDPGHMAASLLPIPPVKMQCVVGLFQILLKETLLLLAMTESGITQET, (SEQIDNO:6257); CCDS55171.1, Del, T_7, CPRYAKVIRERVGSRFVTSSHRVQDSTSVTTSPEGTVVFPTASGPIT, (SEQIDNO:6259); CCDS7480.1, Ins, T_7, LLYRGCVAHWWPLPWANICQGCSGLLWHGLVGTNSEIQEWPLFITCR, (SEQIDNO:6260); CCDS9701.1, Del, T_7, MVRESGHLEIVHMKTTDIMNTTMISSKECHRIRGGMLQVLGELLLHL, (SEQIDNO:6261); CCDS9489.1, Del, T_7, PSFTWLLMLEVCFPQSSHPCSEFNNVEFTVNKLVTHWPLGFLLLSWL, (SEQIDNO:6262); CCDS9607.1, Del, T_7, RRSSPCSSTCSMPRTEPPAPEMIPSSALRSAPWARLCHKPRSLSHYC, (SEQIDNO:6263); CCDS35088.1, Ins, T_7, LLYVHPLHHLANELGHRKTQGIFYMLSSPDCGDHILWYHLLYVCKT, (SEQIDNO:6264); CCDS14587.1, Del, T_7, LMIRVCILICMPEHTSTLKTKRTLFCSGIALMVMYSLTIKVRNIPL, (SEQIDNO:6265); CCDS4844.1, Ins, T_7, RRHQPNDCRDSYEPSFNPFPLHFHHSFVKQGTRICNCTTCQTPSG, (SEQIDNO:6267); CCDS46694.1, Ins, T_7, YLVYFSPGSGHGCTLPRSKQLSRLLRLSGKTNVPGVWMRRLPQVH, (SEQIDNO:6268); CCDS11622.2, Ins, T_7, KSYFGPFELLGSILDCWNYRLHSEILFHGLKMPYFIGAFFHHAF, (SEQIDNO:6270); CCDS2226.1, Ins, T 7, LYNSFGFNAKRSKCSIFQRGIKDGKEHEKQKQDVGFNARSCKQY, (SEQIDNO:6271); CCDS6216.1, Del, T_7, PPCFLPYLLKLRSSIGSATHPMQVFHTPTVIKCNTQFQLMLTPV, (SEQIDNO:6273); CCDS 11160.1, Ins, T_7, PSHRARASRARQLPRRSGLRGPARGTSGAPSRAVLGGCSTRPGQ, (SEQIDNO:6274); CCDS46495.1, Del, T_7, QEVAWVLKPLLPIQVCWSWQCAQKENMQRLCFLTTVSVIPSGTT, (SEQIDNO:6275); CCDS4016.1, Del, T_7, VMPLHHGLTQKMISETCSVRSFMDLKIKLAMKIGGVSLTSFLFP, (SEQIDNO:6277); CCDS35063.1, Del, T_7, WSGLNIVSARLNISRKSTQMPCTSRRGPPPAPWGSAAPAGQGLN, (SEQIDNO:6278); CCDS5363.1, Ins, T_7, LYDYSVTCIYCSVFCILRLFSPEPGATGSASGGWLEQYGKCSK, (SEQIDNO:6279); CCDS47554.1, Del, T_7, PHYLVVDLGWTVTPFGMSYTRPVLHAWLLAVSSSWLPKWPQES, (SEQIDNO:6280); CCDS7926.1, Del, T 7, ANMDAQPMMPTPNIIAELPRCTGRRSGSWGVRPWLGMALIFG, (SEQIDNO:6282); CCDS41444.1, Ins, T_7, AWLESDLYYAEAFGNESTFWKPSVSDSTPAFSDSSRGTAQSV, (SEQIDNO:6283); CCDS861.1, Del, T 7, CVWPHWGLKPIRNTAMFQYYLQNRLIYILRWKKSQIRKNSLL, (SEQIDNO:6284); CCDS3646.1, Ins, T 7, FCVPGNYFLPGAHCRSSSICFQRLDQRPAVFLYKQQHQSISG, (SEQIDNO:6285); CCDS10315.1, Del, T_7, KGAGATVELLFMSSTPNQAQSSILTGLTPIDPRKRVNVWSSI, (SEQIDNO:6287); CCDS59415.1, Del, T_7, PQPFSSCWPWPSTLESPSASWAAALGTGAFPGPTSWAGWQCS, (SEQIDNO:6289); CCDS45880.1, Del, T_7, CLRLHMQMDWYMTQSWGAKPTMACSRWQDLGDSILYIQHLT, (SEQIDNO:6290); CCDS7421.1, Del, T_7, EQRNTLKKMNTASFSVSMQEVQMPLLVESIPITILMFLMNT, (SEQIDNO:6291); CCDS13515.1, Ins, T_7, FGESRGPLGQPLGGPQLSEQRSGPLPKQQHRPELLGDLWKC, (SEQIDNO:6292); CCDS58279.1, Del, T_7, FSEGTMNVPASTEFMDFMMNVKEDTTLNYGKLSQTVLTVYR, (SEQIDNO:6293); CCDS2171.1, Del, T_7, FTSVRLKIGVLIYLGEQKIPTKKLIIIHWRKFVQILIFSTV, (SEQIDNO:6294); CCDS32205.1, Ins, T 7, HGDVYCICPVWCSVAGMVCLLLERSPENSVLDWCCHLPGNA, (SEQIDNO:6295); CCDS45841.1, Del, T_7, LTWPSLTSLWFQSLGRMKVVNVNLDFFRNGTSLPSHHSWNS, (SEQIDNO:6296); CCDS14521.1, Ins, T_7, RFCVLFYYYGGNCGLFYLLFCSKATECKSSKQEAEAIKGRC, (SEQIDNO:6297); CCDS34732.1, Del, T_7, TLGGSVTQKLKMTATGVPVIPPLVISWWAGTRSLWLLLPVG, (SEQIDNO:6298); CCDS47723.2, Ins, T_7, KLPVFNATAVRRRHYYTVALLGGGLTASRGGLGHKCRQIH, (SEQIDNO:6299); CCDS14127.1, Ins, T_7, QPSLPSSPNPDPFLGLPSLLPAPELLHDEELRDHSAAHVL, (SEQIDNO:6300); CCDS 1790.1, Del, T_7, QTQPGGSKDFKQLNMNFMNWVCSRITLWRGGISRITVMEV, (SEQIDNO:6301); CCDS30587.1, Ins, T 7, WPDEQWEEHRDQCHALGQSSALWDWPHHQLLPAGRGHRWP, (SEQIDNO:6302); CCDS47774.1, Del, T_7, AILVRKSVTFALIPGIGVAYQIQMKKEKRNQRWMADLGS, (SEQIDNO:6303); CCDS31669.1, Ins, T_7, FQGYSVCETRKELFRSKSYLSSSYYLPQDFGCQLSNSSV, (SEQIDNO:6304); CCDS11021.1, Ins, T 7, ILWYNDGHVFPPSDQLQPQRCSDNCDVCGSDPSIKSFHL, (SEQIDNO:6305); CCDS4446.1, Del, T_7, LTAKFKMGLKVFVSSTPEKADQVARLLLNLNQKMKSNWP, (SEQIDNO:6306); CCDS2293.1, Ins, T_7, LVKYCTRSHHQVKSYTPSGIQSVHESCESYSYLGAIAWH, (SEQIDNO:6307); CCDS3609.1, Del, T_7, PVWPLIMLLQIIPSQQVLLMGLWSVALVLVKDSSLLPPA, (SEQIDNO:6308); CCDS34342.1, Del, T_7, CLDRAGHPAVRREPASSLICRVPNSQLGMSASRLPSRT, (SEQIDNO:6310); CCDS54873.1, Del, T_7, KICVKALKLVTPVRSWWDILPCIESVLEWLVSSLSSVY, (SEQIDNO:6312); CCDS31977.1, Ins, T 7, LFIILPWAHCMWNFMCVFGHCPLGNQTAATEKEKISVN, (SEQIDNO:6313); CCDS31815.1, Ins, T_7, PGHLYSHFVGQLSYCHSYQCGSRTSNTHVLLSSKSVTS, (SEQIDNO:6314); CCDS9255.2, Del, T_7, QHCPSISTGRVQPWESHLEKSLIPLSMNQTCRPCLGRQ, (SEQIDNO:6315); CCDS2926.1, Del, T_7, QKLTWRNINFCLNTQVFSLMSRRGNTSSTNLKYVRRMI, (SEQIDNO:6316); CCDS10129.2, Del, T_7, QQLLGFLLVPISQEIWRIPKMPSPEEPCWPFSSPLLPT, (SEQIDNO:6317); CCDS8689.1, Ins, T_7, SGWCICSPYWLAFDRHDLRNLWIFSLVQGLLSCRCWLY, (SEQIDNO:6318); CCDS6632.1, Del, T_7, VPCSVLVKLISIQKMRKIMINMEFELESNFEVVLNCMN, (SEQIDNO:6319); CCDS3436.1, Del, T_7, AHLLVYILWAVDGRKKKNKWNAMVVLNKSLNRVHLLG, (SEQIDNO:6320); CCDS8961.1, Del, T_7, PPLYSWLHMLKTKELMSTRQLFCYLLWLSLICLLGLL, (SEQIDNO:6321); CCDS3114.1, Ins, T_7, SDSIFACSRRRLWVSLSWSDNSSSETDSKHSHYDGHL, (SEQIDNO:6322); CCDS9347.1, Ins, T_7, SHPKVFTGFHSFSIFCFYYFSLPYILLLEERQKKLQI, (SEQIDNO:6323); CCDS42012.1, Ins, T_7, TPYMGGGHSWSSSGFCNCPYLLLLYNVDCYLHECHCH, (SEQIDNO:6325); CCDS47907.1, Del, T_7, ANQEISIVKTIVVCLKVGCVILKMTVVMAAMKKIAQ, (SEQIDNO:6326); CCDS31378.1, Del, T_7, CIVTPMFFHMHSAFTRMSLNSPVLIPPSTDCTQLCL, (SEQIDNO:6327); CCDS 14521.1, Del, T_7, FLCPFLLLRRQLWAILSFILLEGYGMQELKAGSRGN, (SEQIDNO:6328); CCDS46718.1, Ins, T 7, FQVPHGVWDRGSGEEPAGKPEDEQHRAAQAGPAAFR, (SEQIDNO:6329); CCDS 1456.1, Ins, T_7, FTDTHHTDSKPLALQYPLLEYSQSCCSPKSSQTARC, (SEQIDNO:6330); CCDS31977.1, Ins, T_7, KETLSCGRFTVSSLHTAGPKSRIHFSWLGSSNAVCS, (SEQIDNO:6331); CCDS32020.1, Ins, T_7, LHCYCPGKPLDSGNSASPCSPAPISYVLFFRSSLFH, (SEQIDNO:6332); CCDS53894.1, Del, T_7, LPSSLTSSKISSTCYFIMTGLKSLQTTKPMSSVKIK, (SEQIDNO:6333); CCDS3416.1, Del, T_7, QIMKFQTSRRKKLHESRSGLCMKLGDPMWNPAGKAA, (SEQIDNO:6334); CCDS13629.1, Ins, T_7, QSEYCCSKTNSTKKPGNINKRIPCIIWISTSEKRSG, (SEQIDNO:6335); CCDS 1197.1, Del, T_7, RSHFSWAMVGWRLSFFSLASLWPMCLRGCWPQSLCA, (SEQIDNO:6336); CCDS11587.1, Ins, T_7, TSLQCLVLESETKEQESQETGESNSSRGKSEKLVCL, (SEQIDNO:6338); CCDS5149.1, Del, T_7, VKSWLFSNLPVQIPTSMRTWSWPEQFLGWWDPCPQL, (SEQIDNO:6339); CCDS33237.1, Del, T_7, CSSISWSCSSWPGFLRPLCGMKLASLPPSWWIRWR, (SEQIDNO:6340); CCDS 11887.1, Ins, T_7, FGMVHPCHHIILGIRDPSHLSRLFQHEYLLEPVEA, (SEQIDNO:6341); CCDS9727.2, Ins, T_7, HRFQAFWLICWRNPCCGGVFIFSSNMAVVHCSRGF, (SEQIDNO:6342); CCDS6707.1, Ins, T_7, IREGTAPDYSLRVALHSEPADNSRGCGGSFPCPCH, (SEQIDNO:6343); CCDS47522.1, Del, T_7, ISLTTRGTMRRLLPSTWTGSWTSAGSLPWPAGWST, (SEQIDNO:6344); CCDS47510.1, Ins, T_7, LYVYFISVFSDSLYICIRYHPRHSSFTANRIPKCP, (SEQIDNO:6345); CCDS31083.1, Ins, T_7, PRKPSRQRGSSDESWQTAATSRHVSPSNEESETGF, (SEQIDNO:6346); CCDS43218.1, Del, T_7, SGKMKSCQELDLLPEKLITEDVSLPCPERSVWKGT, (SEQIDNO:6347); CCDS45338.1, Ins, T_7, SIKHSIWSSSSALGLSLYLQTRVLHKNKGRRKSGI, (SEQIDNO:6348); CCDS6766.1, Del, T_7, SMKKLITCGMEALRPKQEKNSNTCFRSLHCAGLSL, (SEQIDNO:6349); CCDS31915.1, Ins, T_7, FKLYSPLCYYVFVSGRAYGWRPNIQIWSAGTGTK, (SEQIDNO:6350); CCDS13133.1, Del, T_7, GLALTCLSVCSTQTSVLLFFIKKSSLPLASWFLM, (SEQIDNO:6351); CCDS31977.1, Del, T_7, IHYSSLGSLYVELYVCVWSLSFGESDCCYRERKN, (SEQIDNO:6352); CCDS54483.1, Del, T_7, ISMEVKFKDARVVQFEQTAWIVLIEQIVCRHFLA, (SEQIDNO:6353); CCDS14161.1, Ins, T_7, KSRRSFNWEVPTDISKEETYRAHGRSFCTSCSIP, (SEQIDNO:6354); CCDS12419.1, Del, T_7, RIQMKKWPKSTGRRGTSVGASLGTIMQSVQTPAP, (SEQIDNO:6355); CCDS1661.1, Del, T_7, RMDSKPWKASNLPLKRCLRIFTRSNRPRMKKEGS, (SEQIDNO:6356); CCDS2079.1, Del, T_7, VESVVILMKTMEMGRTFNQSFKKFRKLKNLRQKK, (SEQIDNO:6357); CCDS7394.1, Del, T_7, VTLLYVNTVTLAPNVNMESSRNAHSPATPSAKRK, (SEQIDNO:6358); CCDS9376.1, Del, T_7, ASLLVFILPALNLVRVSSLKKKKYQVSLALNGT, (SEQIDNO:6359); CCDS5004.1, Ins, T_7, CICLNKKDSQGGYSIYYNQETIKSSNEAYRSDS, (SEQIDNO:6360); CCDS34120.1, Ins, T_7, FICHANISQGRVPEQLPIKVQSRRNTYRYKGAE, (SEQIDNO:6361); CCDS45065.1, Ins, T_7, GCGDSKSDGRTSCSSCQSSKIFLCWDRGVPCGL, (SEQIDNO:6362); CCDS12271.1, Del, T_7, GLVYFVCMKELTLERNRMNVSSVVKPSLFTVPI, (SEQIDNO:6363); CCDS32918.1, Del, T_7, GPVYYICMKERTLERNHMNVSSVLKPFLFTVPI, (SEQIDNO:6364); CCDS45988.1, Del, T_7, GPVYYVCMKELTLERNHMNVSSVLKPSLFTVPI, (SEQIDNO:6366); CCDS58789.1, Del, T_7, LPQTHTGIHQLGVYTPTLGSPPAICLLIWALTT, (SEQIDNO:6367); CCDS 14231.1, Del, T_7, LVELQKAIKCTIPWWNIALRLHQEKMFETLPRY, (SEQIDNO:6368); CCDS44927.1, Ins, T_7, LWRLAEQKSNFLLLYWDDCGNCGLSANHHFYTI, (SEQIDNO:6369); CCDS58971.1, Ins, T_7, NKPMYIDFWDGKPTYVLLKQGYVISIFGHFCAW, (SEQIDNO:6371); CCDS2194.1, Del, T_7, PNMQIHLSLLFMIALLQLEKMPPMWLSVLSGRS, (SEQIDNO:6372); CCDS56540.1, Del, T_7, PPCFLPYLLKLRSSIGSATHPMQVFHTPTVGEI, (SEQIDNO:6373); CCDS4038.1, Del, T_7, TCGDLEESANKPANLLKSSKKMLLHVHWSLLEI, (SEQIDNO:6374); CCDS7335.1, Ins, T_7, TLKLGAPGLPSESFCPATASHTPQPPRTRATAW, (SEQIDNO:6375); CCDS10900.1, Del, T_7, VKVQTHISVIPSLESSPLGVQDWGNPSALIQNS, (SEQIDNO:6376); CCDS5370.1, Ins, T_7, YRCICLPAKRPARSRLVPLGSLGVFTCSWTGPR, (SEQIDNO:6377); CCDS9804.1, Ins, T_7, AADCDSFFVCFDFPVYCRASYVSTTMFWSQRN, (SEQIDNO:6378); CCDS3630.1, Ins, T_7, CRSMQKVLVENDCGRFRKCTMLRHIQSLSIYL, (SEQIDNO:6379); CCDS12149.2, Ins, T_7, FFVTGCDACSESREGHVQVLRSPPQPEFRAVF, (SEQIDNO:6380); CCDS53691.1, Ins, T_7, IAFHFHETKCGVYIQRNWGPHDQRQQSEDEVL, (SEQIDNO:6381); CCDS34759.1, Del, T_7, IGPRLNKLLLTVNTRIYGDRQSAMSSFFIGFL, (SEQIDNO:6382); CCDS7480.1, Del, T_7, IVQRMCCALVAATMGQHLPRLLRITVAWLGRY, (SEQIDNO:6383); CCDS42696.1, Del, T_7, LAAPRPRHAPSFSVAAPSSLWRRQSGPCMMPS, (SEQIDNO:6384); CCDS54612.1, Del, T_7, LRLLNIMGVPILFWCTFQAEWPSSFRMWICRI, (SEQIDNO:6385); CCDS3528.1, Del, T_7, LVVSCWLRYLTYRLCTVSASLLATQLKGTVED, (SEQIDNO:6386); CCDS56443.1, Del, T_7, RLFLPRWKSGMKGRLKKHEAKPIRKPSKIRLH, (SEQIDNO:6387); CCDS31826.2, Ins, T_7, SPKFIFLRNLIYSYLCSKILIQYLNWEQNNNV, (SEQIDNO:6388); CCDS42116.2, Ins, T_7, SRDRVSSCCSCWSRTPDLKRSAHLRLPKCGII, (SEQIDNO:6389); CCDS8022.1, Del, T_7, THWCLTPCRRHFSLIRARLELVANTKLRSQIA, (SEQIDNO:6390); CCDS2736.1, Del, T_7, TMTFRASRVRTRPGSLLPSPPLSYTAWCFSSA, (SEQIDNO:6391); CCDS2602.1, Del, T_7, WRQLWGSSQVKVALRVGGKFALYLKALAMQHR, (SEQIDNO:6392); CCDS 1954.1, Ins, T_7, AESALWDQEVEEDLGRALPGQSPRRSAARVL, (SEQIDNO:6393); CCDS31028.1, Del, T_7, GSVCMEKAPLRICVTLWSRLVLALSCCCLCS, (SEQIDNO:6395); CCDS55516.1, Del, T_7, HLNKWELDTLSFKHVNFFQKGRRLGDKIRAT, (SEQIDNO:6396); CCDS748.1, Del, T_7, LQRKISILQRVKAKFLNELHHLRNQNASKMR, (SEQIDNO:6397); CCDS31021.1, Ins, T_7, LYPLSQLQYAEDLLLRRKVQQMHYLSAHQVV, (SEQIDNO:6398); CCDS8342.1, Del, T_7, PVAKRSSICTECFAGYVLVFLSLHSMVDSSK, (SEQIDNO:6399); CCDS10128.1, Ins, T_7, SSSLQWLETRQKVGNSLPIIILGACYIISSI, (SEQIDNO:6400); CCDS2987.1, Del, T_7, STCWLLFSFSTSTFIKQCGGILTIILLLVHH, (SEQIDNO:6401); CCDS34061.1, Del, T_7, TCSFKEHFSLRNCIPAPVPVLSSTMLPVQYF, (SEQIDNO:6402); CCDS6195.1, Del, T_7, TYKENGPFLSTELGFTLLKLLVHWVTYIPSK, (SEQIDNO:6403); CCDS14238.1, Ins, T_7, CLFVLFYFCRDLFLPLEQDAFFSPSALGHL, (SEQIDNO:6405); CCDS8992.2, Del, T_7, DFIGYSSSGEAASTNYCTGNLLFLLFFIQQ, (SEQIDNO:6406); CCDS4987.1, Del, T_7, DQVSQRKYQNWSFILTKLTVSSFPTLVTGL, (SEQIDNO:6407); CCDS42654.1, Del, T_7, GKKEILWPLPIAPGWFSFFMPFKMIGICTW, (SEQIDNO:6408); CCDS34680.1, Del, T_7, IIKMSYLKDIWSILYQEVVIFQGIQTMNIN, (SEQIDNO:6410); CCDS3553.1, Ins, T_7, NQARLWTCWNARICSSHRHVSSSEKLYPES, (SEQIDNO:6411); CCDS54446.1, Del, T_7, SMSTLRCRPPTLRMKRLPVAVCSCTPPEST, (SEQIDNO:6413); CCDS56326.1, Del, T_7, VPMSLACSITVSFVGQISTLVLDVSSISHW, (SEQIDNO:6415); CCDS8132.1, Ins, T_7, CCGHSLRGQEARAAGLPLHTPELGSAVQS, (SEQIDNO:6416); CCDS3828.1, Ins, T_7, CVSSWEGKTMQFFPVGRKWARNKNYSWML, (SEQIDNO:6417); CCDS55882.1, Del, T_7, LAYLHIITSESTMKRPQTSSPTGMKRIIL, (SEQIDNO:6419); CCDS47894.1, Del, T_7, LDSYSFQSCCLFHCPCKQLRYHFHITCIL, (SEQIDNO:6420); CCDS 1082.2, Ins, T_7, LHSGDLQPYPTPPPRAVPSLPRLCCLLGA, (SEQIDNO:6421); CCDS10271.1, Ins, T_7, LSNRDLHHPVGWHPWPGGQRLDCSPVYTG, (SEQIDNO:6422); CCDS2967.1, Ins, T_7, LWPQCIFSSPYFCNCIPGPFSGFHSEVPG, (SEQIDNO:6423); CCDS31827.1, Del, T_7, LYFLELPSFTSWPPCLMIVMWPSANPCIT, (SEQIDNO:6424); CCDS34730.1, Ins, T_7, NATHRLYCNGIIKIFDDVSDILQSLYSSS, (SEQIDNO:6425); CCDS13515.1, Del, T_7, RGKQRAPWTAPRRATVIRTAVWTTSKTAT, (SEQIDNO:6426); CCDS9383.1, Del, T_7, RLLLPTHLNQNVQVQVLLFSWMKRDIKKA, (SEQIDNO:6427); CCDS13575.1, Ins, T_7, SAGSSGRNSLESSGTSPKICIGKSRTTDE, (SEQIDNO:6428); CCDS42696.1, Ins, T_7, YWLPQGQDMHLHSPWRRRAVYGGDRAVPA, (SEQIDNO:6430); CCDS43987.1, Del, T_7, FWIELTFFRIQCYPPFGTWDWSYLGLIL, (SEQIDNO:6431); CCDS9498.1, Ins, T_7, GLHSFADINARFCKLWSSAFCWKTGQVQ, (SEQIDNO:6432); CCDS34081.1, Ins, T_7, LENYSGGASSRQNQHLDLGSQAGTSLPN, (SEQIDNO:6433); CCDS5605.1, Del, T_7, LSSFRVSRLGKLARSSPEDCGQWLLKQC, (SEQIDNO:6436); CCDS1476.1, Ins, T_7, LVCVLSYGCVASFCFRCRALSRASSSGQ, (SEQIDNO:6437); CCDS11685.1, Del, T_7, QFRFLCFWFITLLKMLWFPSNLFQTYIF, (SEQIDNO:6440); CCDS34924.1, Ins, T_7, SAIDTVFFITFLHSSVNYLFSFYVASYF, (SEQIDNO:6441); CCDS11224.2, Del, T_7, SMIMYSLPLTYSNLHTLTILKKYMLSQN, (SEQIDNO:6442); CCDS33544.1, Del, T_7, VIMSITGKKEESNRSKALSEATPFHWIT, (SEQIDNO:6443); CCDS4542.1, Ins, T 7, WPDQACHWRPILLTLGCGDHPSEWDCGN, (SEQIDNO:6444); CCDS44825.1, Del, T_7, ATRHLDSSGSPSHLAGAYTKITSFGRL, (SEQIDNO:6445); CCDS56578.1, Del, T_7, CLSFFKLTLEKMKKFLGSKGGRSTTEG, (SEQIDNO:6446); CCDS6132.1, Ins, T_7, CVALRVSVDAEENNRQITKRRCFITSI, (SEQIDNO:6448); CCDS5868.1, Ins, T_7, CVVFHVFGLEQCLVCDLAQYLVLCEDY, (SEQIDNO:6449); CCDS47063.1, Ins, T_7, FYWEIDIRTTRRKGKAACWPGYIGFLC, (SEQIDNO:6450); CCDS34917.1, Ins, T_7, HGFIMVYIINDRNTKAEIPGTQSIYTL, (SEQIDNO:6451); CCDS6644.1, Del, T_7, HIQDMNLLVYVAKSFLGISRWLCSFGR, (SEQIDNO:6452); CCDS58971.1, Ins, T_7, ICCGAGEGITLHQFEAKSSGEKGFSSI, (SEQIDNO:6453); CCDS43473.1, Del, T_7, LFCTFGSQFYQPEAILNQSIDLKDAKK, (SEQIDNO:6455); CCDS33471.1, Del, T_7, LKRLFVKMTRKSQQERPVPRRSSDQDS, (SEQIDNO:6456); CCDS830.1, Ins, T_7, LLFCGDIYVLLCALANVCFWNIAIWNN, (SEQIDNO:6457); CCDS33544.1, Ins, T_7, LLLCPLLGKRRNPTGQRPFQKQLHFIG, (SEQIDNO:6458); CCDS35198.1, Del, T_7, PSCTYILHSSPKRSLLGAVNMAGMGTM, (SEQIDNO:6459); CCDS13575.1, Del, T_7, SGFLGEKFSGVIWNFSENMYWQVKNNR, (SEQIDNO:6462); CCDS11995.1, Ins, T_7, SGSFRSFDEGIVHSKRPSGMDLFNVVQ, (SEQIDNO:6463); CCDS5140.1, Ins, T_7, SYFRSAHLHYRNCYGRSWILAPKRTFY, (SEQIDNO:6464); CCDS6045.1, Del, T_7, TMILQTLAWWRQEQNVEREWCATMVNV, (SEQIDNO:6465); CCDS33379.1, Del, T_7, TPFCTLSAKHVPLSISGSPALELLGPW, (SEQIDNO:6466); CCDS11995.1, Del, T_7, TVVIFSTSGFLQPTLIFLKLSWKSTHL, (SEQIDNO:6467); CCDS3622.1, Del, T_7, YVSWELQLQSRQMQDYYLIIPNQLLKR, (SEQIDNO:6468); CCDS42012.1, Del, T_7, ALHGWWAQLEFFRLLQLSLSAAAVQC, (SEQIDNO:6469); CCDS4542.1, Del, T_7, ARSSLSLEANSPYLGVWRPPLRMGLW, (SEQIDNO:6470); CCDS11266.1, Ins, T_7, FKDRDNYNREAVTAYLIHIINVTGIG, (SEQIDNO:6472); CCDS 1916.1, Del, T_7, KILILMKMKGDCLEVKNLSQVKVPEI, (SEQIDNO:6474); CCDS3073.1, Ins, T_7, LARPKCTSQGVEVCDFGVSAWRPKSH, (SEQIDNO:6475); CCDS1891.2, Ins, T_7, LLRNVLHDQCTMDCGHFLLWRHGGEL, (SEQIDNO:6476); CCDS3436.1, Ins, T_7, LPTSLCISYGQWMEEKKRTNGTRWLF, (SEQIDNO:6477); CCDS46872.2, Del, T_7, MNFKEQIVKSSLPKPWKKHPVPHPKV, (SEQIDNO:6478); CCDS54796.1, Del, T_7, PSKKIDFLYLSRYAIRLRNLADDYHL, (SEQIDNO:6479); CCDS58627.1, Ins, T_7, PVYHSQSEEVLFPLLLAYIASSAYFT, (SEQIDNO:6480); CCDS5773.1, Ins, T_7, QLDQTNFEERIQTAPGIVRHIPNPFC, (SEQIDNO:6481); CCDS41352.1, Del, T_7, SCVFYLERFKKNITDCSKMSPVVLDV, (SEQIDNO:6482); CCDS56604.1, Ins, T_7, SEPLCAGLLYTSLDSSNADLPAGTFG, (SEQIDNO:6483); CCDS5239.1, Del, T_7, TYWFFFLFFFSFFYFLWILWLITQQV, (SEQIDNO:6484); CCDS9393.1, Del, T_7, VIPVIVVLKIKKSMTNTCLNIQNALN, (SEQIDNO:6485); CCDS7113.1, Del, T_7, VSVPHSGRVLSAQLMLTNVRFTQEHP, (SEQIDNO:6486); CCDS33275.1, Del, T_7, ECLKKQFFIVNTKTSATLDCQPKAY, (SEQIDNO:6487); CCDS7256.1, Ins, T_7, FLWHCCRSWMWFIIHCNSDHYVPVF, (SEQIDNO:6488); CCDS45263.1, Ins, T_7, FSVKCMAECFSWCYSTKYSLQNQCC, (SEQIDNO:6489); CCDS47894.1, Ins, T_7, FWTHTPFRAAASSIAHANNSGITSI, (SEQIDNO:6490); CCDS47475.1, Del, T_7, GEVLDRLGVGFMWEESKNLVQRFLD, (SEQIDNO:6491); CCDS55176.1, Del, T_7, GEVLDRLGVGFTWEESKNLVQRFLD, (SEQIDNO:6492); CCDS 11926.2, Del, T_7, ISFGYPRWTLKALVYSQNGVLIWLM, (SEQIDNO:6493); CCDS33981.1, Del, T_7, KISSVGLVLVHSITGTASLPVRNAK, (SEQIDNO:6494); CCDS41350.1, Del, T_7, LHILSQSILLIQSQSLKKTYDWSGV, (SEQIDNO:6495); CCDS35397.1, Del, T_7, MWTSHPRCPDGPQLEQRKGQVHIAM, (SEQIDNO:6496); CCDS31591.1, Del, T_7, PSSSTPGSSLSRPAGTPPPGGWTSP, (SEQIDNO:6497); CCDS47564.1, Del, T_7, QNRSATIRLSLRVMLSLEILFSGKM, (SEQIDNO:6498); CCDS3535.1, Del, T_7, QQLQHPLPHLLQLSLLQLHLLQPHL, (SEQIDNO:6499); CCDS42501.1, Del, T_7, RALSGTSRRTRRPSVSSARRLRHWN, (SEQIDNO:6500); CCDS7419.1, Del, T_7, RRFFRGWEQLMTVSNKRVQLKHLPV, (SEQIDNO:6501); CCDS14628.1, Ins, T_7, SECLGRSPRTQPRPQDEDGIDTQPL, (SEQIDNO:6502); CCDS11685.1, Del, T_7, SWKKLNMLLPLKNIAFLKQHWNRFL, (SEQIDNO:6503); CCDS1891.2, Del, T_7, TQERATRPVYYGLWTFPSVETRWRT, (SEQIDNO:6504); CCDS830.1, Del, T_7, VVLRGHLCSIVRTGKRMFLEHCDLE, (SEQIDNO:6505); CCDS5605.1, Ins, T_7, YFLPSGFHVWESWRDPHQKTAVNGF, (SEQIDNO:6506); CCDS43436.2, Del, T_7, ATPVANVSAGAPTSIATSLFTIPS, (SEQIDNO:6508); CCDS45750.1, Del, T_7, CQLCQVWSLFVEHFLHCMRILCLC, (SEQIDNO:6509); CCDS13111.1, Del, T_7, ILFLMKQQSAACFSATEMTLPGMS, (SEQIDNO:6510); CCDS4071.2, Del, T_7, KKQLRNFPLCRHIISLLYAFFMPF, (SEQIDNO:6511); CCDS34730.1, Ins, T_7, LASFETSGYAYYSTGKRTVKQRCT, (SEQIDNO:6512); CCDS31417.1, Del, T_7, LEISPLQISVSLLALSLKCWFTSW, (SEQIDNO:6513); CCDS45879.1, Ins, T_7, LTFIRNRYKRTCSSTILERLQAQY, (SEQIDNO:6514); CCDS4300.1, Del, T_7, PASFIFSWVPPVTSLWAFLEYCAL, (SEQIDNO:6515); CCDS41774.1, Ins, T_7, PNCGYLQEISNSLHCSRAKFNNKC, (SEQIDNO:6516); CCDS2222.1, Del, T_7, QSIWYLLQLKPMVKYPPHRFCRCF, (SEQIDNO:6517); CCDS1954.1, Del, T_7, RVSALGPRGGGRPGPCSTRPVPTA, (SEQIDNO:6519); CCDS5116.1, Ins, T_7, RYGSKSSCVDVFSRKSYQCRLLHS, (SEQIDNO:6520); CCDS6069.1, Ins, T_7, YYGWQCMVGNSYHHMVFSSCAKVG, (SEQIDNO:6521); CCDS34852.1, Ins, T_7, CCVPYITSSRSFVFWSKRVCLGE, (SEQIDNO:6522); CCDS43246.1, Ins, T_7, FHVWRTDSGTQENRERKHNMDSV, (SEQIDNO:6523); CCDS33527.2, Ins, T_7, FPRHPQFAFTYFGDCYRRKQRCV, (SEQIDNO:6524); CCDS9813.1, Del, T_7, HSFTWGKCLKPITLLWTTLLLHS, (SEQIDNO:6525); CCDS46500.1, Del, T_7, KGRMETSSQLPKRLRSLNGPCRL, (SEQIDNO:6526); CCDS34209.1, Ins, T_7, LETELGSGICHRYFMPHDALLQL, (SEQIDNO:6527); CCDS30769.2, Del, T_7, LETVNVKRKLISVCIILMMKLMK, (SEQIDNO:6528); CCDS34475.1, Del, T_7, LFCTFGSQFYQPKRNILSMVAWT, (SEQIDNO:6529); CCDS43328.1, Del, T_7, LICFRKFLLKKRKENKNLLKITV, (SEQIDNO:6530); CCDS33391.1, Del, T_7, LLHFLEKSPLEEWNQWVMHLCWH, (SEQIDNO:6531); CCDS59173.1, Del, T_7, LSLLPPPCTKCIKDGKTTAARSL, (SEQIDNO:6533); CCDS43127.1, Ins, T_7, LVDCFNKDGSVRPWRSRSLPQKK, (SEQIDNO:6534); CCDS44221.1, Del, T_7, MLTCTTLSVRPCSVTVWCCRASR, (SEQIDNO:6535); CCDS9727.2, Del, T_7, PFSGFLAHLLEESLLRGCFHFLV, (SEQIDNO:6536); CCDS9056.1, Ins, T_7, SAANVSKTLANPASTKKSTGIKK, (SEQIDNO:6537); CCDS59530.1, Ins, T_7, SQMLFASWHAGVSATLSWRPHSH, (SEQIDNO:6538); CCDS7847.1, Del, T_7, SWFSVGPVWPCWSGSSVAPGVCH, (SEQIDNO:6539); CCDS7831.1, Del, T_7, VWFSVFPAWSCWSGSSVDPGRCR, (SEQIDNO:6540); CCDS7830.1, Del, T_7, VWFSVGPAWSCWSGFSVDPGRCR, (SEQIDNO:6541); CCDS9255.2, Ins, T_7, ASIVLQSAQDEYNRGSHIWRSL, (SEQIDNO:6542); CCDS43187.1, Ins, T_7, CFFCDFFIYFYIIHHVVSSCLC, (SEQIDNO:6543); CCDS103.1, Del, T_7, DLWQLLLFLRAVPPALLIHTKS, (SEQIDNO:6544); CCDS33817.1, Ins, T_7, FTCLEHIRVSNYINWHLTCNTY, (SEQIDNO:6545); CCDS46856.2, Ins, T_7, GCQQLPLRKCHFSSSQWLQSKC, (SEQIDNO:6547); CCDS31866.1, Del, T_7, GTLRRTSNQLRSAGYQQRRQNK, (SEQIDNO:6548); CCDS31772.1, Del, T_7, HLLSFFLFLISCLLAKLRLFFP, (SEQIDNO:6549); CCDS41769.1, Del, T_7, HLLSFFPFLISCLLAKLRLFFP, (SEQIDNO:6550); CCDS10110.1, Del, T_7, HQQIKMERQDQILQDRLWKLKK, (SEQIDNO:6551); CCDS35373.1, Del, T 7, KKKMERSTSIKNQSSLASQKFP, (SEQIDNO:6552); CCDS9026.1, Ins, T_7, LGTLCGSKNSARQHWCMELHKP, (SEQIDNO:6553); CCDS47090.2, Ins, T_7, RDCPWEFVERKEDRQNKEGYRE, (SEQIDNO:6554); CCDS46801.1, Ins, T_7, SGSRFSWEVIPSEPESFVSLPV, (SEQIDNO:6555); CCDS35126.1, Del, T_7, SLGSRELTSPTLSSSSCSWLFT, (SEQIDNO:6556); CCDS33804.1, Del, T_7, YTLDQICLKKEVMIYQLLFYLQ, (SEQIDNO:6557); CCDS3828.1, Del, T_7, CVLLGRKNNAIFSSGQKMGQE, (SEQIDNO:6558); CCDS33373.1, Ins, T_7, FLATLNQRIPDKETQAFLQKI, (SEQIDNO:6559); CCDS5757.1, Ins, T_7, FLIGNSQKSDCFCVFFYLPDN, (SEQIDNO:6560); CCDS55599.1, Del, T_7, GEMLRRSSMSQERIILIHMLD, (SEQIDNO:6561); CCDS47040.1, Del, T_7, GGGTQDSVWQACRKSQVPLLS, (SEQIDNO:6562); CCDS43327.1, Del, T_7, HVALSLMMMISQKQGLMKMKI, (SEQIDNO:6564); CCDS9308.2, Del, T_7, IPGKRTCVKNLQELLMIIYQI, (SEQIDNO:6565); CCDS 1536.1, Del, T_7, KKYCHALSLSFATFGCFGSLP, (SEQIDNO:6566); CCDS3793.1, Ins, T_7, LMAHCIQSPPSSRNYCQFHWL, (SEQIDNO:6567); CCDS3151.2, Del, T_7, LMWMFPLNQKGMPPVVLLSKI, (SEQIDNO:6568); CCDS6940.1, Del, T_7, MFAHSQMRLNSKNAHLPIISF, (SEQIDNO:6569); CCDS33337.1, Del, T_7, NFRWWKGLPGLSKVFLTVMHQ, (SEQIDNO:6570); CCDS33801.1, Del, T_7, PLHLVELQNVFSWQQWHMIAM, (SEQIDNO:6571); CCDS45146.1, Del, T_7, PTASSWLSMTRSFCSSTWCCC, (SEQIDNO:6572); CCDS13643.1, Ins, T_7, QSFAAKNISSTFDFIVANNGL, (SEQIDNO:6573); CCDS5239.1, Ins, T_7, RHTGFSFCFSSLSSISCGYYG, (SEQIDNO:6574); CCDS46801.1, Del, T_7, RISLFLGSHPFRARILCLTPC, (SEQIDNO:6575); CCDS5236.2, Ins, T_7, SKFTSLDSAAYCHERCWQFSN, (SEQIDNO:6576); CCDS41252.1, Ins, T_7, SKPGFQLLLARGHHERCPFAC, (SEQIDNO:6577); CCDS7121.1, Del, T_7, SSYILLELLVNFLQYTLPCRM, (SEQIDNO:6578); CCDS45002.1, Ins, T_7, TAKRRKPCLCTVSLASTSGKL, (SEQIDNO:6579); CCDS55659.1, Del, T_7, VSPAFLNFMDLLLTIKLELCV, (SEQIDNO:6580); CCDS865.1, Del, T_7, APTRSPTTSNYRKWRCCVNW, (SEQIDNO:6581); CCDS2064.1, Ins, T_7, FFASWITISGCWMAYSDTSL, (SEQIDNO:6582); CCDS31401.1, Ins, T_7, FFYDTSFWPKHSPLYPYSFG, (SEQIDNO:6583); CCDS1457.1, Ins, T_7, FTGSLLALYGVTIAFIYLPF, (SEQIDNO:6584); CCDS34042.1, Del, T_7, IADQSLYHHLIQFAILITQV, (SEQIDNO:6585); CCDS43187.1, Del, T_7, IFLYYSSCCIQLPLLTRFFR, (SEQIDNO:6586); CCDS56396.1, Ins, T_7, IKIQVSPGTGGRHRIPRGRS, (SEQIDNO:6587); CCDS55882.1, Del, T_7, IVSSGPPAPISRDLRVLSQG, (SEQIDNO:6588); CCDS47040.1, Ins, T_7, LEAGHRTVYGKLAGNLRFHC, (SEQIDNO:6589); CCDS2672.2, Del, T_7, LLTLDMEASLLFFLQRSQHR, (SEQIDNO:6590); CCDS34209.1, Del, T_7, MRWIKQMKLRETRANRCSLF, (SEQIDNO:6591); CCDS31563.1, Ins, T_7, MSCVHDDSAGKPPHHGHRDL, (SEQIDNO:6592); CCDS53543.1, Del, T_7, NAMLMAQYLMNIVGHFQNQQ, (SEQIDNO:6593); CCDS34917.1, Del, T_7, PAALSQSCLRNVWHWPCCGL, (SEQIDNO:6594); CCDS10141.1, Del, T_7, QIHKYRSTKIQIPIAGLFYI, (SEQIDNO:6595); CCDS30855.1, Del, T_7, WIAEVWPWCWQSASSPSSPQ, (SEQIDNO:6596); CCDS30735.1, Del, T_7, AFYLASVLKKASKERGLCC, (SEQIDNO:6597); CCDS32064.1, Ins, T_7, FTWFAWCHNAYYGFYCSSW, (SEQIDNO:6599); CCDS55189.1, Del, T_7, GAEKATIFKVPRLAPALPI, (SEQIDNO:6600); CCDS4642.1, Del, T_7, LPIYHSWIFVTPHVQSHKC, (SEQIDNO:6601); CCDS41634.1, Ins, T_7, LQRNRYVSFWLEIKDIGRA, (SEQIDNO:6602); CCDS45501.1, Del, T_7, LSIYGTGRYHLFLIITIFV, (SEQIDNO:6603); CCDS11900.1, Ins, T_7, LTSDSNAPEAAAGICVLCH, (SEQIDNO:6604); CCDS55321.1, Del, T_7, MNLTREPSRVLKSLWKEWH, (SEQIDNO:6606); CCDS46252.1, Del, T_7, PRSETLSKAQIINSAATSP, (SEQIDNO:6607); CCDS2363.1, Ins, T_7, SIQLGPFSCQLLLLQPINF, (SEQIDNO:6608); CCDS31378.1, Ins, T_7, SVLSLPCSFTCILPSPGCH, (SEQIDNO:6609); CCDS41629.1, Del, T_7, TGTAPALMPSCIIINQEAA, (SEQIDNO:6610); CCDS11021.1, Ins, T_7, VHLPHHTDWKSAHHLGHLC, (SEQIDNO:6611); CCDS32024.1, Del, T_7, VIFLESPNLPAWTLTSLKY, (SEQIDNO:6612); CCDS9026.1, Del, T_7, WNSLWVKEFGEAALVYGIT, (SEQIDNO:6613); CCDS449.1, Ins, T_7, YLCLAVHISCVSGSCHNLC, (SEQIDNO:6614); CCDS7593.1, Ins, T_7, YQLPARVKVCQQLSKGKLR, (SEQIDNO:6615); CCDS6204.1, Ins, T_7, YSWSKCRTRINSPTDRSQH, (SEQIDNO:6616); CCDS58789.1, Ins, T_7, CCPKPILEFTNWGYIPQH, (SEQIDNO:6617); CCDS3598.1, Ins, T_7, CKQSIWIFAGSAASSDYW, (SEQIDNO:6618); CCDS34342.1, Ins, T_7, CVWTGLVILLSGENQPAL, (SEQIDNO:6619); CCDS44717.1, Del, T_7, DQSSITSHIEFSKTCILS, (SEQIDNO:6620); CCDS58079.1, Ins, T_7, FDPRKLDCWGKRCQGPKV, (SEQIDNO:6621); CCDS33799.1, Ins, T_7, FHCNWRNHRMFSLGNNGI, (SEQIDNO:6622); CCDS33801.1, Ins, T_7, FLCIWWNYRMFSLGNNGI, (SEQIDNO:6623); CCDS58463.1, Ins, T_7, FLGGWIWSIDCICQLQQI, (SEQIDNO:6624); CCDS33800.1, Ins, T_7, FPCNHCNHRMFSLGNNGI, (SEQIDNO:6625); CCDS45984.2, Ins, T_7, FSQFVKKTREKSHWRETL, (SEQIDNO:6626); CCDS13171.1, Ins, T_7, FSQGARLLQGHREHADAP, (SEQIDNO:6627); CCDS33797.1, Ins, T_7, FVCNQCNHGMFSLGNDGI, (SEQIDNO:6628); CCDS31220.1, Del, T_7, GAIGCCQPMLLRHGIYLN, (SEQIDNO:6629); CCDS34071.1, Ins, T_7, GWLCSNRKLEIQRCITCF, (SEQIDNO:6630); CCDS31715.1, Ins, T_7, ILRCCDHHISSVAFPASW, (SEQIDNO:6631); CCDS7980.1, Del, T_7, LFLECCQLYLKGEMQHIW, (SEQIDNO:6632); CCDS9300.2, Del, T_7, LKCFFQIFKKLKQNLEIL, (SEQIDNO:6633); CCDS865.1, Ins, T_7, LLLLGAPLHQTTESGGAV, (SEQIDNO:6634); CCDS31506.1, Del, T_7, LPSCPFWMSCSHLSLPPR, (SEQIDNO:6635); CCDS10034.1, Del, T_7, LRFLRTMKQWPKFCSAGI, (SEQIDNO:6636); CCDS12713.1, Ins, T_7, LSVIPYETYENSHWRETV, (SEQIDNO:6637); CCDS31373.1, Del, T_7, PCWQPQTLHLTPAFFPKC, (SEQIDNO:6638); CCDS5757.1, Del, T_7, PHWKLPKIRLLLRVFLFA, (SEQIDNO:6639); CCDS3622.1, Ins, T_7, PMSLGNCSCNPDKCKIII, (SEQIDNO:6640); CCDS5147.1, Del, T_7, QALIQFRELGVKTYFRPS, (SEQIDNO:6641); CCDS3114.1, Del, T_7, RLYLCLFKKKVMGLFIVV, (SEQIDNO:6642); CCDS9303.1, Del, T_7, SEQTNHIRIAISLSVEAD, (SEQIDNO:6643); CCDS31772.1, Del, T_7, TSFIIQKQDLCFLLFLHI, (SEQIDNO:6644); CCDS41769.1, Del, T_7, TSFIIQKQDLCFLLFLRI, (SEQIDNO:6645); CCDS10314.1, Ins, T_7, CRPWKQIGRADPHFQGP, (SEQIDNO:6648); CCDS31827.1, Ins, T_7, CYISWSYRVLPPGLHVL, (SEQIDNO:6649); CCDS53879.1, Ins, T_7, FFSGWGKFALHHSIPET, (SEQIDNO:6650); CCDS33391.1, Ins, T_7, FFSTSWRRAHWKSGTSG, (SEQIDNO:6651); CCDS5688.1, Ins, T_7, FHCPRPSGSGSLCLLLL, (SEQIDNO:6652); CCDS13133.1, Del, T_7, GLLVRIAQQMARSSMKG, (SEQIDNO:6653); CCDS7113.1, Ins, T_7, HGWIQAIWRLHCFLYLQ, (SEQIDNO:6654); CCDS34120.1, Del, T_7, HMPRKHFTRQSTGTIAY, (SEQIDNO:6655); CCDS700.1, Del, T_7, IIGATLILSMTGQEQSL, (SEQIDNO:6656); CCDS32235.1, Del, T_7, KPSRIMMNLEVQDEPLA, (SEQIDNO:6657); CCDS2595.1, Del, T_7, KTRTVMKSTYLDSLSVN, (SEQIDNO:6658); CCDS11036.1, Del, T_7, KTTSSSLCWNLSLEGLT, (SEQIDNO:6659); CCDS31825.1, Ins, T_7, LHILGGYRIFPSSCSVL, (SEQIDNO:6660); CCDS55845.1, Del, T_7, LYPSLLNQSPCLELLKV, (SEQIDNO:6663); CCDS34475.1, Ins, T_7, LYSALLGHNFTSQKEIS, (SEQIDNO:6664); CCDS34535.1, Del, T_7, MKLEEILGNAALMMTLT, (SEQIDNO:6665); CCDS2223.2, Del, T_7, NAVRKSETKLQVPLQPL, (SEQIDNO:6666); CCDS43218.1, Del, T_7, NPEFVSFFQASRCWITR, (SEQIDNO:6667); CCDS11685.1, Ins, T_7, PLWIIICIFCFNADTSF, (SEQIDNO:6668); CCDS5843.1, Del, T_7, PMLLSPLVRLLQPSICQ, (SEQIDNO:6669); CCDS2154.1, Del, T_7, QCMERRLVNLIPLRLFY, (SEQIDNO:6670); CCDS6047.1, Del, T_7, QKSTCRSILKTRRRLSC, (SEQIDNO:6671); CCDS1082.2, Del, T_7, TFWRPSTLSDTSSSCCS, (SEQIDNO:6673); CCDS10091.1, Ins, T_7, YGDGIQKTLCYGICEVL, (SEQIDNO:6674); CCDS1842.1, Ins, T_7, CHLSCLQSTSYHSNQL, (SEQIDNO:6675); CCDS2129.1, Ins, T_7, CTLFYIRFKIPVLWEN, (SEQIDNO:6676); CCDS8677.1, Ins, T_7, CWIWSYSFHGLQVAEK, (SEQIDNO:6677); CCDS14188.1, Ins, T_7, DLLQEWSPLREEPVCF, (SEQIDNO:6678); CCDS2073.1, Ins, T_7, FIPGGEKISKCFLPGG, (SEQIDNO:6679); CCDS9815.2, Ins, T_7, FSHHVFSLWKERCSSL, (SEQIDNO:6680); CCDS34209.1, Del, T_7, GDRIGIWDLSQIFYAT, (SEQIDNO:6681); CCDS8718.1, Ins, T_7, HKRKPIKRAEPSRQSF, (SEQIDNO:6682); CCDS3459.1, Ins, T_7, HYGVCDDYFDHSKSGL, (SEQIDNO:6683); CCDS34064.1, Del, T_7, ISRFGPGLMEPSMQNS, (SEQIDNO:6684); CCDS47270.1, Del, T_7, KKSRRKKKMVLICPLS, (SEQIDNO:6685); CCDS1404.1, Del, T_7, KPQICPMILRTLSCTG, (SEQIDNO:6686); CCDS3646.1, Del, T_7, LCSWELFSSWSSLPEF, (SEQIDNO:6687); CCDS31772.1, Del, T_7, LFLISCLLAKLRLFFP, (SEQIDNO:6688); CCDS47082.1, Del, T_7, LQSHVPKSCLSRRESM, (SEQIDNO:6690); CCDS31005.1, Ins, T_7, LVCVLSYGCVASFCFR, (SEQIDNO:6691); CCDS43473.1, Ins, T_7, LYSALLGHNFTSQKQF, (SEQIDNO:6692); CCDS4981.1, Del, T_7, MATSSHRFLEDMLPAK, (SEQIDNO:6693); CCDS41769.1, Del, T_7, PFLISCLLAKLRLFFP, (SEQIDNO:6694); CCDS34355.1, Del, T_7, QPLCLQTQLAQNCMKL, (SEQIDNO:6695); CCDS11921.1, Del, T_7, RPLQPSDSVLMMLFMA, (SEQIDNO:6696); CCDS6359.2, Ins, T_7, SGSGKPAASRDDAPQR, (SEQIDNO:6698); CCDS34975.1, Ins, T_7, SIAHAQFPGRVRRALL, (SEQIDNO:6699); CCDS53944.1, Ins, T_7, STRRTDLEQRNSLLLL, (SEQIDNO:6700); CCDS11965.1, Ins, T_7, STRWKPCLNHHWFLVE, (SEQIDNO:6701); CCDS31514.1, Ins, T_7, SYAWRHGVSPSDSDGL, (SEQIDNO:6702); CCDS31880.1, Ins, T_7, VFGLSSYGNQPSKSLL, (SEQIDNO:6703); CCDS8294.1, Del, T_7, VVMEKVDTTIMTLLQI, (SEQIDNO:6704); CCDS9933.1, Del, T_7, WRAQTIILSLLPVMVL, (SEQIDNO:6705); CCDS47564.1, Ins, T_7, FRTGQPPSAYLCVSC, (SEQIDNO:6706); CCDS32033.1, Ins, T_7, FTGYNRVFLFISYGL, (SEQIDNO:6707); CCDS34701.1, Ins, T_7, GRARGLGWDVEGGGR, (SEQIDNO:6708); CCDS55514.1, Del, T_7, HLNKGRRLGDKIRAT, (SEQIDNO:6709); CCDS2987.1, Ins, T_7, HLPVGCSFHSVHQHL, (SEQIDNO:6710); CCDS32064.1, Del, T_7, HLVCLVPQCLLWILL, (SEQIDNO:6711); CCDS4996.1, Del, T_7, IVLDYFLFLQMLPCL, (SEQIDNO:6712); CCDS42374.1, Del, T_7, IVQLLIGHSLLLQKL, (SEQIDNO:6713); CCDS43187.1, Ins, T_7, IYFYIIHHVVSSCLC, (SEQIDNO:6714); CCDS55377.1, Del, T_7, KHLLCFRILPWRTSL, (SEQIDNO:6715); CCDS33237.1, Ins, T_7, LAPLFPGPVRPGQVF, (SEQIDNO:6716); CCDS3244.1, Del, T_7, LERVKLLIIPASMKK, (SEQIDNO:6717); CCDS55659.1, Ins, T_7, LFLQLFSISWTYYSL, (SEQIDNO:6718); CCDS43436.2, Ins, T_7, LLHLWQMFQQALLPL, (SEQIDNO:6719); CCDS54771.1, Del, T_7, LLMMMTTSREMPSLN, (SEQIDNO:6720); CCDS14021.1, Del, T_7, LMPPRLCLNLRVKMS, (SEQIDNO:6721); CCDS43700.1, Ins, T_7, LPFWDRPAAQTTLWI, (SEQIDNO:6722); CCDS9815.2, Del, T_7, LPSCLLPLERKVLIS, (SEQIDNO:6723); CCDS3949.1, Del, T_7, LVRSQLLEKFLQLRF, (SEQIDNO:6724); CCDS3776.1, Del, T_7, MHLYLELCRFSTGGV, (SEQIDNO:6725); CCDS31669.1, Del, T_7, PRLFSVRDKKRALQV, (SEQIDNO:6726); CCDS2119.1, Del, T_7, QMLKVTLLLAQQIQL, (SEQIDNO:6727); CCDS47069.1, Del, T_7, QTAESATVRMEYIFS, (SEQIDNO:6728); CCDS45621.1, Ins, T_7, SHPSAATKYEPGSYI, (SEQIDNO:6729); CCDS4125.1, Del, T_7, SNLEKVLNNHLLRPV, (SEQIDNO:6730); CCDS33402.1, Del, T_7, TYFFRIAGVCLMTEN, (SEQIDNO:6731); CCDS5636.1, Del, T_7, VDSSSYHPFKINQNI, (SEQIDNO:6732); CCDS47445.1, Del, T_7, VIVNLSTMGPSVNLM, (SEQIDNO:6733); CCDS4987.1, Ins, T_7, WIRSARENIRIGVSY, (SEQIDNO:6734); CCDS46932.1, Del, T_7, ASWLTSCYFFLEEK, (SEQIDNO:6737); CCDS9056.1, Del, T_7, CCKCLQNIGKSSKH, (SEQIDNO:6738); CCDS5868.1, Del, T_7, CCVSCFWTRAVSGL, (SEQIDNO:6739); CCDS31772.1, Ins, T_7, FIYSLFFFSSSPVS, (SEQIDNO:6741); CCDS41769.1, Ins, T_7, FIYSLFFLSSSPVS, (SEQIDNO:6742); CCDS34867.1, Ins, T_7, FRNHLLYFGCKIQP, (SEQIDNO:6743); CCDS10141.1, Ins, T_7, FRYTNTGAPRSKFL, (SEQIDNO:6744); CCDS14470.1, Ins, T_7, GRTGRCHRRICFQT, (SEQIDNO:6745); CCDS11583.1, Del, T_7, KQRKGTSCQFQDII, (SEQIDNO:6746); CCDS35397.1, Ins, T_7, LCGQATPGAQMVPN, (SEQIDNO:6747); CCDS33268.1, Del, T_7, LKKKMLSLKNILLN, (SEQIDNO:6748); CCDS9717.1, Ins, T_7, LLPIWHDLCFGELL, (SEQIDNO:6749); CCDS55882.1, Ins, T_7, LLSPLGPQLPSPGT, (SEQIDNO:6750); CCDS4124.1, Del, T_7, LTFYIISSEKITPW, (SEQIDNO:6751); CCDS5678.1, Ins, T_7, LVESISIFSHSTSD, (SEQIDNO:6752); CCDS7113.1, Ins, T_7, LYLSPTVEGSSLLS, (SEQIDNO:6753); CCDS45002.1, Ins, T_7, LYYKPSAFKNSTSN, (SEQIDNO:6754); CCDS54430.1, Del, T_7, NIIILKEEDNQKKN, (SEQIDNO:6755); CCDS9987.2, Del, T_7, QCLSGSISMDIGTK, (SEQIDNO:6756); CCDS45485.1, Del, T_7, QTWKKNHLTQTTLK, (SEQIDNO:6757); CCDS11887.1, Del, T_7, RNGTSLPSHHSWNS, (SEQIDNO:6758); CCDS31416.1, Del, T_7, SATCLWLTSVSLPT, (SEQIDNO:6759); CCDS1661.1, Ins, T_7, SGWTQSRGKPQTFH, (SEQIDNO:6760); CCDS33677.1, Del, T_7, SLPETALKQYACTV, (SEQIDNO:6761); CCDS53545.1, Del, T_7, TARPVMMTSQIPKE, (SEQIDNO:6762); CCDS41805.1, Del, T_7, TLSLYGHLYMRVLP, (SEQIDNO:6763); CCDS58294.1, Del, T_7, VLWKASGTIQFNCK, (SEQIDNO:6764); CCDS58683.1, Ins, T_7, VPGFNGDAYDVSHL, (SEQIDNO:6765); CCDS9697.1, Ins, T_7, WNIFNKYSEDRRRE, (SEQIDNO:6767); CCDS14487.1, Del, T_7, YSKSLEFVLRKSKH, (SEQIDNO:6769); CCDS43455.1, Ins, T_7, ASCLSDGFLPASA, (SEQIDNO:6770); CCDS4902.1, Ins, T_7, CSSGAAAKSSFEE, (SEQIDNO:6771); CCDS46446.1, Ins, T_7, FFILKRRLFCSTL, (SEQIDNO:6773); CCDS2325.1, Ins, T_7, FPVWVCERSEDCK, (SEQIDNO:6774); CCDS3535.1, Ins, T_7, FSSCSTRCPTYCS, (SEQIDNO:6775); CCDS47406.1, Del, T_7, FTTLRFSSDCRRS, (SEQIDNO:6776); CCDS4966.1, Del, T_7, GTQMLNIRTNIEV, (SEQIDNO:6777); CCDS4048.1, Ins, T_7, GWVLFKWIWYPEQ, (SEQIDNO:6778); CCDS11685.1, Ins, T_7, HSSDFYVFGSSLF, (SEQIDNO:6779); CCDS41252.1, Del, T_7, KTWISAPPGKRSS, (SEQIDNO:6780); CCDS45501.1, Ins, T_7, LCLYMALGVTTYS, (SEQIDNO:6781); CCDS8677.1, Del, T_7, LDMELLFPWLTGC, (SEQIDNO:6782); CCDS664.1, Del, T_7, LHCILQRTMDMNR, (SEQIDNO:6783); CCDS34640.1, Ins, T_7, LQECRRERKTRES, (SEQIDNO:6785); CCDS2955.1, Del, T_7, LRHSVKKKVLVRL, (SEQIDNO:6786); CCDS2955.1, Del, T_7, LSISYHQNWKNLH, (SEQIDNO:6787); CCDS12216.2, Del, T_7, PGNQSALLKKRQR, (SEQIDNO:6789); CCDS7593.1, Del, T_7, PTASQSQGLSAAV, (SEQIDNO:6790); CCDS54360.1, Ins, T_7, PVQDNIPRWINSI, (SEQIDNO:6791); CCDS5756.1, Ins, T_7, RDISNSSNWKQDG, (SEQIDNO:6792); CCDS1790.1, Ins, T_7, SKLSQVVRKTSSN, (SEQIDNO:6793); CCDS42319.1, Ins, T_7, VLFCFGGDRLTLH, (SEQIDNO:6795); CCDS33379.1, Ins, T_7, VPLFAHCQQNTSL, (SEQIDNO:6796); CCDS4658.1, Del, T_7, WETFLVWIFLILQ, (SEQIDNO:6797); CCDS31504.1, Ins, T_7, WRHRDLHSHRDGL, (SEQIDNO:6798); CCDS10091.1, Del, T_7, WRWNTKNSLLWNL, (SEQIDNO:6799); CCDS41326.1, Ins, T_7, WRYSGGALWAGDQ, (SEQIDNO:6800); CCDS2415.1, Ins, T_7, WTPYNHYGLDLWL, (SEQIDNO:6801); CCDS46227.1, Ins, T_7, YAKRSRLFEQVGW, (SEQIDNO:6802); CCDS6649.1, Ins, T_7, YALFFQLCTQQMF, (SEQIDNO:6803); CCDS6262.1, Ins, T_7, YFFVKFKSWWCRT, (SEQIDNO:6804); CCDS3949.1, Ins, T_7, YWSDHSCWKSSSS, (SEQIDNO:6805); CCDS41634.1, Del, T_7, AKKQICLILVGD, (SEQIDNO:6806); CCDS1620.1, Del, T_7, AVQILLTVYQTK, (SEQIDNO:6808); CCDS54774.1, Ins, T_7, CDKKCQSCCLHL, (SEQIDNO:6809); CCDS45021.1, Ins, T_7, CGNWHCPNFYPL, (SEQIDNO:6810); CCDS45060.1, Del, T_7, CGSIIILLEIHY, (SEQIDNO:6811); CCDS6132.1, Del, T_7, CGSSCVRGCGGK, (SEQIDNO:6812); CCDS6316.2, Ins, T_7, CTYICRIWILSL, (SEQIDNO:6813); CCDS14485.1, Ins, T_7, EFYSRGKWWRLW, (SEQIDNO:6814); CCDS5236.2, Del, T_7, EIYLIGFSSILP, (SEQIDNO:6815); CCDS47554.1, Ins, T_7, FLITLWWTWGGQ, (SEQIDNO:6816); CCDS10322.1, Del, T_7, FLVPLAIIMALQ, (SEQIDNO:6817); CCDS2194.1, Ins, T_7, FQTCKYTYHCCS, (SEQIDNO:6818); CCDS5685.1, Ins, T_7, GCGREIVCTDSI, (SEQIDNO:6819); CCDS47430.1, Ins, T_7, GKCYHPDVSNTK, (SEQIDNO:6820); CCDS56596.1, Ins, T_7, GSPNPFLYRQRK, (SEQIDNO:6821); CCDS11021.1, Del, T_7, IMVQRWACISAL, (SEQIDNO:6822); CCDS13662.1, Ins, T_7, KGVQILERKCTI, (SEQIDNO:6823); CCDS43663.1, Ins, T_7, LGELPCISRIFN, (SEQIDNO:6824); CCDS46446.1, Del, T_7, LHPQKETLLLNI, (SEQIDNO:6825); CCDS33773.1, Del, T_7, LRHSSTGHTLFE, (SEQIDNO:6826); CCDS41826.1, Ins, T_7, LSESNSVFQSFH, (SEQIDNO:6827); CCDS9297.1, Del, T_7, LTLKIKIFIGKM, (SEQIDNO:6828); CCDS9743.1, Del, T_7, MAFEQNLPHTQV, (SEQIDNO:6829); CCDS45065.1, Del, T_7, MRRLEERWENKL, (SEQIDNO:6830); CCDS33527.2, Del, T_7, PKASTVCFYLFW, (SEQIDNO:6831); CCDS46718.1, Del, T_7, PSATRCMGSRQW, (SEQIDNO:6832); CCDS42656.1, Del, T_7, QWMTCFLYSQFH, (SEQIDNO:6834); CCDS56604.1, Del, T_7, RASVCWVTVYFP, (SEQIDNO:6835); CCDS45880.1, Ins, T_7, SASDSICKWTGT, (SEQIDNO:6836); CCDS33773.1, Ins, T_7, SHCAQTDSTRVF, (SEQIDNO:6837); CCDS8667.1, Del, T_7, SKCSKFLRRCNL, (SEQIDNO:6838); CCDS6710.2, Ins, T_7, SLSQAGHRFWIC, (SEQIDNO:6839); CCDS59173.1, Ins, T_7, SSPSYPHPVQNA, (SEQIDNO:6840); CCDS6160.1, Ins, T_7, SWVYPQISGLSL, (SEQIDNO:6841); CCDS56606.1, Del, T_7, THCLLVSSRSIC, (SEQIDNO:6842); CCDS6463.1, Ins, T_7, WPCSHLFNSWSD, (SEQIDNO:6843); CCDS13072.1, Ins, T_7, WSLLSSYWLYHF, (SEQIDNO:6844); CCDS2293.1, Ins, T_7, YDSCYSRIRRES, (SEQIDNO:6845); CCDS8435.1, Ins, T_7, YTIRVSSHMARR, (SEQIDNO:6846); CCDS5729.1, Ins, T_7, CLEAWRIKIPK, (SEQIDNO:6847); CCDS58048.1, Ins, T_7, CLFALLAQCLC, (SEQIDNO:6848); CCDS9987.2, Ins, T_7, CNAFPAASRWI, (SEQIDNO:6849); CCDS6308.1, Del, T_7, ECWFQQQIEML, (SEQIDNO:6850); CCDS748.1, Ins, T_7, FFKGRYPYFRE, (SEQIDNO:6851); CCDS9617.1, Ins, T_7, FFPKVHPVYLC, (SEQIDNO:6852); CCDS2119.1, Ins, T_7, FIFYCNSPMPL, (SEQIDNO:6853); CCDS3707.1, Ins, T_7, FLYLGHIHFNL, (SEQIDNO:6855); CCDS31880.1, Del, T_7, FWSIKLWKPTK, (SEQIDNO:6856); CCDS59106.1, Del, T_7, GMRVVNTLGHY, (SEQIDNO:6857); CCDS3499.1, Ins, T_7, GVCIFCTIYKP, (SEQIDNO:6858); CCDS664.1, Ins, T_7, HSIAYCSVLWT, (SEQIDNO:6860); CCDS5907.1, Ins, T_7, HYFSVHLSLHL, (SEQIDNO:6861); CCDS55124.1, Del, T_7, ITITLKIFLLT, (SEQIDNO:6862); CCDS861.1, Del, T_7, KDCVLLGCFFS, (SEQIDNO:6863); CCDS6281.1, Ins, T_7, KEDKKCTQFGT, (SEQIDNO:6864); CCDS33351.2, Del, T_7, KNMIGVKYKSY, (SEQIDNO:6865); CCDS13261.1, Del, T_7, KSWILWKIVFI, (SEQIDNO:6866); CCDS1576.1, Ins, T_7, LCLEYFISKSI, (SEQIDNO:6868); CCDS56326.1, Ins, T_7, LCQCHLPAVLL, (SEQIDNO:6869); CCDS10202.1, Del, T_7, LCYLKQTQRKA, (SEQIDNO:6870); CCDS42654.1, Ins, T_7, LGRKRYYGLCQ, (SEQIDNO:6871); CCDS34119.1, Ins, T_7, LHHSLCLRYHL, (SEQIDNO:6872); CCDS53879.1, Del, T_7, LLRLGEICIAS, (SEQIDNO:6873); CCDS44322.1, Del, T_7, LMTCFKPVWFI, (SEQIDNO:6874); CCDS9376.1, Ins, T_7, LPLCSCLSFLP, (SEQIDNO:6875); CCDS13171.1, Del, T_7, LPRSQTASRTS, (SEQIDNO:6876); CCDS11887.1, Del, T_7, LTWPSLTSLWV, (SEQIDNO:6877); CCDS6160.1, Del, T_7, LVMVMVWIRLL, (SEQIDNO:6878); CCDS1284.1, Del, T_7, LVSGLSFVSLY, (SEQIDNO:6879); CCDS5636.1, Ins, T_7, LWTPHLIIHSR, (SEQIDNO:6880); CCDS41939.1, Del, T_7, NLCVWVTLKAI, (SEQIDNO:6881); CCDS4991.1, Del, T_7, NPRKATQSFHF, (SEQIDNO:6882); CCDS35089.1, Del, T_7, PICSSSTPSCK, (SEQIDNO:6883); CCDS13642.1, Del, T_7, PLETCATSYCR, (SEQIDNO:6884); CCDS2505.1, Del, T_7, QIAGVCLKTKN, (SEQIDNO:6885); CCDS2506.1, Del, T_7, QIAGVCLMTEN, (SEQIDNO:6886); CCDS11983.1, Del, T_7, QYLPLILFLSG, (SEQIDNO:6887); CCDS33402.1, Del, T_7, RIAGVCLMTEN, (SEQIDNO:6888); CCDS41963.1, Del, T_7, SFQKKLMKNPP, (SEQIDNO:6889); CCDS41878.1, Del, T_7, SSILIKMFAYW, (SEQIDNO:6891); CCDS3077.1, Ins, T_7, SYYVHEAKSSV, (SEQIDNO:6892); CCDS34439.1, Del, T_7, TSSRTPPSASW, (SEQIDNO:6893); CCDS47005.1, Ins, T_7, VFLTSIHSVAL, (SEQIDNO:6896); CCDS31502.1, Del, T_7, VSTWEHCWVIC, (SEQIDNO:6897); CCDS7846.1, Del, T_7, VWFSVGPAWSC, (SEQIDNO:6898); CCDS47246.1, Del, T_7, WNYMKLLLEQQ, (SEQIDNO:6899); CCDS7130.1, Ins, T_7, YCKICKMQKHI, (SEQIDNO:6900); CCDS31591.1, Ins, T_7, CLLHLLLVLH, (SEQIDNO:6902); CCDS8961.1, Ins, T_7, CPHYILGSIC, (SEQIDNO:6903); CCDS41326.1, Del, T_7, EILRRSSLGR, (SEQIDNO:6904); CCDS3671.1, Ins, T_7, ESTRFLAFKE, (SEQIDNO:6905); CCDS2093.1, Del, T_7, FFTLCMRSLS, (SEQIDNO:6906); CCDS4902.1, Del, T_7, FKWSSGKILI, (SEQIDNO:6907); CCDS9336.1, Ins, T_7, FLLSIKCFSK, (SEQIDNO:6908); CCDS11685.1, Del, T_7, FMDYHLYFLL, (SEQIDNO:6909); CCDS7980.1, Ins, T_7, FYFWNVVNYI, (SEQIDNO:6911); CCDS7419.1, Ins, T_7, GEGSSVAGSN, (SEQIDNO:6912); CCDS31373.1, Ins, T_7, GHVGSHRHCT, (SEQIDNO:6913); CCDS12315.1, Del, T_7, KRWIRKIKCI, (SEQIDNO:6915); CCDS47430.1, Del, T_7, KVLSPRCFEH, (SEQIDNO:6916); CCDS31514.1, Del, T_7, LCLEARSVSF, (SEQIDNO:6917); CCDS31028.1, Ins, T_7, LEVFAWRKLH, (SEQIDNO:6918); CCDS5907.1, Del, T_7, LFFCSSFITF, (SEQIDNO:6919); CCDS5933.1, Del, T_7, LLEKVGLNFV, (SEQIDNO:6920); CCDS41943.1, Ins, T_7, LPDCNLPGNS, (SEQIDNO:6921); CCDS45955.1, Del, T_7, LVFWTPSCTI, (SEQIDNO:6922); CCDS31162.2, Del, T_7, LVWLKCTFQT, (SEQIDNO:6924); CCDS8132.1, Del, T_7, LWTQPTWPRS, (SEQIDNO:6925); CCDS13261.1, Del, T_7, MGSVLMQCIC, (SEQIDNO:6926); CCDS2602.1, Ins, T_7, PGDSSGAVHK, (SEQIDNO:6927); CCDS31083.1, Del, T_7, QEAIPSEGFK, (SEQIDNO:6928); CCDS9159.1, Ins, T_7, QLGSKRQGRF, (SEQIDNO:6929); CCDS2200.1, Ins, T_7, SCNFLRRGIL, (SEQIDNO:6930); CCDS45955.1, Del, T_7, SFLHVWMTCS, (SEQIDNO:6931); CCDS56026.1, Del, T_7, TEQNLVIKKN, (SEQIDNO:6932); CCDS14428.1, Del, T_7, TKFEMQYLAR, (SEQIDNO:6933); CCDS55317.1, Del, T_7, TQTPDKVSKP, (SEQIDNO:6934); CCDS7335.1, Del, T_7, TQTWGSGTTK, (SEQIDNO:6935); CCDS34640.1, Del, T_7, TRVQKREKNS, (SEQIDNO:6936); CCDS7214.1, Ins, T_7, VYPWCWNEWK, (SEQIDNO:6938); CCDS7121.1, Ins, T_7, YHLISCWSCW, (SEQIDNO:6939); CCDS1594.1, Ins, T_7, YQERANKKQW, (SEQIDNO:6940); CCDS30587.1, Del, T_7, AGRAMGRAP, (SEQIDNO:6941); CCDS31502.1, Ins, T_7, ASLLGNTVG, (SEQIDNO:6942); CCDS55405.1, Ins, T_7, CKTSHRQMC, (SEQIDNO:6943); CCDS43328.1, Ins, T_7, CSFASESSC, (SEQIDNO:6944); CCDS41771.1, Del, T_7, DQLMIFGLY, (SEQIDNO:6945); CCDS30963.1, Del, T_7, DRVMMKKEL, (SEQIDNO:6946); CCDS1836.1, Del, T_7, EMILFLMTF, (SEQIDNO:6947); CCDS55039.1, Ins, T_7, ESSLLCYQR, (SEQIDNO:6948); CCDS56540.1, Ins, T_7, FHPVFFHIY, (SEQIDNO:6949); CCDS9813.1, Ins, T_7, FIHLLGGSV, (SEQIDNO:6950); CCDS31400.1, Ins, T_7, FILDTSFWP, (SEQIDNO:6951); CCDS31281.1, Ins, T_7, FKRSSKVPK, (SEQIDNO:6952); CCDS44927.1, Ins, T_7, FLLFKREIE, (SEQIDNO:6953); CCDS643.1, Ins, T_7, FLLSRSRGY, (SEQIDNO:6954); CCDS34355.1, Ins, T_7, FSLFAYKHN, (SEQIDNO:6956); CCDS776.1, Del, T_7, FSQKELREH, (SEQIDNO:6957); CCDS14238.1, Del, T_7, FVCSFLFLS, (SEQIDNO:6958); CCDS46097.1, Del, T_7, GAQALLNMR, (SEQIDNO:6959); CCDS47443.1, Ins, T_7, GKTWRQNDR, (SEQIDNO:6960); CCDS11010.1, Del, T_7, GSQQMRLGT, (SEQIDNO:6962); CCDS12936.1, Ins, T_7, GVFSNWPFK, (SEQIDNO:6963); CCDS34226.1, Del, T_7, HIFLSLKAT, (SEQIDNO:6964); CCDS2362.1, Del, T_7, IAFSSQLFL, (SEQIDNO:6965); CCDS47510.1, Del, T_7, ICLFYLCLF, (SEQIDNO:6966); CCDS31021.1, Del, T_7, IPVIPAPIC, (SEQIDNO:6967); CCDS54212.1, Del, T_7, IVNQTPHLQ, (SEQIDNO:6968); CCDS42411.1, Del, T_7, KVMKLKISF, (SEQIDNO:6969); CCDS14489.1, Ins, T_7, LFKRISSVC, (SEQIDNO:6971); CCDS42375.1, Ins, T_7, LGAMGNNDM, (SEQIDNO:6972); CCDS14487.1, Ins, T_7, LIQRVWSLC, (SEQIDNO:6973); CCDS45146.1, Ins, T_7, LQLPLPGSL, (SEQIDNO:6974); CCDS41550.1, Del, T_7, MSCFSTMST, (SEQIDNO:6976); CCDS907.1, Del, T_7, NISLREQHV, (SEQIDNO:6977); CCDS1945.1, Del, T_7, PAWEPVLNP, (SEQIDNO:6978); CCDS33549.1, Del, T_7, PCGNLIEAP, (SEQIDNO:6979); CCDS34701.1, Del, T_7, PCTRLGLGC, (SEQIDNO:6980); CCDS34867.1, Del, T_7, PKSSPLFRM, (SEQIDNO:6981); CCDS6195.1, Ins, T_7, PLTKRTVLS, (SEQIDNO:6982); CCDS45501.1, Del, T_7, PSILIKSYI, (SEQIDNO:6983); CCDS583.1, Ins, T_7, PTDWSIYGL, (SEQIDNO:6984); CCDS14073.1, Del, T_7, QNSSGLIPH, (SEQIDNO:6985); CCDS7588.1, Del, T_7, QVMVVGIVL, (SEQIDNO:6986); CCDS47584.1, Del, T_7, QWISIRPRR, (SEQIDNO:6987); CCDS1536.1, Ins, T_7, RKSIAMHCH, (SEQIDNO:6988); CCDS2211.1, Del, T_7, RKSLATTVP, (SEQIDNO:6989); CCDS10094.1, Del, T_7, RVRIITLRT, (SEQIDNO:6990); CCDS4455.1, Ins, T_7, SGRAERTWL, (SEQIDNO:6991); CCDS34917.1, Ins, T_7, SQLLCPKAA, (SEQIDNO:6992); CCDS2222.1, Ins, T_7, SRAYGIFCN, (SEQIDNO:6993); CCDS5783.1, Ins, T_7, SRIKFGRLS, (SEQIDNO:6994); CCDS14781.1, Ins, T_7, SRWTNNIFH, (SEQIDNO:6995); CCDS31513.1, Del, T_7, SWELPITQR, (SEQIDNO:6996); CCDS58423.1, Del, T_7, TCLKMSIGA, (SEQIDNO:6997); CCDS53953.1, Del, T_7, TIDLVTTCI, (SEQIDNO:6998); CCDS34789.1, Del, T_7, TNKMKTQNV, (SEQIDNO:7000); CCDS9347.1, Del, T_7, TPESLYWLS, (SEQIDNO:7001); CCDS41399.1, Del, T_7, TPQVLPLTS, (SEQIDNO:7002); CCDS47834.1, Del, T_7, TSFHLFHTA, (SEQIDNO:7003); CCDS41343.1, Del, T_7, VKKSRSHFR, (SEQIDNO:7005); CCDS6778.1, Del, T_7, WRDFPSTQG, (SEQIDNO:7006); CCDS44717.1, Ins, T_7, WTSLQLLHI, (SEQIDNO:7007); CCDS103.1, Ins, T_7, WTYGSCCSF, (SEQIDNO:7008); CCDS605.1, Ins, T_7, YGNGICVWR, (SEQIDNO:7009); CCDS55121.1, Del, T_7, YLPEIIRIQ, (SEQIDNO:7010); CCDS6047.1, Ins, T_7, YRKVLAGAS, (SEQIDNO:7011); CCDS4356.1, Del, T_7, ARPGLMRG, (SEQIDNO:7012); CCDS30777.1, Ins, T_7, CQADPKCH, (SEQIDNO:7013); CCDS1285.1, Ins, T_7, CQLPFALR, (SEQIDNO:7014); CCDS45485.1, Ins, T_7, CRHGRRTI, (SEQIDNO:7015); CCDS45060.1, Ins, T_7, CVAPLLFC, (SEQIDNO:7016); CCDS31772.1, Ins, T_7, FFSSSPVS, (SEQIDNO:7017); CCDS58947.1, Del, T_7, FIIVSSHS, (SEQIDNO:7018); CCDS41769.1, Ins, T_7, FLSSSPVS, (SEQIDNO:7019); CCDS12216.2, Ins, T_7, FQGTNLPF, (SEQIDNO:7021); CCDS30902.1, Ins, T_7, FRKTRDLK, (SEQIDNO:7022); CCDS47069.1, Ins, T_7, FRLQNPQR, (SEQIDNO:7023); CCDS2154.1, Ins, T_7, FSAWKEDL, (SEQIDNO:7024); CCDS9383.1, Ins, T_7, FVFSCLLI, (SEQIDNO:7025); CCDS14231.1, Ins, T_7, FWSSCKRP, (SEQIDNO:7026); CCDS7179.1, Ins, T_7, GKSCSAFA, (SEQIDNO:7027); CCDS58060.1, Ins, T_7, GNVGGQGL, (SEQIDNO:7029); CCDS14647.1, Ins, T_7, HLGFCETS, (SEQIDNO:7030); CCDS33817.1, Del, T_7, HMPGTYSS, (SEQIDNO:7031); CCDS861.1, Ins, T_7, IASGLTGV, (SEQIDNO:7032); CCDS1916.1, Del, T_7, IQLKVMKT, (SEQIDNO:7033); CCDS4379.2, Del, T_7, LADLSLTK, (SEQIDNO:7035); CCDS12149.2, Del, T_7, LCHRLRCL, (SEQIDNO:7036); CCDS31285.1, Ins, T_7, LCNSVCSQ, (SEQIDNO:7037); CCDS30735.1, Ins, T_7, LLFTWQVF, (SEQIDNO:7038); CCDS47907.1, Ins, T_7, LPTRKFPL, (SEQIDNO:7039); CCDS3707.1, Del, T_7, LVFGAHSF, (SEQIDNO:7040); CCDS4984.1, Del, T_7, LVPLSFLP, (SEQIDNO:7041); CCDS34071.1, Del, T_7, MAMFQQKT, (SEQIDNO:7043); CCDS43686.1, Del, T_7, MFHYFLTF, (SEQIDNO:7044); CCDS45430.1, Del, T_7, MMIITGRC, (SEQIDNO:7045); CCDS5685.1, Del, T_7, MWSRDCLY, (SEQIDNO:7046); CCDS6947.1, Ins, T_7, PFDGIEYF, (SEQIDNO:7047); CCDS58463.1, Del, T_7, PWGLDLEY, (SEQIDNO:7048); CCDS13737.1, Del, T_7, QSPSITTF, (SEQIDNO:7049); CCDS5777.1, Del, T_7, RESLSAVE, (SEQIDNO:7050); CCDS3499.1, Del, T_7, RLHFLHYL, (SEQIDNO:7051); CCDS42173.1, Del, T_7, RQETSTPA, (SEQIDNO:7052); CCDS41963.1, Del, T_7, RSLLLTCC, (SEQIDNO:7053); CCDS34917.1, Del, T_7, RVHYGLYY, (SEQIDNO:7054); CCDS43235.1, Ins, T_7, SCYLPSYL, (SEQIDNO:7055); CCDS34490.1, Ins, T_7, SEIVSFRW, (SEQIDNO:7056); CCDS7407.1, Del, T_7, SNRMKWKY, (SEQIDNO:7057); CCDS14238.1, Ins, T_7, SPSALGHL, (SEQIDNO:7058); CCDS7647.1, Ins, T_7, SRKSILEI, (SEQIDNO:7060); CCDS31997.1, Del, T_7, SSLHLTKK, (SEQIDNO:7061); CCDS3569.1, Ins, T_7, STKAQNPF, (SEQIDNO:7062); CCDS33268.1, Ins, T_7, VSKRRCYP, (SEQIDNO:7063); CCDS55171.1, Ins, T_7, YARDMQKL, (SEQIDNO:7064); CCDS3609.1, Del, T_7, YWECSAAC, (SEQIDNO:7065); CCDS34627.1, Ins, T_8, SQHECRSPTGHCLPESPSPEPRLPGCCGPCTVPGRSTAQRGQARGGPPGSAVAHQGISDTRAP CSTAGPPGDFGC, (SEQIDNO:7067); CCDS3974.1, Del, T_8, CRASLRTDCLLAVVIQFQISTYQAQLRISLRKQLLLFLVAKKTKQDILTHIIECLKKMKTSTSL GMI, (SEQIDNO:7068); CCDS44248.1, Ins, T_8, FRDPRDGIPGPQRPLPLRGIQGEVWQAQQEERVQRGAVGDREQPYCQGFRLSVLPEKELCGR A, (SEQIDNO:7070); CCDS12270.1, Del, T_8, GPVYYVCMKELTLERNRMNVSSVLKPFLFTVPIEDMRECTLGRNRMNVSSVLKPCLIPVPI, (SEQIDNO:7071); CCDS53391.1, Ins, T_8, CNLPSDIVGECTYCLHNMACSKPSSSHVLFPWPSLFPGAMVHQCHHSSALGSLSYPGW, (SEQIDNO:7073); CCDS35316.1, Del, T_8, ILMMMTICHTVLPGGGIPPMTMRATRMAWQEEGEARVQVAIPSQSTLKTNGKRGVTK, (SEQIDNO:7074); CCDS41653.1, Ins, T_8, YHCRSLPNKSNKVSDSIWKTWEGSITYFTVLLRFGIFYCTYTLNIQLFQFVSKTK, (SEQIDNO:7075); CCDS31747.1, Ins, T_8, FLTSGQWDIFYVVEQQVHKVCHVSLKCLSLVPLIYSHSGKQQAATDSCEATVAS, (SEQIDNO:7076); CCDS31820.1, Ins, T_8, FSCGNTDGHLDISYSLLHKHHPDNSENSFYESKEKSLFHLLLPYDSCLHLLQ, (SEQIDNO:7077); CCDS46554.1, Del, T_8, IFWNIKIRLLAWKESTIFQMLIIEKVLKIHKSMIQMKTHSRNITVQKNKNT, (SEQIDNO:7078); CCDS31698.1, Del, T_8, SVFVLFLNATCWQPWPAIATWPSAAHCSTGSSCPLGSVLCWWLLSSQ, (SEQIDNO:7079); CCDS31571.1, Ins, T_8, YHCRSLPNKSNKVSDSIWNYKYFNHKHHLHNYYNYCSNSNNNRVVSF, (SEQIDNO:7080); CCDS8623.1, Ins, T_8, FLLLHRCSHGNPFHYYGNNMECCIPKLIIQPRSSNSLDRKLLWPMS, (SEQIDNO:7081); CCDS30638.1, Del, T_8, LLAHMYGFSTYGTQKGECVCLQCLSGSSLFILKQPLDLKISKTFM, (SEQIDNO:7082); CCDS53554.1, Del, T_8, CRRLVSTLNFSAHLLMWTPCTVTWPICTLSSSKTHSTSMHMQQS, (SEQIDNO:7083); CCDS43042.1, Ins, T_8, WHRSISGKALHTNYHAYNLHEPKCISGAGDAIHAESVHHHFPP, (SEQIDNO:7085); CCDS763.1, Ins, T_8, FIPQTDNEIFTRKCCSVYSSPWHSFHYCTDHSLEFTYEVNWK, (SEQIDNO:7086); CCDS3271.1, Ins, T_8, FLIVFETFPHDTKLVLEPLGPNSEHSHRAVLLLSSYRFDPI, (SEQIDNO:7087); CCDS44398.1, Del, T 8, QPPKALQPPRAPQLPNAPQPPSIPQPPRAPQPLKLLQCL, (SEQIDNO:7088); CCDS 12232.1, Del, T 8, WRPPTRELGARWDAGLSGRKAPRVALSRPPGPQCPSGCA, (SEQIDNO:7089); CCDS14635.1, Del, T_8, EQKSLSINVQNVSTRLPLECTHSVGYLESLLFTSNAIA, (SEQIDNO:7090); CCDS43437.1, Del, T_8, CGCGHLSTLPSWPCKAFALASALGGAGASFFVLGTIL, (SEQIDNO:7091); CCDS47161.1, Del, T_8, CLRMMMWEKELEVCALFWRRQRQKAIPTLNVIVMSLQ, (SEQIDNO:7092); CCDS7404.2, Ins, T 8, CGVPDILHSGLRTNGMDPFHCRLCDHALCSTWECCQ, (SEQIDNO:7093); CCDS35401.1, Ins, T_8, FRTIYFNYHHRWKQVNKYCWCSRRAPSSFEFPSAWK, (SEQIDNO:7094); CCDS9032.1, Ins, T_8, SSWICCCRASIICSQHGVLYFGSPWMAENINKKCI, (SEQIDNO:7095); CCDS7761.1, Del, T_8, CVGCQFIVPTRGAPLMARVHTEHSRVLLSPSFTC, (SEQIDNO:7096); CCDS3155.1, Ins, T_8, LFSFPCWNYWKLFCNLGFYTEEYESQVCEHLLN, (SEQIDNO:7097); CCDS6244.1, Ins, T_8, FWSFCVLQFNWSDERCDWSSYSQSELLPRLHG, (SEQIDNO:7098); CCDS32208.1, Del, T_8, PLENTQIPRTSPLWKITMVLTYPNCVVHVLMF, (SEQIDNO:7099); CCDS45750.1, Del, T_8, SSLLNFLRVQYIWRKLQMYFVFYKQSSLPCSL, (SEQIDNO:7100); CCDS9086.1, Ins, T_8, FLLFYWDVCVLLRSLADLCFRHVEIWKSGCN, (SEQIDNO:7101); CCDS1800.1, Ins, T_8, FGRCNCCDKVCITGWEPIYAVHETDFKGKA, (SEQIDNO:7102); CCDS9879.1, Ins, T_8, FPKDKKICTHGQPVHLDIGNHLQCCHVVGR, (SEQIDNO:7103); CCDS43187.1, Del, T_8, GSNSNLGMKCGIQNQMLVIQQEAGFGILHT, (SEQIDNO:7104); CCDS43968.1, Del, T_8, PWTLKKLLKTWLQMIIHLTYQRQLIDSSAT, (SEQIDNO:7105); CCDS3895.1, Del, T_8, QSRIPSKKCSLRKSTKSYKSCTNLRNLAPT, (SEQIDNO:7106); CCDS47753.1, Del, T_8, ESLPAFLRCHLKTEVPCGLWLRSMGEIAS, (SEQIDNO:7107); CCDS3123.1, Ins, T_8, FEEKWCSSIPAKQSWRKHDVGNLSGCRIR, (SEQIDNO:7108); CCDS33705.1, Del, T_8, LCFSCLDWIASLLKLKDRSHPWLIFTHPS, (SEQIDNO:7110); CCDS42040.1, Del, T_8, SKTQRRYKLSSLKEDLLQRSAFTSQAPLY, (SEQIDNO:7111); CCDS46062.1, Ins, T_8, LWESLYPEVTPHRTSENTHRRETLCLQ, (SEQIDNO:7113); CCDS8411.1, Del, T_8, SPLWDCSTTTGTHHGCLWEIPSVTLLA, (SEQIDNO:7114); CCDS6460.1, Ins, T_8, FPPARSLFTTEPIFQSSRGLMDGGSY, (SEQIDNO:7116); CCDS10239.1, Ins, T_8, LYPPTRPTENKFPTRHKYPKKQTIGK, (SEQIDNO:7117); CCDS46640.1, Del, T_8, SVVATPKTMSLMAVNHRALFFVAVNH, (SEQIDNO:7118); CCDS31112.1, Del, T_8, YMWSFCFSPLWLMTAMWLSASHFTTL, (SEQIDNO:7119); CCDS6343.1, Del, T_8, TKLMVWLQYGQAGKIRFTVLLFPPC, (SEQIDNO:7120); CCDS1331.1, Del, T_8, CPAWIPTTRLTFVSKLWRYLFMRS, (SEQIDNO:7121); CCDS3895.1, Ins, T_8, FSQESLQRNVHSGRVQNLTRAVPI, (SEQIDNO:7122); CCDS54989.1, Del, T_8, SPVASRGWVKTLRAPTSTITGCPS, (SEQIDNO:7123); CCDS2621.1, Ins, T_8, YWSHWQCFDRQHNPLHPQDGDSRV, (SEQIDNO:7124); CCDS43437.1, Ins, T_8, FVAVDIFQHCLLGLAKPSLWLRL, (SEQIDNO:7125); CCDS1585.1, Del, T_8, SLKRMRESPQSLPNSLTSLRSWR, (SEQIDNO:7126); CCDS33511.1, Del, T_8, TLSCLGIFPDGHIYFDFYDLLFC, (SEQIDNO:7127); CCDS55323.1, Del, T_8, MNSVNLSSPKARDWTVSELLKN, (SEQIDNO:7129); CCDS9086.1, Del, T_8, SALLLGCLCFIALIGRLMFQAC, (SEQIDNO:7130); CCDS43661.1, Del, T_8, VTEGNCSNYPAIILFSRSLSSS, (SEQIDNO:7131); CCDS3687.1, Ins, T_8, WNGKHWITHGRGCGPIPDHLPS, (SEQIDNO:7132); CCDS9676.1, Del, T_8, CGEVLDRLGVGFMWDERKSLL, (SEQIDNO:7133); CCDS10123.1, Del, T_8, LELILETSAQKLLCWLCKKMD, (SEQIDNO:7135); CCDS34167.1, Del, T_8, LHYLMRWPINLIRRWTSFWEI, (SEQIDNO:7136); CCDS9032.1, Del, T_8, QLDLLLPSEYYMFPAWGSVFW, (SEQIDNO:7137); CCDS8684.1, Del, T_8, SCRKIQINHKKKEKFHYHCMC, (SEQIDNO:7138); CCDS34057.1, Del, T_8, TLNSLRWPLQLSNLAGSQISW, (SEQIDNO:7139); CCDS12934.1, Ins, T_8, FGFWLVVVSEGAQKGRVLEI, (SEQIDNO:7140); CCDS41399.1, Ins, T_8, FGIECGEDREDWNSTSVAAI, (SEQIDNO:7141); CCDS4065.1, Ins, T_8, FLCQSNGSVDCNRKLSGIWT, (SEQIDNO:7142); CCDS31434.1, Del, T_8, SRRNRRYRKIYGELKMSLQA, (SEQIDNO:7143); CCDS7404.2, Del, T_8, WSSRHTSLRITHKRHGSIPL, (SEQIDNO:7144); CCDS45503.1, Ins, T_8, YSNICANPSPLADLFTLALT, (SEQIDNO:7145); CCDS43042.1, Del, T_8, APLNQRKSSTYKLPRLQSP, (SEQIDNO:7146); CCDS34627.1, Del, T_8, ATRMPEPHRALPTRVPFP, (SEQIDNO:7147); CCDS3084.1, Ins, T_8, FLPSSNAHRSKEGSCHSR, (SEQIDNO:7148); CCDS55673.1, Del, T_8, TLILSTSQELCVIYVKEV, (SEQIDNO:7149); CCDS1308.1, Del, T_8, EIFGATESTFLFLEIYM, (SEQIDNO:7150); CCDS12320.1, Ins, T_8, FHCIWMPGQFAPDHDGL, (SEQIDNO:7151); CCDS31820.1, Ins, T_8, FHLLGGDRILPSGCHVL, (SEQIDNO:7152); CCDS9491.1, Ins, T_8, FLDSFVHHDWVLLGHYS, (SEQIDNO:7153); CCDS31821.1, Ins, T_8, FNSFGINRIFSSGCHVL, (SEQIDNO:7154); CCDS31747.1, Del, T_8, PYKWPMGYFLCCGTTST, (SEQIDNO:7155); CCDS34002.1, Del, T_8, SRNSTLLIEENLHHSKN, (SEQIDNO:7156); CCDS11901.1, Del, T_8, AMTDDQRMVKNKQLLM, (SEQIDNO:7157); CCDS3687.1, Del, T_8, EWQALDYSRSWLWTDT, (SEQIDNO:7158); CCDS12232.1, Ins, T_8, FGGRLPGSWERAGTPD, (SEQIDNO:7159); CCDS5352.1, Ins, T_8, FHPDLWNQSWHYNKSK, (SEQIDNO:7160); CCDS32008.1, Del, T_8, SRSHLDFSPYWMKKVK, (SEQIDNO:7162); CCDS5933.1, Del, T_8, WCTAVVAPTYFLHFTH, (SEQIDNO:7163); CCDS14570.1, Ins, T_8, FHAFNFGSRFSVCFD, (SEQIDNO:7164); CCDS35057.1, Del, T_8, MNSVNLSSPKASLLR, (SEQIDNO:7165); CCDS31424.1, Ins, T_8, YWYFSRCRTSFFCSH, (SEQIDNO:7166); CCDS32086.1, Ins, T_8, FATWSALVRLFPVE, (SEQIDNO:7167); CCDS10141.1, Ins, T_8, FEFCDIQLSMGSSR, (SEQIDNO:7168); CCDS31434.1, Ins, T_8, FPGEIADTERSMEN, (SEQIDNO:7169); CCDS43612.1, Ins, T_8, FPLFLCQRCDSSIC, (SEQIDNO:7170); CCDS3892.1, Ins, T_8, FQFSCCQSKLHSTG, (SEQIDNO:7171); CCDS31112.1, Ins, T_8, CICGASVSHHYGS, (SEQIDNO:7172); CCDS1515.1, Ins, T_8, FQFAEWENFNTSR, (SEQIDNO:7173); CCDS5729.1, Del, T_8, SQEILVIALLLKR, (SEQIDNO:7174); CCDS45014.1, Del, T_8, TLSCLGIFPDGHI, (SEQIDNO:7175); CCDS1308.1, Ins, T_8, WKFSGQQNPLFYF, (SEQIDNO:7176); CCDS7106.1, Ins, T_8, YALLKASWKRKQS, (SEQIDNO:7177); CCDS45085.1, Del, T_8, YSASGHGVRITKS, (SEQIDNO:7179); CCDS32086.1, Del, T_8, CHLVSTCQTIPS, (SEQIDNO:7180); CCDS9231.1, Ins, T_8, CQTQARVDGYNR, (SEQIDNO:7181); CCDS32008.1, Ins, T_8, FPEAIWISHLIG, (SEQIDNO:7182); CCDS8632.1, Ins, T_8, FPILYFFYFDDL, (SEQIDNO:7183); CCDS31561.1, Del, T_8, SLSAWVCLSASS, (SEQIDNO:7184); CCDS14624.1, Ins, T_8, YYIRAAKEASNL, (SEQIDNO:7185); CCDS3974.1, Ins, T_8, FAGLHSGQTVF, (SEQIDNO:7186); CCDS34097.1, Ins, T_8, FIQVRSCRKRS, (SEQIDNO:7187); CCDS5729.1, Ins, T_8, FRRRYWLLPCF, (SEQIDNO:7188); CCDS46580.1, Ins, T_8, FVGTRTSTSEK, (SEQIDNO:7189); CCDS1792.1, Del, T_8, ILKQVARMCLI, (SEQIDNO:7190); CCDS34146.2, Ins, T_8, LHSSIYKMSFS, (SEQIDNO:7191); CCDS12320.1, Del, T_8, SLHLDAWTICS, (SEQIDNO:7192); CCDS4697.1, Del, T_8, VVCLSRRMKRC, (SEQIDNO:7193); CCDS47753.1, Ins, T_8, WNHCLPFSGVI, (SEQIDNO:7194); CCDS31551.1, Del, T_8, WWDSMTSFLSW, (SEQIDNO:7195); CCDS1331.1, Ins, T_8, FALPGYLPQG, (SEQIDNO:7196); CCDS861.1, Ins, T_8, FHDVCGRENI, (SEQIDNO:7197); CCDS53693.1, Ins, T_8, FNFLKEEQSL, (SEQIDNO:7198); CCDS4446.1, Ins, T_8, FTAQPCESTH, (SEQIDNO:7199); CCDS11295.1, Del, T_8, IEQNLIGKKN, (SEQIDNO:7200); CCDS14520.1, Ins, T_8, LAPCNFYLFT, (SEQIDNO:7201); CCDS46472.1, Del, T_8, NHQKGKFMNF, (SEQIDNO:7202); CCDS58915.1, Del, T_8, QQFTLRSFSI, (SEQIDNO:7203); CCDS33817.1, Del, T_8, SVLRTSLKSF, (SEQIDNO:7204); CCDS4447.1, Del, T_8, TTLSFVLIEE, (SEQIDNO:7205); CCDS47894.1, Del, T_8, CRNMKQQIY, (SEQIDNO:7206); CCDS47622.1, Ins, T_8, FALCSRKNL, (SEQIDNO:7207); CCDS54989.1, Ins, T_8, FLRSHPGAG, (SEQIDNO:7208); CCDS42040.1, Ins, T_8, FPRLKEDTS, (SEQIDNO:7209); CCDS41355.1, Ins, T_8, FQKCIYINS, (SEQIDNO:7210); CCDS6201.1, Del, T_8, RMRKWRDQI, (SEQIDNO:7211); CCDS33705.1, Ins, T_8, YYASLAWTG, (SEQIDNO:7212); CCDS53554.1, Ins, T_8, FAEGLSQL, (SEQIDNO:7213); CCDS31561.1, Ins, T_8, FLCRHGSV, (SEQIDNO:7214); CCDS3883.1, Ins, T_8, FRRVREVP, (SEQIDNO:7215); CCDS43328.1, Ins, T_8, FSHQHGRN, (SEQIDNO:7216); CCDS45085.1, Ins, T_8, FTVPVGTA, (SEQIDNO:7217); CCDS46286.1, Del, T_8, KLQEANGI, (SEQIDNO:7219); CCDS12270.1, Ins, T_8, LAQFITYA, (SEQIDNO:7220); CCDS6244.1, Del, T_8, LEFLCSPV, (SEQIDNO:7221); CCDS2621.1, Del, T_8, LVSLAMFR, (SEQIDNO:7223); CCDS12934.1, Del, T_8, RILACCGI, (SEQIDNO:7224); CCDS43612.1, Del, T_8, SFIPLSEM, (SEQIDNO:7225); CCDS4065.1, Del, T_8, SLSKQWQC, (SEQIDNO:7226); CCDS8632.1, Del, T_8, SYTIFLLF, (SEQIDNO:7227); CCDS6896.2, Del, T_8, TGLTRMMR, (SEQIDNO:7228); CCDS4697.1, Ins, T_8, WWSVYQEG, (SEQIDNO:7229); CCDS35114.1, Del, T_9, FRSPSWSLPFPGSSCLSPSPGLCLGVQSSGQDSRERVWLQPAPLLPAAQDAGWGTGARVVPPT DQSAAPRWGALQRCSEACTSLATGTCRGDTR, (SEQIDNO:7230); CCDS43176.1, Ins, T_9, FVYVCDAVAGCAAGPHQHRPHHHHGADDRSYARADSPSLCGSRHLLCCPVNGAVPVYGQT GI, (SEQIDNO:7231); CCDS55691.1, Ins, T_9, FCRSPSRHCYGIQDIKNHLVLSNSQWQTARINRWLISCCCIPIHCGGIQFFPKILQ, (SEQIDNO:7232); CCDS44954.1, Del, T_9, LSAIRKMTYFTSWILDSDSTHYIPSCNRKSCYKRMWS, (SEQIDNO:7233); CCDS54758.1, Del, T_9,LPILMMPSKKLILYLFLCCPTLSFWQREQPSRT, (SEQIDNO:7234); CCDS8942.1, Del, T_9, CYLAGSKMTSLTSGWNGKRQSCSQLCLLPCTI, (SEQIDNO:7235); CCDS43176.1, Del, T_9, CLRVRCGGWLCGWTSSASPSSPPRG, (SEQIDNO:7236); CCDS2223.2, Ins, T_9, FADAEESIGSLCDICSCQECPCCP, (SEQIDNO:7237); CCDS8638.1, Ins, T_9, FNSNNGFICSRKLCQWLHSTGKFH, (SEQIDNO:7238); CCDS5044.1, Ins, T_9, FPSTLDFGVLYFNFMLYLGDVSTE, (SEQIDNO:7239); CCDS9457.1, Del, T_9, YMKKNLTPSFAMKYLPPICTLGDQ, (SEQIDNO:7240); CCDS44615.1, Ins, T_9, FYHLWLSINRHREKVNQAFGA, (SEQIDNO:7241); CCDS30978.1, Del, T_9, SIPHVPKVTAAHSVTVKLQ, (SEQIDNO:7242); CCDS10926.1, Ins, T_9, FCNVRQSERWNSLLPKQK, (SEQIDNO:7243); CCDS449.1, Ins, T_9, FICCINWSKGAFCCIWFL, (SEQIDNO:7244); CCDS33545.1, Ins, T_9, FSIWVTSTCFSTDMGTS, (SEQIDNO:7245); CCDS3455.1, Del, T_9, LPILMMPSKKLILYLFL, (SEQIDNO:7246); CCDS5605.1, Ins, T_9, FNPNWSFQCWPGCPMY, (SEQIDNO:7247); CCDS35114.1, Ins, T_9, FSAPPPGPSHFLAPPV, (SEQIDNO:7248); CCDS55624.1, Ins, T_9, FCTIGRKMEKQSDL, (SEQIDNO:7249); CCDS55691.1, Del, T_9, LPLTFSTLLWDSRH, (SEQIDNO:7250); CCDS44615.1, Del, T_9, LSSLALYQSPQRKG, (SEQIDNO:7251); CCDS3500.1, Ins, T_9, FSNHDRWKHNICV, (SEQIDNO:7252); CCDS55621.1, Ins, T_9, FVPLAERWKNSL, (SEQIDNO:7254); CCDS2223.2, Del, T_9, CRCRREYRVTM, (SEQIDNO:7255); CCDS55621.1, Del, T_9, CTIGRKMEKQS, (SEQIDNO:7256); CCDS58143.1, Ins, T_9, FNLVFSPILSL, (SEQIDNO:7257); CCDS55624.1, Del, T_9, LYHWPKDGKTV, (SEQIDNO:7258); CCDS55780.1, Ins, T_9, FCWILSCKT, (SEQIDNO:7259); CCDS33545.1, Del, T_9, FNLGYLNLF, (SEQIDNO:7260); CCDS30978.1, Ins, T_9, FLFHMYQR, (SEQIDNO:7261); CCDS47916.1, Del, T_9, FTIGITWI, (SEQIDNO:7262); CCDS8942.1, Ins, T_9, FVIWLGAR, (SEQIDNO:7263); CCDS700.1, Del, T_9, LILWSSRQ, (SEQIDNO:7264).

### EXAMPLES

The following examples are given for the purpose of illustrating various cases of the invention and are not meant to limit the present invention in any fashion. The present examples, along with the methods described herein are presently representative of preferred cases, are exemplary. Changes therein and other uses within the spirit of the invention as defined by the scope of the claims will occur to those skilled in the art.

### Example 1: Screening Human Cancer Patients For Vaccine Candidates

The peptide sequence of the FSP array was from our human or dog FS database. It includes the most of the MS FS antigens (homopolymer size >6nt, peptide size >9 a.a) and potential FSP caused by mis-splicing of each exon. All FS antigens were segmented into 10 to 15 a.a long peptides. The array was synthesized by Nimblegen with photolithography. There were about 400K peptides of each FSP array, including ~14K MS FSPs. Total 99 cancer serum from 6 different cancer and 64 noncancer normal controls were analyzed with the 400K array **(****FIG. 8A****).** Cancer patients had much higher IgG reactive to the FSPs. However, there is still a small portion FSPs don't have IgG positive reactive in cancers **(****FIG. 8A****).** It was determined that positive IgG reactive FSPs have better protection **(****FIG. 9****,** **FIG. 10****,** **FIG. 11****).** Based on the IgG reactivity to FSPs, 20 best reactive FS antigens can be selected for each cancer patient as personalized cancer vaccine, including GBM and pancreatic cancer patients, which have low or no DNA neo-antigens for their personalized cancer vaccine **(****FIG. 8B** and **FIG. 8C****).**

### Example 2: Screening Dog Cancer Patients For Vaccine Candidates

Blood from dogs with various cancers was used to screen vaccine candidate peptides. 830 spotted peptides corresponding to 374 different FS antigens from 209 genes in dogs. Sera from dogs with 8 different cancers, (N=116), including mammary cancer, were compared to sera from dogs without cancer (N=52). Results of the screen are shown in **FIG. 9****.** Red spots indicate the normalized florescent value of the peptide was 5 fold higher than in the corresponding sample. There were a total 487 FS peptides had at least one example of 5 fold higher reactivity. The dogs with cancer also showed higher antibody reactive to the FS peptide. Each type of cancer has its own reactive profile, as well as each individual dogs.

Data from individual dogs is shown in **FIG. 13A** and **FIG. 13B****.** Screening of 6 dogs with cancer for specific cancer vaccine candidates. In **FIG. 13A****,** a corner of the scanned FS array of each cancer dog analysis is shown. In **FIG. 13B****.** data is shown of normalized antibody active to each peptide of each cancer dog. These data showed that each dog has personal highly reactive FS peptides.

T-cell reactivity was determined for candidate vaccine peptides. For a specific dog screened using the arrays in **FIG. 13A** and **FIG. 13B****,** 5 FS antigens that were highly antibody reactive were tested in ELI-Spot assays. PBMCs from the dogs' blood were purified, stimulated with pooled peptides from each FS antigen candidates and the number of positive cells counted. The number for positive for each FS antigen stimulant is shown. As can be seen all 5 FS antigens showed significant T-cell reactivity compare to the medium control **(****FIG. 14****).**

### Example 3: Cancer Vaccine for Dogs

A safety trial of FS peptide based cancer vaccine was conducted in dogs. The cancer vaccine was formulated with 21 FS antigens. Three healthy dogs were immunized with the vaccine according to the timeline shown in **FIG. 15A****.** An ELISA analysis was conducted on the antibody reactivity of the immunization, shown in **FIG. 15B****.** ELISPOT analysis was conducted to measure the specific IFNγ releasing PBMCs to the vaccine antigens after immunization, shown in **FIG. 15C****.** Standard clinicopathologic analysis showed the vaccine is safe for these three dog.

### Example 4: Frameshift Peptide Vaccines Protect Against Cancer

To determine whether any MS Indel event could produce a FS in any constitutive gene and could therefore be a protective antigen against most or all tumor types, three predicted MS FSs in mouse genes were selected using the criteria of FS peptide length and predicted H2-D epitopes **(Table 3).** Each FS peptide was tested individually in the 4T1-BALB/c mouse breast tumor model with the prophylactic vaccination of gene vaccine/peptide boost. Each FS peptide vaccination significantly slowed the tumor growth compared to the mock immunized mice **(****FIG. 16A****).** Each FS antigen can elicit specific IFNγ releasing splenocytes **(****FIG. 16B****).** These results indicate that any FSs produced by MS can confer protection. Peptides tested are listed in Table 1.

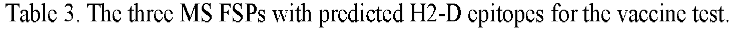

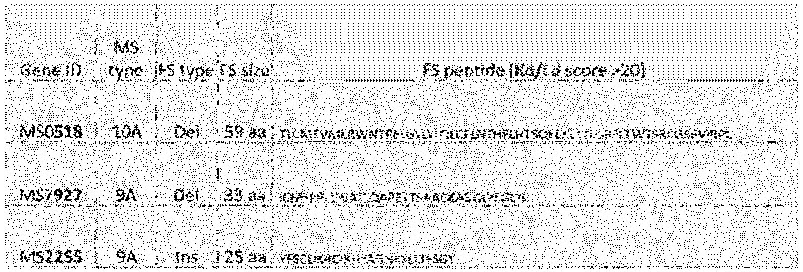

In another demonstration, two different pools of mouse FS antigen of the different MS length were selected (Table 4) and tested in the same mouse tumor model. Both FS pooled vaccine significantly retarded tumor growth comparing to the control group **(****FIG. 17A** and **FIG. 17B****).**

**Table 4. Two pools of the FS antigens from the different MS length.**

| | **Short MS pool** | | | |
|---|---|---|---|---|
| **Ag ID** | **Ref Gene** | **MS type** | **INDEL** | **peptide size (aa)** |
| Mus385 | NM 146792.2 | 8T | Deletion | 32 |
| Mus392 | NM 029998.3 | 8T | Deletion | 51 |
| Mus023 | NM 147003.1 | 8T | Insertion | 19 |
| Mus263 | NM 001114663.1 | 8A | Insertion | 20 |
| Mus942 | NM 001081355.3 | 8A | Insertion | 37 |
| Mus671 | NM 001163189.1 | 8A | Insertion | 49 |
| Mus334 | NM 130448.3 | 8A | Deletion | 29 |
| Mus051 | NM 033618.3 | 8A | Insertion | 26 |
| Mus587 | NM 001081302.1 | 8A | Insertion | 32 |
| MS255 | NM 010086.4 | 9A | Insertion | 24 |
| MS927 | NM 053009.3 | 9A | Deletion | 33 |
| MS518 | NM 153511.3 | 10A | Deletion | 59 |

| | **Long MS pool** | | | |
|---|---|---|---|---|
| **Ag ID** | **Ref Gene** | **MS type** | **INDEL** | **peptide size (aa)** |
| Mus487 | NM 001109759.1 | 10A | Deletion | 44 |
| Mus413 | NM 027009.2 | 10A | Deletion | 27 |
| Mus274 | NM 001081345.2 | 10A | Insertion | 14 |
| Mus694 | NM 028787.4 | 10T | Insertion | 45 |
| Mus281 | NM 001160399.1 | 11A | Deletion | 39 |
| Mus951 | NM 025441.3 | 11A | Deletion | 36 |
| Mus461 | NM 001033226.4 | 13T | Deletion | 22 |
| Mus528 | NM 001034881.3 | 14T | Deletion | 54 |
| Mus414 | NM 028664.1 | 18T | Deletion | 28 |

The FS vaccine was also tested in a mouse ovarian cancer model (ID8-C57BL6). A pool of FSP from 10 MS FS and 3 mis-splicing FS was used to immunize, as well as a pool of peptides from the point DNA mutation if the ID8 cells were immunized the mice after the tumor cell injection. Both vaccines showed the significant protection comparing to the control mice which were immunized with PBS **(****FIG. 18****).** The same sets of the vaccines combined with immune checkpoint treatment also showed the significant protection. This experiment shows our FS variants have the same protection as the neo-antigens from the DNA mutations.

### Example 5: Personalized Vaccine Test in Mouse Tumor Models

Protection by a personalized pool of FS neoantigens was tested in the 4T1 BALB/c tumor model **(****FIG. 19****).** Serum from BALB/c mice (n=10/group) were collected two days previous and 7-days post subcutaneous injection of 4T1 tumor cells (500 cells/mouse). Both sera were run on microarray slides containing peptides for human FS neoantigens and the 10 mouse homologs neoantigens with higher median normalized signal on the array were selected as targets for the vaccines formulations for each mice. Mice were vaccinated subcutaneously on days 12 and 19. For the immunization group with the combined immunotherapy, the antibody treatment, anti-PD-L1 and CTLA-4, were administrated intraperitoneally on days 12, 15, 19, 22. Control group (non-treated) was immunized with PBS at the same schedule as the vaccine groups. Non-reactive groups were immunized with FS antigens without signal in the microarray before and after tumor challenge, and followed the same vaccine regimen as the other groups. The test shows that the personalized positive FSPs selected from the FSP array have significant protection as a vaccine. It also shows again that the negative IgG reactive FSPs don't have the protection.

The personalized cancer vaccine was also tested in a spontaneous mouse breast tumor model **(****FIG. 20****).** Female FVB/N mice (n= 22) were evaluated weekly for the presence of palpable tumor, when tumor was detected sample blood was collected and run on microarray slides containing peptides for human FS neoantigens. The 10 mouse homologs neoantigens with higher median normalized signal compared to age matched FVB/NJ samples on the array were selected as targets for the vaccines formulations for each mice. Mice were vaccinated subcutaneously with pool of Fs peptide with minimum two doses and maximum seven doses, as needed to maintain tumor control, with intervals 1-2 weeks between doses. Each vaccine dose was followed by checkpoint inhibitor treatment (antibody anti-PD-L1 and anti-CTLA-4) (CPI), two doses, three days a part. As a control, animals were immunized with PBS (non-treated) at the same schedule as the vaccine groups or only with CPI treatment (CPI only), twice per animal. Personalized Fs vaccine conferred 68.2 % of protection. Both personalized cancer vaccine tests with our SPCV system showed significant protection in two different mouse tumor models. It suggests the SPCV system may also work with human cancer treatment.

### Example 4: Personalized Cancer Vaccine

An individual presenting with histologically confirmed stage 1 breast cancer is in need of treatment. A blood sample is obtained from the individual and serum from the blood sample is applied to a peptide array having approximately 20,000 spotted FS peptides. After incubation, the array is washed and the patient's bound antibodies are detected using a fluorescently labeled secondary antibody. FS peptides are found to bind specifically to the patient's sera and not to sera from healthy individuals. These reactive FS peptides are prepared in a vaccine preparation including a poly I:C adjuvant and administered to the individual with a checkpoint inhibitor or combination of checkpoint inhibitors after receiving a lumpectomy surgery. The individual is monitored for breast cancer recurrence and is cancer-free for five years.

### Example 5: Clinical Trial for a Personalized Cancer Vaccine

Ten individuals presenting with histologically confirmed stage 1 breast cancer are in need of treatment. Blood samples are obtained from each individual and serum from the blood samples were applied to peptide arrays having approximately 20,000 spotted FS peptides. After incubation, the arrays are washed and the patients' bound antibodies are detected using a fluorescently labeled secondary antibody. FS peptides are found to specifically bind to the patients' sera but not to sera from healthy individuals. Reactive FS peptides are prepared in a vaccine preparation for each patient including a poly I:C adjuvant. The FS vaccine components are chosen from pre-synthesized reagents. Each individual is given their vaccine after receiving lumpectomy surgery. Vaccinated individuals are monitored for breast cancer recurrence and nine of the ten individuals remain cancer free for five years. Ten individuals receive a lumpectomy surgery only and six of the ten individuals remain cancer free for five years. This Example demonstrates that a personalized cancer vaccine provides increased efficacy in breast cancer treatment compared to lumpectomy surgery only.

While preferred cases have been shown and described herein, it will be obvious to those skilled in the art that such cases are provided by way of example only. Numerous variations, changes, and substitutions will now occur to those skilled in the art. It should be understood that various alternatives to the cases described herein may be employed in practicing the invention. It is intended that the following claims define the scope of the invention and that methods and structures within the scope of these claims.

## Claims

1. A personalized vaccine composition for use in reducing tumor size, inhibiting tumor growth, reducing tumor burden, increasing survival, or increasing cancer-free survival in an individual wherein the vaccine composition comprises:
one or more peptides determined to have immunoreactivity with a biological sample of the cancer from the individual, wherein the one or more peptides comprise peptides encoded by a frameshifted mRNA expressed by a cancer cell, and wherein the one or more peptides comprise peptides having a sequence selected from SEQ ID NO: 1-7264, and
wherein administering the vaccine composition elicits an immune response in the individual against the cancer.

2. The vaccine composition for use according to claim 1, wherein the one or more peptides elicit a positive response in an antibody assay performed on the biological sample of the cancer from the individual.

3. The vaccine composition for use according to claim 1, wherein the one or more peptides elicit a positive response in a T cell assay performed on the biological sample of the cancer from the individual.

4. The vaccine composition for use according to claim 1, wherein the biological sample of the cancer is selected from the group consisting of blood, plasma, serum, thymus, bone marrow, spleen, lymph node, bronchoalveolar lavage, breast, central nervous system, cerebrospinal fluid, eye, tears, gastrointestinal tract, saliva, feces, urine, heart, kidney, liver, lung, muscle, pancreas, peripheral nervous system, saliva, skin, thyroid, trachea, and tumor.

5. The vaccine composition for use according to any one of claims 1 to 4, wherein determining immunoreactivity of the one or more peptides to the biological sample of the cancer is identified using antibody reactivity.

6. The vaccine composition for use according to any one of claims 1 to 5, wherein determining immunoreactivity of the one or more peptides to the biological sample of the cancer is performed using a T cell response.

7. The vaccine composition for use according to claim 1, wherein a frameshifted mRNA is created in a splicing error or a transcription insertion or deletion error.

8. The vaccine composition for use according to any one of claims 1 to 7, wherein the vaccine composition comprises a pharmaceutically acceptable adjuvant or excipient.

9. The vaccine composition for use according to any one of claims 1 to 8, wherein the cancer is selected from the group consisting of Acute lymphoblastic leukemia, Acute monocytic leukemia, Acute myeloid leukemia, Acute promyelocytic leukemia, Adenocarcinoma, Adult T-cell leukemia, Astrocytoma, Bladder cancer, Bone Cancer, Brain Tumor, Breast Cancer, Burkitt's lymphoma, Carcinoma, Cervical Cancer, Chronic Lymphocytic Leukemia, Chronic myelogenous leukemia, Colon Cancer, Colorectal cancer, Endometrial cancer, Glioblastoma multiforme, Glioma, Hepatocellular carcinoma, Hodgkin's lymphoma, Inflammatory breast cancer, Kidney Cancer, Leukemia, Lung cancer, Lymphoma, Malignant Mesothelioma, Medulloblastoma, Melanoma, Multiple myeloma, Neuroblastoma, Non-Hodgkin Lymphoma, Non-Small Cell Lung Cancer, Ovarian Cancer, Pancreatic Cancer, Pituitary tumor, Prostate cancer, Retinoblastoma, Skin Cancer, Small Cell Lung Cancer, Squamous cell carcinoma, Stomach cancer, T-cell leukemia, T-cell lymphoma, Thyroid cancer, and Wilms' tumor.

10. A method of determining whether an individual has a cancer, the method comprising:
(a) determining immunoreactivity of a biological sample of the cancer derived from the individual to the one or more peptides of claim 1; and
(b) determining that the individual has the cancer when the sample of the cancer derived from the individual is immunoreactive with the said one or more peptides.

11. The vaccine composition for use according to claim 1, wherein the vaccine composition includes 1, 2, 3, 4, 5, 6, 7, 8, 9, 10, 11, 12, 13, 14, 15, 16, 17, 18, 19, 20, or more frameshift peptides determined to have immunoreactivity with the biological sample of the cancer.

## Patentansprüche

1. Personalisierte Impfstoffzusammensetzung zur Verwendung bei der Verringerung der Tumorgröße, der Hemmung des Tumorwachstums, der Verringerung der Tumorlast, der Erhöhung des Überlebens oder der Erhöhung des krebsfreien Überlebens in einem Individuum, wobei die Impfstoffzusammensetzung Folgendes umfasst:
ein oder mehrere Peptide, von denen bestimmt wurde, dass sie Immunreaktivität mit einer biologischen Probe des Krebses des Individuums aufweisen, wobei das eine oder die mehreren Peptide Peptide umfassen, die von einer rahmenverschobenen mRNA kodiert werden, die von einer Krebszelle exprimiert wird, und wobei das eine oder die mehreren Peptide Peptide mit einer Sequenz umfassen, die aus SEQ ID NO: 1-7264 ausgewählt ist, und
wobei die Verabreichung der Impfstoffzusammensetzung eine Immunreaktion des Individuums gegen den Krebs hervorruft.

2. Impfstoffzusammensetzung zur Verwendung nach Anspruch 1, wobei das eine oder die mehreren Peptide eine positive Reaktion in einem Antikörperassay auslösen, der an der biologischen Probe des Krebses von dem Individuum durchgeführt wird.

3. Impfstoffzusammensetzung zur Verwendung nach Anspruch 1, wobei das eine oder die mehreren Peptide eine positive Reaktion in einem T-Zell-Assay hervorrufen, der an der biologischen Probe des Krebses des Individuums durchgeführt wird.

4. Impfstoffzusammensetzung zur Verwendung nach Anspruch 1, wobei die biologische Probe des Krebses ausgewählt ist aus der Gruppe bestehend aus Blut, Plasma, Serum, Thymus, Knochenmark, Milz, Lymphknoten, bronchoalveolärer Lavage, Brust, zentralem Nervensystem, Zerebrospinalflüssigkeit, Auge, Tränen, Magen-Darm-Trakt, Speichel, Fäkalien, Urin, Herz, Niere, Leber, Lunge, Muskel, Bauchspeicheldrüse, peripherem Nervensystem, Speichel, Haut, Schilddrüse, Luftröhre und Tumor.

5. Impfstoffzusammensetzung zur Verwendung nach einem der Ansprüche 1 bis 4, wobei die Bestimmung der Immunreaktivität des einen oder der mehreren Peptide gegenüber der biologischen Probe des Krebses unter Verwendung der Antikörperreaktivität identifiziert wird.

6. Impfstoffzusammensetzung zur Verwendung nach einem der Ansprüche 1 bis 5, wobei die Bestimmung der Immunreaktivität des einen oder der mehreren Peptide gegenüber der biologischen Probe des Krebses unter Verwendung einer T-Zell-Reaktion durchgeführt wird.

7. Impfstoffzusammensetzung zur Verwendung nach Anspruch 1, wobei eine rahmenverschobene mRNA durch einen Spleißfehler oder einen Transkriptionsinsertions- oder -deletionsfehler erzeugt wird.

8. Impfstoffzusammensetzung zur Verwendung nach einem der Ansprüche 1 bis 7, wobei die Impfstoffzusammensetzung ein pharmazeutisch annehmbares Adjuvans oder einen pharmazeutisch annehmbaren Hilfsstoff umfasst.

9. Impfstoffzusammensetzung zur Verwendung nach einem der Ansprüche 1 bis 8, wobei der Krebs ausgewählt ist aus der Gruppe bestehend aus akuter lymphoblastischer Leukämie, akuter monozytärer Leukämie, akuter myeloischer Leukämie, akuter promyelozytärer Leukämie, Adenokarzinom, adulter T-Zell-Leukämie, Astrozytom, Blasenkrebs, Knochenkrebs, Hirntumor, Brustkrebs, Burkitt-Lymphom, Karzinom, Gebärmutterhalskrebs, chronischer lymphatischer Leukämie, chronischer myeloischer Leukämie, Kolonkrebs, Kolorektalkrebs, Endometriumkrebs, Glioblastoma multiforme, Gliom, hepatozellulärem Karzinom, Hodgkin-Lymphom, entzündlichem Brustkrebs, Nierenkrebs, Leukämie, Lungenkrebs, Lymphom, malignem Mesotheliom, Medulloblastom, Melanom, multiplem Myelom, Neuroblastom, Non-Hodgkin-Lymphom, nicht-kleinzelligem Lungenkarzinom, Eierstockkrebs, Bauchspeicheldrüsenkrebs, Hypophysentumor, Prostatakrebs, Retinoblastom, Hautkrebs, kleinzelligem Lungenkrebs, Plattenepithelkarzinom, Magenkrebs, T-Zell-Leukämie, T-Zell-Lymphom, Schilddrüsenkrebs und Wilms-Tumor.

10. Verfahren zur Bestimmung, ob ein Individuum einen Krebs hat, wobei das Verfahren Folgendes umfasst:
a) Bestimmen der Immunreaktivität einer biologischen Probe des Krebses, die von dem Individuum stammt, gegenüber dem einen oder mehreren Peptiden nach Anspruch 1; und
b) Bestimmen, dass das Individuum den Krebs hat, wenn die von dem Individuum stammende Probe des Krebses mit dem einen oder den mehreren Peptiden immunreaktiv ist.

11. Impfstoffzusammensetzung zur Verwendung nach Anspruch 1, wobei die Impfstoffzusammensetzung 1, 2, 3, 4, 5, 6, 7, 8, 9, 10, 11, 12, 13, 14, 15, 16, 17, 18, 19, 20 oder mehr Frameshift-Peptide enthält, von denen bestimmt wurde, dass sie eine Immunreaktivität mit der biologischen Probe des Krebses haben.

## Revendications

1. Composition de vaccin personnalisée pour une utilisation dans la réduction de taille tumorale, l'inhibition de croissance tumorale, la réduction de charge tumorale, l'accroissement de survie, ou l'accroissement de survie sans rechute chez un individu, dans laquelle la composition de vaccin comprend :
un ou plusieurs peptides déterminés comme présentant une immunoréactivité avec un échantillon biologique du cancer provenant de l'individu, dans laquelle les un ou plusieurs peptides comprennent des peptides codés par un ARNm déphasé exprimé par une cellule cancéreuse, et dans laquelle les un ou plusieurs peptides comprennent des peptides ayant une séquence choisie parmi SEQ ID N° : 1 à 7264, et
dans laquelle l'administration de la composition de vaccin déclenche une réponse immunitaire chez l'individu contre le cancer.

2. Composition de vaccin pour une utilisation selon la revendication 1, dans laquelle les un ou plusieurs peptides déclenchent une réponse positive dans un dosage d'anticorps réalisé sur l'échantillon biologique du cancer provenant de l'individu.

3. Composition de vaccin pour une utilisation selon la revendication 1, dans laquelle les un ou plusieurs peptides déclenchent une réponse positive dans un dosage de lymphocytes T réalisé sur l'échantillon biologique du cancer provenant de l'individu.

4. Composition de vaccin pour une utilisation selon la revendication 1, dans laquelle l'échantillon biologique du cancer est choisi dans le groupe constitué par le sang, le plasma, le sérum, le thymus, la moelle osseuse, la rate, un ganglion lymphatique, un lavage bronchoalvéolaire, le sein, le système nerveux central, le liquide céphalo-rachidien, l'œil, les larmes, le tube digestif, la salive, les selles, l'urine, le cœur, le rein, le foie, le poumon, le muscle, le pancréas, le système nerveux périphérique, la salive, la peau, la thyroïde, la trachée, et une tumeur.

5. Composition de vaccin pour une utilisation selon l'une quelconque des revendications 1 à 4, dans laquelle la détermination de l'immunoréactivité des un ou plusieurs peptides à l'échantillon biologique du cancer est identifiée à l'aide d'une réactivité d'anticorps.

6. Composition de vaccin pour une utilisation selon l'une quelconque des revendications 1 à 5, dans laquelle la détermination de l'immunoréactivité des un ou plusieurs peptides à l'échantillon biologique du cancer est réalisée à l'aide d'une réponse des lymphocytes T.

7. Composition de vaccin pour une utilisation selon la revendication 1, dans laquelle un ARNm déphasé est créé dans une erreur épissage ou une insertion par transcription ou une erreur de délétion.

8. Composition de vaccin pour une utilisation selon l'une quelconque des revendications 1 à 7, dans laquelle la composition de vaccin comprend un adjuvant ou excipient pharmaceutiquement acceptable.

9. Composition de vaccin pour une utilisation selon l'une quelconque des revendications 1 à 8, dans laquelle le cancer est choisi dans le groupe constitué par la leucémie aiguë lymphoblastique, la leucémie aiguë monocytaire, la leucémie aiguë myéloïde, la leucémie aiguë promyélocytaire, l'adénocarcinome, la leucémie à cellules T de l'adulte, l'astrocytome, le cancer de la vessie, le cancer des os, une tumeur cérébrale, le cancer du sein, le lymphome de Burkitt, un carcinome, la cancer du col de l'utérus, la leucémie chronique lymphocytaire, la leucémie chronique myélogène, le cancer du côlon, le cancer colorectal, le cancer de l'endomètre, le glioblastome multiforme, le gliome, le carcinome hépatocellulaire, le lymphome de Hodgkin, le cancer du sein inflammatoire, le cancer du rein, la leucémie, le cancer du poumon, le lymphome, le mésothéliome malin, le médulloblastome, le mélanome, le myélome multiple, le neuroblastome, le lymphome non hodgkinien, le cancer du poumon non à petites cellules, le cancer de l'ovaire, le cancer du pancréas, une tumeur hypophysaire, le cancer de la prostate, le rétinoblastome, le cancer de la peau, le cancer du poumon à petites cellules, le carcinome épidermoïde, le cancer de l'estomac, la leucémie à cellules T, le lymphome à cellules T, le cancer de la thyroïde, et la tumeur de Wilms.

10. Procédé de détermination du fait qu'un individu est atteint ou non d'un cancer, le procédé comprenant :
(a) la détermination d'une immunoréactivité d'un échantillon biologique du cancer dérivé de l'individu face aux un ou plusieurs peptides de la revendication 1 ; et
(b) la détermination du fait que l'individu est atteint du cancer lorsque l'échantillon du cancer dérivé de l'individu est immunoréactif avec lesdits un ou plusieurs peptides.

11. Composition de vaccin pour une utilisation selon la revendication 1, dans laquelle la composition de vaccin comporte 1, 2, 3, 4, 5, 6, 7, 8, 9, 10, 11, 12, 13, 14, 15, 16, 17, 18, 19, 20 peptides déphasés ou plus qui sont déterminés comme présentant une immunoréactivité avec l'échantillon biologique du cancer.
